(19) Europäisches Patentamt
European Patent Office
Office européen des brevets

(11) EP 3 455 253 B1

(12) **EUROPEAN PATENT SPECIFICATION**

(45) Date of publication and mention of the grant of the patent:
**07.04.2021 Bulletin 2021/14**

(21) Application number: **17724512.3**

(22) Date of filing: **08.05.2017**

(51) Int Cl.:
***C07K 16/24*** *(2006.01)* ***C07K 14/715*** *(2006.01)*
***C07K 16/14*** *(2006.01)* ***C07K 16/28*** *(2006.01)*
***A61K 39/395*** *(2006.01)* ***C07K 14/705*** *(2006.01)*
***A61K 47/68*** *(2017.01)* ***A61P 35/00*** *(2006.01)*

(86) International application number:
**PCT/EP2017/060863**

(87) International publication number:
**WO 2017/194438 (16.11.2017 Gazette 2017/46)**

(54) **C-TERMINALLY FUSED TNF FAMILY LIGAND TRIMER-CONTAINING ANTIGEN BINDING MOLECULES**

C-TERMINAL-FUSIONIERTE ANTIGENBINDENDE MOLEKÜLE MIT LIGANDENTRIMER DER TNF-FAMILIE

MOLÉCULES DE LIAISON À UN ANTIGÈNE CONTENANT UN TRIMÈRE D'UN LIGAND DE LA FAMILLE DU TNF FUSIONNÉ EN TERMINAISON C

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2016 EP 16169237**

(43) Date of publication of application:
**20.03.2019 Bulletin 2019/12**

(73) Proprietor: **F. Hoffmann-La Roche AG**
**4070 Basel (CH)**

(72) Inventors:
- **BRUENKER, Peter**
  **8335 Hittnau (CH)**
- **KOLLER, Claudia Ferrara**
  **6300 Zug (CH)**
- **GRAU-RICHARDS, Sandra**
  **8903 Birmensdorf (CH)**
- **KLEIN, Christian**
  **8906 Bonstetten (CH)**
- **MOESSNER, Ekkehard**
  **8280 Kreuzlingen (CH)**
- **UMANA, Pablo**
  **8832 Wollerau (CH)**
- **AMANN, Maria**
  **8600 Duebendorf (CH)**
- **CLAUS, Christina**
  **Ennetbaden 5408 (CH)**
- **XU, Wei**
  **8952 Schlieren (CH)**

(74) Representative: **Klostermeyer-Rauber, Dörte**
**F. Hoffmann-La Roche AG**
**Corporate Law Patents (CLP)**
**Grenzacherstrasse 124**
**4070 Basel (CH)**

(56) References cited:
**WO-A1-2010/010051** **WO-A1-2012/130471**
**WO-A1-2014/161845** **WO-A1-2014/180754**
**WO-A1-2015/183902** **WO-A1-2015/184203**
**WO-A1-2016/075278** **WO-A1-2016/156291**
**WO-A2-2004/039841** **WO-A2-2012/020006**
**AU-A1- 2011 265 482** **US-A1- 2013 287 802**

- **MÜLLER DAFNE ET AL: "A novel antibody-4-1BBL fusion protein for targeted costimulation in cancer immunotherapy", JOURNAL OF IMMUNOTHERAPY, LIPPINCOTT WILLIAMS & WILKINS, USA, vol. 31, no. 8, 1 October 2008 (2008-10-01), pages 714-722, XP009183889, ISSN: 1537-4513**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 455 253 B1

- HORNIG NORA ET AL: "Combination of a Bispecific Antibody and Costimulatory Antibody-Ligand Fusion Proteins for Targeted Cancer Immunotherapy", JOURNAL OF IMMUNOTHERAPY, LIPPINCOTT WILLIAMS & WILKINGS INC, US, vol. 35, no. 5, 1 June 2012 (2012-06-01), pages 418-429, XP009163394, ISSN: 1524-9557, DOI: 10.1097/CJI.0B013E3182594387
- V. KERMER ET AL: "Combining antibody-directed presentation of IL-15 and 4-1BBL in a trifunctional fusion protein for cancer immunotherapy.", MOLECULAR CANCER THERAPEUTICS, vol. 13, no. 1, 6 November 2013 (2013-11-06), pages 112-121, XP055200723, US ISSN: 1535-7163, DOI: 10.1158/1535-7163.MCT-13-0282
- NICOLE MÜLLER ET AL: "Activity of soluble OX40 ligand is enhanced by oligomerization and cell surface immobilization", FEBS JOURNAL, vol. 275, no. 9, 1 May 2008 (2008-05-01), pages 2296-2304, XP055015512, ISSN: 1742-464X, DOI: 10.1111/j.1742-4658.2008.06382.x
- BAUER: "Targeted bioactivity of membrane-anchored TNF by an antibody-derived TNF fusion protein", THE JOURNAL OF IMMUNOLOGY, vol. 172, no. 6, 1 January 2004 (2004-01-01), page 3930, XP055005065, ISSN: 0022-1767
- EDWIN BREMER: "Targeting of the Tumor Necrosis Factor Receptor Superfamily for Cancer Immunotherapy", ISRN ONCOLOGY, vol. 176, no. 2, 1 January 2013 (2013-01-01), pages 974-25, XP055184622, ISSN: 2090-5661, DOI: 10.1186/1479-5876-9-204

## Description

## FIELD OF THE INVENTION

The invention relates to novel 4-1BB ligand (4-1BBL) trimer-containing antigen binding molecules comprising (a) a Fab domain capable of specific binding to a target cell antigen, (b) a Fc domain composed of a first and a second subunit capable of stable association, and (c) a a first polypeptide comprising two ectodomains of 4-1BBL comprising the amino acid sequence of SEQ ID NO:5 that are connected to each other by a peptide linker and a second polypeptide comprising one ectodomain of said 4-1BBL comprising the amino acid sequence of SEQ ID NO:5, wherein the first polypeptide is fused at its N-terminus to the C-terminus to the first subunit of the Fc domain comprising the amino acid substitutions Y349C, T366S, L368A and Y407V (numbering according to Kabat EU index) of the hole and wherein the second polypeptide is fused at its N-terminus to the C-terminus of the second subunit of the Fc domain the amino acid substitutions S354C and T366W (numbering according to Kabat EU index) of the knob and wherein the VH and CH1 domain of the Fab domain capable of specific binding to a target cell antigen is fused at the C-terminus to the N-terminus of the second subunit of the Fc domain comprising the knob mutations, wherein the TNF family ligand trimer-containing antigen binding molecule is monovalent for the binding to the target cell antigen.. The invention further relates to methods of producing these molecules and to methods of using the same.

## BACKGROUND

Ligands interacting with molecules of the TNF (tumor necrosis factor) receptor superfamily have pivotal roles in the organization and function of the immune system. While regulating normal functions such as immune responses, hematopoiesis and morphogenesis, the TNF family ligands (also called cytokines) play a role in tumorgenesis, transplant rejection, septic shock, viral replication, bone resorption, rheumatoid arthritis and diabetes (Aggarwal, 2003). The TNF ligand family comprises 18 genes encoding 19 type II (i.e. intracellular N terminus and extracellular C-terminus) transmembrane proteins, characterized by the presence of a conserved C-terminal domain coined the 'TNF homology domain' (THD). This domain is responsible for receptor binding and is thus critical for the biological activity of the TNF ligand family members. The sequence identity between family members is ~20-30% (Bodmer, 2002). Members of the TNF ligand family exert their biological function as self-assembling, noncovalent trimers (Banner et al, 1993). Thus, the TNF family ligands form a trimer that is able to bind to and to activate the corresponding receptors of TNFR superfamily.

4-1BB (CD137), a member of the TNF receptor superfamily, has been first identified as a molecule whose expression is induced by T-cell activation (Kwon and Weissman, 1989). Subsequent studies demonstrated expression of 4-1BB in T- and B-lymphocytes (Snell et al., 2011; Zhang et al., 2010), NK-cells (Lin et al., 2008), NKT-cells (Kim et al., 2008), monocytes (Kienzle and von Kempis, 2000; Schwarz et al., 1995), neutrophils (Heinisch et al., 2000), mast (Nishimoto et al., 2005) and dendritic cells as well as cells of non-hematopoietic origin such as endothelial and smooth muscle cells (Broll et al., 2001; Olofsson et al., 2008). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., 2002; von Kempis et al., 1997; Zhang et al., 2010).

Expression of 4-1BB ligand (4-1BBL or CD137L) is more restricted and is observed on professional antigen presenting cells (APC) such as B-cells, dendritic cells (DCs) and macrophages. Inducible expression of 4-1BBL is characteristic for T-cells, including both $\alpha\beta$ and $\gamma\delta$ T-cell subsets, and endothelial cells (reviewed in Shao and Schwarz, 2011).

CD137 signaling is known to stimulate IFN$\gamma$ secretion and proliferation of NK cells (Buechele et al., 2012; Lin et al., 2008; Melero et al., 1998) as well as to promote DC activation as indicated by their increased survival and capacity to secret cytokines and upregulate co-stimulatory molecules (Choi et al., 2009; Futagawa et al., 2002; Wilcox et al., 2002). However, CD137 is best characterized as a co-stimulatory molecule which modulates TCR-induced activation in both the $CD4^+$ and $CD8^+$ subsets of T-cells. In combination with TCR triggering, agonistic 4-1BB-specific antibodies enhance proliferation of T-cells, stimulate lymphokine secretion and decrease sensitivity of T-lymphocytes to activation-induced cells death (reviewed in (reviewed in Snell et al., 2011).

In line with these co-stimulatory effects of 4-1BB antibodies on T-cells in vitro, their administration to tumor bearing mice leads to potent anti-tumor effects in many experimental tumor models (Melero et al., 1997; Narazaki et al., 2010). However, 4-1BB usually exhibits its potency as an anti-tumor agent only when administered in combination with other immunomodulatory compounds (Curran et al., 2011; Guo et al., 2013; Morales-Kastresana et al., 2013; Teng et al., 2009; Wei et al., 2013), chemotherapeutic reagents (Ju et al., 2008; Kim et al., 2009), tumor-specific vaccination (Cuadros et al., 2005; Lee et al., 2011) or radiotherapy (Shi and Siemann, 2006). In vivo depletion experiments demonstrated that $CD8^+$ T-cells play the most critical role in anti-tumoral effect of 4-1BB-specific antibodies. However, depending on the tumor model or combination therapy, which includes anti-4-1BB, contributions of other types of cells such as DCs, NK-cells or $CD4^+$ T-cells have been reported (Melero et al., 1997; Murillo et al., 2009; Narazaki et al., 2010; Stagg et al., 2011).

In addition to their direct effects on different lymphocyte subsets, 4-1BB agonists can also induce infiltration and retention of activated T-cells in the tumor through 4-1BB-mediated upregulation of intercellular adhesion molecule 1 (ICAM1) and vascular cell adhesion molecule 1 (VCAM1) on tumor vascular endothelium (Palazon et al., 2011).

4-1BB triggering may also reverse the state of T-cell anergy induced by exposure to soluble antigen that may contribute to disruption of immunological tolerance in the tumor microenvironment or during chronic infections (Wilcox et al., 2004).

It appears that the immunomodulatory properties of 4-1BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li and Ravetch, 2011; Teng et al., 2009). However, systemic administration of 4-1BB-specific agonistic antibodies with the functionally active Fc domain also induces expansion of $CD8^+$ T-cells associated with liver toxicity (Dubrot et al., 2010) that is diminished or significantly ameliorated in the absence of functional Fc-receptors in mice. In human clinical trials (ClinicalTrials.gov, NCT00309023), Fc-competent 4-1BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone and Ascierto, 2012).

Collectively, the available pre-clinical and clinical data clearly demonstrate that there is a high clinical need for effective 4-1BB agonists. However, new generation drug candidates should not only effectively engage 4-1BB on the surface of hematopoietic and endothelial cells but also be capable of achieving that through mechanisms other than binding to Fc-receptors in order to avoid uncontrollable side effects. The latter may be accomplished through preferential binding to and oligomerization on tumor-specific or tumor-associated moieties.

Fusion proteins composed of one extracellular domain of a 4-1BB ligand and a single chain antibody fragment (Mueller et al., 2008; Hornig et al., 2012) or a single 4-1BB ligand fused to the C-terminus of a heavy chain (Zhang et al, 2007) have been made. Mueller et al., J. Immunotherapy 2008, 31, 714-722, disclose a fusion protein as homo trimeric molecule wherein a VH-VL single chain fragment (scFv) of the FAP antibody scFv36 is connected via an amino acid linker to the extracellular domain of 4-1BBL. Hornig et al., J. Immunotherapy 2012, 35, 418-429, describe a similar fusion protein, wherein the antibody moiety A5, specific for human endoglin (EDG,) is arranged in the scFv format and connected via a peptide linker to an extracellular domain of 4-1BBL. WO 2010/010051 discloses the generation of fusion proteins that consist of three TNF ligand ectodomains linked to each other and fused to an antibody part.

However, there is still a need of new antigen binding molecules that combine a Fab domain capable of preferred binding to tumor-specific or tumor-associated targets with a moiety capable of forming a costimulatory TNF ligand trimer and that have at the same time sufficient stability to be pharmaceutically useful. The antigen binding molecules of the present invention comprise both and surprisingly they provide a trimeric and thus biologically active TNF ligand, although one of the trimerizing TNF ligand ectodomains is located on another polypeptide than the other two TNF ligand ectodomains of the molecule. Surprisingly, it has been found that one Fab domain capable of specific binding to a target cell antigen is sufficient to provide the preferred targeting properties. The antigen binding molecules of this invention are particularly stable and robust.

## SUMMARY OF THE INVENTION

In one aspect, the invention provides a 4-1BB ligand (4-1BBL) trimer-containing antigen binding molecule comprising

(a) one Fab domain capable of specific binding to a target cell antigen,
(b) a Fc domain composed of a first and a second subunit capable of stable association, and
(c) a first polypeptide comprising two ectodomains of 4-1BBL comprising the amino acid sequence of SEQ ID NO:5 that are connected to each other by a peptide linker and a second polypeptide comprising one ectodomain of said 4-1BBL comprising the amino acid sequence of SEQ ID NO:5, wherein the first polypeptide is fused at its N-terminus to the C-terminus to the first subunit of the Fc domain comprising the amino acid substitutions Y349C, T366S, L368A and Y407V (numbering according to Kabat EU index) of the hole and wherein the second polypeptide is fused at its N-terminus to the C-terminus of the second subunit of the Fc domain the amino acid substitutions S354C and T366W (numbering according to Kabat EU index) of the knob and wherein the VH and CH1 domain of the Fab domain capable of specific binding to a target cell antigen is fused at the C-terminus to the N-terminus of the second subunit of the Fc domain comprising the knob mutations, wherein the TNF family ligand trimer-containing antigen binding molecule is monovalent for the binding to the target cell antigen.

In a particular aspect, the 4-1BB ligand costimulates human T-cell activation. Thus, the TNF family ligand trimer-containing antigen binding molecule comprises (a) one Fab domain capable of specific binding to a target cell antigen, (b) a Fc domain composed of a first and a second subunit capable of stable association, and
(c) a first polypeptide comprising two ectodomains of 4-1BBL comprising the amino acid sequence of SEQ ID NO:5 that are connected to each other by a peptide linker and a second polypeptide comprising one ectodomain of said 4-1BBL comprising the amino acid sequence of SEQ ID NO:5, wherein the first polypeptide is fused at its N-terminus to the C-terminus to the first subunit of the Fc domain comprising the amino acid substitutions Y349C, T366S, L368A and Y407V (numbering according to Kabat EU index) of the hole and wherein the second polypeptide is fused at its N-terminus to the C-terminus of the second subunit of the Fc domain the amino acid substitutions S354C and T366W (numbering according to Kabat EU index) of the knob and wherein the VH and CH1 domain of the Fab domain capable of specific binding to a target cell antigen is fused at the C-terminus to the N-terminus of the second subunit of the Fc domain comprising the knob mutations, wherein the TNF family ligand trimer-containing antigen binding molecule is monovalent for the binding to the target cell antigen and wherein the 4-1BBL costimulates human T-cell activation.

In another aspect, provided is a 4-1BBL trimer-containing antigen binding molecule of the invention, wherein the target cell antigen is selected from the group consisting of Fibroblast Activation Protein (FAP), CD19, Carcinoembryonic Antigen (CEA), Melanoma-associated Chondroitin Sulfate Proteoglycan (MCSP), Epidermal Growth Factor Receptor (EGFR), CD20 and CD33.

In a particular aspect, the target cell antigen is Fibroblast Activation Protein (FAP).

In one aspect, the invention provides a 4-1BBL trimer-containing antigen binding molecule, wherein the Fab domain capable of specific binding to FAP comprises

(a) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO: 15, and a VL domain comprising (iv) CDR-L1 comprising the amino acid sequence of SEQ ID NO:16, (v) CDR-L2 comprising the amino acid sequence of SEQ ID NO: 17 and (vi) CDR-L3 comprising the amino acid sequence of SEQ ID NO:18 or
(b) a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

In a particular aspect, the invention provides a 4-1BBL trimer-containing antigen binding molecule, wherein the Fab domain capable of specific binding to FAP comprises a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

In a further aspect, provided is a 4-1BBL trimer-containing antigen binding molecule as described herein before, wherein the Fab domain capable of specific binding to FAP comprises a variable heavy chain comprising an amino acid sequence of SEQ ID NO:25 and a variable light chain comprising an amino acid sequence of SEQ ID NO:26 or wherein the Fab domain capable of specific binding to FAP comprises a variable heavy chain comprising an amino acid sequence of SEQ ID NO:27 and a variable light chain comprising an amino acid sequence of SEQ ID NO:28.

In another particular aspect, the target cell antigen is CD19.

In one aspect, the invention provides a 4-1BBL trimer-containing antigen binding molecule, wherein the Fab domain capable of specific binding to CD19 comprises

(a) a VH domain comprising (i) CDR-H1 comprising the amino acid sequence of SEQ ID NO:29, (ii) CDR-H2 comprising the amino acid sequence of SEQ ID NO:30 and (iii) CDR-H3 comprising the amino acid sequence of SEQ ID NO:31, and a VL domain comprising (iv) CDR-L1 comprising the amino acid sequence of SEQ ID NO:32, (v) CDR-L2 comprising the amino acid sequence of SEQ ID NO:33 and (vi) CDR-L3 comprising the amino acid sequence of SEQ ID NO:34 or
(b) a VH domain comprising (i) CDR-H1 comprising the amino acid sequence of SEQ ID NO:35, (ii) CDR-H2 comprising the amino acid sequence of SEQ ID NO:36 and (iii) CDR-H3 comprising the amino acid sequence of SEQ ID NO:37, and a VL domain comprising (iv) CDR-L1 comprising the amino acid sequence of SEQ ID NO:38, (v) CDR-L2 comprising the amino acid sequence of SEQ ID NO:39 and (vi) CDR-L3 comprising the amino acid sequence of SEQ ID NO:40.

In a particular aspect, the invention provides a 4-1BBL trimer-containing antigen binding molecule, wherein the Fab domain capable of specific binding to CD19 comprises a VH domain comprising (i) CDR-H1 comprising the amino acid sequence of SEQ ID NO:35, (ii) CDR-H2 comprising the amino acid sequence of SEQ ID NO:36 and (iii) CDR-H3 comprising the amino acid sequence of SEQ ID NO:37, and a VL domain comprising (iv) CDR-L1 comprising the amino acid sequence of SEQ ID NO:38, (v) CDR-L2 comprising the amino acid sequence of SEQ ID NO:39 and (vi) CDR-L3 comprising the amino acid sequence of SEQ ID NO:40.

In a further aspect, provided is a 4-1BBL trimer-containing antigen binding molecule as described herein before, wherein the Fab domain capable of specific binding to CD19 comprises a variable heavy chain comprising an amino acid sequence of SEQ ID NO:41 and a variable light chain comprising an amino acid sequence of SEQ ID NO:42 or wherein the Fab domain capable of specific binding to FAP comprises a variable heavy chain comprising an amino acid sequence of SEQ ID NO:43 and a variable light chain comprising an amino acid sequence of SEQ ID NO:44.

In another particular aspect, the target cell antigen is CEA.

In one aspect, the invention provides a 4-1BBL trimer-containing antigen binding molecule, wherein the Fab domain capable of specific binding to CEA comprises a VH domain comprising (i) CDR-H1 comprising the amino acid sequence of SEQ ID NO:45, (ii) CDR-H2 comprising the amino acid sequence of SEQ ID NO:46 and (iii) CDR-H3 comprising the amino acid sequence of SEQ ID NO:47, and a VL domain comprising (iv) CDR-L1 comprising the amino acid sequence of SEQ ID NO:48, (v) CDR-L2 comprising the amino acid sequence of SEQ ID NO:49 and (vi) CDR-L3 comprising the amino acid sequence of SEQ ID NO:50.

In a particular aspect, provided is a 4-1BBL trimer-containing antigen binding molecule as described herein before, wherein the Fab domain capable of specific binding to CEA comprises a variable heavy chain comprising an amino acid sequence of SEQ ID NO:51 and a variable light chain comprising an amino acid sequence of SEQ ID NO:52.

In a further aspect, the Fc domain is an IgG, particularly an IgG1 Fc domain or an IgG4 Fc domain. More particularly, the Fc domain is an IgG1 Fc domain.

In particular, the 4-1BBL trimer-containing antigen binding molecule of the invention comprises a Fab domain capable of specific binding to a target cell antigen, wherein the VH and CH1 domain of the Fab domain are fused at the C-terminus to the N-terminus of a CH2 domain in the Fc domain.

In another aspect, the invention is concerned with a 4-1BBL trimer-containing antigen binding molecule as defined herein before, comprising (b) an Fc domain composed of a first and a second subunit capable of stable association, wherein the Fc domain comprises one or more amino acid substitution that reduces binding to an Fc receptor, in particular towards Fcγ receptor.

In particular, the Fc domain comprises amino acid substitutions at positions 234 and 235 (numbering according to Kabat EU index) and/or 329 (numbering according to Kabat EU index) of the IgG heavy chains. More particularly, provided is a trimeric 4-1BBL-containing antigen binding molecule according to the invention which comprises an IgG1 Fc domain with the amino acid substitutions L234A, L235A and P329G (numbering according to Kabat EU index).

According to another aspect of the invention, there is provided an isolated polynucleotide encoding a 4-1BBL trimer-containing antigen binding molecule as defined herein before. The invention further provides a vector, particularly an expression vector, comprising the isolated polynucleotide of the invention and a host cell comprising the isolated polynucleotide or the vector of the invention. In some embodiments the host cell is a eukaryotic cell, particularly a mammalian cell.

In another aspect, provided is a method for producing the 4-1BBL trimer-containing antigen binding molecule of the invention, comprising the steps of (i) culturing the host cell of the invention under conditions suitable for expression of the antigen binding molecule, and (ii) recovering the antigen binding molecule. The invention also encompasses a 4-1BBL-containing antigen binding molecule produced by the method of the invention.

The invention further provides a pharmaceutical composition comprising the 4-1BBL trimer-containing antigen binding molecule of the invention and at least one pharmaceutically acceptable excipient.

Also encompassed by the invention is the 4-1BBL trimer-containing antigen binding molecule of the invention, or the pharmaceutical composition of the invention, for use as a medicament. In one aspect is provided the 4-1BBL-containing antigen binding molecule of the invention, or the pharmaceutical composition of the invention, for use in the treatment of a disease in an individual in need thereof. The present invention also provides the 4-1BBL trimer-containing antigen binding molecule of the invention, or the pharmaceutical composition of the invention, for use (i) in stimulating T cell response, (ii) in supporting survival of activated T cells, (iii) in the treatment of infections, (iv) in the treatment of cancer, (v) in delaying progression of cancer, or (vi) in prolonging the survival of a patient suffering from cancer. In a specific embodiment, provided is the 4-1BBL trimer-containing antigen binding molecule of the invention, or the pharmaceutical composition of the invention, for use in the treatment of cancer.

Also provided is the use of the 4-1BBL trimer-containing antigen binding molecule of the invention for the manufacture of a medicament for the treatment of a disease in an individual in need thereof, in particular for the manufacture of a medicament for the treatment of cancer. The present invention also provides the use of the 4-1BBL trimer-containing antigen binding molecule of the invention, or the pharmaceutical composition of the invention, in the manufacture of a medicament for up-regulating or prolonging cytotoxic T cell activity. In any of the above embodiments the individual is preferably a mammal, particularly a human.

## BRIEF DESCRIPTION OF THE DRAWINGS

**Figure 1** shows the components for the assembly of split trimeric human 4-1BB ligands. Figure (1A) shows the polypeptide comprising the dimeric 4-1BB ligand that is fused at the N-terminus to the C-terminus of a human CH3 domain by a peptide linker $(G4S)_2$ and Figure (1B) shows the polypeptide comprising the monomeric 4-1BB ligand that is fused at the N-terminus to the C-terminus of the other CH3 domain within a Fc domain by a peptide linker $(G4S)_2$. Figure (1C) shows a dimeric OX40 ligand that is fused at the N-terminus by a peptide linker $(G4S)_2$ to the C-terminus of a human CH3 domain and Figure (ID) shows a monomeric ligand fused at the N-terminus to the C-terminus of the other human CH3 domain within the Fc domain by a peptide linker $(G4S)_2$.

**Figures 2A to 2D** are schematic drawings of the structures of the monovalent targeted 4-1BBL-trimer-containing antigen binding molecules of the invention. In **Figure 2A** is shown the antigen binding molecule as construct of type x.11, wherein the Fab domain capable of specific binding to a target cell antigen is attached to the Fc knob chain, the dimeric 4-1BBL is fused to the C-terminus of the Fc hole chain and the monomeric 4-1BBL is fused to the C-terminus of the Fc knob chain. Constructs 2.11, 1.11, 3.11, 4.11, 5.11 and Control H are corresponding examples. A construct of type x.12 is shown in **Figure 2B.** The Fab domain capable of specific binding to a target cell antigen is attached to the Fc knob chain, the dimeric 4-1BBL is also fused to the C-terminus of the Fc knob chain and the monomeric 4-1BBL is fused to the C-terminus of the Fc hole chain. Constructs 2.12, 1.12, 3.12, 4.12, 5.12 and Control I are examples for these antigen binding molecules. In **Figure 2C** is shown the antigen binding molecule as construct of type x.13, wherein the Fab domain capable of specific binding to a target cell antigen is attached to the Fc hole chain, the dimeric 4-1BBL is fused to the C-terminus of the Fc hole chain and the monomeric 4-1BBL is fused to the C-terminus of the Fc knob chain. Constructs 2.13, 1.13, 3.13, 4.13, 5.13 and Control J are corresponding examples. **Figure 2D** relates to constructs of type x.14, wherein the Fab domain capable of specific binding to a target cell antigen is attached to the Fc hole chain, the dimeric 4-1BBL is also fused to the C-terminus of the Fc knob chain and the monomeric 4-1BBL is fused to the C-terminus of the Fc hole chain. Constructs 2.14, 1.14, 3.14, 4.14, 5.14 and Control K are examples therefore.The preparation of all these constructs is described in Example 1. The VH, VL, CL and CH1 domains symbolize the Fab domain, the thick black point stands for the knob-into-hole modification.

**Figures 3A to 3D** are schematic drawings of further molecules described herein. **Figure 3A** shows Control F, a hu IgG1 with two DP47 (germline) untargeted Fab domains. **Figure 3B** shows Control D, an untargeted 4-1BBL trimer-containing antigen binding molecule, wherein the dimeric 4-1BBL is fused to the N-terminus of CL domain in the Fc knob heavy chain and the monomeric 4-1BBL is fused to the N-terminus of a CH1 domain of a light chain. * symbolizes amino acid modifications in the CH1 and CL domain (so-called charged residues). **Figure 3C** shows the same construct as Figure 3B, however the DP47 Fab domain is replaced by FAP(4B9) Fab domain. The preparation and production of these constructs is described in Example 1.11. The thick black point stands for the knob-into-hole modification. **Figure 3D** is a drawing of the monomeric 4-1BB Fc(kih) construct as prepared in Example 3.1.

**Figure 4A** shows the setup of the SPR experiments for simultaneous binding of the monovalent targeted split trimeric C-terminal 4-1BB ligand-containing antigen binding molecules of the invention. The simultaneous binding to human 4-1BB and human FAP of the FAP (4B9)-targeted split 4-1BBL (71-248) Fc kih antigen binding molecule Construct 2.11 is shown in **Figure 4B.** Simultaneous binding to human 4-1BB and human CD19 of CD19(8B8-018)-targeted split 4-1BBL (71-248) Fc kih antigen binding molecule (Construct 3.11) is shown in **Figure 4C.** Simultaneous binding to human 4-1BB and human CD19 of the CD19(8B8-2B11)-targeted split 4-1BBL (71-248) Fc kih antigen binding molecule (construct 4.11) is shown in **Figure 4D.**

**Figures 5A to 5D** are schematic drawings of the structures of the monovalent targeted 4-1BBL-trimer-containing antigen binding molecules of the invention that comprise a further Fab domain that is not capable for binding to a target cell antigen. In **Figure 5A** is shown the antigen binding molecule as construct of type x.15, wherein the Fab domain not capable of specific binding to a target cell antigen (DP47 germline) is attached to the Fc knob chain, the Fab domain capable of specific binding to the antigen (FAP) is connected to the Fc hole chain, the dimeric 4-1BBL is fused to the C-terminus of the Fc hole chain and the monomeric 4-1BBL is fused to the C-terminus of the Fc knob chain. Construct 2.15 is an example thereof. A construct of type x.16 is shown in Figure **5B.** The Fab domain not capable of specific binding to a target cell antigen (DP47 germline) is attached to the Fc knob chain, the Fab domain capable of specific binding to the antigen (FAP) is connected to the Fc hole chain, the dimeric 4-1BBL is also fused to the C-terminus of the Fc knob chain and the monomeric 4-1BBL is fused to the C-terminus of the Fc hole chain. Construct 2.16 is an example thereof. In Figure 5C is shown the antigen binding molecule as construct of type x.17, wherein the Fab domain capable of specific binding to a target cell antigen is attached to the Fc knob chain, the Fab domain not capable of specific binding to a target cell antigen (DP47 germline) is connected to the Fc hole chain, the dimeric 4-1BBL is fused to the C-terminus of the Fc hole chain and the monomeric 4-1BBL is fused to the C-terminus of the Fc knob chain. Construct 2.17 is an example thereof. Figure **5D** relates to constructs of type x.18, wherein the Fab domain capable of specific binding to a target cell antigen is attached to the Fc knob chain, the Fab domain not capable of specific binding to a target cell antigen (DP47 germline) is attached to the Fc hole chain, the dimeric 4-1BBL is also fused to the C-terminus of the Fc knob chain and the monomeric 4-1BBL is fused to the C-terminus of the Fc hole chain. Construct 2.18 is an example thereof. The preparation of all these constructs is described in Example 4.

**Figure 6A** shows the setup of the SPR experiments for simultaneous binding of the DP47/ monovalent FAP targeted split trimeric C-terminal 4-1BB ligand-containing antigen binding molecules of the invention. The simultaneous binding to human 4-1BB and human FAP of the DP47/ FAP (4B9)-targeted split 4-1BBL (71-254) Fc kih antigen binding molecule Construct 2.15 is shown in **Figure 6B.**

In Figures 7A to 7H is shown the binding of FAP-targeted split trimeric 4-1BB ligand Fc fusion antigen binding molecules to freshly isolated PBMCs. Shown is the geometric (geo) mean of fluorescence intensity of PE-conjugated secondary detection antibody versus the concentration of tested constructs. As negative controls control F and control D and as positive control construct 2.4 (described in Example 1.11) were used. In Figures 7A to 7D the binding of Construct 2.11 and in Figures 7E to 7H the binding of Construct 2.15 is illustrated. The structure of construct 2.11 is shown in Figure 2A and the structure of Construct 2.15 is shown in Figure 5A. Binding was monitored on fresh human $CD45^+$ $CD3^+$ $CD8^{neg}$ $CD4^+$ T cells (Figures 7A or 7E), fresh human $CD45^+$ $CD3^+$ $CD4^{neg}$ $CD8^+$ T cells (Figures 7B or 7F), fresh human $CD45^+$ $CD3^+$ $CD4^{neg}$ $CD8^{neg}$ $\gamma\delta$ T cells (Figures 7C or 7G) and $CD45^+$ $CD3^{neg}$ $CD19^+$ B cells (Figures 7D or 7H). Because the majority of fresh isolated PBMCs do not express 4-1BB or FAP, no binding could be detected.

Figures 8A to 8F illustrate the binding of FAP-targeted split trimeric 4-1BB ligand Fc fusion antigen binding molecules to activated human PBMCs. Shown is the geo mean of fluorescence intensity of PE-conjugated secondary detection antibody versus the concentration of tested constructs. As negative controls control F and control D and as positive control construct 2.4 as described in Example 1.11 were used. In Figures 8A to 8C the binding of Construct 2.11 and in Figures 8D to 8F the binding of Construct 2.15 is illustrated. After activation with agonistic anti-human CD28 and anti-human CD3 antibody 4-1BB expression is upregulated on the majority of $CD3^+$ $CD8^+$ T cells and to a lesser extend to the majority $CD3^+$ $CD4^+$ and $CD3^+$ $CD4^{neg}$ $CD8^{neg}$ $\gamma\delta$ T cells. All constructs containing a fused 4-1BB split trimeric ligand (control D, construct 2.4, constructs 2.11 and 2.15) bind with a quite similar affinity to human 4-1BB, independent if they are FAP (4B9)-targeted (construct 2.4 and 2.11) or DP47-untargeted (control D), whereas the untargeted molecule without a fused 4-1BB split trimeric ligand (control F) does not bind to the activated human T cells.

Figures 9A to 9D show the binding of FAP-targeted or untargeted split trimeric human 4-1BB ligand Fc (kih) fusion antigen binding molecules to human-FAP expressing human melanoma MV-3 cells and WM-266-4 cells. Binding was detected with R-Phycoerythrin-fluorochrome conjugated anti-human IgG Fcγ-specific goat IgG F(ab')2 fragment. As negative controls control F and D and as positive control Construct 2.4 as described in Example 1.11 were used. Shown is the geo mean of fluorescence intensity versus the concentration of tested constructs and controls. In **Figures 9A** (MV-3 cells) and **9B** (WM-266-4 cells) the binding of Construct 2.11 in comparison to the control molecules is shown and in **Figures 9C** (MV-3 cells) and **9D** (WM-266-4 cells) the binding of Construct 2.15 in comparison to the control molecules is shown. All FAP-targeted constructs bind with a similar affinity to human FAP.

Figure 10 shows a scheme that illustrates the general principal of the NFkB activity assay described in Example 6.3 using a reporter cell line. Shown is the activation assay set up with human 4-1BB expressing HeLa reporter cell line. A crosslinking of 4-1BB expressed on the reporter cells induces NFκB activation and NFκB-mediated Luciferase expression. After lysis of the cells Luciferase can catalyze the oxidation of Luciferin to Oxyluciferin. This chemical reaction correlates positively with the strength of NFκB-mediated luciferase expression and can be measured by the strength of light emission (units of released light).

In Figure 11 it is shown that the activation of the NFkB signaling pathway by FAP-targeted 4-1BB ligand trimer-containing Fc(kih) fusion antigen binding molecule is strictly dependent on its binding to FAP-expressing target cells. Human 4-1BB expressing NFkB reporter HeLa cells were co-cultured with the indicated tumor cells exhibiting different levels of cell surface FAP expression. Units of released light (URL) are measured for 0.5 s/well and plotted against the used concentration of Construct 2.11 or Construct 2.15 or (as positive control) Construct 2.4 or (as negative controls) Control D or F. Human 4-1BB-expressing HeLa-reporter cells were incubated for 6 h in the absence (**Figure 11A**) or presence of crosslinking human-FAP expressing human melanoma cell line MV-3 (see Figure 11B) or 3T3-huFAP clone 19 (see Figure 11C) and then Luciferase activity was assessed as described in Example 6.3. The cell ratio of 4-1BB-expressing HeLa reporter cell to tumor cells are indicated in each set up.

Figure 12 shows a schematic scheme of the human PBMC Activation assas as described in Example 6.4. Activation of PBMCs is achieved in the presence of FAP-expressing Fibroblasts, agonistic anti-human CD3 antibody and bispecific anti-FAP split trimeric 4-1BBL fusion antigen binding molecule construct 2.11. The content per well in the plate was: 45 Gy irradiated $2x10^4$ NIH/3T3-human FAP clone 19, 7.5 x $10^6$ CFSE-labeled human PBMCs, 0.5 nM agonistic anti-human CD3 human IgG wt (clone V9) and $10^{-5}$ nM to 10 nM titrated concentrations of construct 2.11, construct 2.4, control D or control F. The incubation time was 4 days.

In Figures **13A** and **13B** results in the human PBMC activation assay in the presence of FAP+ fibroblasts, agonistic CD3 antibody and FAP-targeted split trimeric 4-1BBL fusion molecules are shown. The percentage of CD25 and CD137 (4-1BB) expressing $CD8^+$ T cells are plotted against the used concentration of construct 2.11 or as positive control construct 2.4 or as negative control molecules control D and control F (see Example 1.11).

**Figure 14** shows the schematic set-up of the NLV-specific $CD8^+$ T cells activation in the presence of NLV-peptide pulsed FAP-expressing MV-3 human melanoma cells and bispecific anti-FAP split trimeric 4-1BBL fusion molecule construct 2.11. The content per well in the plate was: $5x10^4$ human melanoma cells MV-3, pulsed with no, $10^{-9}$ or $10^{-8}$ M NLV-peptide, 6250 CFSE-labeled NLV-specific CD8 T cells and $10^{-5}$ nM to 10 nM titrated concentrations of construct 2.11, construct 2.4, control D or control F. The incubation time was 24 h + 4 h in the presence of Brefeldin A and Monesin.

In **Figures 15A to 15F** results are shown from the activation assay with HLA-A2-NLV-specific CD8 T cells and NLV-pulsed HLA-$A2^+$ $FAP^+$ human melanoma cell line MV-3 in the presence of titrated concentration of different FAP-targeted or untargeted split trimeric human 4-1BB ligand Fc (kih) antigen binding molecules. The percentage of CD137 (4-1BB) and IFNγ expressing NLV-specific $CD8^+$ T cells are plotted against the used concentration of the test compounds (construct 2.11 or as positive control construct 2.4 or as negative controls control D and control F). All FAP-targeted split trimeric human 4-1BB ligand Fc (kih) antigen binding molecules show a similar activation improvement of HLA-A2-NLV-peptide specific CD8 T cells shown in **Figures 15A-C** as CD137 (4-1BB)-upregulation (positive feedback loop) and in **Figures 15D-F** as IFNγ expression after 24 h of stimulation. Differences of curves lie in the range of normal error deviation and are not significant.

**Figures 16A and 16B** show the binding of CD19-targeted split trimeric 4-1BBL Fc (kih) antigen binding molecules to 4-1BB expressing activated human CD4 and CD8 T cells from human PBMCs, respectively (see Example 7.1). Cells were gated on CD4 and CD8, and the binding curves were expressed by the mean fluorescent intensity of the secondary antibody against human Fc.

**Figure 17** shows the binding of CD19-targeted split trimeric 4-1BBL fusion molecules to CD19-expressing B lymphoma cell line (Example 7.2). The binding curves of the tested compounds (CD19-targeted split trimeric 4-1BBL Fc (kih) antigen binding molecules and untargeted Control D) to WSU-DLCL2 cells were expressed by the mean fluorescent intensity of the secondary antibody against human Fc.

**Figure 18** shows a scheme that illustrates the general principal of the NFkB activity assay described in Example 7.3 using a reporter cell line and CD19 expressing tumor cell lines. Shown is the activation assay set up with human 4-1BB expressing HeLa reporter cell line. A crosslinking of 4-1BB expressed on the reporter cells induces NFκB activation and NFκB-mediated Luciferase expression. After lysis of the cells Luciferase can catalyze the oxidation of Luciferin to Oxyluciferin. This chemical reaction correlates positively with the strength of NFκB-mediated luciferase expression and can be measured by the strength of light emission (units of released light).

**Figures 19A to 19D** show the NFκB-activation of luciferase in the 4-1BB expressing reporter cell line in the presence of CD 19 expressing tumor cell lines and CD19-targeted split trimeric 4-1BBL Fc (kih) fusion antigen binding molecules as measured with the assay described in Example 7.3. Different test compounds containing either the anti-human CD19-binder clone 8B8-018 (construct 3.3 and 3.11) or the anti-human CD19 binder clone 8B8-2B11 (construct 4.1 and 4.11) were incubated at different concentrations (shown as nM on the x-axis) with 4-1BB expressing reporter cell line and no CD19-expressing cells **(Figure 19A)** or CD19-expressing tumor cell lines OCI-Ly18, Nalm6 and SU-DHL-8 **(Figure 19B, Figure 19C** and **Figure 19D,** respectively) at a E:T ratio 1:5 for 6 hours. The monovalent CD19-targeted split trimeric 4-1BBL Fc (kih) fusion antigen binding molecules (Constructs 3.11 and 4.11) could induce a nice Luciferase activity measured by the units of released light (URL) measured for 0.5s/well (shown on the y-axis) similar to the positive CD19-targeted control molecules Constructs 3.4 and 4.1 (as described in Example 1.11). The non-CD19-targeted control molecules Control F and D induce no Luciferase activity. All values were baseline corrected by substracting the blank control (no construct added or control added).

**Figure 20** shows IFNγ release in activated PBMCs via agonistic CD3 antibody in combination with CD19-targeted split trimeric 4-1BBL fusion molecules. IFN-γ releases were measured by ELISA in the culture supernatant of human PBMCs in the presence of different targeted- or untargeted- CD19-targeted split trimeric 4-1BBL Fc (kih) antigen binding molecules.

## DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term **"antigen binding molecule"** refers in its broadest sense to a molecule that specifically binds an antigenic determinant. Examples of antigen binding molecules are antibodies, antibody fragments and scaffold antigen binding proteins.

As used herein, the term **"moiety capable of specific binding to a target cell antigen"** or "antigen binding domain capable of specific binding to a target cell antigen" refers to a polypeptide molecule that specifically binds to an antigenic determinant. In one aspect, the antigen binding moiety is able to activate signaling through its target cell antigen. In a particular aspect, the antigen binding moiety is able to direct the entity to which it is attached (e.g. the TNF family ligand trimer) to a target site, for example to a specific type of tumor cell or tumor stroma bearing the antigenic determinant. Moieties capable of specific binding to a target cell antigen include antibodies and fragments thereof as further defined herein. In addition, moieties capable of specific binding to a target cell antigen include scaffold antigen binding proteins as further defined herein, e.g. binding domains which are based on designed repeat proteins or designed repeat domains (see e.g. WO 2002/020565).

As used herein, the term **"Fab domain capable of specific binding to a target cell antigen"** refers to a Fab domain that specifically binds to an antigenic determinant. In one aspect, the antigen binding moiety is able to activate signaling through its target cell antigen. In a particular aspect, the antigen binding moiety is able to direct the entity to which it is attached (e.g. the TNF family ligand trimer) to a target site, for example to a specific type of tumor cell or tumor stroma bearing the antigenic determinant.

In relation to an antibody or Fab domain, the term "moiety capable of specific binding to a target cell antigen" refers to the part of the molecule that comprises the area which specifically binds to and is complementary to part or all of an antigen. A moiety capable of specific antigen binding may be provided, for example, by one or more antibody variable domains (also called antibody variable regions). Particularly, a moiety capable of specific antigen binding comprises an antibody light chain variable region (VL) and an antibody heavy chain variable region (VH).

The term **"antibody"** herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term **"monoclonal antibody"** as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term **"monospecific"** antibody as used herein denotes an antibody that has one or more binding sites each of which bind to the same epitope of the same antigen. The term **"bispecific"** means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term **"valent"** as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule.

The term **"monovalent to an antigen"** as used within the current application denotes the presence of only one binding site for said antigen in the antigen binding molecule.The term **"monovalent to a target cell antigen"** as used within the current application denotes the presence of only one binding site for said target cell antigen in the antigen binding molecule.

The terms "full length antibody", "intact antibody", and "whole antibody" are used herein interchangeably to refer to an antibody having a structure substantially similar to a native antibody structure. **"Native antibodies"** refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CHI, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma$1 (IgG1), $\gamma$2 (IgG2), $\gamma$3 (IgG3), $\gamma$4 (IgG4), $\alpha$1 (IgA1) and $\alpha$2 (IgA2). The light chain of an antibody may be assigned to one of two types, called kappa ($\kappa$) and lambda ($\lambda$), based on the amino acid sequence of its constant domain.

An **"antibody fragment"** refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "**Fab domain"** as used herein denotes a Fab fragment or cross-Fab fragment as defined herein after.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term "**Fab fragment"** refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an F(ab')$_2$ fragment that has two antigen-combining sites (two Fab fragments) and a part of the Fc region.

The term "cross-Fab **fragment"** or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab $_{(VLVH)}$. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as

$CrossFab_{(CLCH1)}$.

A "single chain Fab fragment" or "**scFab**" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or **"x-scFab"** is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A **"single-chain variable fragment (scFv)"** is a fusion protein of the variable regions of the heavy ($V_H$) and light chains ($V_L$) of an antibody, connected with a short linker peptide of ten to about 25 amino acids. The linker is usually rich in glycine for flexibility, as well as serine or threonine for solubility, and can either connect the N-terminus of the $V_H$ with the C-terminus of the $V_L$, or *vice versa.* This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

**"Scaffold antigen binding proteins"** are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), $V_{NAR}$ fragments, a fibronectin (AdNectin), a C-type lectin domain (Tetranectin); a variable domain of a new antigen receptor beta-lactamase ($V_{NAR}$ fragments), a human gamma-crystallin or ubiquitin (Affilin molecules); a kunitz type domain of human protease inhibitors, microbodies such as the proteins from the knottin family, peptide aptamers and fibronectin (adnectin).

An **"antigen binding molecule that binds to the same epitope"** as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term **"antigen binding domain"** refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term **"antigenic determinant"** is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

By **"specific binding"** is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of $\leq$ 1 $\mu$M, $\leq$ 100 nM, $\leq$ 10 nM, $\leq$ 1 nM, $\leq$ 0.1 nM, $\leq$ 0.01 nM, or $\leq$ 0.001 nM (e.g. $10^{-8}$ M or less, e.g. from $10^{-8}$ M to $10^{-13}$ M, e.g. from $10^{-9}$ M to $10^{-13}$ M).

**"Affinity"** or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants (koff and kon, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A **"target cell antigen"** as used herein refers to an antigenic determinant presented on the surface of a target cell, for example a cell in a tumor such as a cancer cell or a cell of the tumor stroma. In certain embodiments, the target cell antigen is an antigen on the surface of a tumor cell. In one embodiment, target cell antigen is selected from the group consisting of Fibroblast Activation Protein (FAP), Carcinoembryonic Antigen (CEA), Melanoma-associated Chondroitin Sulfate Proteoglycan (MCSP), Epidermal Growth Factor Receptor (EGFR), CD19, CD20 and CD33. In particular, the target cell antigen is Fibroblast Activation Protein (FAP).

The term **"Fibroblast activation protein (FAP)",** also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length," unprocessed FAP as well as any form of FAP which results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO:53), or NCBI (www.ncbi.nlm.nih.gov/) RefSeq NP_004451.2. The extracellular domain (ECD) of human FAP extends from amino acid position 26 to 760. The amino acid and nucleotide sequences of a His-tagged human FAP ECD is shown in SEQ ID NOs 54 and 55, respectively. The amino acid sequence of mouse FAP is shown in UniProt accession no. P97321 (version 126, SEQ ID NO:56), or NCBI RefSeq NP_032012.1. The extracellular domain (ECD) of mouse FAP extends from amino acid position 26 to 761. SEQ ID NOs 57 and 58 show the amino acid and nucleotide sequences, respectively, of a His-tagged mouse FAP ECD. SEQ ID NOs 59 and 60 show the amino acid and nucleotide sequences, respectively, of a His-tagged cynomolgus FAP ECD. Preferably, an anti-FAP binding molecule of the invention binds to the extracellular domain of FAP. Exemplary anti-FAP binding molecules are described in International Patent Application No. WO 2012/020006 A2.

The term **"Carcinoembroynic antigen** (CEA)", also known as Carcinoembryonic antigen-related cell adhesion molecule 5 (CEACAM5), refers to any native CEA from any vertebrate source, including mammals such as primates (e.g. humans) non-human primates (e.g. cynomolgus monkeys) and rodents (e.g. mice and rats), unless otherwise indicated. The amino acid sequence of human CEA is shown in UniProt accession no. P06731 (version 151, SEQ ID NO:61). CEA has long been identified as a tumor-associated antigen (Gold and Freedman, J Exp Med., 121:439-462, 1965; Berinstein N. L., J Clin Oncol., 20:2197-2207, 2002). Originally classified as a protein expressed only in fetal tissue, CEA has now been identified in several normal adult tissues. These tissues are primarily epithelial in origin, including cells of the gastrointestinal, respiratory, and urogential tracts, and cells of colon, cervix, sweat glands, and prostate (Nap et al., Tumour Biol., 9(2-3): 145-53, 1988; Nap et al., Cancer Res., 52(8):2329-23339, 1992). Tumors of epithelial origin, as well as their metastases, contain CEA as a tumor associated antigen. While the presence of CEA itself does not indicate transformation to a cancerous cell, the distribution of CEA is indicative. In normal tissue, CEA is generally expressed on the apical surface of the cell (Hammarström S., Semin Cancer Biol. 9(2):67-81 (1999)), making it inaccessible to antibody in the blood stream. In contrast to normal tissue, CEA tends to be expressed over the entire surface of cancerous cells (Hammarström S., Semin Cancer Biol. 9(2):67-81 (1999)). This change of expression pattern makes CEA accessible to antibody binding in cancerous cells. In addition, CEA expression increases in cancerous cells. Furthermore, increased CEA expression promotes increased intercellular adhesions, which may lead to metastasis (Marshall J., Semin Oncol., 30(a Suppl. 8):30-6, 2003). The prevalence of CEA expression in various tumor entities is generally very high. In concordance with published data, own analyses performed in tissue samples confirmed its high prevalence, with approximately 95% in colorectal carcinoma (CRC), 90% in pancreatic cancer, 80% in gastric cancer, 60% in non-small cell lung cancer (NSCLC, where it is co-expressed with HER3), and 40% in breast cancer; low expression was found in small cell lung cancer and glioblastoma.

CEA is readily cleaved from the cell surface and shed into the blood stream from tumors, either directly or via the lymphatics. Because of this property, the level of serum CEA has been used as a clinical marker for diagnosis of cancers and screening for recurrence of cancers, particularly colorectal cancer (Goldenberg D M., The International Journal of Biological Markers, 7:183-188, 1992; Chau I., et al., J Clin Oncol., 22:1420-1429, 2004; Flamini et al., Clin Cancer Res; 12(23):6985-6988, 2006).

The term **"Melanoma-associated Chondroitin Sulfate Proteoglycan (MCSP)",** also known as Chondroitin Sulfate Proteoglycan 4 (CSPG4) refers to any native MCSP from any vertebrate source, including mammals such as primates (e.g. humans) non-human primates (e.g. cynomolgus monkeys) and rodents (e.g. mice and rats), unless otherwise indicated. The amino acid sequence of human MCSP is shown in UniProt accession no. Q6UVK1 (version 103, SEQ ID NO:62). The term **"Epidermal Growth Factor Receptor (EGFR)",** also named Proto-oncogene c-ErbB-1 or Receptor tyrosine-protein kinase erbB-1, refers to any native EGFR from any vertebrate source, including mammals such as primates (e.g. humans) non-human primates (e.g. cynomolgus monkeys) and rodents (e.g. mice and rats), unless otherwise indicated. The amino acid sequence of human EGFR is shown in UniProt accession no. P00533 (version 211, SEQ ID NO:63).

The term **"CD19"** refers to B-lymphocyte antigen CD19, also known as B-lymphocyte surface antigen B4 or T-cell surface antigen Leu-12 and includes any native CD19 from any vertebrate source, including mammals such as primates (e.g. humans) non-human primates (e.g. cynomolgus monkeys) and rodents (e.g. mice and rats), unless otherwise indicated. The amino acid sequence of human CD19 is shown in Uniprot accession no. P15391 (version 160, SEQ ID NO:64). The term encompasses "full-length" unprocessed human CD19 as well as any form of human CD19 that results from processing in the cell as long as the antibody as reported herein binds thereto. CD19 is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias. The expression of CD19 on plasma cells further suggests it may be expressed on differentiated B cell tumors such as multiple myeloma. Therefore, the CD19 antigen is a target for immunotherapy in the treatment of non-Hodgkin's lymphoma, chronic lymphocytic leukemia and/or acute lymphoblastic leukemia.

**"CD20"** refers to B-lymphocyte antigen CD20, also known as membrane-spanning 4-domains subfamily A member 1 (MS4A1), B-lymphocyte surface antigen B1 or Leukocyte surface antigen Leu-16, and includes any native CD20 from any vertebrate source, including mammals such as primates (e.g. humans) non-human primates (e.g. cynomolgus monkeys) and rodents (e.g. mice and rats), unless otherwise indicated. The amino acid sequence of human CD20 is shown in Uniprot accession no. P11836 (version 149, SEQ ID NO:65). **"CD33"** refers to Myeloid cell surface antigen CD33, also known as SIGLEC3 or gp67, and includes any native CD33 from any vertebrate source, including mammals such as primates (e.g. humans) non-human primates (e.g. cynomolgus monkeys) and rodents (e.g. mice and rats), unless otherwise indicated. The amino acid sequence of human CD33 is shown in Uniprot accession no. P20138 (version 157, SEQ ID NO:66).

The term **"variable region"** or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (LI, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (HI), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE A. CDR Definitions[1]**

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$ CDR1 | 24-34 | 26-32 | 24-34 |
| $V_L$ CDR2 | 50-56 | 50-52 | 50-56 |
| $V_L$ CDR3 | 89-97 | 91-96 | 89-97 |

[1] Numbering of all CDR definitions in Table A is according to the numbering conventions set forth by Kabat et al. (see below).
[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

As used herein, the term "affinity matured" in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "**acceptor human framework**" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term **"chimeric"** antibody refers to an antibody in which a portion of the heavy and/or light chain is derived from a particular source or species, while the remainder of the heavy and/or light chain is derived from a different source or species.

The **"class"** of an antibody refers to the type of constant domain or constant region possessed by its heavy chain. There are five major classes of antibodies: IgA, IgD, IgE, IgG, and IgM, and several of these may be further divided into subclasses (isotypes), e.g. $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$ respectively..

A **"humanized"** antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A **"humanized form"** of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A **"human"** antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or **"Fc region"** herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333, expressly incorporated herein by reference). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The **"knob-into-hole"** technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)). The numbering is according to EU index of Kabat et al, Sequences of Proteins of Immunological Interest, 5th Ed. Public Health Service, National Institutes of Health, Bethesda, MD, 1991.

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g.,

Bowie, J. U. et al., Science 247:1306-10 (1990)).

The term **"effector functions"** refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

An "**activating Fc receptor**" is an Fc receptor that following engagement by an Fc region of an antibody elicits signaling events that stimulate the receptor-bearing cell to perform effector functions. Activating Fc receptors include Fc$\gamma$RIIIa (CD16a), Fc$\gamma$RI (CD64), Fc$\gamma$RIIa (CD32), and Fc$\alpha$RI (CD89). A particular activating Fc receptor is human Fc$\gamma$RIIIa (see UniProt accession no. P08637, version 141).

The term "**TNF ligand family** member" or "TNF family ligand" refers to a proinflammatory cytokine. Cytokines in general, and in particular the members of the TNF ligand family, play a crucial role in the stimulation and coordination of the immune system. At present, nineteen cyctokines have been identified as members of the TNF (tumour necrosis factor) ligand superfamily on the basis of sequence, functional, and structural similarities. All these ligands are type II transmembrane proteins with a C-terminal extracellular domain (ectodomain), N-terminal intracellular domain and a single transmembrane domain. The C-terminal extracellular domain, known as TNF homology domain (THD), has 20-30% amino acid identity between the superfamily members and is responsible for binding to the receptor. The TNF ectodomain is also responsible for the TNF ligands to form trimeric complexes that are recognized by their specific receptors.

Members of the TNF ligand family are selected from the group consisting of Lymphotoxin $\alpha$ (also known as LTA or TNFSF1), TNF (also known as TNFSF2), LT$\beta$ (also known as TNFSF3), OX40L (also known as TNFSF4), CD40L (also known as CD154 or TNFSF5), FasL (also known as CD95L, CD178 or TNFSF6), CD27L (also known as CD70 or TNFSF7), CD30L (also known as CD153 or TNFSF8), 4-1BBL (also known as TNFSF9), TRAIL (also known as APO2L, CD253 or TNFSF10), RANKL (also known as CD254 or TNFSF11), TWEAK (also known as TNFSF12), APRIL (also known as CD256 or TNFSF13), BAFF (also known as CD257 or TNFSF13B), LIGHT (also known as CD258 or TNFSF14), TL1A (also known as VEGI or TNFSF15), GITRL (also known as TNFSF18), EDA-A1 (also known as ectodysplasin A1) and EDA-A2 (also known as ectodysplasin A2). The term refers to any native TNF family ligand from any vertebrate source, including mammals such as primates (e.g. humans), non-human primates (e.g. cynomolgus monkeys) and rodents (e.g. mice and rats), unless otherwise indicated. In specific embodiments of the invention, the TNF ligand family member is selected from the group consisting of OX40L, FasL, CD27L, TRAIL, 4-1BBL, CD40L and GITRL. In a particular embodiment, the TNF ligand family member is selected from 4-1BBL and OX40L.

Further information, in particular sequences, of the TNF ligand family members may be obtained from publically accessible databases such as Uniprot (www.uniprot.org). For instance, the human TNF ligands have the following amino acid sequences: human Lymphotoxin $\alpha$ (UniProt accession no. P01374, SEQ ID NO:67), human TNF (UniProt accession no. P01375, SEQ ID NO:68), human Lymphotoxin $\beta$ (UniProt accession no. Q06643, SEQ ID NO:69), human OX40L (UniProt accession no. P23510, SEQ ID NO:70), human CD40L (UniProt accession no. P29965, SEQ ID NO:71), human FasL (UniProt accession no. P48023, SEQ ID NO:72), human CD27L (UniProt accession no. P32970, SEQ ID NO:73), human CD30L (UniProt accession no. P32971, SEQ ID NO:74), 4-1BBL (UniProt accession no. P41273, SEQ ID NO:75), TRAIL (UniProt accession no. P50591, SEQ ID NO:76), RANKL (UniProt accession no. 014788, SEQ ID NO:77), TWEAK (UniProt accession no. O43508, SEQ ID NO:78), APRIL (UniProt accession no. O75888, SEQ ID NO:79), BAFF (UniProt accession no. Q9Y275, SEQ ID NO:80), LIGHT (UniProt accession no. O43557, SEQ ID NO:81), TL1A (UniProt accession no. 095150, SEQ ID NO:82), GITRL (UniProt accession no. Q9UNG2, SEQ ID NO:83) and ectodysplasin A (UniProt accession no. Q92838, SEQ ID NO:84).

An **"ectodomain"** is the domain of a membrane protein that extends into the extracellular space (i.e. the space outside the target cell). Ectodomains are usually the parts of proteins that initiate contact with surfaces, which leads to signal transduction. The ectodomain of TNF ligand family member as defined herein thus refers to the part of the TNF ligand protein that extends into the extracellular space (the extracellular domain), but also includes shorter parts or fragments thereof that are responsible for the trimerization and for the binding to the corresponding TNF receptor. The term "ectodomain of a TNF ligand family member or a fragment thereof" thus refers to the extracellular domain of the TNF ligand family member that forms the extracellular domain or to parts thereof that are still able to bind to the receptor (receptor binding domain).

The term "**costimulatory TNF ligand family member**" or "costimulatory TNF family ligand" refers to a subgroup of TNF ligand family members, which are able to costimulate proliferation and cytokine production of T-cells. These TNF family ligands can costimulate TCR signals upon interaction with their corresponding TNF receptors and the interaction with their receptors leads to recruitment of TNFR-associated factors (TRAF), which initiate signalling cascades that result in T-cell activation. Costimulatory TNF family ligands are selected from the group consisting of 4-1BBL, OX40L, GITRL, CD70, CD30L and LIGHT, more particularly the costimulatory TNF ligand family member is selected from 4-1BBL and OX40L.

As described herein before, 4-1BBL is a type II transmembrane protein and one member of the TNF ligand family. Complete or full length 4-1BBL having the amino acid sequence of SEQ ID NO:42 has been described to form trimers on the surface of cells. The formation of trimers is enabled by specific motives of the ectodomain of 4-1BBL. Said motives are designated herein as "trimerization region". The amino acids 50-254 of the human 4-1BBL sequence (SEQ ID NO:85) form the extracellular domain of 4-1BBL, but even fragments thereof are able to form the trimers. In specific embodiments of the invention, the term "ectodomain of 4-1BBL or a fragment thereof" refers to a polypeptide having an amino acid sequence selected from SEQ ID NO:4 (amino acids 52-254 of human 4-1BBL), SEQ ID NO: 1 (amino acids 71-254 of human 4-1BBL), SEQ ID NO:3 (amino acids 80-254 of human 4-1BBL) and SEQ ID NO:2 (amino acids 85-254 of human 4-1BBL) or a polypeptide having an amino acid sequence selected from SEQ ID NO:5 (amino acids 71-248 of human 4-1BBL), SEQ ID NO:8 (amino acids 52-248 of human 4-1BBL), SEQ ID NO:7 (amino acids 80-248 of human 4-1BBL) and SEQ ID NO:6 (amino acids 85-248 of human 4-1BBL), but also other fragments of the ectodomain capable of trimerization are included herein.

As described herein before, OX40L is another type II transmembrane protein and a further member of the TNF ligand family. Complete or full length human OX40L has the amino acid sequence of SEQ ID NO:70. The amino acids 51-183 of the human OX40L sequence (SEQ ID NO: 11) form the extracellular domain of OX40L, but even fragments thereof that are able to form the trimers. In specific embodiments of the invention, the term "ectodomain of OX40L or a fragment thereof" refers to a polypeptide having an amino acid sequence selected from SEQ ID NO:11 (amino acids 51-183 of human OX40L) or SEQ ID NO:12 (amino acids 52-183 of human OX40L), but also other fragments of the ectodomain capable of trimerization are included herein.

The term "peptide linker" refers to a peptide comprising one or more amino acids, typically about 2 to 20 amino acids. Peptide linkers are known in the art or are described herein. Suitable, non-immunogenic linker peptides are, for example, $(G_4S)_n$, $(SG_4)_n$ or $G_4(SG_4)_n$ peptide linkers, wherein "n" is generally a number between 1 and 10, typically between 1 and 4, in particular 2, i.e. the peptides selected from the group consisting of GGGGS (SEQ ID NO:86), GGGGSGGGGS (SEQ ID NO:87), SGGGGSGGGG (SEQ ID NO:88), $(G_4S)_3$ or GGGGSGGGGSGGGGS (SEQ ID NO:89), GGGGSGGGGSGGGG or $G4(SG4)_2$ (SEQ ID NO:90), and $(G_4S)_4$ or GGGGSGGGGSGGGGSGGGGS (SEQ ID NO:91), but also include the sequences GSPGSSSSGS (SEQ ID NO:92), GSGSGSGS (SEQ ID NO:93), GSGSGNGS (SEQ ID NO:94), GGSGSGSG (SEQ ID NO:95), GGSGSG (SEQ ID NO:96), GGSG (SEQ ID NO:97), GGSGNGSG (SEQ ID NO:98), GGNGSGSG (SEQ ID NO:99) and GGNGSG (SEQ ID NO: 100). Peptide linkers of particular interest are $(G4S)_1$ or GGGGS (SEQ ID NO:86), $(G_4S)_2$ or GGGGSGGGGS (SEQ ID NO:87) and GSPGSSSSGS (SEQ ID NO:92), more particularly $(G_4S)_2$ or GGGGSGGGGS (SEQ ID NO:87).

The term "amino acid" as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

A **"single chain fusion protein"** as used herein refers to a single chain polypeptide composed of one or two ectodomains of said TNF ligand family member fused to a part of antigen binding moiety or Fc part. The fusion may occur by directly linking the N or C-terminal amino acid of the antigen binding moiety via a peptide linker to the C- or N-terminal amino acid of the ectodomain of said TNF ligand family member.

By "fused" or "connected" is meant that the components (e.g. a polypeptide and an ectodomain of said TNF ligand family member) are linked by peptide bonds, either directly or via one or more peptide linkers.

**"Percent (%) amino acid sequence identity"** with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the TNF ligand trimer-containing antigen binding molecules provided herein are contemplated. For example, it may be desirable to improve the binding affinity and/or other biological properties of the TNF ligand trimer-containing antigen binding molecules. Amino acid sequence variants of the TNF ligand trimer-containing antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE B**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

The term "**amino acid sequence variants**" includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include TNF family ligand trimer-containing antigen binding molecule with an N-terminal methionyl residue. Other insertional variants of the molecule include the fusion to the N- or C-terminus to a polypeptide which increases the serum half-life of the TNF ligand trimer-containing antigen binding molecules.

In certain embodiments, the TNF family ligand trimer-containing antigen binding molecules provided herein are altered to increase or decrease the extent to which the antibody is glycosylated. Glycosylation variants of the molecules may be conveniently obtained by altering the amino acid sequence such that one or more glycosylation sites is created or removed. Where the TNF ligand trimer-containing antigen binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in TNF family ligand trimer-containing antigen binding molecule may be made in order to create variants with certain improved properties. In one aspect, variants of TNF family ligand trimer-containing antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of the TNF family ligand trimer-containing antigen binding molecules of the invention include those with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain embodiments, it may be desirable to create **cysteine engineered variants** of the TNF family ligand trimer-containing antigen binding molecule of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the TNF family ligand trimer-containing antigen binding molecules provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the bispecific antibody derivative will be used in a therapy under defined conditions, etc. In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

In another aspect, immunoconjugates of the TNF family ligand trimer-containing antigen binding molecules provided herein maybe obtained. An **"immunoconjugate"** is an antibody conjugated to one or more heterologous molecule(s), including but not limited to a cytotoxic agent.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term **"vector"** or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms **"host cell",** "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An **"effective amount"** of an agent refers to the amount that is necessary to result in a physiological change in the cell or tissue to which it is administered.

A **"therapeutically effective amount"** of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An **"individual"** or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term **"pharmaceutical composition"** refers to a preparation which is in such form as to permit the biological activity of an active ingredient contained therein to be effective, and which contains no additional components which are unacceptably toxic to a subject to which the formulation would be administered.

A **"pharmaceutically acceptable excipient"** refers to an ingredient in a pharmaceutical composition, other than an active ingredient, which is nontoxic to a subject. A pharmaceutically acceptable excipient includes, but is not limited to, a buffer, a stabilizer, or a preservative.

The term **"package insert"** is used to refer to instructions customarily included in commercial packages of therapeutic products, that contain information about the indications, usage, dosage, administration, combination therapy, contraindications and/or warnings concerning the use of such therapeutic products.

As used herein, **"treatment"** (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the invention are used to delay development of a disease or to slow the progression of a disease.

The term **"cancer"** as used herein refers to proliferative diseases, such as lymphomas, carcinoma, lymphoma, blastoma, sarcoma, leukemia, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colorectal cancer (CRC), pancreatic cancer, breast cancer, triple-negative breast cancer , uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, melanoma, multiple myeloma, B-cell cancer (lymphoma), chronic lymphocytic leukemia (CLL), acute lymphoblastic leukemia (ALL), hairy cell leukemia, chronic myeloblastic leukemia, including refractory versions of any of the above cancers, or a combination of one or more of the above cancers.

**4-1BB ligand (4-1BBL) trimer-containing antigen binding molecules**

The invention provides novel 4-1BBL trimer-containing antigen binding molecules with particularly advantageous properties such as producibility, stability, robustness, binding affinity, biological activity, targeting efficiency and reduced toxicity.

In a first aspect, disclosed is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain.

In a particular aspect, disclosed is a costimulatory 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain. Thus, the 4-1BBL trimer-containing antigen binding molecule comprises (a) a Fab domain capable of specific binding to a target cell antigen, (b) a Fc domain composed of a first and a second subunit capable of stable association, and (c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, wherein the 4-1BBL costimulates human T-cell activation.

In another particular aspect, the 4-1BBL trimer-containing antigen binding molecule comprises (a) a Fab domain capable of specific binding to a target cell antigen, (b) a Fc domain composed of a first and a second subunit capable of stable association, and (c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, wherein the ectodomains of 4-1BBL are identical in all instances.

In a further aspect, the ectodomain of 4-1BBL comprises the amino acid sequence selected from the group consisting of SEQ ID NO:1, SEQ ID NO: 2, SEQ ID NO:3, SEQ ID NO:4, SEQ ID NO:5, SEQ ID NO:6, SEQ ID NO:7 and SEQ ID NO:8, particularly the amino acid sequence of SEQ ID NO:1 or SEQ ID NO:5.

In a particular aspect, the ectodomain of 4-1BBL comprises the amino acid sequence selected from the group consisting of SEQ ID NO:5, SEQ ID NO:6, SEQ ID NO:7 and SEQ ID NO:8. More particularly, the ectodomain of a 4-1BBL comprises the amino acid sequence of SEQ ID NO:5.

In a further aspect, the 4-1BBL trimer-containing antigen binding molecule comprises

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL or fragments thereof comprises an amino acid sequence selected from the group consisting of SEQ ID NO:9 and SEQ ID NO: 10 and the second polypeptide comprising one ectodomain of said 4-1BBL or a fragment thereof comprises the amino acid sequence selected from the group consisting of SEQ ID NO: 1 and SEQ ID NO:5. In one aspect, the first polypeptide comprising two ectodomains of 4-1BBL or fragments thereof comprises an amino acid sequence SEQ ID NO:9 and the second polypeptide comprising one ectodomain of said 4-1BBL or a fragment thereof comprises the amino acid sequence of SEQ ID NO:1. In a particular aspect, the first polypeptide comprising two ectodomains of 4-1BBL or fragments thereof comprises an amino acid sequence of SEQ ID NO: 10 and the second polypeptide comprising one ectodomain of said 4-1BBL or a fragment thereof comprises the amino acid sequence of SEQ ID NO:5.

In another aspect, provided is a 4-1BBL trimer-containing antigen binding molecule, wherein the target cell antigen is selected from the group consisting of Fibroblast Activation Protein (FAP), CD19, Carcinoembryonic Antigen (CEA), Melanoma-associated Chondroitin Sulfate Proteoglycan (MCSP), Epidermal Growth Factor Receptor (EGFR), CD20 and CD33. In one aspect, the target cell antigen is selected from the group consisting of Fibroblast Activation Protein (FAP), CD19 and Carcinoembryonic Antigen (CEA).

In a particular aspect, provided is a 4-1BBL trimer-containing antigen binding molecule, wherein the Fab domain capable of specific binding to a target cell antigen is a Fab or a crossover Fab capable of specific binding to a target cell antigen.

In a particular aspect, the target cell antigen is Fibroblast Activation Protein (FAP).

In one aspect, provided is a 4-1BBL trimer-containing antigen binding molecule, wherein the Fab domain capable of specific binding to FAP comprises

(a) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO: 15, and a VL domain comprising (iv) CDR-L1 comprising the amino acid sequence of SEQ ID NO:16, (v) CDR-L2 comprising the amino acid sequence of SEQ ID NO: 17 and (vi) CDR-L3 comprising the amino acid sequence of SEQ ID NO:18 or

(b) a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

In a particular aspect, provided is a 4-1BBL trimer-containing antigen binding molecule, wherein the Fab domain capable of specific binding to FAP comprises a VH domain comprising (i) CDR-H1 comprising the amino acid sequence of SEQ ID NO:19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

In a further aspect, provided is a 4-1BBL trimer-containing antigen binding molecule as described herein before, wherein the Fab domain capable of specific binding to FAP comprises a variable heavy chain comprising an amino acid sequence of SEQ ID NO:25 and a variable light chain comprising an amino acid sequence of SEQ ID NO:26 or wherein the Fab domain capable of specific binding to FAP comprises a variable heavy chain comprising an amino acid sequence of SEQ ID NO:27 and a variable light chain comprising an amino acid sequence of SEQ ID NO:28.

In another particular aspect, the target cell antigen is CD19.

In one aspect, provided is a 4-1BBL trimer-containing antigen binding molecule, wherein the Fab domain capable of specific binding to CD19 comprises

(a) a VH domain comprising (i) CDR-H1 comprising the amino acid sequence of SEQ ID NO:29, (ii) CDR-H2 comprising the amino acid sequence of SEQ ID NO:30 and (iii) CDR-H3 comprising the amino acid sequence of SEQ ID NO:31, and a VL domain comprising (iv) CDR-L1 comprising the amino acid sequence of SEQ ID NO:32, (v) CDR-L2 comprising the amino acid sequence of SEQ ID NO:33 and (vi) CDR-L3 comprising the amino acid sequence of SEQ ID NO:34 or

(b) a VH domain comprising (i) CDR-H1 comprising the amino acid sequence of SEQ ID NO:35, (ii) CDR-H2 comprising the amino acid sequence of SEQ ID NO:36 and (iii) CDR-H3 comprising the amino acid sequence of SEQ ID NO:37, and a VL domain comprising (iv) CDR-L1 comprising the amino acid sequence of SEQ ID NO:38, (v) CDR-L2 comprising the amino acid sequence of SEQ ID NO:39 and (vi) CDR-L3 comprising the amino acid sequence of SEQ ID NO:40.

In a particular aspect, provided is a 4-1BBL trimer-containing antigen binding molecule, wherein the Fab domain capable of specific binding to CD19 comprises a VH domain comprising (i) CDR-H1 comprising the amino acid sequence of SEQ ID NO:35, (ii) CDR-H2 comprising the amino acid sequence of SEQ ID NO:36 and (iii) CDR-H3 comprising the amino acid sequence of SEQ ID NO:37, and a VL domain comprising (iv) CDR-L1 comprising the amino acid sequence of SEQ ID NO:38, (v) CDR-L2 comprising the amino acid sequence of SEQ ID NO:39 and (vi) CDR-L3 comprising the amino acid sequence of SEQ ID NO:40.

In a further aspect, provided is a 4-1BBL trimer-containing antigen binding molecule as described herein before, wherein the Fab domain capable of specific binding to CD19 comprises a variable heavy chain comprising an amino acid sequence of SEQ ID NO:41 and a variable light chain comprising an amino acid sequence of SEQ ID NO:42 or wherein the Fab domain capable of specific binding to FAP comprises a variable heavy chain comprising an amino acid sequence of SEQ ID NO:43 and a variable light chain comprising an amino acid sequence of SEQ ID NO:44.

In another particular aspect, the target cell antigen is CEA.

In one aspect, provided is a 4-1BBL trimer-containing antigen binding molecule, wherein the Fab domain capable of specific binding to CEA comprises a VH domain comprising (i) CDR-H1 comprising the amino acid sequence of SEQ ID NO:45, (ii) CDR-H2 comprising the amino acid sequence of SEQ ID NO:46 and (iii) CDR-H3 comprising the amino acid sequence of SEQ ID NO:47, and a VL domain comprising (iv) CDR-L1 comprising the amino acid sequence of SEQ ID NO:48, (v) CDR-L2 comprising the amino acid sequence of SEQ ID NO:49 and (vi) CDR-L3 comprising the amino acid sequence of SEQ ID NO:50.

In a particular aspect, provided is a 4-1BBL trimer-containing antigen binding molecule as described herein before, wherein the Fab domain capable of specific binding to CEA comprises a variable heavy chain comprising an amino acid sequence of SEQ ID NO:51 and a variable light chain comprising an amino acid sequence of SEQ ID NO:52.

In a further aspect, the Fc domain is an IgG, particularly an IgG1 Fc domain or an IgG4 Fc domain. More particularly, the Fc domain is an IgG1 Fc domain. In a particular aspect, the Fc domain comprises a modification promoting the association of the first and second subunit of the Fc domain.

In particular, the 4-1BBL trimer-containing antigen binding molecule of the invention comprises a Fab domain capable of specific binding to a target cell antigen, wherein the Fab heavy chain is fused at the C-terminus to the N-terminus of a CH2 domain in the Fc domain.

In another aspect, the invention is concerned with a 4-1BBL trimer-containing antigen binding molecule as defined herein before, comprising (b) an Fc domain composed of a first and a second subunit capable of stable association, wherein the Fc domain comprises one or more amino acid substitution that reduces binding to an Fc receptor, in particular towards Fcγ receptor.

**Fc domain modifications reducing Fc receptor binding and/or effector function**

The Fc domain of the 4-1BBL trimer-containing antigen binding molecules of the invention consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

The Fc domain confers favorable pharmacokinetic properties to the antigen binding molecules, including a long serum half-life which contributes to good accumulation in the target tissue and a favorable tissue-blood distribution ratio. At the same time it may, however, lead to undesirable targeting of the bispecific antibodies to cells expressing Fc receptors rather than to the preferred antigen-bearing cells. Accordingly, in particular aspects, the Fc domain of the 4-1BBL trimer-containing antigen binding molecule exhibits reduced binding affinity to an Fc receptor and/or reduced effector function, as compared to a native IgG1 Fc domain. In one aspect, the Fc does not substantially bind to an Fc receptor and/or does not induce effector function. In a particular aspect the Fc receptor is an Fcγ receptor. In one aspect, the Fc receptor is a human Fc receptor. In a specific aspect, the Fc receptor is an activating human Fcγ receptor, more specifically human FcγRIIIa, FcγRI or FcγRIIa, most specifically human FcγRIIIa. In one aspect, the Fc domain does not induce effector function. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

In certain aspects, one or more amino acid modifications may be introduced into the Fc region of a 4-1BBL trimer-containing antigen binding molecule provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In a particular aspect, provided is a 4-1BBL trimer-containing antigen binding molecule comprising

(a) a Fab domain capable of specific binding to a target cell antigen,

(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, wherein the Fc domain comprises one or more amino acid substitution that reduces binding to an Fc receptor, in particular towards Fcγ receptor.

In one aspect, the Fc domain of the 4-1BBL trimer-containing antigen binding molecule of the invention comprises one or more amino acid mutation that reduces the binding affinity of the Fc domain to an Fc receptor and/or effector function. Typically, the same one or more amino acid mutation is present in each of the two subunits of the Fc domain. In particular, the Fc domain comprises an amino acid substitution at a position of E233, L234, L235, N297, P331 and P329 (EU numbering). In particular, the Fc domain comprises amino acid substitutions at positions 234 and 235 (EU numbering) and/or 329 (EU numbering) of the IgG heavy chains. More particularly, provided is a trimeric TNF family ligand-containing antigen binding molecule according to the invention which comprises an Fc domain with the amino acid substitutions L234A, L235A and P329G ("P329G LALA", EU numbering) in the IgG heavy chains. The amino acid substitutions L234A and L235A refer to the so-calledLALA mutation. The "P329G LALA" combination of amino acid substitutions almost completely abolishes Fcγ receptor binding of a human IgG1 Fc domain and is described in International Patent Appl. Publ. No. WO 2012/130831 A1 which also describes methods of preparing such mutant Fc domains and methods for determining its properties such as Fc receptor binding or effector functions. "EU numbering" refers to the numbering according to EU index of Kabat et al, Sequences of Proteins of Immunological Interest, 5th Ed. Public Health Service, National Institutes of Health, Bethesda, MD, 1991.

Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In another aspect, the Fc domain is an IgG4 Fc domain. IgG4 antibodies exhibit reduced binding affinity to Fc receptors and reduced effector functions as compared to IgG1 antibodies. In a more specific aspect, the Fc domain is an IgG4 Fc domain comprising an amino acid substitution at position S228 (Kabat numbering), particularly the amino acid substitution S228P. In a more specific aspect, the Fc domain is an IgG4 Fc domain comprising amino acid substitutions L235E and S228P and P329G (EU numbering). Such IgG4 Fc domain mutants and their Fcγ receptor binding properties are also described in WO 2012/130831.

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or bispecific antibodies of the invention comprising an Fc domain, can be measured by methods known in the art. A suitable assay for measuring ADCC is described herein. Other examples of in vitro assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998).

In some aspects, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the bispecific antibodies of the invention is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

In a particular aspect, the Fc domain comprises a modification promoting the association of the first and second subunit of the Fc domain.

**Fc domain modifications promoting heterodimerization**

In one aspect, the 4-1BBL trimer-containing antigen binding molecules of the invention comprise (a) a Fab domain capable of specific binding to a target cell antigen, (b) a Fc domain composed of a first and a second subunit capable of stable association, and (c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, wherein the Fc domain comprises one or more amino acid substitution that reduces binding to an Fc receptor, in particular towards Fcγ receptor. Thus, they comprise different moieties, fused to one or the other of the two subunits of the Fc domain that are typically comprised in two non-identical polypetide chains ("heavy chains"). Recombinant co-expression of these polypeptides and subsequent dimerization leads to several possible combinations of the two polypeptides. To improve the yield and purity of the 4-1BBL trimer-containing antigen binding molecules in recombinant production, it will thus be advantageous to introduce in the Fc domain of the 4-1BBL trimer-containing antigen binding molecules of the invention a modification promoting the association of the desired polypeptides.

Accordingly, the Fc domain of the 4-1BBL trimer-containing antigen binding molecules of the invention comprises a modification promoting the association of the first and the second subunit of the Fc domain. The site of most extensive protein-protein interaction between the two subunits of a human IgG Fc domain is in the CH3 domain of the Fc domain. Thus, said modification is particularly in the CH3 domain of the Fc domain.

In a specific aspect, said modification is a so-called "knob-into-hole" modification, comprising a "knob" modification in one of the two subunits of the Fc domain and a "hole" modification in the other one of the two subunits of the Fc domain. Thus, in a particular aspect, the invention relates to a 4-1BBL trimer-containing antigen binding molecule as described herein before which comprises an IgG molecule, wherein the Fc part of the first heavy chain comprises a first dimerization module and the Fc part of the second heavy chain comprises a second dimerization module allowing a heterodimerization of the two heavy chains of the IgG molecule and the first dimerization module comprises knobs and the second dimerization module comprises holes according to the knob into hole technology.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular aspect, in the CH3 domain of the first subunit of the Fc domain of the 4-1BBL trimer-containing antigen binding molecules of the invention an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). More particularly, in the second subunit of the Fc domain additionally the threonine residue at position 366 is replaced with a serine residue (T366S) and the leucine residue at position 368 is replaced with an alanine residue (L368A). More particularly, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). The introduction of these two cysteine residues results in the formation of a disulfide bridge between the two subunits of the Fc domain. The disulfide bridge further stabilizes the dimer (Carter, J Immunol Methods 248, 7-15 (2001)).

Thus, in a particular aspect, the Fc domain comprises a modification promoting the association of the first and second subunit of the Fc domain. In a particular aspect, provided is a 4-1BBL trimer-containing antigen binding molecule as defined herein before, wherein the first subunit of the Fc domain comprises the amino acid substitutions S354C and T366W (numbering according to Kabat EU index) of the knob and the second subunit of the Fc domain comprises the amino acid substitutions Y349C, T366S, L368A and Y407V (numbering according to Kabat EU index) of the hole.

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

In a further aspect, provided is a 4-1BBL trimer-containing antigen binding molecule as defined herein before, wherein the first polypeptide comprising two ectodomains of 4-1BBL or fragments thereof that are connected to each other by a peptide linker is fused at its N-terminus to the C-terminus of the second subunit of the Fc domain (hole) and the second polypeptide comprising one ectodomain of said 4-1BBL or a fragment thereof is fused at its N-terminus to the C-terminus of the first subunit of the Fc domain (knob).

In another aspect, provided is a 4-1BBL trimer-containing antigen binding molecule as defined herein before, wherein the first polypeptide comprising two ectodomains of 4-1BBL or fragments thereof that are connected to each other by a peptide linker is fused at its N-terminus to the C-terminus of the first subunit of the Fc domain (knob) and the second polypeptide comprising one ectodomain of said 4-1BBL or a fragment thereof is fused at its N-terminus to the C-terminus of the second subunit of the Fc domain (hole).

In one aspect, provided is a 4-1BBL trimer-containing antigen binding molecule, wherein the Fab domain capable of specific binding to a target cell antigen is fused at the C-terminus to the N-terminus of the first subunit of the Fc domain (knob). In another aspect, provided is a 4-1BBL trimer-containing antigen binding molecule, wherein the Fab domain capable of specific binding to a target cell antigen is fused at the C-terminus to the N-terminus of the second subunit of the Fc domain (hole).

In a particular aspect, provided is a 4-1BBL trimer-containing antigen binding molecule, wherein the Fab domain capable of specific binding to a target cell antigen is fused at the C-terminus to the N-terminus of the first subunit of the Fc domain (knob), the first polypeptide comprising two ectodomains of 4-1BBL or fragments thereof that are connected to each other by a peptide linker is fused at its N-terminus to the C-terminus of the second subunit of the Fc domain (hole) and the second polypeptide comprising one ectodomain of said 4-1BBL or a fragment thereof is fused at its N-terminus to the C-terminus of the first subunit of the Fc domain (knob).

In a further aspect, provided is a 4-1BBL trimer-containing antigen binding molecule as defined before further comprising (d) a Fab domain that is not capable of specific binding to a target cell antigen. Thus, provided is a 4-1BBL trimer-containing antigen binding molecule comprising

(a) a Fab domain capable of specific binding to a target cell antigen,
(b) a Fc domain composed of a first and a second subunit capable of stable association,
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, and (d) a Fab domain that is not capable of specific binding to a target cell antigen.

These molecules comprising a Fab domain that is not capable of specific binding to a target cell antigen have a full IgG-like structure while at the same time they have the advantage of only one binding site for the target cell antigen (monovalent binding). They may thus have a better Pk profile and/or less side effects caused for instance by anti-drug antibodies.

**Modifications in the CH1/CL domains**

To further improve correct pairing in antigen binding molecules comprising two different Fab domains, the 4-1BBL trimer-containing antigen binding molecules can contain different charged amino acid substitutions (so-called "charged residues"). These modifications are introduced in the crossed or non-crossed CH1 and CL domains. In a particular aspect, the invention relates to a 4-1BBL trimer-containing antigen binding molecule, wherein in one of CL domains the amino acid at position 123 (EU numbering) has been replaced by arginine (R) and the amino acid at position 124 (EU numbering) has been substituted by lysine (K) and wherein in one of the CH1 domains the the amino acids at position 147 (EU numbering) and at position 213 (EU numbering) have been substituted by glutamic acid (E).

More particularly, the invention relates to a 4-1BBL trimer-containing antigen binding molecule comprising

(a) a Fab domain capable of specific binding to a target cell antigen,
(b) a Fc domain composed of a first and a second subunit capable of stable association,

(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain, and (d) a Fab domain that is not capable of specific binding to a target cell antigen being a crossover Fab, wherein in the CL domain the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

**Particular 4-1BBL trimer-containing antigen binding molecules**

In one aspect, provided is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the second subunit of the Fc domain comprising the knob mutations and wherein the second polypeptide is fused at its N-terminus to the C-terminus of the first subunit of the Fc domain comprising the hole mutations.

In a particular aspect, provided is a TNF family ligand trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the second subunit of the Fc domain comprising the knob mutations and wherein the second polypeptide is fused at its N-terminus to the C-terminus of the first subunit of the Fc domain comprising the hole mutations and wherein the VH and CH1 domain of the Fab domain capable of specific binding to a target cell antigen is fused at the C-terminus to the N-terminus of the second subunit of the Fc domain comprising the knob mutations.

In another aspect, provided is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the second subunit of the Fc domain comprising the knob mutations and wherein the second polypeptide is fused at its N-terminus to the C-terminus of the first subunit of the Fc domain comprising the hole mutations and wherein the VH and CH1 domain of the Fab domain capable of specific binding to a target cell antigen is fused at the C-terminus to the N-terminus of the first subunit of the Fc domain comprising the hole mutations.

In a further aspect, provided is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the first subunit of the Fc domain comprising the hole mutations and wherein the second polypeptide is fused at its N-terminus to the C-terminus of the second subunit of the Fc domain comprising the knob mutations.

In a particular aspect, provided is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the first subunit of the Fc domain comprising the hole mutations and wherein the second polypeptide is fused at its N-terminus to the C-terminus of the second subunit of the Fc domain comprising the knob mutations and wherein the VH and CH1 domain of the Fab domain capable of specific binding to a target cell antigen is fused at the C-terminus to the N-terminus of the second subunit of the Fc domain comprising the knob mutations.

In another aspect, provided is a 4-1BBL trimer-containing antigen binding molecule comprising

(a) a Fab domain capable of specific binding to a target cell antigen,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to the first subunit of the Fc domain comprising the hole mutations and wherein the second polypeptide is fused at its N-terminus to the C-terminus of the second subunit of the Fc domain comprising the knob mutations and wherein the VH and CH1 domain of the Fab domain capable of specific binding to a target cell antigen is fused at the C-terminus to the N-terminus of the first subunit of the Fc domain comprising the hole mutations.

**4-1BBL trimer-containing antigen binding molecules, wherein the target cell antigen is FAP**

In one aspect, provided is a 4-1BBL trimer-containing antigen binding molecule, wherein the target cell antigen is Fibroblast Activation Protein (FAP).

In a particular aspect, provided is a 4-1BBL trimer-containing antigen binding molecule as defined herein before, wherein the Fab domain capable of specific binding to FAP comprises a heavy chain variable region comprising an amino acid sequence that is at least about 95%, 96%, 97%, 98%, 99% or 100% identical to the amino acid sequence of SEQ ID NO:25 and a light chain variable region comprising an amino acid sequence that is at least about 95%, 96%, 97%, 98%, 99% or 100% identical to the amino acid sequence of SEQ ID NO:26.

In another particular aspect, provided is a 4-1BBL trimer-containing antigen binding molecule as defined herein before, wherein the Fab domain capable of specific binding to FAP comprises a heavy chain variable region comprising an amino acid sequence that is at least about 95%, 96%, 97%, 98%, 99% or 100% identical to the amino acid sequence of SEQ ID NO:27 and a light chain variable region comprising an amino acid sequence that is at least about 95%, 96%, 97%, 98%, 99% or 100% identical to the amino acid sequence of SEQ ID NO:28.

In one aspect, the Fab domain capable of specific binding to FAP comprises a heavy chain variable region comprising an amino acid sequence of SEQ ID NO:25 and a light chain variable region comprising an amino acid sequence of SEQ ID NO:26 or a heavy chain variable region comprising an amino acid sequence of SEQ ID NO:27 and a light chain variable region comprising an amino acid sequence of SEQ ID NO:28. In a particular aspect, the Fab domain capable of specific binding to FAP comprises a heavy chain variable region comprising the amino acid sequence of SEQ ID NO:25 and a light chain variable region comprising the amino acid sequence of SEQ ID NO:26. In another particular aspect, the Fab domain capable of specific binding to FAP comprises a heavy chain variable region comprising the amino acid sequence of SEQ ID NO:27 and a light chain variable region comprising the amino acid sequence of SEQ ID NO:28. In a specific aspect, the Fab domain capable of specific binding to FAP comprises a VH domain consisting of amino acid sequence of SEQ ID NO:27 and a VL domain consisting of the amino acid sequence of SEQ ID NO:28.

In a further aspect, provided is a 4-1BBL trimer-containing antigen binding molecule comprising

(a) a Fab domain capable of specific binding to FAP comprising a VH domain comprising an amino acid sequence of SEQ ID NO:25 and a VL domain comprising an amino acid sequence of SEQ ID NO:26, or a VH domain comprising an amino acid sequence of SEQ ID NO:27 and a VL domain comprising an amino acid sequence of SEQ ID NO:28,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain.

In another aspect, provided is a 4-1BBL trimer-containing antigen binding molecule comprising

(a) a Fab domain capable of specific binding to FAP comprising a VH domain comprising an amino acid sequence of SEQ ID NO:25 and a VL domain comprising an amino acid sequence of SEQ ID NO:26, or a VH domain comprising an amino acid sequence of SEQ ID NO:27 and a VL domain comprising an amino acid sequence of SEQ ID NO:28,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL or fragments thereof comprises an amino acid sequence selected from the group consisting of SEQ ID NO:9 and SEQ ID NO: 10 and the second polypeptide comprising one ectodomain of said 4-1BBL or a fragment thereof comprises the amino acid sequence selected from the group consisting of SEQ ID NO: 1 and SEQ ID NO:5.

In a particular aspect, provided is a 4-1BBL trimer-containing antigen binding molecule comprising

(a) a Fab domain capable of specific binding to FAP comprising a VH domain comprising an amino acid sequence of SEQ ID NO:27 and a VL domain comprising an amino acid sequence of SEQ ID NO:28,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL comprises an amino acid sequence of SEQ ID NO: 10 and the second polypeptide comprising one ectodomain of said 4-1BBL comprises the amino acid sequence of SEQ ID NO:5.

In another aspect, provided is a 4-1BBL trimer-containing antigen binding molecule, wherein the antigen binding molecule comprises

(i) a light chain comprising the VL domain of the Fab domain capable of specific binding to FAP,
(ii) a fusion polypeptide comprising a first subunit of a Fc domain and a first polypeptide comprising two ectodomains of 4-1BBL or fragments thereof that are connected to each other by a peptide linker, wherein the first polypeptide is fused at its N-terminus to the C-terminus of the first subunit of the Fc domain, and
(iii) a heavy chain comprising the VH domain of the Fab domain capable of specific binding to FAP, a second subunit of the Fc domain and and a second polypeptide comprising one ectodomain of said 4-1BBL or a fragment thereof, wherein the variable heavy chain of the Fab domain capable of specific binding to FAP is fused at its C-terminus to the N-terminus of the second subunit of the Fc domain and wherein the second polypeptide comprising one ectodomain of said 4-1BBL or a fragment thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 106,
(ii) a fusion polypeptide comprising an amino acid sequence that is at least about 95%, 96%, 97%, 98%, 99% or 100% identical to the amino acid sequence of SEQ ID NO: 104, and
(iii) a heavy chain comprising an amino acid sequence that is at least about 95%, 96%, 97%, 98%, 99% or 100% identical to the amino acid sequence of SEQ ID NO: 105.

More particularly, the 4-1BBL trimer-containing antigen binding molecule comprises (i) a light chain comprising the amino acid sequence of SEQ ID NO: 106,

(ii) a fusion polypeptide comprising the amino acid sequence of SEQ ID NO: 104, and
(iii) a heavy chain comprising the amino acid sequence of SEQ ID NO: 105.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:114,
(ii) a fusion polypeptide comprising an amino acid sequence that is at least about 95%, 96%, 97%, 98%, 99% or 100% identical to the amino acid sequence of SEQ ID NO:104, and
(iii) a heavy chain comprising an amino acid sequence that is at least about 95%, 96%, 97%, 98%, 99% or 100% identical to the amino acid sequence of SEQ ID NO:113.

More particularly, the 4-1BBL trimer-containing antigen binding molecule comprises (i) a light chain comprising the amino acid sequence of SEQ ID NO:114,

(ii) a fusion polypeptide comprising the amino acid sequence of SEQ ID NO:104, and
(iii) a heavy chain comprising the amino acid sequence of SEQ ID NO:113.

In a further aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:106,
(ii) a fusion polypeptide comprising an amino acid sequence that is at least about 95%, 96%, 97%, 98%, 99% or 100% identical to the amino acid sequence of SEQ ID NO:178, and
(iii) a heavy chain comprising an amino acid sequence that is at least about 95%, 96%, 97%, 98%, 99% or 100% identical to the amino acid sequence of SEQ ID NO:177.

More particularly, the 4-1BBL trimer-containing antigen binding molecule comprises (i) a light chain comprising the amino acid sequence of SEQ ID NO:106,

(ii) a fusion polypeptide comprising the amino acid sequence of SEQ ID NO:178, and
(iii) a heavy chain comprising the amino acid sequence of SEQ ID NO:177.

In yet another aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:114,
(ii) a fusion polypeptide comprising an amino acid sequence that is at least about 95%, 96%, 97%, 98%, 99% or 100% identical to the amino acid sequence of SEQ ID NO:178, and
(iii) a heavy chain comprising an amino acid sequence that is at least about 95%, 96%, 97%, 98%, 99% or 100% identical to the amino acid sequence of SEQ ID NO:182.

More particularly, the 4-1BBL trimer-containing antigen binding molecule comprises (i) a light chain comprising the amino acid sequence of SEQ ID NO:114,

(ii) a fusion polypeptide comprising the amino acid sequence of SEQ ID NO:178, and
(iii) a heavy chain comprising the amino acid sequence of SEQ ID NO:182.

In another aspect, provided is a 4-1BBL trimer-containing antigen binding molecule, wherein the antigen binding molecule comprises

(i) a light chain comprising the VL domain of the Fab domain capable of specific binding to FAP,
(ii) a fusion polypeptide comprising a first subunit of a Fc domain and a second polypeptide comprising one ectodomain of said 4-1BBL or a fragment thereof, wherein the second polypeptide is fused at its N-terminus to the C-terminus of the first subunit of the Fc domain, and
(iii) a heavy chain comprising the VH domain of the Fab domain capable of specific binding to FAP, a second subunit of the Fc domain and and a first polypeptide comprising two ectodomains of 4-1BBL or fragments thereof that are connected to each other by a peptide linker, wherein the variable heavy chain of the Fab domain capable of specific binding to FAP is fused at its C-terminus to the N-terminus of the second subunit of the Fc domain and wherein the first polypeptide comprising two ectodomains of 4-1BBL or fragments thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:106,
(ii) a fusion polypeptide comprising an amino acid sequence that is at least about 95%, 96%, 97%, 98%, 99% or 100% identical to the amino acid sequence of SEQ ID NO:109, and
(iii) a heavy chain comprising an amino acid sequence that is at least about 95%, 96%, 97%, 98%, 99% or 100% identical to the amino acid sequence of SEQ ID NO:110.

More particularly, the 4-1BBL trimer-containing antigen binding molecule comprises (i) a light chain comprising the amino acid sequence of SEQ ID NO:106,

(ii) a fusion polypeptide comprising the amino acid sequence of SEQ ID NO:109, and
(iii) a heavy chain comprising the amino acid sequence of SEQ ID NO:110.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:114,
(ii) a fusion polypeptide comprising an amino acid sequence that is at least about 95%, 96%, 97%, 98%, 99% or 100% identical to the amino acid sequence of SEQ ID NO:109, and
(iii) a heavy chain comprising an amino acid sequence that is at least about 95%, 96%, 97%, 98%, 99% or 100% identical to the amino acid sequence of SEQ ID NO:116.

More particularly, the 4-1BBL trimer-containing antigen binding molecule comprises (i) a light chain comprising the amino acid sequence of SEQ ID NO:114,

(ii) a fusion polypeptide comprising the amino acid sequence of SEQ ID NO:109, and
(iii) a heavy chain comprising the amino acid sequence of SEQ ID NO:116.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:106,
(ii) a fusion polypeptide comprising an amino acid sequence that is at least about 95%, 96%, 97%, 98%, 99% or 100% identical to the amino acid sequence of SEQ ID NO:174, and
(iii) a heavy chain comprising an amino acid sequence that is at least about 95%, 96%, 97%, 98%, 99% or 100% identical to the amino acid sequence of SEQ ID NO:173.

More particularly, the 4-1BBL trimer-containing antigen binding molecule comprises (i) a light chain comprising the amino acid sequence of SEQ ID NO:106,

(ii) a fusion polypeptide comprising the amino acid sequence of SEQ ID NO:174, and
(iii) a heavy chain comprising the amino acid sequence of SEQ ID NO:173.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:114,
(ii) a fusion polypeptide comprising an amino acid sequence that is at least about 95%, 96%, 97%, 98%, 99% or 100% identical to the amino acid sequence of SEQ ID NO:174, and
(iii) a heavy chain comprising an amino acid sequence that is at least about 95%, 96%, 97%, 98%, 99% or 100% identical to the amino acid sequence of SEQ ID NO:180.

More particularly, the 4-1BBL trimer-containing antigen binding molecule comprises (i) a light chain comprising the amino acid sequence of SEQ ID NO:114,

(ii) a fusion polypeptide comprising the amino acid sequence of SEQ ID NO:174, and
(iii) a heavy chain comprising the amino acid sequence of SEQ ID NO:180.

In a further aspect, provided is a 4-1BBL trimer-containing antigen binding molecule, wherein the antigen binding molecule comprises

(i) a first light chain comprising the VL domain of the Fab domain capable of specific binding to FAP,
(ii) a first heavy chain comprising the VH domain of the Fab domain capable of specific binding to FAP, a first subunit of a Fc domain and a second polypeptide comprising one ectodomain of said 4-1BBL or a fragment thereof, wherein the variable heavy chain of the Fab domain capable of specific binding to FAP is fused at its C-terminus to the N-terminus of the second subunit of the Fc domain and wherein the second polypeptide is fused at its N-terminus to the C-terminus of the first subunit of the Fc domain,
(iii) a second heavy chain comprising the VH domain of a Fab domain that is not capable of specific binding to a target cell antigen, a second subunit of the Fc domain and and a first polypeptide comprising two ectodomains of 4-1BBL or fragments thereof that are connected to each other by a peptide linker, wherein the variable heavy chain of the Fab domain not capable of specific binding to a target cell antigen is fused at its C-terminus to the N-terminus of the second subunit of the Fc domain and wherein the first polypeptide comprising two ectodomains of 4-1BBL or fragments thereof is fused at its N-terminus to the C-terminus of the second subunit of the Fc domain, and
(iv) a second light chain comprising the VL domain of the Fab domain that is not capable of specific binding to a target cell antigen.

In a particular aspect, provided is a 4-1BBL trimer-containing antigen binding molecule, wherein the antigen binding molecule comprises

(i) a first light chain comprising an amino acid sequence that is at least about 95%, 96%, 97%, 98%, 99% or 100% identical to the amino acid sequence of SEQ ID NO: 106,
(ii) a first heavy chain comprising an amino acid sequence that is at least about 95%, 96%, 97%, 98%, 99% or 100% identical to the amino acid sequence of SEQ ID NO:217,
(iii) a second heavy chain comprising an amino acid sequence that is at least about 95%, 96%, 97%, 98%, 99% or 100% identical to the amino acid sequence of SEQ ID NO:218, and
(iv) a second light chain comprising an amino acid sequence that is at least about 95%, 96%, 97%, 98%, 99% or 100% identical to the amino acid sequence of SEQ ID NO:214.

More particularly, the 4-1BBL trimer-containing antigen binding molecule comprises (i) a first light chain comprising the amino acid sequence of SEQ ID NO: 106,

(ii) a first heavy chain comprising the amino acid sequence of SEQ ID NO:217,
(iii) a second heavy chain comprising the amino acid sequence of SEQ ID NO:218, and
(iv) a second light chain comprising the amino acid sequence of SEQ ID NO:214.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 106,
(ii) a first heavy chain comprising an amino acid sequence that is at least about 95%, 96%, 97%, 98%, 99% or 100% identical to the amino acid sequence of SEQ ID NO:222,
(iii) a second heavy chain comprising an amino acid sequence that is at least about 95%, 96%, 97%, 98%, 99% or 100% identical to the amino acid sequence of SEQ ID NO:221, and
(iv) a second light chain comprising an amino acid sequence that is at least about 95%, 96%, 97%, 98%, 99% or 100% identical to the amino acid sequence of SEQ ID NO:214.

More particularly, the 4-1BBL trimer-containing antigen binding molecule comprises (i) a first light chain comprising the amino acid sequence of SEQ ID NO: 106,

(ii) a first heavy chain comprising the amino acid sequence of SEQ ID NO:222,
(iii) a second heavy chain comprising the amino acid sequence of SEQ ID NO:221, and
(iv) a second light chain comprising the amino acid sequence of SEQ ID NO:214.

In another aspect, provided is a 4-1BBL trimer-containing antigen binding molecule, wherein the antigen binding molecule comprises

(i) a first light chain comprising the VL domain of the Fab domain capable of specific binding to FAP,
(ii) a first heavy chain comprising the VH domain of the Fab domain capable of specific binding to FAP, a first subunit of a Fc domain and and a first polypeptide comprising two ectodomains of a TNF ligand family member or fragments thereof that are connected to each other by a peptide linker, wherein the variable heavy chain of the Fab domain capable of specific binding to FAP is fused at its C-terminus to the N-terminus of the second subunit of the Fc domain and wherein the first polypeptide comprising ectodomains of 4-1BBL or fragments thereof is fused at its N-terminus to the C-terminus of the first subunit of the Fc domain,
(iii) a second heavy chain comprising the VH domain of a Fab domain that is not capable of specific binding to a target cell antigen, a second subunit of the Fc domain and and a second polypeptide comprising one ectodomain of said 4-1BBL or a fragment thereof, wherein the variable heavy chain of the Fab domain not capable of specific binding to a target cell antigen is fused at its C-terminus to the N-terminus of the second subunit of the Fc domain and wherein the second polypeptide comprising one ectodomain of 4-1BBL or fragments thereof is fused at its N-terminus to the C-terminus of the second subunit of the Fc domain, and
(iv) a second light chain comprising the VL domain of the Fab domain that is not capable of specific binding to a target cell antigen.

In a particular aspect, provided is a 4-1BBL trimer-containing antigen binding molecule, wherein the antigen binding molecule comprises

(i) a first light chain comprising an amino acid sequence that is at least about 95%, 96%, 97%, 98%, 99% or 100% identical to the amino acid sequence of SEQ ID NO: 106,
(ii) a first heavy chain comprising an amino acid sequence that is at least about 95%, 96%, 97%, 98%, 99% or 100% identical to the amino acid sequence of SEQ ID NO:212,
(iii) a second heavy chain comprising an amino acid sequence that is at least about 95%, 96%, 97%, 98%, 99% or 100% identical to the amino acid sequence of SEQ ID NO:213, and
(iv) a second light chain comprising an amino acid sequence that is at least about 95%, 96%, 97%, 98%, 99% or 100% identical to the amino acid sequence of SEQ ID NO:214.

More particularly, the 4-1BBL trimer-containing antigen binding molecule comprises (i) a first light chain comprising the amino acid sequence of SEQ ID NO: 106,

(ii) a first heavy chain comprising the amino acid sequence of SEQ ID NO:212,
(iii) a second heavy chain comprising the amino acid sequence of SEQ ID NO:213, and
(iv) a second light chain comprising the amino acid sequence of SEQ ID NO:214.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:106,
(ii) a first heavy chain comprising an amino acid sequence that is at least about 95%, 96%, 97%, 98%, 99% or 100% identical to the amino acid sequence of SEQ ID NO:226,
(iii) a second heavy chain comprising an amino acid sequence that is at least about 95%, 96%, 97%, 98%, 99% or 100% identical to the amino acid sequence of SEQ ID NO:225, and
(iv) a second light chain comprising an amino acid sequence that is at least about 95%, 96%, 97%, 98%, 99% or 100% identical to the amino acid sequence of SEQ ID NO:214.

More particularly, the 4-1BBL trimer-containing antigen binding molecule comprises (i) a first light chain comprising the amino acid sequence of SEQ ID NO: 106,

(ii) a first heavy chain comprising the amino acid sequence of SEQ ID NO:226,

(iii) a second heavy chain comprising the amino acid sequence of SEQ ID NO:225, and
(iv) a second light chain comprising the amino acid sequence of SEQ ID NO:214.

**4-1BBL trimer-containing antigen binding molecules, wherein the target cell antigen is CD19**

In one aspect, provided is a 4-1BBL trimer-containing antigen binding molecule, wherein the target cell antigen is CD19.

In a particular aspect, provided is a 4-1BBL trimer-containing antigen binding molecule as defined herein before, wherein the Fab domain capable of specific binding to CD19 comprises a heavy chain variable region comprising an amino acid sequence that is at least about 95%, 96%, 97%, 98%, 99% or 100% identical to the amino acid sequence of SEQ ID NO:41 and a light chain variable region comprising an amino acid sequence that is at least about 95%, 96%, 97%, 98%, 99% or 100% identical to the amino acid sequence of SEQ ID NO:42.

In another particular aspect, provided is a 4-1BBL trimer-containing antigen binding molecule as defined herein before, wherein the Fab domain capable of specific binding to CD 19 comprises a heavy chain variable region comprising an amino acid sequence that is at least about 95%, 96%, 97%, 98%, 99% or 100% identical to the amino acid sequence of SEQ ID NO:43 and a light chain variable region comprising an amino acid sequence that is at least about 95%, 96%, 97%, 98%, 99% or 100% identical to the amino acid sequence of SEQ ID NO:44.

In one aspect, the Fab domain capable of specific binding to CD19 comprises a variable heavy chain comprising an amino acid sequence of SEQ ID NO:41 and a variable light chain comprising an amino acid sequence of SEQ ID NO:42 or a variable heavy chain comprising an amino acid sequence of SEQ ID NO:43 and a variable light chain comprising an amino acid sequence of SEQ ID NO:44. In a particular aspect, the Fab domain capable of specific binding to FAP comprises a heavy chain variable region comprising the amino acid sequence of SEQ ID NO:41 and a light chain variable region comprising the amino acid sequence of SEQ ID NO:42. In another particular aspect, the Fab domain capable of specific binding to FAP comprises a heavy chain variable region comprising the amino acid sequence of SEQ ID NO:43 and a light chain variable region comprising the amino acid sequence of SEQ ID NO:44. In a specific aspect, the Fab domain capable of specific binding to FAP comprises a VH domain consisting of amino acid sequence of SEQ ID NO:43 and a VL domain consisting of the amino acid sequence of SEQ ID NO:44

In a further aspect, provided is a 4-1BBL trimer-containing antigen binding molecule comprising

(a) a Fab domain capable of specific binding to CD19 comprising a VH domain comprising an amino acid sequence of SEQ ID NO:41 and a VL domain comprising an amino acid sequence of SEQ ID NO:42, or a VH domain comprising an amino acid sequence of SEQ ID NO:43 and a VL domain comprising an amino acid sequence of SEQ ID NO:44,
(b) a Fc domain composed of a first and a second subunit capable of stable association, and
(c) a first polypeptide comprising two ectodomains of a TNF ligand family member or fragments thereof that are connected to each other by a peptide linker and a second polypeptide comprising one ectodomain of said TNF ligand family member or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain.

In another aspect, provided is a 4-1BBL trimer-containing antigen binding molecule comprising

(a) a Fab domain capable of specific binding to CD19 comprising a VH domain comprising an amino acid sequence of SEQ ID NO:41 and a VL domain comprising an amino acid sequence of SEQ ID NO:42, or a VH domain comprising an amino acid sequence of SEQ ID NO:43 and a VL domain comprising an amino acid sequence of SEQ ID NO:44,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL or fragments thereof comprises an amino acid sequence selected from the group consisting of SEQ ID NO:9 and SEQ ID NO: 10 and the second polypeptide comprising one ectodomain of said 4-1BBL or a fragment thereof comprises the amino acid sequence selected from the group consisting of SEQ ID NO: 1 and SEQ ID NO:5.

In a particular aspect, provided is a 4-1BBL trimer-containing antigen binding molecule comprising

(a) a Fab domain capable of specific binding to CD19 comprising a VH domain comprising an amino acid sequence of SEQ ID NO:43 and a VL domain comprising an amino acid sequence of SEQ ID N0:44,

(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL comprising an amino acid sequence of SEQ ID NO: 10 and the second polypeptide comprising one ectodomain of said TNF ligand family member comprising the amino acid sequence of SEQ ID NO:5.

In another aspect, provided is a 4-1BBL trimer-containing antigen binding molecule, wherein the antigen binding molecule comprises

(i) a light chain comprising the variable light chain of the Fab domain capable of specific binding to CD 19,
(ii) a fusion polypeptide comprising a first subunit of a Fc domain and a first polypeptide comprising two ectodomains of 4-1BBL or fragments thereof that are connected to each other by a peptide linker, wherein the first polypeptide is fused at its N-terminus to the C-terminus of the first subunit of the Fc domain, and
(iii) a heavy chain comprising the variable heavy chain of the Fab domain capable of specific binding to CD19, a second subunit of the Fc domain and and a second polypeptide comprising one ectodomain of said 4-1BBL or a fragment thereof, wherein the variable heavy chain of the Fab domain capable of specific binding to CD19 is fused at its C-terminus to the N-terminus of the second subunit of the Fc domain and wherein the second polypeptide comprising one ectodomain of said 4-1BBL or a fragment thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 120,
(ii) a fusion polypeptide comprising an amino acid sequence that is at least about 95%, 96%, 97%, 98%, 99% or 100% identical to the amino acid sequence of SEQ ID NO:104, and
(iii) a heavy chain comprising an amino acid sequence that is at least about 95%, 96%, 97%, 98%, 99% or 100% identical to the amino acid sequence of SEQ ID NO:119.

More particularly, the 4-1BBL trimer-containing antigen binding molecule comprises (i) a light chain comprising the amino acid sequence of SEQ ID NO: 120,

(ii) a fusion polypeptide comprising the amino acid sequence of SEQ ID NO:104, and
(iii) a heavy chain comprising the amino acid sequence of SEQ ID NO:119.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:126,
(ii) a fusion polypeptide comprising an amino acid sequence that is at least about 95%, 96%, 97%, 98%, 99% or 100% identical to the amino acid sequence of SEQ ID NO:104, and
(iii) a heavy chain comprising an amino acid sequence that is at least about 95%, 96%, 97%, 98%, 99% or 100% identical to the amino acid sequence of SEQ ID NO:125.

More particularly, the 4-1BBL trimer-containing antigen binding molecule comprises (i) a light chain comprising the amino acid sequence of SEQ ID NO:126,

(ii) a fusion polypeptide comprising the amino acid sequence of SEQ ID NO:174, and
(iii) a heavy chain comprising the amino acid sequence of SEQ ID NO:125.

In a further aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 120,
(ii) a fusion polypeptide comprising an amino acid sequence that is at least about 95%, 96%, 97%, 98%, 99% or 100% identical to the amino acid sequence of SEQ ID NO:178, and
(iii) a heavy chain comprising an amino acid sequence that is at least about 95%, 96%, 97%, 98%, 99% or 100% identical to the amino acid sequence of SEQ ID NO: 186.

More particularly, the 4-1BBL trimer-containing antigen binding molecule comprises (i) a light chain comprising the amino acid sequence of SEQ ID NO: 120,

(ii) a fusion polypeptide comprising the amino acid sequence of SEQ ID NO: 178, and
(iii) a heavy chain comprising the amino acid sequence of SEQ ID NO: 186.

In yet another aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 126,
(ii) a fusion polypeptide comprising an amino acid sequence that is at least about 95%, 96%, 97%, 98%, 99% or 100% identical to the amino acid sequence of SEQ ID NO: 178, and
(iii) a heavy chain comprising an amino acid sequence that is at least about 95%, 96%, 97%, 98%, 99% or 100% identical to the amino acid sequence of SEQ ID NO: 190.

More particularly, the 4-1BBL trimer-containing antigen binding molecule comprises (i) a light chain comprising the amino acid sequence of SEQ ID NO: 126,

(ii) a fusion polypeptide comprising the amino acid sequence of SEQ ID NO: 178, and
(iii) a heavy chain comprising the amino acid sequence of SEQ ID NO: 190.

In another aspect, provided is a 4-1BBL trimer-containing antigen binding molecule, wherein the antigen binding molecule comprises

(i) a light chain comprising the variable light chain of the Fab domain capable of specific binding to CD 19,
(ii) a fusion polypeptide comprising a first subunit of a Fc domain and a second polypeptide comprising one ectodomain of said 4-1BBL or a fragment thereof, wherein the second polypeptide is fused at its N-terminus to the C-terminus of the first subunit of the Fc domain, and
(iii) a heavy chain comprising the variable heavy chain of the Fab domain capable of specific binding to CD 19, a second subunit of the Fc domain and and a first polypeptide comprising two ectodomains of 4-1BBL or fragments thereof that are connected to each other by a peptide linker, wherein the variable heavy chain of the Fab domain capable of specific binding to CD19 is fused at its C-terminus to the N-terminus of the second subunit of the Fc domain and wherein the first polypeptide comprising two ectodomains of 4-1BBL or fragments thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 120,
(ii) a fusion polypeptide comprising an amino acid sequence that is at least about 95%, 96%, 97%, 98%, 99% or 100% identical to the amino acid sequence of SEQ ID NO:109, and
(iii) a heavy chain comprising an amino acid sequence that is at least about 95%, 96%, 97%, 98%, 99% or 100% identical to the amino acid sequence of SEQ ID NO:122.

More particularly, the 4-1BBL trimer-containing antigen binding molecule comprises (i) a light chain comprising the amino acid sequence of SEQ ID NO: 120,

(ii) a fusion polypeptide comprising the amino acid sequence of SEQ ID NO:109, and
(iii) a heavy chain comprising the amino acid sequence of SEQ ID NO:122.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO:126,
(ii) a fusion polypeptide comprising an amino acid sequence that is at least about 95%, 96%, 97%, 98%, 99% or 100% identical to the amino acid sequence of SEQ ID NO:109, and
(iii) a heavy chain comprising an amino acid sequence that is at least about 95%, 96%, 97%, 98%, 99% or 100% identical to the amino acid sequence of SEQ ID NO:128.

More particularly, the 4-1BBL trimer-containing antigen binding molecule comprises (i) a light chain comprising the amino acid sequence of SEQ ID NO:126,

(ii) a fusion polypeptide comprising the amino acid sequence of SEQ ID NO:109, and
(iii) a heavy chain comprising the amino acid sequence of SEQ ID NO:128.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 120,
(ii) a fusion polypeptide comprising an amino acid sequence that is at least about 95%, 96%, 97%, 98%, 99% or 100% identical to the amino acid sequence of SEQ ID NO:174, and
(iii) a heavy chain comprising an amino acid sequence that is at least about 95%, 96%, 97%, 98%, 99% or 100% identical to the amino acid sequence of SEQ ID NO:184.

More particularly, the 4-1BBL trimer-containing antigen binding molecule comprises (i) a light chain comprising the amino acid sequence of SEQ ID NO:120,

(ii) a fusion polypeptide comprising the amino acid sequence of SEQ ID NO:174, and
(iii) a heavy chain comprising the amino acid sequence of SEQ ID NO:184.

In another particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 126,
(ii) a fusion polypeptide comprising an amino acid sequence that is at least about 95%, 96%, 97%, 98%, 99% or 100% identical to the amino acid sequence of SEQ ID NO: 174, and
(iii) a heavy chain comprising an amino acid sequence that is at least about 95%, 96%, 97%, 98%, 99% or 100% identical to the amino acid sequence of SEQ ID NO: 188.

More particularly, the 4-1BBL trimer-containing antigen binding molecule comprises (i) a light chain comprising the amino acid sequence of SEQ ID NO: 126,

(ii) a fusion polypeptide comprising the amino acid sequence of SEQ ID NO: 174, and
(iii) a heavy chain comprising the amino acid sequence of SEQ ID NO: 188.

**4-1BBL trimer-containing antigen binding molecules, wherein the target cell antigen is CEA**

In one aspect, provided is a 4-1BBL trimer-containing antigen binding molecule, wherein the target cell antigen is CEA.

In a particular aspect, provided is a 4-1BBL trimer-containing antigen binding molecule as defined herein before, wherein the Fab domain capable of specific binding to CEA comprises a heavy chain variable region comprising an amino acid sequence that is at least about 95%, 96%, 97%, 98%, 99% or 100% identical to the amino acid sequence of SEQ ID NO:51 and a light chain variable region comprising an amino acid sequence that is at least about 95%, 96%, 97%, 98%, 99% or 100% identical to the amino acid sequence of SEQ ID NO:52.

In one aspect, the Fab domain capable of specific binding to CEA comprises a variable heavy chain comprising an amino acid sequence of SEQ ID NO:51 and a variable light chain comprising an amino acid sequence of SEQ ID NO:52 or a variable heavy chain comprising an amino acid sequence of SEQ ID NO: 163 and a variable light chain comprising an amino acid sequence of SEQ ID NO:164. In a particular aspect, the Fab domain capable of specific binding to CEA comprises a heavy chain variable region comprising the amino acid sequence of SEQ ID NO:51 and a light chain variable region comprising the amino acid sequence of SEQ ID NO:52. In a specific aspect, the Fab domain capable of specific binding to CEA comprises a VH domain consisting of amino acid sequence of SEQ ID NO:51 and a VL domain consisting of the amino acid sequence of SEQ ID NO:52.

In a further aspect, the invention provides a 4-1BBL trimer-containing antigen binding molecule comprising

(a) a Fab domain capable of specific binding to CEA comprising a VH domain comprising an amino acid sequence of SEQ ID NO:51 and a VL domain comprising an amino acid sequence of SEQ ID NO:52,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain.

In another aspect, the invention provides a 4-1BBL trimer-containing antigen binding molecule comprising

(a) a Fab domain capable of specific binding to CEA comprising a VH domain comprising an amino acid sequence of SEQ ID NO:51 and a VL domain comprising an amino acid sequence of SEQ ID NO:52,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL or fragments thereof comprises an amino acid sequence selected from the group consisting of SEQ ID NO:9 and SEQ ID NO: 10 and the second polypeptide comprising one ectodomain of said 4-1BBL or a fragment thereof comprises the amino acid sequence selected from the group consisting of SEQ ID NO: 1 and SEQ ID NO:5.

In a particular aspect, provided is a 4-1BBL trimer-containing antigen binding molecule comprising

(a) a Fab domain capable of specific binding to CEA comprising a VH domain comprising an amino acid sequence of SEQ ID NO:51 and a VL domain comprising an amino acid sequence of SEQ ID NO:52,
(b) a Fc domain composed of a first and a second subunit capable of stable association, and
(c) a first polypeptide comprising two ectodomains of 4-1BBL or fragments thereof that are connected to each other by a peptide linker and a second polypeptide comprising one ectodomain of said 4-1BBL or a fragment thereof, wherein the first polypeptide is fused at its N-terminus to the C-terminus to one of the subunits of the Fc domain and wherein the second polypeptide is fused at its N-terminus to the C-terminus of the other subunit of the Fc domain and wherein the first polypeptide comprising two ectodomains of 4-1BBL comprising an amino acid sequence of SEQ ID NO:10 and the second polypeptide comprising one ectodomain of said 4-1BBL comprising the amino acid sequence of SEQ ID NO:5.

In another aspect, provided is a 4-1BBL trimer-containing antigen binding molecule, wherein the antigen binding molecule comprises

(i) a light chain comprising the variable light chain of the Fab domain capable of specific binding to CEA,
(ii) a fusion polypeptide comprising a first subunit of a Fc domain and a first polypeptide comprising two ectodomains of 4-1BBL or fragments thereof that are connected to each other by a peptide linker, wherein the first polypeptide is fused at its N-terminus to the C-terminus of the first subunit of the Fc domain, and
(iii) a heavy chain comprising the variable heavy chain of the Fab domain capable of specific binding to CEA, a second subunit of the Fc domain and and a second polypeptide comprising one ectodomain of said 4-1BBL or a fragment thereof, wherein the variable heavy chain of the Fab domain capable of specific binding to CEA is fused at its C-terminus to the N-terminus of the second subunit of the Fc domain and wherein the second polypeptide comprising one ectodomain of said 4-1BBL or a fragment thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 156,
(ii) a fusion polypeptide comprising an amino acid sequence that is at least about 95%, 96%, 97%, 98%, 99% or 100% identical to the amino acid sequence of SEQ ID NO: 104, and
(iii) a heavy chain comprising an amino acid sequence that is at least about 95%, 96%, 97%, 98%, 99% or 100% identical to the amino acid sequence of SEQ ID NO: 155.

More particularly, the 4-1BBL trimer-containing antigen binding molecule comprises (i) a light chain comprising the amino acid sequence of SEQ ID NO: 156,

(ii) a fusion polypeptide comprising the amino acid sequence of SEQ ID NO: 104, and
(iii) a heavy chain comprising the amino acid sequence of SEQ ID NO: 155.

In a further aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 156,
(ii) a fusion polypeptide comprising an amino acid sequence that is at least about 95%, 96%, 97%, 98%, 99% or 100% identical to the amino acid sequence of SEQ ID NO: 178, and
(iii) a heavy chain comprising an amino acid sequence that is at least about 95%, 96%, 97%, 98%, 99% or 100% identical to the amino acid sequence of SEQ ID NO: 194.

More particularly, the 4-1BBL trimer-containing antigen binding molecule comprises (i) a light chain comprising the amino acid sequence of SEQ ID NO: 156,

(ii) a fusion polypeptide comprising the amino acid sequence of SEQ ID NO: 178, and
(iii) a heavy chain comprising the amino acid sequence of SEQ ID NO: 194.

In another aspect, provided is a 4-1BBL trimer-containing antigen binding molecule, wherein the antigen binding molecule comprises

(i) a light chain comprising the variable light chain of the Fab domain capable of specific binding to CEA,
(ii) a fusion polypeptide comprising a first subunit of a Fc domain and a second polypeptide comprising one ectodomain of said 4-1BBL or a fragment thereof, wherein the second polypeptide is fused at its N-terminus to the C-terminus of the first subunit of the Fc domain, and
(iii) a heavy chain comprising the variable heavy chain of the Fab domain capable of specific binding to CEA, a second subunit of the Fc domain and and a first polypeptide comprising two ectodomains of 4-1BBL or fragments thereof that are connected to each other by a peptide linker, wherein the variable heavy chain of the Fab domain capable of specific binding to CEA is fused at its C-terminus to the N-terminus of the second subunit of the Fc domain and wherein the first polypeptide comprising two ectodomains of 4-1BBL or fragments thereof is fused at its N-terminus to the C-terminus of the second subunit of the Fc domain.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 156,
(ii) a fusion polypeptide comprising an amino acid sequence that is at least about 95%, 96%, 97%, 98%, 99% or 100% identical to the amino acid sequence of SEQ ID NO: 109, and
(iii) a heavy chain comprising an amino acid sequence that is at least about 95%, 96%, 97%, 98%, 99% or 100% identical to the amino acid sequence of SEQ ID NO:158.

More particularly, the 4-1BBL trimer-containing antigen binding molecule comprises (i) a light chain comprising the amino acid sequence of SEQ ID NO: 156,

(ii) a fusion polypeptide comprising the amino acid sequence of SEQ ID NO: 109, and
(iii) a heavy chain comprising the amino acid sequence of SEQ ID NO: 158.

In a particular aspect, provided is a 4-1BBL trimer-containing antigen binding molecule, wherein the antigen binding molecule comprises

(i) a light chain comprising an amino acid sequence that is at least about 95%, 96%, 97%, 98%, 99% or 100% identical to the amino acid sequence of SEQ ID NO: 156,
(ii) a fusion polypeptide comprising an amino acid sequence that is at least about 95%, 96%, 97%, 98%, 99% or 100% identical to the amino acid sequence of SEQ ID NO: 174, and
(iii) a heavy chain comprising an amino acid sequence that is at least about 95%, 96%, 97%, 98%, 99% or 100% identical to the amino acid sequence of SEQ ID NO: 192.

More particularly, the 4-1BBL trimer-containing antigen binding molecule comprises (i) a light chain comprising the amino acid sequence of SEQ ID NO: 156,

(ii) a fusion polypeptide comprising the amino acid sequence of SEQ ID NO: 174, and
(iii) a heavy chain comprising the amino acid sequence of SEQ ID NO: 192.

**Polynucleotides**

The invention further provides isolated polynucleotides encoding a 4-1BBL trimer-containing antigen binding molecule as described herein or a fragment thereof.

The isolated polynucleotides encoding 4-1BBL trimer-containing antigen binding molecules may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes the entire 4-1BBL trimer-containing antigen binding molecule according to the invention as described herein. In particular, the isolated polynucleotide encodes a polypeptide comprised in the 4-1BBL trimer-containing antigen binding molecule according to the invention as described herein.

In one aspect, the disclosure is directed to an isolated polynucleotide encoding a 4-1BBL trimer-containing antigen binding molecule, wherein the polynucleotide comprises (a) a sequence that encodes a Fab domain capable of specific binding to a target cell antigen, (b) a sequence that encodes a first subunit of a Fc domain and first polypeptide comprising two ectodomains of a 4-1BBL or fragments thereof that are connected to each other by a peptide linker and (c) a sequence that encodes a second subunit of a Fc domain and and a second polypeptide comprising one ectodomain of said 4-1BBL or a fragment thereof.

In certain aspects, provided an isolated polynucleotide encoding a 4-1BBL trimer-containing antigen binding molecule, wherein the polynucleotide comprises (a) a sequence that encodes a Fab domain capable of specific binding to a target cell antigen, (b) a sequence that encodes a first subunit of a Fc domain and first polypeptide comprising two ectodomains of 4-1BBL or fragments thereof that are connected to each other by a peptide linker, (c) a sequence that encodes a second subunit of a Fc domain and and a second polypeptide comprising one ectodomain of said 4-1BBL or a fragment thereof, and (d) a sequence that encodes a Fab domain that is not capable of specific binding to a target cell antigen.

In another aspect, provided is an isolated polynucleotide encoding a 4-1BB ligand trimer-containing antigen binding molecule, wherein the polynucleotide comprises (a) a sequence that encodes a Fab domain capable of specific binding to a target cell antigen, (b) a sequence that encodes a first subunit of a Fc domain and first polypeptide comprising two ectodomains of 4-1BBL or fragments thereof that are connected to each other by a peptide linker and (c) a sequence that encodes a second subunit of a Fc domain and and a second polypeptide comprising one ectodomain of said 4-1BBL or a fragment thereof.

In a further aspect, the disclosure is directed to an isolated polynucleotide comprising a sequence that encodes a polypeptide comprising two 4-1BBL fragments comprising an amino acid sequence that is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 and SEQ ID NO:8, and to a polynucleotide comprising a sequence that encodes a polypeptide comprising one 4-1BBL fragment comprising an amino acid sequence that is is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

In another aspect, the disclosure is directed to an isolated polynucleotide comprising a sequence that encodes a polypeptide comprising two 4-1BBL fragments comprising an amino acid sequence that is at least about 90%, 95%, 98% or 100% identical to an amino acid sequence shown in SEQ ID NO:9 or SEQ ID NO: 10, and to a polynucleotide comprising a sequence that encodes a polypeptide comprising one 4-1BBL fragment comprising an amino acid sequence that is is at least about 90%, 95%, 98% or 100% identical to an amino acid sequence shown in SEQ ID NO:1 or SEQ ID NO:5.

In further aspects, the disclosure relates to the polynucleotides comprising a sequence that is at least about 90%, 95%, 98% or 100% identical to the specific cDNA sequences disclosed herein. In a particular aspect, the invention relates to a polynucleotide comprising a sequence that is identical to one of the specific cDNA sequences disclosed herein.

In certain aspects, the polynucleotide or nucleic acid is DNA. In other embodiments, a polynucleotide of the present invention is RNA, for example, in the form of messenger RNA (mRNA). RNA of the present invention may be single stranded or double stranded.

## Recombinant Methods

4-1BBL trimer-containing antigen binding molecules of the invention may be obtained, for example, by solid-state peptide synthesis (e.g. Merrifield solid phase synthesis) or recombinant production. For recombinant production one or more polynucleotide encoding the 4-1BBL trimer-containing antigen binding molecule or polypeptide fragments thereof, e.g., as described above, is isolated and inserted into one or more vectors for further cloning and/or expression in a host cell. Such polynucleotide may be readily isolated and sequenced using conventional procedures. In one aspect of the invention, a vector, preferably an expression vector, comprising one or more of the polynucleotides of the invention is provided. Methods which are well known to those skilled in the art can be used to construct expression vectors containing the coding sequence of the 4-1BBL trimer-containing antigen binding molecule (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the TNF family ligand trimer-containing antigen binding molecule or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the 4-1BBL trimer-containing antigen binding molecule of the invention or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions may be associated with additional coding regions which encode secretory or signal peptides, which direct the secretion of a polypeptide encoded by a polynucleotide of the present invention. For example, if secretion of the 4-1BBL trimer-containing antigen binding molecule or polypeptide fragments thereof is desired, DNA encoding a signal sequence may be placed upstream of the nucleic acid encoding a TNF family ligand trimer-containing antigen binding molecule of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a 4-1BBL trimer-containing antigen binding molecule of the invention or polypeptide fragments thereof.

In a further aspect of the invention, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) a 4-1BBL trimer-containing antigen binding molecule of the invention of the invention. As used herein, the term "host cell" refers to any kind of cellular system which can be engineered to generate the fusion proteins of the invention or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In one aspect, a method of producing a 4-1BBL trimer-containing antigen binding molecule of the invention or polypeptide fragments thereof is provided, wherein the method comprises culturing a host cell comprising polynucleotides encoding the 4-1BBL trimer-containing antigen binding molecule or polypeptide fragments thereof, as provided herein, under conditions suitable for expression of the 4-1BBL trimer-containing antigen binding molecule or polypeptide fragments thereof, and recovering the 4-1BBL trimer-containing antigen binding molecule or polypeptide fragments thereof from the host cell (or host cell culture medium).

In the 4-1BBL trimer-containing antigen binding molecule, the components (a Fab domain capable of specific binding to a target cell antigen, one polypeptide comprising two ectodomains of 4-1BBL or fragments thereof and a polypeptide comprising one ectodomain of said 4-1BBL or a fragment thereof) are not genetically fused to each other. The polypeptides are designed such that its components (two ectodomains of 4-1BBL or fragments thereof and other components such as CH or CL) are fused to each other directly or through a linker sequence. The composition and length of the linker may be determined in accordance with methods well known in the art and may be tested for efficacy. Examples of linker sequences between different components of the antigen binding molecules of the invention are found in the sequences provided herein. Additional sequences may also be included to incorporate a cleavage site to separate the individual components of the fusion protein if desired, for example an endopeptidase recognition sequence.

In certain embodiments the moieties capable of specific binding to a target cell antigen (e.g. Fab fragments) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used in the invention. Non-limiting immunoglobulins useful in the present invention can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins according to the invention are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the Fab domains capable of specific binding to a target cell antigen (e.g. Fab fragments) comprised in the antigen binding molecules of the present invention are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules of the invention to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain embodiments, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch.14 (Cold Spring Harbor Laboratory, Cold Spring Harbor, NY).

4-1BBL trimer-containing antigen binding molecules of the invention prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the 4-1BBL trimer-containing antigen binding molecule binds. For example, for affinity chromatography purification of fusion proteins of the invention, a matrix with protein A or protein G may be used. Sequential Protein A or G affinity chromatography and size exclusion chromatography can be used to isolate an antigen binding molecule essentially as described in the Examples. The purity of the TNF ligand trimer-containing antigen binding molecule or fragments thereof can be determined by any of a variety of well-known analytical methods including gel electrophoresis, high pressure liquid chromatography, and the like. For example, the 4-1BBL trimer-containing antigen binding molecules expressed as described in the Examples were shown to be intact and properly assembled as demonstrated by reducing and non-reducing SDS-PAGE.

**Assays**

The antigen binding molecules provided herein may be identified, screened for, or characterized for their physical/chemical properties and/or biological activities by various assays known in the art.

**1. Affinity assays**

The affinity of the 4-1BBL trimer-containing antigen binding molecule provided herein for the corresponding TNF receptor can be determined in accordance with the methods set forth in the Examples by surface plasmon resonance (SPR), using standard instrumentation such as a BIAcore instrument (GE Healthcare), and receptors or target proteins such as may be obtained by recombinant expression. The affinity of the 4-1BBL trimer-containing antigen binding molecule for the target cell antigen can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 3. According to one aspect, $K_D$ is measured by surface plasmon resonance using a BIACORE® T100 machine (GE Healthcare) at 25 °C.

### 2. Binding assays and other assays

Binding of the 4-1BBL trimer-containing antigen binding molecule provided herein to the corresponding receptor expressing cells may be evaluated using cell lines expressing the particular receptor or target antigen, for example by flow cytometry (FACS). In one aspect, fresh peripheral blood mononuclear cells (PBMCs) expressing the TNF receptor are used in the binding assay (Example 6.1 and 7.1). These cells are used directly after isolation (naive PMBCs) or after stimulation (activated PMBCs). In another aspect, activated mouse splenocytes (expressing the TNF receptor molecule) were used to demonstrate the binding of the 4-1BBL trimer-containing antigen binding molecule to the corresponding TNF receptor expressing cells.

In a further aspect, cancer cell lines expressing the target cell antigen, for example FAP, were used to demonstrate the binding of the antigen binding molecules to the target cell antigen (Example 6.2). In another aspect, cancer cell lines expressing CD19 were used to demonstrate the binding of the antigen binding molecules to CD19 (Example 7.2).

In another aspect, competition assays may be used to identify an antigen binding molecule that competes with a specific antibody or antigen binding molecule for binding to the target or TNF receptor, respectively. In certain embodiments, such a competing antigen binding molecule binds to the same epitope (e.g., a linear or a conformational epitope) that is bound by a specific anti-target antibody or a specific anti-TNF receptor antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

### 3. Activity assays

In one aspect, assays are provided for identifying 4-1BBL trimer-containing antigen binding molecules that bind to a specific target cell antigen and to a specific TNF receptor having biological activity. Biological activity may include, e.g., agonistic signalling through the TNF receptor on cells expressing the target cell antigen. 4-1BBL trimer-containing antigen binding molecules identified by the assays as having such biological activity in vitro are also provided.

In certain aspects, a 4-1BBL trimer-containing antigen binding molecule of the invention is tested for such biological activity. Assays for detecting the biological activity of the molecules of the invention are those described in Examples 6 and 7. Furthermore, assays for detecting cell lysis (e.g. by measurement of LDH release), induced apoptosis kinetics (e.g. by measurement of Caspase 3/7 activity) or apoptosis (e.g. using the TUNEL assay) are well known in the art. In addition the biological activity of such complexes can be assessed by evaluating their effects on survival, proliferation and lymphokine secretion of various lymphocyte subsets such as NK cells, NKT-cells or $\gamma\delta$ T-cells or assessing their capacity to modulate phenotype and function of antigen presenting cells such as dendritic cells, monocytes/macrophages or B-cells.

### Pharmaceutical Compositions, Formulations and Routes of Administation

In a further aspect, the invention provides pharmaceutical compositions comprising any of the 4-1BBL trimer-containing antigen binding molecules provided herein, e.g., for use in any of the below therapeutic methods. In one embodiment, a pharmaceutical composition comprises any of the 4-1BBL trimer-containing antigen binding molecules provided herein and at least one pharmaceutically acceptable excipient. In another embodiment, a pharmaceutical composition comprises any of the 4-1BBL trimer-containing antigen binding molecules provided herein and at least one additional therapeutic agent, e.g., as described below.

Pharmaceutical compositions of the present invention comprise a therapeutically effective amount of one or more 4-1BBL trimer-containing antigen *binding* molecules dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one 4-1BBL trimer-containing antigen binding molecule and optionally an additional active ingredient will be known to those of skill in the art in light of the present disclosure, as exemplified by Remington's Pharmaceutical Sciences, 18th Ed. Mack Printing Company, 1990, incorporated herein by reference. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the 4-1BBL trimer-containing antigen binding molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the fusion proteins may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the fusion proteins of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The 4-1BBL trimer-containing antigen binding molecules may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

**Therapeutic methods and compositions**

Any of the 4-1BBL trimer-containing antigen binding molecules provided herein may be used in therapeutic methods.

For use in therapeutic methods, 4-1BBL trimer-containing antigen binding molecules can be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, 4-1BBL trimer-containing antigen binding molecules of the invention for use as a medicament are provided. In further aspects, 4-1BBL trimer-containing antigen binding molecules of the invention for use in treating a disease, in particular for use in the treatment of cancer, are provided. In certain aspects, 4-1BBL trimer-containing antigen binding molecules of the invention for use in a method of treatment are provided. In one aspect, the invention provides a 4-1BBL trimer-containing antigen binding molecule as described herein for use in the treatment of a disease in an individual in need thereof. In certain aspects, the invention provides a 4-1BBL trimer-containing antigen binding molecule for use in a method of treating an individual having a disease comprising administering to the individual a therapeutically effective amount of the fusion protein. In certain aspects, the disease to be treated is cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia. Thus, a 4-1BBL trimer-containing antigen binding molecule as described herein for use in the treatment of cancer is provided. The subject, patient, or "individual" in need of treatment is typically a mammal, more specifically a human.

In another aspect, provided is a 4-1BBL trimer-containing antigen binding molecule as described herein for use in the treatment of infectious diseases, in particular for the treatment of viral infections. In a further aspect, provided is a 4-1BBL trimer-containing antigen binding molecule as described herein for use in the treatment of autoimmune diseases such as for example Lupus disease.

In one aspect, provided is a 4-1BBL trimer-containing antigen binding molecule for use in treating head and neck squamous cell carcinoma (HNSCC), breast cancer, colorectal cancer (CRC), pancreatic cancer (PAC), gastric cancer, non-small-cell lung carcinoma (NSCLC) and Mesothelioma, wherein the target cell antigen is FAP.

In a further aspect, the disclosure relates to the use of a 4-1BBL trimer-containing antigen binding molecule in the manufacture or preparation of a medicament for the treatment of a disease in an individual in need thereof. In one aspect, the medicament is for use in a method of treating a disease comprising administering to an individual having the disease a therapeutically effective amount of the medicament. In certain embodiments the disease to be treated is a proliferative disorder, particularly cancer. Thus, in one aspect, the disclosure relates to the use of a 4-1BBL trimer-containing antigen binding molecule in the manufacture or preparation of a medicament for the treatment of cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia.. Other cell proliferation disorders that can be treated using a 4-1BBL trimer-containing antigen binding molecule include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan may recognize that in some cases the 4-1BBL trimer-containing antigen binding molecule may not provide a cure but may only provide partial benefit. In some aspects, a physiological change having some benefit is also considered therapeutically beneficial. Thus, in some aspects, an amount of 4-1BBL trimer-containing antigen binding molecule that provides a physiological change is considered an "effective amount" or a "therapeutically effective amount".

In a further aspect, the disclosure relates to the use of a 4-1BBL trimer-containing antigen binding molecule as described herein in the manufacture or preparation of a medicament for the treatment of infectious diseases, in particular for the treatment of viral infections or for the treatment of autoimmune diseases, for example Lupus disease.

In a further aspect, provided is a method for treating a disease in an individual, comprising administering to said individual a therapeutically effective amount of a 4-1BBL trimer-containing antigen binding molecule. In one aspect a composition is administered to said individual, comprising a fusion protein in a pharmaceutically acceptable form. In certain aspects, the disease to be treated is a proliferative disorder. In a particular aspect, the disease is cancer. In another aspect, the disease is an infectious disease or an autoimmune disease. In certain aspects, the method further comprises administering to the individual a therapeutically effective amount of at least one additional therapeutic agent, e.g. an anti-cancer agent if the disease to be treated is cancer. An "individual" according to any of the above embodiments may be a mammal, preferably a human.

For the prevention or treatment of disease, the appropriate dosage of a 4-1BBL trimer-containing antigen binding molecule (when used alone or in combination with one or more other additional therapeutic agents) will depend on the type of disease to be treated, the route of administration, the body weight of the patient, the type of fusion protein, the severity and course of the disease, whether the fusion protein is administered for preventive or therapeutic purposes, previous or concurrent therapeutic interventions, the patient's clinical history and response to the fusion protein, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The 4-1BBL trimer-containing antigen binding molecule is suitably administered to the patient at one time or over a series of treatments. Depending on the type and severity of the disease, about 1 μg/kg to 15 mg/kg (e.g. 0.1 mg/kg - 10 mg/kg) of 4-1BBL trimer-containing antigen binding molecule can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the fusion protein would be in the range from about 0.005 mg/kg to about 10 mg/kg. In other examples, a dose may also comprise from about 1 μg/kg body weight, about 5 μg/kg body weight, about 10 μg/kg body weight, about 50 μg/kg body weight, about 100 μg/kg body weight, about 200 μg/kg body weight, about 350 μg/kg body weight, about 500 μg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg body weight or more per administration, and any range derivable therein. In examples of a derivable range from the numbers listed herein, a range of about 5 mg/kg body weight to about 100 mg/kg body weight, about 5 μg/kg body weight to about 500 mg/kg body weight etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the fusion protein). An initial higher loading dose, followed by one or more lower doses may be administered. However, other dosage regimens may be useful. The progress of this therapy is easily monitored by conventional techniques and assays.

The 4-1BBL trimer-containing antigen binding molecules will generally be used in an amount effective to achieve the intended purpose. For use to treat or prevent a disease condition, the 4-1BBL trimer-containing antigen binding molecules, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from in vitro assays, such as cell culture assays. A dose can then be formulated in animal models to achieve a circulating concentration range that includes the $IC_{50}$ as determined in cell culture. Such information can be used to more accurately determine useful doses in humans.

Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the 4-1BBL trimer-containing antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the 4-1BBL trimer-containing antigen binding molecule may not be related to plasma concentration. One skilled in the art will be able to optimize therapeutically effective local dosages without undue experimentation.

A therapeutically effective dose of the 4-1BBL trimer-containing antigen binding molecules described herein will generally provide therapeutic benefit without causing substantial toxicity. Toxicity and therapeutic efficacy of a fusion protein can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. 4-1BBL trimer-containing antigen binding molecules that exhibit large therapeutic indices are preferred. In one aspect, the 4-1BBL trimer-containing antigen binding molecule according to the present invention exhibits a high therapeutic index. The data obtained from cell culture assays and animal studies can be used in formulating a range of dosages suitable for use in humans. The dosage lies preferably within a range of circulating concentrations that include the $ED_{50}$ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1, incorporated herein by reference in its entirety).

The attending physician for patients treated with fusion proteins of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

**Other agents and treatments**

The 4-1BBL trimer-containing antigen binding molecules may be administered in combination with one or more other agents in therapy. For instance, a fusion protein may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain aspects, an additional therapeutic agent is another anti-cancer agent or immunotherapy.

Such other agents are suitably present in combination in amounts that are effective for the purpose intended. The effective amount of such other agents depends on the amount of fusion protein used, the type of disorder or treatment, and other factors discussed above. The TNF family ligand trimer-containing antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the 4-1BBL trimer-containing antigen binding molecule can occur prior to, simultaneously, and/or following, administration of the additional therapeutic agent and/or adjuvant.

**Articles of Manufacture**

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is a 4-1BBL trimer-containing antigen binding molecule.

The label or package insert indicates that the composition is used for treating the condition of choice. Moreover, the article of manufacture may comprise (a) a first container with a composition contained therein, wherein the composition comprises a 4-1BBL trimer-containing antigen binding molecule; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQID NO: | Name | Sequence |
|---|---|---|
| 1 | Human (hu) 4-1BBL (71-254) | REGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAG AAALALTVDLPPASSEARNSAFGFQGRLLHLSAGQ RLGVHLHTEARARHAWQLTQGATVLGLFRVTPEIP AGLPSPRSE |
| 2 | hu 4-1BBL (85-254) | LDLRQGMFAQLVAQNVLLIDGPLSWYSDPGLAGVS LTGGLSYKEDTKELVVAKAGVYYVFFQLELRRVV AGEGSGSVSLALHLQPLRSAAGAAALALTVDLPPA SSEARNSAFGFQGRLLHLSAGQRLGVHLHTEARAR HAWQLTQGATVLGLFRVTPEIPAGLPSPRSE |
| 3 | hu 4-1BBL (80-254) | DPAGLLDLRQGMFAQLVAQNVLLIDGPLSWYSDPG LAGVSLTGGLSYKEDTKELVVAKAGVYYVFFQLEL RRVVAGEGSGSVSLALHLQPLRSAAGAAALALTVD LPPASSEARNSAFGFQGRLLHLSAGQRLGVHLHTEA RARHAWQLTQGATVLGLFRVTPEIPAGLPSPRSE |
| 4 | hu 4-1BBL (52-254) | PWAVSGARASPGSAASPRLREGPELSPDDPAGLLDL RQGMFAQLVAQNVLLIDGPLSWYSDPGLAGVSLTG GLSYKEDTKELVVAKAGVYYVFFQLELRRVVAGE GSGSVSLALHLQPLRSAAGAAALALTVDLPPASSEA RNSAFGFQGRLLHLSAGQRLGVHLHTEARARHAW QLTQGATVLGLFRVTPEIPAGLPSPRSE |
| 5 | hu 4-1BBL (71-248) | REGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAG AAALALTVDLPPASSEARNSAFGFQGRLLHLSAGQ RLGVHLHTEARARHAWQLTQGATVLGLFRVTPEIP AGL |
| 6 | hu 4-1BBL (85-248) | LDLRQGMFAQLVAQNVLLIDGPLSWYSDPGLAGVS LTGGLSYKEDTKELVVAKAGVYYVFFQLELRRVV AGEGSGSVSLALHLQPLRSAAGAAALALTVDLPPA SSEARNSAFGFQGRLLHLSAGQRLGVHLHTEARAR HAWQLTQGATVLGLFRVTPEIPAGL |
| 7 | hu 4-1BBL (80-248) | DPAGLLDLRQGMFAQLVAQNVLLIDGPLSWYSDPG LAGVSLTGGLSYKEDTKELVVAKAGVYYVFFQLEL RRVVAGEGSGSVSLALHLQPLRSAAGAAALALTVD LPPASSEARNSAFGFQGRLLHLSAGQRLGVHLHTEA RARHAWQLTQGATVLGLFRVTPEIPAGL |

(continued)

| SEQID NO: | Name | Sequence |
|---|---|---|
| 8 | hu 4-1BBL (52-248) | PWAVSGARASPGSAASPRLREGPELSPDDPAGLLDL RQGMFAQLVAQNVLLIDGPLSWYSDPGLAGVSLTG GLSYKEDTKELVVAKAGVYYVFFQLELRRVVAGE GSGSVSLALHLQPLRSAAGAAALALTVDLPPASSEA RNSAFGFQGRLLHLSAGQRLGVHLHTEARARHAW QLTQGATVLGLFRVTPEIPAGL |
| 9 | dimeric hu 4-1BBL (71-254) connected by $(G4S)_2$ linker | REGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAG AAALALTVDLPPASSEARNSAFGFQGRLLHLSAGQ RLGVHLHTEARARHAWQLTQGATVLGLFRVTPEIP AGLPSPRSEGGGGSGGGGSREGPELSPDDPAGLLDL RQGMFAQLVAQNVLLIDGPLSWYSDPGLAGVSLTG GLSYKEDTKELVVAKAGVYYVFFQLELRRVVAGE GSGSVSLALHLQPLRSAAGAAALALTVDLPPASSEA RNSAFGFQGRLLHLSAGQRLGVHLHTEARARHAW QLTQGATVLGLFRVTPEIPAGLPSPRSE |
| 10 | dimeric hu 4-1BBL (71-248) connected by $(G4S)_2$ linker | REGPELSPDDPAGLLDLRQGMFAQLVAQNVLLIDG PLSWYSDPGLAGVSLTGGLSYKEDTKELVVAKAGV YYVFFQLELRRVVAGEGSGSVSLALHLQPLRSAAG AAALALTVDLPPASSEARNSAFGFQGRLLHLSAGQ RLGVHLHTEARARHAWQLTQGATVLGLFRVTPEIP AGLGGGGSGGGGSREGPELSPDDPAGLLDLRQGMF AQLVAQNVLLIDGPLSWYSDPGLAGVSLTGGLSYK EDTKELVVAKAGVYYVFFQLELRRVVAGEGSGSVS LALHLQPLRSAAGAAALALTVDLPPASSEARNSAF GFQGRLLHLSAGQRLGVHLHTEARARHAWQLTQG ATVLGLFRVTPEIPAGL |
| 11 | hu OX40L (51-183) | QVSHRYPRIQSIKVQFTEYKKEKGFILTSQKEDEIMK VQNNSVIINCDGFYLISLKGYFSQEVNISLHYQKDEE PLFQLKKVRSVNSLMVASLTYKDKVYLNVTTDNTS LDDFHVNGGELILIHQNPGEFCVL |
| 12 | hu OX40L (52-183) | VSHRYPRIQSIKVQFTEYKKEKGFILTSQKEDEIMKV QNNSVIINCDGFYLISLKGYFSQEVNISLHYQKDEEP LFQLKKVRSVNSLMVASLTYKDKVYLNVTTDNTSL DDFHVNGGELILIHQNPGEFCVL |
| 13 | FAP(28H1) CDR-H1 | SHAMS |
| 14 | FAP(28H1) CDR-H2 | AIWASGEQYYADSVKG |
| 15 | FAP(28H1) CDR-H3 | GWLGNFDY |
| 16 | FAP(28H1) CDR-L1 | RASQSVSRSYLA |
| 17 | FAP(28H1) CDR-L2 | GASTRAT |
| 18 | FAP(28H1) CDR-L3 | QQGQVIPPT |
| 19 | FAP(4B9) CDR-H1 | SYAMS |
| 20 | FAP(4B9) CDR-H2 | AIIGSGASTYYADSVKG |

(continued)

| SEQID NO: | Name | Sequence |
|---|---|---|
| 21 | FAP(4B9) CDR-H3 | GWFGGFNY |
| 22 | FAP(4B9) CDR-L1 | RASQSVTSSYLA |
| 23 | FAP(4B9) CDR-L2 | VGSRRAT |
| 24 | FAP(4B9) CDR-L3 | QQGIMLPPT |
| 25 | FAP(28H1) VH | EVQLLESGGGLVQPGGSLRLSCAASGFTFSSHAMS WVRQAPGKGLEWVSAIWASGEQYYADSVKGRFTI SRDNSKNTLYLQMNSLRAEDTAVYYCAKGWLGNF DYWGQGTLVTVSS |
| 26 | FAP(28H1) VL | EIVLTQSPGTLSLSPGERATLSCRASQSVSRSYLAW YQQKPGQAPRLLIIGASTRATGIPDRFSGSGSGTDFT LTISRLEPEDFAVYYCQQGQVIPPTFGQGTKVEIK |
| 27 | FAP(4B9) VH | EVQLLESGGGLVQPGGSLRLSCAASGFTFSSYAMS WVRQAPGKGLEWVSAIIGSGASTYYADSVKGRFTI SRDNSKNTLYLQMNSLRAEDTAVYYCAKGWFGGF NYWGQGTLVTVSS |
| 28 | FAP(4B9) VL | EIVLTQSPGTLSLSPGERATLSCRASQSVTSSYLAWY QQKPGQAPRLLINVGSRRATGIPDRFSGSGSGTDFT LTISRLEPEDFAVYYCQQGIMLPPTFGQGTKVEIK |
| 29 | CD19 (8B8-018) CDR-H1 | DYIMH |
| 30 | CD19 (8B8-018) CDR-H2 | YINPYNDGSKYTEKFQG |
| 31 | CD19 (8B8-018) CDR-H3 | GTYYYGSALFDY |
| 32 | CD19 (8B8-018) CDR-L1 | KSSQSLENPNGNTYLN |
| 33 | CD19 (8B8-018) CDR-L2 | RVSKRFS |
| 34 | CD19 (8B8-018) CDR-L3 | LQLTHVPYT |
| 35 | CD19 (8B8-2B11) CDR-H1 | DYIMH |
| 36 | CD19 (8B8-2B11) CDR-H2 | YINPYNDGSKYTEKFQG |
| 37 | CD19 (8B8-2B11) CDR-H3 | GTYYYGPQLFDY |
| 38 | CD19 (8B8-2B11) CDR-L1 | KSSQSLETSTGTTYLN |
| 39 | CD19 (8B8-2B11) CDR-L2 | RVSKRFS |
| 40 | CD19 (8B8-2B11) CDR-H3 | LQLLEDPYT |
| 41 | CD19 (8B8-018) VH | QVQLVQSGAEVKKPGASVKVSCKASGYTFTDYIM HWVRQAPGQGLEWMGYINPYNDGSKYTEKFQGR VTMTSDTSISTAYMELSRLRSDDTAVYYCARGTYY YGSALFDYWGQGTTVTVSS |
| 42 | CD19 (8B8-018) VL | DIVMTQTPLSLSVTPGQPASISCKSSQSLENPNGNTY LNWYLQKPGQSPQLLIYRVSKRFSGVPDRFSGSGSG TDFTLKISRVEAEDVGVYYCLQLTHVPYTFGQGTK LEIK |

(continued)

| SEQID NO: | Name | Sequence |
|---|---|---|
| 43 | CD19 (8B8-2B11) VH | QVQLVQSGAEVKKPGASVKVSCKASGYTFTDYIMHWVRQAPGQGLEWMGYINPYNDGSKYTEKFQGRVTMTSDTSISTAYMELSRLRSDDTAVYYCARGTYYYGPQLFDYWGQGTTVTVSS |
| 44 | CD19 (8B8-2B11) VL | DIVMTQTPLSLSVTPGQPASISCKSSQSLETSTGTTYLNWYLQKPGQSPQLLIYRVSKRFSGVPDRFSGSGSGTDFTLKISRVEAEDVGVYYCLQLLEDPYTFGQGTKLEIK |
| 45 | CEA CDR-H1 | DTYMH |
| 46 | CEA CDR-H2 | RIDPANGNSKYVPKFQG |
| 47 | CEA CDR-H3 | FGYYVSDYAMAY |
| 48 | CEA CDR-L1 | RAGESVDIFGVGFLH |
| 49 | CEA CDR-L2 | RASNRAT |
| 50 | CEA CDR-L3 | QQTNEDPYT |
| 51 | Humanized CEA binder VH | QVQLVQSGAEVKKPGSSVKVSCKASGFNIKDTYMHWVRQAPGQGLEWMGRIDPANGNSKYVPKFQGRVTITADTSTSTAYMELSSLRSEDTAVYYCAPFGYYVSDYAMAYWGQGTLVTVSS |
| 52 | Humanized CEA binder VL | EIVLTQSPATLSLSPGERATLSCRAGESVDIFGVGFLHWYQQKPGQAPRLLIYRASNRATGIPARFSGSGSGTDFTLTISSLEPEDFAVYYCQQTNEDPYTFGQGTKLEIK |
| 53 | Human (hu) FAP | UniProt no. Q12884 |
| 54 | hu FAP ectodomain+poly-lys-tag+his$_6$-tag | RPSRVHNSEENTMRALTLKDILNGTFSYKTFFPNWISGQEYLHQSADNNIVLYNIETGQSYTILSNRTMKSVNASNYGLSPDRQFVYLESDYSKLWRYSYTATYYIYDLSNGEFVRGNELPRPIQYLCWSPVGSKLAYVYQNNIYLKQRPGDPPFQITFNGRENKIFNGIPDWVYEEEMLATKYALWWSPNGKFLAYAEFNDTDIPVIAYSYYGDEQYPRTINIPYPKAGAKNPVVRIFIIDTTYPAYVGPQEVPVPAMIASSDYYFSWLTWVTDERVCLQWLKRVQNVSVLSICDFREDWQTWDCPKTQEHIEESRTGWAGGFFVSTPVFSYDAISYYKIFSDKDGYKHIHYIKDTVENAIQITSGKWEAINIFRVTQDSLFYSSNEFEEYPGRRNIYRISIGSYPPSKKCVTCHLRKERCQYYTASFSDYAKYYALVCYGPGIPISTLHDGRTDQEIKILEENKELENALKNIQLPKEEIKKLEVDEITLWYKMILPPQFDRSKKYPLLIQVYGGPCSQSVRSVFAVNWISYLASKEGMVIALVDGRGTAFQGDKLLYAVYRKLGVYEVEDQITAVRKFIEMGFIDEKRIAIWGWSYGGYVSSLALASGTGLFKCGIAVAPVSSWEYYASVYTERFMGLPTKDDNLEHYKNSTVMARAEYFRNVDYLLIHGTADDNVHFQNSAQIAKALVNAQVDFQAMWYSDQNHGLSGLSTNHLYTHMTHFLKQCFSLSDGKKKKKKGHHHH |

(continued)

| SEQID NO: | Name | Sequence |
| --- | --- | --- |
| | | HH |

(continued)

| SEQID NO: | Name | Sequence |
|---|---|---|
| 55 | nucleotide sequence of hu FAP ectodomain+poly-lys-tag+his$_6$-tag | CGCCCTTCAAGAGTTCATAACTCTGAAGAAAATA CAATGAGAGCACTCACACTGAAGGATATTTTAAA TG GAACATTTTCTTATAAAACATTTTTCCAAACTGG ATTTCAGGACAAGAATATCTTCATCAATCTGCAG ATAACAATATAGTACTTTATAATATTGAAACAGG ACAATCATATACCATTTTGAGTAATAGAACCATG AAAAGTGTGAATGCTTCAAATTACGGCTTATCAC CTGATCGGCAATTTGTATATCTAGAAAGTGATTA TTCAAAGCTTTGGAGATACTCTTACACAGCAACA TATTACATCTATGACCTTAGCAATGGAGAATTTG TAAGAGGAAATGAGCTTCCTCGTCCAATTCAGTA TTTATGCTGGTCGCCTGTTGGGAGTAAATTAGCA TATGTCTATCAAAACAATATCTATTTGAAACAAA GACCAGGAGATCCACCTTTTCAAATAACATTTAA TGGAAGAGAAAATAAAATATTTAATGGAATCCCA GACTGGGTTTATGAAGAGGAAATGCTTGCTACAA AATATGCTCTCTGGTGGTCTCCTAATGGAAAATTT TTGGCATATGCGGAATTTAATGATACGGATATAC CAGTTATTGCCTATTCCTATTATGGCGATGAACA ATATCCTAGAACAATAAATATTCCATACCCAAAG GCTGGAGCTAAGAATCCCGTTGTTCGGATATTTA TTATCGATACCACTTACCCTGCGTATGTAGGTCCC CAGGAAGTGCCTGTTCCAGCAATGATAGCCTCAA GTGATTATTATTTCAGTTGGCTCACGTGGGTTACT GATGAACGAGTATGTTTGCAGTGGCTAAAAAGAG TCCAGAATGTTTCGGTCCTGTCTATATGTGACTTC AGGGAAGACTGGCAGACATGGGATTGTCCAAAG ACCCAGGAGCATATAGAAGAAAGCAGAACTGGA TGGGCTGGTGGATTCTTTGTTTCAACACCAGTTTT CAGCTATGATGCCATTTCGTACTACAAAATATTT AGTGACAAGGATGGCTACAAACATATTCACTATA TCAAAGACACTGTGGAAAATGCTATTCAAATTAC AAGTGGCAAGTGGGAGGCCATAAATATATTCAG AGTAACACAGGATTCACTGTTTTATTCTAGCAAT GAATTTGAAGAATACCCTGGAAGAAGAAACATCT ACAGAATTAGCATTGGAAGCTATCCTCCAAGCAA GAAGTGTGTTACTTGCCATCTAAGGAAAGAAAGG TGCCAATATTACACAGCAAGTTTCAGCGACTACG CCAAGTACTATGCACTTGTCTGCTACGGCCCAGG CATCCCCATTTCCACCCTTCATGATGGACGCACTG ATCAAGAAATTAAAATCCTGGAAGAAAACAAGG AATTGGAAAATGCTTTGAAAAATATCCAGCTGCC TAAAGAGGAAATTAAGAAACTTGAAGTAGATGA AATTACTTTATGGTACAAGATGATTCTTCCTCCTC AATTTGACAGATCAAAGAAGTATCCCTTGCTAAT TCAAGTGTATGGTGGTCCCTGCAGTCAGAGTGTA AGGTCTGTATTTGCTGTTAATTGGATATCTTATCT TGCAAGTAAGGAAGGGATGGTCATTGCCTTGGTG GATGGTCGAGGAACAGCTTTCCAAGGTGACAAAC TCCTCTATGCAGTGTATCGAAAGCTGGGTGTTTAT |

(continued)

| SEQID NO: | Name | Sequence |
|---|---|---|
| | | GAAGTTGAAGACCAGATTACAGCTGTCAGAAAAT TCATAGAAATGGGTTTCATTGATGAAAAAAGAAT AGCCATATGGGGCTGGTCCTATGGAGGATACGTT TCATCACTGGCCCTTGCATCTGGAACTGGTCTTTT CAAATGTGGTATAGCAGTGGCTCCAGTCTCCAGC TGGGAATATTACGCGTCTGTCTACACAGAGAGAT TCATGGGTCTCCCAACAAAGGATGATAATCTTGA GCACTATAAGAATTCAACTGTGATGGCAAGAGCA GAATATTTCAGAAATGTAGACTATCTTCTCATCC ACGGAACAGCAGATGATAATGTGCACTTTCAAAA CTCAGCACAGATTGCTAAAGCTCTGGTTAATGCA CAAGTGGATTTCCAGGCAATGTGGTACTCTGACC AGAACCACGGCTTATCCGGCCTGTCCACGAACCA CTTATACACCCACATGACCCACTTCCTAAAGCAG TGTTTCTCTTTGTCAGACGGCAAAAAGAAAAAGA AAAAGGGCCACCACCATCACCATCAC |
| 56 | mouse FAP | UniProt no. P97321 |
| 57 | Murine FAP ectodomain+poly-lys-tag+his$_6$-tag | RPSRVYKPEGNTKRALTLKDILNGTFSYKTYFPNWI SEQEYLHQSEDDNIVFYNIETRESYIILSNSTMKSVN ATDYGLSPDRQFVYLESDYSKLWRYSYTATYYIYD LQNGEFVRGYELPRPIQYLCWSPVGSKLAYVYQNN IYLKQRPGDPPFQITYTGRENRIFNGIPDWVYEEEML ATKYALWWSPDGKFLAYVEFNDSDIPIIAYSYYGD GQYPRTINIPYPKAGAKNPVVRVFIVDTTYPHHVGP MEVPVPEMIASSDYYFSWLTWVSSERVCLQWLKR VQNVSVLSICDFREDWHAWECPKNQEHVEESRTG WAGGFFVSTPAFSQDATSYYKIFSDKDGYKHIHYIK DTVENAIQITSGKWEAIYIFRVTQDSLFYSSNEFEGY PGRRNIYRISIGNSPPSKKCVTCHLRKERCQYYTASF SYKAKYYALVCYGPGLPISTLHDGRTDQEIQVLEEN KELENSLRNIQLPKVEIKKLKDGGLTFWYKMILPPQ FDRSKKYPLLIQVYGGPCSQSVKSVFAVNWITYLAS KEGIVIALVDGRGTAFQGDKFLHAVYRKLGVYEVE DQLTAVRKFIEMGFIDEERIAIWGWSYGGYVSSLAL ASGTGLFKCGIAVAPVSSWEYYASIYSERFMGLPTK DDNLEHYKNSTVMARAEYFRNVDYLLIHGTADDN VHFQNSAQIAKALVNAQVDFQAMWYSDQNHGILS GRSQNHLYTHMTHFLKQCFSLSDGKKKKKGHHH HHH |

(continued)

| SEQID NO: | Name | Sequence |
|---|---|---|
| 58 | nucleotide sequence of murine FAP ectodomain+poly-lys-tag+$his_6$-tag | CGTCCCTCAAGAGTTTACAAACCTGAAGGAAACA CAAAGAGAGCTCTTACCTTGAAGGATATTTTAAA TGGAACATTCTCATATAAAACATATTTTCCCAACT GGATTTCAGAACAAGAATATCTTCATCAATCTGA GGATGATAACATAGTATTTTATAATATTGAAACA AGAGAATCATATATCATTTTGAGTAATAGCACCA TGAAAAGTGTGAATGCTACAGATTATGGTTTGTC ACCTGATCGGCAATTTGTGTATCTAGAAAGTGAT TATTCAAAGCTCTGGCGATATTCATACACAGCGA CATACTACATCTACGACCTTCAGAATGGGGAATT TGTAAGAGGATACGAGCTCCCTCGTCCAATTCAG TATCTATGCTGGTCGCCTGTTGGGAGTAAATTAG CATATGTATATCAAAACAATATTTATTTGAAACA |

(continued)

| SEQID NO: | Name | Sequence |
|---|---|---|
| | | AAGACCAGGAGATCCACCTTTTCAAATAACTTAT ACTGGAAGAGAAAATAGAATATTTAATGGAATA CCAGACTGGGTTTATGAAGAGGAAATGCTTGCCA CAAAATATGCTCTTTGGTGGTCTCCAGATGGAAA ATTTTTGGCATATGTAGAATTTAATGATTCAGATA TACCAATTATTGCCTATTCTTATTATGGTGATGGA CAGTATCCTAGAACTATAAATATTCCATATCCAA AGGCTGGGGCTAAGAATCCGGTTGTTCGTGTTTT TATTGTTGACACCACCTACCCTCACCACGTGGGC CCAATGGAAGTGCCAGTTCCAGAAATGATAGCCT CAAGTGACTATTATTTCAGCTGGCTCACATGGGT GTCCAGTGAACGAGTATGCTTGCAGTGGCTAAAA AGAGTGCAGAATGTCTCAGTCCTGTCTATATGTG ATTTCAGGGAAGACTGGCATGCATGGGAATGTCC AAAGAACCAGGAGCATGTAGAAGAAAGCAGAAC AGGATGGGCTGGTGGATTCTTTGTTTCGACACCA GCTTTTAGCCAGGATGCCACTTCTTACTACAAAA TATTTAGCGACAAGGATGGTTACAAACATATTCA CTACATCAAAGACACTGTGGAAAATGCTATTCAA ATTACAAGTGGCAAGTGGGAGGCCATATATATAT TCCGCGTAACACAGGATTCACTGTTTTATTCTAGC AATGAATTTGAAGGTTACCCTGGAAGAAGAAAC ATCTACAGAATTAGCATTGGAAACTCTCCTCCGA GCAAGAAGTGTGTTACTTGCCATCTAAGGAAAGA AAGGTGCCAATATTACACAGCAAGTTTCAGCTAC AAAGCCAAGTACTATGCACTCGTCTGCTATGGCC CTGGCCTCCCCATTTCCACCCTCCATGATGGCCGC ACAGACCAAGAAATACAAGTATTAGAAGAAAAC AAAGAACTGGAAAATTCTCTGAGAAATATCCAGC TGCCTAAAGTGGAGATTAAGAAGCTCAAAGACG GGGGACTGACTTTCTGGTACAAGATGATTCTGCC TCCTCAGTTTGACAGATCAAAGAAGTACCCTTTG CTAATTCAAGTGTATGGTGGTCCTTGTAGCCAGA GTGTTAAGTCTGTGTTTGCTGTTAATTGGATAACT TATCTCGCAAGTAAGGAGGGGATAGTCATTGCCC TGGTAGATGGTCGGGGCACTGCTTTCCAAGGTGA CAAATTCCTGCATGCCGTGTATCGAAAACTGGGT GTATATGAAGTTGAGGACCAGCTCACAGCTGTCA GAAAATTCATAGAAATGGGTTTCATTGATGAAGA AAGAATAGCCATATGGGGCTGGTCCTACGGAGGT TATGTTTCATCCCTGGCCCTTGCATCTGGAACTGG TCTTTTCAAATGTGGCATAGCAGTGGCTCCAGTCT CCAGCTGGGAATATTACGCATCTATCTACTCAGA GAGATTCATGGGCCTCCCAACAAAGGACGACAAT CTCGAACACTATAAAAATTCAACTGTGATGGCAA GAGCAGAATATTTCAGAAATGTAGACTATCTTCT CATCCACGGAACAGCAGATGATAATGTGCACTTT CAGAACTCAGCACAGATTGCTAAAGCTTTGGTTA ATGCACAAGTGGATTTCCAGGCGATGTGGTACTC TGACCAGAACCATGGTATATTATCTGGGCGCTCC CAGAATCATTTATATACCCACATGACGCACTTCC TCAAGCAATGCTTTTCTTTATCAGACGGCAAAAA |

(continued)

| SEQID NO: | Name | Sequence |
|---|---|---|
| | | GAAAAAGAAAAAGGGCCACCACCATCACCATCAC |
| 59 | Cynomolgus FAP ectodomain+poly-lys-tag+his$_6$-tag | RPPRVHNSEENTMRALTLKDILNGTFSYKTFFPNWISGQEYLHQSADNNIVLYNIETGQSYTILSNRTMKSVNASNYGLSPDRQFVYLESDYSKLWRYSYTATYYIYDLSNGEFVRGNELPRPIQYLCWSPVGSKLAYVYQNNIYLKQRPGDPPFQITFNGRENKIFNGIPDWVYEEEMLATKYALWWSPNGKFLAYAEFNDTDIPVIAYSYYGDEQYPRTINIPYPKAGAKNPFVRIFIIDTTYPAYVGPQEVPVPAMIASSDYYFSWLTWVTDERVCLQWLKRVQNVSVLSICDFREDWQTWDCPKTQEHIEESRTGWAGGFFVSTPVFSYDAISYYKIFSDKDGYKHIHYIKDTVENAIQITSGKWEAINIFRVTQDSLFYSSNEFEDYPGRRNIYRISIGSYPPSKKCVTCHLRKERCQYYTASFSDYAKYYALVCYGPGIPISTLHDGRTDQEIKILEENKELENALKNIQLPKEEIKKLEVDEITLWYKMILPPQFDRSKKYPLLIQVYGGPCSQSVRSVFAVNWISYLASKEGMVIALVDGRGTAFQGDKLLYAVYRKLGVYEVEDQITAVRKFIEMGFIDEKRIAIWGWSYGGYVSSLALASGTGLFKCGIAVAPVSSWEYYASVYTERFMGLPTKDDNLEHYKNSTVMARAEYFRNVDYLLIHGTADDNVHFQNSAQIAKALVNAQVDFQAMWYSDQNHGLSGLSTNHLYTHMTHFLKQCFSLSDGKKKKKKGHHHHHH |

(continued)

| SEQID NO: | Name | Sequence |
|---|---|---|
| 60 | nucleotide sequence of cynomolgus FAP ectodomain+poly-lys-tag+his$_6$-tag | CGCCCTCCAAGAGTTCATAACTCTGAAGAAAATA CAATGAGAGCACTCACACTGAAGGATATTTTAAA TGGGACATTTTCTTATAAAACATTTTTCCAAACT GGATTTCAGGACAAGAATATCTTCATCAATCTGC AGATAACAATATAGTACTTTATAATATTGAAACA GGACAATCATATACCATTTTGAGTAACAGAACCA TGAAAAGTGTGAATGCTTCAAATTATGGCTTATC ACCTGATCGGCAATTTGTATATCTAGAAAGTGAT TATTCAAAGCTTTGGAGATACTCTTACACAGCAA CATATTACATCTATGACCTTAGCAATGGAGAATT TGTAAGAGGAAATGAGCTTCCTCGTCCAATTCAG TATTTATGCTGGTCGCCTGTTGGGAGTAAATTAG CATATGTCTATCAAAACAATATCTATTTGAAACA AAGACCAGGAGATCCACCTTTTCAAATAACATTT AATGGAAGAGAAAATAAAATATTTAATGGAATC CCAGACTGGGTTTATGAAGAGGAAATGCTTGCTA CAAAATATGCTCTCTGGTGGTCTCCTAATGGAAA ATTTTTGGCATATGCGGAATTTAATGATACAGAT ATACCAGTTATTGCCTATTCCTATTATGGCGATGA ACAATATCCCAGAACAATAAATATTCCATACCCA AAGGCCGGAGCTAAGAATCCTTTTGTTCGGATAT TTATTATCGATACCACTTACCCTGCGTATGTAGGT CCCCAGGAAGTGCCTGTTCCAGCAATGATAGCCT CAAGTGATTATTATTTCAGTTGGCTCACGTGGGTT ACTGATGAACGAGTATGTTTGCAGTGGCTAAAAA GAGTCCAGAATGTTTCGGTCTTGTCTATATGTGAT TTCAGGGAAGACTGGCAGACATGGGATTGTCCAA AGACCCAGGAGCATATAGAAGAAAGCAGAACTG |

(continued)

| SEQID NO: | Name | Sequence |
|---|---|---|
| | | GATGGGCTGGTGGATTCTTTGTTTCAACACCAGTT TTCAGCTATGATGCCATTTCATACTACAAAATATT TAGTGACAAGGATGGCTACAAACATATTCACTAT ATCAAAGACACTGTGGAAAATGCTATTCAAATTA CAAGTGGCAAGTGGGAGGCCATAAATATATTCAG AGTAACACAGGATTCACTGTTTTATTCTAGCAAT GAATTTGAAGATTACCCTGGAAGAAGAAACATCT ACAGAATTAGCATTGGAAGCTATCCTCCAAGCAA GAAGTGTGTTACTTGCCATCTAAGGAAAGAAAGG TGCCAATATTACACAGCAAGTTTCAGCGACTACG CCAAGTACTATGCACTTGTCTGCTATGGCCCAGG CATCCCCATTTCCACCCTTCATGACGGACGCACT GATCAAGAAATTAAAATCCTGGAAGAAAACAAG GAATTGGAAAATGCTTTGAAAAATATCCAGCTGC CTAAAGAGGAAATTAAGAAACTTGAAGTAGATG AAATTACTTTATGGTACAAGATGATTCTTCCTCCT CAATTTGACAGATCAAAGAAGTATCCCTTGCTAA TTCAAGTGTATGGTGGTCCCTGCAGTCAGAGTGT AAGGTCTGTATTTGCTGTTAATTGGATATCTTATC TTGCAAGTAAGGAAGGGATGGTCATTGCCTTGGT GGATGGTCGGGGAACAGCTTTCCAAGGTGACAA ACTCCTGTATGCAGTGTATCGAAAGCTGGGTGTT TATGAAGTTGAAGACCAGATTACAGCTGTCAGAA AATTCATAGAAATGGGTTTCATTGATGAAAAAAG AATAGCCATATGGGGCTGGTCCTATGGAGGATAT GTTTCATCACTGGCCCTTGCATCTGGAACTGGTCT TTTCAAATGTGGGATAGCAGTGGCTCCAGTCTCC AGCTGGGAATATTACGCGTCTGTCTACACAGAGA GATTCATGGGTCTCCCAACAAAGGATGATAATCT TGAGCACTATAAGAATTCAACTGTGATGGCAAGA GCAGAATATTTCAGAAATGTAGACTATCTTCTCA TCCACGGAACAGCAGATGATAATGTGCACTTTCA AAACTCAGCACAGATTGCTAAAGCTCTGGTTAAT GCACAAGTGGATTTCCAGGCAATGTGGTACTCTG ACCAGAACCACGGCTTATCCGGCCTGTCCACGAA CCACTTATACACCCACATGACCCACTTCCTAAAG CAGTGTTTCTCTTTGTCAGACGGCAAAAAGAAAA AGAAAAAGGGCCACCACCATCACCATCAC |
| 61 | human CEA | UniProt no. P06731 |
| 62 | human MCSP | UniProt no. Q6UVK1 |
| 63 | human EGFR | UniProt no. P00533 |
| 64 | human CD19 | UniProt no. P15391 |
| 65 | human CD20 | Uniprot no. P11836 |
| 66 | human CD33 | UniProt no. P20138 |
| 67 | human Lymphotoxin α | UniProt no. P01374 |
| 68 | human TNF | UniProt no. P01375 |

(continued)

| SEQID NO: | Name | Sequence |
|---|---|---|
| 69 | human Lymphotoxin β | UniProt no. Q06643 |
| 70 | human OX40L | UniProt no. P23510 |
| 71 | human CD40L | UniProt no. P29965 |
| 72 | human FasL | UniProt no. P48023 |
| 73 | human CD27L | UniProt no. P32970 |
| 74 | human CD30L | UniProt no. P32971 |
| 75 | human 4-1BBL | UniProt no. P41273 |
| 76 | human TRAIL | UniProt no. P50591 |
| 77 | human RANKL | UniProt no. 014788 |
| 78 | human TWEAK | UniProt no. O43508 |
| 79 | human APRIL | UniProt no. O75888 |
| 80 | human BAFF | UniProt no. Q9Y275 |
| 81 | human LIGHT | UniProt no. 043557 |
| 82 | human TL1A | UniProt no. 095150 |
| 83 | human GITRL | UniProt no. Q9UNG2 |
| 84 | human ectodysplasin A | UniProt no. Q92838 |
| 85 | hu 4-1BBL (50-254) | ACPWAVSGARASPGSAASPRLREGPELSPDDPAGL LDLRQGMFAQLVAQNVLLIDGPLSWYSDPGLAGVS LTGGLSYKEDTKELVVAKAGVYYVFFQLELRRVV AGEGSGSVSLALHLQPLRSAAGAAALALTVDLPPA SSEARNSAFGFQGRLLHLSAGQRLGVHLHTEARAR HAWQLTQGATVLGLFRVTPEIPAGLPSPRSE |
| 86 | Peptide linker G4S | GGGGS |
| 87 | Peptide linker $(G4S)_2$ | GGGGSGGGGS |
| 88 | Peptide linker $(SG4)_2$ | SGGGGSGGGG |
| 89 | Peptide linker $(G_4S)_3$ | GGGGSGGGGSGGGGS |
| 90 | Peptide linker $G4(SG4)_2$ | GGGGSGGGGSGGGG |
| 91 | Peptide linker $(G_4S)_4$ | GGGGSGGGGSGGGGSGGGGS |
| 92 | Peptide linker | GSPGSSSSGS |
| 93 | Peptide linker | GSGSGSGS |
| 94 | Peptide linker | GSGSGNGS |
| 95 | Peptide linker | GGSGSGSG |
| 96 | Peptide linker | GGSGSG |
| 97 | Peptide linker | GGSG |
| 98 | Peptide linker | GGSGNGSG |
| 99 | Peptide linker | GGNGSGSG |
| 100 | Peptide linker | GGNGSG |

(continued)

| SEQID NO: | Name | Sequence |
|---|---|---|
| 101 | nucleotide sequence of Fc hole dimeric 4-1BBL (71-248) chain | see Table 1 |
| 102 | nucleotide sequence of anti-FAP (4B9) Fc knob monomeric 4-1BBL (71-248) | see Table 1 |
| 103 | nucleotide sequence of anti-FAP (4B9) light chain | see Table 1 |
| 104 | Fc hole dimeric 4-1BB (71-248) ligand chain | see Table 1 |
| 105 | anti-FAP(4B9) Fc knob monomeric 41-BBL (71-248) | see Table 1 |
| 106 | anti-FAP(4B9) light chain | see Table 1 |
| 107 | nucleotide sequence of Fc hole monomeric 4-1BBL (71-248) chain | see Table 2 |
| 108 | nucleotide sequence of anti-FAP (4B9) Fc knob dimeric 4-1BBL (71-248) | see Table 2 |
| 109 | Fc hole monomeric 4-1BB ligand (71-248) chain | see Table 2 |
| 110 | anti-FAP(4B9) Fc knob dimeric 4-1BBL (71-248) | see Table 2 |
| 111 | nucleotide sequence of anti-FAP (28H1) Fc knob monomeric 4-1BBL (71-248) | see Table 3 |
| 112 | nucleotide sequence of anti-FAP (28H1) light chain | see Table 3 |
| 113 | anti-FAP(28H1) Fc knob monomeric 4-1BBL (71-248) | see Table 3 |
| 114 | anti-FAP(28H1) light chain | see Table 3 |
| 115 | nucleotide sequence of anti-FAP (28H1) Fc knob dimeric 4-1BBL (71-248) | see Table 4 |
| 116 | anti-FAP(28H1) Fc knob dimeric 4-1BBL (71-248) | see Table 4 |
| 117 | nucleotide sequence of anti-CD19(8B8-018) Fc knob monomeric 4-1BBL (71-248) | see Table 5 |
| 118 | nucleotide sequence of anti-CD19(8B8-018) light chain | see Table 5 |
| 119 | anti-CD19(8B8-018) Fc knob monomeric 4-1-BBL (71-248) | see Table 5 |
| 120 | anti-CD19(8B8-018) light chain | see Table 5 |

(continued)

| SEQID NO: | Name | Sequence |
|---|---|---|
| 121 | nucleotide sequence of anti-CD19(8B8-018) Fc knob dimeric 4-1BBL (71-248) | see Table 6 |
| 122 | anti-CD19(8B8-018) Fc knob dimeric 4-1BB (71-248) | see Table 6 |
| 123 | nucleotide sequence of anti-CD19(8B8-2B11) Fc knob monomeric 4-1BBL (71-248) | see Table 7 |
| 124 | nucleotide sequence of anti-CD19(8B8-2B11) light chain | see Table 7 |
| 125 | anti- CD19(8B8-2B11) Fc knob monomeric 4-1BBL (71-248) | see Table 7 |
| 126 | anti- CD19(8B8-2B11) light chain | see Table 7 |
| 127 | nucleotide sequence of anti-CD19(8B8-2B11) Fc knob dimeric 4-1BBL (71-248) | see Table 8 |
| 128 | anti- CD19(8B8-2B11) Fc knob dimeric 4-1BB ligand (71-248) | see Table 8 |
| 129 | HVR-L1 of anti-CD19 8B8 | NSNGNT |
| 130 | HVR-L2 of anti-CD 19 8B8 | KFNG |
| 131 | Nucleotide sequence of Fc hole chain with HYRF mutation | see Table 11 |
| 132 | Nucleotide sequence of human CD19 antigen Fc knob chain avi tag | see Table 11 |
| 133 | Fc hole chain with HYRF mutation | see Table 11 |
| 134 | human CD19 antigen Fc knob chain avi tag | see Table 11 |
| 135 | Nucleotide sequence of cynomolgus CD19 antigen Fc knob chain avi tag | see Table 11 |
| 136 | cynomolgus CD19 antigen Fc knob chain avi tag | see Table 11 |
| 137 | Nucleotide sequence CD19 (8B8) VH Parental clone | CAAGTTCAATTGGTTCAATCTGGTGCTGAAGTAA<br>AAAAACCGGGCGCTTCCGTTAAAGTGAGCTGCAA<br>AGCATCTGGTTACACCTTCACTGACTATATCATGC<br>ACTGGGTTCGTCAGGCCCCGGGCCAGGGTCTGGA<br>GTGGATGGGCTACATTAACCCATACAACGACGGT<br>TCCAAATATACCGAGAAATTCCAGGGCCGCGTCA<br>CGATGACCAGCGACACTTCTATCTCCACCGCGTA<br>CATGGAACTGTCTAGACTGCGTTCTGACGACACC<br>GCTGTTTACTATTGTGCACGCGGTACTTACTACTA<br>CGGTTCCGCCCTCTTTGATTACTGGGGCCAAGGT<br>ACCACGGTGACCGTAAGCTCT |

(continued)

| SEQID NO: | Name | Sequence |
|---|---|---|
| 138 | Nucleotide sequence CD19 (8B8) VL Parental clone | GATATTGTTATGACTCAAACTCCACTGTCTCTGTC CGTGACCCCGGGTCAGCCAGCGAGCATTTCTTGC AAATCCAGCCAATCTCTGGAAAACTCCAACGGCA ACACGTACCTGAACTGGTATCTCCAGAAACCGGG TCAGAGCCCGCAGCTGCTGATCTACCGTGTATCT AAGCGCTTCTCCGGCGTTCCTGATCGTTTCAGCG GTTCTGGATCCGGCACCGACTTTACTCTGAAAAT CAGCCGTGTGGAAGCTGAAGACGTTGGCGTCTAC TATTGTCTGCAGTTGACCCACGTTCCGTACACCTT CGGTCAAGGAACTAAACTGGAAATTAAA |
| 139 | CD19 L1 reverse random | see Table 13 |
| 140 | CD 19 L2 forward random | see Table 13 |
| 141 | CD19 H1 reverse random | see Table 13 |
| 142 | CD19 H2 forward random | see Table 13 |
| 143 | CD19 H3 reverse constant | see Table 13 |
| 144 | LMB3 | see Table 13 |
| 145 | CD19 L1 forward constant | see Table 14 |
| 146 | CD19 L3 reverse random | see Table 14 |
| 147 | CD19 L3 forward constant | see Table 14 |
| 148 | CD19 H3 reverse random | see Table 14 |

(continued)

| SEQID NO: | Name | Sequence |
|---|---|---|
| 149 | Nucleotide sequence SNAP tag human CD19ECD-PDGFR | GGCCGCCGCTAGCGGCATCGACTACAAGGACGA<br>CGATGACAAGGCCGGCATCGATGCCATCATGGAC<br>AAAGACTGCGAAATGAAGCGCACCACCCTGGAT<br>AGCCCTCTGGGCAAGCTGGAACTGTCTGGGTGCG<br>AACAGGGCCTGCACGAGATCAAGCTGCTGGGCA<br>AAGGAACATCTGCCGCCGACGCCGTGGAAGTGCC<br>TGCCCCAGCCGCCGTGCTGGGCGGACCAGAGCCA<br>CTGATGCAGGCCACCGCCTGGCTCAACGCCTACT<br>TTCACCAGCCTGAGGCCATCGAGGAGTTCCCTGT<br>GCCAGCCCTGCACCACCCAGTGTTCCAGCAGGAG<br>AGCTTTACCCGCCAGGTGCTGTGGAAACTGCTGA<br>AAGTGGTGAAGTTCGGAGAGGTCATCAGCTACCA<br>GCAGCTGGCCGCCCTGGCCGGCAATCCCGCCGCC<br>ACCGCCGCCGTGAAAACCGCCCTGAGCGGAAATC<br>CCGTGCCCATTCTGATCCCCTGCCACCGGGTGGT<br>GTCTAGCTCTGGCGCCGTGGGGGGCTACGAGGGC<br>GGGCTCGCCGTGAAAGAGTGGCTGCTGGCCCACG<br>AGGGCCACAGACTGGGCAAGCCTGGGCTGGGTG<br>ATATCCCCGAGGAACCCCTGGTCGTGAAGGTGGA<br>AGAGGGCGACAATGCCGTGCTGCAGTGCCTGAA<br>GGGCACCTCCGATGGCCCTACCCAGCAGCTGACC<br>TGGTCCAGAGAGAGCCCCCTGAAGCCCTTCCTGA<br>AGCTGTCTCTGGGCCTGCCTGGCCTGGGCATCCA<br>TATGAGGCCTCTGGCCATCTGGCTGTTCATCTTCA<br>ACGTGTCCCAGCAGATGGGCGGCTTCTACCTGTG<br>TCAGCCTGGCCCCCCATCTGAGAAGGCTTGGCAG<br>CCTGGCTGGACCGTGAACGTGGAAGGATCCGGCG<br>AGCTGTTCCGGTGGAACGTGTCCGATCTGGGCGG<br>CCTGGGATGCGGCCTGAAGAACAGATCTAGCGA<br>GGGCCCCAGCAGCCCCAGCGGCAAACTGATGAG<br>CCCCAAGCTGTACGTGTGGGCCAAGGACAGACCC<br>GAGATCTGGGAGGGCGAGCCTCCTTGCCTGCCCC<br>CTAGAGACAGCCTGAACCAGAGCCTGAGCCAGG<br>ACCTGACAATGGCCCCTGGCAGCACACTGTGGCT<br>GAGCTGTGGCGTGCCACCCGACTCTGTGTCTAGA<br>GGCCCTCTGAGCTGGACCCACGTGCACCCTAAGG<br>GCCCTAAGAGCCTGCTGAGCCTGGAACTGAAGGA<br>CGACAGGCCCGCCAGAGATATGTGGGTCATGGA<br>AACCGGCCTGCTGCTGCCTAGAGCCACAGCCCAG<br>GATGCCGGCAAGTACTACTGCCACAGAGGCAACC<br>TGACCATGAGCTTCCACCTGGAAATCACCGCCAG<br>ACCCGTGCTGTGGCACTGGCTGCTGAGAACAGGC<br>GGCTGGAAGGTCGACGAACAAAAACTCATCTCA<br>GAAGAGGATCTGAATGCTGTGGGCCAGGACACG<br>CAGGAGGTCATCGTGGTGCCACACTCCTTGCCCT<br>TTAAGGTGGTGGTGATCTCAGCCATCCTGGCCCT<br>GGTGGTGCTCACCATCATCTCCCTTATCATCCTCA<br>TCATGCTTTGGCAGAAGAAGCCACGT |

(continued)

| SEQID NO: | Name | Sequence |
|---|---|---|
| 150 | Nucleotide sequence SNAP tag cynomolgus CD19ECD-PDGFR | CCGGCCGCCGCTAGCGGCATCGACTACAAGGACG<br>ACGATGACAAGGCCGGCATCGATGCCATCATGGA<br>CAAAGACTGCGAAATGAAGCGCACCACCCTGGA<br>TAGCCCTCTGGGCAAGCTGGAACTGTCTGGGTGC<br>GAACAGGGCCTGCACGAGATCAAGCTGCTGGGC<br>AAAGGAACATCTGCCGCCGACGCCGTGGAAGTG<br>CCTGCCCCAGCCGCCGTGCTGGGCGGACCAGAGC<br>CACTGATGCAGGCCACCGCCTGGCTCAACGCCTA<br>CTTTCACCAGCCTGAGGCCATCGAGGAGTTCCCT<br>GTGCCAGCCCTGCACCACCCAGTGTTCCAGCAGG<br>AGAGCTTTACCCGCCAGGTGCTGTGGAAACTGCT<br>GAAAGTGGTGAAGTTCGGAGAGGTCATCAGCTAC<br>CAGCAGCTGGCCGCCCTGGCCGGCAATCCCGCCG<br>CCACCGCCGCCGTGAAAACCGCCCTGAGCGGAA<br>ATCCCGTGCCCATTCTGATCCCCTGCCACCGGGT<br>GGTGTCTAGCTCTGGCGCCGTGGGGGGCTACGAG<br>GGCGGGCTCGCCGTGAAAGAGTGGCTGCTGGCCC<br>ACGAGGGCCACAGACTGGGCAAGCCTGGGCTGG<br>GTGATATCCCCCAGGAACCCCTGGTCGTGAAGGT<br>GGAAGAGGGCGACAATGCCGTGCTCCAGTGTCTC<br>GAGGGCACCTCCGATGGCCCTACACAGCAGCTCG<br>TGTGGTGCAGAGACAGCCCCTTCGAGCCCTTCCT<br>GAACCTGTCTCTGGGCCTGCCTGGCATGGGCATC<br>AGAATGGGCCCTCTGGGCATCTGGCTGCTGATCT<br>TCAACGTGTCCAACCAGACCGGCGGCTTCTACCT<br>GTGTCAGCCTGGCCTGCCAAGCGAGAAGGCTTGG<br>CAGCCTGGATGGACCGTGTCCGTGGAAGGATCTG<br>GCGAGCTGTTCCGGTGGAACGTGTCCGATCTGGG<br>CGGCCTGGGATGCGGCCTGAAGAACAGAAGCAG<br>CGAGGGCCCTAGCAGCCCCAGCGGCAAGCTGAA<br>TAGCAGCCAGCTGTACGTGTGGGCCAAGGACAG<br>ACCCGAGATGTGGGAGGGCGAGCCTGTGTGTGGC<br>CCCCCTAGAGATAGCCTGAACCAGAGCCTGAGCC<br>AGGACCTGACAATGGCCCCTGGCAGCACACTGTG<br>GCTGAGCTGTGGCGTGCCACCCGACTCTGTGTCC<br>AGAGGCCCTCTGAGCTGGACACACGTGCGGCCTA<br>AGGGCCCTAAGAGCAGCCTGCTGAGCCTGGAACT<br>GAAGGACGACCGGCCCGACCGGGATATGTGGGT<br>GGTGGATACAGGCCTGCTGCTGACCAGAGCCACA<br>GCCCAGGATGCCGGCAAGTACTACTGCCACAGAG<br>GCAACTGGACCAAGAGCTTTTACCTGGAAATCAC<br>CGCCAGACCCGCCCTGTGGCACTGGCTGCTGAGA<br>ATCGGAGGCTGGAAGGTCGACGAGCAGAAGCTG<br>ATCTCCGAAGAGGACCTGAACGCCGTGGGCCAG<br>GATACCCAGGAAGTGATCGTGGTGCCCCACAGCC<br>TGCCCTTCAAGGTGGTCGTGATCAGCGCCATTCT<br>GGCCCTGGTGGTGCTGACCATCATCAGCCTGATC<br>ATCCTGATTATGCTGTGGCAGAAAAAGCCCCGC |

(continued)

| SEQID NO: | Name | Sequence |
|---|---|---|
| 151 | Polypeptide sequence SNAP tag human CD19 ECD-PDGFR | PAAASGIDYKDDDDKAGIDAIMDKDCEMKRTTLDSPLGKLELSGCEQGLHEIKLLGKGTSAADAVEVPAPAAVLGGPEPLMQATAWLNAYFHQPEAIEEFPVPALHHPVFQQESFTRQVLWKLLKVVKFGEVISYQQLAALAGNPAATAAVKTALSGNPVPILIPCHRVVSSSGAVGGYEGGLAVKEWLLAHEGHRLGKPGLGDIPEEPLVVKVEEGDNAVLQCLKGTSDGPTQQLTWSRESPLKPFLKLSLGLPGLGIHMRPLAIWLFIFNVSQQMGGFYLCQPGPPSEKAWQPGWTVNVEGSGELFRWNVSDLGGLGCGLKNRSSEGPSSPSGKLMSPKLYVWAKDRPEIWEGEPPCLPPRDSLNQSLSQDLTMAPGSTLWLSCGVPPDSVSRGPLSWTHVHPKGPKSLLSLELKDDRPARDMWVMETGLLLPRATAQDAGKYYCHRGNLTMSFHLEITARPVLWHWLLRTGGWKVDEQKLISEEDLNAVGQDTQEVIVVPHSLPFKVVVISAILALVVLTIISLIILIMLWQKKPR |
| 152 | Polypeptide sequence SNAP tag cynomolgus CD19 ECD-PDGFR | PAAASGIDYKDDDDKAGIDAIMDKDCEMKRTTLDSPLGKLELSGCEQGLHEIKLLGKGTSAADAVEVPAPAAVLGGPEPLMQATAWLNAYFHQPEAIEEFPVPALHHPVFQQESFTRQVLWKLLKVVKFGEVISYQQLAALAGNPAATAAVKTALSGNPVPILIPCHRVVSSSGAVGGYEGGLAVKEWLLAHEGHRLGKPGLGDIPQEPLVVKVEEGDNAVLQCLEGTSDGPTQQLVWCRDSPFEPFLNLSLGLPGMGIRMGPLGIWLLIFNVSNQTGGFYLCQPGLPSEKAWQPGWTVSVEGSGELFRWNVSDLGGLGCGLKNRSSEGPSSPSGKLNSSQLYVWAKDRPEMWEGEPVCGPPRDSLNQSLSQDLTMAPGSTLWLSCGVPPDSVSRGPLSWTHVRPKGPKSSLLSLELKDDRPDRDMWVVDTGLLLTRATAQDAGKYYCHRGNWTKSFYLEITARPALWHWLLRIGGWKVDEQKLISEEDLNAVGQDTQEVIVVPHSLPFKVVVISAILALVVLTIISLIILIMLWQKKPR |
| 153 | nucleotide sequence of anti-CEA (T84.66-LCHA) Fc knob monomeric 4-1BBL (71-248) | see Table 17 |
| 154 | nucleotide sequence of anti-CEA (T84.66-LCHA) light chain | see Table 17 |
| 155 | anti- CEA(T84.66-LCHA) Fc knob monomeric 4-1BBL (71-248) | see Table 17 |
| 156 | anti- CEA(T84.66-LCHA) light chain | see Table 17 |
| 157 | nucleotide sequence of anti-CEA (T84.66-LCHA) Fc knob dimeric 4-1BBL (71-248) | see Table 18 |
| 158 | anti- CEA(T84.66-LCHA) Fc knob dimeric 4-1BBL (71-248) | see Table 18 |

(continued)

| SEQID NO: | Name | Sequence |
|---|---|---|
| 159 | anti-mu CEA T84.66 VH | MKCSWVIFFL MAVVTGVNSE VQLQQSGAEL VEPGASVKLS CTASGFNIKD TYMHWVKQRP EQGLEWIGRI DPANGNSKYV PKFQGKATIT ADTSSNTAYL QLTSLTSEDT AVYYCAPFGY YVSDYAMAYW GQGTSVTVSS |
| 160 | anti-mu CEA T84.66 VL | METDTLLLWV LLLWVPGSTG DIVLTQSPAS LAVSLGQRAT MSCRAGESVD IFGVGFLHWY QQKPGQPPKL LIYRASNLES GIPVRFSGTG SRTDFTLIID PVEADDVATY YCQQTNEDPY TFGGGTKLEI K |
| 161 | IGHV1-69*08 IMGT Acc No. Z14309 | TAAGGGGCTT CCTAGTCCTA AGGCTGAGGA AGGGATCCTG GTTTAGTTAA AGAGGATTTT ATTCACCCCT GTGTCCTCTC CACAGGTGTC CAGTCCCAGG TCCAGCTGGT GCAATCTGGG GCTGAGGTGA AGAAGCCTGG GTCCTCGGTG AAGGTCTCCT GCAAGGCTTC TGGAGGCACC TTCAGCAGCT ATACTATCAG CTGGGTGCGA CAGGCCCCTG GACAAGGGCT TGAGTGGATG GGAAGGATCA TCCCTATCCT TGGTACAGCA AACTACGCAC AGAAGTTCCA GGGCAGAGTC ACGATTACCG CGGACAAATC CACGAGCACA GCCTACATGG AGCTGAGCAG CCTGAGATCT GAGGACACGG CCGTGTATTA CTGTGCGAGA GA |

(continued)

| SEQID NO: | Name | Sequence |
|---|---|---|
| 162 | IGKV3-11*01 IMGT Acc No. | CTGCAGCTGG AAGCTCAGCT CCCACCCAGC TGCTTTGCAT GTCCCTCCCA GCTGCCCTAC CTTCCAGAGC CCATATCAAT GCCTGTGTCA GAGCCCTGGG GAGGAACTGC TCAGTTAGGA CCCAGAGGGA ACCATGGAAG CCCCAGCTCA GCTTCTCTTC CTCCTGCTAC TCTGGCTCCC AGGTGAGGGG AACATGAGGT GGTTTTGCAC ATTAGTGAAA ACTCTTGCCA CCTCTGCTCA GCAAGAAATA TAATTAAAAT TCAAAGTATA TCAACAATTT TGGCTCTACT CAAAGACAGT TGGTTTGATC TTGATTACAT GAGTGCATTT CTGTTTTATT TCCAATTTCA GATACCACCG GAGAAATTGT GTTGACACAG TCTCCAGCCA CCCTGTCTTT GTCTCCAGGG GAAAGAGCCA CCCTCTCCTG CAGGGCCAGT CAGAGTGTTA GCAGCTACTT AGCCTGGTAC CAACAGAAAC CTGGCCAGGC TCCCAGGCTC CTCATCTATG ATGCATCCAA CAGGGCCACT GGCATCCCAG CCAGGTTCAG TGGCAGTGGG TCTGGGACAG ACTTCACTCT CACCATCAGC AGCCTAGAGC CTGAAGATTT TGCAGTTTAT TACTGTCAGC AGCGTAGCAA CTGGCCTCCC ACAGTGATTC CACATGAAAC AAAAACCCCA ACAAGACCAT CAGTGTTTAC TAGATTATTA TACCAGCTGC TTCCTTTACA GACAGCTAGT GGGGTGGCCA CTCAGTGTTA GCATCTCAGC TCTATTTGGC CATTTTGGAG TTCAAGT |
| 163 | Parental CEA binder VH | see Table 20 |
| 164 | Parental CEA binder VL | see Table 20 |
| 165 | nucleotide sequence of DP47 Fc knob monomeric 4-1BBL (71-248) | see Table 21 |
| 166 | nucleotide sequence of DP47 light chain | see Table 21 |
| 167 | DP47 Fc knob monomeric 4-1BBL (71-248) | see Table 21 |
| 168 | DP47 light chain | see Table 21 |
| 169 | nucleotide sequence of DP47 Fc knob dimeric 4-1BBL (71-248) | see Table 22 |
| 170 | DP47 Fc knob dimeric 4-1BBL (71-248) | see Table 22 |
| 171 | nucleotide sequence of anti-FAP (4B9) Fc hole dimeric 4-1BB ligand (71-248) | see Table 23 |
| 172 | nucleotide sequence of Fc knob monomeric 4-1BBL (71-248) | see Table 23 |

(continued)

| SEQID NO: | Name | Sequence |
|---|---|---|
| 173 | anti-FAP(4B9) Fc hole dimeric 4-1BB ligand (71-248) | see Table 23 |
| 174 | Fc knob monomeric 4-1BBL (71-248) | see Table 23 |
| 175 | nucleotide sequence of anti-FAP (4B9) Fc hole monomeric 4-1BBL (71-248) | see Table 24 |
| 176 | nucleotide sequence of Fc knob dimeric 4-1BB ligand (71-248) | see Table 24 |
| 177 | anti-FAP(4B9) Fc hole monomeric 4-1BBL (71-248) | see Table 24 |
| 178 | Fc knob dimeric 4-1BB ligand (71-248) | see Table 24 |
| 179 | nucleotide sequence of anti-FAP (28H1) Fc hole dimeric 4-1BB ligand (71-248) | see Table 25 |
| 180 | anti-FAP(28H1) Fc hole dimeric 4-1BB ligand (71-248) | see Table 25 |
| 181 | nucleotide sequence of anti-FAP (28H1) Fc hole monomeric 4-1BBL (71-248) | see Table 26 |
| 182 | anti-FAP(28H1) Fc hole monomeric 4-1BBL (71-248) | see Table 26 |
| 183 | nucleotide sequence of anti-CD19(8B8-018) Fc hole dimeric 4-1BB ligand (71-248) chain | see Table 27 |
| 184 | anti- CD19(8B8-018) Fc hole dimeric 4-1BB (71-248) ligand chain | see Table 27 |
| 185 | nucleotide sequence of anti-CD19(8B8-018) Fc hole monomeric 4-1BBL (71-248) chain | see Table 28 |
| 186 | anti- CD19(8B8-018) Fc hole monomeric 4-1BBL (71-248) chain | see Table 28 |
| 187 | nucleotide sequence of anti-CD19(8B8-2B11) Fc hole dimeric 4-1BB ligand (71-248) chain | see Table 29 |
| 188 | anti- CD19(8B8-2B11) Fc hole dimeric 4-1BB ligand (71-248) chain | see Table 29 |
| 189 | nucleotide sequence of anti-CD19(8B8-2B11) Fc hole monomeric 4-1BB ligand (71-248) chain | see Table 30 |

(continued)

| SEQID NO: | Name | Sequence |
|---|---|---|
| 190 | anti- CD19(8B8-2B11) Fc hole monomeric 4-1BB ligand (71-248) chain | see Table 30 |
| 191 | nucleotide sequence of anti-CEA (T84.66-LCHA) Fc hole dimeric 4-1BB ligand (71-248) chain | see Table 31 |
| 192 | anti- CEA(T84.66-LCHA) Fc hole dimeric 4-1BB ligand (71-248) chain | see Table 31 |
| 193 | nucleotide sequence of anti-CEA (T84.66-LCHA) Fc hole monomeric 4-1BBL (71-248) chain | see Table 32 |
| 194 | anti- CEA(T84.66-LCHA) Fc hole monomeric 4-1BBL (71-248) chain | see Table 32 |
| 195 | nucleotide sequence of DP47 Fc hole dimeric 4-1BB ligand (71-248) chain | see Table 33 |
| 196 | DP47 Fc hole dimeric 4-1BB ligand (71-248) chain | see Table 33 |
| 197 | nucleotide sequence of DP47 Fc hole monomeric 4-1BBL (71-248) chain | see Table 34 |
| 198 | DP47 Fc hole monomeric 4-1BBL (71-248) chain | see Table 34 |
| 199 | nucleotide sequence of dimeric 4-1BB ligand (71-248) - CL* Fc knob chain | see Table 35 |
| 200 | nucleotide sequence of monomeric 4-1BBL (71-248) -CH1* | see Table 35 |
| 201 | nucleotide sequence of DP47 Fc hole chain | see Table 35 |
| 202 | dimeric 4-1BB ligand (71-248) - CL* Fc knob chain | see Table 35 |
| 203 | monomeric 4-1BBL (71-248) -CHI* | see Table 35 |
| 204 | DP47 Fc hole chain | see Table 35 |
| 205 | nucleotide sequence of DP47 heavy chain (huIgG1 PGLALA) | see Table 38 |
| 206 | DP47 heavy chain (huIgG1 PGLALA) | see Table 38 |
| 207 | nucleotide sequence of human 4-1BB His | see Table 40 |

(continued)

| SEQID NO: | Name | Sequence |
|---|---|---|
| 208 | human 4-1BB His | see Table 40 |
| 209 | nucleotide sequence of FAP(4B9) Fc hole dimeric 4-1BB ligand (71-254) chain | see Table 44 |
| 210 | nucleotide sequence of DP47 Fc knob monomeric 4-1BB (71-254) ligand | see Table 44 |
| 211 | nucleotide sequence of DP47 (VL-CH1) light chain | see Table 44 |
| 212 | anti-FAP(4B9) Fc hole dimeric 4-1BB ligand (71-254) chain | see Table 44 |
| 213 | DP47 Fc knob monomeric 4-1BB (71-254) ligand | see Table 44 |
| 214 | DP47 (VL-CH1) light chain | see Table 44 |
| 215 | nucleotide sequence of anti-FAP (4B9) Fc hole monomeric 4-1BBL (71-254) chain | see Table 45 |
| 216 | nucleotide sequence of DP47 (VH-CL) Fc knob dimeric 4-1BB ligand (71-254) chain | see Table 45 |
| 217 | anti-FAP(4B9) Fc hole monomeric 4-1BBL (71-254) chain | see Table 45 |
| 218 | DP47 (VH-CL) Fc knob dimeric 4-1BB ligand (71-254) chain | see Table 45 |
| 219 | nucleotide sequence of DP47 (VH-CL) Fc hole dimeric 4-1BB ligand (71-254) chain | see Table 45 |
| 220 | nucleotide sequence of FAP (4B9) Fc knob monomeric 4-1BB (71-254) ligand | see Table 46 |
| 221 | DP47(VH-CL) Fc hole dimeric 4-1BB ligand (71-254) chain | see Table 46 |
| 222 | FAP (4B9) Fc knob monomeric 4-1BB (71-254) ligand | see Table 46 |
| 223 | nucleotide sequence of DP47 (VH-CL) Fc hole monomeric 4-1BBL (71-254) chain | see Table 47 |
| 224 | nucleotide sequence of FAP (4B9) Fc knob dimeric 4-1BB ligand (71-254) chain | see Table 47 |
| 225 | DP47 (VH-CL) Fc hole monomeric 4-1BBL (71-254) chain | see Table 47 |

(continued)

| SEQID NO: | Name | Sequence |
|---|---|---|
| 226 | FAP (4B9) Fc knob dimeric 4-1BB ligand (71-254) chain | see Table 47 |
| 227 | nucleotide sequence of DP47 (VHCL) Fc knob monomeric 4-1BB (71-248) ligand | see Table 48 |
| 228 | DP47 (VH-CL) Fc knob monomeric 4-1BB (71-248) ligand | see Table 48 |
| 229 | nucleotide sequence of DP47 (VH-CL) Fc knob dimeric 4-1BB ligand (71-248) chain | see Table 49 |
| 230 | DP47 (VH-CL) Fc knob dimeric 4-1BB ligand (71-248) chain | see Table 49 |
| 231 | nucleotide sequence of DP47 (VH-CL) Fc hole dimeric 4-1BB ligand (71-248) chain | see Table 50 |
| 232 | DP47(VH-CL) Fc hole dimeric 4-1BB ligand (71-248) chain | see Table 50 |
| 233 | nucleotide sequence of DP47 (VH-CL) Fc hole monomeric 4-1BBL (71-248) chain | see Table 51 |
| 234 | DP47 (VH-CL) Fc hole monomeric 4-1BBL (71-248) chain | see Table 51 |
| 235 | nucleotide sequence of anti-FAP (4B9) Fc hole chain | see Table 36 |
| 236 | anti-FAP (4B9) Fc hole chain | see Table 36 |
| 237 | Nucleotide sequence of anti-CD19(8B8-018) Fc hole chain | see Table 37a |
| 238 | anti-CD19(8B8-018) Fc hole chain | see Table 37a |
| 239 | nucleotide sequence of dimeric hu 4-1BBL (71-254) - CL* Fc knob chain | see Table 37b |
| 240 | nucleotide sequence of monomeric hu 4-1BBL (71-254) - CH1* | see Table 37b |
| 241 | nucleotide sequence of anti-CD19(8B8-2B11) Fc hole chain | see Table 37b |
| 242 | Dimeric hu 4-1BBL (71-254) - CL* Fc knob chain | see Table 37b |
| 243 | Monomeric hu 4-1BBL (71-254) - CH1* | see Table 37b |
| 244 | CD19(8B8-2B11) Fc hole chain | see Table 37b |
| 245 | avi tag | GLNDIFEAQKIEWHE |

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the EU numbering systems according to Kabat (Kabat, E.A., et al., Sequences of Proteins of Immunological Interest, 5th ed., Public Health Service, National Institutes of Health, Bethesda, MD (1991)) as defined above.

## EXAMPLES

The following are examples of methods and compositions of the invention. It is understood that various other embodiments may be practiced, given the general description provided above.

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM $KH_2PO_4/K_2HPO_4$, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VH/VL exchange (VH/VL CrossMabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 μg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Determination of binding and binding affinity of multispecific antibodies to the respective antigens using surface plasmon resonance (SPR) (BIACORE)

Binding of the generated antibodies to the respective antigens is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C). Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

## Example 1

### Preparation of monovalent targeted human 4-1BB ligand trimer-containing Fc fusion antigen binding molecules

#### 1.1 Preparation of monovalent FAP-targeted 4-1BB ligand trimer-containing Fc (kih) fusion antigen binding molecule, wherein the 4-1BB ligands are C-terminally fused (Constructs 1.11, 2.11, 1.12 and 2.12) (FAP on the knob chain)

The DNA sequence encoding part of the ectodomain (amino acid 71-248) of human 4-1BB ligand was synthetized according to the P41273 sequence of Uniprot database (SEQ ID NO:75).

A polypeptide containing two ectodomains of 4-1BB ligand, separated by (G4S)2 linkers was subcloned in frame to the C-terminus of human IgG1 Fc hole or knob chain (Merchant, Zhu 1998), as depicted in **Figure 1a:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 1b:** human IgG1 Fc, (G4S)2 connector, human 4-1BB ligand.

The variable region of heavy and light chain DNA sequences encoding a binder specific for fibroblast activation protein (FAP), clone 4B9 or clone 28H1, were subcloned in frame with either the constant heavy chain of the knob or the constant light chain of human IgG1. The generation and preparation of the FAP binders is described in WO 2012/020006 A2, which is incorporated herein by reference.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

For all constructs the knobs into holes heterodimerization technology was used as described in Carter, J Immunol Methods 248, 7-15 (2001). Combination of the huIgG1 Fc hole chain containing the Y349C/T366S/L368A/Y407V mutations in the CH3 domain, the anti-FAP huIgG1 knob chain containing the S354C/T366W mutations in the CH3 domain and the anti-FAP light chain allows generation of a heterodimer, which includes an assembled trimeric 4-1BB ligand and one FAP binding Fab **(Figures 2A and 2B).**

Table 1 shows the cDNA and amino acid sequences of the monovalent FAP(4B9)-targeted 4-1BB ligand (71-248) trimer-containing Fc (kih) fusion antigen binding molecule (Construct 2.11).

**Table 1: Sequences of FAP(4B9)-targeted human 4-1BB ligand trimer containing Fc (kih) fusion molecule Construct 2.11**

| SEQ ID NO: | Description | Sequence |
|---|---|---|
| 101 | nucleotide sequence of Fc hole dimeric 4-1BB ligand (71-248) chain | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTGGAGGCGGCGGA AGCGGAGGAGGAGGATCCAGAGAGGGCCCTGAGCTGAG |

(continued)

| SEQ ID NO: | Description | Sequence |
|---|---|---|
| | | CCCTGATGATCCTGCCGGACTGCTGGACCTGCGGCAGGG AATGTTTGCCCAGCTGGTGGCCCAGAACGTGCTGCTGAT CGATGGCCCCCTGTCCTGGTACAGCGATCCTGGACTGGC TGGCGTGTCACTGACAGGCGGCCTGAGCTACAAAGAGG ACACCAAAGAACTGGTGGTGGCCAAGGCCGGCGTGTAC TACGTGTTCTTTCAGCTGGAACTGCGGAGAGTGGTGGCC GGCGAAGGATCTGGCTCTGTGTCTCTGGCCCTGCATCTG CAGCCTCTGAGATCTGCTGCTGGCGCCGCTGCTCTGGCA CTGACAGTGGATCTGCCTCCTGCCAGCAGCGAGGCCCG GAATAGCGCATTTGGGTTTCAAGGCAGGCTGCTGCACCT GTCTGCCGGCCAGAGGCTGGGAGTGCATCTGCACACAG AGGCCAGGGCTAGACACGCCTGGCAGCTGACACAGGGC GCTACAGTGCTGGGCCTGTTCAGAGTGACCCCCGAGATT CCAGCAGGCCTGGGAGGCGGCGGATCTGGCGGCGGAGG ATCTAGAGAAGGACCCGAGCTGTCCCCCGACGATCCCG CTGGGCTGCTGGATCTGAGACAGGGCATGTTCGCTCAGC TGGTGGCTCAGAATGTGCTGCTGATTGACGGACCTCTGA GCTGGTACTCCGACCCAGGGCTGGCAGGGGTGTCCCTG ACTGGGGGACTGTCCTACAAAGAAGATACAAAAGAACT GGTGGTGGCTAAAGCTGGGGTGTACTATGTGTTTTTCA GCTGGAACTGAGGCGGGTGGTGGCTGGGGAGGGCTCAG GATCTGTGTCCCTGGCTCTGCATCTGCAGCCACTGCGCT CTGCAGCAGGGGCTGCAGCACTGGCCCTGACTGTGGAC CTGCCCCCAGCTTCTTCCGAGGCCAGAAACAGCGCCTTC GGGTTCCAAGGACGCCTGCTGCATCTGAGCGCCGGACA GCGCCTGGGAGTGCATCTGCATACTGAAGCCAGAGCCC GGCATGCTTGGCAGCTGACTCAGGGGGCAACTGTGCTG GGACTGTTTCGCGTGACACCTGAGATCCCAGCCGGGCTC |

(continued)

| SEQ ID NO: | Description | Sequence |
|---|---|---|
| 102 | nucleotide sequence of anti-FAP(4B9) Fc knob monomeric 4-1BB (71-248) ligand | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCATCGGTCTTCCCCCT GGCACCCTCCTCCAAGAGCACCTCTGGGGGCACAGCGG CCCTGGGCTGCCTGGTCAAGGACTACTTCCCCGAACCGG TGACGGTGTCGTGGAACTCAGGCGCCCTGACCAGCGGC GTGCACACCTTCCCGGCTGTCCTACAGTCCTCAGGACTC TACTCCCTCAGCAGCGTGGTGACCGTGCCCTCCAGCAGC TTGGGCACCCAGACCTACATCTGCAACGTGAATCACAA GCCCAGCAACACCAAGGTGGACAAGAAAGTTGAGCCCA AATCTTGTGACAAAACTCACACATGCCCACCGTGCCCAG CACCTGAAGCTGCAGGGGGACCGTCAGTCTTCCTCTTCC CCCCAAAACCCAAGGACACCCTCATGATCTCCCGGACCC CTGAGGTCACATGCGTGGTGGTGGACGTGAGCCACGAA GACCCTGAGGTCAAGTTCAACTGGTACGTGGACGGCGT GGAGGTGCATAATGCCAAGACAAAGCCGCGGGAGGAGC AGTACAACAGCACGTACCGTGTGGTCAGCGTCCTCACCG TCCTGCACCAGGACTGGCTGAATGGCAAGGAGTACAAG TGCAAGGTCTCCAACAAAGCCCTCGGCGCCCCCATCGA |

(continued)

| SEQ ID NO: | Description | Sequence |
|---|---|---|
| | | GAAAACCATCTCCAAAGCCAAAGGGCAGCCCCGAGAAC CACAGGTGTACACCCTGCCCCCCTGCAGAGATGAGCTG ACCAAGAACCAGGTGTCCCTGTGGTGTCTGGTCAAGGGC TTCTACCCCAGCGATATCGCCGTGGAGTGGGAGAGCAA CGGCCAGCCTGAGAACAACTACAAGACCACCCCCCCTG TGCTGGACAGCGACGGCAGCTTCTTCCTGTACTCCAAAC TGACCGTGGACAAGAGCCGGTGGCAGCAGGGCAACGTG TTCAGCTGCAGCGTGATGCACGAGGCCCTGCACAACCA CTACACCCAGAAGTCCCTGAGCCTGAGCCCCGGCGGAG GCGGCGGAAGCGGAGGAGGAGGATCCAGAGAGGGCCC TGAGCTGAGCCCTGATGATCCTGCCGGACTGCTGGACCT GCGGCAGGGAATGTTTGCCCAGCTGGTGGCCCAGAACG TGCTGCTGATCGATGGCCCCCTGTCCTGGTACAGCGATC CTGGACTGGCTGGCGTGTCACTGACAGGCGGCCTGAGCT ACAAAGAGGACACCAAAGAACTGGTGGTGGCCAAGGCC GGCGTGTACTACGTGTTCTTTCAGCTGGAACTGCGGAGA GTGGTGGCCGGCGAAGGATCTGGCTCTGTGTCTCTGGCC CTGCATCTGCAGCCTCTGAGATCTGCTGCTGGCGCCGCT GCTCTGGCACTGACAGTGGATCTGCCTCCTGCCAGCAGC GAGGCCCGGAATAGCGCATTTGGGTTTCAAGGCAGGCT GCTGCACCTGTCTGCCGGCCAGAGGCTGGGAGTGCATCT GCACACAGAGGCCAGGGCTAGACACGCCTGGCAGCTGA CACAGGGCGCTACAGTGCTGGGCCTGTTCAGAGTGACC CCCGAGATTCCTGCCGGGCTC |
| 103 | nucleotide sequence of anti-FAP(4B9) light chain | GAGATCGTGCTGACCCAGTCCCCCGGCACCCTGTCTCTG AGCCCTGGCGAGAGAGCCACCCTGTCCTGCAGAGCCTC CCAGTCCGTGACCTCCTCCTACCTCGCCTGGTATCAGCA GAAGCCCGGCCAGGCCCCTCGGCTGCTGATCAACGTGG GCAGTCGGAGAGCCACCGGCATCCCTGACCGGTTCTCCG GCTCTGGCTCCGGCACCGACTTCACCCTGACCATCTCCC GGCTGGAACCCGAGGACTTCGCCGTGTACTACTGCCAGC AGGGCATCATGCTGCCCCCCACCTTTGGCCAGGGCACCA AGGTGGAAATCAAGCGTACGGTGGCTGCACCATCTGTCT TCATCTTCCCGCCATCTGATGAGCAGTTGAAATCTGGAA CTGCCTCTGTTGTGTGCCTGCTGAATAACTTCTATCCCAG AGAGGCCAAAGTACAGTGGAAGGTGGATAACGCCCTCC AATCGGGTAACTCCCAGGAGAGTGTCACAGAGCAGGAC AGCAAGGACAGCACCTACAGCCTCAGCAGCACCCTGAC GCTGAGCAAAGCAGACTACGAGAAACACAAAGTCTACG CCTGCGAAGTCACCCATCAGGGCCTGAGCTCGCCCGTCA CAAAGAGCTTCAACAGGGGAGAGTGT |

(continued)

| SEQ ID NO: | Description | Sequence |
|---|---|---|
| 104 | Fc hole dimeric 4-1BB (71-248) ligand chain | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVCTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGGGGGSGGGGSREGPE LSPDDPAGLLDLRQGMFAQLVAQNVLLIDGPLSWYSDPGL AGVSLTGGLSYKEDTKELVVAKAGVYYVFFQLELRRVVA GEGSGSVSLALHLQPLRSAAGAAALALTVDLPPASSEARN SAFGFQGRLLHLSAGQRLGVHLHTEARARHAWQLTQGAT VLGLFRVTPEIPAGLGGGGSGGGGSREGPELSPDDPAGLLD LRQGMFAQLVAQNVLLIDGPLSWYSDPGLAGVSLTGGLSY KEDTKELVVAKAGVYYVFFQLELRRVVAGEGSGSVSLAL HLQPLRSAAGAAALALTVDLPPASSEARNSAFGFQGRLLH LSAGQRLGVHLHTEARARHAWQLTQGATVLGLFRVTPEIP AGL |
| 105 | anti-FAP(4B9) Fc knob monomeric 41-BB (71-248) ligand | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGL |
| 106 | anti-FAP(4B9) light chain | EIVLTQSPGTLSLSPGERATLSCRASQSVTSSYLAWYQQKP GQAPRLLINVGSRRATGIPDRFSGSGSGTDFTLTISRLEPEDF AVYYCQQGIMLPPTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

Table 2 shows the cDNA and amino acid sequences of the monovalent FAP(4B9)-targeted 4-1BB ligand (71-248) trimer-containing Fc (kih) fusion antigen binding molecule (Construct 2.12).

**Table 2: Sequences of FAP(4B9)-targeted human 4-1BB ligand trimer containing Fc (kih) fusion molecule Construct 2.12 (comparative example)**

| SEQ ID NO: | Description | Sequence |
|---|---|---|
| 107 | nucleotide sequence of Fc hole monomeric 4-1BBL (71-248) chain | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAGAGAGGGCCCT GAGCTGAGCCCTGATGATCCTGCCGGACTGCTGGACCTG CGGCAGGGAATGTTTGCCCAGCTGGTGGCCCAGAACGT GCTGCTGATCGATGGCCCCCTGTCCTGGTACAGCGATCC TGGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGATCTGCTGCTGGCGCCGCTG CTCTGGCACTGACAGTGGATCTGCCTCCTGCCAGCAGCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC CCGAGATTCCTGCCGGGCTC |

(continued)

| SEQ ID NO: | Description | Sequence |
|---|---|---|
| 108 | nucleotide sequence of anti-FAP(4B9) Fc knob dimeric 4-1BB ligand (71-248) | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCATCGGTCTTCCCCCT GGCACCCTCCTCCAAGAGCACCTCTGGGGGCACAGCGG CCCTGGGCTGCCTGGTCAAGGACTACTTCCCCGAACCGG TGACGGTGTCGTGGAACTCAGGCGCCCTGACCAGCGGC GTGCACACCTTCCCGGCTGTCCTACAGTCCTCAGGACTC TACTCCCTCAGCAGCGTGGTGACCGTGCCCTCCAGCAGC TTGGGCACCCAGACCTACATCTGCAACGTGAATCACAA GCCCAGCAACACCAAGGTGGACAAGAAAGTTGAGCCCA AATCTTGTGACAAAACTCACACATGCCCACCGTGCCCAG CACCTGAAGCTGCAGGGGGACCGTCAGTCTTCCTCTTCC CCCCAAAACCCAAGGACACCCTCATGATCTCCCGGACCC CTGAGGTCACATGCGTGGTGGTGGACGTGAGCCACGAA GACCCTGAGGTCAAGTTCAACTGGTACGTGGACGGCGT GGAGGTGCATAATGCCAAGACAAAGCCGCGGGAGGAGC AGTACAACAGCACGTACCGTGTGGTCAGCGTCCTCACCG TCCTGCACCAGGACTGGCTGAATGGCAAGGAGTACAAG TGCAAGGTCTCCAACAAAGCCCTCGGCGCCCCCATCGA GAAAACCATCTCCAAAGCCAAAGGGCAGCCCCGAGAAC CACAGGTGTACACCCTGCCCCCCTGCAGAGATGAGCTG ACCAAGAACCAGGTGTCCCTGTGGTGTCTGGTCAAGGGC TTCTACCCCAGCGATATCGCCGTGGAGTGGGAGAGCAA CGGCCAGCCTGAGAACAACTACAAGACCACCCCCCCTG TGCTGGACAGCGACGGCAGCTTCTTCCTGTACTCCAAAC TGACCGTGGACAAGAGCCGGTGGCAGCAGGGCAACGTG TTCAGCTGCAGCGTGATGCACGAGGCCCTGCACAACCA |

(continued)

| SEQ ID NO: | Description | Sequence |
|---|---|---|
| | | CTACACCCAGAAGTCCCTGAGCCTGAGCCCCGGCAGAG AGGGCCCTGAGCTGAGCCCTGATGATCCTGCCGGACTGC TGGACCTGCGGCAGGGAATGTTTGCCCAGCTGGTGGCCC AGAACGTGCTGCTGATCGATGGCCCCCTGTCCTGGTACA GCGATCCTGGACTGGCTGGCGTGTCACTGACAGGCGGC CTGAGCTACAAAGAGGACACCAAAGAACTGGTGGTGGC CAAGGCCGGCGTGTACTACGTGTTCTTTCAGCTGGAACT GCGGAGAGTGGTGGCCGGCGAAGGATCTGGCTCTGTGT CTCTGGCCCTGCATCTGCAGCCTCTGAGATCTGCTGCTG GCGCCGCTGCTCTGGCACTGACAGTGGATCTGCCTCCTG CCAGCAGCGAGGCCCGGAATAGCGCATTTGGGTTTCAA GGCAGGCTGCTGCACCTGTCTGCCGGCCAGAGGCTGGG AGTGCATCTGCACACAGAGGCCAGGGCTAGACACGCCT GGCAGCTGACACAGGGCGCTACAGTGCTGGGCCTGTTC AGAGTGACCCCCGAGATTCCAGCAGGCCTGGGAGGCGG CGGATCTGGCGGCGGAGGATCTAGAGAAGGACCCGAGC TGTCCCCCGACGATCCCGCTGGGCTGCTGGATCTGAGAC AGGGCATGTTCGCTCAGCTGGTGGCTCAGAATGTGCTGC TGATTGACGGACCTCTGAGCTGGTACTCCGACCCAGGGC TGGCAGGGGTGTCCCTGACTGGGGGACTGTCCTACAAA GAAGATACAAAAGAACTGGTGGTGGCTAAAGCTGGGGT GTACTATGTGTTTTTTCAGCTGGAACTGAGGCGGGTGGT GGCTGGGGAGGGCTCAGGATCTGTGTCCCTGGCTCTGCA TCTGCAGCCACTGCGCTCTGCAGCAGGGGCTGCAGCACT GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |
| 103 | nucleotide sequence of anti-FAP(4B9) light chain | See Table 1 |
| 109 | Fc hole monomeric 4-1BB ligand (71-248) chain | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVCTLPPSRDELTKNQVSLSCAVKGFYPSDIAVEW ESNGQPENNYKTTPPVLDSDGSFFLVSKLTVDKSRWQQGN VFSCSVMHEALHNHYTQKSLSLSPGREGPELSPDDPAGLL DLRQGMFAQLVAQNVLLIDGPLSWYSDPGLAGVSLTGGLS YKEDTKELVVAKAGVYYVFFQLELRRVVAGEGSGSVSLA LHLQPLRSAAGAAALALTVDLPPASSEARNSAFGFQGRLL HLSAGQRLGVHLHTEARARHAWQLTQGATVLGLFRVTPEI PAGL |

(continued)

| SEQ ID NO: | Description | Sequence |
|---|---|---|
| 110 | anti-FAP(4B9) Fc knob dimeric 4-1BB ligand (71-248) | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKS LSLSPGREGPELSPDDPAGLLDLRQGMFAQLVAQNVLLID GPLSWYSDPGLAGVSLTGGLSYKEDTKELVVAKAGVYYV FFQLELRRVVAGEGSGSVSLALHLQPLRSAAGAAALALTV DLPPASSEARNSAFGFQGRLLHLSAGQRLGVHLHTEARAR HAWQLTQGATVLGLFRVTPEIPAGLGGGGSGGGGSREGPE LSPDDPAGLLDLRQGMFAQLVAQNVLLIDGPLSWYSDPGL AGVSLTGGLSYKEDTKELVVAKAGVYYVFFQLELRRVVA GEGSGSVSLALHLQPLRSAAGAAALALTVDLPPASSEARN SAFGFQGRLLHLSAGQRLGVHLHTEARARHAWQLTQGAT VLGLFRVTPEIPAGL |
| 106 | anti-FAP(4B9) light chain | See Table 1 |

Table 3 shows the cDNA and amino acid sequences of the monovalent FAP(28H1)-targeted 4-1BB ligand trimer-containing Fc (kih) fusion antigen binding molecule (Construct 1.11).

**Table 3: Sequences of FAP(28H1)-targeted human 4-1BB ligand trimer containing Fc (kih) fusion molecule Construct 1.11**

| SEQ ID NO: | Description | Sequence |
|---|---|---|
| 101 | nucleotide sequence of Fc hole dimeric 4-1BB ligand (71-248) chain | See Table 1 |
| 111 | nucleotide sequence of anti-FAP(28H1) Fc knob monomeric 4-1BB ligand (71-248) | GAAGTGCAGCTGCTGGAATCCGGCGGAGGCCTGGTGCA GCCTGGCGGATCTCTGAGACTGTCCTGCGCCGCCTCCGG CTTCACCTTCTCCTCCCACGCCATGTCCTGGGTCCGACA GGCTCCTGGCAAAGGCCTGGAATGGGTGTCCGCCATCTG GGCCTCCGGCGAGCAGTACTACGCCGACTCTGTGAAGG GCCGGTTCACCATCTCCCGGGACAACTCCAAGAACACCC TGTACCTGCAGATGAACTCCCTGCGGGCCGAGGACACC GCCGTGTACTACTGTGCCAAGGGCTGGCTGGGCAACTTC GACTACTGGGGACAGGGCACCCTGGTCACCGTGTCCAG CGCTAGCACCAAGGGCCCATCGGTCTTCCCCCTGGCACC CTCCTCCAAGAGCACCTCTGGGGGCACAGCGGCCCTGG GCTGCCTGGTCAAGGACTACTTCCCCGAACCGGTGACGG TGTCGTGGAACTCAGGCGCCCTGACCAGCGGCGTGCAC |

(continued)

| SEQ ID NO: | Description | Sequence |
|---|---|---|
| | | ACCTTCCCGGCTGTCCTACAGTCCTCAGGACTCTACTCC CTCAGCAGCGTGGTGACCGTGCCCTCCAGCAGCTTGGGC ACCCAGACCTACATCTGCAACGTGAATCACAAGCCCAG CAACACCAAGGTGGACAAGAAAGTTGAGCCCAAATCTT GTGACAAAACTCACACATGCCCACCGTGCCCAGCACCT GAAGCTGCAGGGGGACCGTCAGTCTTCCTCTTCCCCCA AAACCCAAGGACACCCTCATGATCTCCCGGACCCCTGA GGTCACATGCGTGGTGGTGGACGTGAGCCACGAAGACC CTGAGGTCAAGTTCAACTGGTACGTGGACGGCGTGGAG GTGCATAATGCCAAGACAAAGCCGCGGGAGGAGCAGTA CAACAGCACGTACCGTGTGGTCAGCGTCCTCACCGTCCT GCACCAGGACTGGCTGAATGGCAAGGAGTACAAGTGCA AGGTCTCCAACAAAGCCCTCGGCGCCCCCATCGAGAAA ACCATCTCCAAAGCCAAAGGGCAGCCCCGAGAACCACA GGTGTACACCCTGCCCCCCTGCAGAGATGAGCTGACCA AGAACCAGGTGTCCCTGTGGTGTCTGGTCAAGGGCTTCT ACCCCAGCGATATCGCCGTGGAGTGGGAGAGCAACGGC CAGCCTGAGAACAACTACAAGACCACCCCCCCTGTGCT GGACAGCGACGGCAGCTTCTTCCTGTACTCCAAACTGAC CGTGGACAAGAGCCGGTGGCAGCAGGGCAACGTGTTCA GCTGCAGCGTGATGCACGAGGCCCTGCACAACCACTAC ACCCAGAAGTCCCTGAGCCTGAGCCCCGGCGGAGGCGG CGGAAGCGGAGGAGGAGGATCCAGAGAGGGCCCTGAG CTGAGCCCTGATGATCCTGCCGGACTGCTGGACCTGCGG CAGGGAATGTTTGCCCAGCTGGTGGCCCAGAACGTGCTG CTGATCGATGGCCCCCTGTCCTGGTACAGCGATCCTGGA CTGGCTGGCGTGTCACTGACAGGCGGCCTGAGCTACAA AGAGGACACCAAAGAACTGGTGGTGGCCAAGGCCGGCG TGTACTACGTGTTCTTTCAGCTGGAACTGCGGAGAGTGG TGGCCGGCGAAGGATCTGGCTCTGTGTCTCTGGCCCTGC ATCTGCAGCCTCTGAGATCTGCTGCTGGCGCCGCTGCTC TGGCACTGACAGTGGATCTGCCTCCTGCCAGCAGCGAG GCCCGGAATAGCGCATTTGGGTTTCAAGGCAGGCTGCTG CACCTGTCTGCCGGCCAGAGGCTGGGAGTGCATCTGCAC ACAGAGGCCAGGGCTAGACACGCCTGGCAGCTGACACA GGGCGCTACAGTGCTGGGCCTGTTCAGAGTGACCCCCG AGATTCCTGCCGGGCTC |

(continued)

| SEQ ID NO: | Description | Sequence |
|---|---|---|
| 112 | nucleotide sequence of anti-FAP(28H1) light chain | GAGATCGTGCTGACCCAGTCCCCCGGCACCCTGTCTCTG AGCCCTGGCGAGAGAGCCACCCTGTCCTGCAGAGCCTC CCAGTCCGTGTCCCGGTCCTACCTCGCCTGGTATCAGCA GAAGCCCGGCCAGGCCCCTCGGCTGCTGATCATCGGCG CCTCTACCAGAGCCACCGGCATCCCTGACCGGTTCTCCG GCTCTGGCTCCGGCACCGACTTCACCCTGACCATCTCCC GGCTGGAACCCGAGGACTTCGCCGTGTACTACTGCCAGC AGGGCCAGGTCATCCCTCCCACCTTTGGCCAGGGCACCA AGGTGGAAATCAAGCGTACGGTGGCTGCACCATCTGTCT TCATCTTCCCGCCATCTGATGAGCAGTTGAAATCTGGAA CTGCCTCTGTTGTGTGCCTGCTGAATAACTTCTATCCCAG AGAGGCCAAAGTACAGTGGAAGGTGGATAACGCCCTCC AATCGGGTAACTCCCAGGAGAGTGTCACAGAGCAGGAC AGCAAGGACAGCACCTACAGCCTCAGCAGCACCCTGAC GCTGAGCAAAGCAGACTACGAGAAACACAAAGTCTACG CCTGCGAAGTCACCCATCAGGGCCTGAGCTCGCCCGTCA CAAAGAGCTTCAACAGGGGAGAGTGT |
| 104 | Fc hole dimeric 4-1BB ligand (71-248) chain | See Table 1 |
| 113 | anti-FAP(28H1) Fc knob monomeric 4-1BB ligand (71-248) | EVQLLESGGGLVQPGGSLRLSCAASGFTFSSHAMSWVRQA PGKGLEWVSAIWASGEQYYADSVKGRFTISRDNSKNTLYL QMNSLRAEDTAVYYCAKGWLGNFDYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGL |
| 114 | anti-FAP(28H1) light chain | EIVLTQSPGTLSLSPGERATLSCRASQSVSRSYLAWYQQKP GQAPRLLIIGASTRATGIPDRFSGSGSGTDFTLTISRLEPEDF AVYYCQQGQVIPPTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

Table 4 shows the cDNA and amino acid sequences of the monovalent FAP(28H1)-targeted 4-1BB ligand trimer-containing Fc (kih) fusion antigen binding molecule (Construct 1.12).

**Table 4: Sequences of FAP(28H1)-targeted human 4-1BB ligand trimer containing Fc (kih) fusion molecule Construct 1.12 (comparative example)**

| SEQ ID NO: | Description | Sequence |
|---|---|---|
| 107 | nucleotide sequence of Fc hole monomeric 4-1BB ligand (71-248) chain | See Table 2 |

(continued)

| SEQ ID NO: | Description | Sequence |
|---|---|---|
| 115 | nucleotide sequence of anti-FAP(28H1)Fcknob dimeric 4-1BB ligand (71-248) | GAAGTGCAGCTGCTGGAATCCGGCGGAGGCCTGGTGCA GCCTGGCGGATCTCTGAGACTGTCCTGCGCCGCCTCCGG CTTCACCTTCTCCTCCCACGCCATGTCCTGGGTCCGACA GGCTCCTGGCAAAGGCCTGGAATGGGTGTCCGCCATCTG GGCCTCCGGCGAGCAGTACTACGCCGACTCTGTGAAGG GCCGGTTCACCATCTCCCGGGACAACTCCAAGAACACCC TGTACCTGCAGATGAACTCCCTGCGGGCCGAGGACACC GCCGTGTACTACTGTGCCAAGGGCTGGCTGGGCAACTTC GACTACTGGGGACAGGGCACCCTGGTCACCGTGTCCAG CGCTAGCACCAAGGGCCCATCGGTCTTCCCCCTGGCACC CTCCTCCAAGAGCACCTCTGGGGGCACAGCGGCCCTGG GCTGCCTGGTCAAGGACTACTTCCCCGAACCGGTGACGG TGTCGTGGAACTCAGGCGCCCTGACCAGCGGCGTGCAC ACCTTCCCGGCTGTCCTACAGTCCTCAGGACTCTACTCC CTCAGCAGCGTGGTGACCGTGCCCTCCAGCAGCTTGGGC ACCCAGACCTACATCTGCAACGTGAATCACAAGCCCAG CAACACCAAGGTGGACAAGAAAGTTGAGCCCAAATCTT GTGACAAAACTCACACATGCCCACCGTGCCCAGCACCT GAAGCTGCAGGGGGACCGTCAGTCTTCCTCTTCCCCCCA AAACCCAAGGACACCCTCATGATCTCCCGGACCCCTGA GGTCACATGCGTGGTGGTGGACGTGAGCCACGAAGACC CTGAGGTCAAGTTCAACTGGTACGTGGACGGCGTGGAG GTGCATAATGCCAAGACAAAGCCGCGGGAGGAGCAGTA CAACAGCACGTACCGTGTGGTCAGCGTCCTCACCGTCCT GCACCAGGACTGGCTGAATGGCAAGGAGTACAAGTGCA AGGTCTCCAACAAAGCCCTCGGCGCCCCCATCGAGAAA ACCATCTCCAAAGCCAAAGGGCAGCCCCGAGAACCACA GGTGTACACCCTGCCCCCCTGCAGAGATGAGCTGACCA AGAACCAGGTGTCCCTGTGGTGTCTGGTCAAGGGCTTCT ACCCCAGCGATATCGCCGTGGAGTGGGAGAGCAACGGC CAGCCTGAGAACAACTACAAGACCACCCCCCCTGTGCT GGACAGCGACGGCAGCTTCTTCCTGTACTCCAAACTGAC CGTGGACAAGAGCCGGTGGCAGCAGGGCAACGTGTTCA GCTGCAGCGTGATGCACGAGGCCCTGCACAACCACTAC ACCCAGAAGTCCCTGAGCCTGAGCCCCGGCAGAGAGGG CCCTGAGCTGAGCCCTGATGATCCTGCCGGACTGCTGGA CCTGCGGCAGGGAATGTTTGCCCAGCTGGTGGCCCAGA ACGTGCTGCTGATCGATGGCCCCCTGTCCTGGTACAGCG ATCCTGGACTGGCTGGCGTGTCACTGACAGGCGGCCTGA GCTACAAAGAGGACACCAAAGAACTGGTGGTGGCCAAG GCCGGCGTGTACTACGTGTTCTTTCAGCTGGAACTGCGG AGAGTGGTGGCCGGCGAAGGATCTGGCTCTGTGTCTCTG GCCCTGCATCTGCAGCCTCTGAGATCTGCTGCTGGCGCC GCTGCTCTGGCACTGACAGTGGATCTGCCTCCTGCCAGC AGCGAGGCCCGGAATAGCGCATTTGGGTTTCAAGGCAG GCTGCTGCACCTGTCTGCCGGCCAGAGGCTGGGAGTGC ATCTGCACACAGAGGCCAGGGCTAGACACGCCTGGCAG CTGACACAGGGCGCTACAGTGCTGGGCCTGTTCAGAGTG ACCCCCGAGATTCCAGCAGGCCTGGGAGGCGGCGGATC TGGCGGCGGAGGATCTAGAGAAGGACCCGAGCTGTCCC CCGACGATCCCGCTGGGCTGCTGGATCTGAGACAGGGC ATGTTCGCTCAGCTGGTGGCTCAGAATGTGCTGCTGATT GACGGACCTCTGAGCTGGTACTCCGACCCAGGGCTGGC AGGGGTGTCCCTGACTGGGGGACTGTCCTACAAAGAAG |

(continued)

| SEQ ID NO: | Description | Sequence |
|---|---|---|
| | | ATACAAAAGAACTGGTGGTGGCTAAAGCTGGGGTGTACTATGTGTTTTTCAGCTGGAACTGAGGCGGGTGGTGGCTGGGGAGGGCTCAGGATCTGTGTCCCTGGCTCTGCATCTGCAGCCACTGCGCTCTGCAGCAGGGGCTGCAGCACTGGCCCTGACTGTGGACCTGCCCCCAGCTTCTTCCGAGGCCAGAAACAGCGCCTTCGGGTTCCAAGGACGCCTGCTGCATCTGAGCGCCGGACAGCGCCTGGGAGTGCATCTGCATACTGAAGCCAGAGCCCGGCATGCTTGGCAGCTGACTCAGGGGGCAACTGTGCTGGGACTGTTTCGCGTGACACCTGAGATCCCAGCCGGGCTC |
| 112 | nucleotide sequence of anti-FAP(28H1) light chain | See Table 3 |
| 109 | Fc hole monomeric 4-1BB ligand (71-248) chain | See Table 2 |
| 116 | anti-FAP(28H1)Fcknob dimeric 4-1BB ligand (71-248) | EVQLLESGGGLVQPGGSLRLSCAASGFTFSSHAMSWVRQAPGKGLEWVSAIWASGEQYYADSVKGRFTISRDNSKNTLYLQMNSLRAEDTAVYYCAKGWLGNFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 114 | anti-FAP(28H1) light chain | See Table 3 |

**1.2 Preparation of monovalent CD19-targeted 4-1BB ligand trimer-containing Fc (kih) fusion antigen binding molecule, wherein the 4-1BB ligands are C-terminally fused (Constructs 3.11, 4.11, 3.12 and 4.12) (CD19 on the knob chain)**

The molecule was prepared as described in Example 1.1 for the monovalent FAP targeted construct, with the only difference that the anti-FAP binder was replaced by an anti-CD 19 binder.

The variable region of heavy and light chain DNA sequences encoding a binder specific for CD19, clone 8B8-018 or clone 8B8-2B11, were subcloned in frame with either the constant heavy chain of the knob or the constant light chain of human IgG1. The generation of the CD19 clones is described in Example 1.3.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

For all constructs the knobs into holes heterodimerization technology was used as described in Carter, J Immunol Methods 248, 7-15 (2001). Combination of the huIgG1 Fc hole chain containing the Y349C/T366S/L368A/Y407V mutations in the CH3 domain, the anti-FAP huIgG1 knob chain containing the S354C/T366W mutations in the CH3 domain and the anti-FAP light chain allows generation of a heterodimer, which includes an assembled trimeric 4-1BB ligand and one CD19 binding Fab **(Figures 2A and 2B).**

Table 5 shows the cDNA and amino acid sequences of the monovalent CD19(8B8-018)-targeted 4-1BB ligand (71-248) trimer-containing Fc (kih) fusion antigen binding molecule (Construct 3.11).

**Table 5: Sequences of CD19(8B8-018)-targeted human 4-1BB ligand trimer containing Fc (kih) fusion molecule Construct 3.11**

| SEQ ID NO: | Description | Sequence |
|---|---|---|
| 101 | nucleotide sequence of Fc hole dimeric 4-1BB ligand (71-248) chain | see Table 1 |
| 117 | nucleotide sequence of anti-CD19(8B8-018) Fc knob monomeric 4-1BB (71-248) **ligand** | CAGGTCCAGCTGGTGCAGTCCGGCGCCGAGGTCAAGAA ACCCGGGGCTTCTGTGAAGGTTTCATGCAAGGCAAGCG GATACACCTTCACCGACTATATCATGCATTGGGTCAGGC AGGCCCCTGGCCAAGGTCTCGAATGGATGGGCTACATTA |

(continued)

| SEQ ID NO: | Description | Sequence |
|---|---|---|
| | | ACCCATATAATGATGGCTCCAAATACACCGAGAAGTTTC AGGGAAGAGTCACTATGACATCTGACACCAGTATCAGC ACTGCTTACATGGAGCTGTCCCGCCTTCGGTCTGATGAC ACCGCAGTGTATTACTGTGCCAGGGGCACATATTACTAC GGCTCAGCTCTGTTCGACTATTGGGGGCAGGGAACCACA GTAACCGTGAGCTCCGCTAGCACCAAGGGCCCATCGGT CTTCCCCCTGGCACCCTCCTCCAAGAGCACCTCTGGGGG CACAGCGGCCCTGGGCTGCCTGGTCAAGGACTACTTCCC CGAACCGGTGACGGTGTCGTGGAACTCAGGCGCCCTGA CCAGCGGCGTGCACACCTTCCCGGCTGTCCTACAGTCCT CAGGACTCTACTCCCTCAGCAGCGTGGTGACCGTGCCCT CCAGCAGCTTGGGCACCCAGACCTACATCTGCAACGTG AATCACAAGCCCAGCAACACCAAGGTGGACAAGAAAGT TGAGCCCAAATCTTGTGACAAAACTCACACATGCCCACC GTGCCCAGCACCTGAAGCTGCAGGGGGACCGTCAGTCT TCCTCTTCCCCCCAAAACCCAAGGACACCCTCATGATCT CCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTACACCCTGCCCCCCTGCAGA GATGAGCTGACCAAGAACCAGGTGTCCCTGTGGTGTCTG GTCAAGGGCTTCTACCCCAGCGATATCGCCGTGGAGTGG GAGAGCAACGGCCAGCCTGAGAACAACTACAAGACCAC CCCCCCTGTGCTGGACAGCGACGGCAGCTTCTTCCTGTA CTCCAAACTGACCGTGGACAAGAGCCGGTGGCAGCAGG GCAACGTGTTCAGCTGCAGCGTGATGCACGAGGCCCTG CACAACCACTACACCCAGAAGTCCCTGAGCCTGAGCCC CGGCGGAGGCGGCGGAAGCGGAGGAGGAGGATCCAGA GAGGGCCCTGAGCTGAGCCCTGATGATCCTGCCGGACT GCTGGACCTGCGGCAGGGAATGTTTGCCCAGCTGGTGGC CCAGAACGTGCTGCTGATCGATGGCCCCCTGTCCTGGTA CAGCGATCCTGGACTGGCTGGCGTGTCACTGACAGGCG GCCTGAGCTACAAAGAGGACACCAAAGAACTGGTGGTG GCCAAGGCCGGCGTGTACTACGTGTTCTTTCAGCTGGAA CTGCGGAGAGTGGTGGCCGGCGAAGGATCTGGCTCTGT GTCTCTGGCCCTGCATCTGCAGCCTCTGAGATCTGCTGC TGGCGCCGCTGCTCTGGCACTGACAGTGGATCTGCCTCC TGCCAGCAGCGAGGCCCGGAATAGCGCATTTGGGTTTCA AGGCAGGCTGCTGCACCTGTCTGCCGGCCAGAGGCTGG GAGTGCATCTGCACACAGAGGCCAGGGCTAGACACGCC TGGCAGCTGACACAGGGCGCTACAGTGCTGGGCCTGTTC AGAGTGACCCCCGAGATTCCTGCCGGGCTC |

(continued)

| SEQ ID NO: | Description | Sequence |
|---|---|---|
| 118 | nucleotide sequence of anti-CD19(8B8-018) light chain | GACATCGTCATGACCCAGACACCCCTGTCCCTCTCTGTG ACCCCTGGCCAGCCAGCCTCAATTAGCTGCAAGTCCTCT CAAAGTCTGGAGAACCCCAATGGGAACACTTACCTTAAT TGGTATCTGCAGAAACCCGGACAATCCCCTCAACTCCTG ATCTACAGGGTCTCTAAGAGATTCTCAGGCGTGCCAGAT CGCTTTAGCGGTTCCGGGTCTGGCACAGACTTCACCTTG AAGATTAGTCGGGTTGAAGCTGAGGATGTGGGAGTCTA TTACTGTCTGCAGCTCACTCATGTGCCCTACACCTTTGGT CAGGGCACAAAACTGGAGATCAAGCGGACCGTGGCCGC TCCCTCCGTGTTCATCTTCCCACCCTCCGACGAGCAGCT GAAGTCCGGCACCGCCAGCGTGGTGTGCCTGCTGAACA ACTTCTACCCCCGCGAGGCCAAGGTGCAGTGGAAGGTG GACAACGCCCTGCAGTCCGGCAACTCCCAGGAATCCGT GACCGAGCAGGACTCCAAGGACAGCACCTACTCCCTGT CCTCCACCCTGACCCTGTCCAAGGCCGACTACGAGAAGC ACAAGGTGTACGCCTGCGAAGTGACCCACCAGGGCCTG TCCAGCCCCGTGACCAAGTCCTTCAACCGGGGCGAGTGC |
| 104 | Fc hole dimeric 4-1BB (71-248) ligand chain | see Table 1 |
| 119 | anti-CD 19(8B8-018) Fc knob monomeric 4-1-BB (71-248) ligand | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVYTLPPCRD ELTKNQVSLWCLVKGFYPSDIAVEWESNGQPENNYKTTPP VLDSDGSFFLYSKLTVDKSRWQQGNVFSCSVMHEALHNH YTQKSLSLSPGGGGGSGGGGSREGPELSPDDPAGLLDLRQ GMFAQLVAQNVLLIDGPLSWYSDPGLAGVSLTGGLSYKED TKELVVAKAGVYYVFFQLELRRVVAGEGSGSVSLALHLQP LRSAAGAAALALTVDLPPASSEARNSAFGFQGRLLHLSAG QRLGVHLHTEARARHAWQLTQGATVLGLFRVTPEIPAGL |
| 120 | anti-CD 19(8B8-018) light chain | DIVMTQTPLSLSVTPGQPASISCKSSQSLENPNGNTYLNWY LQKPGQSPQLLIYRVSKRFSGVPDRFSGSGSGTDFTLKISRV EAEDVGVYYCLQLTHVPYTFGQGTKLEIKRTVAAPSVFIFP PSDEQLKSGTASVVCLLNNFYPREAKVQWKVDNALQSGN SQESVTEQDSKDSTYSLSSTLTLSKADYEKHKVYACEVTH QGLSSPVTKSFNRGEC |

Table 6 shows the cDNA and amino acid sequences of the monovalent CD19(8B8-018)-targeted 4-1BB ligand (71-248) trimer-containing Fc (kih) fusion antigen binding molecule (Construct 3.12).

**Table 2: Sequences of CD19 (B8-018) targeted human 4-1BB ligand trimer containing Fc (kih) fusion molecule Construct 3.12 (comparative example)**

| SEQ ID NO: | Description | Sequence |
|---|---|---|
| 107 | nucleotide sequence of Fc hole monomeric 4-1BBL (71-248) chain | see Table 2 |
| 121 | nucleotide sequence of anti-CD19(8B8-018) Fc knob dimeric 4-1BB (71-248) ligand | CAGGTCCAGCTGGTGCAGTCCGGCGCCGAGGTCAAGAA ACCCGGGGCTTCTGTGAAGGTTTCATGCAAGGCAAGCG GATACACCTTCACCGACTATATCATGCATTGGGTCAGGC AGGCCCCTGGCCAAGGTCTCGAATGGATGGGCTACATTA ACCCATATAATGATGGCTCCAAATACACCGAGAAGTTTC AGGGAAGAGTCACTATGACATCTGACACCAGTATCAGC ACTGCTTACATGGAGCTGTCCCGCCTTCGGTCTGATGAC ACCGCAGTGTATTACTGTGCCAGGGGCACATATTACTAC GGCTCAGCTCTGTTCGACTATTGGGGGCAGGGAACCACA GTAACCGTGAGCTCCGCTAGCACCAAGGGCCCATCGGT CTTCCCCCTGGCACCCTCCTCCAAGAGCACCTCTGGGGG CACAGCGGCCCTGGGCTGCCTGGTCAAGGACTACTTCCC CGAACCGGTGACGGTGTCGTGGAACTCAGGCGCCCTGA CCAGCGGCGTGCACACCTTCCCGGCTGTCCTACAGTCCT CAGGACTCTACTCCCTCAGCAGCGTGGTGACCGTGCCCT CCAGCAGCTTGGGCACCCAGACCTACATCTGCAACGTG AATCACAAGCCCAGCAACACCAAGGTGGACAAGAAAGT TGAGCCCAAATCTTGTGACAAAACTCACACATGCCCACC GTGCCCAGCACCTGAAGCTGCAGGGGGACCGTCAGTCT TCCTCTTCCCCCCAAAACCCAAGGACACCCTCATGATCT CCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTACACCCTGCCCCCCTGCAGA GATGAGCTGACCAAGAACCAGGTGTCCCTGTGGTGTCTG GTCAAGGGCTTCTACCCCAGCGATATCGCCGTGGAGTGG GAGAGCAACGGCCAGCCTGAGAACAACTACAAGACCAC CCCCCCTGTGCTGGACAGCGACGGCAGCTTCTTCCTGTA CTCCAAACTGACCGTGGACAAGAGCCGGTGGCAGCAGG GCAACGTGTTCAGCTGCAGCGTGATGCACGAGGCCCTG CACAACCACTACACCCAGAAGTCCCTGAGCCTGAGCCC CGGCAGAGAGGGCCCTGAGCTGAGCCCTGATGATCCTG CCGGACTGCTGGACCTGCGGCAGGGAATGTTTGCCCAG CTGGTGGCCCAGAACGTGCTGCTGATCGATGGCCCCCTG TCCTGGTACAGCGATCCTGGACTGGCTGGCGTGTCACTG |

(continued)

| SEQ ID NO: | Description | Sequence |
|---|---|---|
| | | ACAGGCGGCCTGAGCTACAAAGAGGACACCAAAGAACT GGTGGTGGCCAAGGCCGGCGTGTACTACGTGTTCTTTCA GCTGGAACTGCGGAGAGTGGTGGCCGGCGAAGGATCTG GCTCTGTGTCTCTGGCCCTGCATCTGCAGCCTCTGAGAT CTGCTGCTGGCGCCGCTGCTCTGGCACTGACAGTGGATC TGCCTCCTGCCAGCAGCGAGGCCCGGAATAGCGCATTTG GGTTTCAAGGCAGGCTGCTGCACCTGTCTGCCGGCCAGA GGCTGGGAGTGCATCTGCACACAGAGGCCAGGGCTAGA CACGCCTGGCAGCTGACACAGGGCGCTACAGTGCTGGG CCTGTTCAGAGTGACCCCCGAGATTCCAGCAGGCCTGGG AGGCGGCGGATCTGGCGGCGGAGGATCTAGAGAAGGAC CCGAGCTGTCCCCCGACGATCCCGCTGGGCTGCTGGATC TGAGACAGGGCATGTTCGCTCAGCTGGTGGCTCAGAATG TGCTGCTGATTGACGGACCTCTGAGCTGGTACTCCGACC CAGGGCTGGCAGGGGTGTCCCTGACTGGGGGACTGTCCT ACAAAGAAGATACAAAAGAACTGGTGGTGGCTAAAGCT GGGGTGTACTATGTGTTTTTTCAGCTGGAACTGAGGCGG GTGGTGGCTGGGGAGGGCTCAGGATCTGTGTCCCTGGCT CTGCATCTGCAGCCACTGCGCTCTGCAGCAGGGGCTGCA GCACTGGCCCTGACTGTGGACCTGCCCCCAGCTTCTTCC GAGGCCAGAAACAGCGCCTTCGGGTTCCAAGGACGCCT GCTGCATCTGAGCGCCGGACAGCGCCTGGGAGTGCATC TGCATACTGAAGCCAGAGCCCGGCATGCTTGGCAGCTG ACTCAGGGGGCAACTGTGCTGGGACTGTTTCGCGTGACA CCTGAGATCCCAGCCGGGCTC |
| 118 | nucleotide sequence of anti-CD19 (8B8-018) light chain | See Table 5 |
| 109 | Fc hole monomeric 4-1BB ligand (71-248) chain | see Table 2 |
| 122 | anti-CD 19(8B8-018) Fc knob dimeric 4-1BB (71-248) ligand | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVYTLPPCRD ELTKNQVSLWCLVKGFYPSDIAVEWESNGQPENNYKTTPP VLDSDGSFFLYSKLTVDKSRWQQGNVFSCSVMHEALHNH YTQKSLSLSPGREGPELSPDDPAGLLDLRQGMFAQLVAQN VLLIDGPLSWYSDPGLAGVSLTGGLSYKEDTKELVVAKAG VYYVFFQLELRRVVAGEGSGSVSLALHLQPLRSAAGAAAL ALTVDLPPASSEARNSAFGFQGRLLHLSAGQRLGVHLHTE ARARHAWQLTQGATVLGLFRVTPEIPAGLGGGGSGGGGS |

(continued)

| SEQ ID NO: | Description | Sequence |
|---|---|---|
| | | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGL |
| 120 | anti-CD 19(8B8-018) light chain | See Table 5 |

Table 7 shows the cDNA and amino acid sequences of the monovalent CD19 (8B8-2B11)-targeted 4-1BB ligand trimer-containing Fc (kih) fusion antigen binding molecule (Construct 4.11).

**Table 7: Sequences of CD19(2B11)-targeted human 4-1BB ligand trimer containing Fc (kih) fusion molecule Construct 4.11**

| SEQ ID NO: | Description | Sequence |
|---|---|---|
| 101 | nucleotide sequence of Fc hole dimeric 4-1BB ligand (71-248) chain | See Table 1 |
| 123 | nucleotide sequence of anti-CD19(8B8-2B11) Fc knob monomeric 4-1BB (71-248) ligand | CAGGTGCAATTGGTTCAATCTGGTGCTGAAGTAAAAAA ACCGGGCGCTTCCGTTAAAGTGAGCTGCAAAGCATCTGG TTACACCTTCACTGACTATATCATGCACTGGGTTCGTCA GGCCCCGGGCCAGGGTCTGGAGTGGATGGGCTACATTA ACCCATACAACGACGGTTCCAAATATACCGAGAAATTC CAGGGCCGCGTCACGATGACCAGCGACACTTCTATCTCC ACCGCGTACATGGAACTGTCTAGACTGCGTTCTGACGAC ACCGCTGTTTACTATTGTGCACGCGGTACCTACTACTAC GGTCCACAGCTGTTTGATTACTGGGGCCAAGGTACCACG GTGACCGTAAGCTCTGCTAGCACCAAGGGCCCATCGGTC TTCCCCCTGGCACCCTCCTCCAAGAGCACCTCTGGGGGC ACAGCGGCCCTGGGCTGCCTGGTCAAGGACTACTTCCCC GAACCGGTGACGGTGTCGTGGAACTCAGGCGCCCTGAC CAGCGGCGTGCACACCTTCCCGGCTGTCCTACAGTCCTC AGGACTCTACTCCCTCAGCAGCGTGGTGACCGTGCCCTC CAGCAGCTTGGGCACCCAGACCTACATCTGCAACGTGA ATCACAAGCCCAGCAACACCAAGGTGGACAAGAAAGTT GAGCCCAAATCTTGTGACAAAACTCACACATGCCCACC GTGCCCAGCACCTGAAGCTGCAGGGGGACCGTCAGTCT TCCTCTTCCCCCCAAAACCCAAGGACACCCTCATGATCT CCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC |

(continued)

| SEQ ID NO: | Description | Sequence |
| --- | --- | --- |
| | | GGGAGGAGCAGTACAACAGCACGTACCGTGTGGTCAGC<br>GTCCTCACCGTCCTGCACCAGGACTGGCTGAATGGCAAG<br>GAGTACAAGTGCAAGGTCTCCAACAAAGCCCTCGGCGC<br>CCCCATCGAGAAAACCATCTCCAAAGCCAAAGGGCAGC<br>CCCGAGAACCACAGGTGTACACCCTGCCCCCCTGCAGA<br>GATGAGCTGACCAAGAACCAGGTGTCCCTGTGGTGTCTG<br>GTCAAGGGCTTCTACCCCAGCGATATCGCCGTGGAGTGG<br>GAGAGCAACGGCCAGCCTGAGAACAACTACAAGACCAC<br>CCCCCCTGTGCTGGACAGCGACGGCAGCTTCTTCCTGTA<br>CTCCAAACTGACCGTGGACAAGAGCCGGTGGCAGCAGG<br>GCAACGTGTTCAGCTGCAGCGTGATGCACGAGGCCCTG<br>CACAACCACTACACCCAGAAGTCCCTGAGCCTGAGCCC<br>CGGCGGAGGCGGCGGAAGCGGAGGAGGAGGATCCAGA<br>GAGGGCCCTGAGCTGAGCCCTGATGATCCTGCCGGACT<br>GCTGGACCTGCGGCAGGGAATGTTTGCCCAGCTGGTGGC<br>CCAGAACGTGCTGCTGATCGATGGCCCCCTGTCCTGGTA<br>CAGCGATCCTGGACTGGCTGGCGTGTCACTGACAGGCG<br>GCCTGAGCTACAAAGAGGACACCAAAGAACTGGTGGTG<br>GCCAAGGCCGGCGTGTACTACGTGTTCTTTCAGCTGGAA<br>CTGCGGAGAGTGGTGGCCGGCGAAGGATCTGGCTCTGT<br>GTCTCTGGCCCTGCATCTGCAGCCTCTGAGATCTGCTGC<br>TGGCGCCGCTGCTCTGGCACTGACAGTGGATCTGCCTCC<br>TGCCAGCAGCGAGGCCCGGAATAGCGCATTTGGGTTTCA<br>AGGCAGGCTGCTGCACCTGTCTGCCGGCCAGAGGCTGG<br>GAGTGCATCTGCACACAGAGGCCAGGGCTAGACACGCC<br>TGGCAGCTGACACAGGGCGCTACAGTGCTGGGCCTGTTC<br>AGAGTGACCCCCGAGATTCCTGCCGGGCTC |

(continued)

| SEQ ID NO: | Description | Sequence |
|---|---|---|
| 124 | nucleotide sequence of anti-CD19(8B8-2B11) light chain | GATATTGTCATGACTCAAACTCCACTGTCTCTGTCCGTG ACCCCGGGTCAGCCAGCGAGCATTTCTTGCAAATCCAGC CAATCTCTGGAAACCTCCACCGGCACCACGTACCTGAAC TGGTATCTCCAGAAACCGGGTCAGAGCCCGCAGCTGCT GATCTACCGTGTATCTAAGCGCTTCTCCGGCGTTCCTGA TCGTTTCAGCGGTTCTGGATCCGGCACCGACTTTACTCT GAAAATCAGCCGTGTGGAAGCTGAAGACGTTGGCGTCT ACTATTGTCTGCAGCTGCTGGAAGATCCATACACCTTCG GTCAAGGAACTAAACTGGAAATTAAACGTACGGTGGCT GCACCATCTGTCTTCATCTTCCCGCCATCTGATGAGCAG TTGAAATCTGGAACTGCCTCTGTTGTGTGCCTGCTGAAT AACTTCTATCCCAGAGAGGCCAAAGTACAGTGGAAGGT GGATAACGCCCTCCAATCGGGTAACTCCCAGGAGAGTG TCACAGAGCAGGACAGCAAGGACAGCACCTACAGCCTC AGCAGCACCCTGACGCTGAGCAAAGCAGACTACGAGAA ACACAAAGTCTACGCCTGCGAAGTCACCCATCAGGGCC TGAGCTCGCCCGTCACAAAGAGCTTCAACAGGGGAGAG TGTGATATTGTCATGACTCAAACTCCACTGTCTCTGTCCG TGACCCCGGGTCAGCCAGCGAGCATTTCTTGCAAATCCA GCCAATCTCTGGAAACCTCCACCGGCACCACGTACCTGA ACTGGTATCTCCAGAAACCGGGTCAGAGCCCGCAGCTG CTGATCTACCGTGTATCTAAGCGCTTCTCCGGCGTTCCTG ATCGTTTCAGCGGTTCTGGATCCGGCACCGACTTTACTC TGAAAATCAGCCGTGTGGAAGCTGAAGACGTTGGCGTC TACTATTGTCTGCAGCTGCTGGAAGATCCATACACCTTC GGTCAAGGAACGAAACTGGAAATTAAACGTACGGTGGC TGCACCATCTGTCTTCATCTTCCCGCCATCTGATGAGCA GTTGAAATCTGGAACTGCCTCTGTTGTGTGCCTGCTGAA TAACTTCTATCCCAGAGAGGCCAAAGTACAGTGGAAGG TGGATAACGCCCTCCAATCGGGTAACTCCCAGGAGAGT GTCACAGAGCAGGACAGCAAGGACAGCACCTACAGCCT CAGCAGCACCCTGACGCTGAGCAAAGCAGACTACGAGA AACACAAAGTCTACGCCTGCGAAGTCACCCATCAGGGC CTGAGCTCGCCCGTCACAAAGAGCTTCAACAGGGGAGA GTGT |
| 104 | Fc hole dimeric 4-1BB ligand (71-248) chain | See Table 1 |

(continued)

| SEQ ID NO: | Description | Sequence |
|---|---|---|
| 125 | anti- CD19(8B8-2B11) Fc knob monomeric 4-1BB ligand (71-248) | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGPQLFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVYTLPPCRD ELTKNQVSLWCLVKGFYPSDIAVEWESNGQPENNYKTTPP VLDSDGSFFLYSKLTVDKSRWQQGNVFSCSVMHEALHNH YTQKSLSLSPGGGGGSGGGGSREGPELSPDDPAGLLDLRQ GMFAQLVAQNVLLIDGPLSWYSDPGLAGVSLTGGLSYKED TKELVVAKAGVYYVFFQLELRRVVAGEGSGSVSLALHLQP LRSAAGAAALALTVDLPPASSEARNSAFGFQGRLLHLSAG QRLGVHLHTEARARHAWQLTQGATVLGLFRVTPEIPAGL |
| 126 | anti- CD19(8B8-2B11) light chain | DIVMTQTPLSLSVTPGQPASISCKSSQSLETSTGTTYLNWYL QKPGQSPQLLIYRVSKRFSGVPDRFSGSGSGTDFTLKISRVE AEDVGVYYCLQLLEDPYTFGQGTKLEIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC |

Table 8 shows the cDNA and amino acid sequences of the monovalent CD19(8B8-2B11) targeted 4-1BB ligand trimer-containing Fc (kih) fusion antigen binding molecule (Construct 4.12).

**Table 8: Sequences of CD19(8B8-2B11)-targeted human 4-1BB ligand trimer containing Fc (kih) fusion molecule Construct 4.12 (comparative example)**

| SEQ ID NO: | Description | Sequence |
|---|---|---|
| 107 | nucleotide sequence of Fc hole monomeric 4-1BB ligand (71-248) chain | See Table 2 |

(continued)

| SEQ ID NO: | Description | Sequence |
|---|---|---|
| 127 | nucleotide sequence of anti-CD19 (8B8-2B11) Fc knob dimeric 4-1BB (71-248) ligand | CAGGTGCAATTGGTTCAATCTGGTGCTGAAGTAAAAAA ACCGGGCGCTTCCGTTAAAGTGAGCTGCAAAGCATCTGG TTACACCTTCACTGACTATATCATGCACTGGGTTCGTCA GGCCCCGGGCCAGGGTCTGGAGTGGATGGGCTACATTA ACCCATACAACGACGGTTCCAAATATACCGAGAAATTC CAGGGCCGCGTCACGATGACCAGCGACACTTCTATCTCC ACCGCGTACATGGAACTGTCTAGACTGCGTTCTGACGAC ACCGCTGTTTACTATTGTGCACGCGGTACCTACTACTAC GGTCCACAGCTGTTTGATTACTGGGGCCAAGGTACCACG GTGACCGTAAGCTCTGCTAGCACCAAGGGCCCATCGGTC TTCCCCCTGGCACCCTCCTCCAAGAGCACCTCTGGGGGC ACAGCGGCCCTGGGCTGCCTGGTCAAGGACTACTTCCCC GAACCGGTGACGGTGTCGTGGAACTCAGGCGCCCTGAC CAGCGGCGTGCACACCTTCCCGGCTGTCCTACAGTCCTC AGGACTCTACTCCCTCAGCAGCGTGGTGACCGTGCCCTC CAGCAGCTTGGGCACCCAGACCTACATCTGCAACGTGA ATCACAAGCCCAGCAACACCAAGGTGGACAAGAAAGTT GAGCCCAAATCTTGTGACAAAACTCACACATGCCCACC GTGCCCAGCACCTGAAGCTGCAGGGGGACCGTCAGTCT TCCTCTTCCCCCCAAAACCCAAGGACACCCTCATGATCT CCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTACACCCTGCCCCCCTGCAGA GATGAGCTGACCAAGAACCAGGTGTCCCTGTGGTGTCTG GTCAAGGGCTTCTACCCCAGCGATATCGCCGTGGAGTGG GAGAGCAACGGCCAGCCTGAGAACAACTACAAGACCAC CCCCCCTGTGCTGGACAGCGACGGCAGCTTCTTCCTGTA CTCCAAACTGACCGTGGACAAGAGCCGGTGGCAGCAGG GCAACGTGTTCAGCTGCAGCGTGATGCACGAGGCCCTG CACAACCACTACACCCAGAAGTCCCTGAGCCTGAGCCC CGGCAGAGAGGGCCCTGAGCTGAGCCCTGATGATCCTG CCGGACTGCTGGACCTGCGGCAGGGAATGTTTGCCCAG CTGGTGGCCCAGAACGTGCTGCTGATCGATGGCCCCCTG TCCTGGTACAGCGATCCTGGACTGGCTGGCGTGTCACTG ACAGGCGGCCTGAGCTACAAAGAGGACACCAAAGAACT GGTGGTGGCCAAGGCCGGCGTGTACTACGTGTTCTTTCA GCTGGAACTGCGGAGAGTGGTGGCCGGCGAAGGATCTG GCTCTGTGTCTCTGGCCCTGCATCTGCAGCCTCTGAGAT CTGCTGCTGGCGCCGCTGCTCTGGCACTGACAGTGGATC TGCCTCCTGCCAGCAGCGAGGCCCGGAATAGCGCATTTG GGTTTCAAGGCAGGCTGCTGCACCTGTCTGCCGGCCAGA GGCTGGGAGTGCATCTGCACACAGAGGCCAGGGCTAGA CACGCCTGGCAGCTGACACAGGGCGCTACAGTGCTGGG |

(continued)

| SEQ ID NO: | Description | Sequence |
|---|---|---|
| | | CCTGTTCAGAGTGACCCCCGAGATTCCAGCAGGCCTGGG AGGCGGCGGATCTGGCGGCGGAGGATCTAGAGAAGGAC CCGAGCTGTCCCCCGACGATCCCGCTGGGCTGCTGGATC TGAGACAGGGCATGTTCGCTCAGCTGGTGGCTCAGAATG TGCTGCTGATTGACGGACCTCTGAGCTGGTACTCCGACC CAGGGCTGGCAGGGGTGTCCCTGACTGGGGGACTGTCCT ACAAAGAAGATACAAAAGAACTGGTGGTGGCTAAAGCT GGGGTGTACTATGTGTTTTTCAGCTGGAACTGAGGCGG GTGGTGGCTGGGGAGGGCTCAGGATCTGTGTCCCTGGCT CTGCATCTGCAGCCACTGCGCTCTGCAGCAGGGGCTGCA GCACTGGCCCTGACTGTGGACCTGCCCCCAGCTTCTTCC GAGGCCAGAAACAGCGCCTTCGGGTTCCAAGGACGCCT GCTGCATCTGAGCGCCGGACAGCGCCTGGGAGTGCATC TGCATACTGAAGCCAGAGCCCGGCATGCTTGGCAGCTG ACTCAGGGGGCAACTGTGCTGGGACTGTTTCGCGTGACA CCTGAGATCCCAGCCGGGCTC |
| 124 | nucleotide sequence of anti-CD19 (8B8-2B11) light chain | See Table 7 |
| 109 | Fc hole monomeric 4-1BB ligand (71-248) chain | See Table 2 |
| 128 | anti- CD19 (8B8-2B11) Fc knob dimeric 4-1BB ligand (71-248) | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGPQLFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVYTLPPCRD ELTKNQVSLWCLVKGFYPSDIAVEWESNGQPENNYKTTPP VLDSDGSFFLYSKLTVDKSRWQQGNVFSCSVMHEALHNH YTQKSLSLSPGREGPELSPDDPAGLLDLRQGMFAQLVAQN VLLIDGPLSWYSDPGLAGVSLTGGLSYKEDTKELVVAKAG VYYVFFQLELRRVVAGEGSGSVSLALHLQPLRSAAGAAAL ALTVDLPPASSEARNSAFGFQGRLLHLSAGQRLGVHLHTE ARARHAWQLTQGATVLGLFRVTPEIPAGLGGGGSGGGGS REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGL |
| 126 | anti- CD19 (8B8-2B11) light chain | See Table 7 |

### 1.3. Generation of anti-CD19 binders 8B8-018 and 8B8-2B11

### 1.3.1 Generation of anti-CD19 clone 8B8-018

a) Immunization and generation of mouse anti-human CD19 antibodies (hybridomas)

Balb/c mice were immunized six times and boosted with CD19-transfected HEK293 cells (mean receptor density 35,000 per cell). The immune response was monitored by testing serum samples with a CD19-cell-ELISA on human CD19-transfected NIH-3T3 cells. Spleen cells from mice with sufficient titers of anti-human CD19 antibody were used for immortalization by fusion with mouse myeloma cell line P3X63 Ag8.653. Three fusions were carried out and hybridoma supernatants screened by cell-ELISA on human CD19-transfected NIH-3T3 cells and FACS binding assay using Daudi (CD19+) and CD19- cells for anti-human CD19 specific antibodies (see Example 1 of WO 2011/147834).

b) Hybridoma screening and cell biological functional evaluation of anti-CD19 antibodies

A cell ELISA was applied for screening of hybridomas, and to identify those hybridomas that secrete antibodies against human-CD19. NIH3T3 cells transfected with human-CD19 were used as positive cells; non-transfected NIH3T3 cells were used as negative control cells. For the assessment of the positive hybridomas the OD ratio between transfected and non-transfected NIH3T3 cells was quantified.

- Culture Medium: DMEM high glucose (4.5 mg/ml), 10 % FCS, Na-Pyruvate, NEAA, Glutamine
- Antibodies positive control: anti CD19 monoclonal antibody (IgG1) Pharmingen Cat# 555409 c = 1 mg/ml
- Detection antibody: Goat anti-Mouse IgG (H+L) HRP Conjugate Bio-Rad Cat# 170-06516
- Dilution 1: 2000 in 1x ELISA Blocking Reagent
- Other reagents: Fibronectin Roche Cat# 838039 c = 1 mg/ml
- Glutardialdehyde: 25 % stock solution // Grade Agar Scientific #R102 final concentration: 0.05 % in PBS
- ELISA Blocking Reagent: 10x stock solution // Roche Cat# 1112589
- TMB substrate: Roche Cat# 11432559
- Stop Solution: 1 M H2SO4
- BioRad Cat# 170-6516 Dilution 1: 2000 in 1x ELISA Blocking Reagent

Day 1:

- Fibronectin coating: 5 µg/cm$^2$ in PBS; 96well plate = 32 cm$^2$; 160 µg/plate in 6 ml
- PBS, 50 µl/well
- incubate 45 min at RT, aspirate coating solution
- Seed 1.25 x 104 cells/well in 50 µl culture medium in a 96well plate
- incubate 40 hours at 37 °C
- add to upper half of the plate: NIH3T3 cells expressing CD19
- add to lower half of the plate: non-transfected NIH3T3 cells

Day 3:

- Addition of positive control antibody or samples (supernatant or mouse serum) in 50 µl culture medium
- incubate for 2 h at 4 °C
- Remove medium, fix cells with 100 µl Glutardialdehyde (0.05 % in PBS)
- Wash two times with 200 µl PBS
- Addition of detection antibody 1:2000, 50 µl/well
- incubate 2 h at RT
- wash three times with 200 µl PBS
- add 50 µl TMB, incubate for 30 min. at RT,
- stop by addition of 25 µl 1 M H2SO4; read extinction at 450nm/620nm
- Calculation of results: ratio OD NIH3T3 CD19 : OD NIH3T3 non-transfected

The selected antibody demonstrated specific binding to CD19 transfected NIH3T3 cells as compared to untransfected NIH3T3 cells (see Example 2 of WO 2011/147834).

c) Humanization of anti-CD 19 antibody

The CD19 binding specificity of the murine antibody was transferred onto a human acceptor framework to eliminate potential immunogenicity issues arising from sequence stretches that the human body will recognize as foreign. This was done by engrafting the entire complementary determining regions (CDR) of the murine (donor) antibody onto a human (acceptor) antibody framework, and is called CDR-grafting or antibody humanization.

The murine amino acid sequence was aligned with a collection of human germ-line antibody V genes, and sorted according to sequence identity and homology. Before selecting one particular acceptor sequence, the so-called canonical loop structures of the donor antibody have to be determined (Morea, V., et al., Methods, Vol 20, Issue 3 (2000) 267-279). These canonical loop structures are determined by the type of residues present at the so-called canonical positions. These positions lie (partially) outside of the CDR regions, and have to be kept functionally equivalent in the final construct in order to retain the CDR conformation of the parental (donor) antibody. The human germ-line sequence VBASE_VH1_1 was chosen as the acceptor for the heavy chain and sequence VBASE_VK2_5 was chosen for the light chain.

It was found that the wild-type humanized anti-human CD19 antibody 8B8 has three deamidation hotspots in the HVR-L1: **N**S**N**G**N**T (SEQ ID NO: 129). Additionally it was found that in the HVR-H2 a further deamidation hotspot is present: KF**N**G (SEQ ID NO: 130). To address the deamidation hotspot in the HVR-H2 an N (Asn) to Q (Gln) point mutation at position 64 (numbering according to Kabat) has been introduced. To address the deamidation hotspots in the light chain and to obtain a humanized anti-human CD19 antibody with improved deamidation stability a single mutation at position 27e from S (serine) to P (proline) (numbering according to Kabat) was introduced. Thus, clone 8B8-018 with the CDRs as shown in Table 16 below was generated.

**Table 9: Comparison of 8B8-018 with humanized wild-type CD19 antibody 8B8**

| variant → ↓ parameter | wt 8B8 | 8B8-018 |
|---|---|---|
| $K_D$ (BIAcore) [nM] | 5 | 6 |
| $t_{1/2}$ [min] | - | 43.6 |
| human CD19 binding after pH 7.4 incubation [%] | 46 | 95 |
| human CD19 binding after pH 6.0 incubation [%] | 90 | 99 |
| SEC main peak after incubation [%] | > 95 | > 95 |

Additionally, 8B8-018 maintains the cross-reactivity to cynomolgus CD19 as shown in the following **Table 10.**

| EC50 [μg/ml] | wt 8B8 | 8B8-018 |
|---|---|---|
| huCD19 ECD | 0.087 | 0.084 |
| cyCD19 ECD | 0.313 | 0.255 |

**1.3.2 Preparation, purification and characterization of CD19 antigen Fc fusion for phage display campaign**

In order to express and purify the human and cynomolgus CD19 ectodomain in a monomeric state (human CD19 see SEQ ID NO:64), the respective DNA fragment was fused to a human IgG1 Fc gene segment containing the "knob" mutations (human: SEQ ID NO: 132; cynomolgus: SEQ ID NO: 135) and was transfected with an "Fc-hole" (SEQ ID NO: 131) counterpart (Merchant et al., 1998). An IgA cleavage site (PTPPTP) was introduced between the antigen ectodomain and the Fc knob chain. An Avi tag for directed biotinylation was introduced at the C-terminus of the antigen-Fc knob chain and mutations H435R and Y436F were introduced in the Fc hole for purification purposes (Jendeberg L. et al, J. Immunological methods, 1997). Combination of the antigen-Fc knob chain containing the S354C/T366W mutations (human: SEQ ID NO: 134; cynomolgus: SEQ ID NO: 136), with a Fc hole chain containing the Y349C/T366S/L368A/Y407V mutations (SEQ ID NO: 133) allows generation of a heterodimeric Fc fusion fragment which includes a single copy of the CD19 ectodomain (in analogy to the 4-1BB construct in **Figure 3C**). Table 13 lists the cDNA and amino acid sequences of the antigen Fc-fusion construct.

**Table 11: cDNA and Amino acid sequences of monomeric human and cynomolgus CD19 antigen Fc(kih) fusion molecule**

| SEQ ID NO: | Antigen | Sequence |
|---|---|---|
| 131 | Nucleotide sequence of Fc hole chain with HYRF mutation | GACAAAACTCACACATGCCCACCGTGCCCAGCACCTGAAG CTGCAGGGGGACCGTCAGTCTTCCTCTTCCCCCCAAAACCC AAGGACACCCTCATGATCTCCCGGACCCCTGAGGTCACATG CGTGGTGGTGGACGTGAGCCACGAAGACCCTGAGGTCAAG TTCAACTGGTACGTGGACGGCGTGGAGGTGCATAATGCCA AGACAAAGCCGCGGGAGGAGCAGTACAACAGCACGTACC GTGTGGTCAGCGTCCTCACCGTCCTGCACCAGGACTGGCTG AATGGCAAGGAGTACAAGTGCAAGGTCTCCAACAAAGCCC TCGGCGCCCCCATCGAGAAAACCATCTCCAAAGCCAAAGG GCAGCCCCGAGAACCACAGGTGTGCACCCTGCCCCCATCC CGGGATGAGCTGACCAAGAACCAGGTCAGCCTCTCGTGCG CAGTCAAAGGCTTCTATCCCAGCGACATCGCCGTGGAGTG GGAGAGCAATGGGCAGCCGGAGAACAACTACAAGACCAC GCCTCCCGTGCTGGACTCCGACGGCTCCTTCTTCCTCGTGA GCAAGCTCACCGTGGACAAGAGCAGGTGGCAGCAGGGGA ACGTCTTCTCATGCTCCGTGATGCATGAGGCTCTGCACAAC CGCTTCACGCAGAAGAGCCTCTCCCTGTCTCCGGGTAAA |
| 132 | Nucleotide sequence of human CD19 antigen Fc knob chain avi tag | CCCGAGGAACCCCTGGTCGTGAAGGTGGAAGAGGGCGACA ATGCCGTGCTGCAGTGCCTGAAGGGCACCTCCGATGGCCCT ACCCAGCAGCTGACCTGGTCCAGAGAGAGCCCCCTGAAGC CCTTCCTGAAGCTGTCTCTGGGCCTGCCTGGCCTGGGCATC CATATGAGGCCTCTGGCCATCTGGCTGTTCATCTTCAACGT GTCCCAGCAGATGGGCGGCTTCTACCTGTGTCAGCCTGGCC CCCCATCTGAGAAGGCTTGGCAGCCTGGCTGGACCGTGAA |

(continued)

| SEQ ID NO: | Antigen | Sequence |
|---|---|---|
| | | CGTGGAAGGATCCGGCGAGCTGTTCCGGTGGAACGTGTCC GATCTGGGCGGCCTGGGATGCGGCCTGAAGAACAGATCTA GCGAGGGCCCCAGCAGCCCCAGCGGCAAACTGATGAGCCC CAAGCTGTACGTGTGGGCCAAGGACAGACCCGAGATCTGG GAGGGCGAGCCTCCTTGCCTGCCCCCTAGAGACAGCCTGA ACCAGAGCCTGAGCCAGGACCTGACAATGGCCCCTGGCAG CACACTGTGGCTGAGCTGTGGCGTGCCACCCGACTCTGTGT CTAGAGGCCCTCTGAGCTGGACCCACGTGCACCCTAAGGG CCCTAAGAGCCTGCTGAGCCTGGAACTGAAGGACGACAGG CCCGCCAGAGATATGTGGGTCATGGAAACCGGCCTGCTGC TGCCTAGAGCCACAGCCCAGGATGCCGGCAAGTACTACTG CCACAGAGGCAACCTGACCATGAGCTTCCACCTGGAAATC ACCGCCAGACCCGTGCTGTGGCACTGGCTGCTGAGAACAG GCGGCTGGAAGGTCGACGCTAGCGGTGGTAGTCCGACACC TCCGACACCCGGGGGTGGTTCTGCAGACAAAACTCACACA TGCCCACCGTGCCCAGCACCTGAAGCCGCAGGGGGACCGT CAGTCTTCCTCTTCCCCCCAAAACCCAAGGACACCCTCATG ATCTCCCGGACCCCTGAGGTCACATGCGTGGTGGTGGACGT GAGCCACGAAGACCCTGAGGTCAAGTTCAACTGGTACGTG GACGGCGTGGAGGTGCATAATGCCAAGACAAAGCCGCGGG AGGAGCAGTACAACAGCACGTACCGTGTGGTCAGCGTCCT CACCGTCCTGCACCAGGACTGGCTGAATGGCAAGGAGTAC AAGTGCAAGGTCTCCAACAAAGCCCTCGGAGCCCCCATCG AGAAAACCATCTCCAAAGCCAAAGGGCAGCCCCGAGAACC ACAGGTGTACACCCTGCCCCCATGCCGGGATGAGCTGACC AAGAACCAGGTCAGCCTGTGGTGCCTGGTCAAAGGCTTCT ATCCCAGCGACATCGCCGTGGAGTGGGAGAGCAATGGGCA GCCGGAGAACAACTACAAGACCACGCCTCCCGTGCTGGAC TCCGACGGCTCCTTCTTCCTCTACAGCAAGCTCACCGTGGA CAAGAGCAGGTGGCAGCAGGGGAACGTCTTCTCATGCTCC GTGATGCATGAGGCTCTGCACAACCACTACACGCAGAAGA GCCTCTCCCTGTCTCCGGGTAAATCCGGAGGCCTGAACGAC ATCTTCGAGGCCCAGAAGATTGAATGGCACGAG |
| 133 | Fc hole chain with HYRF mutation | DKTHTCPPCPAPEAAGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALGAPIEKTISKAKGQPREP QVCTLPPSRDELTKNQVSLSCAVKGFYPSDIAVEWESNGQPE NNYKTTPPVLDSDGSFFLVSKLTVDKSRWQQGNVFSCSVMHE ALHNRFTQKSLSLSPGK |

(continued)

| SEQ ID NO: | Antigen | Sequence |
|---|---|---|
| 134 | human CD19 antigen Fc knob chain avi tag | PEEPLVVKVEEGDNAVLQCLKGTSDGPTQQLTWSRESPLKPF LKLSLGLPGLGIHMRPLAIWLFIFNVSQQMGGFYLCQPGPPSE KAWQPGWTVNVEGSGELFRWNVSDLGGLGCGLKNRSSEGPS SPSGKLMSPKLYVWAKDRPEIWEGEPPCLPPRDSLNQSLSQDL TMAPGSTLWLSCGVPPDSVSRGPLSWTHVHPKGPKSLLSLEL KDDRPARDMWVMETGLLLPRATAQDAGKYYCHRGNLTMSF HLEITARPVLWHWLLRTGGWKVDASGGSPTPPTPGGGSADK THTCPPCPAPEAAGGPSVFLFPPKPKDTLMISRTPEVTCVVVD VSHEDPEVKFNWYVDGVEVHNAKTKPREEQYNSTYRVVSVL TVLHQDWLNGKEYKCKVSNKALGAPIEKTISKAKGQPREPQV YTLPPCRDELTKNQVSLWCLVKGFYPSDIAVEWESNGQPENN YKTTPPVLDSDGSFFLYSKLTVDKSRWQQGNVFSCSVMHEAL HNHYTQKSLSLSPGKSGGLNDIFEAQKIEWHE |
| 135 | Nucleotide sequence of cynomolgus CD19 antigen Fc knob chain avi tag | CCCCAGGAACCCCTGGTCGTGAAGGTGGAAGAGGGCGACA ATGCCGTGCTCCAGTGCCTGGAAGGCACCTCCGATGGCCCT |

(continued)

| SEQ ID NO: | Antigen | Sequence |
|---|---|---|
| | | ACACAGCAGCTCGTGTGGTGCAGAGACAGCCCCTTCGAGCCCTTCCTGAACCTGTCTCTGGGCCTGCCTGGCATGGGCATCAGAATGGGCCCTCTGGGCATCTGGCTGCTGATCTTCAACGTGTCCAACCAGACCGGCGGCTTCTACCTGTGTCAGCCTGGCCTGCCAAGCGAGAAGGCTTGGCAGCCTGGATGGACCGTGTCCGTGGAAGGATCTGGCGAGCTGTTCCGGTGGAACGTGTCCGATCTGGGCGGCCTGGGATGCGGCCTGAAGAACAGAAGCAGCGAGGGCCCTAGCAGCCCCAGCGGCAAGCTGAATAGCAGCCAGCTGTACGTGTGGGCCAAGGACAGACCCGAGATGTGGGAGGGCGAGCCTGTGTGTGGCCCCCCTAGAGATAGCCTGAACCAGAGCCTGAGCCAGGACCTGACAATGGCCCCTGGCAGCACACTGTGGCTGAGCTGTGGCGTGCCACCCGACTCTGTGTCCAGAGGCCCTCTGAGCTGGACACACGTGCGGCCAAAGGGCCCTAAGAGCAGCCTGCTGAGCCTGGAACTGAAGGACGACCGGCCCGACCGGGATATGTGGGTGGTGGATACAGGCCTGCTGCTGACCAGAGCCACAGCCCAGGATGCCGGCAAGTACTACTGCCACAGAGGCAACTGGACCAAGAGCTTTTACCTGGAAATCACCGCCAGACCCGCCCTGTGGCACTGGCTGCTGAGAATCGGAGGCTGGAAGGTCGACGCTAGCGGTGGTAGTCCGACACCTCCGACACCCGGGGGTGGTTCTGCAGACAAAACTCACACATGCCCACCGTGCCCAGCACCTGAAGCCGCAGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGAGCCCCCATCGAGAAAACCATCTCCAAAGCCAAAGGGCAGCCCCGAGAACCACAGGTGTACACCCTGCCCCCATGCCGGGATGAGCTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAATCCGGAGGCCTGAACGACATCTTCGAGGCCCAGAAGATTGAATGGCACGAG |

(continued)

| SEQ ID NO: | Antigen | Sequence |
|---|---|---|
| 136 | cynomolgus CD19 antigen Fc knob chain avi tag | PQEPLVVKVEEGDNAVLQCLEGTSDGPTQQLVWCRDSPFEPF LNLSLGLPGMGIRMGPLGIWLLIFNVSNQTGGFYLCQPGLPSE KAWQPGWTVSVEGSGELFRWNVSDLGGLGCGLKNRSSEGPS SPSGKLNSSQLYVWAKDRPEMWEGEPVCGPPRDSLNQSLSQD LTMAPGSTLWLSCGVPPDSVSRGPLSWTHVRPKGPKSSLLSLE LKDDRPDRDMWVVDTGLLLTRATAQDAGKYYCHRGNWTKS FYLEITARPALWHWLLRIGGWKVDASGGSPTPPTPGGGSADK THTCPPCPAPEAAGGPSVFLFPPKPKDTLMISRTPEVTCVVVD VSHEDPEVKFNWYVDGVEVHNAKTKPREEQYNSTYRVVSVL TVLHQDWLNGKEYKCKVSNKALGAPIEKTISKAKGQPREPQV YTLPPCRDELTKNQVSLWCLVKGFYPSDIAVEWESNGQPENN YKTTPPVLDSDGSFFLYSKLTVDKSRWQQGNVFSCSVMHEAL HNHYTQKSLSLSPGKSGGLNDIFEAQKIEWHE |

For the production of the monomeric antigen/Fc fusion molecules, exponentially growing suspension CHO cells were co-transfected with two plasmids encoding the two components of fusion protein (knob and hole chains) using standard methods.

Secreted protein was purified from cell culture supernatant by affinity chromatography using Protein A, followed by size exclusion chromatography. For affinity chromatography, the supernatant was loaded on a MabSelect Sure column volume (CV) = 5-15 mL, resin from GE Healthcare) equilibrated with Sodium Phosphate (20 mM), Sodium Citrate (20 mM), 0.5M sodium chloride buffer (pH 7.5). Unbound protein was removed by washing with at least 6 column volumes of the same buffer. The bound protein was eluted using a linear gradient; step 1, 10 CV from 0 to 60% elution buffer (20 mM sodium citrate, 500 mM Sodium chloride buffer (pH 2.5)); step 2, 2 CV from 60 to 100% elution buffer. For the linear gradient an additional 2 column volumes step elution with 100% elution buffer was applied.

The pH of collected fractions was adjusted by adding 1/40 (v/v) of 2M Tris, pH8.0. The protein was concentrated and filtered prior to loading on a HiLoad Superdex 200 column (GE Healthcare) equilibrated with 2mM MOPS, 150 mM sodium chloride, 0.02 % (w/v) sodium azide solution of pH 7.4.

**Table 12** summarizes the yield and final monomer content of monomeric human and cynomolgus CD19 antigen Fc(kih) fusion protein.

**Table 12- Biochemical analysis of monomeric human and cynomolgus CD19 antigen Fc(kih) fusion protein**

| Construct | Monomer [%] (SEC) | Yield [mg/l] |
|---|---|---|
| monomeric human CD19 Fc(kih) fusion protein | 91 | 0.2 |
| monomeric cynomolgus CD19 Fc(kih) fusion protein | 95 | 3.56 |

Part of the purified antigen was in vitro biotinylated using the BirA biotin-protein ligase standard reaction kit (Avidity, Cat. # BirA500) according to the manufacturer's instructions. The biotinylation degree for the human CD19-containing fusion was 94 %, for the respective cynomolgus CD 19 construct 100%. The biotinylated protein was then used for selection, screening and characterization of affinity-matured 8B8-derived clones devoid of the de-amidation hotspots N27d and N28. Two phage display libraries were generated in which a) both asparagine residues at positions 27d and 28 were eliminated and b) additional CDRs of heavy and light chain were randomized in order to select for 8B8 variants with an improved affinity.

### 1.3.3 Generation of 8B8 affinity maturation libraries devoid of CDR-L1 hotspots

Generation of affinity-matured 8B8-derived antibodies without the de-amidation sites N27d and N28, located in CDR-L1, was carried out by phage display using standard protocols (Silacci et al, 2005). In a first step, the VL and VH DNA sequences of the humanized parental clone 8B8 (SEQ ID NO: 137 and SEQ ID NO: 138) were cloned into a phagemid which was then used as a template for randomization. In a next step, two libraries were generated for the selection of favourable clones by phage display. In order to eliminate the above-mentioned hotspot positions, a LCDR1 randomization primer (SEQ ID NO: 139) that only allowed amino acids S T Q E at positions 27d and 28 was used for both libraries. Maturation library 1 was randomized in CDR1 and 2 of both the light and the heavy chain, while maturation library 2 was randomized in CDR1 and 3 of the light chain and in CDR3 of the heavy chain. For the generation of the maturation library 1, randomized in CDR1 and 2 of both the light and the heavy chain, three fragments were assembled by "splicing by overlapping extension" (SOE) PCR and cloned into the phage vector. The following primer combinations were used to generate the library fragments: fragment 1 (LMB3 (SEQ ID NO: 144) and CD19 L1 reverse random (SEQ ID NO: 139), fragment 2 (CD19 L2 forward random (SEQ ID NO: 140) and CD19 H1 reverse random (SEQ ID NO: 141), and fragment 3 (CD19 H2 forward random (SEQ ID NO: 142) and CD19 H3 reverse constant (SEQ ID NO: 143) **(Table 13).** After assembly of sufficient amounts of full length randomized fragment, it was digested with *Nco*I/*Nhe*I alongside with identically treated acceptor phagemid vector. A 3-fold molar excess of library insert was ligated with 10 μg of phagemid vector. Purified ligations were used for 20 transformations resulting in 2 × 10 exp9 transformants. Phagemid particles displaying the 8B8 affinity maturation library were rescued and purified by PEG/NaCl purification to be used for selections.

The generation of the second library, randomized in CDR1 and 3 of the light chain and in CDR3 of the heavy chain, was done similarly. The following primer combinations were used to generate the library fragments: fragment 1 (LMB3 (SEQ ID NO: 144) and CD19 L1 reverse random (SEQ ID NO: 139), fragment 2 (CD19 L1 forward constant (SEQ ID NO 145) and CD19 L3 reverse random (SEQ ID NO: 146, and fragment 3 (CD19 L3 forward constant (SEQ ID NO: 147) and CD19 H3 reverse random (SEQ ID NO: 148) **(Table 14).** After assembly of sufficient amounts of full length randomized fragment, it was digested with *Nco*I/*Kpn*I alongside with identically treated acceptor phagemid vector. A 3-fold molar excess of library insert was ligated with 20ug of phagemid vector. Purified ligations were used for 40 transformations resulting in 2 × 10 exp9 transformants. Phagemid particles displaying the 8B8 affinity maturation library were rescued and purified by PEG/NaCl purification to be used for selections.

**Table 13: Primers for 8B8 affinity maturation and hotspot removal library L1_L2 / H1_H2**

| SEQ ID | Name | Sequence |
|---|---|---|
| 139 | CD19 L1 reverse random | CAG CTG CGG GCT CTG ACC CGG TTT CTG GAG ATA CCA GTT CAG 1 CGT 2 GCC 3 GGA 4 TTC CAG AGA TTG GCT GGA TTT GCA AGA AAT G<br>1: 40% Y, 6% A/S/T/G/P/D/N/E/Q/V, 2: 40% N, 6% A/S/T/Y/G/P/D/E/Q/V, 3: 25% S/T/Q/E, 4: 25% S/T/Q/E |
| 140 | CD19 L2 forward random | CTC CAG AAA CCG GGT CAG AGC CCG CAG CTG CTG ATC TAC 5 GTA TCT 6 CGC 7 8 GGC GTT 9 GAT CGT TTC AGC GGT TCT GGA TCC GGC ACC<br>5: 30% R, 20% E, 5% A/S/T/Y/G/P/D/N/Q/V. 6: 30% K, 20% S, 5% A/N/T/Y/G/P/D/E/Q/V, 7: 40% F, 5% A/S/T/Y/G/P/D/E/Q/V/I/L, 8: 40% S, 6.6% A/T/Y/G/P/D/E/Q/V, 9: 50% P, 50% L |
| 141 | CD19 H1 reverse random | CAT CCA CTC CAG ACC CTG GCC CGG GGC CTG ACG AAC CCA 10 CAT 11 12 13 14 GAA 15 GTA ACC AGA TGC TTT GCA GCT CAC TTT AAC GGA AGC<br>10: 52% H, 4% G/A/S/P/T/N/Y/D/E/Q/V/I, 11: 30% I, 15% Y, 5% G/A/S/T/PN/H/D/E/Q/V, 12: 52% Y, 4% G/A/S/P/T/N/H/D/E/Q/V/I, 13: 30% D, 15% G, 5% A/S/P/Y/N/H/D/E/Q/V/I, 14: 52% T, 4% G/A/S/P/Y/N/H/D/E/Q/V/I, 15: 52% T, 4% G/A/S/P/Y/N/H/D/E/Q/V/I |
| 142 | CD19 H2 forward random | CAG GCC CCG GGC CAG GGT CTG GAG TGG ATG GGC 16 ATT 17 CCA 18 19 20 21 TCC 22 TAT ACC 23 AAA TTC CAG GGC CGC GTC ACG ATG ACC<br>16: 45% Y, 5% A/S/P/T/N/H/D/E/Q/V/I, 17: 52% N, 4% |

(continued)

| SEQ ID | Name | Sequence |
|---|---|---|
| | | G/A/S/P/Y/T/H/D/E/Q/V/I, 18: 40% Y, 5% G/A/S/P/T/N/H/D/E/Q/V/I, 19: 30% N, 15% S, 5% G/A/T/P/Y/H/D/E/Q/V/I, 20: 30% D, 15% G, 5% A/S/T/P/Y/N/H/E/Q/V/I, 21: 52% G, 4% N/A/S/P/Y/T/H/D/E/Q/V/I, 22: 30% K, 15% N, 4% G/A/S/P/Y/T/H/D/E/Q/V/I, 23: 30% E, 15% Q, 5% G/A/S/T/P/Y/N/H/D/V/I |
| 143 | CD19 H3 reverse constant | CGTCACCGGTTCGGGGAAGTAGTCCTTGACCAG |
| 144 | LMB3 | CAGGAAACAGCTATGACCATGATTAC |

**Table 14: Primers for 8B8 affinity maturation and hotspot removal library L1_L3 / H3**

| SEQ ID | Name | Sequence |
|---|---|---|
| 145 | CD19 L1 forward constant | TGGTATCTCCAGAAACCGGGTCAGAGCCCGCAG |
| 139 | CD19 L1 reverse random | See Table 15 |
| 146 | CD19 L3 reverse random | TTT AAT TTC CAG TTT AGT TCC TTG ACC GAA GGT 24 25 26 27 28 29 CTG CAG ACA ATA GTA GAC GCC AAC GTC TTC AGC<br>24: 52% Y, 4% G/A/S/T/N/P/D/E/Q/V/L/I, 25: 52% P, 4% G/A/S/T/Y/N/H/D/E/Q/V/I, 26: 42% V, 10% L, 4% G/A/S/T/Y/N/P/D/E/Q/V/I, 27: 52% H, 4% G/A/S/T/Y/N/P/D/E/Q/V/I, 28: 42% T, 10% I, 4% G/A/S/T/Y/N/P/D/E/Q/V/L, 29: 45% L, 11% G, 4% A/S/T/Y/N/P/D/E/Q/V/I |
| 147 | CD19 L3 forward constant | ACCTTCGGTCAAGGAACTAAACTGGAAATTAAACG |
| 148 | CD19 H3 reverse random | TT GGT GCT AGC AGA GCT TAC GGT CAC CGT GGT ACC TTG GCC CCA GTA ATC AAA 30 31 32 33 34 35 36 37 38 GCG TGC ACA ATA GTA AAC AGC GGT GTC<br>30: 50% L, 3.8% G/A/S/T/P/H/Y/N/D/E/Q/V/I, 31: 50% A, 4.2% G/S/T/P/H/Y/N/D/E/Q/V/I, 32: 50% S, 4.2% G/A/T/P/H/Y/N/D/E/Q/V/I, 33: 50% G, 4.2% S/A/T/P/H/Y/N/D/E/Q/V/I, 34: 50% Y, 4.2% G/A/T/P/H/S/N/D/E/Q/V/I, 35: 50% Y, 4.2% G/A/T/P/H/S/N/D/E/Q/V/I, 36: 50% Y, 4.2% G/A/T/P/H/S/N/D/E/Q/V/I, 37: 50% T, 4.2% G/A/Y/P/H/S/N/D/E/Q/V/I, 38: 50% G, 4.2% Y/A/T/P/H/S/N/D/E/Q/V/I |
| 144 | LMB3 | See Table 15 |

### 1.3.4 Selection of affinity matured 8B8-derived clones devoid of CDR-L1 hotspots N27d and N28

For the selection of affinity-matured clones devoid of the CDR-L1hotspots N27d and N28, two selection approaches by phage display were performed:

In the first approach, the selection was executed on human CD19-Fc fusion protein using both phage display libraries. Panning rounds were performed in solution according to the following pattern: 1. binding of ~ $10^{12}$ phagemid particles to 30nM biotinylated CD19-Fc protein for 0.5 h in a total volume of 1ml, 2. capture of biotinylated CD19-Fc protein and specifically bound phage particles by addition of $5.4 \times 10^7$ streptavidin-coated magnetic beads for 10 min, 3. washing of beads using 5x 1ml PBS/Tween20 and 5x 1ml PBS, 4. elution of phage particles by addition of 1ml 100mM TEA for 10 min and neutralization by adding 500ul 1M Tris/HCl pH 7.4, 5. re-infection of exponentially growing E. *coli* TG1 bacteria, and 6.infection with helperphage VCSM13 and subsequent PEG/NaCl precipitation of phagemid particles to be used in subsequent selection rounds. Selections were carried out over 3 rounds using decreasing antigen concentrations ($30x10^{-9}$M, $10x10^{-9}$M, and $3x10^{-9}$M). In round 2 and 3, capture of antigen:phage complexes was performed using neutravidin plates instead of streptavidin beads. Neutravidin plates were washed with 5x PBS/Tween20 and 5x PBS. In round 3, the neutravidin plate was incubated overnight in 2 liters PBS for an "off-rate" selection before phage was eluted from the plate. Furthermore, cynomolgus CD19-Fc protein was used in round 2 in order to enrich cross-reactive binders.

In the second selection approach, the phage panning was executed on cells transiently expressing either the human or cynomolgus CD19 ECD on the cell surface. For the transient transfection of HEK cells, expression plasmids were generated that harbor the DNA sequences (from 5' to 3') for the following protein segments: A Flag tag, a SNAP tag, the CD19 ECD of either human or cynomolgus origin, and the transmembrane region of the Platelet-derived growth factor receptor (PDGFR) (SEQ ID NOs: 149 and 150). The expression of the respective proteins (SEQ ID NOs: 151 and 152) on the cell surface was confirmed by flow cytometry using an anti-Flag antibody for detection. Both libraries were exposed in the first selection round to cells either expressing the human or cynomolgus CD19 ECD-containing protein fusion. For the subsequent panning rounds, the species of the CD19 ECD was alternated accordingly. Cells transiently transfected with an irrelevant membrane protein were used for pre-clearing.

Panning rounds were performed according to the following pattern:

1. Transfection of HEK cells with constructs expressing either CD19 ECD or an irrelevant transmembrane protein according to the standard procedure described before,
2. Incubation of the cells for total 48h at 37°C in an incubator with a 5% $CO_2$ atmosphere,
3. Isolation of cells by centrifugation (3 min at 250×g) and re-suspension of 1×10E7 CD19 ECD-positive cells and 1×10E7 negative cells in PBS/5% BSA, respectively,
4. Pre-clearing of unspecific phage by incubating the phage library with 1 × 107 CD19-negative cells for 60 min at 4°C using a gently rotating tube rotator,
5. Centrifugation of cells at 250×g for 3min and transfer of supernatant into a fresh tube and addition of 1×10E7 CD19-positive cells and incubation for 60 min at 4 °C by gentle rotation on a tube rotator,
6. Washing of cells by centrifugation for 1 min at 250×g, aspiration of the supernatant, and re-suspension in 1 ml PBS (8 times),
7. Phage elution with 1 ml 100mM TEA, incubation for 5 min at RT, and neutralization of the eluate with 500 ul 1M Tris-HCl, pH7.6,
8. re-infection of exponentially growing *E. coli* TG1 bacteria, and
9. infection with helperphage VCSM13 and subsequent PEG/NaCl precipitation of phagemid particles to be used in subsequent selection rounds. Selections were carried out over 3 rounds.

For both selection approaches, specific binders were identified by ELISA as follows: 100 ul of 30 nM biotinylated CD19-Fc protein per well were coated on neutravidin plates. Fab-containing bacterial supernatants were added and binding Fabs were detected via their Flag-tags using an anti-Flag/HRP secondary antibody.

Clones that were ELISA-positive on recombinant human CD19 were further tested in a cell-based ELISA using cells that were transiently transfected with the human CD19 ECD-containing expression plasmid (SEQ ID NO: 149). This analysis was performed as follows: 48 h after transfection, HEK cells were harvested and centrifuged at 250xg for 5 min. Cells were then re suspended in ice-cold PBS BSA 2% to 4 x $10^6$ cells/ml and incubated for 20 min on ice to block unspecific binding sites. $4 \times 10^5$ cells in 100ul were distributed to each well of a 96 well plate and centrifuged at 250xg and 4°C for 3 min. Supernatant was aspirated off and 50ul bacterial supernatant containing soluble Fab fragments was diluted with 50ul ice-cold PBS/BSA 2%, added to the plate, mixed with the cells and incubated for 1 h at 4°C. Afterwards, cells were washed 3 times with ice cold PBS before 100ul PBS BSA 2% per well containing a 1:2000 dilution of anti-Fab-HRP antibody were added. After an incubation time of 1 h, cells were washed again 3 times with ice-cold PBS. For the development, 100ul "1-step ultra TMB-ELISA" substrate was added per well. After an incubation time of 10 minutes, supernatant was transferred to a new 96-well plate containing 40ul $H_2SO_4$ 1M per well and absorbance was measured 450 nM. Clones exhibiting significant signals over background were subjected to a kinetic screening experiment by SPR-analysis using ProteOn XPR36.

### 1.3.5 Identification of affinity-matured 8B8-derived variants by SPR

In order to further characterize the ELISA-positive clones, the off-rate was measured by surface plasmon resonance and compared with the parental humanized clone 8B8.

For this experiment, 7000 RU of polyclonal anti-human Fab antibody were immobilized on all 6 channels of a GLM chip by Amine coupling (NaAcetate pH4.5, 25 μl/min, 240s) (vertical orientation). Each antibody-containing bacterial supernatant was filtered and 2-fold diluted with PBS, and then injected for 360s at 25 μl/minute to achieve immobilization levels of between 100 and 400 response units (RU) in vertical orientation. Injection of monomeric CD19-Fc: For one-shot kinetics measurements, injection direction was changed to horizontal orientation, three-fold dilution series of purified monomeric CD19-Fc (varying concentration ranges between 150 and 6 nM) were injected simultaneously at 50 μl/min along separate channels 1-4, with association times of 180 s, and dissociation times of 300 s. A human IgG Fc fragment (150nM) was injected in channel 5 as a negative control for specific binding to the affinity matured CD19 variants along with a PBS injection in the 6th channel to provide an "in-line" blank for referencing. Regeneration was performed by two pulses of 10mM glycine pH 1.5 and 50mM NaOH for 30s at 90ul/min (horizontal orientation). Dissociation rate constants ($k_{off}$) were calculated using a simple one-to-one Langmuir binding model in ProteOn Manager v3.1 software by simultaneously fitting the sensorgrams. Clones expressing Fabs with the slowest dissociation rate constants were identified. The variable domains of the corresponding phagemids were sequenced. Importantly, both asparagine residue in CDR-L1 (position 27d and 28) were replaced by a serine or a threonine, demonstrating that both de-amidation sites were removed.

**Table 15: Dissociation constants of parental 8B8 and selected clone 2B11 obtained in screening analysis with bacterial supernatant**

| clone | Dissociation constant kd (1/s) |
|---|---|
| Parental 8B8 | 3.01E-4 |
| 2B11 | 4.37E-6 |

**Table 16** shows the amino acid sequences of the CDRs and variable regions VH and VL of clones 8B8-018 and 8B8-2B11, respectively.

**Table 16:** Sequences of of the CDRs and variable regions VH and VL of clones 8B8-018 and 8B8-2B11

| SEQ ID NO: | Description | Sequence |
|---|---|---|
| 29 | CD19 (8B8-018) CDR-H1 | DYIMH |
| 30 | CD19 (8B8-018) CDR-H2 | YINPYNDGSKYTEKFQG |
| 31 | CD19 (8B8-018) CDR-H3 | GTYYYGSALFDY |
| 32 | CD19 (8B8-018) CDR-L1 | KSSQSLENPNGNTYLN |
| 33 | CD19 (8B8-018) CDR-L2 | RVSKRFS |
| 34 | CD19 (8B8-018) CDR-L3 | LQLTHVPYT |
| 41 | CD19 (8B8-018) VH | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGS ALFDYWGQGTTVTVSS |
| 42 | CD19 (8B8-018) VL | DIVMTQTPLSLSVTPGQPASISCKSSQSLENPNGNTYL NWYLQKPGQSPQLLIYRVSKRFSGVPDRFSGSGSGTD FTLKISRVEAEDVGVYYCLQLTHVPYTFGQGTKLEIK |
| 35 | CD19 (8B8-2B11) CDR-H1 | DYIMH |
| 36 | CD19 (8B8-2B11) CDR-H2 | YINPYNDGSKYTEKFQG |

(continued)

| SEQ ID NO: | Description | Sequence |
|---|---|---|
| 37 | CD19 (8B8-2B11) CDR-H3 | GTYYYGPQLFDY |
| 38 | CD19 (8B8-2B11) CDR-L1 | KSSQSLETSTGTTYLN |
| 39 | CD19 (8B8-2B11) CDR-L2 | RVSKRFS |
| 40 | CD19 (8B8-2B11) CDR-L3 | LQLLEDPYT |
| 43 | CD19 (8B8-2B11) VH | QVQLVQSGAEVKKPGASVKVSCKASGYTFTDYIMH WVRQAPGQGLEWMGYINPYNDGSKYTEKFQGRVT MTSDTSISTAYMELSRLRSDDTAVYYCARGTYYYGP QLFDYWGQGTTVTVSS |
| 44 | CD19 (8B8-2B11) VL | DIVMTQTPLSLSVTPGQPASISCKSSQSLETSTGTTYL NWYLQKPGQSPQLLIYRVSKRFSGVPDRFSGSGSGTD FTLKISRVEAEDVGVYYCLQLLEDPYTFGQGTKLEIK |

**1.4 Preparation of monovalent CEA-targeted 4-1BB ligand trimer-containing Fc (kih) fusion antigen binding molecule, wherein the 4-1BB ligands are C-terminally fused (Constructs 5.11 and 5.12) (CEA on the knob chain)**

The molecule was prepared as described in Example 1.1 for the monovalent FAP targeted construct, with the only difference that the anti-FAP binder was replaced by an anti-CEA binder.

The variable region of heavy and light chain DNA sequences encoding binder specific for carcinoembryonic antigen (CEA), clone T84.66-LCHA, were subcloned in frame with either the constant heavy chain of the knob or the constant light chain of human IgG1. The generation of the CEA clone is described in Example 1.5.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

For all constructs the knobs into holes heterodimerization technology was used as described in Carter, J Immunol Methods 248, 7-15 (2001). Combination of the huIgG1 Fc hole chain containing the Y349C/T366S/L368A/Y407V mutations in the CH3 domain, the anti-FAP huIgG1 knob chain containing the S354C/T366W mutations in the CH3 domain and the anti-FAP light chain allows generation of a heterodimer, which includes an assembled trimeric 4-1BB ligand and one CEA binding Fab **(Figures 2A and 2B).**

Table 17 shows the cDNA and amino acid sequences of the monovalent CEA(T84.66-LCHA)-targeted 4-1BB ligand (71-248) trimer-containing Fc (kih) fusion antigen binding molecule (Construct 5.11).

**Table 17: Sequences of CEA(T84.66-LCHA)-targeted human 4-1BB ligand trimer containing Fc (kih) fusion molecule Construct 5.11**

| SEQ ID NO: | Description | Sequence |
|---|---|---|
| 101 | nucleotide | see Table 1 |
| | sequence of Fc hole dimeric 4-1BB ligand (71-248) chain | |

(continued)

| SEQ ID NO: | Description | Sequence |
|---|---|---|
| 153 | nucleotide sequence of anti-CEA(T84.66-LCHA) Fc knob monomeric 4-1BB (71-248) ligand | CAGGTGCAGCTGGTGCAGTCTGGCGCCGAAGTGAAGAA<br>ACCCGGCAGCAGCGTGAAGGTGTCCTGCAAGGCCAGCG<br>GCTTCAACATCAAGGACACCTACATGCACTGGGTGCGCC<br>AGGCCCCTGGACAGGGACTGGAATGGATGGGCAGAATC<br>GACCCCGCCAACGGCAACAGCAAATACGTGCCCAAGTT<br>CCAGGGCAGAGTGACCATCACCGCCGACACCAGCACCT<br>CCACCGCCTACATGGAACTGAGCAGCCTGCGGAGCGAG<br>GACACCGCCGTGTACTACTGTGCCCCCTTCGGCTACTAC<br>GTGTCCGACTACGCCATGGCCTATTGGGGCCAGGGCAC<br>ACTCGTGACCGTGTCCTCTGCTAGCACCAAGGGCCCATC<br>GGTCTTCCCCCTGGCACCCTCCTCCAAGAGCACCTCTGG<br>GGGCACAGCGGCCCTGGGCTGCCTGGTCAAGGACTACT<br>TCCCCGAACCGGTGACGGTGTCGTGGAACTCAGGCGCC<br>CTGACCAGCGGCGTGCACACCTTCCCGGCTGTCCTACAG<br>TCCTCAGGACTCTACTCCCTCAGCAGCGTGGTGACCGTG<br>CCCTCCAGCAGCTTGGGCACCCAGACCTACATCTGCAAC<br>GTGAATCACAAGCCCAGCAACACCAAGGTGGACAAGAA<br>AGTTGAGCCCAAATCTTGTGACAAAACTCACACATGCCC<br>ACCGTGCCCAGCACCTGAAGCTGCAGGGGGACCGTCAG<br>TCTTCCTCTTCCCCCCAAAACCCAAGGACACCCTCATGA<br>TCTCCCGGACCCCTGAGGTCACATGCGTGGTGGTGGACG<br>TGAGCCACGAAGACCCTGAGGTCAAGTTCAACTGGTAC<br>GTGGACGGCGTGGAGGTGCATAATGCCAAGACAAAGCC<br>GCGGGAGGAGCAGTACAACAGCACGTACCGTGTGGTCA<br>GCGTCCTCACCGTCCTGCACCAGGACTGGCTGAATGGCA<br>AGGAGTACAAGTGCAAGGTCTCCAACAAAGCCCTCGGC<br>GCCCCCATCGAGAAAACCATCTCCAAAGCCAAAGGGCA<br>GCCCCGAGAACCACAGGTGTACACCCTGCCCCCCTGCA<br>GAGATGAGCTGACCAAGAACCAGGTGTCCCTGTGGTGT<br>CTGGTCAAGGGCTTCTACCCCAGCGATATCGCCGTGGAG<br>TGGGAGAGCAACGGCCAGCCTGAGAACAACTACAAGAC<br>CACCCCCCCTGTGCTGGACAGCGACGGCAGCTTCTTCCT<br>GTACTCCAAACTGACCGTGGACAAGAGCCGGTGGCAGC<br>AGGGCAACGTGTTCAGCTGCAGCGTGATGCACGAGGCC<br>CTGCACAACCACTACACCCAGAAGTCCCTGAGCCTGAG<br>CCCCGGCGGAGGCGGCGGAAGCGGAGGAGGAGGATCC<br>AGAGAGGGCCCTGAGCTGAGCCCTGATGATCCTGCCGG<br>ACTGCTGGACCTGCGGCAGGGAATGTTTGCCCAGCTGGT<br>GGCCCAGAACGTGCTGCTGATCGATGGCCCCCTGTCCTG<br>GTACAGCGATCCTGGACTGGCTGGCGTGTCACTGACAGG<br>CGGCCTGAGCTACAAAGAGGACACCAAAGAACTGGTGG<br>TGGCCAAGGCCGGCGTGTACTACGTGTTCTTTCAGCTGG<br>AACTGCGGAGAGTGGTGGCCGGCGAAGGATCTGGCTCT<br>GTGTCTCTGGCCCTGCATCTGCAGCCTCTGAGATCTGCT<br>GCTGGCGCCGCTGCTCTGGCACTGACAGTGGATCTGCCT<br>CCTGCCAGCAGCGAGGCCCGGAATAGCGCATTTGGGTTT<br>CAAGGCAGGCTGCTGCACCTGTCTGCCGGCCAGAGGCT<br>GGGAGTGCATCTGCACACAGAGGCCAGGGCTAGACACG |

(continued)

| SEQ ID NO: | Description | Sequence |
|---|---|---|
| | | CCTGGCAGCTGACACAGGGCGCTACAGTGCTGGGCCTG TTCAGAGTGACCCCCGAGATTCCTGCCGGGCTC |
| 154 | nucleotide sequence of anti-CEA(T84.66-LCHA) light chain | GAGATCGTGCTGACCCAGAGCCCTGCCACCCTGTCACTG TCTCCAGGCGAGAGAGCCACCCTGAGCTGTAGAGCCGG CGAGAGCGTGGACATCTTCGGCGTGGGATTTCTGCACTG GTATCAGCAGAAGCCCGGCCAGGCCCCCAGACTGCTGA TCTACAGAGCCAGCAACCGGGCCACAGGCATCCCCGCC AGATTTTCTGGCTCTGGCAGCGGCACCGACTTCACCCTG ACAATCAGCAGCCTGGAACCCGAGGACTTCGCCGTGTA CTACTGCCAGCAGACCAACGAGGACCCCTACACCTTTGG CCAGGGCACCAAGCTGGAAATCAAGCGTACGGTGGCTG CACCATCTGTCTTCATCTTCCCGCCATCTGATGAGCAGTT GAAATCTGGAACTGCCTCTGTTGTGTGCCTGCTGAATAA CTTCTATCCCAGAGAGGCCAAAGTACAGTGGAAGGTGG ATAACGCCCTCCAATCGGGTAACTCCCAGGAGAGTGTC ACAGAGCAGGACAGCAAGGACAGCACCTACAGCCTCAG CAGCACCCTGACGCTGAGCAAAGCAGACTACGAGAAAC ACAAAGTCTACGCCTGCGAAGTCACCCATCAGGGCCTG AGCTCGCCCGTCACAAAGAGCTTCAACAGGGGAGAGTG T |
| 104 | Fc hole dimeric 4-1BB (71-248) ligand chain | see Table 1 |
| 155 | anti- CEA (T84.66-LCHA) Fc knob monomeric 4-1-BB (71-248) ligand | QVQLVQSGAEVKKPGSSVKVSCKASGFNIKDTYMHWVRQ APGQGLEWMGRIDPANGNSKYVPKFQGRVTITADTSTSTA YMELSSLRSEDTAVYYCAPFGYYVSDYAMAYWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALGAPIEKTISKAKGQPREPQVYTLPPCR DELTKNQVSLWCLVKGFYPSDIAVEWESNGQPENNYKTTP PVLDSDGSFFLYSKLTVDKSRWQQGNVFSCSVMHEALHN HYTQKSLSLSPGGGGGSGGGGSREGPELSPDDPAGLLDLR QGMFAQLVAQNVLLIDGPLSWYSDPGLAGVSLTGGLSYKE DTKELVVAKAGVYYVFFQLELRRVVAGEGSGSVSLALHL QPLRSAAGAAALALTVDLPPASSEARNSAFGFQGRLLHLS AGQRLGVHLHTEARARHAWQLTQGATVLGLFRVTPEIPA GL |
| 156 | anti-CEA(T84.66-LCHA) light chain | EIVLTQSPATLSLSPGERATLSCRAGESVDIFGVGFLHWYQ QKPGQAPRLLIYRASNRATGIPARFSGSGSGTDFTLTISSLEP EDFAVYYCQQTNEDPYTFGQGTKLEIKRTVAAPSVFIFPPS DEQLKSGTASVVCLLNNFYPREAKVQWKVDNALQSGNSQ ESVTEQDSKDSTYSLSSTLTLSKADYEKHKVYACEVTHQG LSSPVTKSFNRGEC |

Table 18 shows the cDNA and amino acid sequences of the monovalent CEA(T84.66-LCHA)-targeted 4-1BB ligand (71-248) trimer-containing Fc (kih) fusion antigen binding molecule (Construct 5.12).

**Table 18: Sequences of CEA(T84.66-LCHA)-targeted human 4-1BB ligand trimer containing Fc (kih) fusion molecule Construct 5.12 (comparative example)**

| SEQ ID NO: | Description | Sequence |
|---|---|---|
| 107 | nucleotide sequence of Fc hole monomeric 4-1BBL (71-248) chain | see Table 2 |
| 157 | nucleotide sequence of anti-CEA(T84.66-LCHA) Fc knob dimeric 4-1BB (71-248) ligand | CAGGTGCAGCTGGTGCAGTCTGGCGCCGAAGTGAAGAA ACCCGGCAGCAGCGTGAAGGTGTCCTGCAAGGCCAGCG GCTTCAACATCAAGGACACCTACATGCACTGGGTGCGCC AGGCCCCTGGACAGGGACTGGAATGGATGGGCAGAATC GACCCCGCCAACGGCAACAGCAAATACGTGCCCAAGTT CCAGGGCAGAGTGACCATCACCGCCGACACCAGCACCT CCACCGCCTACATGGAACTGAGCAGCCTGCGGAGCGAG GACACCGCCGTGTACTACTGTGCCCCCTTCGGCTACTAC GTGTCCGACTACGCCATGGCCTATTGGGGCCAGGGCAC ACTCGTGACCGTGTCCTCTGCTAGCACCAAGGGCCCATC GGTCTTCCCCCTGGCACCCTCCTCCAAGAGCACCTCTGG GGGCACAGCGGCCCTGGGCTGCCTGGTCAAGGACTACT TCCCCGAACCGGTGACGGTGTCGTGGAACTCAGGCGCC CTGACCAGCGGCGTGCACACCTTCCCGGCTGTCCTACAG TCCTCAGGACTCTACTCCCTCAGCAGCGTGGTGACCGTG CCCTCCAGCAGCTTGGGCACCCAGACCTACATCTGCAAC GTGAATCACAAGCCCAGCAACACCAAGGTGGACAAGAA AGTTGAGCCCAAATCTTGTGACAAAACTCACACATGCCC ACCGTGCCCAGCACCTGAAGCTGCAGGGGGACCGTCAG TCTTCCTCTTCCCCCCAAAACCCAAGGACACCCTCATGA TCTCCCGGACCCCTGAGGTCACATGCGTGGTGGTGGACG TGAGCCACGAAGACCCTGAGGTCAAGTTCAACTGGTAC GTGGACGGCGTGGAGGTGCATAATGCCAAGACAAAGCC GCGGGAGGAGCAGTACAACAGCACGTACCGTGTGGTCA GCGTCCTCACCGTCCTGCACCAGGACTGGCTGAATGGCA AGGAGTACAAGTGCAAGGTCTCCAACAAAGCCCTCGGC GCCCCCATCGAGAAAACCATCTCCAAAGCCAAAGGGCA GCCCCGAGAACCACAGGTGTACACCCTGCCCCCCTGCA GAGATGAGCTGACCAAGAACCAGGTGTCCCTGTGGTGT CTGGTCAAGGGCTTCTACCCCAGCGATATCGCCGTGGAG TGGGAGAGCAACGGCCAGCCTGAGAACAACTACAAGAC CACCCCCCCTGTGCTGGACAGCGACGGCAGCTTCTTCCT GTACTCCAAACTGACCGTGGACAAGAGCCGGTGGCAGC AGGGCAACGTGTTCAGCTGCAGCGTGATGCACGAGGCC |

(continued)

| SEQ ID NO: | Description | Sequence |
|---|---|---|
| | | CTGCACAACCACTACACCCAGAAGTCCCTGAGCCTGAG CCCCGGCAGAGAGGGCCCTGAGCTGAGCCCTGATGATC CTGCCGGACTGCTGGACCTGCGGCAGGGAATGTTTGCCC AGCTGGTGGCCCAGAACGTGCTGCTGATCGATGGCCCCC TGTCCTGGTACAGCGATCCTGGACTGGCTGGCGTGTCAC TGACAGGCGGCCTGAGCTACAAAGAGGACACCAAAGAA CTGGTGGTGGCCAAGGCCGGCGTGTACTACGTGTTCTTT CAGCTGGAACTGCGGAGAGTGGTGGCCGGCGAAGGATC TGGCTCTGTGTCTCTGGCCCTGCATCTGCAGCCTCTGAG ATCTGCTGCTGGCGCCGCTGCTCTGGCACTGACAGTGGA TCTGCCTCCTGCCAGCAGCGAGGCCCGGAATAGCGCATT TGGGTTTCAAGGCAGGCTGCTGCACCTGTCTGCCGGCCA GAGGCTGGGAGTGCATCTGCACACAGAGGCCAGGGCTA GACACGCCTGGCAGCTGACACAGGGCGCTACAGTGCTG GGCCTGTTCAGAGTGACCCCCGAGATTCCAGCAGGCCTG GGAGGCGGCGGATCTGGCGGCGGAGGATCTAGAGAAGG ACCCGAGCTGTCCCCCGACGATCCCGCTGGGCTGCTGGA TCTGAGACAGGGCATGTTCGCTCAGCTGGTGGCTCAGAA TGTGCTGCTGATTGACGGACCTCTGAGCTGGTACTCCGA CCCAGGGCTGGCAGGGGTGTCCCTGACTGGGGGACTGT CCTACAAAGAAGATACAAAAGAACTGGTGGTGGCTAAA GCTGGGGTGTACTATGTGTTTTTCAGCTGGAACTGAGG CGGGTGGTGGCTGGGGAGGGCTCAGGATCTGTGTCCCTG GCTCTGCATCTGCAGCCACTGCGCTCTGCAGCAGGGGCT GCAGCACTGGCCCTGACTGTGGACCTGCCCCCAGCTTCT TCCGAGGCCAGAAACAGCGCCTTCGGGTTCCAAGGACG CCTGCTGCATCTGAGCGCCGGACAGCGCCTGGGAGTGC ATCTGCATACTGAAGCCAGAGCCCGGCATGCTTGGCAG CTGACTCAGGGGGCAACTGTGCTGGGACTGTTTCGCGTG ACACCTGAGATCCCAGCCGGGCTC |
| 154 | nucleotide sequence of anti-CEA(T84.66-LCHA) light chain | See Table 17 |
| 109 | Fc hole monomeric 4-1BB ligand (71-248) chain | see Table 2 |
| 158 | anti-CEA(T84.66-LCHA) Fc knob dimeric 4-1BB (71-248) ligand | QVQLVQSGAEVKKPGSSVKVSCKASGFNIKDTYMHWVRQ APGQGLEWMGRIDPANGNSKYVPKFQGRVTITADTSTSTA YMELSSLRSEDTAVYYCAPFGYYVSDYAMAYWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALGAPIEKTISKAKGQPREPQVYTLPPCR DELTKNQVSLWCLVKGFYPSDIAVEWESNGQPENNYKTTP |

(continued)

| SEQ ID NO: | Description | Sequence |
|---|---|---|
| | | PVLDSDGSFFLYSKLTVDKSRWQQGNVFSCSVMHEALHN HYTQKSLSLSPGREGPELSPDDPAGLLDLRQGMFAQLVAQ NVLLIDGPLSWYSDPGLAGVSLTGGLSYKEDTKELVVAKA GVYYVFFQLELRRVVAGEGSGSVSLALHLQPLRSAAGAAA LALTVDLPPASSEARNSAFGFQGRLLHLSAGQRLGVHLHT EARARHAWQLTQGATVLGLFRVTPEIPAGLGGGGSGGGGS REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGL |
| 156 | anti-CEA(T84.66-LCHA) light chain | See Table 17 |

**1.5 Generation of anti-CEA clone T84.66-LCHA**

**1.5.1 Humanization of anti-CEA clone T84.66**

Novel humanized variants of the murine antibody T84.66 (Wagener et al., J Immunol 130, 2308 (1983), Neumaier et al., J Immunol 135, 3604 (1985)) were developed by grafting of the CDRs onto human germline framework acceptor sequences.

Humanization of an antibody from non-human origin consists essentially of transplanting the CDR residues from the non-human antibody (donor) onto the framework of a human (acceptor) antibody. Normally the acceptor framework is selected by aligning the sequence of the donor to a collection of potential acceptor sequences and choosing one that has either reasonable homology to the donor, or shows similar amino acids at some positions critical for structure and activity. In the present case, the search for the antibody acceptor framework was performed by aligning the mouse T84.66 protein (NCBI Acc No: CAA36980 for the heavy chain (SEQ ID NO: 159), and CAA36979 (SEQ ID NO: 160) for the light chain) sequence to a collection of human germ-line sequences and picking that human sequence that showed high sequence identity. Here, the sequence IGHV1-69*08 from the IMGT database was chosen as the heavy chain framework acceptor sequence (IMGT Acc No. Z14309, SEQ ID NO:161), and the IGKV3-11*01 sequence (IMGT Acc No. X01668, SEQ ID NO: 162) was chosen to be the framework acceptor for the light chain. Onto these two acceptor frameworks, the three complementary determining regions (CDRs) of the mouse heavy and light variable domains were grafted. Since the framework 4 (FR4) region is not part of the variable region of the germ line V gene, the alignment for that position was done individually. The JH4 sequence was chosen for the heavy chain, and the JK2 sequence was chosen for the light chain.

**11.1.2 Binding of different humanized variants of T84.66 IgG to cells**

The binding of different humanized variants of T84.66 IgG was tested on CEA-expressing human gastric adenocarcinoma cells (MKN45, DSMZ ACC 409).

Cells were harvested, counted, checked for viability and re-suspended at $2x10^6$ cells/ml in FACS buffer (100 μl PBS 0.1% BSA). 100 μl of cell suspension (containing $0.2x10^6$ cells) were incubated in round-bottom 96-well plate for 30 min at 4°C with increasing concentrations of the CEA IgG (4 ng/ml- 60 μg/ml), washed twice with cold PBS 0.1% BSA, re-incubated for further 30 min at 4°C with the PE-conjugated AffiniPure F(ab')2 Fragment goat anti-human IgG Fcg Fragment Specific secondary antibody (Jackson Immuno Research Lab PE #109-116-170), washed twice with cold PBS 0.1% BSA and immediately analyzed by FACS using a FACS CantoII (Software FACS Diva). Binding curves and EC50 values were obtained and calculated using GraphPadPrism5.

**Table 19** shows the different binding data of selected humanized variants of the T84.66 IgG to human CEA, expressed on MKN45 cells. Based on the calculated EC50 binding values the humanized variant 1 was selected for further evaluation.

**Table 19: Binding of different humanized variants of T84.66 IgGs to cells**

| | EC50 (μg/ml) |
|---|---|
| Parental chimeric T84.66 | 0.99 |
| Humanized variant 1 | 1.5 |
| Humanized variant 2 | 8.6 |
| Humanized variant 3 | 1.4 |
| Humanized variant 4 | 3.1 |

Humanized variant 1 is termed in the following T84.66-LCHA. The amino acid sequences of its CDRs and of the VH and VL as well as the amino acid sequences of the VH and VL domain of the parental chimeric T84.66 clone are shown in Table 20.

**Table 20: Amino acid sequences of the variable domains of CEA clone T84.66-LCHA and its parental antibody T84.66**

| Description | Sequence | SEQ ID NO: |
|---|---|---|
| CEA CDR-H1 | DTYMH | 45 |
| CEA CDR-H2 | RIDPANGNSKYVPKFQG | 46 |
| CEA CDR-H3 | FGYYV SDYAMAY | 47 |
| CEA CDR-L1 | RAGESVDIFGVGFLH | 48 |
| CEA CDR-L2 | RASNRAT | 49 |
| CEA CDR-L3 | QQTNEDPYT | 50 |
| Parental CEA binder VH | EVQLQQSGAELVEPGASVKLSCTASGFNIKDTYMHWVKQRPEQ GLEWIGRIDPANGNSKYVPKFQGKATITADTSSNTAYLQLTSLTS EDTAVYYCAPFGYYVSDYAMAYWGQGTSVTVSS | 163 |
| Parental CEA binder VL | DIVLTQSPASLAVSLGQRATMSCRAGESVDIFGVGFLHWYQQKP GQPPKLLIYRASNLESGIPVRFSGTGSRTDFTLIIDPVEADDVATY YCQQTNEDPYTFGGGTKLEIK | 164 |
| Humanized CEA binder CEA (T84.66-LCHA) VH | QVQLVQSGAEVKKPGSSVKVSCKASGFNIKDTYMHWVRQAPGQ GLEWMGRIDPANGNSKYVPKFQGRVTITADTSTSTAYMELSSLR SEDTAVYYCAPFGYYVSDYAMAYWGQGTLVTVSS | 51 |
| Humanized CEA binder CEA (T84.66-LCHA) VL | EIVLTQSPATLSLSPGERATLSCRAGESVDIFGVGFLHWYQQKPG QAPRLLIYRASNRATGIPARFSGSGSGTDFTLTISSLEPEDFAVYY CQQTNEDPYTFGQGTKLEIK | 52 |

**1.6 Preparation of monovalent untargeted 4-1BB ligand trimer-containing Fc (kih) fusion antigen binding molecules, wherein the 4-1BB ligands are C-terminally fused (Controls H and I) (DP47 on the knob chain)**

The molecule was prepared as described in Example 1.1 for the monovalent FAP targeted construct, with the only difference that the anti-FAP binder (VH-VL) was replaced by a germline control, termed DP47, not binding to the antigen.

Table 21 shows the cDNA and amino acid sequences of the monovalent untargeted 4-1BB ligand (71-248) trimer-containing Fc (kih) fusion antigen binding molecule (Control H).

**Table 21: Sequences of monovalent DP47-untargeted human 4-1BB ligand trimer containing Fc (kih) fusion molecule Control H**

| SEQ ID NO: | Description | Sequence |
|---|---|---|
| 101 | nucleotide sequence of Fc hole dimeric 4-1BB ligand (71-248) chain | see Table 1 |
| 165 | nucleotide sequence of DP47 Fc knob monomeric 4-1BB (71-248) ligand | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA |

(continued)

| SEQ ID NO: | Description | Sequence |
|---|---|---|
| | | AGGGCCGGTTCACCATCTCCAGAGACAATTCCAAGAACACGCTGTATCTGCAGATGAACAGCCTGAGAGCCGAGGACACGGCCGTATATTACTGTGCGAAAGGCAGCGGATTTGACTACTGGGGCCAAGGAACCCTGGTCACCGTCTCGAGTGCTAGCACCAAGGGCCCATCGGTCTTCCCCCTGGCACCCTCCTCCAAGAGCACCTCTGGGGGCACAGCGGCCCTGGGCTGCCTGGTCAAGGACTACTTCCCCGAACCGGTGACGGTGTCGTGGAACTCAGGCGCCCTGACCAGCGGCGTGCACACCTTCCCGGCTGTCCTACAGTCCTCAGGACTCTACTCCCTCAGCAGCGTGGTGACCGTGCCCTCCAGCAGCTTGGGCACCCAGACCTACATCTGCAACGTGAATCACAAGCCCAGCAACACCAAGGTGGACAAGAAAGTTGAGCCCAAATCTTGTGACAAAACTCACACATGCCCACCGTGCCCAGCACCTGAAGCTGCAGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTACACCCTGCCCCCCTGCAGAGATGAGCTGACCAAGAACCAGGTGTCCCTGTGGTGTCTGGTCAAGGGCTTCTACCCCAGCGATATCGCCGTGGAGTGGGAGAGCAACGGCCAGCCTGAGAACAACTACAAGACCACCCCCCCTGTGCTGGACAGCGACGGCAGCTTCTTCCTGTACTCCAAACTGACCGTGGACAAGAGCCGGTGGCAGCAGGGCAACGTGTTCAGCTGCAGCGTGATGCACGAGGCCCTGCACAACCACTACACCCAGAAGTCCCTGAGCCTGAGCCCCGGCGGAGGCGGCGGAAGCGGAGGAGGAGGATCCAGAGAGGGCCCTGAGCTGAGCCCTGATGATCCTGCCGGACTGCTGGACCTGCGGCAGGGAATGTTTGCCCAGCTGGTGGCCCAGAACGTGCTGCTGATCGATGGCCCCCTGTCCTGGTACAGCGATCCTGGACTGGCTGGCGTGTCACTGACAGGCGGCCTGAGCTACAAAGAGGACACCAAAGAACTGGTGGTGGCCAAGGCCGGCGTGTACTACGTGTTCTTTCAGCTGGAACTGCGGAGAGTGGTGGCCGGCGAAGGATCTGGCTCTGTGTCTCTGGCCCTGCATCTGCAGCCTCTGAGATCTGCTGCTGGCGCCGCTGCTCTGGCACTGACAGTGGATCTGCCTCCTGCCAGCAGCGAGGCCCGGAATAGCGCATTTGGGTTTCAAGGCAGGCTGCTGCACCTGTCTGCCGGCCAGAGGCTGGGAGTGCATCTGCACACAGAGGCCAGGGCTAGACACGCCTGGCAGCTGACACAGGGCGCTACAGTGCTGGGCCTGTTCAGAGTGACCCCCGAGATTCCTGCCGGGCTC |

(continued)

| SEQ ID NO: | Description | Sequence |
|---|---|---|
| 166 | nucleotide sequence of DP47 light chain | GAAATCGTGTTAACGCAGTCTCCAGGCACCCTGTCTTTG TCTCCAGGGGAAAGAGCCACCCTCTCTTGCAGGGCCAGT CAGAGTGTTAGCAGCAGCTACTTAGCCTGGTACCAGCAG AAACCTGGCCAGGCTCCCAGGCTCCTCATCTATGGAGCA TCCAGCAGGGCCACTGGCATCCCAGACAGGTTCAGTGG CAGTGGATCCGGGACAGACTTCACTCTCACCATCAGCAG ACTGGAGCCTGAAGATTTTGCAGTGTATTACTGTCAGCA GTATGGTAGCTCACCGCTGACGTTCGGCCAGGGGACCA AAGTGGAAATCAAACGTACGGTGGCTGCACCATCTGTCT TCATCTTCCCGCCATCTGATGAGCAGTTGAAATCTGGAA CTGCCTCTGTTGTGTGCCTGCTGAATAACTTCTATCCCAG AGAGGCCAAAGTACAGTGGAAGGTGGATAACGCCCTCC AATCGGGTAACTCCCAGGAGAGTGTCACAGAGCAGGAC AGCAAGGACAGCACCTACAGCCTCAGCAGCACCCTGAC GCTGAGCAAAGCAGACTACGAGAAACACAAAGTCTACG CCTGCGAAGTCACCCATCAGGGCCTGAGCTCGCCCGTCA CAAAGAGCTTCAACAGGGGAGAGTGT |
| 104 | Fc hole dimeric 4-1BB (71-248) ligand chain | see Table 1 |
| 167 | DP47 Fc knob monomeric 4-1-BB (71-248) ligand | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGL |
| 168 | DP47 light chain | EIVLTQSPGTLSLSPGERATLSCRASQSVSSSYLAWYQQKP GQAPRLLIYGASSRATGIPDRFSGSGSGTDFTLTISRLEPEDF AVYYCQQYGSSPLTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

Table 22 shows the cDNA and amino acid sequences of the monovalent untargeted 4-1BB ligand (71-248) trimer-containing Fc (kih) fusion antigen binding molecule (Control I).

**Table 22: Sequences of monovalent DP47-untargeted human 4-1BB ligand trimer containing Fc (kih) fusion molecule Control I**

| SEQ ID NO: | Description | Sequence |
|---|---|---|
| 107 | nucleotide sequence of Fc hole monomeric 4-1BBL (71-248) chain | see Table 2 |
| 169 | nucleotide sequence of DP47 Fc knob dimeric 4-1BBL (71-248) | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCATCGGTCTTCCCCCTGGCACCCTC CTCCAAGAGCACCTCTGGGGGCACAGCGGCCCTGGGCT GCCTGGTCAAGGACTACTTCCCCGAACCGGTGACGGTGT CGTGGAACTCAGGCGCCCTGACCAGCGGCGTGCACACC TTCCCGGCTGTCCTACAGTCCTCAGGACTCTACTCCCTC AGCAGCGTGGTGACCGTGCCCTCCAGCAGCTTGGGCAC CCAGACCTACATCTGCAACGTGAATCACAAGCCCAGCA ACACCAAGGTGGACAAGAAAGTTGAGCCCAAATCTTGT GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCCTGCAGAGATGAGCTGACCAAGA ACCAGGTGTCCCTGTGGTGTCTGGTCAAGGGCTTCTACC CCAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCGT GGACAAGAGCCGGTGGCAGCAGGGCAACGTGTTCAGCT GCAGCGTGATGCACGAGGCCCTGCACAACCACTACACC CAGAAGTCCCTGAGCCTGAGCCCCGGCAGAGAGGGCCC TGAGCTGAGCCCTGATGATCCTGCCGGACTGCTGGACCT GCGGCAGGGAATGTTTGCCCAGCTGGTGGCCCAGAACG TGCTGCTGATCGATGGCCCCCTGTCCTGGTACAGCGATC CTGGACTGGCTGGCGTGTCACTGACAGGCGGCCTGAGCT ACAAAGAGGACACCAAAGAACTGGTGGTGGCCAAGGCC GGCGTGTACTACGTGTTCTTTCAGCTGGAACTGCGGAGA GTGGTGGCCGGCGAAGGATCTGGCTCTGTGTCTCTGGCC CTGCATCTGCAGCCTCTGAGATCTGCTGCTGGCGCCGCT GCTCTGGCACTGACAGTGGATCTGCCTCCTGCCAGCAGC GAGGCCCGGAATAGCGCATTTGGGTTTCAAGGCAGGCT GCTGCACCTGTCTGCCGGCCAGAGGCTGGGAGTGCATCT |

(continued)

| SEQ ID NO: | Description | Sequence |
|---|---|---|
| | | GCACACAGAGGCCAGGGCTAGACACGCCTGGCAGCTGACACAGGGCGCTACAGTGCTGGGCCTGTTCAGAGTGACCCCCGAGATTCCAGCAGGCCTGGGAGGCGGCGGATCTGGCGGCGGAGGATCTAGAGAAGGACCCGAGCTGTCCCCGACGATCCCGCTGGGCTGCTGGATCTGAGACAGGGCATGTTCGCTCAGCTGGTGGCTCAGAATGTGCTGCTGATTGACGGACCTCTGAGCTGGTACTCCGACCCAGGGCTGGCAGGGGTGTCCCTGACTGGGGGACTGTCCTACAAAGAAGATACAAAAGAACTGGTGGTGGCTAAAGCTGGGGTGTACTATGTGTTTTTTCAGCTGGAACTGAGGCGGGTGGTGGCTGGGGAGGGCTCAGGATCTGTGTCCCTGGCTCTGCATCTGCAGCCACTGCGCTCTGCAGCAGGGGCTGCAGCACTGGCCCTGACTGTGGACCTGCCCCCAGCTTCTTCCGAGGCCAGAAACAGCGCCTTCGGGTTCCAAGGACGCCTGCTGCATCTGAGCGCCGGACAGCGCCTGGGAGTGCATCTGCATACTGAAGCCAGAGCCCGGCATGCTTGGCAGCTGACTCAGGGGGCAACTGTGCTGGGACTGTTTCGCGTGACACCTGAGATCCCAGCCGGGCTC |
| 166 | nucleotide sequence of DP47 light chain | See Table 21 |
| 109 | Fc hole monomeric 4-1BB ligand (71-248) chain | see Table 2 |
| 170 | DP47 Fc knob dimeric 4-1BBL (71-248) | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCAKGSGFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 168 | DP47 light chain | See Table 21 |

**1.7 Preparation of monovalent FAP-targeted 4-1BB ligand trimer-containing Fc (kih) fusion antigen binding molecule, wherein the 4-1BB ligands are C-terminally fused (Constructs 1.13, 2.13, 1.14 and 2.14) (FAP on the hole chain)**

A polypeptide containing two ectodomains of 4-1BB ligand, separated by (G4S)2 linkers was subcloned in frame to the C-terminus of human IgG1 Fc hole or knob chain (Merchant, Zhu 1998), as depicted in **Figure 1a:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 1b:** human IgG1 Fc, (G4S)2 connector, human 4-1BB ligand.

The variable region of heavy and light chain DNA sequences encoding a binder specific for fibroblast activation protein (FAP), clone 4B9 or clone 28H1, were subcloned in frame with either the constant heavy chain of the hole or the constant light chain of human IgG1. The generation and preparation of the FAP binders is described in WO 2012/020006 A2, which is incorporated herein by reference.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831 A1.

For all constructs the knobs into holes heterodimerization technology was used as described in Carter, J Immunol Methods 248, 7-15 (2001). Combination of the huIgG1 Fc hole chain containing the Y349C/T366S/L368A/Y407V mutations in the CH3 domain, the anti-FAP huIgG1 knob chain containing the S354C/T366W mutations in the CH3 domain and the anti-FAP light chain allows generation of a heterodimer, which includes an assembled trimeric 4-1BB ligand and one FAP binding Fab **(Figures 2C and 2D)**.

Table 23 shows the cDNA and amino acid sequences of the monovalent FAP(4B9)-targeted 4-1BB ligand (71-248) trimer-containing Fc (kih) fusion antigen binding molecule (Construct 2.13).

**Table 23: Sequences of FAP(4B9)-targeted human 4-1BB ligand trimer containing Fc (kih) fusion molecule Construct 2.13 (comparative example)**

| SEQ ID NO: | Description | Sequence |
|---|---|---|
| 171 | nucleotide sequence of anti-FAP(4B9) Fc hole dimeric 4-1BB ligand (71-248) chain | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG |

(continued)

| SEQ ID NO: | Description | Sequence |
|---|---|---|
| | | AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCCTCCGTGTTCCCCCT GGCCCCCAGCAGCAAGAGCACCAGCGGCGGCACAGCCG CTCTGGGCTGCCTGGTCAAGGACTACTTCCCCGAGCCCG TGACCGTGTCCTGGAACAGCGGAGCCCTGACCTCCGGC GTGCACACCTTCCCCGCCGTGCTGCAGAGTTCTGGCCTG TATAGCCTGAGCAGCGTGGTCACCGTGCCTTCTAGCAGC CTGGGCACCCAGACCTACATCTGCAACGTGAACCACAA GCCCAGCAACACCAAGGTGGACAAGAAGGTGGAGCCCA AGAGCTGCGACAAAACTCACACATGCCCACCGTGCCCA GCACCTGAAGCTGCAGGGGGACCGTCAGTCTTCCTCTTC CCCCCAAAACCCAAGGACACCCTCATGATCTCCCGGACC CCTGAGGTCACATGCGTGGTGGTGGACGTGAGCCACGA AGACCCTGAGGTCAAGTTCAACTGGTACGTGGACGGCG TGGAGGTGCATAATGCCAAGACAAAGCCGCGGGAGGAG CAGTACAACAGCACGTACCGTGTGGTCAGCGTCCTCACC GTCCTGCACCAGGACTGGCTGAATGGCAAGGAGTACAA GTGCAAGGTCTCCAACAAAGCCCTCGGCGCCCCCATCG AGAAAACCATCTCCAAAGCCAAAGGGCAGCCCCGAGAA CCACAGGTGTGCACCCTGCCCCCATCCCGGGATGAGCTG ACCAAGAACCAGGTCAGCCTCTCGTGCGCAGTCAAAGG CTTCTATCCCAGCGACATCGCCGTGGAGTGGGAGAGCA ATGGGCAGCCGGAGAACAACTACAAGACCACGCCTCCC GTGCTGGACTCCGACGGCTCCTTCTTCCTCGTGAGCAAG CTCACCGTGGACAAGAGCAGGTGGCAGCAGGGGAACGT CTTCTCATGCTCCGTGATGCATGAGGCTCTGCACAACCA CTACACGCAGAAGAGCCTCTCCCTGTCTCCGGGTGGAGG CGGCGGAAGCGGAGGAGGAGGATCCAGAGAGGGCCCT GAGCTGAGCCCTGATGATCCTGCCGGACTGCTGGACCTG CGGCAGGGAATGTTTGCCCAGCTGGTGGCCCAGAACGT GCTGCTGATCGATGGCCCCCTGTCCTGGTACAGCGATCC TGGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGATCTGCTGCTGGCGCCGCTG CTCTGGCACTGACAGTGGATCTGCCTCCTGCCAGCAGCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC CCGAGATTCCAGCAGGCCTGGGAGGCGGCGGATCTGGC GGCGGAGGATCTAGAGAAGGACCCGAGCTGTCCCCCGA CGATCCCGCTGGGCTGCTGGATCTGAGACAGGGCATGTT CGCTCAGCTGGTGGCTCAGAATGTGCTGCTGATTGACGG ACCTCTGAGCTGGTACTCCGACCCAGGGCTGGCAGGGG TGTCCCTGACTGGGGGACTGTCCTACAAAGAAGATACA AAAGAACTGGTGGTGGCTAAAGCTGGGGTGTACTATGT GTTTTTTCAGCTGGAACTGAGGCGGGTGGTGGCTGGGGA GGGCTCAGGATCTGTGTCCCTGGCTCTGCATCTGCAGCC ACTGCGCTCTGCAGCAGGGGCTGCAGCACTGGCCCTGA |

(continued)

| SEQ ID NO: | Description | Sequence |
|---|---|---|
| | | CTGTGGACCTGCCCCCAGCTTCTTCCGAGGCCAGAAACA GCGCCTTCGGGTTCCAAGGACGCCTGCTGCATCTGAGCG CCGGACAGCGCCTGGGAGTGCATCTGCATACTGAAGCC AGAGCCCGGCATGCTTGGCAGCTGACTCAGGGGGCAAC TGTGCTGGGACTGTTTCGCGTGACACCTGAGATCCCAGC CGGGCTC |
| 172 | nucleotide sequence of Fc knob monomeric 4-1BB (71-248) ligand | GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCCTGCAGAGATGAGCTGACCAAGA ACCAGGTGTCCCTGTGGTGTCTGGTCAAGGGCTTCTACC CCAGCGATATCGCCGTGGAGTGGGAGAGCAACGGCCAG CCTGAGAACAACTACAAGACCACCCCCCCTGTGCTGGA CAGCGACGGCAGCTTCTTCCTGTACTCCAAACTGACCGT GGACAAGAGCCGGTGGCAGCAGGGCAACGTGTTCAGCT GCAGCGTGATGCACGAGGCCCTGCACAACCACTACACC CAGAAGTCCCTGAGCCTGAGCCCCGGCGGAGGCGGCGG AAGCGGAGGAGGAGGATCCAGAGAGGGCCCTGAGCTGA GCCCTGATGATCCTGCCGGACTGCTGGACCTGCGGCAGG GAATGTTTGCCCAGCTGGTGGCCCAGAACGTGCTGCTGA TCGATGGCCCCCTGTCCTGGTACAGCGATCCTGGACTGG CTGGCGTGTCACTGACAGGCGGCCTGAGCTACAAAGAG GACACCAAAGAACTGGTGGTGGCCAAGGCCGGCGTGTA CTACGTGTTCTTTCAGCTGGAACTGCGGAGAGTGGTGGC CGGCGAAGGATCTGGCTCTGTGTCTCTGGCCCTGCATCT GCAGCCTCTGAGATCTGCTGCTGGCGCCGCTGCTCTGGC ACTGACAGTGGATCTGCCTCCTGCCAGCAGCGAGGCCC GGAATAGCGCATTTGGGTTTCAAGGCAGGCTGCTGCACC TGTCTGCCGGCCAGAGGCTGGGAGTGCATCTGCACACA GAGGCCAGGGCTAGACACGCCTGGCAGCTGACACAGGG CGCTACAGTGCTGGGCCTGTTCAGAGTGACCCCCGAGAT TCCTGCCGGGCTC |
| 103 | nucleotide sequence of anti-FAP(4B9) light chain | see Table 1 |

(continued)

| SEQ ID NO: | Description | Sequence |
|---|---|---|
| 173 | anti-FAP(4B9) Fc hole dimeric 4-1BB (71-248) ligand chain | EVQLLESGGGLVQPGGSLRLSCAASGFTFSSYAMSWVRQAPGKGLEWVSAIIGSGASTYYADSVKGRFTISRDNSKNTLYLQMNSLRAEDTAVYYCAKGWFGGFNYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVCTLPPSRDELTKNQVSLSCAVKGFYPSDIAVEWESNGQPENNYKTTPPVLDSDGSFFLVSKLTVDKSRWQQGNVFSCSVMHEALHNHYTQKSLSLSPGGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 174 | Fc knob monomeric 41-BBL (71-248) | DKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 106 | anti-FAP(4B9) light chain | see Table 1 |

Table 24 shows the cDNA and amino acid sequences of the monovalent FAP(4B9)-targeted 4-1BB ligand (71-248) trimer-containing Fc (kih) fusion antigen binding molecule (Construct 2.14).

**Table 24: Sequences of FAP(4B9)-targeted human 4-1BB ligand trimer containing Fc (kih) fusion molecule Construct 2.14 (comparative example)**

| SEQ ID NO: | Description | Sequence |
|---|---|---|
| 175 | nucleotide sequence of anti-FAP(4B9) Fc hole monomeric 4-1BBL (71-248) chain | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCCTCCGTGTTCCCCCT GGCCCCCAGCAGCAAGAGCACCAGCGGCGGCACAGCCG |

(continued)

| SEQ ID NO: | Description | Sequence |
|---|---|---|
| | | CTCTGGGCTGCCTGGTCAAGGACTACTTCCCCGAGCCCG<br>TGACCGTGTCCTGGAACAGCGGAGCCCTGACCTCCGGC<br>GTGCACACCTTCCCCGCCGTGCTGCAGAGTTCTGGCCTG<br>TATAGCCTGAGCAGCGTGGTCACCGTGCCTTCTAGCAGC<br>CTGGGCACCCAGACCTACATCTGCAACGTGAACCACAA<br>GCCCAGCAACACCAAGGTGGACAAGAAGGTGGAGCCCA<br>AGAGCTGCGACAAAACTCACACATGCCCACCGTGCCCA<br>GCACCTGAAGCTGCAGGGGGACCGTCAGTCTTCCTCTTC<br>CCCCCAAAACCCAAGGACACCCTCATGATCTCCCGGACC<br>CCTGAGGTCACATGCGTGGTGGTGGACGTGAGCCACGA<br>AGACCCTGAGGTCAAGTTCAACTGGTACGTGGACGGCG<br>TGGAGGTGCATAATGCCAAGACAAAGCCGCGGGAGGAG<br>CAGTACAACAGCACGTACCGTGTGGTCAGCGTCCTCACC<br>GTCCTGCACCAGGACTGGCTGAATGGCAAGGAGTACAA<br>GTGCAAGGTCTCCAACAAAGCCCTCGGCGCCCCCATCG<br>AGAAAACCATCTCCAAAGCCAAAGGGCAGCCCCGAGAA<br>CCACAGGTGTGCACCCTGCCCCCATCCCGGGATGAGCTG<br>ACCAAGAACCAGGTCAGCCTCTCGTGCGCAGTCAAAGG<br>CTTCTATCCCAGCGACATCGCCGTGGAGTGGGAGAGCA<br>ATGGGCAGCCGGAGAACAACTACAAGACCACGCCTCCC<br>GTGCTGGACTCCGACGGCTCCTTCTTCCTCGTGAGCAAG<br>CTCACCGTGGACAAGAGCAGGTGGCAGCAGGGGAACGT<br>CTTCTCATGCTCCGTGATGCATGAGGCTCTGCACAACCA<br>CTACACGCAGAAGAGCCTCTCCCTGTCTCCGGGTGGAGG<br>CGGCGGAAGCGGAGGAGGAGGATCCAGAGAGGGCCCT<br>GAGCTGAGCCCTGATGATCCTGCCGGACTGCTGGACCTG<br>CGGCAGGGAATGTTTGCCCAGCTGGTGGCCCAGAACGT<br>GCTGCTGATCGATGGCCCCCTGTCCTGGTACAGCGATCC<br>TGGACTGGCTGGCGTGTCACTGACAGGCGGCCTGAGCTA<br>CAAAGAGGACACCAAAGAACTGGTGGTGGCCAAGGCCG<br>GCGTGTACTACGTGTTCTTTCAGCTGGAACTGCGGAGAG<br>TGGTGGCCGGCGAAGGATCTGGCTCTGTGTCTCTGGCCC<br>TGCATCTGCAGCCTCTGAGATCTGCTGCTGGCGCCGCTG<br>CTCTGGCACTGACAGTGGATCTGCCTCCTGCCAGCAGCG<br>AGGCCCGGAATAGCGCATTTGGGTTTCAAGGCAGGCTG<br>CTGCACCTGTCTGCCGGCCAGAGGCTGGGAGTGCATCTG<br>CACACAGAGGCCAGGGCTAGACACGCCTGGCAGCTGAC<br>ACAGGGCGCTACAGTGCTGGGCCTGTTCAGAGTGACCC<br>CCGAGATTCCTGCCGGGCTC |

(continued)

| SEQ ID NO: | Description | Sequence |
|---|---|---|
| 176 | nucleotide sequence of Fc knob dimeric 4-1BB ligand (71-248) | GACAAAACTCACACATGCCCACCGTGCCCAGCACCTGA<br>AGCTGCAGGGGGACCGTCAGTCTTCCTCTTCCCCCCAAA<br>ACCCAAGGACACCCTCATGATCTCCCGGACCCCTGAGGT<br>CACATGCGTGGTGGTGGACGTGAGCCACGAAGACCCTG<br>AGGTCAAGTTCAACTGGTACGTGGACGGCGTGGAGGTG<br>CATAATGCCAAGACAAAGCCGCGGGAGGAGCAGTACAA<br>CAGCACGTACCGTGTGGTCAGCGTCCTCACCGTCCTGCA<br>CCAGGACTGGCTGAATGGCAAGGAGTACAAGTGCAAGG<br>TCTCCAACAAAGCCCTCGGCGCCCCCATCGAGAAAACC<br>ATCTCCAAAGCCAAAGGGCAGCCCCGAGAACCACAGGT<br>GTACACCCTGCCCCCCTGCAGAGATGAGCTGACCAAGA<br>ACCAGGTGTCCCTGTGGTGTCTGGTCAAGGGCTTCTACC<br>CCAGCGATATCGCCGTGGAGTGGGAGAGCAACGGCCAG<br>CCTGAGAACAACTACAAGACCACCCCCCCTGTGCTGGA<br>CAGCGACGGCAGCTTCTTCCTGTACTCCAAACTGACCGT |
| | | GGACAAGAGCCGGTGGCAGCAGGGCAACGTGTTCAGCT<br>GCAGCGTGATGCACGAGGCCCTGCACAACCACTACACC<br>CAGAAGTCCCTGAGCCTGAGCCCCGGCGGAGGCGGCGG<br>AAGCGGAGGAGGAGGATCCAGAGAGGGCCCTGAGCTGA<br>GCCCTGATGATCCTGCCGGACTGCTGGACCTGCGGCAGG<br>GAATGTTTGCCCAGCTGGTGGCCCAGAACGTGCTGCTGA<br>TCGATGGCCCCCTGTCCTGGTACAGCGATCCTGGACTGG<br>CTGGCGTGTCACTGACAGGCGGCCTGAGCTACAAAGAG<br>GACACCAAAGAACTGGTGGTGGCCAAGGCCGGCGTGTA<br>CTACGTGTTCTTTCAGCTGGAACTGCGGAGAGTGGTGGC<br>CGGCGAAGGATCTGGCTCTGTGTCTCTGGCCCTGCATCT<br>GCAGCCTCTGAGATCTGCTGCTGGCGCCGCTGCTCTGGC<br>ACTGACAGTGGATCTGCCTCCTGCCAGCAGCGAGGCCC<br>GGAATAGCGCATTTGGGTTTCAAGGCAGGCTGCTGCACC<br>TGTCTGCCGGCCAGAGGCTGGGAGTGCATCTGCACACA<br>GAGGCCAGGGCTAGACACGCCTGGCAGCTGACACAGGG<br>CGCTACAGTGCTGGGCCTGTTCAGAGTGACCCCCGAGAT<br>TCCAGCAGGCCTGGGAGGCGGCGGATCTGGCGGCGGAG<br>GATCTAGAGAAGGACCCGAGCTGTCCCCCGACGATCCC<br>GCTGGGCTGCTGGATCTGAGACAGGGCATGTTCGCTCAG<br>CTGGTGGCTCAGAATGTGCTGCTGATTGACGGACCTCTG<br>AGCTGGTACTCCGACCCAGGGCTGGCAGGGGTGTCCCT<br>GACTGGGGGACTGTCCTACAAAGAAGATACAAAAGAAC<br>TGGTGGTGGCTAAAGCTGGGGTGTACTATGTGTTTTTC<br>AGCTGGAACTGAGGCGGGTGGTGGCTGGGGAGGGCTCA<br>GGATCTGTGTCCCTGGCTCTGCATCTGCAGCCACTGCGC<br>TCTGCAGCAGGGGCTGCAGCACTGGCCCTGACTGTGGA<br>CCTGCCCCCAGCTTCTTCCGAGGCCAGAAACAGCGCCTT<br>CGGGTTCCAAGGACGCCTGCTGCATCTGAGCGCCGGAC<br>AGCGCCTGGGAGTGCATCTGCATACTGAAGCCAGAGCC<br>CGGCATGCTTGGCAGCTGACTCAGGGGGCAACTGTGCTG<br>GGACTGTTTCGCGTGACACCTGAGATCCCAGCCGGGCTC |

(continued)

| SEQ ID NO: | Description | Sequence |
|---|---|---|
| 103 | nucleotide sequence of anti-FAP(4B9) light chain | See Table 1 |
| 177 | anti-FAP(4B9) Fc hole monomeric 4-1BB ligand (71-248) chain | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV |
| | | HLHTEARARHAWQLTQGATVLGLFRVTPEIPAGL |
| 178 | Fc knob dimeric 4-1BB ligand (71-248) | DKTHTCPPCPAPEAAGGPSVFLFPPKPKDTLMISRTPEVTC VVVDVSHEDPEVKFNWYVDGVEVHNAKTKPREEQYNSTY RVVSVLTVLHQDWLNGKEYKCKVSNKALGAPIEKTISKAK GQPREPQVYTLPPCRDELTKNQVSLWCLVKGFYPSDIAVE WESNGQPENNYKTTPPVLDSDGSFFLYSKLTVDKSRWQQ GNVFSCSVMHEALHNHYTQKSLSLSPGGGGGSGGGGSRE GPELSPDDPAGLLDLRQGMFAQLVAQNVLLIDGPLSWYSD PGLAGVSLTGGLSYKEDTKELVVAKAGVYYVFFQLELRRV VAGEGSGSVSLALHLQPLRSAAGAAALALTVDLPPASSEA RNSAFGFQGRLLHLSAGQRLGVHLHTEARARHAWQLTQG ATVLGLFRVTPEIPAGLGGGGSGGGGSREGPELSPDDPAGL LDLRQGMFAQLVAQNVLLIDGPLSWYSDPGLAGVSLTGGL SYKEDTKELVVAKAGVYYVFFQLELRRVVAGEGSGSVSL ALHLQPLRSAAGAAALALTVDLPPASSEARNSAFGFQGRL LHLSAGQRLGVHLHTEARARHAWQLTQGATVLGLFRVTP EIPAGL |
| 106 | anti-FAP(4B9) light chain | See Table 1 |

Table 25 shows the cDNA and amino acid sequences of the monovalent FAP(28H1)-targeted 4-1BB ligand trimer-containing Fc (kih) fusion antigen binding molecule (Construct 1.13).

**Table 25: Sequences of FAP(28H1)-targeted human 4-1BB ligand trimer containing Fc (kih) fusion molecule Construct 1.13 (comparative example)**

| SEQ ID NO: | Description | Sequence |
|---|---|---|
| 179 | nucleotide sequence of anti-FAP(28H1) Fc hole dimeric 4-1BB ligand (71-248) chain | GAAGTGCAGCTGCTGGAATCCGGCGGAGGCCTGGTGCA GCCTGGCGGATCTCTGAGACTGTCCTGCGCCGCCTCCGG CTTCACCTTCTCCTCCCACGCCATGTCCTGGGTCCGACA GGCTCCTGGCAAAGGCCTGGAATGGGTGTCCGCCATCTG GGCCTCCGGCGAGCAGTACTACGCCGACTCTGTGAAGG GCCGGTTCACCATCTCCCGGGACAACTCCAAGAACACCC TGTACCTGCAGATGAACTCCCTGCGGGCCGAGGACACC GCCGTGTACTACTGTGCCAAGGGCTGGCTGGGCAACTTC GACTACTGGGGACAGGGCACCCTGGTCACCGTGTCCAG CGCTAGCACCAAGGGCCCCTCCGTGTTCCCCCTGGCCCC CAGCAGCAAGAGCACCAGCGGCGGCACAGCCGCTCTGG GCTGCCTGGTCAAGGACTACTTCCCCGAGCCCGTGACCG TGTCCTGGAACAGCGGAGCCCTGACCTCCGGCGTGCAC ACCTTCCCCGCCGTGCTGCAGAGTTCTGGCCTGTATAGC CTGAGCAGCGTGGTCACCGTGCCTTCTAGCAGCCTGGGC ACCCAGACCTACATCTGCAACGTGAACCACAAGCCCAG CAACACCAAGGTGGACAAGAAGGTGGAGCCCAAGAGCT GCGACAAAACTCACACATGCCCACCGTGCCCAGCACCT |

(continued)

| SEQ ID NO: | Description | Sequence |
|---|---|---|
| | | GAAGCTGCAGGGGGACCGTCAGTCTTCCTCTTCCCCCA AAACCCAAGGACACCCTCATGATCTCCCGGACCCCTGA GGTCACATGCGTGGTGGTGGACGTGAGCCACGAAGACC CTGAGGTCAAGTTCAACTGGTACGTGGACGGCGTGGAG GTGCATAATGCCAAGACAAAGCCGCGGGAGGAGCAGTA CAACAGCACGTACCGTGTGGTCAGCGTCCTCACCGTCCT GCACCAGGACTGGCTGAATGGCAAGGAGTACAAGTGCA AGGTCTCCAACAAAGCCCTCGGCGCCCCCATCGAGAAA ACCATCTCCAAAGCCAAAGGGCAGCCCCGAGAACCACA GGTGTGCACCCTGCCCCCATCCCGGGATGAGCTGACCAA GAACCAGGTCAGCCTCTCGTGCGCAGTCAAAGGCTTCTA TCCCAGCGACATCGCCGTGGAGTGGGAGAGCAATGGGC AGCCGGAGAACAACTACAAGACCACGCCTCCCGTGCTG GACTCCGACGGCTCCTTCTTCCTCGTGAGCAAGCTCACC GTGGACAAGAGCAGGTGGCAGCAGGGGAACGTCTTCTC ATGCTCCGTGATGCATGAGGCTCTGCACAACCACTACAC GCAGAAGAGCCTCTCCCTGTCTCCGGGTGGAGGCGGCG GAAGCGGAGGAGGAGGATCCAGAGAGGGCCCTGAGCTG AGCCCTGATGATCCTGCCGGACTGCTGGACCTGCGGCAG GGAATGTTTGCCCAGCTGGTGGCCCAGAACGTGCTGCTG ATCGATGGCCCCCTGTCCTGGTACAGCGATCCTGGACTG GCTGGCGTGTCACTGACAGGCGGCCTGAGCTACAAAGA GGACACCAAAGAACTGGTGGTGGCCAAGGCCGGCGTGT ACTACGTGTTCTTTCAGCTGGAACTGCGGAGAGTGGTGG CCGGCGAAGGATCTGGCTCTGTGTCTCTGGCCCTGCATC TGCAGCCTCTGAGATCTGCTGCTGGCGCCGCTGCTCTGG CACTGACAGTGGATCTGCCTCCTGCCAGCAGCGAGGCCC GGAATAGCGCATTTGGGTTTCAAGGCAGGCTGCTGCACC TGTCTGCCGGCCAGAGGCTGGGAGTGCATCTGCACACA GAGGCCAGGGCTAGACACGCCTGGCAGCTGACACAGGG CGCTACAGTGCTGGGCCTGTTCAGAGTGACCCCCGAGAT TCCAGCAGGCCTGGGAGGCGGCGGATCTGGCGGCGGAG GATCTAGAGAAGGACCCGAGCTGTCCCCCGACGATCCC GCTGGGCTGCTGGATCTGAGACAGGGCATGTTCGCTCAG CTGGTGGCTCAGAATGTGCTGCTGATTGACGGACCTCTG AGCTGGTACTCCGACCCAGGGCTGGCAGGGGTGTCCCT GACTGGGGGACTGTCCTACAAAGAAGATACAAAAGAAC TGGTGGTGGCTAAAGCTGGGGTGTACTATGTGTTTTTC AGCTGGAACTGAGGCGGGTGGTGGCTGGGGAGGGCTCA GGATCTGTGTCCCTGGCTCTGCATCTGCAGCCACTGCGC TCTGCAGCAGGGGCTGCAGCACTGGCCCTGACTGTGGA CCTGCCCCCAGCTTCTTCCGAGGCCAGAAACAGCGCCTT CGGGTTCCAAGGACGCCTGCTGCATCTGAGCGCCGGAC AGCGCCTGGGAGTGCATCTGCATACTGAAGCCAGAGCC CGGCATGCTTGGCAGCTGACTCAGGGGGCAACTGTGCTG GGACTGTTTCGCGTGACACCTGAGATCCCAGCCGGGCTC |
| 172 | nucleotide sequence of Fc knob monomeric 4-1BB ligand (71-248) | see Table 23 |

(continued)

| SEQ ID NO: | Description | Sequence |
|---|---|---|
| 112 | nucleotide sequence of anti-FAP(28H1) light chain | see Table 3 |
| 180 | anti-FAP(28H1)Fchole dimeric 4-1BB ligand (71-248) chain | EVQLLESGGGLVQPGGSLRLSCAASGFTFSSHAMSWVRQA PGKGLEWVSAIWASGEQYYADSVKGRFTISRDNSKNTLYL QMNSLRAEDTAVYYCAKGWLGNFDYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGLGGGGSG GGGSREGPELSPDDPAGLLDLRQGMFAQLVAQNVLLIDGP LSWYSDPGLAGVSLTGGLSYKEDTKELVVAKAGVYYVFF QLELRRVVAGEGSGSVSLALHLQPLRSAAGAAALALTVDL PPASSEARNSAFGFQGRLLHLSAGQRLGVHLHTEARARHA WQLTQGATVLGLFRVTPEIPAGL |
| 174 | Fc knob monomeric 4-1BB ligand (71-248) | see Table 23 |
| 114 | anti-FAP(28H1) light chain | see Table 3 |

Table 26 shows the cDNA and amino acid sequences of the monovalent FAP(28H1)-targeted 4-1BB ligand trimer-containing Fc (kih) fusion antigen binding molecule (Construct 1.14).

**Table 26: Sequences of FAP(28H1)-targeted human 4-1BB ligand trimer containing Fc (kih) fusion molecule Construct 1.14 (comparative example)**

| SEQ ID NO: | Description | Sequence |
|---|---|---|

| | | |
|---|---|---|
| 181 | nucleotide sequence of anti-FAP(28H1) Fc hole monomeric 4-1BB ligand (71-248) chain | GAAGTGCAGCTGCTGGAATCCGGCGGAGGCCTGGTGCA<br>GCCTGGCGGATCTCTGAGACTGTCCTGCGCCGCCTCCGG<br>CTTCACCTTCTCCTCCCACGCCATGTCCTGGGTCCGACA<br>GGCTCCTGGCAAAGGCCTGGAATGGGTGTCCGCCATCTG<br>GGCCTCCGGCGAGCAGTACTACGCCGACTCTGTGAAGG<br>GCCGGTTCACCATCTCCCGGGACAACTCCAAGAACACCC<br>TGTACCTGCAGATGAACTCCCTGCGGGCCGAGGACACC<br>GCCGTGTACTACTGTGCCAAGGGCTGGCTGGGCAACTTC<br>GACTACTGGGGACAGGGCACCCTGGTCACCGTGTCCAG<br>CGCTAGCACCAAGGGCCCCTCCGTGTTCCCCCTGGCCCC<br>CAGCAGCAAGAGCACCAGCGGCGGCACAGCCGCTCTGG<br>GCTGCCTGGTCAAGGACTACTTCCCCGAGCCCGTGACCG<br>TGTCCTGGAACAGCGGAGCCCTGACCTCCGGCGTGCAC<br>ACCTTCCCCGCCGTGCTGCAGAGTTCTGGCCTGTATAGC<br>CTGAGCAGCGTGGTCACCGTGCCTTCTAGCAGCCTGGGC<br>ACCCAGACCTACATCTGCAACGTGAACCACAAGCCCAG<br>CAACACCAAGGTGGACAAGAAGGTGGAGCCCAAGAGCT<br>GCGACAAAACTCACACATGCCCACCGTGCCCAGCACCT<br>GAAGCTGCAGGGGGACCGTCAGTCTTCCTCTTCCCCCCA<br>AAACCCAAGGACACCCTCATGATCTCCCGGACCCCTGA<br>GGTCACATGCGTGGTGGTGGACGTGAGCCACGAAGACC<br>CTGAGGTCAAGTTCAACTGGTACGTGGACGGCGTGGAG<br>GTGCATAATGCCAAGACAAAGCCGCGGGAGGAGCAGTA<br>CAACAGCACGTACCGTGTGGTCAGCGTCCTCACCGTCCT<br>GCACCAGGACTGGCTGAATGGCAAGGAGTACAAGTGCA<br>AGGTCTCCAACAAAGCCCTCGGCGCCCCCATCGAGAAA<br>ACCATCTCCAAAGCCAAAGGGCAGCCCCGAGAACCACA<br>GGTGTGCACCCTGCCCCCATCCCGGGATGAGCTGACCAA<br>GAACCAGGTCAGCCTCTCGTGCGCAGTCAAAGGCTTCTA<br>TCCCAGCGACATCGCCGTGGAGTGGGAGAGCAATGGGC<br>AGCCGGAGAACAACTACAAGACCACGCCTCCCGTGCTG<br>GACTCCGACGGCTCCTTCTTCCTCGTGAGCAAGCTCACC<br>GTGGACAAGAGCAGGTGGCAGCAGGGGAACGTCTTCTC<br>ATGCTCCGTGATGCATGAGGCTCTGCACAACCACTACAC<br>GCAGAAGAGCCTCTCCCTGTCTCCGGGTGGAGGCGGCG<br>GAAGCGGAGGAGGAGGATCCAGAGAGGGCCCTGAGCTG<br>AGCCCTGATGATCCTGCCGGACTGCTGGACCTGCGGCAG<br>GGAATGTTTGCCCAGCTGGTGGCCCAGAACGTGCTGCTG<br>ATCGATGGCCCCCTGTCCTGGTACAGCGATCCTGGACTG<br>GCTGGCGTGTCACTGACAGGCGGCCTGAGCTACAAAGA<br>GGACACCAAAGAACTGGTGGTGGCCAAGGCCGGCGTGT<br>ACTACGTGTTCTTTCAGCTGGAACTGCGGAGAGTGGTGG<br>CCGGCGAAGGATCTGGCTCTGTGTCTCTGGCCCTGCATC<br>TGCAGCCTCTGAGATCTGCTGCTGGCGCCGCTGCTCTGG<br>CACTGACAGTGGATCTGCCTCCTGCCAGCAGCGAGGCCC<br>GGAATAGCGCATTTGGGTTTCAAGGCAGGCTGCTGCACC<br>TGTCTGCCGGCCAGAGGCTGGGAGTGCATCTGCACACA<br>GAGGCCAGGGCTAGACACGCCTGGCAGCTGACACAGGG<br>CGCTACAGTGCTGGGCCTGTTCAGAGTGACCCCCGAGAT<br>TCCTGCCGGGCTC |
| 176 | nucleotide sequence of Fc knob dimeric 4- | see Table 24 |

| | | |
|---|---|---|
| | 1BBL (71-248) | |
| 112 | nucleotide sequence of anti-FAP(28H1) light chain | See Table 3 |
| 182 | anti-FAP(28H1) Fc hole monomeric 4-1BBL (71-248) chain | EVQLLESGGGLVQPGGSLRLSCAASGFTFSSHAMSWVRQA PGKGLEWVSAIWASGEQYYADSVKGRFTISRDNSKNTLYL QMNSLRAEDTAVYYCAKGWLGNFDYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGL |
| 178 | Fc knob dimeric 4-1BB ligand (71-248) | see Table 24 |
| 114 | anti-FAP(28H1) light chain | See Table 3 |

### 1.8 Preparation of monovalent CD19-targeted 4-1BB ligand trimer-containing Fc (kih) fusion antigen binding molecule, wherein the 4-1BB ligands are C-terminally fused (Constructs 3.13, 4.13, 3.14 and 4.14) (CD19 on the hole chain)

The molecules are prepared as described in Example 1.7 for the monovalent FAP- targeted 4-1BB ligand trimer-containing Fc (kih) fusion antigen binding molecules, with the only difference that the anti-FAP binder was replaced by an anti-CD 19 binder.

The variable region of heavy and light chain DNA sequences encoding a binder specific for CD19, clone 8B8-018 or 8B8-2B11, are subcloned in frame with either the constant heavy chain of the hole or the constant light chain of human IgG1.

Combination of the anti-CD19 huIgG1 Fc hole chain containing the Y349C/T366S/L368A/Y407V mutations, the huIgG1 knob chain containing the S354C/T366W mutations and the anti-CD 19 light chain allows generation of a heterodimer, which includes an assembled trimeric 4-1BB ligand and one CD19 binding Fab (Figures 2C or 2D).

Table 27 shows, respectively, the cDNA and amino acid sequences of the monovalent targeted CD19(8B8-018) split trimeric 4-1BB ligand (71-248) C-terminal Fc (kih) fusion antigen binding molecule (construct 3.13).

**Table 27: Sequences of CD19(8B8-018)-targeted human 4-1BB ligand trimer containing Fc (kih) fusion molecule Construct 3.13 (comparative example)**

| SEQ ID NO: | Description | Sequence |
|---|---|---|
| 183 | nucleotide sequence ofanti-CD19(8B8-018) Fc hole dimeric 4-1BB ligand (71-248) chain | CAGGTCCAGCTGGTGCAGTCCGGCGCCGAGGTCAAGAA ACCCGGGGCTTCTGTGAAGGTTTCATGCAAGGCAAGCG GATACACCTTCACCGACTATATCATGCATTGGGTCAGGC AGGCCCCTGGCCAAGGTCTCGAATGGATGGGCTACATTA ACCCATATAATGATGGCTCCAAATACACCGAGAAGTTTC AGGGAAGAGTCACTATGACATCTGACACCAGTATCAGC ACTGCTTACATGGAGCTGTCCCGCCTTCGGTCTGATGAC ACCGCAGTGTATTACTGTGCCAGGGGCACATATTACTAC GGCTCAGCTCTGTTCGACTATTGGGGGCAGGGAACCACA GTAACCGTGAGCTCCGCTAGCACCAAGGGCCCCTCCGTG TTCCCCCTGGCCCCCAGCAGCAAGAGCACCAGCGGCGG CACAGCCGCTCTGGGCTGCCTGGTCAAGGACTACTTCCC CGAGCCCGTGACCGTGTCCTGGAACAGCGGAGCCCTGA CCTCCGGCGTGCACACCTTCCCCGCCGTGCTGCAGAGTT CTGGCCTGTATAGCCTGAGCAGCGTGGTCACCGTGCCTT CTAGCAGCCTGGGCACCCAGACCTACATCTGCAACGTG AACCACAAGCCCAGCAACACCAAGGTGGACAAGAAGGT GGAGCCCAAGAGCTGCGACAAAACTCACACATGCCCAC CGTGCCCAGCACCTGAAGCTGCAGGGGGACCGTCAGTC TTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTGCACCCTGCCCCCATCCCGG GATGAGCTGACCAAGAACCAGGTCAGCCTCTCGTGCGC AGTCAAAGGCTTCTATCCCAGCGACATCGCCGTGGAGTG GGAGAGCAATGGGCAGCCGGAGAACAACTACAAGACC ACGCCTCCCGTGCTGGACTCCGACGGCTCCTTCTTCCTC GTGAGCAAGCTCACCGTGGACAAGAGCAGGTGGCAGCA GGGGAACGTCTTCTCATGCTCCGTGATGCATGAGGCTCT GCACAACCACTACACGCAGAAGAGCCTCTCCCTGTCTCC |

(continued)

| SEQ ID NO: | Description | Sequence |
|---|---|---|
| | | GGGTGGAGGCGGCGGAAGCGGAGGAGGAGGATCCAGA GAGGGCCCTGAGCTGAGCCCTGATGATCCTGCCGGACT GCTGGACCTGCGGCAGGGAATGTTTGCCCAGCTGGTGGC CCAGAACGTGCTGCTGATCGATGGCCCCCTGTCCTGGTA CAGCGATCCTGGACTGGCTGGCGTGTCACTGACAGGCG GCCTGAGCTACAAAGAGGACACCAAAGAACTGGTGGTG GCCAAGGCCGGCGTGTACTACGTGTTCTTTCAGCTGGAA CTGCGGAGAGTGGTGGCCGGCGAAGGATCTGGCTCTGT GTCTCTGGCCCTGCATCTGCAGCCTCTGAGATCTGCTGC TGGCGCCGCTGCTCTGGCACTGACAGTGGATCTGCCTCC TGCCAGCAGCGAGGCCCGGAATAGCGCATTTGGGTTTCA AGGCAGGCTGCTGCACCTGTCTGCCGGCCAGAGGCTGG GAGTGCATCTGCACACAGAGGCCAGGGCTAGACACGCC TGGCAGCTGACACAGGGCGCTACAGTGCTGGGCCTGTTC AGAGTGACCCCCGAGATTCCAGCAGGCCTGGGAGGCGG CGGATCTGGCGGCGGAGGATCTAGAGAAGGACCCGAGC TGTCCCCCGACGATCCCGCTGGGCTGCTGGATCTGAGAC AGGGCATGTTCGCTCAGCTGGTGGCTCAGAATGTGCTGC TGATTGACGGACCTCTGAGCTGGTACTCCGACCCAGGGC TGGCAGGGGTGTCCCTGACTGGGGGACTGTCCTACAAA GAAGATACAAAAGAACTGGTGGTGGCTAAAGCTGGGGT GTACTATGTGTTTTTTCAGCTGGAACTGAGGCGGGTGGT GGCTGGGGAGGGCTCAGGATCTGTGTCCCTGGCTCTGCA TCTGCAGCCACTGCGCTCTGCAGCAGGGGCTGCAGCACT GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |
| 172 | nucleotide sequence of Fc knob monomeric 4-1BB (71-248) ligand | see Table 23 |
| 118 | nucleotide sequence ofanti-CD19(8B8-018) light chain | see Table 5 |
| 184 | anti- CD19(8B8-018) Fc hole dimeric 4-1BB (71-248) ligand chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP |

(continued)

| SEQ ID NO: | Description | Sequence |
|---|---|---|
| | | VLDSDGSFFLVSKLTVDKSRWQQGNVFSCSVMHEALHNHYTQKSLSLSPGGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGLGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 174 | Fc knob monomeric 41-BBL (71-248) | see Table 23 |
| 120 | anti-FAP(4B9) light chain | see Table 5 |

Table 28 shows the cDNA and amino acid sequences of the monovalent CD19(8B8-018)-targeted 4-1BB ligand (71-248) trimer-containing Fc (kih) fusion antigen binding molecule (Construct 3.14).

**Table 28: Sequences of CD19(8B8-018)-targeted human 4-1BB ligand trimer containing Fc (kih) fusion molecule Construct 3.14 (comparative example)**

| SEQ ID NO: | Description | Sequence |
|---|---|---|
| 185 | nucleotide sequence ofanti-CD19(8B8-018) Fc hole monomeric 4-1BBL (71-248) chain | CAGGTCCAGCTGGTGCAGTCCGGCGCCGAGGTCAAGAAACCCGGGGCTTCTGTGAAGGTTTCATGCAAGGCAAGCGGATACACCTTCACCGACTATATCATGCATTGGGTCAGGCAGGCCCCTGGCCAAGGTCTCGAATGGATGGGCTACATTAACCCATATAATGATGGCTCCAAATACACCGAGAAGTTTCAGGGAAGAGTCACTATGACATCTGACACCAGTATCAGCACTGCTTACATGGAGCTGTCCCGCCTTCGGTCTGATGACACCGCAGTGTATTACTGTGCCAGGGGCACATATTACTACGGCTCAGCTCTGTTCGACTATTGGGGGCAGGGAACCACAGTAACCGTGAGCTCCGCTAGCACCAAGGGCCCCTCCGTGTTCCCCCTGGCCCCCAGCAGCAAGAGCACCAGCGGCGGCACAGCCGCTCTGGGCTGCCTGGTCAAGGACTACTTCCCCGAGCCCGTGACCGTGTCCTGGAACAGCGGAGCCCTGACCTCCGGCGTGCACACCTTCCCCGCCGTGCTGCAGAGTTCTGGCCTGTATAGCCTGAGCAGCGTGGTCACCGTGCCTTCTAGCAGCCTGGGCACCCAGACCTACATCTGCAACGTGAACCACAAGCCCAGCAACACCAAGGTGGACAAGAAGGTGGAGCCCAAGAGCTGCGACAAAACTCACACATGCCCACCGTGCCCAGCACCTGAAGCTGCAGGGGGACCGTCAGTC |

(continued)

| SEQ ID NO: | Description | Sequence |
|---|---|---|
| | | TTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTGCACCCTGCCCCCATCCCGG GATGAGCTGACCAAGAACCAGGTCAGCCTCTCGTGCGC AGTCAAAGGCTTCTATCCCAGCGACATCGCCGTGGAGTG GGAGAGCAATGGGCAGCCGGAGAACAACTACAAGACC ACGCCTCCCGTGCTGGACTCCGACGGCTCCTTCTTCCTC GTGAGCAAGCTCACCGTGGACAAGAGCAGGTGGCAGCA GGGGAACGTCTTCTCATGCTCCGTGATGCATGAGGCTCT GCACAACCACTACACGCAGAAGAGCCTCTCCCTGTCTCC GGGTGGAGGCGGCGGAAGCGGAGGAGGAGGATCCAGA GAGGGCCCTGAGCTGAGCCCTGATGATCCTGCCGGACT GCTGGACCTGCGGCAGGGAATGTTTGCCCAGCTGGTGGC CCAGAACGTGCTGCTGATCGATGGCCCCCTGTCCTGGTA CAGCGATCCTGGACTGGCTGGCGTGTCACTGACAGGCG GCCTGAGCTACAAAGAGGACACCAAAGAACTGGTGGTG GCCAAGGCCGGCGTGTACTACGTGTTCTTTCAGCTGGAA CTGCGGAGAGTGGTGGCCGGCGAAGGATCTGGCTCTGT GTCTCTGGCCCTGCATCTGCAGCCTCTGAGATCTGCTGC TGGCGCCGCTGCTCTGGCACTGACAGTGGATCTGCCTCC TGCCAGCAGCGAGGCCCGGAATAGCGCATTTGGGTTTCA AGGCAGGCTGCTGCACCTGTCTGCCGGCCAGAGGCTGG GAGTGCATCTGCACACAGAGGCCAGGGCTAGACACGCC TGGCAGCTGACACAGGGCGCTACAGTGCTGGGCCTGTTC AGAGTGACCCCCGAGATTCCTGCCGGGCTC |
| 176 | nucleotide sequence of Fc knob dimeric 4-1BB ligand (71-248) | see Table 24 |
| 118 | nucleotide sequence ofanti-CD19(8B8-018) light chain | See Table 5 |
| 186 | anti- CD19(8B8-018) Fc hole monomeric 4-1BB ligand (71-248) chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD |

(continued)

| SEQ ID NO: | Description | Sequence |
|---|---|---|
| | | ELTKNQVSLSCAVKGFYPSDIAVEWESNGQPENNYKTTPP VLDSDGSFFLVSKLTVDKSRWQQGNVFSCSVMHEALHNH YTQKSLSLSPGGGGGSGGGGSREGPELSPDDPAGLLDLRQ GMFAQLVAQNVLLIDGPLSWYSDPGLAGVSLTGGLSYKED TKELVVAKAGVYYVFFQLELRRVVAGEGSGSVSLALHLQP LRSAAGAAALALTVDLPPASSEARNSAFGFQGRLLHLSAG QRLGVHLHTEARARHAWQLTQGATVLGLFRVTPEIPAGL |
| 178 | Fc knob dimeric 4-1BB ligand (71-248) | see Table 24 |
| 120 | anti-CD19(8B8-018) light chain | See Table 5 |

Table 29 shows the cDNA and amino acid sequences of the monovalent CD19(8B8-2B11)-targeted 4-1BB ligand trimer-containing Fc (kih) fusion antigen binding molecule (Construct 4.13).

**Table 29: Sequences of CD19(8B8-2B11)-targeted human 4-1BB ligand trimer containing Fc (kih) fusion molecule Construct 4.13 (comparative example)**

| SEQ ID NO: | Description | Sequence |
|---|---|---|
| 187 | nucleotide sequence of anti-CD19 (8B8-2B11) Fc hole dimeric 4-1BB ligand (71-248) chain | CAGGTGCAATTGGTTCAATCTGGTGCTGAAGTAAAAAA ACCGGGCGCTTCCGTTAAAGTGAGCTGCAAAGCATCTGG TTACACCTTCACTGACTATATCATGCACTGGGTTCGTCA GGCCCCGGGCCAGGGTCTGGAGTGGATGGGCTACATTA ACCCATACAACGACGGTTCCAAATATACCGAGAAATTC CAGGGCCGCGTCACGATGACCAGCGACACTTCTATCTCC ACCGCGTACATGGAACTGTCTAGACTGCGTTCTGACGAC ACCGCTGTTTACTATTGTGCACGCGGTACCTACTACTAC GGTCCACAGCTGTTTGATTACTGGGGCCAAGGTACCACG GTGACCGTAAGCTCTGCTAGCACCAAGGGCCCCTCCGTG TTCCCCCTGGCCCCCAGCAGCAAGAGCACCAGCGGCGG CACAGCCGCTCTGGGCTGCCTGGTCAAGGACTACTTCCC CGAGCCCGTGACCGTGTCCTGGAACAGCGGAGCCCTGA CCTCCGGCGTGCACACCTTCCCCGCCGTGCTGCAGAGTT CTGGCCTGTATAGCCTGAGCAGCGTGGTCACCGTGCCTT CTAGCAGCCTGGGCACCCAGACCTACATCTGCAACGTG AACCACAAGCCCAGCAACACCAAGGTGGACAAGAAGGT GGAGCCCAAGAGCTGCGACAAAACTCACACATGCCCAC CGTGCCCAGCACCTGAAGCTGCAGGGGGACCGTCAGTC TTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC |

(continued)

| SEQ ID NO: | Description | Sequence |
|---|---|---|
| | | GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTGCACCCTGCCCCCATCCCGG GATGAGCTGACCAAGAACCAGGTCAGCCTCTCGTGCGC AGTCAAAGGCTTCTATCCCAGCGACATCGCCGTGGAGTG GGAGAGCAATGGGCAGCCGGAGAACAACTACAAGACC ACGCCTCCCGTGCTGGACTCCGACGGCTCCTTCTTCCTC GTGAGCAAGCTCACCGTGGACAAGAGCAGGTGGCAGCA GGGGAACGTCTTCTCATGCTCCGTGATGCATGAGGCTCT GCACAACCACTACACGCAGAAGAGCCTCTCCCTGTCTCC GGGTGGAGGCGGCGGAAGCGGAGGAGGAGGATCCAGA GAGGGCCCTGAGCTGAGCCCTGATGATCCTGCCGGACT GCTGGACCTGCGGCAGGGAATGTTTGCCCAGCTGGTGGC CCAGAACGTGCTGCTGATCGATGGCCCCCTGTCCTGGTA CAGCGATCCTGGACTGGCTGGCGTGTCACTGACAGGCG GCCTGAGCTACAAAGAGGACACCAAAGAACTGGTGGTG GCCAAGGCCGGCGTGTACTACGTGTTCTTTCAGCTGGAA CTGCGGAGAGTGGTGGCCGGCGAAGGATCTGGCTCTGT GTCTCTGGCCCTGCATCTGCAGCCTCTGAGATCTGCTGC TGGCGCCGCTGCTCTGGCACTGACAGTGGATCTGCCTCC TGCCAGCAGCGAGGCCCGGAATAGCGCATTTGGGTTTCA AGGCAGGCTGCTGCACCTGTCTGCCGGCCAGAGGCTGG GAGTGCATCTGCACACAGAGGCCAGGGCTAGACACGCC TGGCAGCTGACACAGGGCGCTACAGTGCTGGGCCTGTTC AGAGTGACCCCCGAGATTCCAGCAGGCCTGGGAGGCGG CGGATCTGGCGGCGGAGGATCTAGAGAAGGACCCGAGC TGTCCCCCGACGATCCCGCTGGGCTGCTGGATCTGAGAC AGGGCATGTTCGCTCAGCTGGTGGCTCAGAATGTGCTGC TGATTGACGGACCTCTGAGCTGGTACTCCGACCCAGGGC TGGCAGGGGTGTCCCTGACTGGGGGACTGTCCTACAAA GAAGATACAAAAGAACTGGTGGTGGCTAAAGCTGGGGT GTACTATGTGTTTTTCAGCTGGAACTGAGGCGGGTGGT GGCTGGGGAGGGCTCAGGATCTGTGTCCCTGGCTCTGCA TCTGCAGCCACTGCGCTCTGCAGCAGGGGCTGCAGCACT GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |
| 172 | nucleotide sequence of Fc knob monomeric 4-1BB ligand (71-248) | see Table 23 |
| 124 | nucleotide sequence of anti-CD19 (8B8-2B11) light chain | see Table 7 |

(continued)

| SEQ ID NO: | Description | Sequence |
|---|---|---|
| 188 | anti-CD19(8B8-2B11) Fc hole dimeric 4-1BB ligand (71-248) chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGPQLFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGGGGGSGGGGSREGPELSPDDPAGLLDLRQ GMFAQLVAQNVLLIDGPLSWYSDPGLAGVSLTGGLSYKED TKELVVAKAGVYYVFFQLELRRVVAGEGSGSVSLALHLQP LRSAAGAAALALTVDLPPASSEARNSAFGFQGRLLHLSAG QRLGVHLHTEARARHAWQLTQGATVLGLFRVTPEIPAGLG GGGSGGGGSREGPELSPDDPAGLLDLRQGMFAQLVAQNV LLIDGPLSWYSDPGLAGVSLTGGLSYKEDTKELVVAKAGV YYVFFQLELRRVVAGEGSGSVSLALHLQPLRSAAGAAALA LTVDLPPASSEARNSAFGFQGRLLHLSAGQRLGVHLHTEA RARHAWQLTQGATVLGLFRVTPEIPAGL |
| 174 | Fc knob monomeric 4-1BB ligand (71-248) | see Table 23 |
| 126 | anti-CD19(8B8-2B11) light chain | see Table 7 |

Table 30 shows the cDNA and amino acid sequences of the monovalent CD19(8B8-2B11)-targeted 4-1BB ligand trimer-containing Fc (kih) fusion antigen binding molecule (Construct 4.14).

**Table 30: Sequences of CD19(8B8-2B11)-targeted human 4-1BB ligand trimer containing Fc (kih) fusion molecule Construct 4.14 (comparative example)**

| SEQ ID NO: | Description | Sequence |
|---|---|---|

| | | |
|---|---|---|
| 189 | nucleotide sequence of anti-CD19(8B8-2B11) Fc hole monomeric 4-1BB ligand (71-248) chain | CAGGTGCAATTGGTTCAATCTGGTGCTGAAGTAAAAAA ACCGGGCGCTTCCGTTAAAGTGAGCTGCAAAGCATCTGG TTACACCTTCACTGACTATATCATGCACTGGGTTCGTCA GGCCCCGGGCCAGGGTCTGGAGTGGATGGGCTACATTA ACCCATACAACGACGGTTCCAAATATACCGAGAAATTC CAGGGCCGCGTCACGATGACCAGCGACACTTCTATCTCC ACCGCGTACATGGAACTGTCTAGACTGCGTTCTGACGAC ACCGCTGTTTACTATTGTGCACGCGGTACCTACTACTAC GGTCCACAGCTGTTTGATTACTGGGGCCAAGGTACCACG GTGACCGTAAGCTCTGCTAGCACCAAGGGCCCCTCCGTG TTCCCCCTGGCCCCCAGCAGCAAGAGCACCAGCGGCGG CACAGCCGCTCTGGGCTGCCTGGTCAAGGACTACTTCCC CGAGCCCGTGACCGTGTCCTGGAACAGCGGAGCCCTGA CCTCCGGCGTGCACACCTTCCCCGCCGTGCTGCAGAGTT CTGGCCTGTATAGCCTGAGCAGCGTGGTCACCGTGCCTT CTAGCAGCCTGGGCACCCAGACCTACATCTGCAACGTG AACCACAAGCCCAGCAACACCAAGGTGGACAAGAAGGT GGAGCCCAAGAGCTGCGACAAAACTCACACATGCCCAC CGTGCCCAGCACCTGAAGCTGCAGGGGGACCGTCAGTC TTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTGCACCCTGCCCCCATCCCGG GATGAGCTGACCAAGAACCAGGTCAGCCTCTCGTGCGC AGTCAAAGGCTTCTATCCCAGCGACATCGCCGTGGAGTG GGAGAGCAATGGGCAGCCGGAGAACAACTACAAGACC ACGCCTCCCGTGCTGGACTCCGACGGCTCCTTCTTCCTC GTGAGCAAGCTCACCGTGGACAAGAGCAGGTGGCAGCA GGGGAACGTCTTCTCATGCTCCGTGATGCATGAGGCTCT GCACAACCACTACACGCAGAAGAGCCTCTCCCTGTCTCC GGGTGGAGGCGGCGGAAGCGGAGGAGGAGGATCCAGA GAGGGCCCTGAGCTGAGCCCTGATGATCCTGCCGGACT GCTGGACCTGCGGCAGGGAATGTTTGCCCAGCTGGTGGC CCAGAACGTGCTGCTGATCGATGGCCCCCTGTCCTGGTA CAGCGATCCTGGACTGGCTGGCGTGTCACTGACAGGCG GCCTGAGCTACAAAGAGGACACCAAAGAACTGGTGGTG GCCAAGGCCGGCGTGTACTACGTGTTCTTTCAGCTGGAA CTGCGGAGAGTGGTGGCCGGCGAAGGATCTGGCTCTGT GTCTCTGGCCCTGCATCTGCAGCCTCTGAGATCTGCTGC TGGCGCCGCTGCTCTGGCACTGACAGTGGATCTGCCTCC TGCCAGCAGCGAGGCCCGGAATAGCGCATTTGGGTTTCA AGGCAGGCTGCTGCACCTGTCTGCCGGCCAGAGGCTGG GAGTGCATCTGCACACAGAGGCCAGGGCTAGACACGCC TGGCAGCTGACACAGGGCGCTACAGTGCTGGGCCTGTTC AGAGTGACCCCCGAGATTCCTGCCGGGCTC |
| 176 | nucleotide sequence of Fc knob dimeric 4- | see Table 24 |

| | | |
|---|---|---|
| | 1BBL (71-248) | |
| 124 | nucleotide sequence of anti-CD19(8B8-2B11) light chain | See Table 7 |
| 190 | anti- CD19(8B8-2B11) Fc hole monomeric 4-1BBL (71-248) chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGPQLFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGGGGGSGGGGSREGPELSPDDPAGLLDLRQ GMFAQLVAQNVLLIDGPLSWYSDPGLAGVSLTGGLSYKED TKELVVAKAGVYYVFFQLELRRVVAGEGSGSVSLALHLQP LRSAAGAAALALTVDLPPASSEARNSAFGFQGRLLHLSAG QRLGVHLHTEARARHAWQLTQGATVLGLFRVTPEIPAGL |
| 178 | Fc knob dimeric 4-1BB ligand (71-248) | see Table 24 |
| 126 | anti- CD19(8B8-2B11) light chain | See Table 7 |

**1.9 Preparation of monovalent CEA-targeted 4-1BB ligand trimer-containing Fc (kih) fusion antigen binding molecule, wherein the 4-1BB ligands are C-terminally fused (Constructs 5.13 and 5.14) (CEA on the hole chain)**

The molecules are prepared as described in Example 1.7 for the monovalent FAP-targeted construct, with the only difference that the anti-FAP binder was replaced by an anti-CEA binder.

The variable region of heavy and light chain DNA sequences encoding binder specific for carcinoembryonic antigen (CEA), clone T84.66-LCHA, are subcloned in frame with either the constant heavy chain of the knob or the constant light chain of human IgG1. The generation of the CEA clone is described in Example 1.5.

Combination of the huIgG1 Fc hole chain containing the Y349C/T366S/L368A/Y407V mutations in the CH3 domain, the anti-CEA huIgG1 knob chain containing the S354C/T366W mutations in the CH3 domain and the anti-CEA light chain allows generation of a heterodimer, which includes an assembled trimeric 4-1BB ligand and one CEA binding Fab **(Figures 2C and 2D)**.

Table 31 shows the cDNA and amino acid sequences of the monovalent CEA(T84.66-LCHA)-targeted 4-1BB ligand (71-248) trimer-containing Fc (kih) fusion antigen binding molecule (Construct 5.13).

**Table 17: Sequences of CEA(T84.66-LCHA)-targeted human 4-1BB ligand trimer containing Fc (kih) fusion molecule Construct 5.13 (comparative example)**

| SEQ ID NO: | Description | Sequence |
|---|---|---|
| 191 | nucleotide sequence of anti-CEA(T84.66-LCHA) Fc hole dimeric 4-1BB ligand (71-248) chain | CAGGTGCAGCTGGTGCAGTCTGGCGCCGAAGTGAAGAA ACCCGGCAGCAGCGTGAAGGTGTCCTGCAAGGCCAGCG GCTTCAACATCAAGGACACCTACATGCACTGGGTGCGCC AGGCCCCTGGACAGGGACTGGAATGGATGGGCAGAATC GACCCCGCCAACGGCAACAGCAAATACGTGCCCAAGTT CCAGGGCAGAGTGACCATCACCGCCGACACCAGCACCT CCACCGCCTACATGGAACTGAGCAGCCTGCGGAGCGAG GACACCGCCGTGTACTACTGTGCCCCCTTCGGCTACTAC GTGTCCGACTACGCCATGGCCTATTGGGGCCAGGGCAC ACTCGTGACCGTGTCCTCTGCTAGCACCAAGGGCCCCTC CGTGTTCCCCCTGGCCCCCAGCAGCAAGAGCACCAGCG GCGGCACAGCCGCTCTGGGCTGCCTGGTCAAGGACTACT TCCCCGAGCCCGTGACCGTGTCCTGGAACAGCGGAGCC CTGACCTCCGGCGTGCACACCTTCCCCGCCGTGCTGCAG AGTTCTGGCCTGTATAGCCTGAGCAGCGTGGTCACCGTG CCTTCTAGCAGCCTGGGCACCCAGACCTACATCTGCAAC GTGAACCACAAGCCCAGCAACACCAAGGTGGACAAGAA GGTGGAGCCCAAGAGCTGCGACAAAACTCACACATGCC CACCGTGCCCAGCACCTGAAGCTGCAGGGGGACCGTCA GTCTTCCTCTTCCCCCCAAAACCCAAGGACACCCTCATG ATCTCCCGGACCCCTGAGGTCACATGCGTGGTGGTGGAC GTGAGCCACGAAGACCCTGAGGTCAAGTTCAACTGGTA CGTGGACGGCGTGGAGGTGCATAATGCCAAGACAAAGC CGCGGGAGGAGCAGTACAACAGCACGTACCGTGTGGTC AGCGTCCTCACCGTCCTGCACCAGGACTGGCTGAATGGC AAGGAGTACAAGTGCAAGGTCTCCAACAAAGCCCTCGG CGCCCCCATCGAGAAAACCATCTCCAAAGCCAAAGGGC AGCCCCGAGAACCACAGGTGTGCACCCTGCCCCCATCCC GGGATGAGCTGACCAAGAACCAGGTCAGCCTCTCGTGC GCAGTCAAAGGCTTCTATCCCAGCGACATCGCCGTGGA GTGGGAGAGCAATGGGCAGCCGGAGAACAACTACAAGA CCACGCCTCCCGTGCTGGACTCCGACGGCTCCTTCTTCC TCGTGAGCAAGCTCACCGTGGACAAGAGCAGGTGGCAG CAGGGGAACGTCTTCTCATGCTCCGTGATGCATGAGGCT |

(continued)

| SEQ ID NO: | Description | Sequence |
|---|---|---|
| | | CTGCACAACCACTACACGCAGAAGAGCCTCTCCCTGTCT CCGGGTGGAGGCGGCGGAAGCGGAGGAGGAGGATCCA GAGAGGGCCCTGAGCTGAGCCCTGATGATCCTGCCGGA CTGCTGGACCTGCGGCAGGGAATGTTTGCCCAGCTGGTG GCCCAGAACGTGCTGCTGATCGATGGCCCCCTGTCCTGG TACAGCGATCCTGGACTGGCTGGCGTGTCACTGACAGGC GGCCTGAGCTACAAAGAGGACACCAAAGAACTGGTGGT GGCCAAGGCCGGCGTGTACTACGTGTTCTTTCAGCTGGA ACTGCGGAGAGTGGTGGCCGGCGAAGGATCTGGCTCTG TGTCTCTGGCCCTGCATCTGCAGCCTCTGAGATCTGCTG CTGGCGCCGCTGCTCTGGCACTGACAGTGGATCTGCCTC CTGCCAGCAGCGAGGCCCGGAATAGCGCATTTGGGTTTC AAGGCAGGCTGCTGCACCTGTCTGCCGGCCAGAGGCTG GGAGTGCATCTGCACACAGAGGCCAGGGCTAGACACGC CTGGCAGCTGACACAGGGCGCTACAGTGCTGGGCCTGTT CAGAGTGACCCCCGAGATTCCAGCAGGCCTGGGAGGCG GCGGATCTGGCGGCGGAGGATCTAGAGAAGGACCCGAG CTGTCCCCCGACGATCCCGCTGGGCTGCTGGATCTGAGA CAGGGCATGTTCGCTCAGCTGGTGGCTCAGAATGTGCTG CTGATTGACGGACCTCTGAGCTGGTACTCCGACCCAGGG CTGGCAGGGGTGTCCCTGACTGGGGGACTGTCCTACAAA GAAGATACAAAAGAACTGGTGGTGGCTAAAGCTGGGGT GTACTATGTGTTTTTTCAGCTGGAACTGAGGCGGGTGGT GGCTGGGGAGGGCTCAGGATCTGTGTCCCTGGCTCTGCA TCTGCAGCCACTGCGCTCTGCAGCAGGGGCTGCAGCACT GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |
| 172 | nucleotide sequence of Fc knob monomeric 4-1BBL (71-248) | see Table 23 |
| 154 | nucleotide sequence of anti-CEA(T84.66-LCHA) light chain | see Table 17 |
| 192 | anti-CEA(T84.66-LCHA) Fc hole dimeric 4-1BB (71-248) ligand | QVQLVQSGAEVKKPGSSVKVSCKASGFNIKDTYMHWVRQ APGQGLEWMGRIDPANGNSKYVPKFQGRVTITADTSTSTA YMELSSLRSEDTAVYYCAPFGYYVSDYAMAYWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALGAPIEKTISKAKGQPREPQVCTLPPSR |

(continued)

| SEQ ID NO: | Description | Sequence |
|---|---|---|
| | chain | DELTKNQVSLSCAVKGFYPSDIAVEWESNGQPENNYKTTP PVLDSDGSFFLVSKLTVDKSRWQQGNVFSCSVMHEALHN HYTQKSLSLSPGGGGGSGGGGSREGPELSPDDPAGLLDLR QGMFAQLVAQNVLLIDGPLSWYSDPGLAGVSLTGGLSYKE DTKELVVAKAGVYYVFFQLELRRVVAGEGSGSVSLALHL QPLRSAAGAAALALTVDLPPASSEARNSAFGFQGRLLHLS AGQRLGVHLHTEARARHAWQLTQGATVLGLFRVTPEIPA GLGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGL |
| 174 | Fc knob monomeric 4-1-BB (71-248) ligand | see Table 23 |
| 156 | anti-CEA(T84.66-LCHA) light chain | see Table 17 |

Table 32 shows the cDNA and amino acid sequences of the monovalent CEA(T84.66-LCHA)-targeted 4-1BB ligand (71-248) trimer-containing Fc (kih) fusion antigen binding molecule (Construct 5.14).

**Table 32: Sequences of CEA(T84.66-LCHA)-targeted human 4-1BB ligand trimer containing Fc (kih) fusion molecule Construct 5.14 (comparative example)**

| SEQ ID NO: | Description | Sequence |
|---|---|---|
| 193 | nucleotide sequence of anti-CEA(T84.66-LCHA) Fc hole monomeric 4-1BBL (71-248) chain | CAGGTGCAGCTGGTGCAGTCTGGCGCCGAAGTGAAGAA ACCCGGCAGCAGCGTGAAGGTGTCCTGCAAGGCCAGCG GCTTCAACATCAAGGACACCTACATGCACTGGGTGCGCC AGGCCCCTGGACAGGGACTGGAATGGATGGGCAGAATC GACCCCGCCAACGGCAACAGCAAATACGTGCCCAAGTT CCAGGGCAGAGTGACCATCACCGCCGACACCAGCACCT CCACCGCCTACATGGAACTGAGCAGCCTGCGGAGCGAG GACACCGCCGTGTACTACTGTGCCCCCTTCGGCTACTAC GTGTCCGACTACGCCATGGCCTATTGGGGCCAGGGCAC ACTCGTGACCGTGTCCTCTGCTAGCACCAAGGGCCCCTC CGTGTTCCCCCTGGCCCCCAGCAGCAAGAGCACCAGCG GCGGCACAGCCGCTCTGGGCTGCCTGGTCAAGGACTACT TCCCCGAGCCCGTGACCGTGTCCTGGAACAGCGGAGCC CTGACCTCCGGCGTGCACACCTTCCCCGCCGTGCTGCAG |

(continued)

| SEQ ID NO: | Description | Sequence |
|---|---|---|
| | | AGTTCTGGCCTGTATAGCCTGAGCAGCGTGGTCACCGTG CCTTCTAGCAGCCTGGGCACCCAGACCTACATCTGCAAC GTGAACCACAAGCCCAGCAACACCAAGGTGGACAAGAA GGTGGAGCCCAAGAGCTGCGACAAAACTCACACATGCC CACCGTGCCCAGCACCTGAAGCTGCAGGGGGACCGTCA GTCTTCCTCTTCCCCCCAAAACCCAAGGACACCCTCATG ATCTCCCGGACCCCTGAGGTCACATGCGTGGTGGTGGAC GTGAGCCACGAAGACCCTGAGGTCAAGTTCAACTGGTA CGTGGACGGCGTGGAGGTGCATAATGCCAAGACAAAGC CGCGGGAGGAGCAGTACAACAGCACGTACCGTGTGGTC AGCGTCCTCACCGTCCTGCACCAGGACTGGCTGAATGGC AAGGAGTACAAGTGCAAGGTCTCCAACAAAGCCCTCGG CGCCCCCATCGAGAAAACCATCTCCAAAGCCAAAGGGC AGCCCCGAGAACCACAGGTGTGCACCCTGCCCCCATCCC GGGATGAGCTGACCAAGAACCAGGTCAGCCTCTCGTGC GCAGTCAAAGGCTTCTATCCCAGCGACATCGCCGTGGA GTGGGAGAGCAATGGGCAGCCGGAGAACAACTACAAGA CCACGCCTCCCGTGCTGGACTCCGACGGCTCCTTCTTCC TCGTGAGCAAGCTCACCGTGGACAAGAGCAGGTGGCAG CAGGGGAACGTCTTCTCATGCTCCGTGATGCATGAGGCT CTGCACAACCACTACACGCAGAAGAGCCTCTCCCTGTCT CCGGGTGGAGGCGGCGGAAGCGGAGGAGGAGGATCCA GAGAGGGCCCTGAGCTGAGCCCTGATGATCCTGCCGGA CTGCTGGACCTGCGGCAGGGAATGTTTGCCCAGCTGGTG GCCCAGAACGTGCTGCTGATCGATGGCCCCCTGTCCTGG TACAGCGATCCTGGACTGGCTGGCGTGTCACTGACAGGC GGCCTGAGCTACAAAGAGGACACCAAAGAACTGGTGGT GGCCAAGGCCGGCGTGTACTACGTGTTCTTTCAGCTGGA ACTGCGGAGAGTGGTGGCCGGCGAAGGATCTGGCTCTG TGTCTCTGGCCCTGCATCTGCAGCCTCTGAGATCTGCTG CTGGCGCCGCTGCTCTGGCACTGACAGTGGATCTGCCTC CTGCCAGCAGCGAGGCCCGGAATAGCGCATTTGGGTTTC AAGGCAGGCTGCTGCACCTGTCTGCCGGCCAGAGGCTG GGAGTGCATCTGCACACAGAGGCCAGGGCTAGACACGC CTGGCAGCTGACACAGGGCGCTACAGTGCTGGGCCTGTT CAGAGTGACCCCCGAGATTCCTGCCGGGCTC |
| 176 | nucleotide sequence of Fc knob dimeric 4-1BB (71-248) ligand | see Table 24 |
| 154 | nucleotide sequence of anti-CEA(T84.66-LCHA) light chain | See Table 17 |
| 194 | anti-CEA(T84.66-LCHA) Fc hole monomeric 4-1BB ligand (71-248) chain | QVQLVQSGAEVKKPGSSVKVSCKASGFNIKDTYMHWVRQ APGQGLEWMGRIDPANGNSKYVPKFQGRVTITADTSTSTA |

(continued)

| SEQ ID NO: | Description | Sequence |
|---|---|---|
| | | YMELSSLRSEDTAVYYCAPFGYYVSDYAMAYWGQGTLV TVSSASTKGPSVFPLAPSSKSTSGGTAALGCLVKDYFPEPV TVSWNSGALTSGVHTFPAVLQSSGLYSLSSVVTVPSSSLGT QTYICNVNHKPSNTKVDKKVEPKSCDKTHTCPPCPAPEAA GGPSVFLFPPKPKDTLMISRTPEVTCVVVDVSHEDPEVKFN WYVDGVEVHNAKTKPREEQYNSTYRVVSVLTVLHQDWL NGKEYKCKVSNKALGAPIEKTISKAKGQPREPQVCTLPPSR DELTKNQVSLSCAVKGFYPSDIAVEWESNGQPENNYKTTP PVLDSDGSFFLVSKLTVDKSRWQQGNVFSCSVMHEALHN HYTQKSLSLSPGGGGGSGGGGSREGPELSPDDPAGLLDLR QGMFAQLVAQNVLLIDGPLSWYSDPGLAGVSLTGGLSYKE DTKELVVAKAGVYYVFFQLELRRVVAGEGSGSVSLALHL QPLRSAAGAAALALTVDLPPASSEARNSAFGFQGRLLHLS AGQRLGVHLHTEARARHAWQLTQGATVLGLFRVTPEIPA GL |
| 178 | Fc knob dimeric 4-1BB (71-248) ligand | see Table 24 |
| 156 | anti-CEA(T84.66-LCHA) light chain | See Table 17 |

**1.10 Preparation of monovalent untargeted 4-1BB ligand trimer-containing Fc (kih) fusion antigen binding molecules, wherein the 4-1BB ligands are C-terminally fused (Controls J and K) (DP47 on the hole chain)**

The molecule was prepared as described in Example 1.7 for the monovalent FAP targeted construct, with the only difference that the anti-FAP binder (VH-VL) was replaced by a germline control, termed DP47, not binding to the antigen.

Table 33 shows the cDNA and amino acid sequences of the monovalent untargeted 4-1BB ligand (71-248) trimer-containing Fc (kih) fusion antigen binding molecule (Control J).

**Table 33: Sequences of monovalent DP47-untargeted human 4-1BB ligand trimer containing Fc (kih) fusion molecule Control J**

| SEQ ID NO: | Description | Sequence |
|---|---|---|
| 195 | nucleotide sequence of DP47 Fc hole dimeric 4-1BB ligand (71-248) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA |

(continued)

| SEQ ID NO: | Description | Sequence |
|---|---|---|
| | | GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCCTCCGTGTTCCCCCTGGCCCCCAG CAGCAAGAGCACCAGCGGCGGCACAGCCGCTCTGGGCT GCCTGGTCAAGGACTACTTCCCCGAGCCCGTGACCGTGT CCTGGAACAGCGGAGCCCTGACCTCCGGCGTGCACACC TTCCCCGCCGTGCTGCAGAGTTCTGGCCTGTATAGCCTG AGCAGCGTGGTCACCGTGCCTTCTAGCAGCCTGGGCACC CAGACCTACATCTGCAACGTGAACCACAAGCCCAGCAA CACCAAGGTGGACAAGAAGGTGGAGCCCAAGAGCTGCG ACAAAACTCACACATGCCCACCGTGCCCAGCACCTGAA GCTGCAGGGGGACCGTCAGTCTTCCTCTTCCCCCCAAAA CCCAAGGACACCCTCATGATCTCCCGGACCCCTGAGGTC ACATGCGTGGTGGTGGACGTGAGCCACGAAGACCCTGA GGTCAAGTTCAACTGGTACGTGGACGGCGTGGAGGTGC ATAATGCCAAGACAAAGCCGCGGGAGGAGCAGTACAAC AGCACGTACCGTGTGGTCAGCGTCCTCACCGTCCTGCAC CAGGACTGGCTGAATGGCAAGGAGTACAAGTGCAAGGT CTCCAACAAAGCCCTCGGCGCCCCCATCGAGAAAACCA TCTCCAAAGCCAAAGGGCAGCCCCGAGAACCACAGGTG TGCACCCTGCCCCCATCCCGGGATGAGCTGACCAAGAA CCAGGTCAGCCTCTCGTGCGCAGTCAAAGGCTTCTATCC CAGCGACATCGCCGTGGAGTGGGAGAGCAATGGGCAGC CGGAGAACAACTACAAGACCACGCCTCCCGTGCTGGAC TCCGACGGCTCCTTCTTCCTCGTGAGCAAGCTCACCGTG GACAAGAGCAGGTGGCAGCAGGGGAACGTCTTCTCATG CTCCGTGATGCATGAGGCTCTGCACAACCACTACACGCA GAAGAGCCTCTCCCTGTCTCCGGGTGGAGGCGGCGGAA GCGGAGGAGGAGGATCCAGAGAGGGCCCTGAGCTGAGC CCTGATGATCCTGCCGGACTGCTGGACCTGCGGCAGGG AATGTTTGCCCAGCTGGTGGCCCAGAACGTGCTGCTGAT CGATGGCCCCCTGTCCTGGTACAGCGATCCTGGACTGGC TGGCGTGTCACTGACAGGCGGCCTGAGCTACAAAGAGG ACACCAAAGAACTGGTGGTGGCCAAGGCCGGCGTGTAC TACGTGTTCTTTCAGCTGGAACTGCGGAGAGTGGTGGCC GGCGAAGGATCTGGCTCTGTGTCTCTGGCCCTGCATCTG CAGCCTCTGAGATCTGCTGCTGGCGCCGCTGCTCTGGCA CTGACAGTGGATCTGCCTCCTGCCAGCAGCGAGGCCCG GAATAGCGCATTTGGGTTTCAAGGCAGGCTGCTGCACCT GTCTGCCGGCCAGAGGCTGGGAGTGCATCTGCACACAG AGGCCAGGGCTAGACACGCCTGGCAGCTGACACAGGGC GCTACAGTGCTGGGCCTGTTCAGAGTGACCCCCGAGATT CCAGCAGGCCTGGGAGGCGGCGGATCTGGCGGCGGAGG ATCTAGAGAAGGACCCGAGCTGTCCCCCGACGATCCCG CTGGGCTGCTGGATCTGAGACAGGGCATGTTCGCTCAGC TGGTGGCTCAGAATGTGCTGCTGATTGACGGACCTCTGA GCTGGTACTCCGACCCAGGGCTGGCAGGGGTGTCCCTG ACTGGGGGACTGTCCTACAAAGAAGATACAAAAGAACT GGTGGTGGCTAAAGCTGGGGTGTACTATGTGTTTTTCA GCTGGAACTGAGGCGGGTGGTGGCTGGGGAGGGCTCAG GATCTGTGTCCCTGGCTCTGCATCTGCAGCCACTGCGCT |

(continued)

| SEQ ID NO: | Description | Sequence |
|---|---|---|
| | | CTGCAGCAGGGGCTGCAGCACTGGCCCTGACTGTGGAC CTGCCCCCAGCTTCTTCCGAGGCCAGAAACAGCGCCTTC GGGTTCCAAGGACGCCTGCTGCATCTGAGCGCCGGACA GCGCCTGGGAGTGCATCTGCATACTGAAGCCAGAGCCC GGCATGCTTGGCAGCTGACTCAGGGGGCAACTGTGCTG GGACTGTTTCGCGTGACACCTGAGATCCCAGCCGGGCTC |
| 172 | nucleotide sequence of Fc knob monomeric 4-1BBL (71-248) | see Table 23 |
| 166 | nucleotide sequence of DP47 light chain | see Table 21 |
| 196 | DP47 Fc hole dimeric 4-1BB ligand (71-248) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVCTLPPSRDELTKNQVS LSCAVKGFYPSDIAVEWESNGQPENNYKTTPPVLDSDGSFF LVSKLTVDKSRWQQGNVFSCSVMHEALHNHYTQKSLSLS PGGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGLGGGGSGGG GSREGPELSPDDPAGLLDLRQGMFAQLVAQNVLLIDGPLS WYSDPGLAGVSLTGGLSYKEDTKELVVAKAGVYYVFFQL ELRRVVAGEGSGSVSLALHLQPLRSAAGAAALALTVDLPP ASSEARNSAFGFQGRLLHLSAGQRLGVHLHTEARARHAW QLTQGATVLGLFRVTPEIPAGL |
| 174 | Fc knob monomeric 4-1-BB (71-248) ligand | see Table 23 |
| 168 | DP47 light chain | see Table 21 |

Table 34 shows the cDNA and amino acid sequences of the monovalent untargeted 4-1BB ligand (71-248) trimer-containing Fc (kih) fusion antigen binding molecule (Control K).

**Table 34: Sequences of monovalent DP47-untargeted human 4-1BB ligand trimer containing Fc (kih) fusion molecule Control K**

| SEQ ID NO: | Description | Sequence |
|---|---|---|
| 197 | nucleotide sequence of DP47 Fc hole monomeric 4-1BBL (71-248) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCCTCCGTGTTCCCCCTGGCCCCCAG CAGCAAGAGCACCAGCGGCGGCACAGCCGCTCTGGGCT GCCTGGTCAAGGACTACTTCCCCGAGCCCGTGACCGTGT CCTGGAACAGCGGAGCCCTGACCTCCGGCGTGCACACC TTCCCCGCCGTGCTGCAGAGTTCTGGCCTGTATAGCCTG AGCAGCGTGGTCACCGTGCCTTCTAGCAGCCTGGGCACC CAGACCTACATCTGCAACGTGAACCACAAGCCCAGCAA CACCAAGGTGGACAAGAAGGTGGAGCCCAAGAGCTGCG ACAAAACTCACACATGCCCACCGTGCCCAGCACCTGAA GCTGCAGGGGGACCGTCAGTCTTCCTCTTCCCCCCAAAA CCCAAGGACACCCTCATGATCTCCCGGACCCCTGAGGTC ACATGCGTGGTGGTGGACGTGAGCCACGAAGACCCTGA GGTCAAGTTCAACTGGTACGTGGACGGCGTGGAGGTGC ATAATGCCAAGACAAAGCCGCGGGAGGAGCAGTACAAC AGCACGTACCGTGTGGTCAGCGTCCTCACCGTCCTGCAC CAGGACTGGCTGAATGGCAAGGAGTACAAGTGCAAGGT CTCCAACAAAGCCCTCGGCGCCCCCATCGAGAAAACCA TCTCCAAAGCCAAAGGGCAGCCCCGAGAACCACAGGTG TGCACCCTGCCCCCATCCCGGGATGAGCTGACCAAGAA CCAGGTCAGCCTCTCGTGCGCAGTCAAAGGCTTCTATCC CAGCGACATCGCCGTGGAGTGGGAGAGCAATGGGCAGC CGGAGAACAACTACAAGACCACGCCTCCCGTGCTGGAC TCCGACGGCTCCTTCTTCCTCGTGAGCAAGCTCACCGTG GACAAGAGCAGGTGGCAGCAGGGGAACGTCTTCTCATG CTCCGTGATGCATGAGGCTCTGCACAACCACTACACGCA GAAGAGCCTCTCCCTGTCTCCGGGTGGAGGCGGCGGAA GCGGAGGAGGAGGATCCAGAGAGGGCCCTGAGCTGAGC CCTGATGATCCTGCCGGACTGCTGGACCTGCGGCAGGG AATGTTTGCCCAGCTGGTGGCCCAGAACGTGCTGCTGAT CGATGGCCCCCTGTCCTGGTACAGCGATCCTGGACTGGC TGGCGTGTCACTGACAGGCGGCCTGAGCTACAAAGAGG ACACCAAAGAACTGGTGGTGGCCAAGGCCGGCGTGTAC TACGTGTTCTTTCAGCTGGAACTGCGGAGAGTGGTGGCC GGCGAAGGATCTGGCTCTGTGTCTCTGGCCCTGCATCTG CAGCCTCTGAGATCTGCTGCTGGCGCCGCTGCTCTGGCA CTGACAGTGGATCTGCCTCCTGCCAGCAGCGAGGCCCG GAATAGCGCATTTGGGTTTCAAGGCAGGCTGCTGCACCT GTCTGCCGGCCAGAGGCTGGGAGTGCATCTGCACACAG |

(continued)

| SEQ ID NO: | Description | Sequence |
|---|---|---|
| | | AGGCCAGGGCTAGACACGCCTGGCAGCTGACACAGGGC GCTACAGTGCTGGGCCTGTTCAGAGTGACCCCCGAGATT CCTGCCGGGCTC |
| 176 | nucleotide sequence of Fc knob dimeric 4-1BBL (71-248) | see Table 24 |
| 166 | nucleotide sequence of DP47 light chain | See Table 21 |
| 198 | DP47 Fc hole monomeric 4-1BBL (71-248) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVCTLPPSRDELTKNQVS LSCAVKGFYPSDIAVEWESNGQPENNYKTTPPVLDSDGSFF LVSKLTVDKSRWQQGNVFSCSVMHEALHNHYTQKSLSLS PGGGGGSGGGGSREGPELSPDDPAGLLDLRQGMFAQLVA QNVLLIDGPLSWYSDPGLAGVSLTGGLSYKEDTKELVVAK AGVYYVFFQLELRRVVAGEGSGSVSLALHLQPLRSAAGAA ALALTVDLPPASSEARNSAFGFQGRLLHLSAGQRLGVHLH TEARARHAWQLTQGATVLGLFRVTPEIPAGL |
| 178 | Fc knob dimeric 4-1BBL (71-248) | see Table 24 |
| 168 | DP47 light chain | See Table 21 |

**1.11 Preparation of additional Control molecules**

Control D, a molecule with N-terminal fused 4-1BBL moieties as depicted In Figure 3B was prepared in combination with the germline control, termed DP47, not binding to the antigen.

The molecule comprises the Pro329Gly, Leu234Ala and Leu235Ala mutations in the constant region of the knob and hole heavy chains to abrogate binding to Fc gamma receptors according to the method described in International Patent Appl. Publ. No. WO 2012/130831 A1.

The polypeptide encoding the dimeric 4-1BB ligand fused to human CL domain was subcloned in frame with the human IgG1 heavy chain CH2 and CH3 domains on the knob and the polypeptide encoding the monomeric 4-1BB ligand was fused to human CH1 domain. To improve correct pairing the following mutations have been introduced in the crossed CH-CL. In the dimeric 4-1BB ligand fused to human CL, E123R and Q124K. In the monomeric 4-1BB ligand fused to human CHI, K147E and K213E.

Table 35 shows the cDNA and amino acid sequences of the monovalent "untargeted" 4-1BB ligand (71-248) trimer-containing Fc (kih) fusion antigen binding molecule (Control D).

**Table 35: Sequences of monovalent DP47-untargeted human 4-1BB ligand trimer containing Fc (kih) fusion molecule with CH-CL cross and with charged residues* (Control D)**

| SEQ ID NO: | Description | Sequence |
|---|---|---|
| 199 | nucleotide sequence of dimeric 4-1BB ligand (71-248)-CL* Fc knob chain | AGAGAGGGCCCTGAGCTGAGCCCCGATGATCCTGCTGG<br>ACTGCTGGACCTGCGGCAGGGCATGTTTGCTCAGCTGGT<br>GGCCCAGAACGTGCTGCTGATCGATGGCCCCCTGTCCTG<br>GTACAGCGATCCTGGACTGGCTGGCGTGTCACTGACAGG<br>CGGCCTGAGCTACAAAGAGGACACCAAAGAACTGGTGG<br>TGGCCAAGGCCGGCGTGTACTACGTGTTCTTTCAGCTGG<br>AACTGCGGAGAGTGGTGGCCGGCGAAGGATCTGGCTCT<br>GTGTCTCTGGCCCTGCATCTGCAGCCTCTGAGATCTGCT<br>GCTGGCGCCGCTGCTCTGGCACTGACAGTGGATCTGCCT<br>CCTGCCAGCAGCGAGGCCCGGAATAGCGCATTTGGGTTT<br>CAAGGCAGGCTGCTGCACCTGTCTGCCGGCCAGAGGCT<br>GGGAGTGCATCTGCACACAGAGGCCAGGGCTAGACACG<br>CCTGGCAGCTGACACAGGGCGCTACAGTGCTGGGCCTG<br>TTCAGAGTGACCCCCGAGATTCCAGCCGGACTGGGAGG<br>CGGCGGATCTGGCGGCGGAGGATCTAGAGAAGGACCCG<br>AGCTGTCCCCTGACGATCCAGCCGGGCTGCTGGATCTGA<br>GACAGGGAATGTTCGCCCAGCTGGTGGCTCAGAATGTG<br>CTGCTGATTGACGGACCTCTGAGCTGGTACTCCGACCCA<br>GGGCTGGCAGGGGTGTCCCTGACTGGGGGACTGTCCTAC<br>AAAGAAGATACAAAAGAACTGGTGGTGGCTAAAGCTGG<br>GGTGTACTATGTGTTTTTCAGCTGGAACTGAGGCGGGT<br>GGTGGCTGGGGAGGGCTCAGGATCTGTGTCCCTGGCTCT<br>GCATCTGCAGCCACTGCGCTCTGCAGCAGGGGCTGCAG<br>CACTGGCCCTGACTGTGGACCTGCCCCCAGCTTCTTCCG<br>AGGCCAGAAACAGCGCCTTCGGGTTCCAAGGACGCCTG<br>CTGCATCTGAGCGCCGGACAGCGCCTGGGAGTGCATCT<br>GCATACTGAAGCCAGAGCCCGGCATGCTTGGCAGCTGA<br>CTCAGGGGGCAACTGTGCTGGGACTGTTTCGCGTGACAC<br>CTGAGATCCCCGCTGGACTGGGCGGAGGCGGTTCCGGA<br>GGGGGAGGATCTCGTACGGTGGCTGCACCATCTGTCTTT<br>ATCTTCCCACCCAGCGACCGGAAGCTGAAGTCTGGCAC<br>AGCCAGCGTCGTGTGCCTGCTGAATAACTTCTACCCCCG<br>CGAGGCCAAGGTGCAGTGGAAGGTGGACAATGCCCTGC<br>AGAGCGGCAACAGCCAGGAAAGCGTGACCGAGCAGGA<br>CAGCAAGGACTCCACCTACAGCCTGAGCAGCACCCTGA<br>CCCTGAGCAAGGCCGACTACGAGAAGCACAAGGTGTAC<br>GCCTGCGAAGTGACCCACCAGGGCCTGTCTAGCCCCGTG |

(continued)

| SEQ ID NO: | Description | Sequence |
|---|---|---|
| | | ACCAAGAGCTTCAACCGGGGCGAGTGCGACAAGACCCA CACCTGTCCTCCATGCCCTGCCCCTGAAGCTGCTGGCGG CCCTAGCGTGTTCCTGTTCCCCCCAAAGCCCAAGGACAC CCTGATGATCAGCCGGACCCCTGAAGTGACCTGCGTGGT GGTGGATGTGTCCCACGAGGACCCTGAAGTGAAGTTCA ATTGGTACGTGGACGGCGTGGAAGTGCACAATGCCAAG ACCAAGCCGCGGGAGGAGCAGTACAACAGCACGTACCG TGTGGTCAGCGTCCTCACCGTCCTGCACCAGGACTGGCT GAATGGCAAGGAGTACAAGTGCAAGGTCTCCAACAAAG CCCTCGGCGCCCCCATCGAGAAAACCATCTCCAAAGCC AAAGGGCAGCCCCGAGAACCACAGGTGTACACCCTGCC CCCATGCCGGGATGAGCTGACCAAGAACCAGGTCAGCC TGTGGTGCCTGGTCAAAGGCTTCTATCCCAGCGACATCG CCGTGGAGTGGGAGAGCAATGGGCAGCCGGAGAACAAC TACAAGACCACGCCTCCCGTGCTGGACTCCGACGGCTCC TTCTTCCTCTACAGCAAGCTCACCGTGGACAAGAGCAGG TGGCAGCAGGGGAACGTCTTCTCATGCTCCGTGATGCAT GAGGCTCTGCACAACCACTACACGCAGAAGAGCCTCTC CCTGTCTCCGGGTAAA |
| 200 | nucleotide sequence of monomeric 4-1BBL (71-248)-CH1* | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGATCTGCT GCTGGCGCCGCTGCTCTGGCACTGACAGTGGATCTGCCT CCTGCCAGCAGCGAGGCCCGGAATAGCGCATTTGGGTTT CAAGGCAGGCTGCTGCACCTGTCTGCCGGCCAGAGGCT GGGAGTGCATCTGCACACAGAGGCCAGGGCTAGACACG CCTGGCAGCTGACACAGGGCGCTACAGTGCTGGGCCTG TTCAGAGTGACCCCCGAGATTCCAGCCGGACTGGGAGG CGGAGGTTCCGGAGGCGGAGGATCTGCTAGCACAAAGG GCCCCAGCGTGTTCCCTCTGGCCCCTAGCAGCAAGAGCA CATCTGGCGGAACAGCCGCCCTGGGCTGCCTGGTGGAA GATTACTTCCCCGAGCCCGTGACCGTGTCCTGGAATTCT GGCGCCCTGACAAGCGGCGTGCACACCTTTCCAGCCGTG CTGCAGAGCAGCGGCCTGTACTCTCTGAGCAGCGTCGTG ACAGTGCCCAGCAGCTCTCTGGGCACCCAGACCTACATC TGCAACGTGAACCACAAGCCCAGCAACACCAAGGTGGA CGAGAAGGTGGAACCCAAGTCCTGC |

(continued)

| SEQ ID NO: | Description | Sequence |
|---|---|---|
| 201 | nucleotide sequence of DP47 Fc hole chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCATCGGTCTTCCCCCTGGCACCCTC CTCCAAGAGCACCTCTGGGGGCACAGCGGCCCTGGGCT GCCTGGTCAAGGACTACTTCCCCGAACCGGTGACGGTGT CGTGGAACTCAGGCGCCCTGACCAGCGGCGTGCACACC TTCCCGGCTGTCCTACAGTCCTCAGGACTCTACTCCCTC AGCAGCGTGGTGACCGTGCCCTCCAGCAGCTTGGGCAC CCAGACCTACATCTGCAACGTGAATCACAAGCCCAGCA ACACCAAGGTGGACAAGAAAGTTGAGCCCAAATCTTGT GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTGCACCCTGCCCCCATCCCGGGATGAGCTGACCAAGA ACCAGGTCAGCCTCTCGTGCGCAGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCGTGAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAAA |
| 166 | nucleotide sequence of DP47 light chain | see Table 21 |

(continued)

| SEQ ID NO: | Description | Sequence |
|---|---|---|
| 202 | dimeric 4-1BB ligand (71-248)-CL* Fc knob chain | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLGGGGSGGGGSREGPELSPDDPA GLLDLRQGMFAQLVAQNVLLIDGPLSWYSDPGLAGVSLTG GLSYKEDTKELVVAKAGVYYVFFQLELRRVVAGEGSGSV SLALHLQPLRSAAGAAALALTVDLPPASSEARNSAFGFQG RLLHLSAGQRLGVHLHTEARARHAWQLTQGATVLGLFRV TPEIPAGLGGGGSGGGGSRTVAAPSVFIFPPSDRKLKSGTAS VVCLLNNFYPREAKVQWKVDNALQSGNSQESVTEQDSKD STYSLSSTLTLSKADYEKHKVYACEVTHQGLSSPVTKSFNR GECDKTHTCPPCPAPEAAGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALGAPIEKTI SKAKGQPREPQVYTLPPCRDELTKNQVSLWCLVKGFYPSD IAVEWESNGQPENNYKTTPPVLDSDGSFFLYSKLTVDKSR WQQGNVFSCSVMHEALHNHYTQKSLSLSPGK |
| 203 | monomeric 4-1BBL (71-248)-CH1* | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLGGGGSGGGGSASTKGPSVFPLA PSSKSTSGGTAALGCLVEDYFPEPVTVSWNSGALTSGVHTF PAVLQSSGLYSLSSVVTVPSSSLGTQTYICNVNHKPSNTKV DEKVEPKSC |
| 204 | DP47 Fc hole chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVCTLPPSRDELTKNQVS LSCAVKGFYPSDIAVEWESNGQPENNYKTTPPVLDSDGSFF LVSKLTVDKSRWQQGNVFSCSVMHEALHNHYTQKSLSLS PGK |
| 168 | DP47 light chain | see Table 21 |

A FAP-targeted variant of Control D was prepared, wherein instead of DP47 the variable region of heavy and light chain DNA sequences encoding a binder specific for fibroblast activation protein (FAP), clone 4B9, were subcloned in frame with either the constant heavy chain of the hole or the constant light chain of human IgG1 . Table 36 shows the cDNA and amino acid sequences of the monovalent FAP-targeted 4-1BB ligand (71-248) trimer-containing Fc (kih) fusion antigen binding molecule (Construct 2.4).

**Table 36: Sequences of monovalent FAP(4B9)-targeted human 4-1BB ligand (71-248) containing Fc (kih) fusion molecule Construct 2.4**

| SEQ ID NO: | Description | Sequence |
|---|---|---|
| 199 | nucleotide sequence of dimeric hu 4-1BBL (71-248)-CL* Fc knob chain | see Table 35 |
| 200 | nucleotide sequence of monomeric hu 4-1BBL (71-248)-CH1* | see Table 35 |
| 235 | nucleotide sequence of anti-FAP (4B9) Fc hole chain | GAGGTGCAGCTGCTCGAAAGCGGCGGAGGACTGGTGCA<br>GCCTGGCGGCAGCCTGAGACTGTCTTGCGCCGCCAGCG<br>GCTTCACCTTCAGCAGCTACGCCATGAGCTGGGTCCGCC<br>AGGCCCCTGGCAAGGGACTGGAATGGGTGTCCGCCATC<br>ATCGGCTCTGGCGCCAGCACCTACTACGCCGACAGCGTG<br>AAGGGCCGGTTCACCATCAGCCGGGACAACAGCAAGAA<br>CACCCTGTACCTGCAGATGAACAGCCTGCGGGCCGAGG<br>ACACCGCCGTGTACTACTGCGCCAAGGGATGGTTCGGC<br>GGCTTCAACTACTGGGGACAGGGCACCCTGGTCACAGT<br>GTCCAGCGCTAGCACCAAGGGCCCCTCCGTGTTCCCCCT<br>GGCCCCCAGCAGCAAGAGCACCAGCGGCGGCACAGCCG<br>CTCTGGGCTGCCTGGTCAAGGACTACTTCCCCGAGCCCG<br>TGACCGTGTCCTGGAACAGCGGAGCCCTGACCTCCGGC<br>GTGCACACCTTCCCCGCCGTGCTGCAGAGTTCTGGCCTG<br>TATAGCCTGAGCAGCGTGGTCACCGTGCCTTCTAGCAGC<br>CTGGGCACCCAGACCTACATCTGCAACGTGAACCACAA<br>GCCCAGCAACACCAAGGTGGACAAGAAGGTGGAGCCCA<br>AGAGCTGCGACAAAACTCACACATGCCCACCGTGCCCA<br>GCACCTGAAGCTGCAGGGGGACCGTCAGTCTTCCTCTTC<br>CCCCCAAAACCCAAGGACACCCTCATGATCTCCCGGACC<br>CCTGAGGTCACATGCGTGGTGGTGGACGTGAGCCACGA<br>AGACCCTGAGGTCAAGTTCAACTGGTACGTGGACGGCG<br>TGGAGGTGCATAATGCCAAGACAAAGCCGCGGGAGGAG<br>CAGTACAACAGCACGTACCGTGTGGTCAGCGTCCTCACC<br>GTCCTGCACCAGGACTGGCTGAATGGCAAGGAGTACAA<br>GTGCAAGGTCTCCAACAAAGCCCTCGGCGCCCCCATCG<br>AGAAAACCATCTCCAAAGCCAAAGGGCAGCCCCGAGAA<br>CCACAGGTGTGCACCCTGCCCCCATCCCGGGATGAGCTG<br>ACCAAGAACCAGGTCAGCCTCTCGTGCGCAGTCAAAGG<br>CTTCTATCCCAGCGACATCGCCGTGGAGTGGGAGAGCA<br>ATGGGCAGCCGGAGAACAACTACAAGACCACGCCTCCC<br>GTGCTGGACTCCGACGGCTCCTTCTTCCTCGTGAGCAAG<br>CTCACCGTGGACAAGAGCAGGTGGCAGCAGGGGAACGT<br>CTTCTCATGCTCCGTGATGCATGAGGCTCTGCACAACCA<br>CTACACGCAGAAGAGCCTCTCCCTGTCTCCGGGTAAA |
| 103 | nucleotide sequence of anti-FAP(4B9) light chain | see Table 1 |
| 202 | Dimeric hu 4-1BBL (71-248) - CL* Fc knob chain | see Table 35 |

(continued)

| SEQ ID NO: | Description | Sequence |
|---|---|---|
| 203 | Monomeric hu 4-1BBL (71-248)-CH1* | see Table 35 |
| 236 | anti-FAP (4B9) Fc hole chain | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGK |
| 106 | anti-FAP (4B9) light chain | see Table 1 |

CD19-targeted variants were also prepared, wherein instead of DP47 the variable region of heavy and light chain DNA sequences encoding a binder specific for CD19, clones 8B8-018 and 8B8-2B11, were subcloned in frame with either the constant heavy chain of the hole or the constant light chain of human IgG1. The DNA sequence encoding part of the ectodomain (amino acids 71-254 or amino acids 71-248) of human 4-1BB ligand was synthetized according to the P41273 sequence of Uniprot database. Table 37a shows the cDNA and amino acid sequences of the monovalent CD19(8B8-018) targeted split trimeric 4-1BB ligand (71-248) Fc (kih) fusion antigen binding molecule with crossed CH-CL and charged residues (construct 3.4). Table 37b shows the cDNA and amino acid sequences of the monovalent CD19-targeted 4-1BB ligand (71-254) trimer-containing Fc (kih) fusion antigen binding molecule (Construct 4.1).

**Table 37a: cDNA and amino acid sequences of monovalent CD19(8B8-018) targeted split trimeric 4-1BB ligand (71-248) Fc (kih) fusion containing CH-CL cross with charged residues (construct 3.4). * charged residues**

| SEQ ID NO: | Description | Sequence |
|---|---|---|
| 199 | nucleotide sequence of dimeric hu 4-1BBL (71-248) - CL* Fc knob chain | see Table 35 |
| 200 | nucleotide sequence of monomeric hu 4-1BBL (71-248)-CH1* | see Table 35 |

(continued)

| SEQ ID NO: | Description | Sequence |
|---|---|---|
| 237 | Nucleotide sequence anti-CD19 (8B8-018) Fc hole chain | CAGGTCCAGCTGGTGCAGTCCGGCGCCGAGGTCAAGAA ACCCGGGGCTTCTGTGAAGGTTTCATGCAAGGCAAGCG GATACACCTTCACCGACTATATCATGCATTGGGTCAGGC AGGCCCCTGGCCAAGGTCTCGAATGGATGGGCTACATTA ACCCATATAATGATGGCTCCAAATACACCGAGAAGTTTC AGGGAAGAGTCACTATGACATCTGACACCAGTATCAGC ACTGCTTACATGGAGCTGTCCCGCCTTCGGTCTGATGAC ACCGCAGTGTATTACTGTGCCAGGGGCACATATTACTAC GGCTCAGCTCTGTTCGACTATTGGGGGCAGGGAACCACA GTAACCGTGAGCTCCGCTAGCACCAAGGGCCCCTCCGTG TTCCCCCTGGCCCCCAGCAGCAAGAGCACCAGCGGCGG CACAGCCGCTCTGGGCTGCCTGGTCAAGGACTACTTCCC CGAGCCCGTGACCGTGTCCTGGAACAGCGGAGCCCTGA CCTCCGGCGTGCACACCTTCCCCGCCGTGCTGCAGAGTT CTGGCCTGTATAGCCTGAGCAGCGTGGTCACCGTGCCTT CTAGCAGCCTGGGCACCCAGACCTACATCTGCAACGTG AACCACAAGCCCAGCAACACCAAGGTGGACAAGAAGGT GGAGCCCAAGAGCTGCGACAAAACTCACACATGCCCAC CGTGCCCAGCACCTGAAGCTGCAGGGGGACCGTCAGTC TTCCTCTTCCCCCCAAAACCCAAGGACACCCTCATGATC TCCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCGC GGGAGGAGCAGTACAACAGCACGTACCGTGTGGTCAGC GTCCTCACCGTCCTGCACCAGGACTGGCTGAATGGCAAG GAGTACAAGTGCAAGGTCTCCAACAAAGCCCTCGGCGC CCCCATCGAGAAAACCATCTCCAAAGCCAAAGGGCAGC CCCGAGAACCACAGGTGTGCACCCTGCCCCCATCCCGG GATGAGCTGACCAAGAACCAGGTCAGCCTCTCGTGCGC AGTCAAAGGCTTCTATCCCAGCGACATCGCCGTGGAGTG GGAGAGCAATGGGCAGCCGGAGAACAACTACAAGACC ACGCCTCCCGTGCTGGACTCCGACGGCTCCTTCTTCCTC GTGAGCAAGCTCACCGTGGACAAGAGCAGGTGGCAGCA GGGGAACGTCTTCTCATGCTCCGTGATGCATGAGGCTCT GCACAACCACTACACGCAGAAGAGCCTCTCCCTGTCTCC GGGTAAA |
| 118 | Nucleotide sequence anti-CD19 (8B8-018) light chain | see Table 5 |
| 202 | Dimeric hu 4-1BBL (71-248) - CL* Fc knob chain | see Table 35 |
| 203 | Monomeric hu 4-1BBL (71-248) - CH1* | see Table 35 |
| 238 | anti-CD19(8B8-018) Fc hole chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGSALFDYWGQGTTVT |

(continued)

| SEQ ID NO: | Description | Sequence |
|---|---|---|
| | | VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGK |
| 120 | anti-CD19(8B8-018) light chain | see Table 5 |

**Table 37b: Sequences of monovalent CD19(8B8-2B11)-targeted human 4-1BB ligand (71-254) containing Fc (kih) fusion molecule Construct 4.1**

| SEQ ID NO: | Description | Sequence |
|---|---|---|
| 239 | nucleotide sequence of dimeric hu 4-1BBL (71-254)-CL* Fc knob chain | AGAGAGGGCCCTGAGCTGAGCCCCGATGATCCTGCTGG<br>ACTGCTGGACCTGCGGCAGGGCATGTTTGCTCAGCTGGT<br>GGCCCAGAACGTGCTGCTGATCGATGGCCCCCTGTCCTG<br>GTACAGCGATCCTGGACTGGCTGGCGTGTCACTGACAGG<br>CGGCCTGAGCTACAAAGAGGACACCAAAGAACTGGTGG<br>TGGCCAAGGCCGGCGTGTACTACGTGTTCTTTCAGCTGG<br>AACTGCGGAGAGTGGTGGCCGGCGAAGGATCTGGCTCT<br>GTGTCTCTGGCCCTGCATCTGCAGCCTCTGAGAAGCGCT<br>GCTGGCGCTGCAGCTCTGGCACTGACAGTGGATCTGCCT<br>CCTGCCAGCTCCGAGGCCCGGAATAGCGCATTTGGGTTT<br>CAAGGCAGGCTGCTGCACCTGTCTGCCGGCCAGAGGCT<br>GGGAGTGCATCTGCACACAGAGGCCAGGGCTAGACACG<br>CCTGGCAGCTGACACAGGGCGCTACAGTGCTGGGCCTG<br>TTCAGAGTGACCCCCGAGATTCCAGCCGGCCTGCCTTCT<br>CCAAGAAGCGAAGGCGGAGGCGGATCTGGCGGCGGAG<br>GATCTAGAGAGGGACCCGAACTGTCCCCTGACGATCCA<br>GCCGGGCTGCTGGATCTGAGACAGGGAATGTTCGCCCA<br>GCTGGTGGCTCAGAATGTGCTGCTGATTGACGGACCTCT<br>GAGCTGGTACTCCGACCCAGGGCTGGCAGGGGTGTCCC<br>TGACTGGGGGACTGTCCTACAAAGAAGATACAAAAGAA<br>CTGGTGGTGGCTAAAGCTGGGGTGTACTATGTGTTTTTT<br>CAGCTGGAACTGAGGCGGGTGGTGGCTGGGGAGGGCTC<br>AGGATCTGTGTCCCTGGCTCTGCATCTGCAGCCACTGCG<br>CTCTGCTGCTGGCGCAGCTGCACTGGCTCTGACTGTGGA<br>CCTGCCACCAGCCTCTAGCGAGGCCAGAAACAGCGCCT<br>TCGGGTTCCAAGGACGCCTGCTGCATCTGAGCGCCGGAC<br>AGCGCCTGGGAGTGCATCTGCATACTGAAGCCAGAGCC<br>CGGCATGCTTGGCAGCTGACTCAGGGGGCAACTGTGCTG<br>GGACTGTTTCGCGTGACACCTGAGATCCCTGCCGGACTG<br>CCAAGCCCTAGATCAGAAGGGGGCGGAGGTTCCGGAGG<br>GGGAGGATCTCGTACGGTGGCTGCACCATCTGTCTTTAT<br>CTTCCCACCCAGCGACCGGAAGCTGAAGTCTGGCACAG<br>CCAGCGTCGTGTGCCTGCTGAATAACTTCTACCCCCGCG |

(continued)

| SEQ ID NO: | Description | Sequence |
|---|---|---|
| | | AGGCCAAGGTGCAGTGGAAGGTGGACAATGCCCTGCAG AGCGGCAACAGCCAGGAAAGCGTGACCGAGCAGGACA GCAAGGACTCCACCTACAGCCTGAGCAGCACCCTGACC CTGAGCAAGGCCGACTACGAGAAGCACAAGGTGTACGC CTGCGAAGTGACCCACCAGGGCCTGTCTAGCCCCGTGAC CAAGAGCTTCAACCGGGGCGAGTGCGACAAGACCCACA CCTGTCCTCCATGCCCTGCCCCTGAAGCTGCTGGCGGCC CTAGCGTGTTCCTGTTCCCCCCAAAGCCCAAGGACACCC TGATGATCAGCCGGACCCCTGAAGTGACCTGCGTGGTGG TGGATGTGTCCCACGAGGACCCTGAAGTGAAGTTCAATT GGTACGTGGACGGCGTGGAAGTGCACAATGCCAAGACC AAGCCGCGGGAGGAGCAGTACAACAGCACGTACCGTGT GGTCAGCGTCCTCACCGTCCTGCACCAGGACTGGCTGAA TGGCAAGGAGTACAAGTGCAAGGTCTCCAACAAAGCCC TCGGCGCCCCCATCGAGAAAACCATCTCCAAAGCCAAA GGGCAGCCCCGAGAACCACAGGTGTACACCCTGCCCCC ATGCCGGGATGAGCTGACCAAGAACCAGGTCAGCCTGT GGTGCCTGGTCAAAGGCTTCTATCCCAGCGACATCGCCG TGGAGTGGGAGAGCAATGGGCAGCCGGAGAACAACTAC AAGACCACGCCTCCCGTGCTGGACTCCGACGGCTCCTTC TTCCTCTACAGCAAGCTCACCGTGGACAAGAGCAGGTG GCAGCAGGGGAACGTCTTCTCATGCTCCGTGATGCATGA GGCTCTGCACAACCACTACACGCAGAAGAGCCTCTCCCT GTCTCCGGGTAAA |
| 240 | nucleotide sequence of monomeric hu 4-1BBL (71-254)-CH1* | AGAGAGGGCCCTGAGCTGAGCCCCGATGATCCTGCTGG ACTGCTGGACCTGCGGCAGGGCATGTTTGCTCAGCTGGT GGCCCAGAACGTGCTGCTGATCGATGGCCCCCTGTCCTG GTACAGCGATCCTGGACTGGCTGGCGTGTCACTGACAGG CGGCCTGAGCTACAAAGAGGACACCAAAGAACTGGTGG TGGCCAAGGCCGGCGTGTACTACGTGTTCTTTCAGCTGG AACTGCGGAGAGTGGTGGCCGGCGAAGGATCTGGCTCT GTGTCTCTGGCCCTGCATCTGCAGCCTCTGAGAAGCGCT GCTGGCGCTGCAGCTCTGGCTCTGACAGTGGATCTGCCT CCTGCCAGCTCCGAGGCCCGGAATAGCGCATTTGGGTTT CAAGGCCGGCTGCTGCACCTGTCTGCCGGCCAGAGACT GGGAGTGCATCTGCACACAGAGGCCAGAGCCAGGCACG CCTGGCAGCTGACACAGGGCGCTACAGTGCTGGGCCTG TTCAGAGTGACCCCCGAGATTCCTGCCGGCCTGCCTAGC CCTAGATCTGAAGGCGGCGGAGGTTCCGGAGGCGGAGG ATCTGCTAGCACAAAGGGCCCCAGCGTGTTCCCTCTGGC CCCTAGCAGCAAGAGCACATCTGGCGGAACAGCCGCCC TGGGCTGCCTGGTGGAAGATTACTTCCCCGAGCCCGTGA CCGTGTCCTGGAATTCTGGCGCCCTGACAAGCGGCGTGC ACACCTTTCCAGCCGTGCTGCAGAGCAGCGGCCTGTACT CTCTGAGCAGCGTCGTGACAGTGCCCAGCAGCTCTCTGG GCACCCAGACCTACATCTGCAACGTGAACCACAAGCCC AGCAACACCAAGGTGGACGAGAAGGTGGAACCCAAGTC CTGC |

(continued)

| SEQ ID NO: | Description | Sequence |
|---|---|---|
| 241 | nucleotide sequence of anti-CD19(8B8-2B11) Fc hole chain | CAGGTGCAATTGGTTCAATCTGGTGCTGAAGTAAAAAA<br>ACCGGGCGCTTCCGTTAAAGTGAGCTGCAAAGCATCTGG<br>TTACACCTTCACTGACTATATCATGCACTGGGTTCGTCA<br>GGCCCCGGGCCAGGGTCTGGAGTGGATGGGCTACATTA<br>ACCCATACAACGACGGTTCCAAATATACCGAGAAATTC<br>CAGGGCCGCGTCACGATGACCAGCGACACTTCTATCTCC<br>ACCGCGTACATGGAACTGTCTAGACTGCGTTCTGACGAC<br>ACCGCTGTTTACTATTGTGCACGCGGTACCTACTACTAC<br>GGTCCACAGCTGTTTGATTACTGGGGCCAAGGTACCACG<br>GTGACCGTAAGCTCTGCTAGCACCAAGGGCCCCTCCGTG<br>TTCCCCCTGGCCCCCAGCAGCAAGAGCACCAGCGGCGG<br>CACAGCCGCTCTGGGCTGCCTGGTCAAGGACTACTTCCC<br>CGAGCCCGTGACCGTGTCCTGGAACAGCGGAGCCCTGA<br>CCTCCGGCGTGCACACCTTCCCCGCCGTGCTGCAGAGTT<br>CTGGCCTGTATAGCCTGAGCAGCGTGGTCACCGTGCCTT<br>CTAGCAGCCTGGGCACCCAGACCTACATCTGCAACGTG<br>AACCACAAGCCCAGCAACACCAAGGTGGACAAGAAGGT<br>GGAGCCCAAGAGCTGCGACAAAACTCACACATGCCCAC<br>CGTGCCCAGCACCTGAAGCTGCAGGGGGACCGTCAGTC<br>TTCCTCTTCCCCCCAAAACCCAAGGACACCCTCATGATC<br>TCCCGGACCCCTGAGGTCACATGCGTGGTGGTGGACGTG<br>AGCCACGAAGACCCTGAGGTCAAGTTCAACTGGTACGT<br>GGACGGCGTGGAGGTGCATAATGCCAAGACAAAGCCGC<br>GGGAGGAGCAGTACAACAGCACGTACCGTGTGGTCAGC<br>GTCCTCACCGTCCTGCACCAGGACTGGCTGAATGGCAAG<br>GAGTACAAGTGCAAGGTCTCCAACAAAGCCCTCGGCGC<br>CCCCATCGAGAAAACCATCTCCAAAGCCAAAGGGCAGC<br>CCCGAGAACCACAGGTGTGCACCCTGCCCCCATCCCGG<br>GATGAGCTGACCAAGAACCAGGTCAGCCTCTCGTGCGC<br>AGTCAAAGGCTTCTATCCCAGCGACATCGCCGTGGAGTG<br>GGAGAGCAATGGGCAGCCGGAGAACAACTACAAGACC<br>ACGCCTCCCGTGCTGGACTCCGACGGCTCCTTCTTCCTC<br>GTGAGCAAGCTCACCGTGGACAAGAGCAGGTGGCAGCA<br>GGGGAACGTCTTCTCATGCTCCGTGATGCATGAGGCTCT<br>GCACAACCACTACACGCAGAAGAGCCTCTCCCTGTCTCC<br>GGGTAAA |
| 124 | nucleotide sequence of anti-CD19(8B8-2B11) light chain | see Table 7 |

(continued)

| SEQ ID NO: | Description | Sequence |
|---|---|---|
| 242 | Dimeric hu 4-1BBL (71-254)-CL* Fc knob chain | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSREGPEL SPDDPAGLLDLRQGMFAQLVAQNVLLIDGPLSWYSDPGLA GVSLTGGLSYKEDTKELVVAKAGVYYVFFQLELRRVVAG EGSGSVSLALHLQPLRSAAGAAALALTVDLPPASSEARNS AFGFQGRLLHLSAGQRLGVHLHTEARARHAWQLTQGATV LGLFRVTPEIPAGLPSPRSEGGGGSGGGGSRTVAAPSVFIFP PSDRKLKSGTASVVCLLNNFYPREAKVQWKVDNALQSGN SQESVTEQDSKDSTYSLSSTLTLSKADYEKHKVYACEVTH QGLSSPVTKSFNRGECDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCKV SNKALGAPIEKTISKAKGQPREPQVYTLPPCRDELTKNQVS LWCLVKGFYPSDIAVEWESNGQPENNYKTTPPVLDSDGSF FLYSKLTVDKSRWQQGNVFSCSVMHEALHNHYTQKSLSL SPGK |
| 243 | Monomeric hu 4-1BBL (71-254)-CH1* | REGPELSPDDPAGLLDLRQGMFAQLVAQNVLLIDGPLSWY SDPGLAGVSLTGGLSYKEDTKELVVAKAGVYYVFFQLELR RVVAGEGSGSVSLALHLQPLRSAAGAAALALTVDLPPASS EARNSAFGFQGRLLHLSAGQRLGVHLHTEARARHAWQLT QGATVLGLFRVTPEIPAGLPSPRSEGGGGSGGGGSASTKGP SVFPLAPSSKSTSGGTAALGCLVEDYFPEPVTVSWNSGALT SGVHTFPAVLQSSGLYSLSSVVTVPSSSLGTQTYICNVNHK PSNTKVDEKVEPKSC |
| 244 | CD19(8B8-2B11) Fc hole chain | QVQLVQSGAEVKKPGASVKVSCKASGYTFTDYIMHWVRQ APGQGLEWMGYINPYNDGSKYTEKFQGRVTMTSDTSISTA YMELSRLRSDDTAVYYCARGTYYYGPQLFDYWGQGTTVT VSSASTKGPSVFPLAPSSKSTSGGTAALGCLVKDYFPEPVT VSWNSGALTSGVHTFPAVLQSSGLYSLSSVVTVPSSSLGTQ TYICNVNHKPSNTKVDKKVEPKSCDKTHTCPPCPAPEAAG GPSVFLFPPKPKDTLMISRTPEVTCVVVDVSHEDPEVKFNW YVDGVEVHNAKTKPREEQYNSTYRVVSVLTVLHQDWLN GKEYKCKVSNKALGAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGK |
| 126 | anti- CD19(8B8-2B11) light chain | see Table 7 |

An additional control used in the assays, termed control F, was an untargeted DP47, germline control, human IgG1, containing the Pro329Gly, Leu234Ala and Leu235Ala mutations (termed PGLALA) to abrogate binding to Fc gamma receptors according to the method described in International Patent Appl. Publ. No. WO 2012/130831 A1.

Table 38 shows the cDNA and amino acid sequences of the "untargeted" DP47 hu IgG1 PGLALA antibody (Control F).

**Table 38: Sequences of DP47-untargeted human IgG1 PGLALA (Control F)**

| SEQ ID NO: | Description | Sequence |
|---|---|---|
| 205 | nucleotide sequence of DP47 heavy chain (huIgG1 PGLALA) | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGTGC TAGCACCAAGGGCCCATCGGTCTTCCCCCTGGCACCCTC CTCCAAGAGCACCTCTGGGGGCACAGCGGCCCTGGGCT GCCTGGTCAAGGACTACTTCCCCGAACCGGTGACGGTGT CGTGGAACTCAGGCGCCCTGACCAGCGGCGTGCACACC TTCCCGGCTGTCCTACAGTCCTCAGGACTCTACTCCCTC AGCAGCGTGGTGACCGTGCCCTCCAGCAGCTTGGGCAC CCAGACCTACATCTGCAACGTGAATCACAAGCCCAGCA ACACCAAGGTGGACAAGAAAGTTGAGCCCAAATCTTGT GACAAAACTCACACATGCCCACCGTGCCCAGCACCTGA AGCTGCAGGGGGACCGTCAGTCTTCCTCTTCCCCCCAAA ACCCAAGGACACCCTCATGATCTCCCGGACCCCTGAGGT CACATGCGTGGTGGTGGACGTGAGCCACGAAGACCCTG AGGTCAAGTTCAACTGGTACGTGGACGGCGTGGAGGTG CATAATGCCAAGACAAAGCCGCGGGAGGAGCAGTACAA CAGCACGTACCGTGTGGTCAGCGTCCTCACCGTCCTGCA CCAGGACTGGCTGAATGGCAAGGAGTACAAGTGCAAGG TCTCCAACAAAGCCCTCGGCGCCCCCATCGAGAAAACC ATCTCCAAAGCCAAAGGGCAGCCCCGAGAACCACAGGT GTACACCCTGCCCCCATCCCGGGATGAGCTGACCAAGA ACCAGGTCAGCCTGACCTGCCTGGTCAAAGGCTTCTATC CCAGCGACATCGCCGTGGAGTGGGAGAGCAATGGGCAG CCGGAGAACAACTACAAGACCACGCCTCCCGTGCTGGA CTCCGACGGCTCCTTCTTCCTCTACAGCAAGCTCACCGT GGACAAGAGCAGGTGGCAGCAGGGGAACGTCTTCTCAT GCTCCGTGATGCATGAGGCTCTGCACAACCACTACACGC AGAAGAGCCTCTCCCTGTCTCCGGGTAAA |
| 166 | nucleotide sequence of DP47 light chain | see Table 21 |

(continued)

| SEQ ID NO: | Description | Sequence |
|---|---|---|
| 206 | DP47 heavy chain (huIgG1 PGLALA) | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCAKGSGFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 168 | DP47 light chain | see Table 21 |

## Example 2

### Production of monovalent targeted human 4-1BB ligand trimer-containing Fc fusion antigen binding molecules

The targeted and untargeted monovalent trimeric 4-1BB ligand C-terminal Fc (kih) fusion antigen binding molecule encoding sequences were cloned into a plasmid vector, which drives expression of the insert from an MPSV promoter and contains a synthetic polyA sequence located at the 3' end of the CDS. In addition, the vector contains an EBV OriP sequence for episomal maintenance of the plasmid.

The split trimeric 4-1BB ligand Fc (kih) fusion antigen binding molecules were produced by co-transfecting HEK293-EBNA cells with the mammalian expression vectors using polyethylenimine. The cells were transfected with the corresponding expression vectors. For variants x. 11, x. 12, x. 13, x. 14 and the control molecules H, I, J and K, at a 1:1:1 ratio ("vector knob chain": "vector hole chain": "vector light chain"). For control D, at a ratio of 1: 1: 1: 1 ("vector knob chain": "vector hole chain": "vector light chain": "vector light chain2") and for human IgG1, control F, at a 1:1 ratio ("vector heavy chain": "vector light chain).

For production in 500 mL shake flasks, 300 million HEK293 EBNA cells were seeded 24 hours before transfection. For transfection cells were centrifuged for 10 minutes at 210 x g, and the supernatant was replaced by 20mL pre-warmed CD CHO medium. Expression vectors (200 μg of total DNA) were mixed in 20 mL CD CHO medium. After addition of 540 μL PEI, the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5% $CO_2$ atmosphere. After the incubation, 160 mL of Excell medium supplemented with 6mM L-Glutamine, 5g/L PEPSOY and 1.2mM valproic acid was added and cells were cultured for 24 hours. One day after transfection 12% Feed were added. After culturing for 7 days, the supernatant was collected by centrifugation for 30-40 minutes at least 400 x g. The solution was sterile filtered (0.22 μm filter), supplemented with sodium azide to a final concentration of 0.01 % (w/v), and kept at 4 °C.

Secreted proteins were purified from cell culture supernatants by affinity chromatography using Protein A, followed by size exclusion chromatography. For affinity chromatography, the supernatant was loaded on a MabSelect Sure column (CV = 5-15 mL, resin from GE Healthcare) equilibrated with Sodium Phosphate (20 mM), Sodium Citrate (20 mM) buffer (pH 7.5). Unbound protein was removed by washing with at least 6 column volumes of the same buffer. The bound protein was eluted using either a linear gradient (20 CV) or a step elution (8 CV) with 20 mM sodium citrate, 100 mM Sodium chloride, 100 mM Glycine buffer (pH 3.0). For the linear gradient an additional 4 column volumes step elution was applied.

The pH of collected fractions was adjusted by adding 1/10 (v/v) of 0.5M sodium phosphate, pH 8.0. The protein was concentrated prior to loading on a HiLoad Superdex 200 column (GE Healthcare) equilibrated with 20 mM Histidine, 140 mM sodium chloride, 0.01% (v/v) Tween20 solution of pH 6.0.

The protein concentration was determined by measuring the optical density (OD) at 280 nm, using a molar extinction coefficient calculated on the basis of the amino acid sequence. Purity and molecular weight of the targeted trimeric 4-1BB ligand Fc (kih) fusion antigen binding molecules was analyzed by SDS-PAGE in the presence and absence of a reducing agent (5 mM 1,4-dithiotreitol) and staining with Coomassie SimpleBlue™ SafeStain (Invitrogen USA) or CE-SDS using Caliper LabChip GXII (Perkin Elmer). The aggregate content of samples was analyzed using a TSKgel G3000 SW XL analytical size-exclusion column (Tosoh) equilibrated in 25 mM $K_2HPO_4$, 125 mM NaCl, 200 mM L-Arginine Monohydrocloride, 0.02 % (w/v) $NaN_3$, pH 6.7 running buffer at 25°C.

Table 39 summarizes the yield and final monomer content of the targeted split trimeric C-terminal 4-1BB ligand Fc(kih) fusion antigen binding molecules.

**Table 39: Biochemical analysis of exemplary targeted split trimeric C-terminal 4-1BB ligand Fc(kih) fusion antigen binding molecules**

| Construct | Monomer [%] (SEC) | Yield [mg/l] |
|---|---|---|
| monovalent targeted FAP(4B9) split trimeric C-terminal 4-1BB ligand (71-248) Fc (kih) fusion antigen binding molecule Construct 2.11 | 97.8 | 19 |
| monovalent targeted CD19(8B8-018) split trimeric C-terminal 4-1BB ligand (71-248) Fc (kih) fusion antigen binding molecule Construct 3.11 | 100 | 16 |
| monovalent targeted CD19(8B8-2B11) split trimeric C-terminal 4-1BB ligand (71-248) Fc (kih) fusion antigen binding molecule Construct 4.11 | 100 | 6 |
| monovalent DP47-untargeted split trimeric human 4-1BB ligand (71-254) Fc (kih) fusion antigen (Control D) | 99.5 | 25.9 |
| germline DP47 human IgG1 PGLALA Control F | 100 | 50 |

## Example 3

## Affinity determination by surface plasmon resonance

### 3.1 Preparation, purification and characterization of antigens or surface plasmon resonance

Human CD19 Fc fusion protein

The preparation, purification and characterization of the human CD19 ectodomain fused to a human IgG1 Fc domain is described in Example 1.3.2.

Human 4-1BB

The ectodomain of human 4-1BB (Q07011, amino acid 24-186) was subcloned in frame with an avi (GLNDIFEAQKIEWHE, SEQ ID NO. 245) and a hexahistidine tag (Table 40).

**Table 40: Sequences of monomeric human 4-1BB His molecule**

| SEQ ID NO: | antigen | Sequence |
|---|---|---|
| 207 | nucleotide sequence of human 4-1BB His | CTGCAGGACCCCTGCAGCAACTGCCCTGCCGGCACCT TCTGCGACAACAACCGGAACCAGATCTGCAGCCCCTG CCCCCCCAACAGCTTCAGCTCTGCCGGCGGACAGCGG ACCTGCGACATCTGCAGACAGTGCAAGGGCGTGTTCA GAACCCGGAAAGAGTGCAGCAGCACCAGCAACGCCG AGTGCGACTGCACCCCCGGCTTCCATTGTCTGGGAGC CGGCTGCAGCATGTGCGAGCAGGACTGCAAGCAGGG CCAGGAACTGACCAAGAAGGGCTGCAAGGACTGCTG CTTCGGCACCTTCAACGACCAGAAGCGGGGCATCTGC CGGCCCTGGACCAACTGTAGCCTGGACGGCAAGAGCG TGCTGGTCAACGGCACCAAAGAACGGGACGTCGTGTG CGGCCCCAGCCCTGCTGATCTGTCTCCTGGGGCCAGC AGCGTGACCCCTCCTGCCCCTGCCAGAGAGCCTGGCC ACTCTCCTCAGGTCGACGAACAGTTATATTTTCAGGG CGGCTCAGGCCTGAACGACATCTTCGAGGCCCAGAAG ATCGAGTGGCACGAGGCTCGAGCTCACCACCATCACC ATCAC |
| 208 | human 4-1BB His | LQDPCSNCPAGTFCDNNRNQICSPCPPNSFSSAGGQRTC DICRQCKGVFRTRKECSSTSNAECDCTPGFHCLGAGCSM CEQDCKQGQELTKKGCKDCCFGTFNDQKRGICRPWTNC SLDGKSVLVNGTKERDVVCGPSPADLSPGASSVTPPAPA REPGHSPQVDEQLYFQGGSGLNDIFEAQKIEWHEARAH HHHHH |

Protein production was performed as described above for the CD19 Fc fusion protein in Example 1.3.2. Secreted proteins were purified from cell culture supernatants by chelating chromatography, followed by size exclusion chromatography.

The first chromatographic step was performed on a NiNTA Superflow Cartridge (5ml, Qiagen) equilibrated in 20mM sodium phosphate, 500nM sodium chloride, pH7.4. Elution was performed by applying a gradient over 12 column volume from 5% to 45% of elution buffer (20mM sodium phosphate, 500nM sodium chloride, 500mM Imidazole, pH7.4).

The protein was concentrated and filtered prior to loading on a HiLoad Superdex 75 column (GE Healthcare) equilibrated with 2mM MOPS, 150mM sodium chloride, 0.02% (w/v) sodium azide solution of pH 7.4. Table 41 summarizes the yield and final monomer content of monomeric human avi His-tagged 4-1BB.

**Table 41: Biochemical analysis of monomeric human 4-1BB avi His**

| Construct | Monomer [%] (SEC) | Yield [mg/l] |
|---|---|---|
| monomeric human 4-1BB avi His | 100 | 16.4 |

**3.2 Affinity Determination**

Binding of targeted split trimeric C-terminal 4-1BB ligand antigen binding molecules to the recombinant 4-1BB or CD19 was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Affinity for human 4-1BB

Anti-human Fc antibody (Biacore, Freiburg/Germany) was directly coupled on a CM5 chip at pH 5.0 using the standard amine coupling kit (Biacore, Freiburg/Germany). The immobilization level was approximately 4000 RU. Targeted split trimeric C-terminal 4-1BB ligand was captured for 90 seconds at concentration of 4 nM. Recombinant human 4-1BB avi his was passed at a concentration range from 2.7 to 2000 nM with a flow of 30 μL/minutes through the flow cells over 120 seconds. The dissociation was monitored for 120 seconds. Bulk refractive index differences were corrected for by subtracting the response obtained on reference flow cell. Here, the antigens were flown over a surface with immobilized anti-human Fc antibody but on which HBS-EP has been injected rather than the antibodies.

Affinity for human CD19

Anti-human Fab antibody (Biacore, Freiburg/Germany) was directly coupled on a CM5 chip at pH 5.0 using the standard amine coupling kit (Biacore, Freiburg/Germany). The immobilization level was approximately 7000 RU. Targeted split trimeric C-terminal 4-1BB ligand was captured for 60 seconds at concentrations between 75 and 100 nM. Recombinant human CD19 Fc(kih) was passed at a concentration range from 7.8 to 500 nM with a flow of 30 μL/minutes through the flow cells over 220 seconds. The dissociation was monitored for 600/2500 seconds. Bulk refractive index differences were corrected for by subtracting the response obtained on reference flow cell. Here, the antigens were flown over a surface with immobilized anti-human Fc antibody but on which HBS-EP has been injected rather than the antibodies.

Kinetic constants were derived using the Biacore T200 Evaluation Software (vAA, Biacore AB, Uppsala/Sweden), to fit rate equations for 1:1 Langmuir binding by numerical integration and used to estimate qualitatively the avidity.

**Table 42: Binding of targeted split trimeric C-terminal 4-1BB ligand antigen binding molecules to recombinant human 4-1BB**

| Construct | Recombinant human 4-1BB | | | |
|---|---|---|---|---|
| | ka (1/Ms) | kd (1/s) | KD (M) | KD (M) |
| Construct 2.11 | 7.62E+04 | 2.63E-02 | 3.47E-07 | 2.079E-08 |
| Construct 4.11 | 7.37E+04 | 2.72E-02 | 3.69E-07 | 1.762E-08 |

Affinity constants for the interaction between targeted split trimeric C-terminal 4-1BB ligand antigen binding molecules and human 4-1BB avi his were determined by fitting to a 1:1 Langmuir binding. Results are the average from three experiments.

**Table 43: Binding of targeted split trimeric C-terminal 4-1BB ligand to recombinant human CD19**

| **Construct** | **Recombinant human CD19** | | | |
|---|---|---|---|---|
| | **ka (1/Ms)** | **kd (1/s)** | **KD (M)** | **KD (M)** |
| Construct 4.11 | 5.22E+04 | 4.57E-05 | **8.85E-10** | **1.32E-10** |

Affinity constants for the interaction between targeted split trimeric C-terminal 4-1BB ligand antigen binding molecules and human CD19 Fc(kih) were determined by fitting to a 1:1 Langmuir binding. Results are the average from three experiments.

**3.3 Determination of simultaneous binding of targeted C-terminal 4-1BB split trimeric ligand Fc fusion antigen binding molecules by surface plasmon resonance**

The capacity of binding simultaneously human 4-1BB Fc(kih) and human FAP, or human CD19, respectively was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated human 4-1BB Fc(kih) was directly coupled to a flow cell of a streptavidin (SA) sensor chip. Immobilization levels up to 200 resonance units (RU) were used. The targeted trimeric C-terminal 4-1BB ligand Fc (kih) fusion molecules were passed at a concentration range of 200 nM with a flow of 30 μL/minute through the flow cells over 90 seconds and dissociation was set to zero seconds. Human FAP or human CD19 Fc(kih) was injected as second analyte with a flow of 30 μL/minute through the flow cells over 90 seconds at a concentration of 500 nM (Figure 4A). The dissociation was monitored for 120 sec. Bulk refractive index differences were corrected for by subtracting the response obtained in a reference flow cell, where no protein was immobilized. The bispecific constructs could bind simultaneously human 4-1BB and human FAP or human CD19, respectively (Figure 4B, 4C and 4D).

**Example 4**

**Preparation and purification of FAP-targeted/DP47 human 4-1BB ligand trimer-containing Fc fusion antigen binding molecules**

**4.1 Preparation of monovalent FAP(4B9)-targeted/DP47 4-1BB ligand trimer-containing Fc (kih) fusion antigen binding molecule, wherein the 4-1BB ligands are C-terminally fused (Constructs 2.15 and 2.16) (FAP on the hole chain)**

The DNA sequence encoding part of the ectodomain (amino acids 71-254 of human 4-1BB ligand was synthetized according to the P41273 sequence of Uniprot database.

A polypeptide containing two ectodomains of 4-1BB ligand, separated by (G4S)2 linkers was subcloned in frame to the C-terminus of human IgG1 Fc hole or knob chain, as depicted in Figure 1A: human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in Figure 1B: human IgG1 Fc, (G4S)2 connector, human 4-1BB ligand.

The variable region of heavy and light chain DNA sequences encoding a binder specific for fibroblast activation protein (FAP), clone 4B9, were subcloned in frame with either the constant heavy chain of the hole or the constant light chain of human IgG1. The variable region of heavy and light chain DNA sequences encoding a non-binding clone, clone DP47, were subcloned in frame with either the constant heavy chain of the knob or the constant light chain of human IgG1. The crossmab technology was applied to reduce the formation of wrongly paired light chains (International patent application No. WO 2010/145792 A1). In this example a crossed Fab unit (VHCL) of the binder DP47 was fused to the knob chain.

The Pro329Gly, Leu234Ala and Leu235Ala mutations have been introduced in the constant region of the knob and hole heavy chains to abrogate binding to Fc gamma receptors (International Patent Appl. Publ. No. WO 2012/130831 A1).

Combination of the anti-FAP huIgG1 Fc hole chain containing the Y349C/T366S/L368A/Y407V mutations, the DP47 huIgG1 Fc knob chain containing the S354C/T366W mutations and the two light chains, anti-FAP and DP47, allows generation of a heterodimer, which includes an assembled trimeric 4-1BB ligand, one FAP binding Fab and one non binding DP47 Fab (Figures 5A and 5B).

Table 44 shows the cDNA and amino acid sequences of the monovalent FAP(4B9)-targeted 4-1BB ligand (71-254) trimer-containing Fc (kih) fusion antigen binding molecule, wherein the dimeric 4-1BBL is fused to the hole chain (Construct 2.15).

**Table 44: Sequences of FAP(4B9)-targeted/DP47 human 4-1BB ligand trimer containing Fc (kih) fusion antigen binding molecule Construct 2.15 (comparative example)**

| SEQ ID NO: | Description | Sequence |
|---|---|---|
| 209 | nucleotide sequence of anti-FAP(4B9) Fc hole dimeric 4-1BB ligand (71-254) chain | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCCTCCGTGTTCCCCCT GGCCCCCAGCAGCAAGAGCACCAGCGGCGGCACAGCCG CTCTGGGCTGCCTGGTCAAGGACTACTTCCCCGAGCCCG TGACCGTGTCCTGGAACAGCGGAGCCCTGACCTCCGGC |

(continued)

| SEQ ID NO: | Description | Sequence |
|---|---|---|
| | | GTGCACACCTTCCCCGCCGTGCTGCAGAGTTCTGGCCTG TATAGCCTGAGCAGCGTGGTCACCGTGCCTTCTAGCAGC CTGGGCACCCAGACCTACATCTGCAACGTGAACCACAA GCCCAGCAACACCAAGGTGGACAAGAAGGTGGAGCCCA AGAGCTGCGACAAAACTCACACATGCCCACCGTGCCCA GCACCTGAAGCTGCAGGGGGACCGTCAGTCTTCCTCTTC CCCCCAAAACCCAAGGACACCCTCATGATCTCCCGGACC CCTGAGGTCACATGCGTGGTGGTGGACGTGAGCCACGA AGACCCTGAGGTCAAGTTCAACTGGTACGTGGACGGCG TGGAGGTGCATAATGCCAAGACAAAGCCGCGGGAGGAG CAGTACAACAGCACGTACCGTGTGGTCAGCGTCCTCACC GTCCTGCACCAGGACTGGCTGAATGGCAAGGAGTACAA GTGCAAGGTCTCCAACAAAGCCCTCGGCGCCCCCATCG AGAAAACCATCTCCAAAGCCAAAGGGCAGCCCCGAGAA CCACAGGTGTGCACCCTGCCCCCATCCCGGGATGAGCTG ACCAAGAACCAGGTCAGCCTCTCGTGCGCAGTCAAAGG CTTCTATCCCAGCGACATCGCCGTGGAGTGGGAGAGCA ATGGGCAGCCGGAGAACAACTACAAGACCACGCCTCCC GTGCTGGACTCCGACGGCTCCTTCTTCCTCGTGAGCAAG CTCACCGTGGACAAGAGCAGGTGGCAGCAGGGGAACGT CTTCTCATGCTCCGTGATGCATGAGGCTCTGCACAACCA CTACACGCAGAAGAGCCTCTCCCTGTCTCCGGGTGGAGG CGGCGGAAGCGGAGGAGGAGGATCCAGAGAGGGCCCT GAGCTGAGCCCCGATGATCCTGCTGGACTGCTGGACCTG CGGCAGGGCATGTTTGCTCAGCTGGTGGCCCAGAACGTG CTGCTGATCGATGGCCCCCTGTCCTGGTACAGCGATCCT GGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGAAGCGCTGCTGGCGCTGCAG CTCTGGCACTGACAGTGGATCTGCCTCCTGCCAGCTCCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC CCGAGATTCCAGCCGGCCTGCCTTCTCCAAGAAGCGAA GGCGGAGGCGGATCTGGCGGCGGAGGATCTAGAGAGGG ACCCGAACTGTCCCCTGACGATCCAGCCGGGCTGCTGGA TCTGAGACAGGGAATGTTCGCCCAGCTGGTGGCTCAGA ATGTGCTGCTGATTGACGGACCTCTGAGCTGGTACTCCG ACCCAGGGCTGGCAGGGGTGTCCCTGACTGGGGGACTG TCCTACAAAGAAGATACAAAAGAACTGGTGGTGGCTAA AGCTGGGGTGTACTATGTGTTTTTTCAGCTGGAACTGAG GCGGGTGGTGGCTGGGGAGGGCTCAGGATCTGTGTCCCT GGCTCTGCATCTGCAGCCACTGCGCTCTGCTGCTGGCGC AGCTGCACTGGCTCTGACTGTGGACCTGCCACCAGCCTC TAGCGAGGCCAGAAACAGCGCCTTCGGGTTCCAAGGAC GCCTGCTGCATCTGAGCGCCGGACAGCGCCTGGGAGTG CATCTGCATACTGAAGCCAGAGCCCGGCATGCTTGGCA GCTGACTCAGGGGGCAACTGTGCTGGGACTGTTTCGCGT GACACCTGAGATCCCTGCCGGACTGCCAAGCCCTAGATC AGAA |

(continued)

| SEQ ID NO: | Description | Sequence |
|---|---|---|
| 210 | nucleotide sequence of DP47 (VHCL) Fc knob monomeric 4-1BB (71-254) ligand | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAGACCCACACCTGTCCCCCTTG TCCTGCCCCTGAAGCTGCTGGCGGCCCTTCTGTGTTCCT GTTCCCCCCAAAGCCCAAGGACACCCTGATGATCAGCC GGACCCCCGAAGTGACCTGCGTGGTGGTGGATGTGTCCC ACGAGGACCCTGAAGTGAAGTTCAATTGGTACGTGGAC GGCGTGGAAGTGCACAACGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTACACCCTGCCCCCCTGCAGAGAT GAGCTGACCAAGAACCAGGTGTCCCTGTGGTGTCTGGTC AAGGGCTTCTACCCCAGCGATATCGCCGTGGAGTGGGA GAGCAACGGCCAGCCTGAGAACAACTACAAGACCACCC CCCCTGTGCTGGACAGCGACGGCAGCTTCTTCCTGTACT CCAAACTGACCGTGGACAAGAGCCGGTGGCAGCAGGGC AACGTGTTCAGCTGCAGCGTGATGCACGAGGCCCTGCA CAACCACTACACCCAGAAGTCCCTGAGCCTGAGCCCCG GCGGAGGCGGCGGAAGCGGAGGAGGAGGATCCAGAGA GGGCCCTGAGCTGAGCCCCGATGATCCTGCTGGACTGCT GGACCTGCGGCAGGGCATGTTTGCTCAGCTGGTGGCCCA GAACGTGCTGCTGATCGATGGCCCCCTGTCCTGGTACAG CGATCCTGGACTGGCTGGCGTGTCACTGACAGGCGGCCT GAGCTACAAAGAGGACACCAAAGAACTGGTGGTGGCCA AGGCCGGCGTGTACTACGTGTTCTTTCAGCTGGAACTGC GGAGAGTGGTGGCCGGCGAAGGATCTGGCTCTGTGTCTC TGGCCCTGCATCTGCAGCCTCTGAGAAGCGCTGCTGGCG CTGCAGCTCTGGCACTGACAGTGGATCTGCCTCCTGCCA GCTCCGAGGCCCGGAATAGCGCATTTGGGTTTCAAGGCA GGCTGCTGCACCTGTCTGCCGGCCAGAGGCTGGGAGTG CATCTGCACACAGAGGCCAGGGCTAGACACGCCTGGCA GCTGACACAGGGCGCTACAGTGCTGGGCCTGTTCAGAGT GACCCCCGAGATTCCAGCCGGCCTGCCTTCTCCAAGAAG CGAA |

(continued)

| SEQ ID NO: | Description | Sequence |
|---|---|---|
| 103 | nucleotide sequence of anti-FAP(4B9) light chain | see Table 1 |
| 211 | nucleotide sequence of DP47 (VL-CH1) light chain | GAAATCGTGTTAACGCAGTCTCCAGGCACCCTGTCTTTG TCTCCAGGGGAAAGAGCCACCCTCTCTTGCAGGGCCAGT CAGAGTGTTAGCAGCAGCTACTTAGCCTGGTACCAGCAG AAACCTGGCCAGGCTCCCAGGCTCCTCATCTATGGAGCA TCCAGCAGGGCCACTGGCATCCCAGACAGGTTCAGTGG CAGTGGATCCGGGACAGACTTCACTCTCACCATCAGCAG ACTGGAGCCTGAAGATTTTGCAGTGTATTACTGTCAGCA GTATGGTAGCTCACCGCTGACGTTCGGCCAGGGGACCA AAGTGGAAATCAAGAGCTCCGCTAGCACCAAAGGCCCT TCCGTGTTTCCTCTGGCTCCTAGCTCCAAGTCCACCTCTG GAGGCACCGCTGCTCTCGGATGCCTCGTGAAGGATTATT TTCCTGAGCCTGTGACAGTGTCCTGGAATAGCGGAGCAC TGACCTCTGGAGTGCATACTTTCCCCGCTGTGCTGCAGT CCTCTGGACTGTACAGCCTGAGCAGCGTGGTGACAGTGC CCAGCAGCAGCCTGGGCACCCAGACCTACATCTGCAAC GTGAACCACAAGCCCAGCAACACCAAGGTGGACAAGAA GGTGGAACCCAAGTCTTGT |
| 212 | anti-FAP(4B9) Fc hole dimeric 4-1BB ligand (71-254) chain | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGLPSPRSEG GGGSGGGGSREGPELSPDDPAGLLDLRQGMFAQLVAQNV LLIDGPLSWYSDPGLAGVSLTGGLSYKEDTKELVVAKAGV YYVFFQLELRRVVAGEGSGSVSLALHLQPLRSAAGAAALA LTVDLPPASSEARNSAFGFQGRLLHLSAGQRLGVHLHTEA RARHAWQLTQGATVLGLFRVTPEIPAGLPSPRSE |

(continued)

| SEQ ID NO: | Description | Sequence |
|---|---|---|
| 213 | DP47 (VH-CL) Fc knob monomeric 4-1BB (71-254) ligand | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRDE LTKNQVSLWCLVKGFYPSDIAVEWESNGQPENNYKTTPPV LDSDGSFFLYSKLTVDKSRWQQGNVFSCSVMHEALHNHY TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGLPS PRSE |
| 106 | anti-FAP(4B9) light chain | see Table 1 |
| 214 | DP47 (VL-CH1) light chain | EIVLTQSPGTLSLSPGERATLSCRASQSVSSSYLAWYQQKP GQAPRLLIYGASSRATGIPDRFSGSGSGTDFTLTISRLEPEDF AVYYCQQYGSSPLTFGQGTKVEIKSSASTKGPSVFPLAPSS KSTSGGTAALGCLVKDYFPEPVTVSWNSGALTSGVHTFPA VLQSSGLYSLSSVVTVPSSSLGTQTYICNVNHKPSNTKVDK KVEPKSC |

Table 45 shows the cDNA and amino acid sequences of the monovalent FAP(4B9)-targeted/DP47 4-1BB ligand (71-254) trimer-containing Fc (kih) fusion antigen binding molecule, wherein the dimeric 4-1BBL is fused to the Fc knob chain (Construct 2.16).

**Table 45: Sequences of FAP(4B9)-targeted human 4-1BB ligand trimer containing Fc (kih) fusion molecule Construct 2.16 (comparative example)**

| SEQ ID NO: | Description | Sequence |
|---|---|---|
| 215 | nucleotide sequence of anti-FAP(4B9) Fc hole monomeric 4-1BBL (71-254) chain | GAGGTGCAGCTGCTCGAAAGCGGCGGAGGACTGGTGCA<br>GCCTGGCGGCAGCCTGAGACTGTCTTGCGCCGCCAGCG<br>GCTTCACCTTCAGCAGCTACGCCATGAGCTGGGTCCGCC<br>AGGCCCCTGGCAAGGGACTGGAATGGGTGTCCGCCATC<br>ATCGGCTCTGGCGCCAGCACCTACTACGCCGACAGCGTG<br>AAGGGCCGGTTCACCATCAGCCGGGACAACAGCAAGAA<br>CACCCTGTACCTGCAGATGAACAGCCTGCGGGCCGAGG<br>ACACCGCCGTGTACTACTGCGCCAAGGGATGGTTCGGC<br>GGCTTCAACTACTGGGGACAGGGCACCCTGGTCACAGT<br>GTCCAGCGCTAGCACCAAGGGCCCCTCCGTGTTCCCCCT<br>GGCCCCCAGCAGCAAGAGCACCAGCGGCGGCACAGCCG<br>CTCTGGGCTGCCTGGTCAAGGACTACTTCCCCGAGCCCG<br>TGACCGTGTCCTGGAACAGCGGAGCCCTGACCTCCGGC<br>GTGCACACCTTCCCCGCCGTGCTGCAGAGTTCTGGCCTG<br>TATAGCCTGAGCAGCGTGGTCACCGTGCCTTCTAGCAGC<br>CTGGGCACCCAGACCTACATCTGCAACGTGAACCACAA<br>GCCCAGCAACACCAAGGTGGACAAGAAGGTGGAGCCCA<br>AGAGCTGCGACAAAACTCACACATGCCCACCGTGCCCA<br>GCACCTGAAGCTGCAGGGGGACCGTCAGTCTTCCTCTTC<br>CCCCCAAAACCCAAGGACACCCTCATGATCTCCCGGACC<br>CCTGAGGTCACATGCGTGGTGGTGGACGTGAGCCACGA<br>AGACCCTGAGGTCAAGTTCAACTGGTACGTGGACGGCG<br>TGGAGGTGCATAATGCCAAGACAAAGCCGCGGGAGGAG |

(continued)

| SEQ ID NO: | Description | Sequence |
|---|---|---|
| | | CAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTGCACCCTGCCCCCATCCCGGGATGAGCTGACCAAGAACCAGGTCAGCCTCTCGTGCGCAGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTGGAGGCGGCGGAAGCGGAGGAGGAGGATCCAGAGAGGGCCCTGAGCTGAGCCCCGATGATCCTGCTGGACTGCTGGACCTGCGGCAGGGCATGTTTGCTCAGCTGGTGGCCCAGAACGTGCTGCTGATCGATGGCCCCCTGTCCTGGTACAGCGATCCTGGACTGGCTGGCGTGTCACTGACAGGCGGCCTGAGCTACAAAGAGGACACCAAAGAACTGGTGGTGGCCAAGGCCGGCGTGTACTACGTGTTCTTTCAGCTGGAACTGCGGAGAGTGGTGGCCGGCGAAGGATCTGGCTCTGTGTCTCTGGCCCTGCATCTGCAGCCTCTGAGAAGCGCTGCTGGCGCTGCAGCTCTGGCACTGACAGTGGATCTGCCTCCTGCCAGCTCCGAGGCCCGGAATAGCGCATTTGGGTTTCAAGGCAGGCTGCTGCACCTGTCTGCCGGCCAGAGGCTGGGAGTGCATCTGCACACAGAGGCCAGGGCTAGACACGCCTGGCAGCTGACACAGGGCGCTACAGTGCTGGGCCTGTTCAGAGTGACCCCCGAGATTCCAGCCGGCCTGCCTTCTCCAAGAAGCGAA |

(continued)

| SEQ ID NO: | Description | Sequence |
|---|---|---|
| 216 | nucleotide sequence of DP47 (VH-CL) Fc knob dimeric 4-1BB ligand (71-254) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAGACCCACACCTGTCCCCCTTG TCCTGCCCCTGAAGCTGCTGGCGGCCCTTCTGTGTTCCT GTTCCCCCCAAAGCCCAAGGACACCCTGATGATCAGCC GGACCCCCGAAGTGACCTGCGTGGTGGTGGATGTGTCCC ACGAGGACCCTGAAGTGAAGTTCAATTGGTACGTGGAC GGCGTGGAAGTGCACAACGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC |

(continued)

| SEQ ID NO: | Description | Sequence |
|---|---|---|
| | | GAGAACCACAGGTGTACACCCTGCCCCCCTGCAGAGAT GAGCTGACCAAGAACCAGGTGTCCCTGTGGTGTCTGGTC AAGGGCTTCTACCCCAGCGATATCGCCGTGGAGTGGGA GAGCAACGGCCAGCCTGAGAACAACTACAAGACCACCC CCCCTGTGCTGGACAGCGACGGCAGCTTCTTCCTGTACT CCAAACTGACCGTGGACAAGAGCCGGTGGCAGCAGGGC AACGTGTTCAGCTGCAGCGTGATGCACGAGGCCCTGCA CAACCACTACACCCAGAAGTCCCTGAGCCTGAGCCCCG GCGGAGGCGGCGGAAGCGGAGGAGGAGGATCCAGAGA GGGCCCTGAGCTGAGCCCCGATGATCCTGCTGGACTGCT GGACCTGCGGCAGGGCATGTTTGCTCAGCTGGTGGCCCA GAACGTGCTGCTGATCGATGGCCCCCTGTCCTGGTACAG CGATCCTGGACTGGCTGGCGTGTCACTGACAGGCGGCCT GAGCTACAAAGAGGACACCAAAGAACTGGTGGTGGCCA AGGCCGGCGTGTACTACGTGTTCTTTCAGCTGGAACTGC GGAGAGTGGTGGCCGGCGAAGGATCTGGCTCTGTGTCTC TGGCCCTGCATCTGCAGCCTCTGAGAAGCGCTGCTGGCG CTGCAGCTCTGGCACTGACAGTGGATCTGCCTCCTGCCA GCTCCGAGGCCCGGAATAGCGCATTTGGGTTTCAAGGCA GGCTGCTGCACCTGTCTGCCGGCCAGAGGCTGGGAGTG CATCTGCACACAGAGGCCAGGGCTAGACACGCCTGGCA GCTGACACAGGGCGCTACAGTGCTGGGCCTGTTCAGAGT GACCCCCGAGATTCCAGCCGGCCTGCCTTCTCCAAGAAG CGAAGGCGGAGGCGGATCTGGCGGCGGAGGATCTAGAG AGGGACCCGAACTGTCCCCTGACGATCCAGCCGGGCTG CTGGATCTGAGACAGGGAATGTTCGCCCAGCTGGTGGCT CAGAATGTGCTGCTGATTGACGGACCTCTGAGCTGGTAC TCCGACCCAGGGCTGGCAGGGGTGTCCCTGACTGGGGG ACTGTCCTACAAAGAAGATACAAAAGAACTGGTGGTGG CTAAAGCTGGGGTGTACTATGTGTTTTTCAGCTGGAAC TGAGGCGGGTGGTGGCTGGGGAGGGCTCAGGATCTGTG TCCCTGGCTCTGCATCTGCAGCCACTGCGCTCTGCTGCT GGCGCAGCTGCACTGGCTCTGACTGTGGACCTGCCACCA GCCTCTAGCGAGGCCAGAAACAGCGCCTTCGGGTTCCA AGGACGCCTGCTGCATCTGAGCGCCGGACAGCGCCTGG GAGTGCATCTGCATACTGAAGCCAGAGCCCGGCATGCTT GGCAGCTGACTCAGGGGGCAACTGTGCTGGGACTGTTTC GCGTGACACCTGAGATCCCTGCCGGACTGCCAAGCCCTA GATCAGAA |
| 103 | nucleotide sequence of anti-FAP(4B9) light chain | See Table 1 |
| 211 | nucleotide sequence of DP47 (VL-CH1) light chain | see Table 42 |
| 217 | anti-FAP(4B9) Fc hole monomeric 4-1BBL (71-254) chain | EVQLLESGGGLVQPGGSLRLSCAASGFTFSSYAMSWVRQA PGKGLEWVSAIIGSGASTYYADSVKGRFTISRDNSKNTLYL |

(continued)

| SEQ ID NO: | Description | Sequence |
|---|---|---|
| | | QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVCTLPPSRDELTKN QVSLSCAVKGFYPSDIAVEWESNGQPENNYKTTPPVLDSD GSFFLVSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGLPSPRSE |
| 218 | DP47 (VH-CL) Fc knob dimeric 4-1BB ligand (71-254) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRDE LTKNQVSLWCLVKGFYPSDIAVEWESNGQPENNYKTTPPV LDSDGSFFLYSKLTVDKSRWQQGNVFSCSVMHEALHNHY TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGLPS PRSEGGGGSGGGGSREGPELSPDDPAGLLDLRQGMFAQLV AQNVLLIDGPLSWYSDPGLAGVSLTGGLSYKEDTKELVVA KAGVYYVFFQLELRRVVAGEGSGSVSLALHLQPLRSAAGA AALALTVDLPPASSEARNSAFGFQGRLLHLSAGQRLGVHL HTEARARHAWQLTQGATVLGLFRVTPEIPAGLPSPRSE |
| 106 | anti-FAP(4B9) light chain | See Table 1 |
| 214 | DP47 (VL-CH1) light chain | see Table 42 |

**4.2 Preparation of monovalent FAP(4B9)-targeted/DP47 4-1BB ligand trimer-containing Fc (kih) fusion antigen binding molecule, wherein the 4-1BB ligands are C-terminally fused (Constructs 2.17 and 2.18) (FAP on the knob chain)**

The DNA sequence encoding part of the ectodomain (amino acids 71-254) of human 4-1BB ligand was synthetized according to the P41273 sequence of Uniprot database.

A polypeptide containing two ectodomains of 4-1BB ligand, separated by (G4S)2 linkers was subcloned in frame to the C-terminus of human IgG1 Fc hole or knob chain, as depicted in Figure 1A: human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in Figure 1B: human IgG1 Fc, (G4S)2 connector, human 4-1BB ligand.

The variable region of heavy and light chain DNA sequences encoding a binder specific for fibroblast activation protein (FAP), clone 4B9, were subcloned in frame with either the constant heavy chain of the knob or the constant light chain of human IgG1. The variable region of heavy and light chain DNA sequences encoding a non-binding clone, clone DP47, were subcloned in frame with either the constant heavy chain of the hole or the constant light chain of human IgG1. The crossmab technology was applied to reduce the formation of wrongly paired light chains (International patent application No. WO 2010/145792 A1). In this example a crossed Fab unit (VHCL) of the binder DP47 was fused to the knob chain.

The Pro329Gly, Leu234Ala and Leu235Ala mutations have been introduced in the constant region of the knob and hole heavy chains to abrogate binding to Fc gamma receptors (International Patent Appl. Publ. No. WO 2012/130831 A1).

Combination of the DP47 huIgG1 Fc hole chain containing the Y349C/T366S/L368A/Y407V mutations, the anti-FAP huIgG1 Fc knob chain containing the S354C/T366W mutations and the two light chains, anti-FAP and DP47, allows generation of a heterodimer, which includes an assembled trimeric 4-1BB ligand, one FAP binding Fab and one non binding DP47 Fab (Figures 5C and 5D).

Table 46 shows the cDNA and amino acid sequences of the monovalent DP47/FAP(4B9)-targeted 4-1BB ligand (71-254) trimer-containing Fc (kih) fusion antigen binding molecule, wherein the dimeric 4-1BBL is fused to Fc hole chain (Construct 2.17).

**Table 46: Sequences of FAP(4B9)-targeted/DP47 human 4-1BB ligand trimer containing Fc (kih) fusion antigen binding molecule Construct 2.17**

| SEQ ID NO: | Description | Sequence |
|---|---|---|
| 219 | nucleotide sequence of DP47(VH-CL) Fc hole dimeric 4-1BB ligand (71-254) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA |

(continued)

| SEQ ID NO: | Description | Sequence |
|---|---|---|
| | | CACGGCCGTATATTACTGTGCGAAAGGCAGCGGATTTGACTACTGGGGCCAAGGAACCCTGGTCACCGTCTCGAGCGCTAGTGTGGCCGCTCCCTCCGTGTTTATCTTTCCCCCATCCGATGAACAGCTGAAAAGCGGCACCGCCTCCGTCGTGTGTCTGCTGAACAATTTTTACCCTAGGGAAGCTAAAGTGCAGTGGAAAGTGGATAACGCACTGCAGTCCGGCAACTCCCAGGAATCTGTGACAGAACAGGACTCCAAGGACAGCACCTACTCCCTGTCCTCCACCCTGACACTGTCTAAGGCTGATTATGAGAAACACAAAGTCTACGCCTGCGAAGTCACCCATCAGGGCCTGAGCTCGCCCGTCACAAAGAGCTTCAACAGGGGAGAGTGTGACAAAACTCACACATGCCCACCGTGCCCAGCACCTGAAGCTGCAGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTGCACCCTGCCCCCATCCCGGGATGAGCTGACCAAGAACCAGGTCAGCCTCTCGTGCGCAGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTGGAGGCGGCGGAAGCGGAGGAGGAGGATCCAGAGAGGGCCCTGAGCTGAGCCCCGATGATCCTGCTGGACTGCTGGACCTGCGGCAGGGCATGTTTGCTCAGCTGGTGGCCCAGAACGTGCTGCTGATCGATGGCCCCCTGTCCTGGTACAGCGATCCTGGACTGGCTGGCGTGTCACTGACAGGCGGCCTGAGCTACAAAGAGGACACCAAAGAACTGGTGGTGGCCAAGGCCGGCGTGTACTACGTGTTCTTTCAGCTGGAACTGCGGAGAGTGGTGGCCGGCGAAGGATCTGGCTCTGTGTCTCTGGCCCTGCATCTGCAGCCTCTGAGAAGCGCTGCTGGCGCTGCAGCTCTGGCACTGACAGTGGATCTGCCTCCTGCCAGCTCCGAGGCCCGGAATAGCGCATTTGGGTTTCAAGGCAGGCTGCTGCACCTGTCTGCCGGCCAGAGGCTGGGAGTGCATCTGCACACAGAGGCCAGGGCTAGACACGCCTGGCAGCTGACACAGGGCGCTACAGTGCTGGGCCTGTTCAGAGTGACCCCCGAGATTCCAGCCGGCCTGCCTTCTCCAAGAAGCGAAGGCGGAGGCGGATCTGGCGGCGGAGGATCTAGAGAGGGACCCGAACTGTCCCCTGACGATCCAGCCGGGCTGCTGGATCTGAGACAGGGAATGTTCGCCCAGCTGGTGGCTCAGAATGTGCTGCTGATTGACGGACCTCTGAGCTGGTACTCCGACCCAGGGCTGGCAGGGGTGTCCCTGACTGGGGGACTGTCCTACAAAGAAGATACAAAAGAACTGGTGGTGGCTAAAGCTGGGGTGTACTATGTGTTTTTTCAGCTGGAACTGAGGCGGGTGGTGGCTGGGGAGGGCTCAGGATCTGTGTCCCTGGCTCTGCATCTGCAGCCACTGCGCTCTGCTGCTGGCGCAGCTGCACTGGCTCTGACTGTGGACCTGCCACCAGCCTCTAGCGAGGCCAGAAACAGCGCCTTCGGGTTCCAA |

(continued)

| SEQ ID NO: | Description | Sequence |
|---|---|---|
| | | GGACGCCTGCTGCATCTGAGCGCCGGACAGCGCCTGGG AGTGCATCTGCATACTGAAGCCAGAGCCCGGCATGCTTG GCAGCTGACTCAGGGGGCAACTGTGCTGGGACTGTTTCG CGTGACACCTGAGATCCCTGCCGGACTGCCAAGCCCTAG ATCAGAA |

(continued)

| SEQ ID NO: | Description | Sequence |
|---|---|---|
| 220 | nucleotide sequence of FAP (4B9) Fc knob monomeric 4-1BB (71-254) ligand | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCCTCCGTGTTCCCCCT GGCCCCCAGCAGCAAGAGCACCAGCGGCGGCACAGCCG CTCTGGGCTGCCTGGTCAAGGACTACTTCCCCGAGCCCG TGACCGTGTCCTGGAACAGCGGAGCCCTGACCTCCGGC GTGCACACCTTCCCCGCCGTGCTGCAGAGTTCTGGCCTG TATAGCCTGAGCAGCGTGGTCACCGTGCCTTCTAGCAGC CTGGGCACCCAGACCTACATCTGCAACGTGAACCACAA GCCCAGCAACACCAAGGTGGACAAGAAGGTGGAGCCCA AGAGCTGCGACAAGACCCACACCTGTCCCCCTTGTCCTG CCCCTGAAGCTGCTGGCGGCCCTTCTGTGTTCCTGTTCCC CCCAAAGCCCAAGGACACCCTGATGATCAGCCGGACCC CCGAAGTGACCTGCGTGGTGGTGGATGTGTCCCACGAG GACCCTGAAGTGAAGTTCAATTGGTACGTGGACGGCGT GGAAGTGCACAACGCCAAGACAAAGCCGCGGGAGGAG CAGTACAACAGCACGTACCGTGTGGTCAGCGTCCTCACC GTCCTGCACCAGGACTGGCTGAATGGCAAGGAGTACAA GTGCAAGGTCTCCAACAAAGCCCTCGGCGCCCCCATCG AGAAAACCATCTCCAAAGCCAAAGGGCAGCCCCGAGAA CCACAGGTGTACACCCTGCCCCCCTGCAGAGATGAGCTG ACCAAGAACCAGGTGTCCCTGTGGTGTCTGGTCAAGGGC TTCTACCCCAGCGATATCGCCGTGGAGTGGGAGAGCAA CGGCCAGCCTGAGAACAACTACAAGACCACCCCCCCTG TGCTGGACAGCGACGGCAGCTTCTTCCTGTACTCCAAAC TGACCGTGGACAAGAGCCGGTGGCAGCAGGGCAACGTG TTCAGCTGCAGCGTGATGCACGAGGCCCTGCACAACCA CTACACCCAGAAGTCCCTGAGCCTGAGCCCCGGCGGAG GCGGCGGAAGCGGAGGAGGAGGATCCAGAGAGGGCCC TGAGCTGAGCCCCGATGATCCTGCTGGACTGCTGGACCT GCGGCAGGGCATGTTTGCTCAGCTGGTGGCCCAGAACGT GCTGCTGATCGATGGCCCCCTGTCCTGGTACAGCGATCC TGGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGAAGCGCTGCTGGCGCTGCAG CTCTGGCACTGACAGTGGATCTGCCTCCTGCCAGCTCCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC |

(continued)

| SEQ ID NO: | Description | Sequence |
|---|---|---|
| | | CCGAGATTCCAGCCGGCCTGCCTTCTCCAAGAAGCGAA |
| 103 | nucleotide sequence of anti-FAP(4B9) light chain | see Table 1 |
| 211 | nucleotide sequence of DP47 (VL-CH1) light chain | see Table 42 |
| 221 | DP47(VH-CL) Fc hole dimeric 4-1BB ligand (71-254) chain | EVQLLESGGGLVQPGGSLRLSCAASGFTFSSYAMSWVRQA<br>PGKGLEWVSAISGSGGSTYYADSVKGRFTISRDNSKNTLYL<br>QMNSLRAEDTAVYYCAKGSGFDYWGQGTLVTVSSASVA<br>APSVFIFPPSDEQLKSGTASVVCLLNNFYPREAKVQWKVD<br>NALQSGNSQESVTEQDSKDSTYSLSSTLTLSKADYEKHKV<br>YACEVTHQGLSSPVTKSFNRGECDKTHTCPPCPAPEAAGG<br>PSVFLFPPKPKDTLMISRTPEVTCVVVDVSHEDPEVKFNWY<br>VDGVEVHNAKTKPREEQYNSTYRVVSVLTVLHQDWLNG<br>KEYKCKVSNKALGAPIEKTISKAKGQPREPQVCTLPPSRDE<br>LTKNQVSLSCAVKGFYPSDIAVEWESNGQPENNYKTTPPV<br>LDSDGSFFLVSKLTVDKSRWQQGNVFSCSVMHEALHNHY<br>TQKSLSLSPGGGGGSGGGGSREGPELSPDDPAGLLDLRQG<br>MFAQLVAQNVLLIDGPLSWYSDPGLAGVSLTGGLSYKEDT<br>KELVVAKAGVYYVFFQLELRRVVAGEGSGSVSLALHLQPL<br>RSAAGAAALALTVDLPPASSEARNSAFGFQGRLLHLSAGQ<br>RLGVHLHTEARARHAWQLTQGATVLGLFRVTPEIPAGLPS<br>PRSEGGGGSGGGGSREGPELSPDDPAGLLDLRQGMFAQLV<br>AQNVLLIDGPLSWYSDPGLAGVSLTGGLSYKEDTKELVVA<br>KAGVYYVFFQLELRRVVAGEGSGSVSLALHLQPLRSAAGA<br>AALALTVDLPPASSEARNSAFGFQGRLLHLSAGQRLGVHL<br>HTEARARHAWQLTQGATVLGLFRVTPEIPAGLPSPRSE |
| 222 | FAP (4B9) Fc knob monomeric 4-1BB (71-254) ligand | EVQLLESGGGLVQPGGSLRLSCAASGFTFSSYAMSWVRQA<br>PGKGLEWVSAIIGSGASTYYADSVKGRFTISRDNSKNTLYL<br>QMNSLRAEDTAVYYCAKGWFGGFNYWGQGTLVTVSSAS<br>TKGPSVFPLAPSSKSTSGGTAALGCLVKDYFPEPVTVSWNS<br>GALTSGVHTFPAVLQSSGLYSLSSVVTVPSSSLGTQTYICN<br>VNHKPSNTKVDKKVEPKSCDKTHTCPPCPAPEAAGGPSVF<br>LFPPKPKDTLMISRTPEVTCVVVDVSHEDPEVKFNWYVDG<br>VEVHNAKTKPREEQYNSTYRVVSVLTVLHQDWLNGKEYK<br>CKVSNKALGAPIEKTISKAKGQPREPQVYTLPPCRDELTKN<br>QVSLWCLVKGFYPSDIAVEWESNGQPENNYKTTPPVLDSD<br>GSFFLYSKLTVDKSRWQQGNVFSCSVMHEALHNHYTQKS<br>LSLSPGGGGGSGGGGSREGPELSPDDPAGLLDLRQGMFAQ<br>LVAQNVLLIDGPLSWYSDPGLAGVSLTGGLSYKEDTKELV<br>VAKAGVYYVFFQLELRRVVAGEGSGSVSLALHLQPLRSAA<br>GAAALALTVDLPPASSEARNSAFGFQGRLLHLSAGQRLGV<br>HLHTEARARHAWQLTQGATVLGLFRVTPEIPAGLPSPRSE |
| 106 | anti-FAP(4B9) light chain | see Table 1 |
| 214 | DP47 (VL-CH1) light chain | see Table 42 |

Table 47 shows the cDNA and amino acid sequences of the monovalent DP47/FAP(4B9)-targeted 4-1BB ligand (71-254) trimer-containing Fc (kih) fusion antigen binding molecule, wherein the dimeric 4-1BBL is fused to the knob chain (Construct 2.18).

**Table 47: Sequences of FAP(4B9)-targeted human 4-1BB ligand trimer containing Fc (kih) fusion molecule Construct 2.18 (comparative example)**

| SEQ ID NO: | Description | Sequence |
|---|---|---|
| 223 | nucleotide sequence of DP47 (VH-CL) Fc hole monomeric 4-1BBL (71-254) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAAACTCACACATGCCCACCGTG CCCAGCACCTGAAGCTGCAGGGGGACCGTCAGTCTTCCT CTTCCCCCCAAAACCCAAGGACACCCTCATGATCTCCCG GACCCCTGAGGTCACATGCGTGGTGGTGGACGTGAGCC ACGAAGACCCTGAGGTCAAGTTCAACTGGTACGTGGAC GGCGTGGAGGTGCATAATGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTGCACCCTGCCCCCATCCCGGGAT GAGCTGACCAAGAACCAGGTCAGCCTCTCGTGCGCAGT CAAAGGCTTCTATCCCAGCGACATCGCCGTGGAGTGGG AGAGCAATGGGCAGCCGGAGAACAACTACAAGACCACG CCTCCCGTGCTGGACTCCGACGGCTCCTTCTTCCTCGTG AGCAAGCTCACCGTGGACAAGAGCAGGTGGCAGCAGGG |

(continued)

| SEQ ID NO: | Description | Sequence |
|---|---|---|
| | | GAACGTCTTCTCATGCTCCGTGATGCATGAGGCTCTGCA<br>CAACCACTACACGCAGAAGAGCCTCTCCCTGTCTCCGGG<br>TGGAGGCGGCGGAAGCGGAGGAGGAGGATCCAGAGAG<br>GGCCCTGAGCTGAGCCCCGATGATCCTGCTGGACTGCTG<br>GACCTGCGGCAGGGCATGTTTGCTCAGCTGGTGGCCCAG<br>AACGTGCTGCTGATCGATGGCCCCCTGTCCTGGTACAGC<br>GATCCTGGACTGGCTGGCGTGTCACTGACAGGCGGCCTG<br>AGCTACAAAGAGGACACCAAAGAACTGGTGGTGGCCAA<br>GGCCGGCGTGTACTACGTGTTCTTTCAGCTGGAACTGCG<br>GAGAGTGGTGGCCGGCGAAGGATCTGGCTCTGTGTCTCT<br>GGCCCTGCATCTGCAGCCTCTGAGAAGCGCTGCTGGCGC<br>TGCAGCTCTGGCACTGACAGTGGATCTGCCTCCTGCCAG<br>CTCCGAGGCCCGGAATAGCGCATTTGGGTTTCAAGGCAG<br>GCTGCTGCACCTGTCTGCCGGCCAGAGGCTGGGAGTGC<br>ATCTGCACACAGAGGCCAGGGCTAGACACGCCTGGCAG<br>CTGACACAGGGCGCTACAGTGCTGGGCCTGTTCAGAGTG<br>ACCCCCGAGATTCCAGCCGGCCTGCCTTCTCCAAGAAGC<br>GAA |

(continued)

| SEQ ID NO: | Description | Sequence |
|---|---|---|
| 224 | nucleotide sequence of FAP (4B9) Fc knob dimeric 4-1BB ligand (71-254) chain | GAGGTGCAGCTGCTCGAAAGCGGCGGAGGACTGGTGCA GCCTGGCGGCAGCCTGAGACTGTCTTGCGCCGCCAGCG GCTTCACCTTCAGCAGCTACGCCATGAGCTGGGTCCGCC AGGCCCCTGGCAAGGGACTGGAATGGGTGTCCGCCATC ATCGGCTCTGGCGCCAGCACCTACTACGCCGACAGCGTG AAGGGCCGGTTCACCATCAGCCGGGACAACAGCAAGAA CACCCTGTACCTGCAGATGAACAGCCTGCGGGCCGAGG ACACCGCCGTGTACTACTGCGCCAAGGGATGGTTCGGC GGCTTCAACTACTGGGGACAGGGCACCCTGGTCACAGT GTCCAGCGCTAGCACCAAGGGCCCCTCCGTGTTCCCCCT GGCCCCCAGCAGCAAGAGCACCAGCGGCGGCACAGCCG CTCTGGGCTGCCTGGTCAAGGACTACTTCCCCGAGCCCG TGACCGTGTCCTGGAACAGCGGAGCCCTGACCTCCGGC GTGCACACCTTCCCCGCCGTGCTGCAGAGTTCTGGCCTG TATAGCCTGAGCAGCGTGGTCACCGTGCCTTCTAGCAGC CTGGGCACCCAGACCTACATCTGCAACGTGAACCACAA GCCCAGCAACACCAAGGTGGACAAGAAGGTGGAGCCCA AGAGCTGCGACAAGACCCACACCTGTCCCCCTTGTCCTG CCCCTGAAGCTGCTGGCGGCCCTTCTGTGTTCCTGTTCCC CCCAAAGCCCAAGGACACCCTGATGATCAGCCGGACCC CCGAAGTGACCTGCGTGGTGGTGGATGTGTCCCACGAG GACCCTGAAGTGAAGTTCAATTGGTACGTGGACGGCGT GGAAGTGCACAACGCCAAGACAAAGCCGCGGGAGGAG CAGTACAACAGCACGTACCGTGTGGTCAGCGTCCTCACC GTCCTGCACCAGGACTGGCTGAATGGCAAGGAGTACAA GTGCAAGGTCTCCAACAAAGCCCTCGGCGCCCCCATCG AGAAAACCATCTCCAAAGCCAAAGGGCAGCCCCGAGAA CCACAGGTGTACACCCTGCCCCCCTGCAGAGATGAGCTG ACCAAGAACCAGGTGTCCCTGTGGTGTCTGGTCAAGGGC TTCTACCCCAGCGATATCGCCGTGGAGTGGGAGAGCAA CGGCCAGCCTGAGAACAACTACAAGACCACCCCCCCTG TGCTGGACAGCGACGGCAGCTTCTTCCTGTACTCCAAAC TGACCGTGGACAAGAGCCGGTGGCAGCAGGGCAACGTG TTCAGCTGCAGCGTGATGCACGAGGCCCTGCACAACCA CTACACCCAGAAGTCCCTGAGCCTGAGCCCCGGCGGAG GCGGCGGAAGCGGAGGAGGAGGATCCAGAGAGGGCCC |

(continued)

| SEQ ID NO: | Description | Sequence |
|---|---|---|
| | | TGAGCTGAGCCCCGATGATCCTGCTGGACTGCTGGACCT GCGGCAGGGCATGTTTGCTCAGCTGGTGGCCCAGAACGT GCTGCTGATCGATGGCCCCCTGTCCTGGTACAGCGATCC TGGACTGGCTGGCGTGTCACTGACAGGCGGCCTGAGCTA CAAAGAGGACACCAAAGAACTGGTGGTGGCCAAGGCCG GCGTGTACTACGTGTTCTTTCAGCTGGAACTGCGGAGAG TGGTGGCCGGCGAAGGATCTGGCTCTGTGTCTCTGGCCC TGCATCTGCAGCCTCTGAGAAGCGCTGCTGGCGCTGCAG CTCTGGCACTGACAGTGGATCTGCCTCCTGCCAGCTCCG AGGCCCGGAATAGCGCATTTGGGTTTCAAGGCAGGCTG CTGCACCTGTCTGCCGGCCAGAGGCTGGGAGTGCATCTG CACACAGAGGCCAGGGCTAGACACGCCTGGCAGCTGAC ACAGGGCGCTACAGTGCTGGGCCTGTTCAGAGTGACCC CCGAGATTCCAGCCGGCCTGCCTTCTCCAAGAAGCGAA GGCGGAGGCGGATCTGGCGGCGGAGGATCTAGAGAGGG ACCCGAACTGTCCCCTGACGATCCAGCCGGGCTGCTGGA TCTGAGACAGGGAATGTTCGCCCAGCTGGTGGCTCAGA ATGTGCTGCTGATTGACGGACCTCTGAGCTGGTACTCCG ACCCAGGGCTGGCAGGGGTGTCCCTGACTGGGGGACTG TCCTACAAAGAAGATACAAAAGAACTGGTGGTGGCTAA AGCTGGGGTGTACTATGTGTTTTTCAGCTGGAACTGAG GCGGGTGGTGGCTGGGGAGGGCTCAGGATCTGTGTCCCT GGCTCTGCATCTGCAGCCACTGCGCTCTGCTGCTGGCGC AGCTGCACTGGCTCTGACTGTGGACCTGCCACCAGCCTC TAGCGAGGCCAGAAACAGCGCCTTCGGGTTCCAAGGAC GCCTGCTGCATCTGAGCGCCGGACAGCGCCTGGGAGTG CATCTGCATACTGAAGCCAGAGCCCGGCATGCTTGGCA GCTGACTCAGGGGGCAACTGTGCTGGGACTGTTTCGCGT GACACCTGAGATCCCTGCCGGACTGCCAAGCCCTAGATC AGAA |
| 103 | nucleotide sequence of anti-FAP(4B9) light chain | See Table 1 |
| 211 | nucleotide sequence of DP47 (VL-CH1) light chain | see Table 42 |
| 225 | DP47 (VH-CL) Fc hole monomeric 4-1BBL (71-254) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRDE LTKNQVSLSCAVKGFYPSDIAVEWESNGQPENNYKTTPPV LDSDGSFFLVSKLTVDKSRWQQGNVFSCSVMHEALHNHY |

(continued)

| SEQ ID NO: | Description | Sequence |
|---|---|---|
| | | TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGLPS PRSE |
| 226 | anti-FAP(4B9) Fc knob dimeric 4-1BB ligand (71-254) chain | EVQLLESGGGLVQPGGSLRLSCAASGFTFSSYAMSWVRQA PGKGLEWVSAIIGSGASTYYADSVKGRFTISRDNSKNTLYL QMNSLRAEDTAVYYCAKGWFGGFNYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICN VNHKPSNTKVDKKVEPKSCDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALGAPIEKTISKAKGQPREPQVYTLPPCRDELTKN QVSLWCLVKGFYPSDIAVEWESNGQPENNYKTTPPVLDSD GSFFLYSKLTVDKSRWQQGNVFSCSVMHEALHNHYTQKS LSLSPGGGGGSGGGGSREGPELSPDDPAGLLDLRQGMFAQ LVAQNVLLIDGPLSWYSDPGLAGVSLTGGLSYKEDTKELV VAKAGVYYVFFQLELRRVVAGEGSGSVSLALHLQPLRSAA GAAALALTVDLPPASSEARNSAFGFQGRLLHLSAGQRLGV HLHTEARARHAWQLTQGATVLGLFRVTPEIPAGLPSPRSEG GGGSGGGGSREGPELSPDDPAGLLDLRQGMFAQLVAQNV LLIDGPLSWYSDPGLAGVSLTGGLSYKEDTKELVVAKAGV YYVFFQLELRRVVAGEGSGSVSLALHLQPLRSAAGAAALA LTVDLPPASSEARNSAFGFQGRLLHLSAGQRLGVHLHTEA RARHAWQLTQGATVLGLFRVTPEIPAGLPSPRSE |
| 106 | anti-FAP(4B9) light chain | See Table 1 |
| 214 | DP47 (VL-CH1) light chain | see Table 42 |

**4.3 Preparation of monovalent FAP(4B9)-targeted/DP47 4-1BB ligand trimer-containing Fc (kih) fusion antigen binding molecule, wherein the 4-1BB ligands are C-terminally fused (Constructs 2.19 and 2.20) (FAP on the hole chain)**

The DNA sequence encoding part of the ectodomain (amino acids 71-248) of human 4-1BB ligand was synthetized according to the P41273 sequence of Uniprot database. Constructs 2.19 aand 2.20 were prepared as described in Example 4.1 for Constructs 2.15 and 2.16.

Table 48 shows the cDNA and amino acid sequences of the monovalent FAP(4B9)-targeted/DP47 4-1BB ligand (71-248) trimer-containing Fc (kih) fusion antigen binding molecule, wherein the dimeric 4-1BBL is fused to the hole chain (Construct 2.19).

**Table 48: Sequences of FAP(4B9)-targeted/DP47 human 4-1BB ligand trimer containing Fc (kih) fusion antigen binding molecule Construct 2.19 (comparative example)**

| SEQ ID NO: | Description | Sequence |
|---|---|---|
| 171 | nucleotide sequence of anti-FAP(4B9) Fc hole dimeric 4-1BB ligand (71-248) chain | see Table 23 |
| 227 | nucleotide sequence of DP47 (VHCL) Fc knob monomeric 4-1BB (71-248) ligand | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAGACCCACACCTGTCCCCCTTG TCCTGCCCCTGAAGCTGCTGGCGGCCCTTCTGTGTTCCT GTTCCCCCCAAAGCCCAAGGACACCCTGATGATCAGCC GGACCCCCGAAGTGACCTGCGTGGTGGTGGATGTGTCCC ACGAGGACCCTGAAGTGAAGTTCAATTGGTACGTGGAC GGCGTGGAAGTGCACAACGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTACACCCTGCCCCCCTGCAGAGAT GAGCTGACCAAGAACCAGGTGTCCCTGTGGTGTCTGGTC AAGGGCTTCTACCCCAGCGATATCGCCGTGGAGTGGGA GAGCAACGGCCAGCCTGAGAACAACTACAAGACCACCC CCCCTGTGCTGGACAGCGACGGCAGCTTCTTCCTGTACT CCAAACTGACCGTGGACAAGAGCCGGTGGCAGCAGGGC AACGTGTTCAGCTGCAGCGTGATGCACGAGGCCCTGCA CAACCACTACACCCAGAAGTCCCTGAGCCTGAGCCCCG GCGGAGGCGGCGGAAGCGGAGGAGGAGGATCCAGAGA GGGCCCTGAGCTGAGCCCTGATGATCCTGCCGGACTGCT GGACCTGCGGCAGGGAATGTTTGCCCAGCTGGTGGCCC |

(continued)

| SEQ ID NO: | Description | Sequence |
|---|---|---|
| | | AGAACGTGCTGCTGATCGATGGCCCCCTGTCCTGGTACAGCGATCCTGGACTGGCTGGCGTGTCACTGACAGGCGGCCTGAGCTACAAAGAGGACACCAAAGAACTGGTGGTGGCCAAGGCCGGCGTGTACTACGTGTTCTTTCAGCTGGAACTGCGGAGAGTGGTGGCCGGCGAAGGATCTGGCTCTGTGTCTCTGGCCCTGCATCTGCAGCCTCTGAGATCTGCTGCTGGCGCCGCTGCTCTGGCACTGACAGTGGATCTGCCTCCTGCCAGCAGCGAGGCCCGGAATAGCGCATTTGGGTTTCAAGGCAGGCTGCTGCACCTGTCTGCCGGCCAGAGGCTGGGAGTGCATCTGCACACAGAGGCCAGGGCTAGACACGCCTGGCAGCTGACACAGGGCGCTACAGTGCTGGGCCTGTTCAGAGTGACCCCCGAGATTCCTGCCGGGCTC |
| 103 | nucleotide sequence of anti-FAP(4B9) light chain | see Table 1 |
| 211 | nucleotide sequence of DP47 (VL-CH1) light chain | see Table 42 |
| 173 | anti-FAP(4B9) Fc hole dimeric 4-1BB ligand (71-248) chain | see Table 23 |
| 228 | DP47 (VH-CL) Fc knob monomeric 4-1BB (71-248) ligand | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCAKGSGFDYWGQGTLVTVSSASVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGGGGGSGGGGSREGPELSPDDPAGLLDLRQGMFAQLVAQNVLLIDGPLSWYSDPGLAGVSLTGGLSYKEDTKELVVAKAGVYYVFFQLELRRVVAGEGSGSVSLALHLQPLRSAAGAAALALTVDLPPASSEARNSAFGFQGRLLHLSAGQRLGVHLHTEARARHAWQLTQGATVLGLFRVTPEIPAGL |
| 106 | anti-FAP(4B9) light chain | see Table 1 |
| 214 | DP47 (VL-CH1) light chain | see Table 42 |

Table 49 shows the cDNA and amino acid sequences of the monovalent FAP(4B9)-targeted/DP47 4-1BB ligand (71-248) trimer-containing Fc (kih) fusion antigen binding molecule, wherein the dimeric 4-1BBL is fused to the Fc knob chain (Construct 2.20).

**Table 49: Sequences of FAP(4B9)-targeted human 4-1BB ligand trimer containing Fc (kih) fusion molecule Construct 2.20 (comparative example)**

| SEQ ID NO: | Description | Sequence |
|---|---|---|
| 175 | nucleotide sequence of anti-FAP(4B9) Fc hole monomeric 4-1BBL (71-248) chain | see Table 24 |
| 229 | nucleotide sequence of DP47 (VH-CL) Fc knob dimeric 4-1BB ligand (71-248) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAGACCCACACCTGTCCCCCTTG TCCTGCCCCTGAAGCTGCTGGCGGCCCTTCTGTGTTCCT GTTCCCCCCAAAGCCCAAGGACACCCTGATGATCAGCC GGACCCCCGAAGTGACCTGCGTGGTGGTGGATGTGTCCC ACGAGGACCCTGAAGTGAAGTTCAATTGGTACGTGGAC GGCGTGGAAGTGCACAACGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTACACCCTGCCCCCCTGCAGAGAT |

(continued)

| | | |
|---|---|---|
| | | GAGCTGACCAAGAACCAGGTGTCCCTGTGGTGTCTGGTC AAGGGCTTCTACCCCAGCGATATCGCCGTGGAGTGGGA GAGCAACGGCCAGCCTGAGAACAACTACAAGACCACCC CCCCTGTGCTGGACAGCGACGGCAGCTTCTTCCTGTACT CCAAACTGACCGTGGACAAGAGCCGGTGGCAGCAGGGC AACGTGTTCAGCTGCAGCGTGATGCACGAGGCCCTGCA CAACCACTACACCCAGAAGTCCCTGAGCCTGAGCCCCG GCGGAGGCGGCGGAAGCGGAGGAGGAGGATCCAGAGA GGGCCCTGAGCTGAGCCCTGATGATCCTGCCGGACTGCT GGACCTGCGGCAGGGAATGTTTGCCCAGCTGGTGGCCC AGAACGTGCTGCTGATCGATGGCCCCCTGTCCTGGTACA GCGATCCTGGACTGGCTGGCGTGTCACTGACAGGCGGC CTGAGCTACAAAGAGGACACCAAAGAACTGGTGGTGGC CAAGGCCGGCGTGTACTACGTGTTCTTTCAGCTGGAACT GCGGAGAGTGGTGGCCGGCGAAGGATCTGGCTCTGTGT CTCTGGCCCTGCATCTGCAGCCTCTGAGATCTGCTGCTG GCGCCGCTGCTCTGGCACTGACAGTGGATCTGCCTCCTG CCAGCAGCGAGGCCCGGAATAGCGCATTTGGGTTTCAA GGCAGGCTGCTGCACCTGTCTGCCGGCCAGAGGCTGGG AGTGCATCTGCACACAGAGGCCAGGGCTAGACACGCCT GGCAGCTGACACAGGGCGCTACAGTGCTGGGCCTGTTC AGAGTGACCCCCGAGATTCCAGCAGGCCTGGGAGGCGG CGGATCTGGCGGCGGAGGATCTAGAGAAGGACCCGAGC TGTCCCCCGACGATCCCGCTGGGCTGCTGGATCTGAGAC AGGGCATGTTCGCTCAGCTGGTGGCTCAGAATGTGCTGC TGATTGACGGACCTCTGAGCTGGTACTCCGACCCAGGGC TGGCAGGGGTGTCCCTGACTGGGGGACTGTCCTACAAA GAAGATACAAAAGAACTGGTGGTGGCTAAAGCTGGGGT GTACTATGTGTTTTTTCAGCTGGAACTGAGGCGGGTGGT GGCTGGGGAGGGCTCAGGATCTGTGTCCCTGGCTCTGCA TCTGCAGCCACTGCGCTCTGCAGCAGGGGCTGCAGCACT GGCCCTGACTGTGGACCTGCCCCCAGCTTCTTCCGAGGC CAGAAACAGCGCCTTCGGGTTCCAAGGACGCCTGCTGC ATCTGAGCGCCGGACAGCGCCTGGGAGTGCATCTGCAT ACTGAAGCCAGAGCCCGGCATGCTTGGCAGCTGACTCA GGGGGCAACTGTGCTGGGACTGTTTCGCGTGACACCTGA GATCCCAGCCGGGCTC |
| 103 | nucleotide sequence of anti-FAP(4B9) light chain | See Table 1 |
| 211 | nucleotide sequence of DP47 (VL-CH1) light chain | see Table 42 |
| 177 | anti-FAP(4B9) Fc hole monomeric 4-1BBL (71-248) | see Table 24 |
| | chain | |

(continued)

| | | |
|---|---|---|
| 230 | DP47 (VH-CL) Fc knob dimeric 4-1BB ligand (71-248) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVYTLPPCRDE LTKNQVSLWCLVKGFYPSDIAVEWESNGQPENNYKTTPPV LDSDGSFFLYSKLTVDKSRWQQGNVFSCSVMHEALHNHY TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGLGG GGSGGGGSREGPELSPDDPAGLLDLRQGMFAQLVAQNVL LIDGPLSWYSDPGLAGVSLTGGLSYKEDTKELVVAKAGVY YVFFQLELRRVVAGEGSGSVSLALHLQPLRSAAGAAALAL TVDLPPASSEARNSAFGFQGRLLHLSAGQRLGVHLHTEAR ARHAWQLTQGATVLGLFRVTPEIPAGL |
| 106 | anti-FAP(4B9) light chain | See Table 1 |
| 214 | DP47 (VL-CH1) light chain | see Table 42 |

**4.4 Preparation of monovalent DP47/FAP(4B9)-targeted 4-1BB ligand trimer-containing Fc (kih) fusion antigen binding molecules, wherein the 4-1BB ligands are C-terminally fused (Constructs 2.21 and 2.22) (FAP on the knob chain)**

The DNA sequence encoding part of the ectodomain (amino acids 71-248) of human 4-1BB ligand was synthetized according to the P41273 sequence of Uniprot database. Constructs 2.21 aand 2.22 were prepared as described in Example 4.2 for Constructs 2.17 and 2.18.

Table 50 shows the cDNA and amino acid sequences of the monovalent DP47/FAP(4B9)-targeted 4-1BB ligand (71-254) trimer-containing Fc (kih) fusion antigen binding molecule, wherein the dimeric 4-1BBL is fused to Fc hole chain (Construct 2.21).

**Table 50: Sequences of FAP(4B9)-targeted/DP47 human 4-1BB ligand trimer containing Fc (kih) fusion antigen binding molecule Construct 2.21**

| SEQ ID NO: | Description | Sequence |
|---|---|---|
| 231 | nucleotide sequence of DP47(VH-CL) Fc hole dimeric 4-1BB ligand (71-248) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAAACTCACACATGCCCACCGTG CCCAGCACCTGAAGCTGCAGGGGGACCGTCAGTCTTCCT CTTCCCCCCAAAACCCAAGGACACCCTCATGATCTCCCG GACCCCTGAGGTCACATGCGTGGTGGTGGACGTGAGCC ACGAAGACCCTGAGGTCAAGTTCAACTGGTACGTGGAC GGCGTGGAGGTGCATAATGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTGCACCCTGCCCCCATCCCGGGAT GAGCTGACCAAGAACCAGGTCAGCCTCTCGTGCGCAGT CAAAGGCTTCTATCCCAGCGACATCGCCGTGGAGTGGG AGAGCAATGGGCAGCCGGAGAACAACTACAAGACCACG CCTCCCGTGCTGGACTCCGACGGCTCCTTCTTCCTCGTG AGCAAGCTCACCGTGGACAAGAGCAGGTGGCAGCAGGG GAACGTCTTCTCATGCTCCGTGATGCATGAGGCTCTGCA CAACCACTACACGCAGAAGAGCCTCTCCCTGTCTCCGGG TGGAGGCGGCGGAAGCGGAGGAGGAGGATCCAGAGAG GGCCCTGAGCTGAGCCCTGATGATCCTGCCGGACTGCTG GACCTGCGGCAGGGAATGTTTGCCCAGCTGGTGGCCCA GAACGTGCTGCTGATCGATGGCCCCCTGTCCTGGTACAG CGATCCTGGACTGGCTGGCGTGTCACTGACAGGCGGCCT GAGCTACAAAGAGGACACCAAAGAACTGGTGGTGGCCA AGGCCGGCGTGTACTACGTGTTCTTTCAGCTGGAACTGC GGAGAGTGGTGGCCGGCGAAGGATCTGGCTCTGTGTCTC TGGCCCTGCATCTGCAGCCTCTGAGATCTGCTGCTGGCG CCGCTGCTCTGGCACTGACAGTGGATCTGCCTCCTGCCA GCAGCGAGGCCCGGAATAGCGCATTTGGGTTTCAAGGC AGGCTGCTGCACCTGTCTGCCGGCCAGAGGCTGGGAGT |

(continued)

| SEQ ID NO: | Description | Sequence |
|---|---|---|
| | | GCATCTGCACACAGAGGCCAGGGCTAGACACGCCTGGC AGCTGACACAGGGCGCTACAGTGCTGGGCCTGTTCAGA GTGACCCCCGAGATTCCTGCCGGGCTC |
| 102 | nucleotide sequence of FAP (4B9) Fc knob monomeric 4-1BB (71-248) ligand | see Table 1 |
| 103 | nucleotide sequence of anti-FAP(4B9) light chain | see Table 1 |
| 211 | nucleotide sequence of DP47 (VL-CH1) light chain | see Table 42 |
| 232 | DP47(VH-CL) Fc hole dimeric 4-1BB ligand (71-248) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRDE LTKNQVSLSCAVKGFYPSDIAVEWESNGQPENNYKTTPPV LDSDGSFFLVSKLTVDKSRWQQGNVFSCSVMHEALHNHY TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGL |
| 105 | FAP (4B9) Fc knob monomeric 4-1BB (71-248) ligand | see Table 1 |
| 106 | anti-FAP(4B9) light chain | see Table 1 |
| 214 | DP47 (VL-CH1) light chain | see Table 42 |

Table 51 shows the cDNA and amino acid sequences of the monovalent DP47/FAP(4B9)-targeted 4-1BB ligand (71-254) trimer-containing Fc (kih) fusion antigen binding molecule, wherein the dimeric 4-1BBL is fused to the knob chain (Construct 2.22).

**Table 51: Sequences of FAP(4B9)-targeted human 4-1BB ligand trimer containing Fc (kih) fusion molecule Construct 2.22 (comparative example)**

| SEQ ID NO: | Description | Sequence |
|---|---|---|
| 233 | nucleotide sequence of DP47 (VH-CL) Fc hole monomeric 4-1BBL (71-248) chain | GAGGTGCAATTGTTGGAGTCTGGGGGAGGCTTGGTACA GCCTGGGGGGTCCCTGAGACTCTCCTGTGCAGCCTCCGG ATTCACCTTTAGCAGTTATGCCATGAGCTGGGTCCGCCA GGCTCCAGGGAAGGGGCTGGAGTGGGTCTCAGCTATTA GTGGTAGTGGTGGTAGCACATACTACGCAGACTCCGTGA AGGGCCGGTTCACCATCTCCAGAGACAATTCCAAGAAC ACGCTGTATCTGCAGATGAACAGCCTGAGAGCCGAGGA CACGGCCGTATATTACTGTGCGAAAGGCAGCGGATTTGA CTACTGGGGCCAAGGAACCCTGGTCACCGTCTCGAGCG CTAGTGTGGCCGCTCCCTCCGTGTTTATCTTTCCCCCATC CGATGAACAGCTGAAAAGCGGCACCGCCTCCGTCGTGT GTCTGCTGAACAATTTTTACCCTAGGGAAGCTAAAGTGC AGTGGAAAGTGGATAACGCACTGCAGTCCGGCAACTCC CAGGAATCTGTGACAGAACAGGACTCCAAGGACAGCAC CTACTCCCTGTCCTCCACCCTGACACTGTCTAAGGCTGA TTATGAGAAACACAAAGTCTACGCCTGCGAAGTCACCC ATCAGGGCCTGAGCTCGCCCGTCACAAAGAGCTTCAAC AGGGGAGAGTGTGACAAAACTCACACATGCCCACCGTG CCCAGCACCTGAAGCTGCAGGGGGACCGTCAGTCTTCCT CTTCCCCCCAAAACCCAAGGACACCCTCATGATCTCCCG GACCCCTGAGGTCACATGCGTGGTGGTGGACGTGAGCC ACGAAGACCCTGAGGTCAAGTTCAACTGGTACGTGGAC GGCGTGGAGGTGCATAATGCCAAGACAAAGCCGCGGGA GGAGCAGTACAACAGCACGTACCGTGTGGTCAGCGTCC TCACCGTCCTGCACCAGGACTGGCTGAATGGCAAGGAG TACAAGTGCAAGGTCTCCAACAAAGCCCTCGGCGCCCC CATCGAGAAAACCATCTCCAAAGCCAAAGGGCAGCCCC GAGAACCACAGGTGTGCACCCTGCCCCCATCCCGGGAT GAGCTGACCAAGAACCAGGTCAGCCTCTCGTGCGCAGT CAAAGGCTTCTATCCCAGCGACATCGCCGTGGAGTGGG AGAGCAATGGGCAGCCGGAGAACAACTACAAGACCACG CCTCCCGTGCTGGACTCCGACGGCTCCTTCTTCCTCGTG AGCAAGCTCACCGTGGACAAGAGCAGGTGGCAGCAGGG GAACGTCTTCTCATGCTCCGTGATGCATGAGGCTCTGCA CAACCACTACACGCAGAAGAGCCTCTCCCTGTCTCCGGG TGGAGGCGGCGGAAGCGGAGGAGGAGGATCCAGAGAG |

(continued)

| SEQ ID NO: | Description | Sequence |
|---|---|---|
| | | GGCCCTGAGCTGAGCCCTGATGATCCTGCCGGACTGCTG GACCTGCGGCAGGGAATGTTTGCCCAGCTGGTGGCCCA GAACGTGCTGCTGATCGATGGCCCCCTGTCCTGGTACAG CGATCCTGGACTGGCTGGCGTGTCACTGACAGGCGGCCT GAGCTACAAAGAGGACACCAAAGAACTGGTGGTGGCCA AGGCCGGCGTGTACTACGTGTTCTTTCAGCTGGAACTGC GGAGAGTGGTGGCCGGCGAAGGATCTGGCTCTGTGTCTC TGGCCCTGCATCTGCAGCCTCTGAGATCTGCTGCTGGCG CCGCTGCTCTGGCACTGACAGTGGATCTGCCTCCTGCCA GCAGCGAGGCCCGGAATAGCGCATTTGGGTTTCAAGGC AGGCTGCTGCACCTGTCTGCCGGCCAGAGGCTGGGAGT GCATCTGCACACAGAGGCCAGGGCTAGACACGCCTGGC AGCTGACACAGGGCGCTACAGTGCTGGGCCTGTTCAGA GTGACCCCCGAGATTCCTGCCGGGCTC |
| 108 | nucleotide sequence of FAP (4B9) Fc knob dimeric 4-1BB ligand (71-248) chain | see Table 2 |
| 103 | nucleotide sequence of anti-FAP(4B9) light chain | See Table 1 |
| 211 | nucleotide sequence of DP47 (VL-CH1) light chain | see Table 42 |
| 234 | DP47 (VH-CL) Fc hole monomeric 4-1BBL (71-248) chain | EVQLLESGGGLVQPGGSLRLSCAASGFTFSSYAMSWVRQA PGKGLEWVSAISGSGGSTYYADSVKGRFTISRDNSKNTLYL QMNSLRAEDTAVYYCAKGSGFDYWGQGTLVTVSSASVA APSVFIFPPSDEQLKSGTASVVCLLNNFYPREAKVQWKVD NALQSGNSQESVTEQDSKDSTYSLSSTLTLSKADYEKHKV YACEVTHQGLSSPVTKSFNRGECDKTHTCPPCPAPEAAGG PSVFLFPPKPKDTLMISRTPEVTCVVVDVSHEDPEVKFNWY VDGVEVHNAKTKPREEQYNSTYRVVSVLTVLHQDWLNG KEYKCKVSNKALGAPIEKTISKAKGQPREPQVCTLPPSRDE LTKNQVSLSCAVKGFYPSDIAVEWESNGQPENNYKTTPPV LDSDGSFFLVSKLTVDKSRWQQGNVFSCSVMHEALHNHY TQKSLSLSPGGGGGSGGGGSREGPELSPDDPAGLLDLRQG MFAQLVAQNVLLIDGPLSWYSDPGLAGVSLTGGLSYKEDT KELVVAKAGVYYVFFQLELRRVVAGEGSGSVSLALHLQPL RSAAGAAALALTVDLPPASSEARNSAFGFQGRLLHLSAGQ RLGVHLHTEARARHAWQLTQGATVLGLFRVTPEIPAGL |
| 110 | anti-FAP(4B9) Fc knob dimeric 4-1BB ligand (71-248) chain | see Table 2 |
| 106 | anti-FAP(4B9) light chain | See Table 1 |
| 214 | DP47 (VL-CH1) light chain | see Table 42 |

**Example 5A**

**Production of monovalent FAP-targeted human DP47/4-1BB ligand trimer-containing Fc fusion antigen binding molecules**

The monovalent FAP-targeted trimeric 4-1BB ligand C-terminal Fc (kih) fusion antigen binding molecule encoding sequences were cloned into a plasmid vector, which drives expression of the insert from an MPSV promoter and contains a synthetic polyA sequence located at the 3' end of the CDS. In addition, the vector contains an EBV OriP sequence for episomal maintenance of the plasmid.

The split trimeric 4-1BB ligand Fc (kih) fusion molecules were produced by co-transfecting HEK293-EBNA cells with the mammalian expression vectors using polyethylenimine. The cells were transfected with the corresponding expression vectors. For constructs 2.15 to 2.22, at a 1:1:1:1 ratio ("vector Fc hole chain": "vector light chain I": "vector Fc knob chain": "vector light chain 2"). The production and purification was carried out as described in Example 2.

Table 52 summarizes the yield and final monomer content of an exemplary DP47/FAP(4B9)-targeted split trimeric C-terminal 4-1BB ligand Fc(kih) fusion antigen binding molecule.

**Table 52: Biochemical analysis of an exemplary targeted split trimeric C-terminal 4-1BB ligand Fc(kih) fusion antigen binding molecule**

| Construct | Monomer [%] (SEC) | Yield [mg/l] |
|---|---|---|
| monovalent FAP(4B9) targeted split trimeric 4-1BB ligand C-terminal Fc (kih) fusion (construct 2.15) | 98.9 | 14.5 |

**Example 5B**

**Determination of simultaneous binding of DP47/FAP(4B9)-targeted C-terminal 4-1BB split trimeric ligand Fc fusion antigen binding molecules by surface plasmon resonance**

The capacity of binding simultaneously to human 4-1BB Fc(kih) and human FAP was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated human 4-1BB Fc(kih) was directly coupled to a flow cell of a streptavidin (SA) sensor chip. Immobilization levels up to 200 resonance units (RU) were used. The targeted trimeric C-terminal 4-1BB ligand Fc (kih) fusion molecules were passed at a concentration range of 200 nM with a flow of 30 μL/minute through the flow cells over 90 seconds and dissociation was set to zero sec. Human FAP was injected as second analyte with a flow of 30 μL/minute through the flow cells over 90 seconds at a concentration of 500 nM (Figure 6A). The dissociation was monitored for 120 sec. Bulk refractive index differences were corrected for by subtracting the response obtained in a reference flow cell, where no protein was immobilized. All bispecific constructs could bind simultaneously human 4-1BB and human FAP (Figure 6B).

**Example 6**

**Functional characterization of monovalent FAP-targeted C-terminal 4-1BB split trimeric ligand Fc fusion antigen binding molecules**

**6.1 Binding on fresh and activated human PBMCs**

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the diluted buffy coat contents were layered above the Histopaque 1077. The tubes were centrifuged for 30 min at 450 x g. PBMCs were then collected from the interface, washed three times with DPBS and resuspended in T cell medium consisting of RPMI 1640 medium (Gibco by Life Technology, Cat. No. 42401-042) supplied with 10 % (v/v) Fetal Bovine Serum (FBS, US-origin, PAN biotech, P30-2009, Lot P150307GI, gamma irradiated mycoplasma free, heat inactivated 35 min 56°C), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 μM β-Mercaptoethanol (SIGMA,

M3148).

PBMCs were used directly after isolation (binding on fresh human PBMCs) or they were stimulated to induce 4-1BB expression on the cell surface of T cells (binding on activated human PBMCs). To induce 4-1BB upregulation on human CD8 T cells, 6-well tissue culture plates (TTP, Cat.-No. 92006) were coated with 10 ug/mL anti-human CD3 (clone OKT3, Mouse IgG2a, BioLegend, Cat.-No. 317315) and 2 ug/mL anti-human CD28 (clone CD28.2, Mouse IgG1, BioLegend, Cat.-No. 302923) in DPBS at 4°C overnigh; shortly before further use plates were washed twice with DPBS to remove not coated antibodies. Freshly isolated PBMCs were set to 1 x $10^6$ cells/mL in T cell medium (same medium as described above), seeded to the aCD3/aCD28 coated 6-well plates and cultured for 3 days at 37°C and 5% $CO_2$.

For binding assay, 0.1 x $10^6$ fresh or activated PBMCs were added to each well of a round-bottom suspension cell 96-well plates (greiner bio-one, cellstar, Cat. No. 650185). Plates were centrifuged 5 minutes with 350 x g and at 4°C and supernatant were discarded. Afterwards cells were stained in 100 μL/well DPBS containing 1:5000 diluted fixable viability dye eF660 (eBioscience, Cat.-No. 65-0864-18) for 30 minutes at 4°C in the dark. Cells were washed once with 200 μL ice cold cold DPBS buffer. Next, 50 μL/well of 4 °C cold FACS buffer (DPBS supplied with 2 % FBS, 5 mM EDTA pH8 (Amresco, Cat. No. E177) and 7.5 mM Sodium azide (Sigma-Aldrich, Cat.-No. S2002)) containing monovalent FAP-targeted C-terminal 4-1BB split trimeric ligand Fc fusion antigen binding molecules (in particular construct 2.11 or construct 2.15), as positive control construct 2.4 and as negative controls Control D and Control F (prepared as described in Example 1.11) were added and cells were incubated for 60 minutes at 4 °C, washed three times with 200 μL/well 4 °C FACS buffer. Cells were further resuspended in 50 μL/well of 4°C cold FACS buffer containing 0.67 μg/mL anti-human CD45-AF488 (clone HI30, mouse igG1k, BioLegend, Cat.-No. 304019), 0.33 μg/mL anti-human CD8a-BV510 (clone SK1, mouse IgG1k, BioLegend, Cat.-No. 344732), 0.23 μg/mL anti-human CD4-BV421 (clone OKT4, mouse IgG2bκ, BioLegend, Cat.-No. 317434), 0.67 μg/mL anti-human CD3-PerCP-Cy5.5 (clone UCHT1, mouse IgG1k, BioLegend, Cat.-No. 300430), 0.67 μg/mL anti-human CD19-PE/Cy7 (clone HIB19, mouse IgG1k, BioLegend, Cat.-No. 302216), 5 μg/mL PE-conjugated AffiniPure anti-human IgG F(ab')2-fragment-specific goat F(ab')2 fragment (Jackson ImmunoResearch, Cat. No. 109 116 098) and incubated for 30 min at 4°C. Cells were washed twice with 200 μL/well 4 °C FACS buffer and then fixed with DPBS supplied with 1 % Formaldehyde (Sigma, HT501320-9.5L). Cells were resuspended in 100 μL/well FACS-buffer and acquired using the MACS Quant Analyzer 10 (Miltenyi Biotech). Data was analyzed using FlowJo V10 (FlowJo, LLC) and GraphPad Prism 6.04 (GraphPad Software, Inc).

Gates were set on living $CD45^+$ $CD3^+$ $CD19^{neg}$ $CD8^{neg}$ $CD4^+$ T cells, $CD45^+$ $CD3^+$ $CD19^{neg}$ $CD4^{neg}$ $CD8^+$ T cells, $CD45^+$ $CD3^+$ $CD4^{neg}$ $CD8^{neg}$ γδ T cells and $CD45^+$ $CD3^{neg}$ $CD19^+$ B cells and Geo Mean of fluorescence intensity of PE-conjugated AffiniPure anti-human IgG IgG Fcγ-fragment-specific goat F(ab')2 fragment was blotted against the titrated concentration of FAP-targeted or untargeted C-terminal 4-1BB split trimeric ligand Fc fusion antigen binding molecules. As shown in Figures 7A-H and Figures 8A-F, only after activation of human PBMCs 4-1BB was upregulated on human $CD4^+$ and $CD8^+$ T cells. In Tables 53a and 53b the $EC_{50}$ values are summarized for the binding curves to activated human PBMCs.

**Table 53a: ECso values of binding curves to activated human PBMCs (construct 2.11) (see Figures 8A-C)**

| | control D | construct 2.4 | construct 2.11 |
|---|---|---|---|
| $EC_{50}$ [nM] on activated $CD4^+$ T cell s | 0.37 | 0.41 | 0.28 |
| $EC_{50}$ [nM] on activated $CD8^+$ T cell s | 0.63 | 1.13 | 0.41 |
| $EC_{50}$ [nM] on activated γδ T cells | 0.28 | 0.64 | 0.36 |

**Table 53b: $EC_{50}$ values of binding curves to activated human PBMCs (construct 2.15) (see Figures 8D-F)**

| | control D | construct 2.4 | construct 2.15 |
|---|---|---|---|
| $EC_{50}$ [nM] on activated $CD4^+$ T cell s | 0.08 | 0.10 | 0.11 |
| $EC_{50}$ [nM] on activated $CD8^+$ T cell s | 0.15 | 0.23 | 0.17 |
| $EC_{50}$ [nM] on activated γδ T cells | 0.09 | 0.11 | 0.12 |

### 6.2 Binding of FAP-expressing tumor cells

For binding assays on FAP expressing cells different FAP-expressing tumor cells were used: human melanoma cell line MV-3 (first published: van Muijen GN, Jansen KF, Cornelissen IM, Smeets DF, Beck JL, Ruiter DJ. Establishment and characterization of a human melanoma cell line (MV3) which is highly metastatic in nude mice. Int J Cancer. 1991 Apr 22;48(1):85-91) and the human melanoma cell line WM-266-4 (ATCC CRL-1676).

0.1 x $10^6$ tumor cells resuspended in DPBS (Gibco by Life Technologies, Cat. No. 14190 326) were added to each well of a round-bottom suspension cell 96-well plates (greiner bio-one, cellstar, Cat. No. 650185). Cells were washed once with 200 μL DPBS. Cells were resuspended in 100 μL/well of 4°C cold DPBS buffer containing 1:5000 diluted Fixable Viability Dye eFluor 660 (eBioscience, Cat. No. 65-0864-18) and plates were incubated for 30 minutes at 4°C. Cells were washed once with 200 μL/well 4 °C cold DPBS buffer and resuspended in 50 μL/well of 4 °C cold FACS buffer (DPBS supplied with 2 % FBS, 5 mM EDTA pH8 (Amresco, Cat. No. E177) and 7.5 mM Sodium azide (Sigma-Aldrich S2002)) containing construct 2.11 or construct 2.15, as positive control construct 2.4 and as negative controls control D and control F at a series of concentrations, followed by incubation for 1 hour at 4 °C. After extensive washing, cells were further stained with 50 μL/well of 4°C cold FACS buffer containing 5 μg/mL PE-conjugated AffiniPure anti-human IgG F(ab')2-fragment-specific goat F(ab')2 fragment (Jackson ImmunoResearch, Cat. No. 109 116 098) for 30 minutes at 4°C. Cells were washed twice with 200 μL/well 4 °C FACS buffer and cells were fixed in 50 μL/well DPBS containing 1 % Formaldehyde (Sigma, HT501320-9.5L). Cells were resuspended in 100 μL/well FACS-buffer and acquired using the MACS Quant Analyzer 10 (Miltenyi Biotech). Data was analyzed using FlowJo V10 (FlowJo, LLC) and GraphPad Prism 6.04 (GraphPad Software, Inc).

Gates were set on living tumor cells and geo means of fluorescence intensity of PE-conjugated AffiniPure anti-human IgG IgG Fcγ-fragment-specific goat F(ab')2 fragment were blotted against the titrated concentration of FAP-targeted or untargeted C-terminal 4-1BB split trimeric ligand Fc fusion antigen binding molecules. As shown in Figures 9A and B, only the FAP-targeted split trimeric 4-1BB ligand fusion molecules construct 2.4 and construct 2.11 bound to FAP-expressing tumor cells MV-3 amd WM-266-4 whereas the untargeted Isotype control control F and the non-targeted split trimeric 4-1BB ligand fusion molecule control D did not bind to the tumor cells. In Figures 9C and 9D it is shown that the trispecific DP47/FAP-targeted split trimeric 4-1BB ligand fusion molecule construct 2.15 also bound to FAP-expressing tumor cells MV-3 amd WM-266-4. In Tables 54a and 54b the $EC_{50}$ values of the $FAP^+$ tumor binding curves are listed.

**Table 54a: $EC_{50}$ values of binding curves to FAP-expressing tumor cells (Figures 9A and B)**

| | Construct 2.4 | Construct 2.11 |
|---|---|---|
| $EC_{50}$ [nM] on MV-3 | 2.16 | 0.82 |
| $EC_{50}$ [nM] on WM-266-4 | 3.43 | 1.16 |

**Table 54a: $EC_{50}$ values of binding curves to FAP-expressing tumor cells (Figures 9C and D)**

| | Construct 2.4 | Construct 2.11 |
|---|---|---|
| $EC_{50}$ [nM] on MV-3 | 2.21 | 3.42 |
| $EC_{50}$ [nM] on WM-266-4 | 1.18 | 2.4 |

### 6.3 Biological Activity: NF-κB activation of human 4-1BB expressing HeLa reporter cell line

#### 6.3.1 Generation of HeLa reporter cells expressing human 4-1BB and NF-κB-luciferase

The human-papilloma-virus-18-induced cervix carcinoma cell line HeLa (ATCC CCL-2) was transduced with a plasmid based on the expression vector pETR10829, which contains the sequence of human 4-1BB (Uniprot accession Q07011) under control of a CMV-promoter and a puromycin resistance gene. Cells were cultured in DMEM-medium (Gibco by Life Technologies Cat. No. 42430-025) supplied with 10% Fetal Bovine Serum (FBS, GIBCO by Life Technologies, Cat. No. 16000-044, Lot. No. 941273, gamma-irradiated mycoplasma free, heat inactivated at 56 °C for 35 minutes), 1 % GlutaMAX-I (GIBCO by Life Technologies, Cat.-No. 35050-038) and 3 μg/mL Puromycin (InvivoGen, Cat-No. ant-pr). 4-1BB-transduced HeLa cells were tested for 4-1BB expression by flow cytometry: 0.2 x 106 living cells were resuspended in 100 μL FACS buffer (DPBS supplied with 2 % FBS, 5 mM EDTA pH 8 (Amresco, Cat. No. E177) and 7.5 mM Sodium Azide (Sigma-Aldrich, Cat. No. S2002)) containing 0.1 μg PerCP/Cy5.5 conjugated anti-human 4-1BB mouse IgG1κ clone 4B4-1 (BioLegend Cat. No. 309814) or its isotype control (PerCP/Cy5.5 conjugated mouse IgG1κ isotype control antibody clone MOPC 21, BioLegend Cat. No. 400150) and incubated for 30 minutes at 4 °C. Cells were washed twice with FACS buffer, resuspended in 300 μL FACS buffer containing 0.06 μg DAPI (Santa Cruz Biotec, Cat. No. Sc-3598) and acquired using a 5-laser LSR-Fortessa (BD Bioscience, DIVA software). Limited dilutions were performed to generate single clones as following: Human-4-1BB transduced HeLa cells were resuspended in medium to a concentration of 10, 5 and 2.5 cells/mL and 200 μL of cell suspensions were transferred to round bottom tissue-culture treated 96-well plates (6 plates/cell concentration, TPP Cat. No. 92697). Single clones were harvested, expanded and tested for 4-1BB expression as described above. The clone with the highest expression of 4-1BB (clone 5) was chosen for subsequent transfection with the NFκB-luciferase expression-vector 5495p Tranlucent HygB. The vector confers transfected cells both with resistance to hygromycin B and capacity to express luciferase under control of NFκB-response element (Panomics, Cat. No. LR0051). Human-4-1BB HeLa clone 5 cells were cultured to 70% confluence. 50 μg (40 μL) linearized (restriction enzymes AseI and SalI) 5495p Tranlucent HygB expression vector were added to a sterile 0.4 cm Gene Pulser/MicroPulser Cuvette (Biorad, Cat.-No, 165-2081). 2.5 x $10^6$ human-4-1BB HeLa clone 5 cells in 400 μl supplement-free DMEM were added and mixed carefully with the plasmid solution. Transfection of cells was performed using a Gene Pulser Xcell total system (Biorad, Cat No. 165 2660) under the following settings: exponential pulse, capacitance 500 μF, voltage 160 V, resistance ∞. Immediately after the pulse transfected cells were transferred to a tissue culture flask 75 $cm^2$ (TPP, Cat. No. 90075) with 15 mL 37 °C warm DMEM-Medium (Gibco by Life Technologies Cat. No. 42430-025) supplied with 10 % FBS and 1 % GlutaMAX I (GIBCO by Life Technologies, Cat. No. 35050 038). Next day, culture medium containing 3 μg/mL Puromycin (InvivoGen, Cat No. ant pr) and 200 μg/mL Hygromycin B (Roche, Cat.-No. 10843555001) was added. Surviving cells were expanded and limited dilution was performed as described above to generate single clones. Clones were tested for 4-1BB expression as described above and for NFκB-Luciferase activity as following: Clones were harvested in selection medium and counted using a Cell Counter Vi-cell xr 2.03 (Beckman Coulter, Cat. No. 731050). Cells were set to a cell density of 0.33 x $10^6$ cells/mL and 150 μL of this cell suspension were transferred to each well of a sterile white 96-well flat bottom tissue culture plate with lid (greiner bio one, Cat. No. 655083) and as a control to normal 96 well flat bottom tissue culture plate (TPP Cat. No. 92096) to test survival and cell density the next day. Cells were incubated at 37°C and 5 % $CO_2$ overnight. The next day 50 μL of medium containing different concentrations of recombinant human tumor necrosis factor alpha (rhTNFα, PeproTech, Cat.-No. 300 01A) were added to each well of a 96-well plate resulting in final concentration of rhTNFα of 100, 50, 25, 12.5, 6.25 and 0 ng/well. Cells were incubated for 6 hours at 37 °C and 5 % $CO_2$ and then washed three times with 200 μL/well DPBS. Reporter Lysis Buffer (Promega, Cat-No: E3971) was added to each well (30 μl) and the plates were stored over night at -20 °C. The next day frozen cell plates and Detection Buffer (Luciferase 1000 Assay System, Promega, Cat. No. E4550) were thawed to room temperature. 100 μL of detection buffer were added to each well and the plate was measured as fast as possible using a SpectraMax M5/M5e microplate reader and the SoftMax Pro Software (Molecular Devices). Measured URLs above control (no rhTNFα added) were taken as luciferase activity. The NFκB-luc-4-1BB-HeLa clone 26 was chosen for further use exhibiting the highest luciferase activity and a considerable level of 4-1BB-expression.

### 6.3.2 NF-κB activation of HeLa reporter cells expressing human 4-1BB co-cultured with FAP-expressing tumor cells

NFκB-luc-4-1BB-HeLa clone 26 cells were washed with DPBS (Gibco by Life Technologies, Cat. No. 14190 326) and treated with 0.05 % Trypsin EDTA (Gibco by Life Technologies Cat. No. 25300 054) for 5 min at 37 °C. Cells were harvested and resuspended in DMEM Medium (Gibco by Life Technologies Cat. No. 42430 025) supplied with 10 % Fetal Calf Serum (FBS, US-origin, PAN biotech, P30-2009, Lot P150307GI, gamma irradiated mycoplasma free, heat inactivated 35 min 56 °C) and 1 % GlutaMAX-I (GIBCO by Life Technologies, Cat. No. 35050 038). Cells were counted using Cell Counter Vi-cell xr 2.03 (Beckman Coulter, Cat. No. 731050) and set to a cell density of 0.2 x $10^6$ cells/mL. 100 μL (2 x $10^4$ cells) of this cell suspension were transferred to each well of a sterile white 96-well flat bottom tissue culture plate with lid (greiner bio one, Cat. No. 655083) and the plates were incubated at 37 °C and 5 % $CO_2$ overnight. The next day 50 μL of medium containing different titrated concentrations of construct 2.11 were added. As positive control construct 2.4 and as negative controls control D and control F (as described in Example 1.11) were added. For FAP-binding-mediated crosslinking the FAP-expressing line MV-3 (first published: van Muijen GN, Jansen KF, Cornelissen IM, Smeets DF, Beck JL, Ruiter DJ. Establishment and characterization of a human melanoma cell line (MV3) which is highly metastatic in nude mice. Int J Cancer. 1991 Apr 22;48(1):85-91), human melanoma cell line WM-266-4 (ATCC CRL-1676) or FAP-expressing mouse fibroblast cell lines NIH/3T3 transfected with human FAP (NIH/3T3-huFAP clone 19) were used.

Therefore FAP expressing tumor cell lines were washed with DPBS (Gibco by Life Technologies, Cat. No. 14190 326) and treated with enzyme-free, PBS-based Cell Dissociation Buffer (Gibco by Life Technologies, Cat.-No. 13151-014) for 10 minutes at 37 °C. Afterwards cell were harvested and counted using Cell Counter Vi-cell xr 2.03. Cells were resuspended in DEM supplied with 10% FBS and 1% L-GlutaMAX-I to 2 x $10^6$ cells/mL and 50 μL were added/well. After adding crosslinking FAP-expressing tumor cells plates were incubated for 6 hours at 37 °C and 5 % $CO_2$. White flat bottom 96-well plates were washed three times with 200 μL/well DPBS. 40 μl fresh prepared Reporter Lysis Buffer (Promega, Cat-No: E3971) were added to each well and the plate were stored over night at -20 °C. The next day frozen plates and detection buffer (Luciferase 1000 Assay System, Promega, Cat. No. E4550) were thawed to room temperature.

100 uL of detection buffer were added to each well and plates were measured as fast as possible using the SpectraMax M5/M5e microplate reader and SoftMax Pro Software (Molecular Devices) with following settings: for luciferase (RLUs), 500 ms integration time, no filter, collecting all wave length and top reading. The setup of the assay is shown in Figure 10 and the activities as measured for Constructs 2.11, 2.15 and 2.4 and Controls are shown in Figure 11. In Table 55 the $EC_{50}$ values of the NFκB-activation-induced Luciferase activity-curves are listed.

**Table 55: $EC_{50}$ values of NFκB-activation-induced Luciferase activity-curves**

| | Construct 2.4 | Construct 2.11 | Construct 2.15 |
|---|---|---|---|
| $EC_{50}$ [nM] on MV-3 | 0.15 | 0.18 | 0.16 |
| $EC_{50}$ [nM] on 3T3-huFAP | 0.06 | 0.08 | 0.10 |

**6.4 Activation assay of human PBMCs in the presence of FAP-expressing cells**

For FAP-binding-mediated crosslinking the FAP-expressing mouse fibroblast cell lines NIH/3T3 transfected with human FAP (NIH/3T3-huFAP clone 19) was used. The cells were generated by transfection of mouse embryonic fibroblast NIH/3T3 cells (ATCC CRL-1658) with the expression pETR4921 plasmid encoding human FAP under a CMV promoter. Cells were maintained DMEM (Gibco by Life Technologies Cat.-No. 42430-025) supplied with 10% Calf Serum (CS, Iron supplemented from formula-fed calves, Sigma-Aldrich, C8056-500 mL) in the presence of 1.5 μg/mL puromycin (InvivoGen, Cat.-No.: ant-pr-5).

NIH/3T3-huFAP clone 19 cells were resuspended in T cell medium consisting of RPMI 1640 (GIBCO by Life Technologies, Cat.-No. 42401-042) supplied with 10% fetal bovine serum (FBS, US-origin, PAN biotech, P30-2009, Lot P150307GI, gamma irradiated mycoplasma free, heat inactivated 35 min 56 °C), 1% L-GlutaMAX-I (GIBCO by Life Technologies, Cat-No. 35050-038), 1 mM Sodium-Pyruvat (SIGMA-Aldrich, Cat.-No. S8636) , 1% MEM-Non essential Aminoacid Solution (SIGMA-Aldrich, Cat.-No. M7145), 50 uM β-Mercaptoethanol (Sigma-Aldrich, Cyt.-No. M3148) and irradiated with 50 Gy (X-Ray Irradiator RS 2000, Rad source). 2 x $10^4$ NIH/3T3-huFAP clone 19 cells in 50 μL T cell medium were seeded to each well of a round bottom tissue culture 96-well plate (TTP, Cat.-No. 92697). 50 μL of T cell medium containing different titrated concentrations of construct 2.11 were added. As positive control construct 2.4 and as negative controls control D and control F as described in Example 1.11 were added. Human PBMCs were labeled in 37 °C warm DPBS containing 40 nM CFDA-SE (SIGMA-Aldrich, Cat.-No. 21888-25MG-F) for 15 min at 37°C. CFSE-labeling was stopped by adding FBS, PBMCs were washed twice and resuspended in T cell medium to a final concentration of 1.5 x $10^6$ cells/mL. 50 μL of this PBMC cell solution were seeded to each well to add 7.5 x $10^4$ CFSE-labeled PBMCs well. Finally a stock solution of T cell medium containing 2 mM agonistic anti-human CD3 antibody (clone V9, human IgG1, described in Rodrigues et al., Int J Cancer Suppl 7, 45-50 (1992) and US patent No. 6,054,297) was prepared and 50 μL/well were added to each well giving a final concentration of 0.5 mM anti-human CD3 human IgG1 clone V9.

Plates were incubated for 4 days at 37°C. Cells were washed with DPBS and stained with 100 μL/well DPBS containing 1:1000 diluted LIVE/DEAD® Fixable Aqua Dead Cell Stain Kit (Molecular Probes by Life Technology, Cat.-No. L34957) for 30 min at 4°C. Cells were washed once with 200 μL/well DPBS and stained with 50 μL FACS buffer (DPBS supplied with 2 % FBS, 5 mM EDTA pH8 (Amresco, Cat. No. E177) and 7.5 mM Sodium azide (Sigma-Aldrich S2002)) containing 0.1 μg/mL PerCP-Cy5.5-conjugated anti-human CD137 mouse IgG1κ (clone 4B4-1, BioLegend, Cat.-No. 309814), 0.1 μg/mL PE/Cy7-conjugated anti-human PD-1 mouse IgG1κ (clone EH12.2H7, BioLegend, Cat.-No. 329918), 0.03 μg/mL APC-conjugated anti-human CD25 mouse IgG1κ (clone BC96, BioLegend, Cat.-No. 302610), 0.06 μg/mL APC/Cy7-conjugated anti-human CD8 Mouse IgG1κ (clone RPA-T8, BioLegend, Cat.-No. 3301016), BV421-conjugated anti-human CD4 Mouse IgG1κ (clone RPA-T4, BioLegend, Cat.-No. 300532) for 30 min at 4°C. Cells were washed twice with 200 μL/well DPBS and incubated for 30 min at 4°C with 50 μL/well freshly prepared FoxP3 Perm/Fix buffer (eBioscience Cat.-No. 00-5123). Cells were washed twice with 200 μL/well DPBS, resuspended in 50 μL/well freshly prepared Perm-buffer (eBioscience Cat.-No 00-8333) supplied with PE-conjugated 1:250 diluted anti-human Granzyme B mouse IgG1κ (clone GB 11, Lot 4269803, BD Pharmingen, Cat.-No. 561142) and incubated for 1 h at 4°C. Plates were washed twice with 200 μL/well DPBS and cells were fixed for 15 min with DPBS containing 1 % Formaldehyde (Sigma, HT501320-9.5L). Cells were resuspended in 100 μL/well FACS-buffer and acquired using the MACS Quant Analyzer 10 (Miltenyi Biotech). Data was analyzed using FlowJo VI0 (FlowJo, LLC) and GraphPad Prism 6.04 (GraphPad Software, Inc).

As shown in **Figure 13,** only the FAP (4B9)-targeted split trimeric 4-1BBL fusion molecules Construct 2.11 and the positive control Construct 2.4 induced a concentration-dependent increase of surface expressed CD25 and 4-1BB (CD137) on $CD8^+$ T cells. The negative control molecules Control F and D did not induce this up-regulation of activation markers. The $EC_{50}$ values of CD25 and 4-1BB up-regulation on $CD8^+$ T cells are shown in **Table 56.**

**Table 56: $EC_{50}$ values of induced up-regulation of activation markers 4-1BB (CD137) and CD25 on $CD8^+$ T cells**

| | Construct 2.4 | Construct 2.11 |
|---|---|---|
| $EC_{50}$ [nM] of CD25-upregulation on $CD8^+$ T cells | 0.03 | 0.07 |
| $EC_{50}$ [nM] of CD137 (4-1BB)-upregulating on $CD8^+$ T cells | 0.08 | 0.11 |

### 6.5 Activation assay of CMV-NLV-specific $CD8^+$ T cells in the presence of FAP-expressing cells

#### 6.5.1 Isolation and culture of NLV-antigen-specific CD8 T cells

Fresh blood was obtained from a HLA-A2$^+$ CMV-infected volunteer. PBMCs were isolated by Ficoll density centrifugation and $CD8^+$ T cells were purified from PBMCs using a negative selection human CD8 T cell isolation Kit according to manufacturer's recommendations (Miltenyi Biotec, Cat. No. 130-094-156). Ten million of isolated CD8 T cells were resuspended in 1 mL sterile DPBS supplemented with 1 % (v/v) FBS along with 50 μL of PE-labeled HLA-A2-pentamer containing the CMV-derived NLVPMVATV peptide (ProImmune, Cat. No. F008-2B) and incubated for 10 min at room temperature. Cells were washed twice with 3 mL sterile DPBS supplied with 1 % (v/v) FBS. Cells were resuspended in 1 mL cells DPBS supplied with 1 % (v/v) FBS containing 1 μg/mL anti-human CD8-FITC (clone LT8, Abcam, Cat. No. Ab28010) and incubated for 30 minutes at 4°C. Cells were washed twice, resupended to a concentration of $5x10^6$ cells/mL in DPBS supplied with 1 % (v/v) FBS, and filtrated through a 30 μm pre-separation nylon-net cell strainer (Miltenyi Biotec, Cat. No. 130-041-407). NLV-peptide-specific $CD8^+$ T cells were isolated by FACS sorting using an ARIA cell sorter (BD Bioscience with DIVA software) with the following settings: 100 μm nozzle and purity sort mask. Sorted cells were collected in a 15 ml polypropylene centrifuge tube (TPP, Cat. No. 91015) containing 5 ml RPMI 1640 medium supplied with 10 % (v/v) FBS, 1 % (v/v) GlutaMAX-I and 400 U/mL Proleukin. Sorted cells were centrifuged for 7 minutes at 350 x g at room temperature and resuspended in same medium to a concentration of 0.53 x $10^6$ cells/mL. 100 μL/well of this cell suspension were added to each well of a previously prepared feeder plate. PHA-L-activated irradiated allogeneic feeder cells were prepared from PBMCs as previously described (Levitsky et al., 1998) and distributed to 96 well culture plates at $2x10^5$ feeder cells per well. After one day of culturing 100 μL medium/well were removed from well containing sorted $CD8^+$ T-cells and replaced by new RPMI 1640 medium supplemented with 10 % (v/v) FBS and 1 % (v/v) GlutaMAX-I and 400 U/mL Proleukin, this was repeated during culture on a regular basis (every 2-4 days). As soon as cells start to proliferate, they were transferred to 24-well flat-bottom tissue culture plate (TPP, 92024). Cells were expanded/split and reactivated with new feeder cell preparation on a regular basis.

#### 6.5.2 Activation assay of NLV-antigen-specific CD8+ T cells

MV-3 cells (already described in Example 6.2) were harvested and resuspended to 1 x $10^6$ cells/mL in T cell medium consisting of RPMI 1640 medium supplemented with 10 % (v/v) FBS, 1 % (v/v) GlutaMAX I, 1 mM Sodium-Pyruvate, 1 % (v/v) MEM non-essential amino acids and 50 μM β-Mercaptoethanol and separated into three tubes. Synthetic NLVPMVATV peptide (obtained from thinkpeptides) was added to a final concentration of $1x10^{-9}$ M or $1x10^{-8}$ M and cells were incubated for 90 min. NLV-peptide pulsed MV-3 cells were washed once with T cell medium and resuspended to a density of 0.5 x $10^6$ cells/mL, distributed (100 μL/well) to a 96-well round bottom cell-suspension plate (Greiner bio-one, cellstar, Cat. No. 650185) and incubated over night at 37°C and 5 % $CO_2$. The next day, FAP-targeted 4-1BB ligand trimer-containing Fc fusion antigen binding molecule (construct 2.11 and as controls construct 2.4 and control F and D) were added in 50 μL of T-cell medium at a range of final concentrations (from 10 to 0.0004 nM). NLV-specific CD8 T cells were harvested, washed and added in 50 μL medium to each well (final tumor: CD8 T cell ratio 0.125). The principle of the assay is shown in Figure 14.

Cells were incubated for 24 h and 50 μL/well were replaced with T cell medium containing 2.64 μL/mL Golgi stop (Protein Transport Inhibitor containing Monesin, BD Bioscience, Cat.-No. 554724, end concentration 0.66 μl/mL) and 4 μL/mL Golgi plug (Protein Transporter Inhibitor containing Brefeldin A, BD Bioscience, Cat.-No. 555029, end concentration 1 μg/mL) were added to each well.

Cells were incubated for 4 h and then plates were washed with 200 μL/well DPBS and stained with 100 μL 4 °C DPBS containing 1:1000 diluted LIVE/DEATH Fixable Aqua Dead Cell Stain (Molecular Probes, Cat.-No. L34957) for 30 minutes at 4°C. Cell were washed with 200 μL DPBS and stained in 50 μL/well FACS buffer (DPBS containing 2% FBS, 5 mM EDTA, 7.5 mM Sodium-Azide) containing 1 μg/mL anti-human CD137-PerCP-Cy5.5 (BioLegend, clone 4B4-1, Cat.-No. 309814), 0.67 μg/mL anti-human CD8-APC-Cy7 (BioLegend, clone RPA-T8, Cat.-No. 301016) and 0.67 μg/mL anti-human PD-1-PE-Cy7 (BioLegend, clone EH12.2H7, Cat.-No. 329918) for 30 min at 4°C. Cells were washed twice with 200 μL/well DBPS, resuspended in 50 uL freshly prepared Perm/Fix-buffer (eBioscience Cat.-No. 00-5123-43 and Cat.-No. 00-5223-56) and incubated for 30 min at 4°C. Cells were washed twice with 200 uL DPBS and resuspended in 50 uL freshly prepared Perm buffer (eBioscience Cat.-No. 00-8333-56) containing 6 ng/mL anti-human Eomes-eF660 (eBioscience, clone WD1928, Cat.-No. 50-4877-42), anti-human IFNy-BV421 (BioLegend, clone 4S.B3, Cat.-No. 502532) and 4 μL/mL anti-human Granzyme B-PE (BD, clone GB11, Cat.-No. 561142). Cells were incubated for 1 h at 4°C and washed twice with 200 uL/well DPBS. For fixation, 50 μL/well DPBS containing 1 % Formaldehyde were added and incubated for 15 min. Cells were resuspended in 100 μL/well FACS buffer and acquired using MACS Quant Analyzer 10 (Miltenyi Biotech). Data was analyzed with FlowJo v10.0.7 and Graph Pad Prism 6.04.

As shown in Figure 15, only the FAP (4B9)-targeted split trimeric 4-1BBL fusion molecules construct 2.11 and the positive control construct 2.4 induced a concentration-dependent increase of expressed 4-1BB (CD137) (Figure 15A) and IFNγ (Figure 15B) on NLV-specific $CD8^+$ T cells. The negative control molecules control F and D did not induce this up-regulation of activation markers. The $EC_{50}$ values of 4-1BB and IFNγ up-regulation on NLV-specific $CD8^+$ T cells are shown in Table 57.

**Table 57: EC50 values of induced upregulation of activation markers 4-1BB (CD137) and IFNγ on NLV-activated $CD8^+$ T cells.**

| TCR stimulus | parameter | Construct 2.4 | Construct 2.11 |
|---|---|---|---|
| 10-9 M peptide | $EC_{50}$ [nM] on $CD137^+$ CD8 | 0.023 | 0.006 |
| 10-8 M peptide | $EC_{50}$ [nM] on $CD137^+$ CD8 | 0.017 | 0.006 |
| 10-8 M peptide | $EC_{50}$ [nM] on $IFN\gamma^+$ CD8 | 0.021 | 0.011 |

## Example 7

### Functional characterization of monovalent CD19-targeted C-terminal 4-1BB split trimeric ligand Fc fusion antigen binding molecules

### 7.1 Binding on activated human PBMCs

To check the binding of 4-1BBL to 4-1BB expressing T cells, human PBMCs were preactivated by TCR stimulation for the upregulation of 4-1BB on T cells for 48 hours. PBMCs were purified from buffy coat by Ficoll (GE Healthcare, Cat No. 17-1440-03) gradient centrifugification, and were then diluted into a concentration of 2.8 x $10^6$/ml resuspended in RPMI medium (Gibco, Cat No. 72400-054) +10% FBS (Gibco, Cat No. 20012-068) and 1% penicillin-Streptomycin (Gibco, Cat No. 15070-063) and 50uM of 2-Mercaptoethanol (Gibco, Cat No. 31350-010). 90ul of cells were added to each well of a round-bottom 96-well plates (greiner bio-one, cellstar, Cat. No. 650185). Then additional 50ul anti-CD3 and anti-CD28 microbeads (Life Technologies, Cat No. 11131D) at 8 x $10^5$ beads/ml were added to wells. Two days later, cells were washed with cold PBS (Gibco, 20012-068) 1 time, and resuspended with 90 ul of cold PBS, and incubated with 10ul of solution containing construct 4.11, construct 3.11, positive control construct 4.1 and negative control Control D for 1 hour at 4 °C. After extensive washing, cells were further stained with 50 μL/well of cold FACS buffer containing 5 μg/mL PE-conjugated AffiniPure anti-human IgG F(ab')2-fragment-specific goat F(ab')2 fragment (Jackson ImmunoResearch, Cat. No. 109 116 098), and additionally with anti-human CD3 (Biolegend, Cat No. 300312), CD4 (Biolegend, Cat No. 317434) and CD8 (Biolegend, Cat No. 344710) Ab for 30 minutes at 4 °C. Cells were washed twice with 200 μL/well 4 °C FACS buffer and cells were fixed in 50 μL/well DPBS containing 1 % Formaldehyde (Sigma, HT501320-9.5L). Cells were resuspended in 100 μL/well FACS-buffer and acquired using the FACS LSR II (BD Biosciences). Data was analyzed using FlowJo V10 (FlowJo, LLC) and GraphPad Prism 6.04 (GraphPad Software, Inc).

The specific binding was gated on pure population of CD4 and CD8 cells. All the constructs showed similar binding properties to 4-1BB expressing CD4 or CD8 T cells in a dose dependent manner (Figure 16). In Table 58 the $EC_{50}$ values of the binding curves are listed.

**Table 58: $EC_{50}$ values of binding curves to $4\text{-}1BB^+$ human T cells**

| | Construct 4.11 | Construct 3.11 | Construct 4.1 | Control D |
|---|---|---|---|---|
| $EC_{50}$ [nM] on CD4 | 0.13 | 0.13 | 0.11 | 0.17 |
| $EC_{50}$ [μg/ml] on CD4 | 0.02 | 0.02 | 0.02 | 0.03 |
| $EC_{50}$ [nM] on CD8 | 0.32 | 0.38 | 0.28 | 0.23 |

(continued)

| | Construct 4.11 | Construct 3.11 | Construct 4.1 | Control D |
|---|---|---|---|---|
| $EC_{50}$ [μg/ml] on CD8 | 0.05 | 0.06 | 0.05 | 0.04 |

### 7.2 Binding of CD19-expressing tumor cells

To check the binding to tumor antigen CD19, the $CD19^+$ WSU-DLCL2 cells (DSMZ No. ACC 575) were used. 0.1 x $10^6$ tumor cells resuspended in DPBS (Gibco by Life Technologies, Cat. No. 14190 326) were added to each well of a round-bottom suspension cell 96-well plates (greiner bio-one, cellstar, Cat. No. 650185). Cells were washed once with 200 μL DPBS. Cells were resuspended in 100 μL/well of 4 °C cold DPBS buffer containing 1:5000 diluted Fixable Viability Dye eFluor 660 (eBioscience, Cat. No. 65-0864-18) and plates were incubated for 30 minutes at 4°C. Cells were washed once with 200 μL/well 4 °C cold DPBS buffer and resuspended in 50 μL/well of 4 °C cold FACS buffer (DPBS supplied with 2 % FBS, 5 mM EDTA pH8 (Amresco, Cat. No. E177) and 7.5 mM Sodium azide (Sigma-Aldrich S2002)) containing Construct 4.11, Construct 3.11, as positive control construct 4.1 and as negative control control D at a series of concentrations, followed by incubation for 1 hour at 4 °C. After extensive washing, cells were further stained with 50 μL/well of 4 °C cold FACS buffer containing 5 μg/mL PE-conjugated AffiniPure anti-human IgG F(ab')2-fragment-specific goat F(ab')2 fragment (Jackson ImmunoResearch, Cat. No. 109 116 098) for 30 minutes at 4 °C. Cells were washed twice with 200 μL/well 4 °C FACS buffer and cells were fixed in 50 μL/well DPBS containing 1 % Formaldehyde (Sigma, HT501320-9.5L). Cells were resuspended in 100 μL/well FACS-buffer and acquired using the FACS LSR II (BD Biosciences). Data was analyzed using FlowJo V10 (FlowJo, LLC) and GraphPad Prism 6.04 (GraphPad Software, Inc).

Gates were set on living tumor cells and geo means of fluorescence intensity of PE-conjugated AffiniPure anti-human IgG IgG Fcγ-fragment-specific goat F(ab')2 fragment were plotted against the titrated concentration of CD19-targeted or untargeted C-terminal 4-1BB split trimeric ligand Fc fusion antigen binding molecules. As shown in **Figure 17,** only the CD19-targeted split trimeric 4-1BB ligand fusion molecules Construct 4.11 and Construct 3.11 and Construct 4.1 bound to CD19-expressing tumor cells WSU-DLCL2, whereas the untargeted split trimeric 4-1BB ligand fusion molecule Control D did not bind to the tumor cells. In Table 59, the $EC_{50}$ values of the $CD19^+$ tumor binding curves are listed.

**Table 59: $EC_{50}$ values of binding curves to $CD19^+$ tumor cells**

| | Construct 4.11 | Construct 3.11 | Construct 4.1 |
|---|---|---|---|
| $EC_{50}$ [nM] | 0.26 | 0.38 | 0.17 |
| $EC_{50}$ [μg/ml] | 0.04 | 0.06 | 0.03 |

### 7.3 Biological Activity: NF-κB activation of HeLa reporter cells expressing human 4-1BB co-cultured with CD19-expressing B cells lymphoma cells

#### 7.3.1 Generation of HeLa reporter cells expressing human 4-1BB and NF-κB-luciferase

The HeLa-human-4-1BB-NFκN-luc reporter cell line was generated as described already in Example 6.3.1.

#### 7.3.2 NFκB activation of HeLa reporter cells expressing human 4-1BB co-cultured with FAP-expressing tumor cells

NFκB-luc-4-1BB-HeLa clone 26 cells were washed with DPBS (Gibco by Life Technologies, Cat. No. 14190 326) and treated with 0.05 % Trypsin EDTA (Gibco by Life Technologies Cat. No. 25300 054) for 5 min at 37 °C. Cells were harvested and resuspended in DMEM Medium (Gibco by Life Technologies Cat. No. 42430 025) supplied with 10 % Fetal Calf Serum (FBS, US-origin, PAN biotech, P30-2009, Lot P150307GI, gamma irradiated mycoplasma free, heat inactivated 35 min 56 °C) and 1 % GlutaMAX-I (GIBCO by Life Technologies, Cat. No. 35050 038). Cells were counted using Cell Counter Vi-cell xr 2.03 (Beckman Coulter, Cat. No. 731050) and set to a cell density of 0.2 x $10^6$ cells/mL. 100 μL (2 x $10^4$ cells) of this cell suspension were transferred to each well of a sterile white 96-well flat bottom tissue culture plate with lid (greiner bio one, Cat. No. 655083) and the plates were incubated at 37 °C and 5 % $CO_2$ overnight. The next day 50 μL of medium containing different titrated concentrations of Construct 3.11 and Construct 4.11 were added. As positive control Constructs 3.4 and 4.1 and as negative controls Control D and Control F were added, which have been described Example 1.11. For CD19-binding-mediated crosslinking the following CD19-expressing cell lines were used: diffuse large B-cell lymphoma cell line OCI-Ly18 (DMSZ ACC 699), acute lymphoblastic leukemia (ALL) cell line Nalm6 (DSMZ ACC-128) and non-Hodgkin's B cell lymphoma (B-NHL) cell line SU-DHL-8 (DSMZ ACC-573). Therefore CD19-expressing suspension cell lines OCI-Ly18, Nalm6 and SU-DHL-8 and were harvested, counted using Cell Counter Vi-cell xr 2.03 and resuspended in DEM supplied with 10% FBS and 1% L-GlutaMAX-I to 2 x $10^6$ cells/mL and 50 μL were added/well. After adding crosslinking CD19-expressing tumor cells lines plates were incubated for 6 hours at 37 °C and 5 % $CO_2$. White flat bottom 96-well plates were washed three times with 200 μL/well DPBS. 40 μl fresh prepared Reporter Lysis Buffer (Promega, Cat-No: E3971) were added to each well and the plate were stored over night at -20 °C. The next day frozen plates and detection buffer (Luciferase 1000 Assay System, Promega, Cat. No. E4550) were thawed to room temperature. 100 uL of detection buffer were added to each well and plates were measured as fast as possible using the SpectraMax M5/M5e microplate reader and SoftMax Pro Software (Molecular Devices) with following settings: for luciferase (RLUs), 500ms integration time, no filter, collecting all wave length and top reading. The setup of the assay is shown in Figure 18 and the activities as measured for Constructs 3.4, 4.1, 3.11 and 4.11 and Controls D and F are shown in Figure 19. In Table 60 the $EC_{50}$ values of the binding curves to CD19-expressing tumor cell lines are listed.

**Table 60: $EC_{50}$ values of binding curves to CD19-expressing tumor cell lines**

| | Construct 3.4 | Construct 3.11 | Construct 4.1 |
|---|---|---|---|
| OCI-Ly18 $EC_{50}$ [nM] | 0.03 | 0.03 | 0.03 |
| Nalm6 $EC_{50}$ [nM] | 0.03 | 0.03 | 0.02 |
| SU-DHL-8 $EC_{50}$ [nM] | 0.03 | 0.03 | 0.03 |

**7.4 Biological Activity: human PBMC activation assay**

To determine the biological activities in physiological relevant settings, we measured the release of effector function molecule IFNγ in activated human PBMCs by costimluating T cells and NK cells via 4-1BBL. PBMCs were purified from buffy coat by Ficoll (GE Healthcare, Cat No. 17-1440-03) gradient centrifugification, and were then diluted into a concentration of 2.2 x $10^6$/ml resuspended in RPMI medium (Gibco, Cat No. 72400-054) +10% FBS (Gibco, Cat No. 20012-068) and 1% penicillin-Streptomycin (Gibco, Cat No. 15070-063) and 50uM of 2-Mercaptoethanol (Gibco, Cat No. 31350-010). 90 μl of cells were added to each well of round-bottom 96-well plates (greiner bio-one, cellstar, Cat. No. 650185). Then 10 μl of solution containing Construct 4.11, Construct 3.11, positive control construct 4.1 and negative control control D were added to the wells at a series of concentrations, and provided with additional 50 μl anti-CD3 and anti-CD28 microbeads (Life Technologies, Cat No. 11131D) at 8 x $10^5$ beads/ml. After 48 hour incubation, the supernatant were collected for the measurement of IFN-y by ELISA (DuoSet Human IFNg ELISA kit, R&D Systems, Cat No. DY285).

**Figure 20** shows that Construct 4.11, Construct 3.11 and the positive control Construct 4.1 stimulated PBMCs to produce a similar amount of IFNγ, whereas the negative control D did not activate T or NK cells due to the lack of cross-linking. In Table 61 the $EC_{50}$ values of the IFNγ curves on activated human PBMCs are listed.

**Table 61: $EC_{50}$ values of of the IFNγ curves on activated human PBMCs**

| | Construct 4.11 | Construct 3.11 | Construct 4.1 |
|---|---|---|---|
| $EC_{50}$ [nM] | 0.19 | 0.13 | 0.06 |
| $EC_{50}$ [μg/ml] | 0.03 | 0.02 | 0.01 |

**Citations:**

Ascierto, P. A., E. Simeone, M. Sznol, Y. X. Fu, and I. Melero (2010), Clinical experiences with anti-CD137 and anti-PDl therapeutic antibodies. Semin Oncol 37:508-516.

Aggarwal B.B. (2003), Signalling pathways of the TNF superfamily: a double-edged sword. Nat. Rev. Immunol. 3(9),745-56.

Banner D. et al (1993), Crystal structure of the soluble human 55 kd TNF receptor-human TNF beta complex:

implications for TNF receptor activation. Cell 73, 431-445.

Bodmer J., Schneider P. and Tschopp, J. (2002), The molecular architecture of the TNF superfamily. Trends in Biochemical Sciences 27(1), 19-26.

Broll, K., Richter, G., Pauly, S., Hofstaedter, F., and Schwarz, H. (2001). CD137 expression in tumor vessel walls. High correlation with malignant tumors. Am J Clin Pathol 115, 543-549.

Buechele, C., Baessler, T., Schmiedel, B.J., Schumacher, C.E., Grosse-Hovest, L., Rittig, K., and Salih, H.R. (2012). 4-1BB ligand modulates direct and Rituximab-induced NK-cell reactivity in chronic lymphocytic leukemia. Eur J Immunol 42, 737-748.

Choi, B.K., Kim, Y.H., Kwon, P.M., Lee, S.C., Kang, S.W., Kim, M.S., Lee, M.J., and Kwon, B.S. (2009). 4-1BB functions as a survival factor in dendritic cells. J Immunol 182, 4107-4115.

Cuadros, C., Dominguez, A.L., Lollini, P.L., Croft, M., Mittler, R.S., Borgstrom, P., and Lustgarten, J. (2005). Vaccination with dendritic cells pulsed with apoptotic tumors in combination with anti-OX40 and anti-4-1BB monoclonal antibodies induces T cell-mediated protective immunity in Her-2/neu transgenic mice. Int J Cancer 116, 934-943.

Curran, M.A., Kim, M., Montalvo, W., Al-Shamkhani, A., and Allison, J.P. (2011). Combination CTLA-4 blockade and 4-1BB activation enhances tumor rejection by increasing T-cell infiltration, proliferation, and cytokine production. PLoS One 6, e19499.

Diehl, L., van Mierlo, G.J., den Boer, A.T., van der Voort, E., Fransen, M., van Bostelen, L., Krimpenfort, P., Melief, C.J., Mittler, R., Toes, R.E., and Offringa, R. (2002). In vivo triggering through 4-1BB enables Th-independent priming of CTL in the presence of an intact CD28 costimulatory pathway. J Immunol 168, 3755-3762.

Dubrot, J., Milheiro, F., Alfaro, C., Palazon, A., Martinez-Forero, I., Perez-Gracia, J.L., Morales-Kastresana, A., Romero-Trevejo, J.L., Ochoa, M.C., Hervas-Stubbs, S., et al. (2010). Treatment with anti-CD137 mAbs causes intense accumulations of liver T cells without selective antitumor immunotherapeutic effects in this organ. Cancer Immunol Immunother 59, 1223-1233.

Futagawa, T., Akiba, H., Kodama, T., Takeda, K., Hosoda, Y., Yagita, H., and Okumura, K. (2002). Expression and function of 4-1BB and 4-1BB ligand on murine dendritic cells. Int Immunol 14, 275-286.

Guo, Z., Cheng, D., Xia, Z., Luan, M., Wu, L., Wang, G., and Zhang, S. (2013). Combined TIM-3 blockade and CD137 activation affords the long-term protection in a murine model of ovarian cancer. J Transl Med 11, 215.

Heinisch, I.V., Daigle, I., Knopfli, B., and Simon, H.U. (2000). CD137 activation abrogates granulocyte-macrophage colony-stimulating factor-mediated anti-apoptosis in neutrophils. Eur J Immunol 30, 3441-3446.

Hornig, N., Kermer, V., Frey, K., Diebolder, P., Kontermann, R.E., Mueller, D. (2012), Combination of a bispecific antibody and costimulatory antibody-ligand fusion proteins for targeted cancer immunotherapy. J. Immunother. 35, 418-429.

Ju, S.A., Cheon, S.H., Park, S.M., Tam, N.Q., Kim, Y.M., An, W.G., and Kim, B.S. (2008). Eradication of established renal cell carcinoma by a combination of 5-fluorouracil and anti-4-1BB monoclonal antibody in mice. Int J Cancer 122, 2784-2790.

Kienzle, G., and von Kempis, J. (2000). CD137 (ILA/4-1BB), expressed by primary human monocytes, induces monocyte activation and apoptosis of B lymphocytes. Int Immunol 12, 73-82.

Kim, D.H., Chang, W.S., Lee, Y.S., Lee, K.A., Kim, Y.K., Kwon, B.S., and Kang, C.Y. (2008). 4-1BB engagement costimulates NKT cell activation and exacerbates NKT cell ligand-induced airway hyperresponsiveness and inflammation. J Immunol 180, 2062-2068.

Kim, Y.H., Choi, B.K., Oh, H.S., Kang, W.J., Mittler, R.S., and Kwon, B.S. (2009). Mechanisms involved in synergistic anticancer effects of anti-4-1BB and cyclophosphamide therapy. Mol Cancer Ther 8, 469-478.

Kwon, B.S., and Weissman, S.M. (1989). cDNA sequences of two inducible T-cell genes. Proc Natl Acad Sci U S A 86, 1963-1967.

Lee, H., Park, H.J., Sohn, H.J., Kim, J.M., and Kim, S.J. (2011). Combinatorial therapy for liver metastatic colon cancer: dendritic cell vaccine and low-dose agonistic anti-4-1BB antibody costimulatory signal. J Surg Res 169, e43-50.

Levitsky, V., de Campos-Lima, P.O., Frisan, T., and Masucci, M.G. (1998). The clonal composition of a peptide-specific oligoclonal CTL repertoire selected in response to persistent EBV infection is stable over time. J Immunol 161, 594-601.

Li, F., and Ravetch, J.V. (2011). Inhibitory Fcgamma receptor engagement drives adjuvant and anti-tumor activities of agonistic CD40 antibodies. Science 333, 1030-1034.

Lin, W., Voskens, C.J., Zhang, X., Schindler, D.G., Wood, A., Burch, E., Wei, Y., Chen, L., Tian, G., Tamada, K., et al. (2008). Fc-dependent expression of CD137 on human NK cells: insights into "agonistic" effects of anti-CD137 monoclonal antibodies. Blood 112, 699-707.

Melero, I., Johnston, J.V., Shufford, W.W., Mittler, R.S., and Chen, L. (1998). NK1.1 cells express 4-1BB (CDw137) costimulatory molecule and are required for tumor immunity elicited by anti-4-1BB monoclonal antibodies. Cell Immunol 190, 167-172.

Melero, I., Shuford, W.W., Newby, S.A., Aruffo, A., Ledbetter, J.A., Hellstrom, K.E., Mittler, R.S., and Chen, L. (1997). Monoclonal antibodies against the 4-1BB T-cell activation molecule eradicate established tumors. Nat Med 3, 682-685.

Merchant, A.M., Zhu, Z., Yuan, J.Q., Goddard, A., Adams, C.W., Presta, L.G., and Carter, P. (1998). An efficient route to human bispecific IgG. Nat Biotechnol 16, 677-681.

Morales-Kastresana, A., Sanmamed, M.F., Rodriguez, I., Palazon, A., Martinez-Forero, I., Labiano, S., Hervas-Stubbs, S., Sangro, B., Ochoa, C., Rouzaut, A., et al. (2013). Combined immunostimulatory monoclonal antibodies extend survival in an aggressive transgenic hepatocellular carcinoma mouse model. Clin Cancer Res 19, 6151-6162.

Mueller, D., Frey, K., Kontermann, R.E. (2008), A novel antibody-4-1BB1 fusion protein for targeted costimulation in cancer immunotherapy, J. Immunother. 31, 714-722.

Murillo, O., Dubrot, J., Palazon, A., Arina, A., Azpilikueta, A., Alfaro, C., Solano, S., Ochoa, M.C., Berasain, C., Gabari, I., et al. (2009). In vivo depletion of DC impairs the anti-tumor effect of agonistic anti-CD137 mAb. Eur J Immunol 39, 2424-2436.

Narazaki, H., Zhu, Y., Luo, L., Zhu, G., and Chen, L. (2010). CD137 agonist antibody prevents cancer recurrence: contribution of CD137 on both hematopoietic and nonhematopoietic cells. Blood 115, 1941-1948.

Nishimoto, H., Lee, S.W., Hong, H., Potter, K.G., Maeda-Yamamoto, M., Kinoshita, T., Kawakami, Y., Mittler, R.S., Kwon, B.S., Ware, C.F., et al. (2005). Costimulation of mast cells by 4-1BB, a member of the tumor necrosis factor receptor superfamily, with the high-affinity IgE receptor. Blood 106, 4241-4248.

Olofsson, P.S., Soderstrom, L.A., Wagsater, D., Sheikine, Y., Ocaya, P., Lang, F., Rabu, C., Chen, L., Rudling, M., Aukrust, P., et al. (2008). CD137 is expressed in human atherosclerosis and promotes development of plaque inflammation in hypercholesterolemic mice. Circulation 117, 1292-1301.

Palazon, A., Teijeira, A., Martinez-Forero, I., Hervas-Stubbs, S., Roncal, C., Penuelas, I., Dubrot, J., Morales-Kastresana, A., Perez-Gracia, J.L., Ochoa, M.C., et al. (2011). Agonist anti-CD137 mAb act on tumor endothelial cells to enhance recruitment of activated T lymphocytes. Cancer Res 71, 801-811.

Schwarz, H., Valbracht, J., Tuckwell, J., von Kempis, J., and Lotz, M. (1995). ILA, the human 4-1BB homologue, is inducible in lymphoid and other cell lineages. Blood 85, 1043-1052.

Shao, Z., and Schwarz, H. (2011). CD137 ligand, a member of the tumor necrosis factor family, regulates immune responses via reverse signal transduction. J Leukoc Biol 89, 21-29.

Shi, W., and Siemann, D.W. (2006). Augmented antitumor effects of radiation therapy by 4-1BB antibody (BMS-469492) treatment. Anticancer Res 26, 3445-3453.

Simeone, E., and Ascierto, P.A. (2012). Immunomodulating antibodies in the treatment of metastatic melanoma: the experience with anti-CTLA-4, anti-CD137, and anti-PD1. J Immunotoxicol 9, 241-247.

Snell, L.M., Lin, G.H., McPherson, A.J., Moraes, T.J., and Watts, T.H. (2011). T-cell intrinsic effects of GITR and 4-1BB during viral infection and cancer immunotherapy. Immunol Rev 244, 197-217.

Stagg, J., Loi, S., Divisekera, U., Ngiow, S.F., Duret, H., Yagita, H., Teng, M.W., and Smyth, M.J. (2011). Anti-ErbB-2 mAb therapy requires type I and IIinterferons and synergizes with anti-PD-1 or anti-CD137 mAb therapy. Proc Natl Acad Sci U S A 108, 7142-7147.

Teng, M.W., Sharkey, J., McLaughlin, N.M., Exley, M.A., and Smyth, M.J. (2009). CD1d-based combination therapy eradicates established tumors in mice. J Immunol 183, 1911-1920.

von Kempis, J., Schwarz, H., and Lotz, M. (1997). Differentiation-dependent and stimulus-specific expression of ILA, the human 4-1BB-homologue, in cells of mesenchymal origin. Osteoarthritis Cartilage 5, 394-406.

Wei, H., Zhao, L., Li, W., Fan, K., Qian, W., Hou, S., Wang, H., Dai, M., Hellstrom, I., Hellstrom, K.E., and Guo, Y. (2013). Combinatorial PD-1 blockade and CD137 activation has therapeutic efficacy in murine cancer models and synergizes with cisplatin. PLoS One 8, e84927.

Wilcox, R.A., Chapoval, A.I., Gorski, K.S., Otsuji, M., Shin, T., Flies, D.B., Tamada, K., Mittler, R.S., Tsuchiya, H., Pardoll, D.M., and Chen, L (2002). Cutting edge: Expression of functional CD137 receptor by dendritic cells. J Immunol 168, 4262-4267.

Wilcox, R.A., Tamada, K., Flies, D.B., Zhu, G., Chapoval, A.I., Blazar, B.R., Kast, W.M., and Chen, L. (2004). Ligation of CD137 receptor prevents and reverses established anergy of CD8+ cytolytic T lymphocytes in vivo. Blood 103, 177-184.

Zhang, N., Sadun, R.E., Arias, R.S., Flanagan, M.L., Sachsman, S.M., Nien, Y, Khawli, L.A., Hu, P., Epstein, A.L. (2007). Targeted and untargeted CD137L fusion proteins for the immunotherapy of experimental solid tumors. Clin. Cancer Res. 13, 2758-2767.

Zhang, X., Voskens, C.J., Sallin, M., Maniar, A., Montes, C.L., Zhang, Y., Lin, W., Li, G., Burch, E., Tan, M., et al. (2010). CD137 promotes proliferation and survival of human B cells. J Immunol 184, 787-795.

**Claims**

**1.** A 4-1BB ligand (4-1BBL) trimer-containing antigen binding molecule comprising

(a) one Fab domain capable of specific binding to a target cell antigen,
(b) a Fc domain composed of a first and a second subunit capable of stable association, and
(c) a first polypeptide comprising two ectodomains of 4-1BBL comprising the amino acid sequence of SEQ ID NO:5 that are connected to each other by a peptide linker and a second polypeptide comprising one ectodomain of said 4-1BBL comprising the amino acid sequence of SEQ ID NO:5, wherein the first polypeptide is fused at its N-terminus to the C-terminus to the first subunit of the Fc domain comprising the amino acid substitutions Y349C, T366S, L368A and Y407V (numbering according to Kabat EU index) of the hole and wherein the second polypeptide is fused at its N-terminus to the C-terminus of the second subunit of the Fc domain comprising the amino acid substitutions S354C and T366W (numbering according to Kabat EU index) of the knob and wherein the VH and CH1 domain of the Fab domain capable of specific binding to a target cell antigen is fused at the C-terminus to the N-terminus of the second subunit of the Fc domain comprising the knob mutations,

wherein the 4-1BBL trimer-containing antigen binding molecule is monovalent for the binding to the target cell antigen.

**2.** The 4-1BBL trimer-containing antigen binding molecule of claim 1, wherein 4-1BBL costimulates human T-cell activation.

**3.** The 4-1BBL trimer-containing antigen binding molecule of claims 1 or 2, wherein the target cell antigen is selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD19, CD20 and CD33.

**4.** The 4-1BBL trimer-containing antigen binding molecule of any one of claims 1 to 3, wherein the target cell antigen is Fibroblast Activation Protein (FAP).

**5.** The 4-1BBL trimer-containing antigen binding molecule of any one of claims 1 to 4, wherein the Fab domain capable of specific binding to FAP comprises

(a) a VH domain comprising (i) CDR-H1 comprising the amino acid sequence of SEQ ID NO: 13, (ii) CDR-H2 comprising the amino acid sequence of SEQ ID NO:14 and (iii) CDR-H3 comprising the amino acid sequence of SEQ ID NO: 15, and a VL domain comprising (iv) CDR-L1 comprising the amino acid sequence of SEQ ID NO:16, (v) CDR-L2 comprising the amino acid sequence of SEQ ID NO: 17 and (vi) CDR-L3 comprising the amino acid sequence of SEQ ID NO:18 or
(b) a VH domain comprising (i) CDR-H1 comprising the amino acid sequence of SEQ ID NO: 19, (ii) CDR-H2 comprising the amino acid sequence of SEQ ID NO:20 and (iii) CDR-H3 comprising the amino acid sequence of SEQ ID NO:21, and a VL domain comprising (iv) CDR-L1 comprising the amino acid sequence of SEQ ID NO:22, (v) CDR-L2 comprising the amino acid sequence of SEQ ID NO:23 and (vi) CDR-L3 comprising the amino acid sequence of SEQ ID NO:24.

**6.** The 4-1BBL trimer-containing antigen binding molecule of any one of claims 1 to 5, wherein the Fab domain capable of specific binding to FAP comprises a variable heavy chain comprising an amino acid sequence of SEQ ID NO:25 and a variable light chain comprising an amino acid sequence of SEQ ID NO:26 or wherein the Fab domain capable of specific binding to FAP comprises a variable heavy chain comprising an amino acid sequence of SEQ ID NO:27 and a variable light chain comprising an amino acid sequence of SEQ ID NO:28.

**7.** The 4-1BBL trimer-containing antigen binding molecule of any one of claims 1 to 6, comprising

(i) a light chain comprising the amino acid sequence of SEQ ID NO: 106,
(ii) a fusion polypeptide comprising the amino acid sequence of SEQ ID NO: 104, and
(iii) a heavy chain comprising the amino acid sequence of SEQ ID NO: 105.

**8.** The TNF family ligand trimer-containing antigen binding molecule of any one of claims 1 to 3, wherein the target cell antigen is CD19.

**9.** The TNF family ligand trimer-containing antigen binding molecule of any one of claims 1 to 3 or 8, wherein Fab domain capable of specific binding to CD19 comprises

(a) a VH domain comprising (i) CDR-H1 comprising the amino acid sequence of SEQ ID NO:29, (ii) CDR-H2 comprising the amino acid sequence of SEQ ID NO:30 and (iii) CDR-H3 comprising the amino acid sequence of SEQ ID NO:31, and a VL domain comprising (iv) CDR-L1 comprising the amino acid sequence of SEQ ID NO:32, (v) CDR-L2 comprising the amino acid sequence of SEQ ID NO:33 and (vi) CDR-L3 comprising the amino acid sequence of SEQ ID NO:34 or
(b) a VH domain comprising (i) CDR-H1 comprising the amino acid sequence of SEQ ID NO:35, (ii) CDR-H2 comprising the amino acid sequence of SEQ ID NO:36 and (iii) CDR-H3 comprising the amino acid sequence of SEQ ID NO:37, and a VL domain comprising (iv) CDR-L1 comprising the amino acid sequence of SEQ ID NO:38, (v) CDR-L2 comprising the amino acid sequence of SEQ ID NO:39 and (vi) CDR-L3 comprising the amino acid sequence of SEQ ID NO:40.

**10.** The TNF family ligand trimer-containing antigen binding molecule of any one of claims 1 to 3, 8 or 9, wherein the Fab domain capable of specific binding to CD19 comprises a variable heavy chain comprising an amino acid sequence of SEQ ID NO:41 and a variable light chain comprising an amino acid sequence of SEQ ID NO:42 or wherein the Fab domain capable of specific binding to FAP comprises a variable heavy chain comprising an amino acid sequence of SEQ ID NO:43 and a variable light chain comprising an amino acid sequence of SEQ ID NO:44.

**11.** The 4-1BBL trimer-containing antigen binding molecule of any one of claims 1 to 3 or 8 to 10, comprising

(i) a light chain comprising the amino acid sequence of SEQ ID NO:120,
(ii) a fusion polypeptide comprising the amino acid sequence of SEQ ID NO:104, and
(iii) a heavy chain comprising the amino acid sequence of SEQ ID NO:119,

or

(i) a light chain comprising the amino acid sequence of SEQ ID NO:126,
(ii) a fusion polypeptide comprising the amino acid sequence of SEQ ID NO:174, and
(iii) a heavy chain comprising the amino acid sequence of SEQ ID NO:125.

**12.** The TNF family ligand trimer-containing antigen binding molecule of any one of claims 1 to 3, wherein the target cell antigen is CEA.

**13.** The TNF family ligand trimer-containing antigen binding molecule of any one of claims 1 to 3 or 12, wherein Fab domain capable of specific binding to CEA comprises a VH domain comprising (i) CDR-H1 comprising the amino acid sequence of SEQ ID NO:45, (ii) CDR-H2 comprising the amino acid sequence of SEQ ID NO:46 and (iii) CDR-H3 comprising the amino acid sequence of SEQ ID NO:47, and a VL domain comprising (iv) CDR-L1 comprising the amino acid sequence of SEQ ID NO:48, (v) CDR-L2 comprising the amino acid sequence of SEQ ID NO:49 and (vi) CDR-L3 comprising the amino acid sequence of SEQ ID NO:50.

**14.** The TNF family ligand trimer-containing antigen binding molecule of any one of claims 1 to 3, 12 or 13, wherein the Fab domain capable of specific binding to CEA comprises a variable heavy chain comprising an amino acid sequence of SEQ ID NO:51 and a variable light chain comprising an amino acid sequence of SEQ ID NO:52.

**15.** The TNF family ligand trimer-containing antigen binding molecule of any one of claims 1 to 14, wherein the Fc domain composed of a first and a second subunit capable of stable association is an IgG domain, in particular an IgG1 domain.

**16.** The TNF family ligand trimer-containing antigen binding molecule of any one of claims 1 to 15, wherein the Fc domain comprises one or more amino acid substitution that reduces binding to an Fc receptor, in particular towards Fcγ receptor.

**17.** The TNF family ligand trimer-containing antigen binding molecule of any one of claims 1 to 16, wherein the Fc domain is an IgG1 Fc domain comprising amino acid substitutions at positions 234 and 235 (numbering according to Kabat EU index) and/or 329 (numbering according to Kabat EU index).

**18.** An isolated polynucleotide encoding the 4-1BBL trimer-containing antigen binding molecule of any one of claims 1 to 17.

**19.** A vector, particularly an expression vector, comprising the isolated polynucleotide of claim 18.

**20.** A host cell comprising the isolated polynucleotide of claim 18 or the vector of claim 19.

**21.** A method for producing the 4-1BBL trimer-containing antigen binding molecule of any one of claims 1 to 17, comprising culturing the host cell of claim 20 under conditions suitable for expression of the antigen binding molecule and recovering the antigen binding molecule.

**22.** A pharmaceutical composition comprising the 4-1BBL trimer-containing antigen binding molecule of any one of claims 1 to 17 and at least one pharmaceutically acceptable excipient.

**23.** The 4-1BBL trimer-containing antigen binding molecule of any one of claims 1 to 17, or the pharmaceutical composition of claim 22, for use as a medicament.

**24.** The 4-1BBL trimer-containing antigen binding molecule of any one of claims 1 to 17, or the pharmaceutical composition of claim 22, for use in the treatment of cancer.

## Patentansprüche

**1.** 4-1BB-Liganden-(4-1BBL)-Trimer enthaltendes antigenbindendes Molekül, umfassend

(a) eine Fab-Domäne, die spezifisch an ein Zielzellenantigen binden kann,
(b) eine Fc-Domäne, bestehend aus einer ersten und einer zweiten Untereinheit, die zu stabiler Assoziation fähig sind, und
(c) ein erstes Polypeptid, umfassend zwei Ektodomänen von 4-1BBL, umfassend die Aminosäuresequenz von SEQ ID NO:5, die durch einen Peptid-Linker miteinander verbunden sind, und ein zweites Polypeptid, umfassend eine Ektodomäne des 4-1BBL, umfassend die Aminosäuresequenz von SEQ ID NO:5, wobei das erste Polypeptid an seinem N-Terminus an den C-Terminus der ersten Untereinheit der Fc-Domäne, umfassend die Aminosäuresubstitutionen Y349C, T366S, L368A und Y407V (Nummerierung gemäß Kabat-EU-Index) des Lochs, fusioniert ist und wobei das zweite Polypeptid an seinem N-Terminus an den C-Terminus der zweiten Untereinheit der Fc-Domäne, umfassend die Aminosäuresubstitutionen S354C und T366W (Nummerierung gemäß Kabat-EU-Index) des Knopfs, fusioniert ist und wobei die VH- und CH1-Domäne der Fab-Domäne, die spezifisch an ein Zielzellenantigen binden kann, am C-Terminus an den N-Terminus der zweiten Untereinheit der Fc-Domäne, umfassend die Knopf-Mutationen, fusioniert ist,

wobei das 4-1BBL-Trimer enthaltende antigenbindende Molekül für die Bindung an das Zielzellenantigen monovalent ist.

**2.** 4-1BBL-Trimer enthaltendes antigenbindendes Molekül nach Anspruch 1, wobei 4-1BBL humane T-Zellenaktivierung costimuliert.

**3.** 4-1BBL-Trimer enthaltendes antigenbindendes Molekül nach den Ansprüchen 1 oder 2, wobei das Zielzellenantigen ausgewählt ist aus der Gruppe, bestehend aus Fibroblasten-Aktivierungsprotein (FAP), Melanom-assoziiertem Chondroitinsulfatproteoglycan (MCSP), epidermalem Wachstumsfaktorrezeptor (EGFR), karzinoembryonalem Antigen (CEA), CD19, CD20 und CD33.

**4.** 4-1BBL-Trimer enthaltendes antigenbindendes Molekül nach einem der Ansprüche 1 bis 3, wobei das Zielzellenantigen Fibroblasten-Aktivierungsprotein (FAP) ist.

**5.** 4-1BBL-Trimer enthaltendes antigenbindendes Molekül nach einem der Ansprüche 1 bis 4, wobei die Fab-Domäne, die spezifisch an FAP binden kann,

(a) eine VH-Domäne, umfassend (i) CDR-H1, umfassend die Aminosäuresequenz von SEQ ID NO:13, (ii) CDR-H2, umfassend die Aminosäuresequenz von SEQ ID NO:14, und (iii) CDR-H3, umfassend die Aminosäuresequenz von SEQ ID NO:15, und eine VL-Domäne, umfassend (iv) CDR-L1, umfassend die Aminosäuresequenz von SEQ ID NO:16, (v) CDR-L2, umfassend die Aminosäuresequenz von SEQ ID NO:17, und (vi) CDR-L3, umfassend die Aminosäuresequenz von SEQ ID NO:18, oder
(b) eine VH-Domäne, umfassend (i) CDR-H1, umfassend die Aminosäuresequenz von SEQ ID NO:19, (ii) CDR-H2, umfassend die Aminosäuresequenz von SEQ ID NO:20, und (iii) CDR-H3, umfassend die Aminosäuresequenz von SEQ ID NO:21, und eine VL-Domäne, umfassend (iv) CDR-L1, umfassend die Aminosäuresequenz von SEQ ID NO:22, (v) CDR-L2, umfassend die Aminosäuresequenz von SEQ ID NO:23, und (vi) CDR-L3, umfassend die Aminosäuresequenz von SEQ ID NO:24, umfasst.

**6.** 4-1BBL-Trimer enthaltendes antigenbindendes Molekül nach einem der Ansprüche 1 bis 5, wobei die Fab-Domäne, die spezifisch an FAP binden kann, eine variable schwere Kette, umfassend eine Aminosäuresequenz von SEQ ID NO:25, und eine variable leichte Kette, umfassend eine Aminosäuresequenz von SEQ ID NO:26, umfasst oder wobei die Fab-Domäne, die spezifisch an FAP binden kann, eine variable schwere Kette, umfassend eine Aminosäuresequenz von SEQ ID NO:27, und eine variable leichte Kette, umfassend eine Aminosäuresequenz von SEQ ID NO:28, umfasst.

**7.** 4-1BBL-Trimer enthaltendes antigenbindendes Molekül nach einem der Ansprüche 1 bis 6, umfassend

(i) eine leichte Kette, umfassend die Aminosäuresequenz von SEQ ID NO:106,
(ii) ein Fusionspolypeptid, umfassend die Aminosäuresequenz von SEQ ID NO:104, und
(iii) eine schwere Kette, umfassend die Aminosäuresequenz von SEQ ID NO:105.

**8.** Ligandentrimer der TNF-Familie enthaltendes antigenbindendes Molekül nach einem der Ansprüche 1 bis 3, wobei das Zielzellenantigen CD19 ist.

**9.** Ligandentrimer der TNF-Familie enthaltendes antigenbindendes Molekül nach einem der Ansprüche 1 bis 3 oder 8, wobei die Fab-Domäne, die spezifisch an CD19 binden kann,

(a) eine VH-Domäne, umfassend (i) CDR-H1, umfassend die Aminosäuresequenz von SEQ ID NO:29, (ii) CDR-H2, umfassend die Aminosäuresequenz von SEQ ID NO:30, und (iii) CDR-H3, umfassend die Aminosäuresequenz von SEQ ID NO:31, und eine VL-Domäne, umfassend (iv) CDR-L1, umfassend die Aminosäuresequenz von SEQ ID NO:32, (v) CDR-L2, umfassend die Aminosäuresequenz von SEQ ID NO:33, und (vi) CDR-L3, umfassend die Aminosäuresequenz von SEQ ID NO:34, oder
(b) eine VH-Domäne, umfassend (i) CDR-H1, umfassend die Aminosäuresequenz von SEQ ID NO:35, (ii) CDR-H2, umfassend die Aminosäuresequenz von SEQ ID NO:36, und (iii) CDR-H3, umfassend die Aminosäuresequenz von SEQ ID NO:37, und eine VL-Domäne, umfassend (iv) CDR-L1, umfassend die Aminosäuresequenz von SEQ ID NO:38, (v) CDR-L2, umfassend die Aminosäuresequenz von SEQ ID NO:39, und (vi) CDR-L3, umfassend die Aminosäuresequenz von SEQ ID NO:40, umfasst.

**10.** Ligandentrimer der TNF-Familie enthaltendes antigenbindendes Molekül nach einem der Ansprüche 1 bis 3, 8 oder 9, wobei die Fab-Domäne, die spezifisch an CD19 binden kann, eine variable schwere Kette, umfassend eine Aminosäuresequenz von SEQ ID NO:41, und eine variable leichte Kette, umfassend eine Aminosäuresequenz von SEQ ID NO:42, umfasst oder wobei die Fab-Domäne, die spezifisch an FAP binden kann, eine variable schwere Kette, umfassend eine Aminosäuresequenz von SEQ ID NO:43, und eine variable leichte Kette, umfassend eine Aminosäuresequenz von SEQ ID NO:44, umfasst.

**11.** 4-1BBL-Trimer enthaltendes antigenbindendes Molekül nach einem der Ansprüche 1 bis 3 oder 8 bis 10, umfassend

(i) eine leichte Kette, umfassend die Aminosäuresequenz von SEQ ID NO:120,
(ii) ein Fusionspolypeptid, umfassend die Aminosäuresequenz von SEQ ID NO:104, und
(iii) eine schwere Kette, umfassend die Aminosäuresequenz von SEQ ID NO:119, oder

(i) eine leichte Kette, umfassend die Aminosäuresequenz von SEQ ID NO:126,
(ii) ein Fusionspolypeptid, umfassend die Aminosäuresequenz von SEQ ID NO:174, und
(iii) eine schwere Kette, umfassend die Aminosäuresequenz von SEQ ID NO:125.

**12.** Ligandentrimer der TNF-Familie enthaltendes antigenbindendes Molekül nach einem der Ansprüche 1 bis 3, wobei das Zielzellenantigen CEA ist.

**13.** Ligandentrimer der TNF-Familie enthaltendes antigenbindendes Molekül nach einem der Ansprüche 1 bis 3 oder 12, wobei die Fab-Domäne, die spezifisch an CEA binden kann, eine VH-Domäne, umfassend (i) CDR-H1, umfassend die Aminosäuresequenz von SEQ ID NO:45, (ii) CDR-H2, umfassend die Aminosäuresequenz von SEQ ID NO:46, und (iii) CDR-H3, umfassend die Aminosäuresequenz von SEQ ID NO:47, und eine VL-Domäne, umfassend (iv) CDR-L1, umfassend die Aminosäuresequenz von SEQ ID NO:48, (v) CDR-L2, umfassend die Aminosäuresequenz von SEQ ID NO:49, und (vi) CDR-L3, umfassend die Aminosäuresequenz von SEQ ID NO:50, umfasst.

**14.** Ligandentrimer der TNF-Familie enthaltendes antigenbindendes Molekül nach einem der Ansprüche 1 bis 3, 12 oder 13, wobei die Fab-Domäne, die spezifisch an CEA binden kann, eine variable schwere Kette, umfassend eine Aminosäuresequenz von SEQ ID NO:51, und eine variable leichte Kette, umfassend eine Aminosäuresequenz von SEQ ID NO:52, umfasst.

**15.** Ligandentrimer der TNF-Familie enthaltendes antigenbindendes Molekül nach einem der Ansprüche 1 bis 14, wobei die Fc-Domäne, bestehend aus einer ersten und einer zweiten Untereinheit, die zu stabiler Assoziation fähig sind, eine IgG-Domäne, insbesondere eine IgGl-Domäne ist.

**16.** Ligandentrimer der TNF-Familie enthaltendes antigenbindendes Molekül nach einem der Ansprüche 1 bis 15, wobei die Fc-Domäne ein oder mehrere Aminosäuresubstitutionen umfasst, die das Binden an einen Fc-Rezeptor, insbesondere an einen Fcγ-Rezeptor, verringert.

**17.** Ligandentrimer der TNF-Familie enthaltendes antigenbindendes Molekül nach einem der Ansprüche 1 bis 16, wobei die Fc-Domäne eine IgG1-Fc-Domäne ist, die Aminosäuresubstitutionen an Positionen 234 und 235 (Nummerierung gemäß Kabat-EU-Index) und/oder 329 (Nummerierung gemäß Kabat-EU-Index) umfasst.

**18.** Isoliertes Polynukleotid, das das 4-1BBL-Trimer enthaltende antigenbindende Molekül nach einem der Ansprüche 1 bis 17 kodiert.

**19.** Vektor, insbesondere ein Expressionsvektor, umfassend das isolierte Polynukleotid nach Anspruch 18.

**20.** Wirtszelle, umfassend das isolierte Polynukleotid nach Anspruch 18 oder den Vektor nach Anspruch 19.

**21.** Verfahren zum Produzieren des 4-1BBL-Trimer enthaltenden antigenbindenden Moleküls nach einem der Ansprüche 1 bis 17, umfassend das Kultivieren der Wirtszelle nach Anspruch 20, unter Bedingungen, die für die Expression des antigenbindenden Moleküls geeignet sind, und Gewinnen des antigenbindenden Moleküls.

**22.** Pharmazeutische Zusammensetzung, umfassend das 4-1BBL-Trimer enthaltende antigenbindende Molekül nach einem der Ansprüche 1 bis 17 und mindestens einen pharmazeutisch unbedenklichen Hilfsstoff.

**23.** 4-1BBL-Trimer enthaltendes antigenbindendes Molekül nach einem der Ansprüche 1 bis 17 oder pharmazeutische Zusammensetzung nach Anspruch 22 zur Verwendung als ein Medikament.

**24.** 4-1BBL-Trimer enthaltendes antigenbindendes Molekül nach einem der Ansprüche 1 bis 17 oder pharmazeutische Zusammensetzung nach Anspruch 22 zur Verwendung bei der Behandlung von Krebs.

## Revendications

**1.** Molécule de liaison à un antigène contenant un trimère d'un ligand 4-1BB (4-1BBL) comprenant

(a) un domaine Fab capable de se lier spécifiquement à un antigène de cellule cible,
(b) un domaine Fc composé d'un premier et d'un second sous-motif capables d'une association stable, et
(c) un premier polypeptide comprenant deux ectodomaines de 4-1BBL comprenant la séquence d'acides aminés de SEQ ID NO : 5 qui sont connectés l'un à l'autre par un lieur peptidique et un second polypeptide comprenant un ectodomaine dudit 4-1BBL comprenant la séquence d'acides aminés de SEQ ID NO : 5, dans laquelle le premier polypeptide est fusionné au niveau de sa terminaison N à la terminaison C du premier sous-motif du domaine Fc comprenant les substitutions d'acides aminés Y349C, T366S, L368A et Y407V (numérotation selon l'indice EU de Kabat) du creux et dans laquelle le second polypeptide est fusionné au niveau de sa terminaison N à la terminaison C du second sous-motif du domaine Fc comprenant les substitutions d'acides aminés S354C et T366W (numérotation selon l'indice EU de Kabat) de la bosse et dans laquelle le domaine VH et CH1 du domaine Fab capable de se lier spécifiquement à un antigène de cellule cible est fusionné au niveau de la terminaison C à la terminaison N du second sous-motif du domaine Fc comprenant les mutations de la bosse,

dans laquelle la molécule se liant à un antigène contenant un trimère de 4-1BBL est monovalente pour la liaison à l'antigène de cellule cible.

2. Molécule de liaison à un antigène contenant un trimère de 4-1BBL selon la revendication 1, dans laquelle le 4-1BBL costimule l'activation des lymphocytes T humains.

3. Molécule de liaison à un antigène contenant un trimère de 4-1BBL selon les revendications 1 ou 2, dans laquelle l'antigène de cellule cible est choisi dans le groupe constitué par la protéine d'activation des fibroblastes (FAP), le protéoglycane de sulfate de chondroïtine associé à un mélanome (MCSP), un récepteur de facteur de croissance épidermique (EGFR), un antigène carcino-embryonnaire (ACE), CD19, CD20 et CD33.

4. Molécule de liaison à un antigène contenant un trimère de 4-1BBL selon l'une quelconque des revendications 1 à 3, dans laquelle l'antigène de cellule cible est la protéine d'activation des fibroblastes (FAP).

5. Molécule de liaison à un antigène contenant un trimère de 4-1BBL selon l'une quelconque des revendications 1 à 4, dans laquelle le domaine Fab capable de se lier spécifiquement à la FAP comprend

(a) un domaine VH comprenant (i) une CDR-H1 comprenant la séquence d'acides aminés de SEQ ID NO : 13, (ii) une CDR-H2 comprenant la séquence d'acides aminés de SEQ ID NO : 14 et (iii) une CDR-H3 comprenant la séquence d'acides aminés de SEQ ID NO : 15, et un domaine VL comprenant (iv) une CDR-L1 comprenant la séquence d'acides aminés de SEQ ID NO : 16, (v) une CDR-L2 comprenant la séquence d'acides aminés de SEQ ID NO : 17 et (vi) une CDR-L3 comprenant la séquence d'acides aminés de SEQ ID NO : 18 ou
(b) un domaine VH comprenant (i) une CDR-H1 comprenant la séquence d'acides aminés de SEQ ID NO : 19, (ii) une CDR-H2 comprenant la séquence d'acides aminés de SEQ ID NO : 20 et (iii) une CDR-H3 comprenant la séquence d'acides aminés de SEQ ID NO : 21, et un domaine VL comprenant (iv) une CDR-L1 comprenant la séquence d'acides aminés de SEQ ID NO : 22, (v) une CDR-L2 comprenant la séquence d'acides aminés de SEQ ID NO : 23 et (vi) une CDR-L3 comprenant la séquence d'acides aminés de SEQ ID NO : 24.

6. Molécule de liaison à un antigène contenant un trimère de 4-1BBL selon l'une quelconque des revendications 1 à 5, dans laquelle le domaine Fab capable de se lier spécifiquement à la FAP comprend une chaîne lourde variable comprenant une séquence d'acides aminés de SEQ ID NO : 25 et une chaîne légère variable comprenant une séquence d'acides aminés de SEQ ID NO : 26 ou dans laquelle le domaine Fab capable de se lier spécifiquement à la FAP comprend une chaîne lourde variable comprenant une séquence d'acides aminés de SEQ ID NO : 27 et une chaîne légère variable comprenant une séquence d'acides aminés de SEQ ID NO : 28.

7. Molécule de liaison à un antigène contenant un trimère de 4-1BBL selon l'une quelconque des revendications 1 à 6, comprenant

(i) une chaîne légère comprenant la séquence d'acides aminés de SEQ ID NO : 106,
(ii) un polypeptide de fusion comprenant la séquence d'acides aminés de SEQ ID NO : 104, et
(iii) une chaîne lourde comprenant la séquence d'acides aminés de SEQ ID NO : 105.

8. Molécule de liaison à un antigène contenant un trimère d'un ligand de la famille du TNF selon l'une quelconque des revendications 1 à 3, dans laquelle l'antigène de cellule cible est CD19.

9. Molécule de liaison à un antigène contenant un trimère d'un ligand de la famille du TNF selon l'une quelconque des revendications 1 à 3 ou 8, dans laquelle le domaine Fab capable de se lier spécifiquement à CD 19 comprend

(a) un domaine VH comprenant (i) une CDR-H1 comprenant la séquence d'acides aminés de SEQ ID NO : 29, (ii) une CDR-H2 comprenant la séquence d'acides aminés de SEQ ID NO : 30 et (iii) une CDR-H3 comprenant la séquence d'acides aminés de SEQ ID NO : 31, et un domaine VL comprenant (iv) une CDR-L1 comprenant la séquence d'acides aminés de SEQ ID NO : 32, (v) une CDR-L2 comprenant la séquence d'acides aminés de SEQ ID NO : 33 et (vi) une CDR-L3 comprenant la séquence d'acides aminés de SEQ ID NO : 34 ou
(b) un domaine VH comprenant (i) une CDR-H1 comprenant la séquence d'acides aminés de SEQ ID NO : 35, (ii) une CDR-H2 comprenant la séquence d'acides aminés de SEQ ID NO : 36 et (iii) une CDR-H3 comprenant la séquence d'acides aminés de SEQ ID NO : 37, et un domaine VL comprenant (iv) une CDR-L1 comprenant la séquence d'acides aminés de SEQ ID NO : 38, (v) une CDR-L2 comprenant la séquence d'acides aminés de SEQ ID NO : 39 et (vi) une CDR-L3 comprenant la séquence d'acides aminés de SEQ ID NO : 40.

10. Molécule de liaison à un antigène contenant un trimère d'un ligand de la famille du TNF selon l'une quelconque des revendications 1 à 3, 8 ou 9, dans laquelle le domaine Fab capable de se lier spécifiquement à CD19 comprend une chaîne lourde variable comprenant une séquence d'acides aminés de SEQ ID NO : 41 et une chaîne légère variable comprenant une séquence d'acides aminés de SEQ ID NO : 42 ou dans laquelle le domaine Fab capable de se lier spécifiquement à la FAP comprend une chaîne lourde variable comprenant une séquence d'acides aminés de SEQ ID NO : 43 et une chaîne légère variable comprenant une séquence d'acides aminés de SEQ ID NO : 44.

**11.** Molécule de liaison à un antigène contenant un trimère de 4-1BBL selon l'une quelconque des revendications 1 à 3 ou 8 à 10, comprenant

(i) une chaîne légère comprenant la séquence d'acides aminés de SEQ ID NO : 120,
(ii) un polypeptide de fusion comprenant la séquence d'acides aminés de SEQ ID NO : 104, et
(iii) une chaîne lourde comprenant la séquence d'acides aminés de SEQ ID NO : 119, ou

(i) une chaîne légère comprenant la séquence d'acides aminés de SEQ ID NO : 126,
(ii) un polypeptide de fusion comprenant la séquence d'acides aminés de SEQ ID NO : 174, et
(iii) une chaîne lourde comprenant la séquence d'acides aminés de SEQ ID NO : 125.

**12.** Molécule de liaison à un antigène contenant un trimère d'un ligand de la famille du TNF selon l'une quelconque des revendications 1 à 3, dans laquelle l'antigène de cellule cible est un ACE.

**13.** Molécule de liaison à un antigène contenant un trimère d'un ligand de la famille du TNF selon l'une quelconque des revendications 1 à 3 ou 12, dans laquelle le domaine Fab capable de se lier spécifiquement à un ACE comprend un domaine VH comprenant (i) une CDR-H1 comprenant la séquence d'acides aminés de SEQ ID NO : 45, (ii) une CDR-H2 comprenant la séquence d'acides aminés de SEQ ID NO : 46 et (iii) une CDR-H3 comprenant la séquence d'acides aminés de SEQ ID NO : 47, et un domaine VL comprenant (iv) une CDR-L1 comprenant la séquence d'acides aminés de SEQ ID NO : 48, (v) une CDR-L2 comprenant la séquence d'acides aminés de SEQ ID NO : 49 et (vi) une CDR-L3 comprenant la séquence d'acides aminés de SEQ ID NO : 50.

**14.** Molécule de liaison à un antigène contenant un trimère d'un ligand de la famille du TNF selon l'une quelconque des revendications 1 à 3, 12 ou 13, dans laquelle le domaine Fab capable de se lier spécifiquement à un ACE comprend une région variable de chaîne lourde comprenant une séquence d'acides aminés de SEQ ID NO : 51 et une région variable de chaîne légère comprenant une séquence d'acides aminés de SEQ ID NO : 52.

**15.** Molécule de liaison à un antigène contenant un trimère d'un ligand de la famille du TNF selon l'une quelconque des revendications 1 à 14, dans laquelle le domaine Fc composé d'un premier et d'un second sous-motif capables d'association stable est un domaine d'IgG, en particulier un domaine d'IgG1.

**16.** Molécule de liaison à un antigène contenant un trimère d'un ligand de la famille du TNF selon l'une quelconque des revendications 1 à 15, dans laquelle le domaine Fc comprend une ou plusieurs substitutions d'acides aminés qui réduisent la liaison à un récepteur Fc, en particulier vis-à-vis du récepteur Fcγ.

**17.** Molécule de liaison à un antigène contenant un trimère d'un ligand de la famille du TNF selon l'une quelconque des revendications 1 à 16, dans laquelle le domaine Fc est un domaine Fc d'IgG1 comprenant des substitutions d'acides aminés en positions 234 et 235 (numérotation selon l'indice EU de Kabat) et/ou 329 (numérotation selon l'indice EU de Kabat).

**18.** Polynucléotide isolé codant pour la molécule de liaison à un antigène contenant un trimère de 4-1BBL selon l'une quelconque des revendications 1 à 17.

**19.** Vecteur, en particulier un vecteur d'expression, comprenant le polynucléotide isolé selon la revendication 18.

**20.** Cellule hôte comprenant le polynucléotide isolé selon la revendication 18 ou le vecteur selon la revendication 19.

**21.** Procédé de production de la molécule de liaison à un antigène contenant un trimère de 4-1BBL selon l'une quelconque des revendications 1 à 17, comprenant la culture de la cellule hôte selon la revendication 20 dans des conditions appropriées pour l'expression de la molécule de liaison à un antigène et la récupération de la molécule de liaison à un antigène.

**22.** Composition pharmaceutique comprenant la molécule de liaison à un antigène contenant un trimère de 4-1BBL selon l'une quelconque des revendications 1 à 17 et au moins un excipient pharmaceutiquement acceptable.

**23.** Molécule de liaison à un antigène contenant un trimère de 4-1BBL selon l'une quelconque des revendications 1 à 17, ou composition pharmaceutique selon la revendication 22, pour une utilisation comme médicament.

**24.** Molécule de liaison à un antigène contenant un trimère de 4-1BBL selon l'une quelconque des revendications 1 à 17, ou composition pharmaceutique selon la revendication 22, pour une utilisation dans le traitement d'un cancer.

Fig. 1

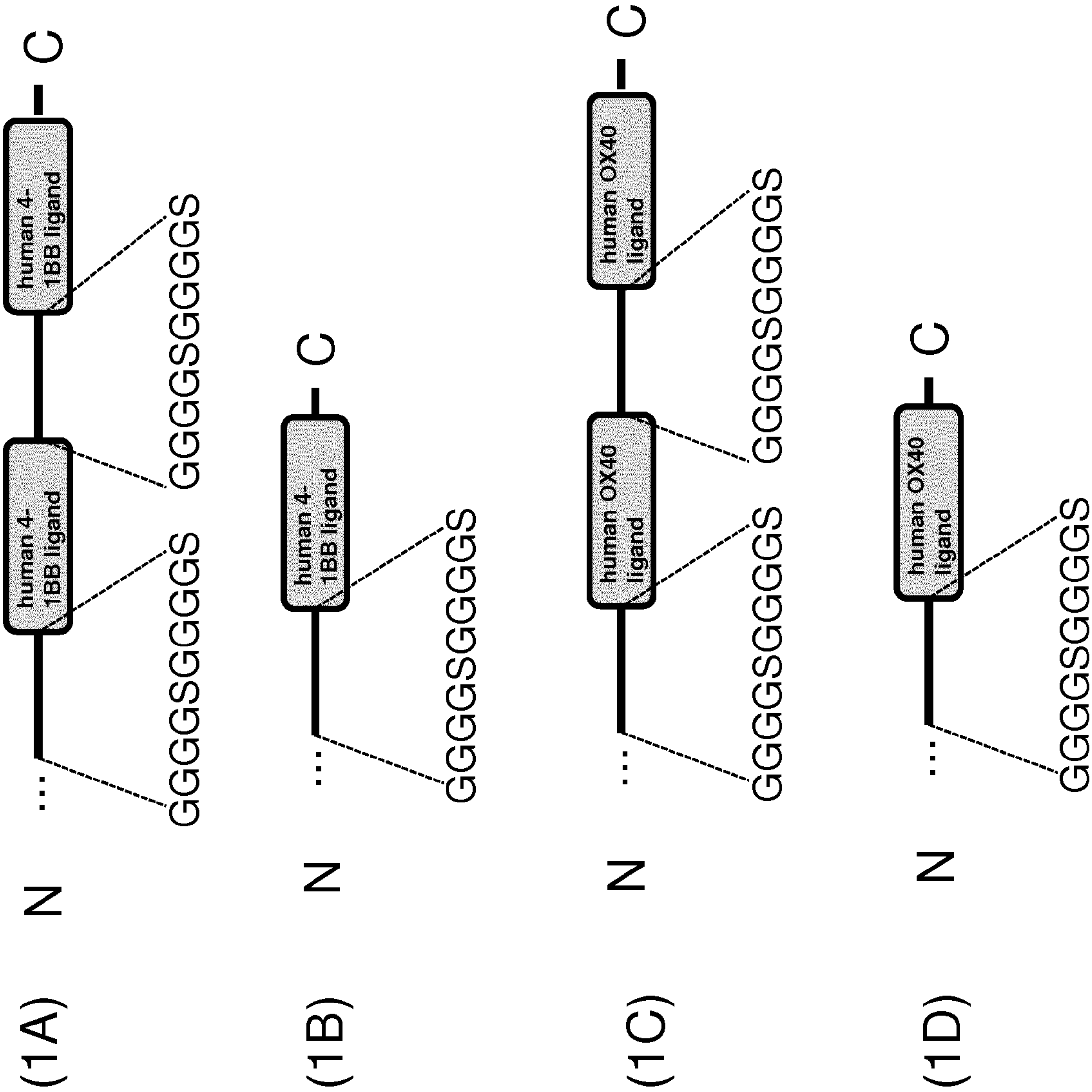
(1A)
N
C
human 4-1BB ligand
human 4-1BB ligand
GGGGSGGGGS
GGGGSGGGGS
(1B)
N
C
human 4-1BB ligand
GGGGSGGGGS
(1C)
N
C
human OX40 ligand
human OX40 ligand
GGGGSGGGGS
GGGGSGGGGS
(1D)
N
C
human OX40 ligand
GGGGSGGGGS

Fig. 2

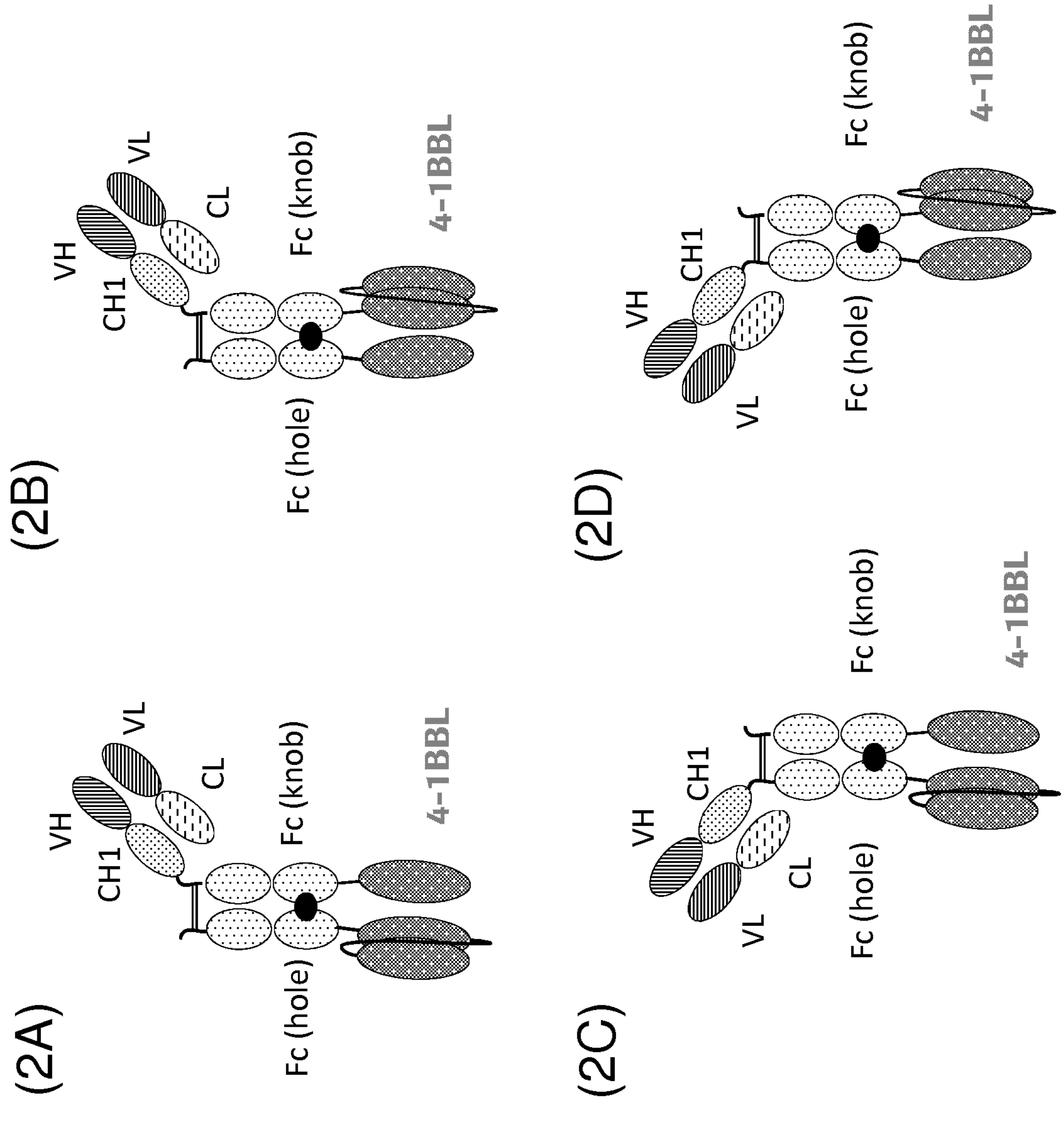

Fig. 3

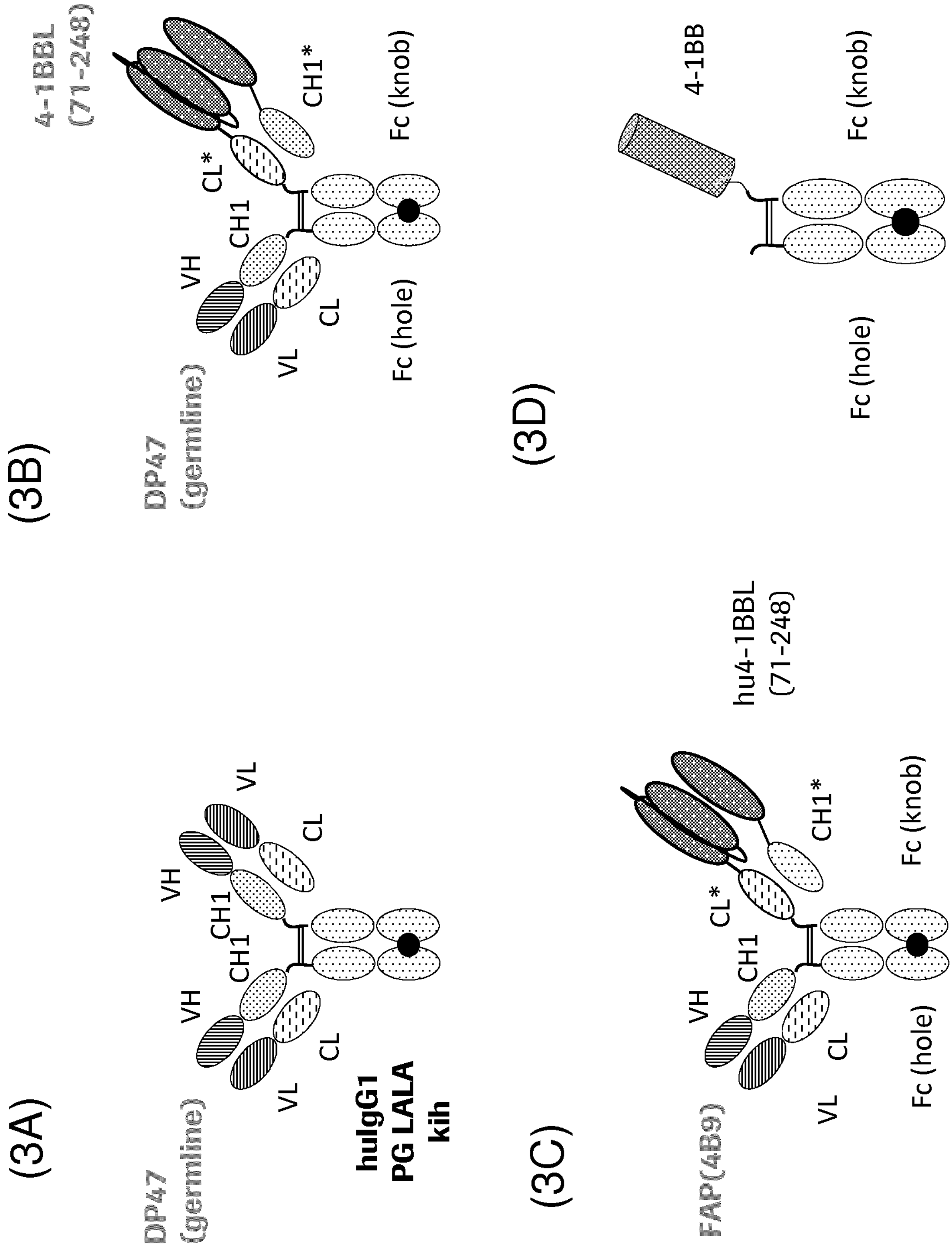
(3A)
DP47
(germline)
hulgG1
PG LALA
kih
VL
VH
CH1
CL
CH1
VH
CL
VL
(3B)
DP47
(germline)
VL
VH
CH1
CL
CL*
CH1*
4-1BBL
(71-248)
Fc (hole)
Fc (knob)
(3C)
FAP(4B9)
VL
VH
CH1
CL
CL*
CH1*
hu4-1BBL
(71-248)
Fc (hole)
Fc (knob)
(3D)
4-1BB
Fc (hole)
Fc (knob)

Fig. 4

(4A) (4B)

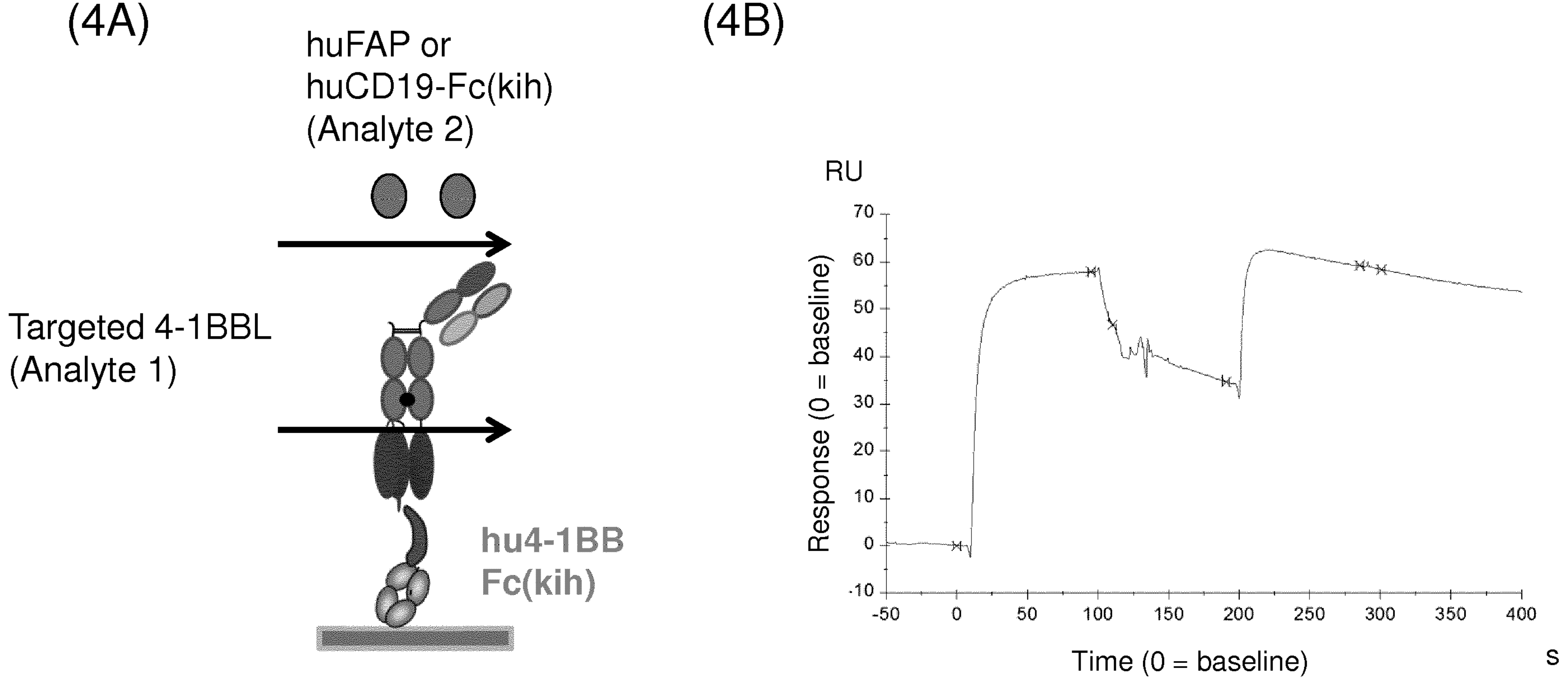
huFAP or
huCD19-Fc(kih)
(Analyte 2)
Targeted 4-1BBL
(Analyte 1)
hu4-1BB
Fc(kih)
RU
Response (0 = baseline)
70
60
50
40
30
20
10
0
-10
-50
0
50
100
150
200
250
300
350
400
Time (0 = baseline)
s

Fig. 4

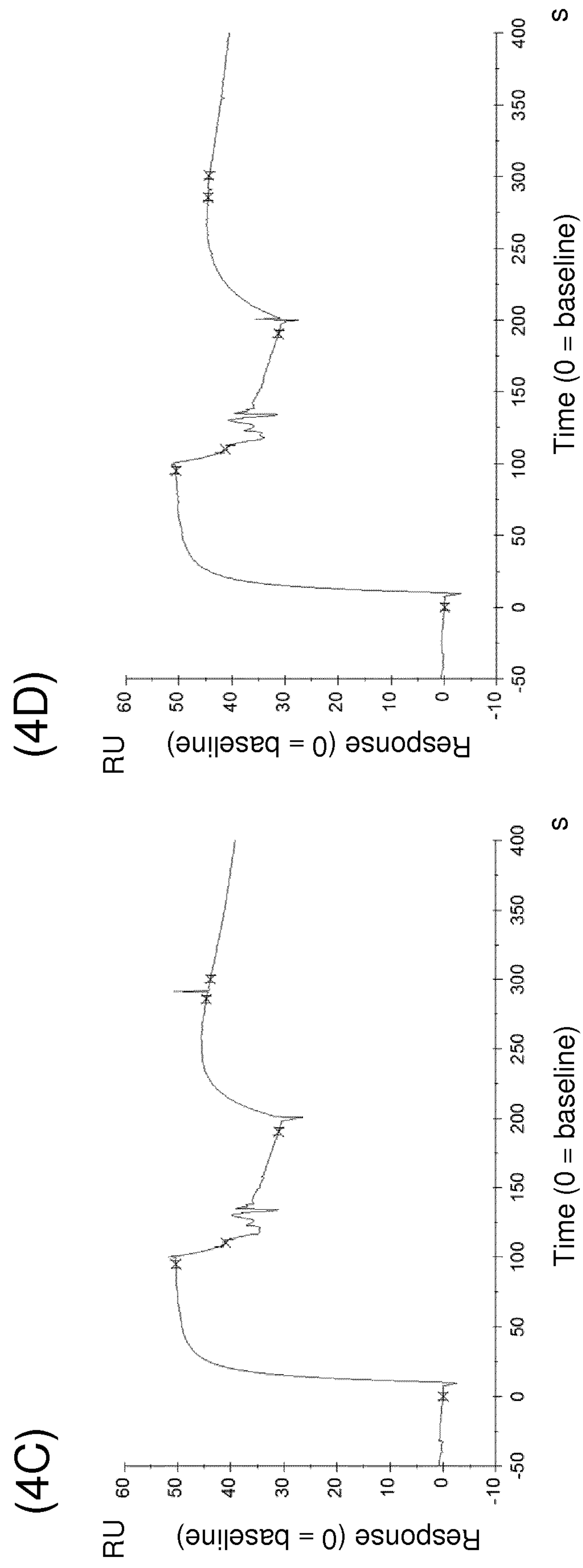
(4C)
RU
Response (0 = baseline)
60
50
40
30
20
10
0
-10
-50
0
50
100
150
200
250
300
350
400
s
Time (0 = baseline)
(4D)
RU
Response (0 = baseline)
60
50
40
30
20
10
0
-10
-50
0
50
100
150
200
250
300
350
400
s
Time (0 = baseline)

Fig. 5

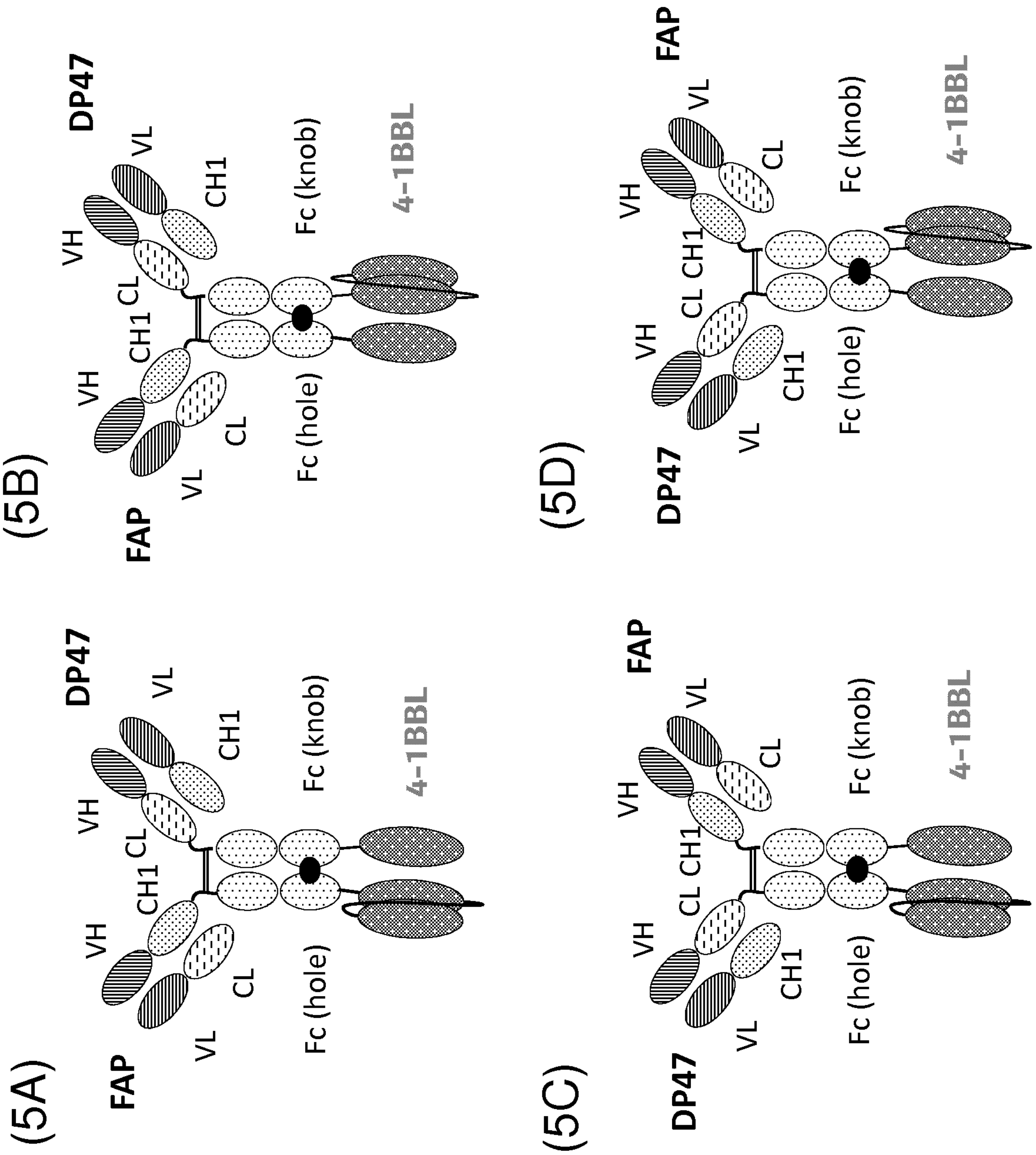
(5A)
FAP
DP47
VH
VL
CH1
CL
VH
VL
CL
CH1
Fc (hole)
Fc (knob)
4-1BBL
(5B)
FAP
DP47
VH
VL
CH1
CL
VH
VL
CL
CH1
Fc (hole)
Fc (knob)
4-1BBL
(5C)
DP47
FAP
VH
VL
CL
CH1
VH
VL
CH1
CL
Fc (hole)
Fc (knob)
4-1BBL
(5D)
DP47
FAP
VH
VL
CL
CH1
VH
VL
CH1
CL
Fc (hole)
Fc (knob)
4-1BBL

Fig. 6

(6A)

(6B)

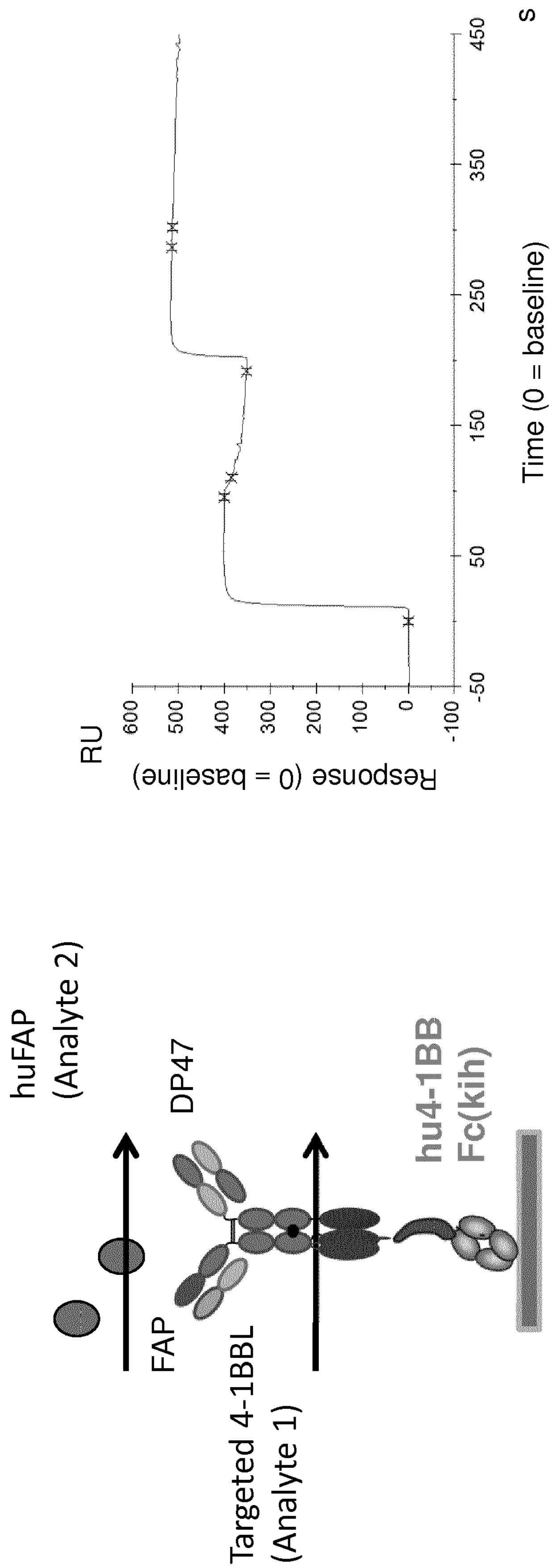
huFAP
(Analyte 2)
DP47
FAP
Targeted 4-1BBL
(Analyte 1)
hu4-1BB
Fc(kih)
RU
Response (0 = baseline)
600
500
400
300
200
100
0
-100
-50
50
150
250
350
450
s
Time (0 = baseline)

Fig. 7

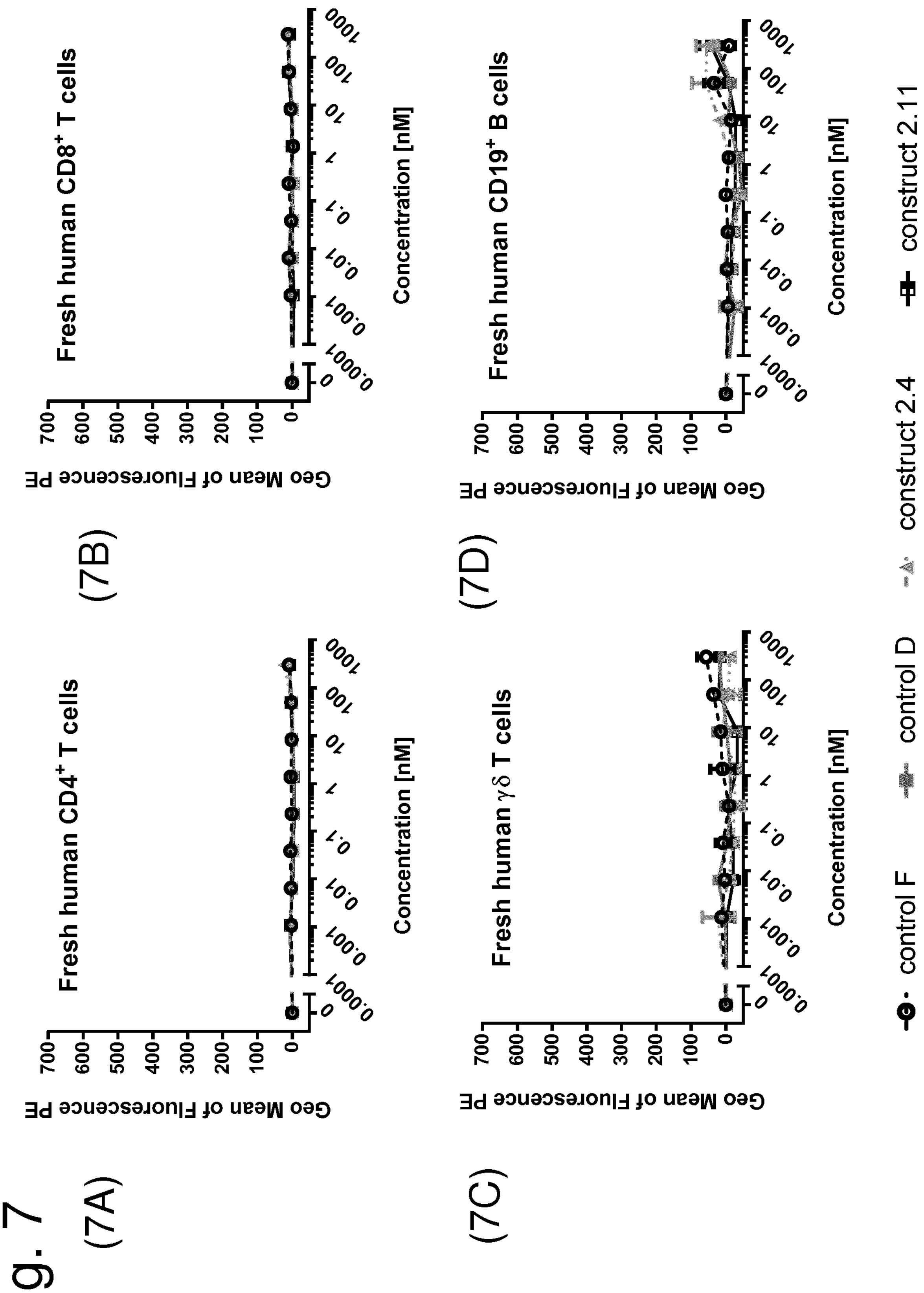
(7A)
Fresh human CD4[+] T cells
(7B)
Fresh human CD8[+] T cells
(7C)
Fresh human γδ T cells
(7D)
Fresh human CD19[+] B cells
Geo Mean of Fluorescence PE
700
600
500
400
300
200
100
0
Concentration [nM]
0
0.0001
0.001
0.01
0.1
1
10
100
1000
control F
control D
construct 2.4
construct 2.11

Fig. 7

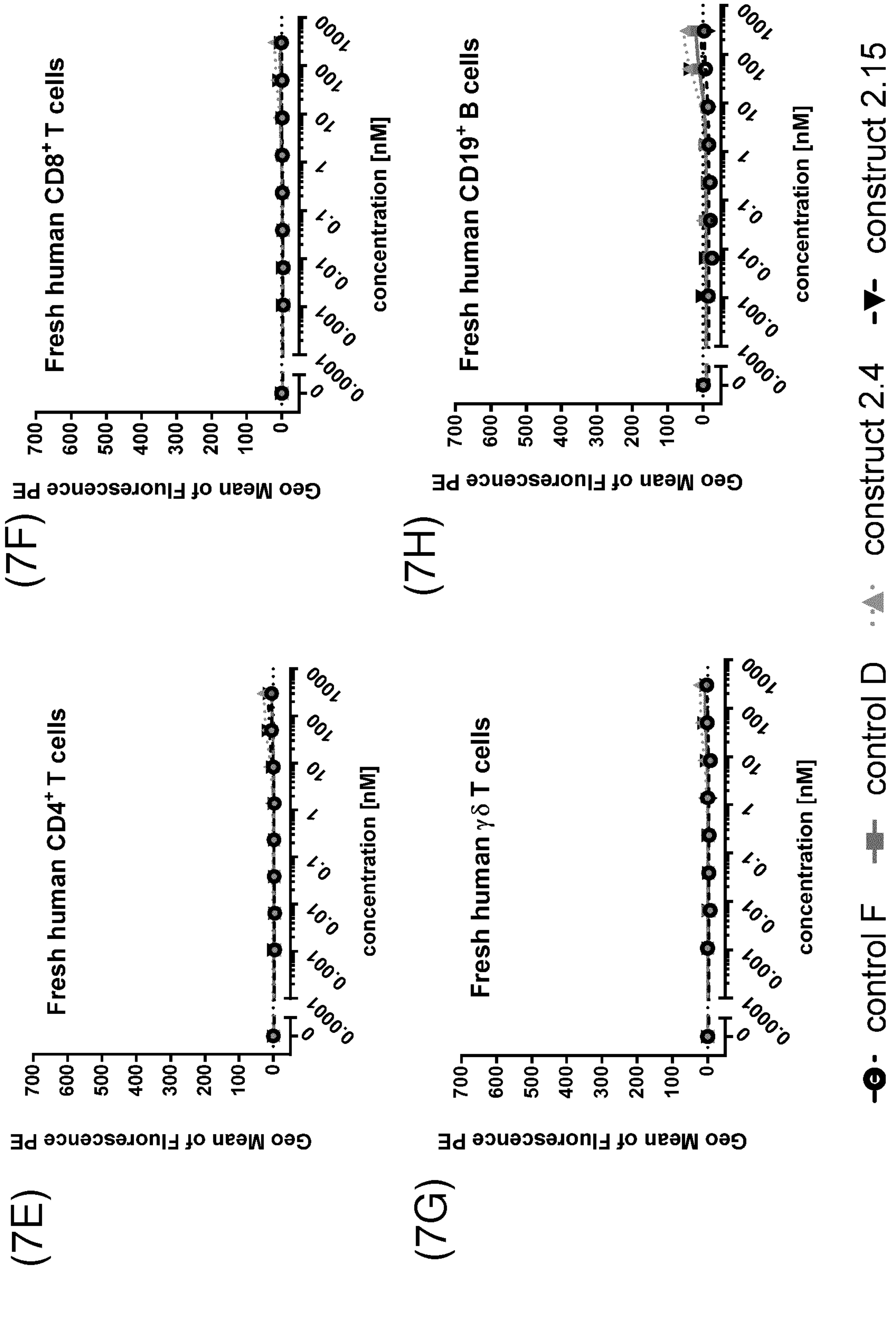

(7E)
Fresh human CD4+ T cells
(7F)
Fresh human CD8+ T cells
(7G)
Fresh human γδ T cells
(7H)
Fresh human CD19+ B cells
Geo Mean of Fluorescence PE
700
600
500
400
300
200
100
0
0
0.0001
0.001
0.01
0.1
1
10
100
1000
concentration [nM]
control F
control D
construct 2.4
construct 2.15

Fig. 8

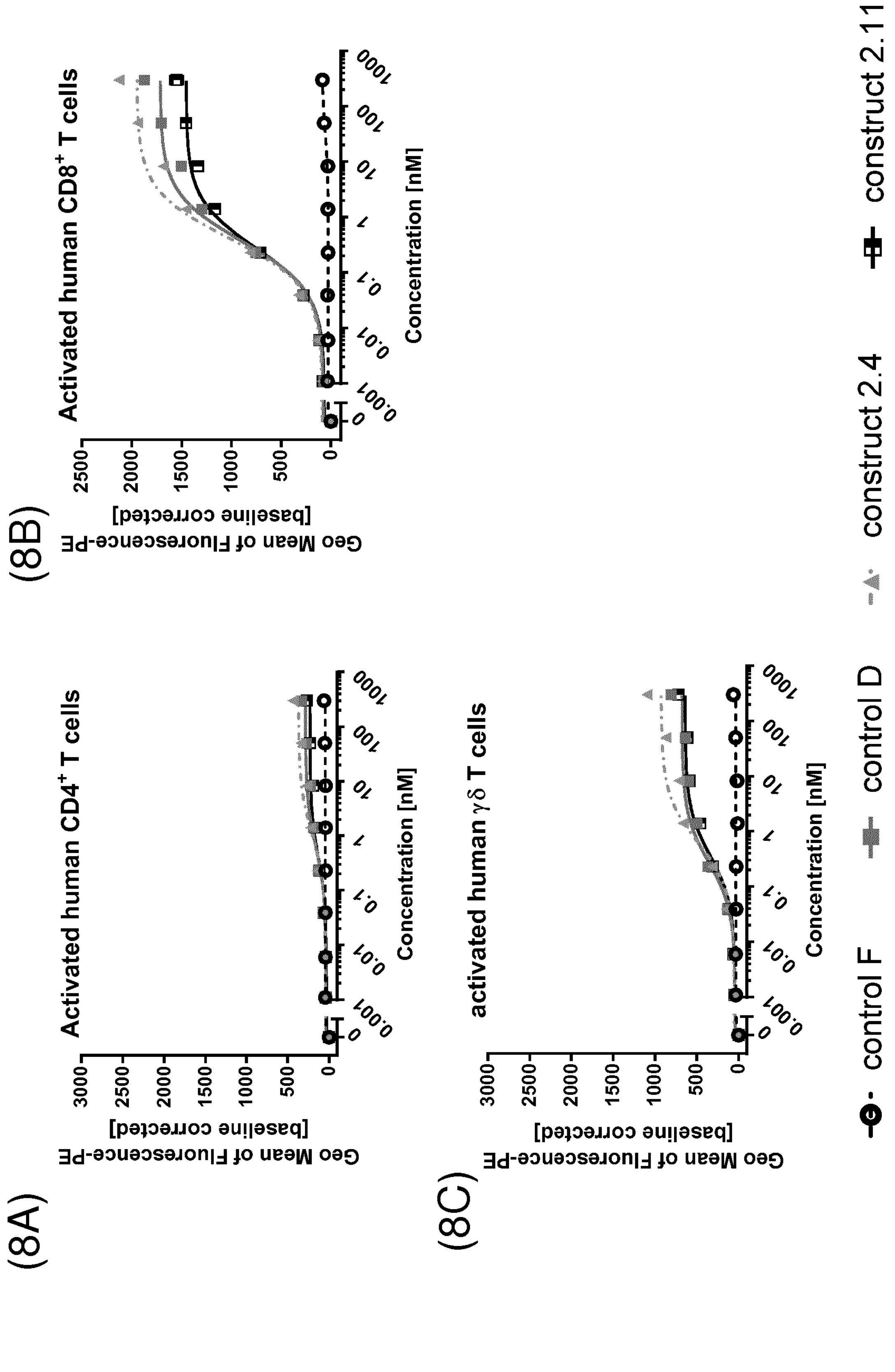
(8A)
Activated human CD4+ T cells
Geo Mean of Fluorescence-PE [baseline corrected]
3000
2500
2000
1500
1000
500
0
0
0.001
0.01
0.1
1
10
100
1000
Concentration [nM]
(8B)
Activated human CD8+ T cells
Geo Mean of Fluorescence-PE [baseline corrected]
2500
2000
1500
1000
500
0
Concentration [nM]
(8C)
activated human γδ T cells
Geo Mean of Fluorescence-PE [baseline corrected]
Concentration [nM]
control F
control D
construct 2.4
construct 2.11

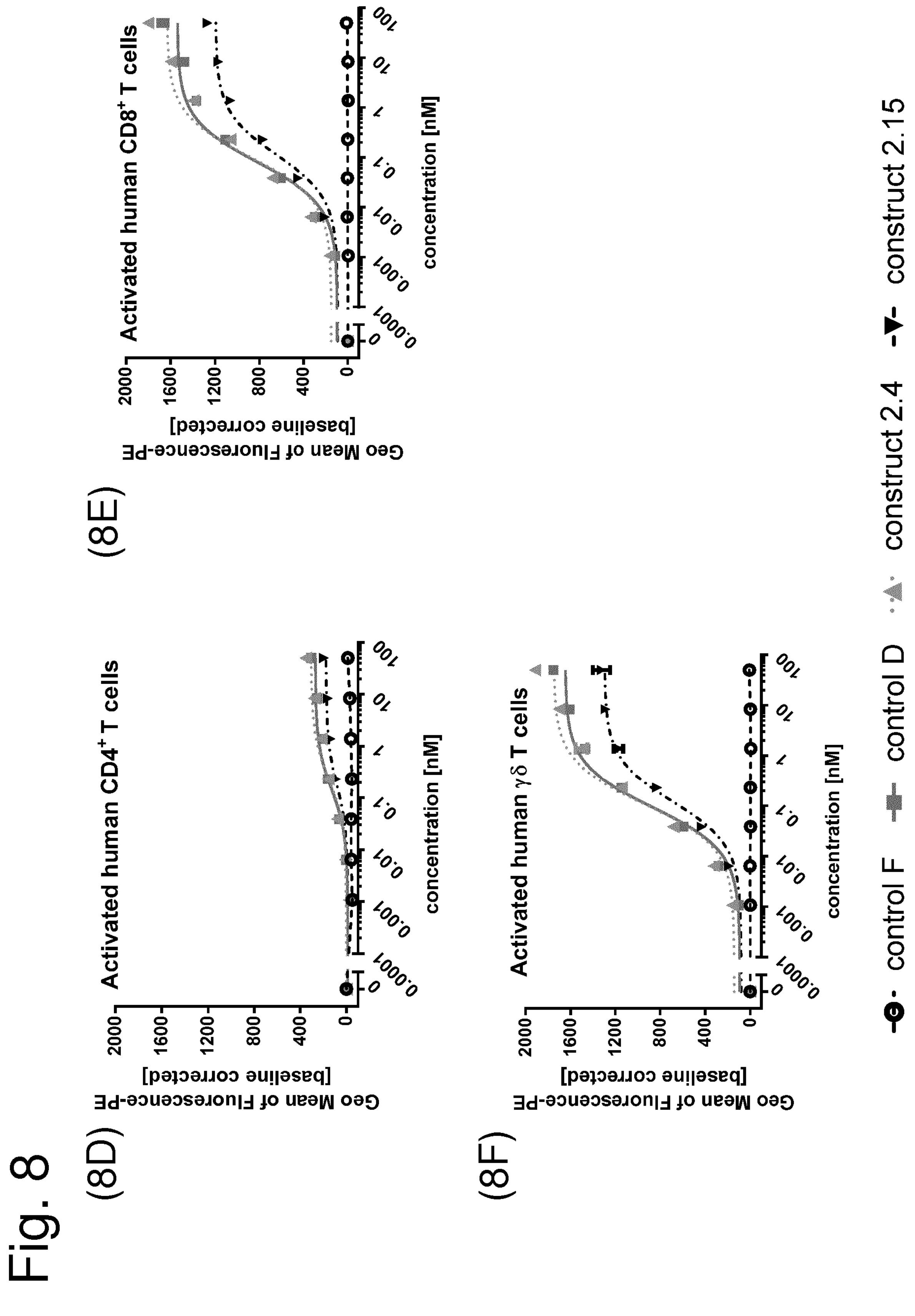
Fig. 8
(8D)
Activated human CD4+ T cells
Geo Mean of Fluorescence-PE [baseline corrected]
2000
1600
1200
800
400
0
0
0.0001
0.001
0.01
0.1
1
10
100
concentration [nM]
(8E)
Activated human CD8+ T cells
Geo Mean of Fluorescence-PE [baseline corrected]
concentration [nM]
(8F)
Activated human γδ T cells
Geo Mean of Fluorescence-PE [baseline corrected]
concentration [nM]
control F
control D
construct 2.4
construct 2.15

Fig. 9

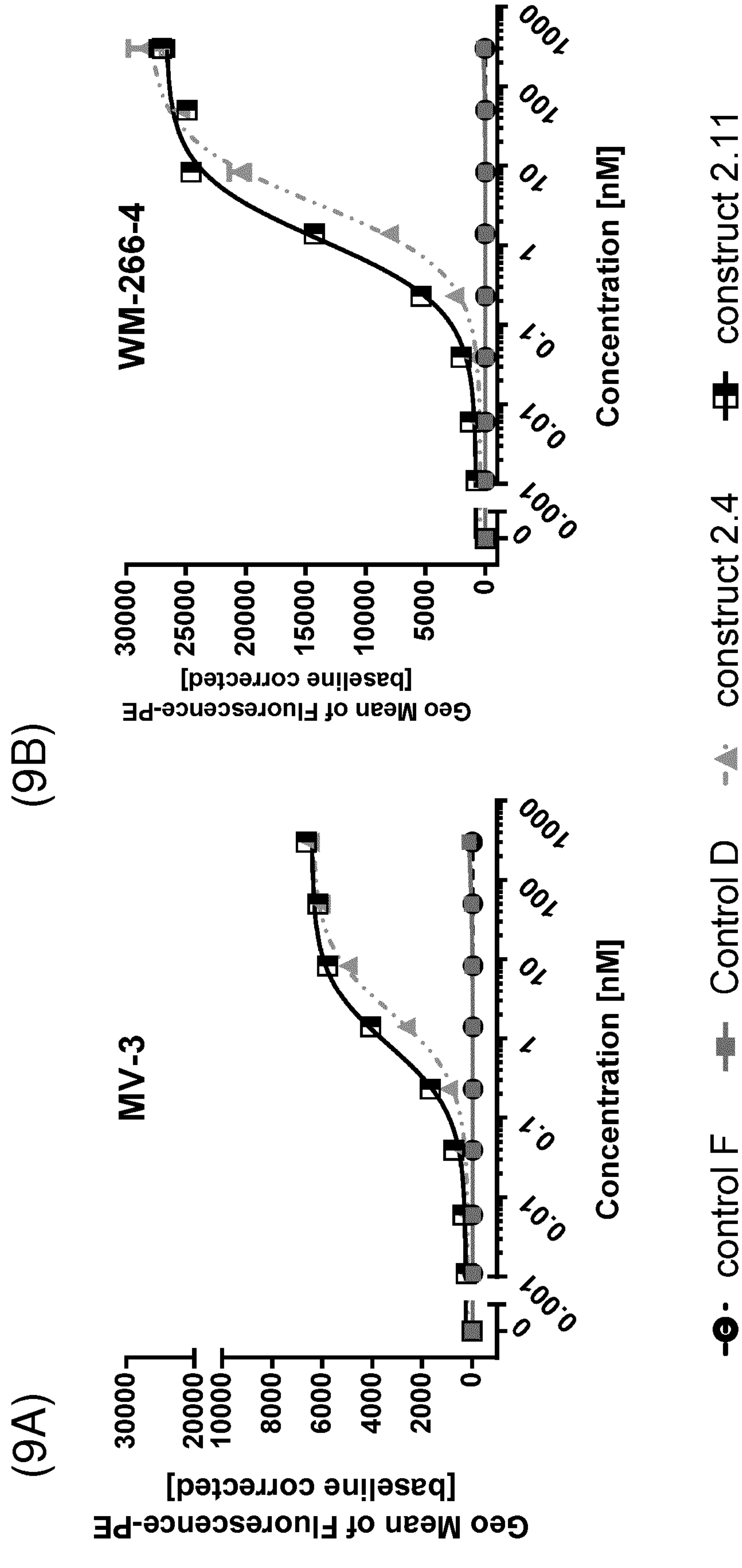
(9A)
MV-3
Geo Mean of Fluorescence-PE [baseline corrected]
30000
20000
10000
8000
6000
4000
2000
0
0
0.001
0.01
0.1
1
10
100
1000
Concentration [nM]
(9B)
WM-266-4
Geo Mean of Fluorescence-PE [baseline corrected]
30000
25000
20000
15000
10000
5000
0
0
0.001
0.01
0.1
1
10
100
1000
Concentration [nM]
control F
Control D
construct 2.4
construct 2.11

Fig. 9

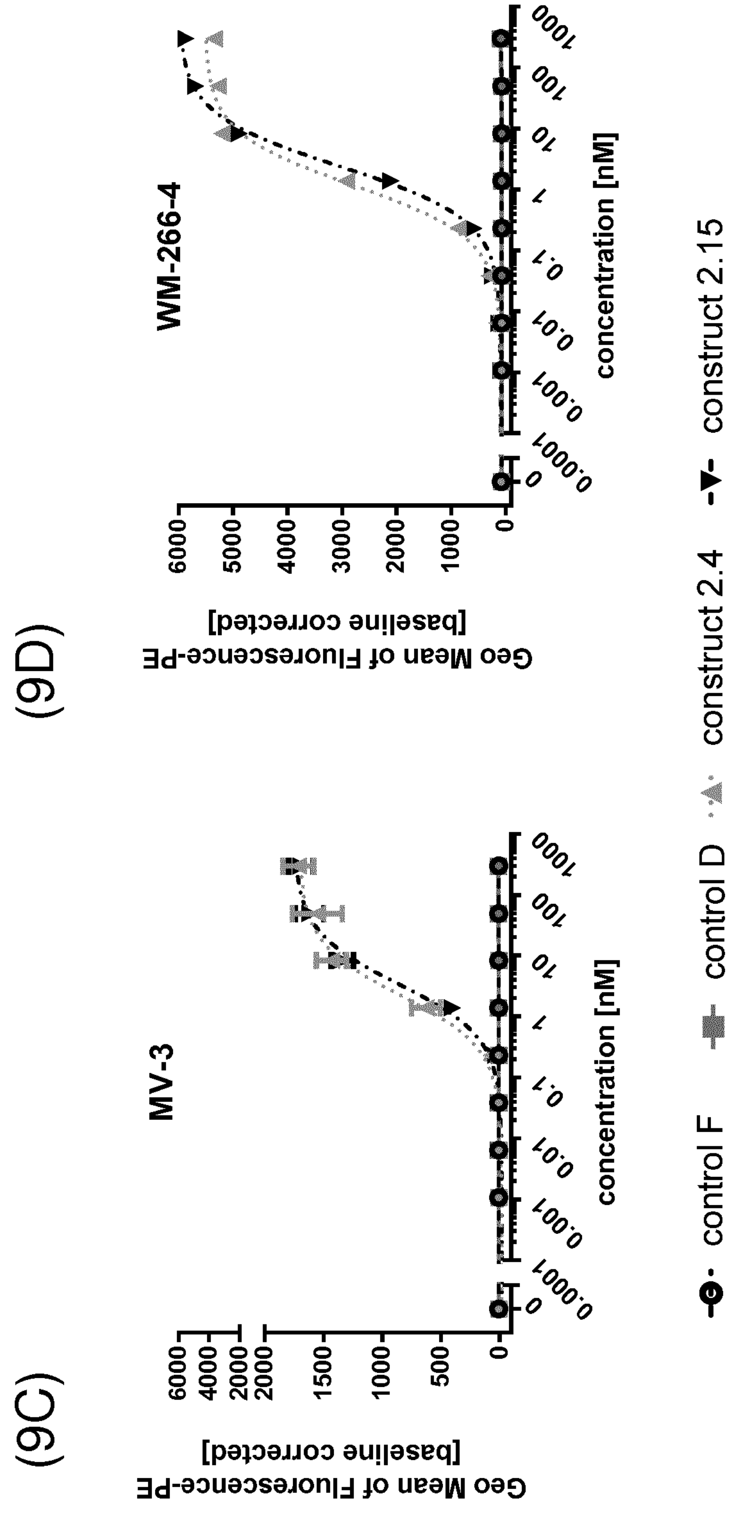
(9C)
MV-3
Geo Mean of Fluorescence-PE [baseline corrected]
6000
4000
2000
2000
1500
1000
500
0
0
0.0001
0.001
0.01
0.1
1
10
100
1000
concentration [nM]
(9D)
WM-266-4
Geo Mean of Fluorescence-PE [baseline corrected]
6000
5000
4000
3000
2000
1000
0
0
0.0001
0.001
0.01
0.1
1
10
100
1000
concentration [nM]
control F
control D
construct 2.4
construct 2.15

Fig. 10

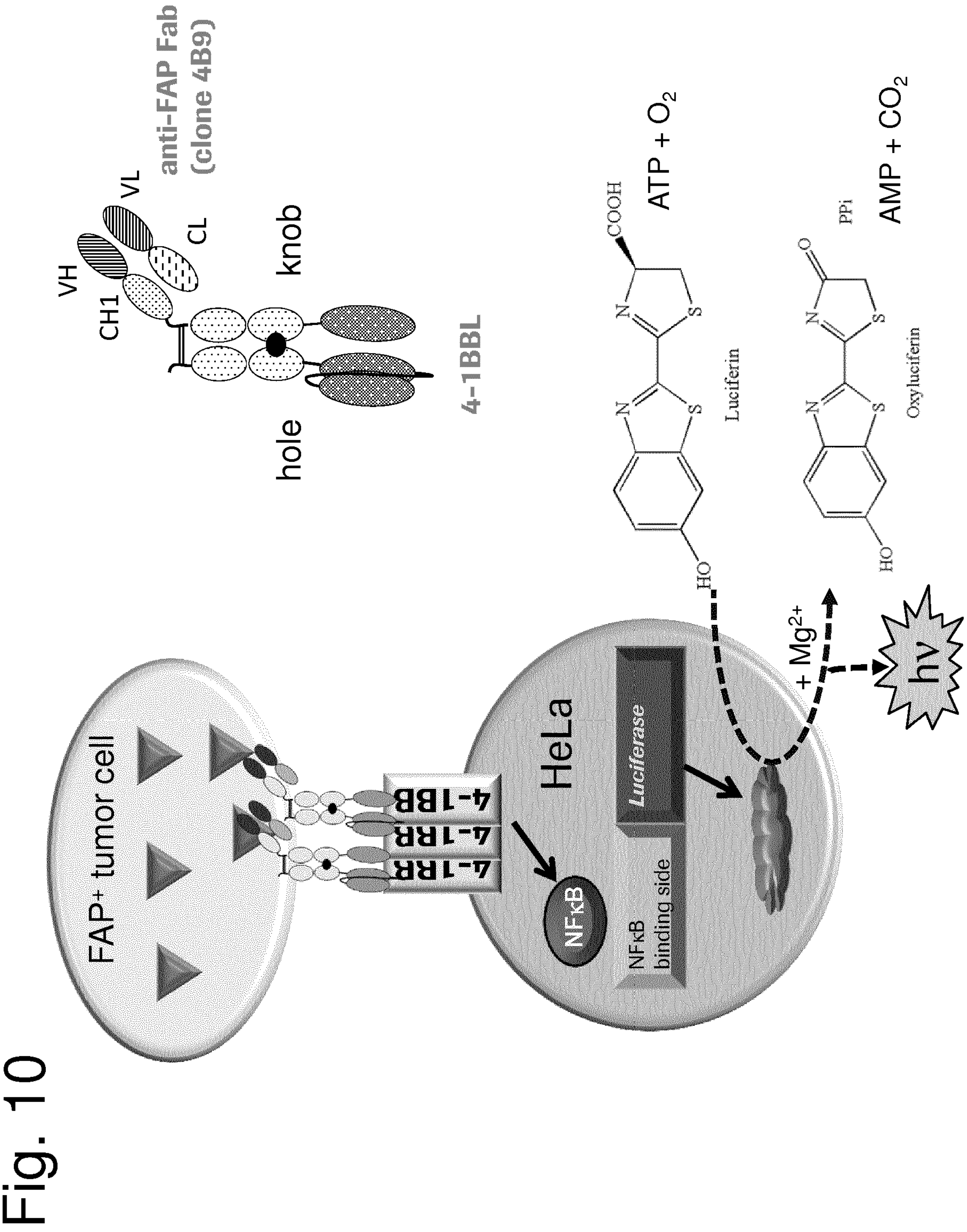
anti-FAP Fab
(clone 4B9)
VH
VL
CH1
CL
knob
hole
4-1BBL
FAP+ tumor cell
4-1BB
4-1BB
4-1BB
HeLa
NFκB
NFκB binding side
Luciferase
Luciferin
Oxyluciferin
ATP + O2
AMP + CO2
PPi
+ Mg2+
hν

Fig. 11

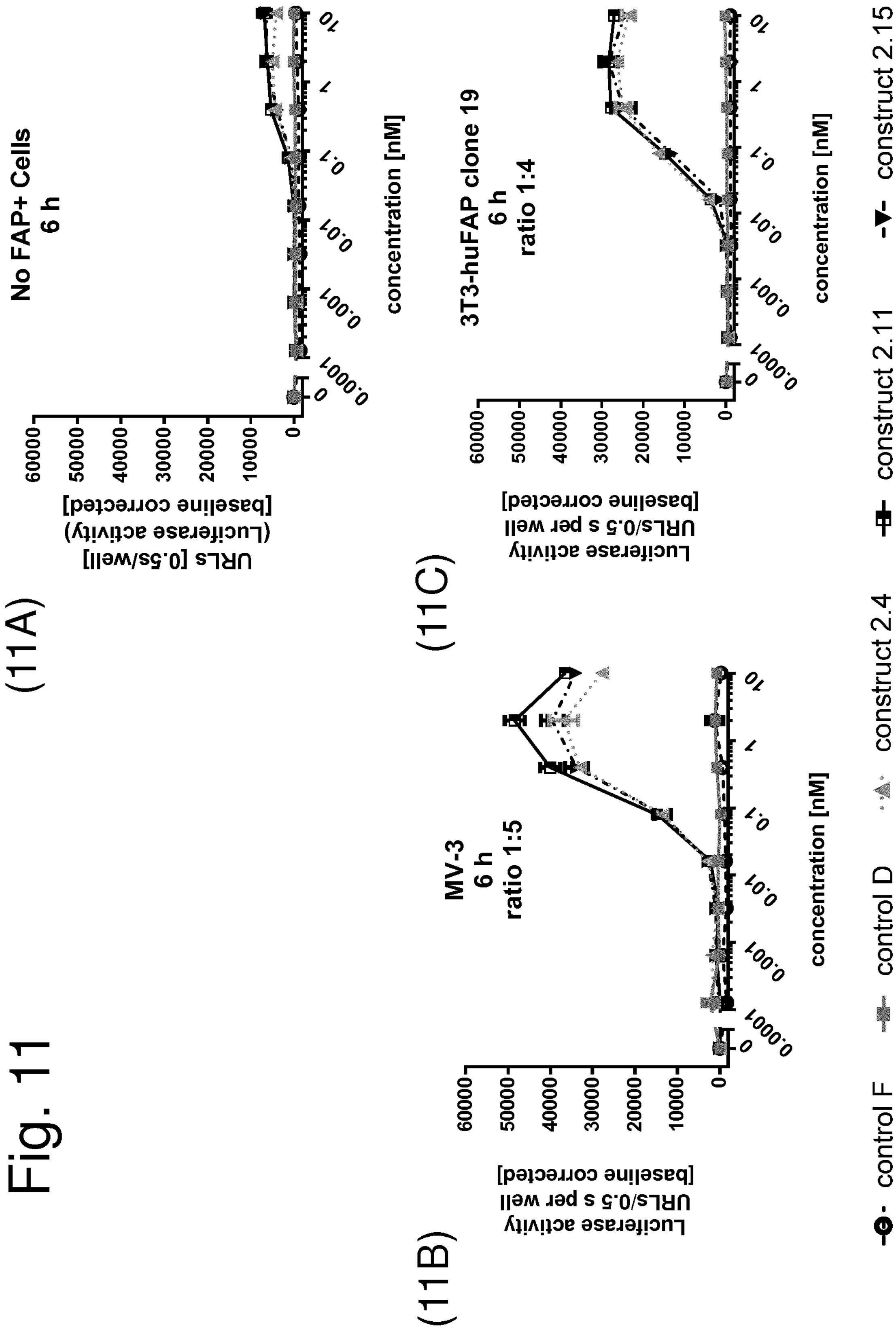
(11A)
No FAP+ Cells
6 h
URLs [0.5s/well]
(Luciferase activity)
[baseline corrected]
concentration [nM]
(11B)
MV-3
6 h
ratio 1:5
Luciferase activity
URLs/0.5 s per well
[baseline corrected]
concentration [nM]
(11C)
3T3-huFAP clone 19
6 h
ratio 1:4
Luciferase activity
URLs/0.5 s per well
[baseline corrected]
concentration [nM]
control F
control D
construct 2.4
construct 2.11
construct 2.15

Fig. 12

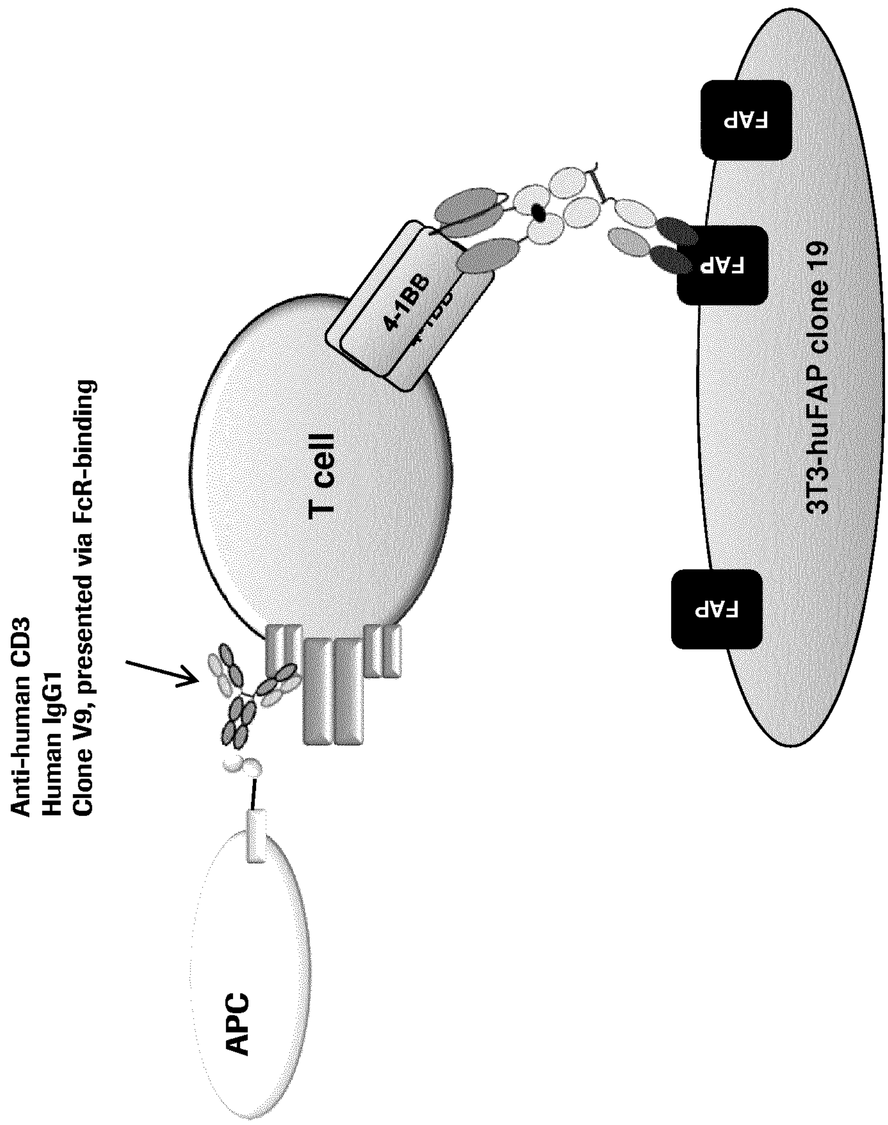
APC
Anti-human CD3
Human IgG1
Clone V9, presented via FcR-binding
T cell
4-1BB
FAP
FAP
FAP
3T3-huFAP clone 19

Fig. 13

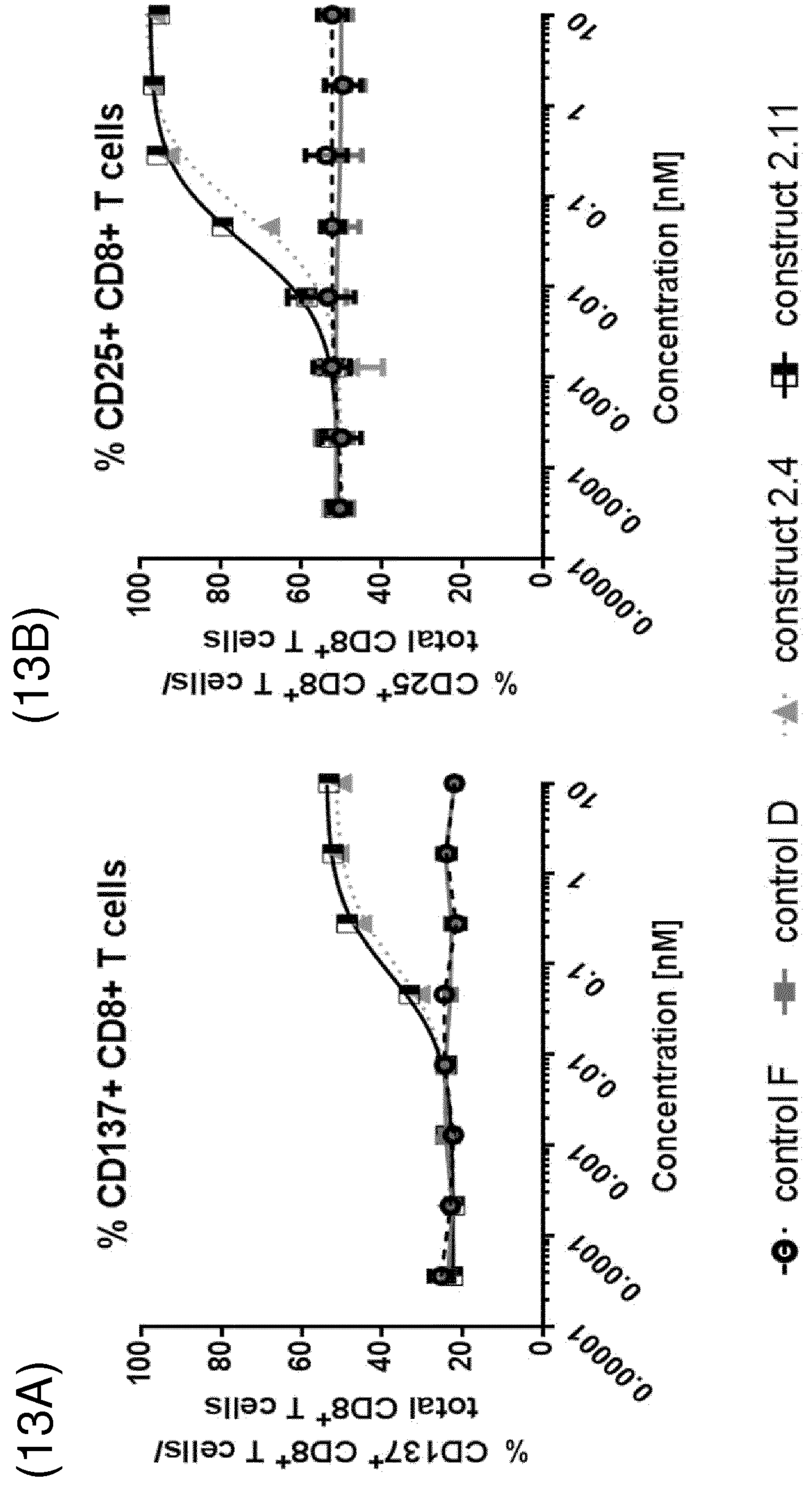
(13A)
% CD137+ CD8+ T cells
% CD137+ CD8+ T cells/
total CD8+ T cells
100
80
60
40
20
0
0.00001
0.0001
0.001
0.01
0.1
1
10
Concentration [nM]
(13B)
% CD25+ CD8+ T cells
% CD25+ CD8+ T cells/
total CD8+ T cells
Concentration [nM]
control F
control D
construct 2.4
construct 2.11

Fig. 14

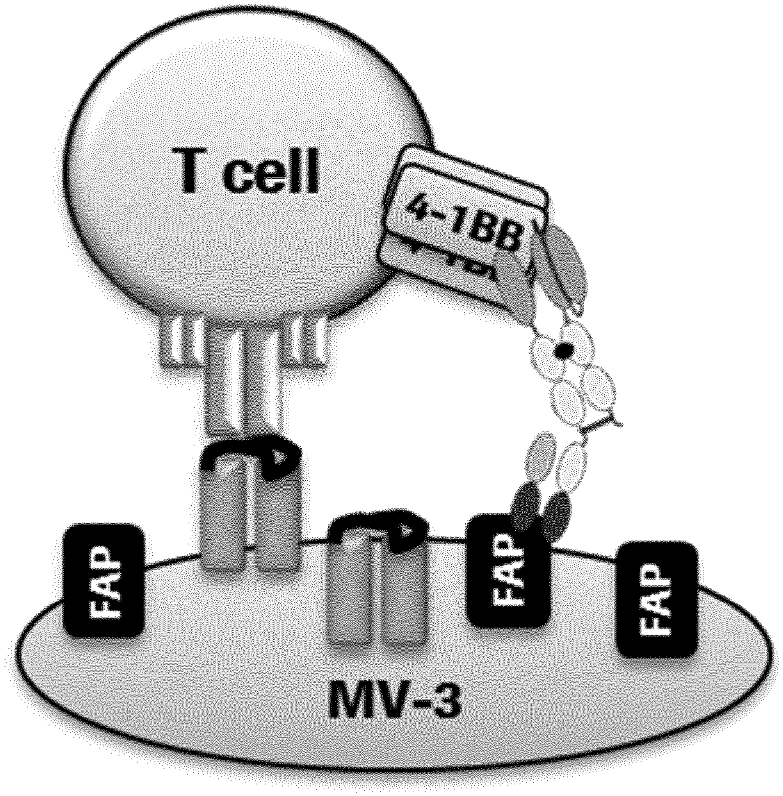

Fig. 15

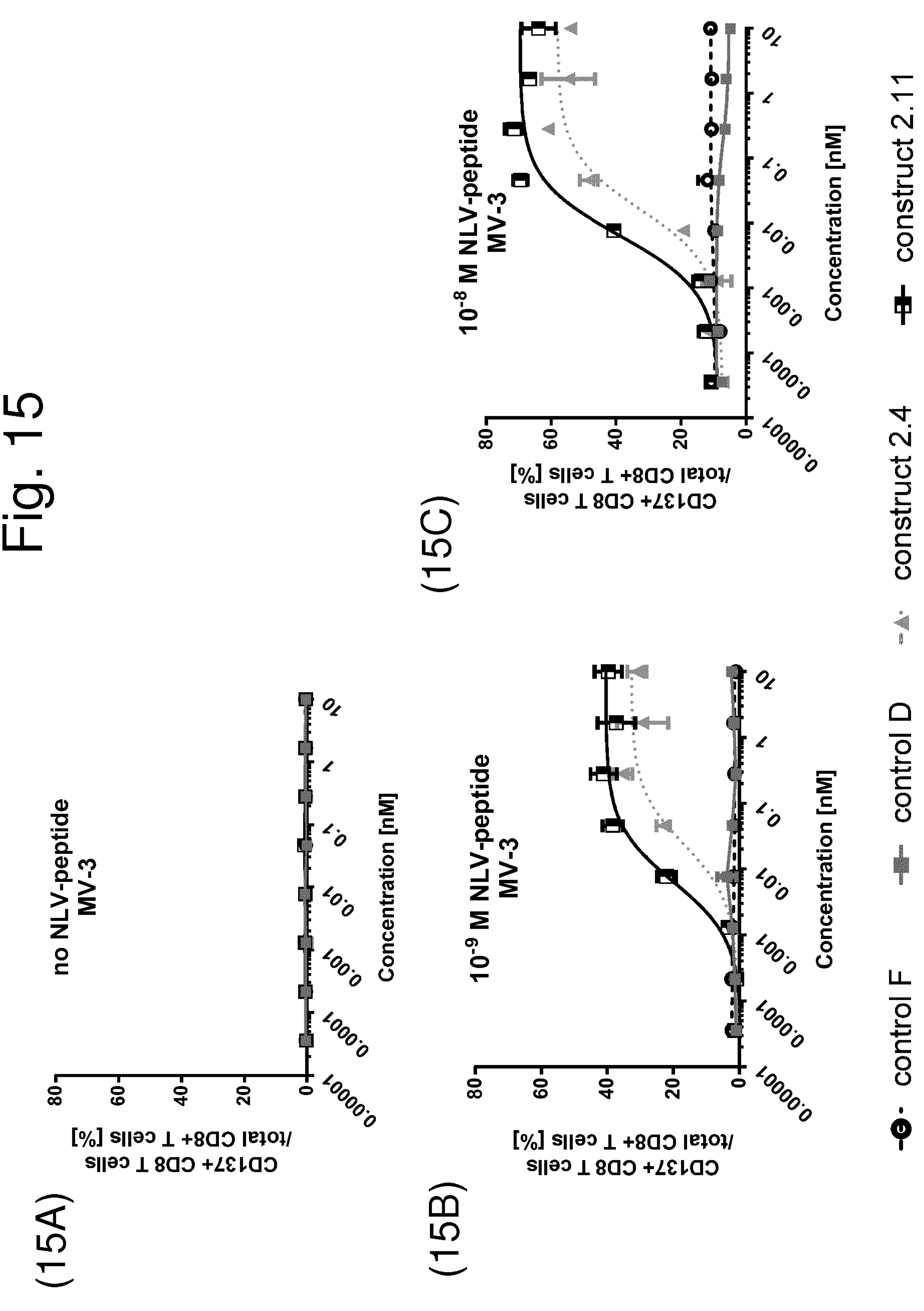
(15A)
no NLV-peptide
MV-3
CD137+ CD8 T cells
/total CD8+ T cells [%]
Concentration [nM]
(15B)
$10^{-9}$ M NLV-peptide
MV-3
(15C)
$10^{-8}$ M NLV-peptide
MV-3
0.00001
0.0001
0.001
0.01
0.1
1
10
0
20
40
60
80
control F
control D
construct 2.4
construct 2.11

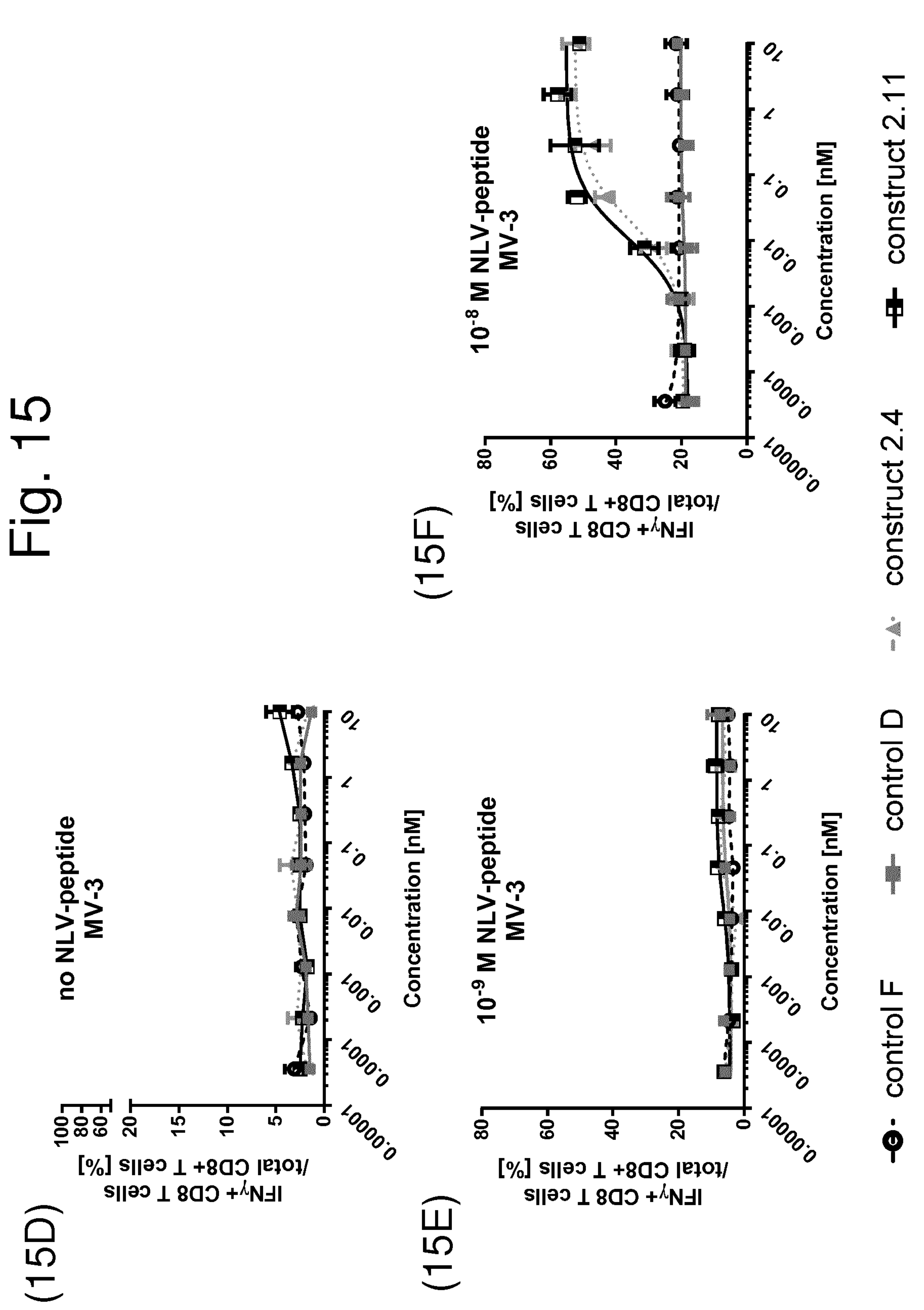
Fig. 15
(15D)
no NLV-peptide
MV-3
IFNγ+ CD8 T cells
/total CD8+ T cells [%]
Concentration [nM]
(15E)
10^-9 M NLV-peptide
MV-3
IFNγ+ CD8 T cells
/total CD8+ T cells [%]
Concentration [nM]
(15F)
10^-8 M NLV-peptide
MV-3
IFNγ+ CD8 T cells
/total CD8+ T cells [%]
Concentration [nM]
control F
control D
construct 2.4
construct 2.11

Fig. 16

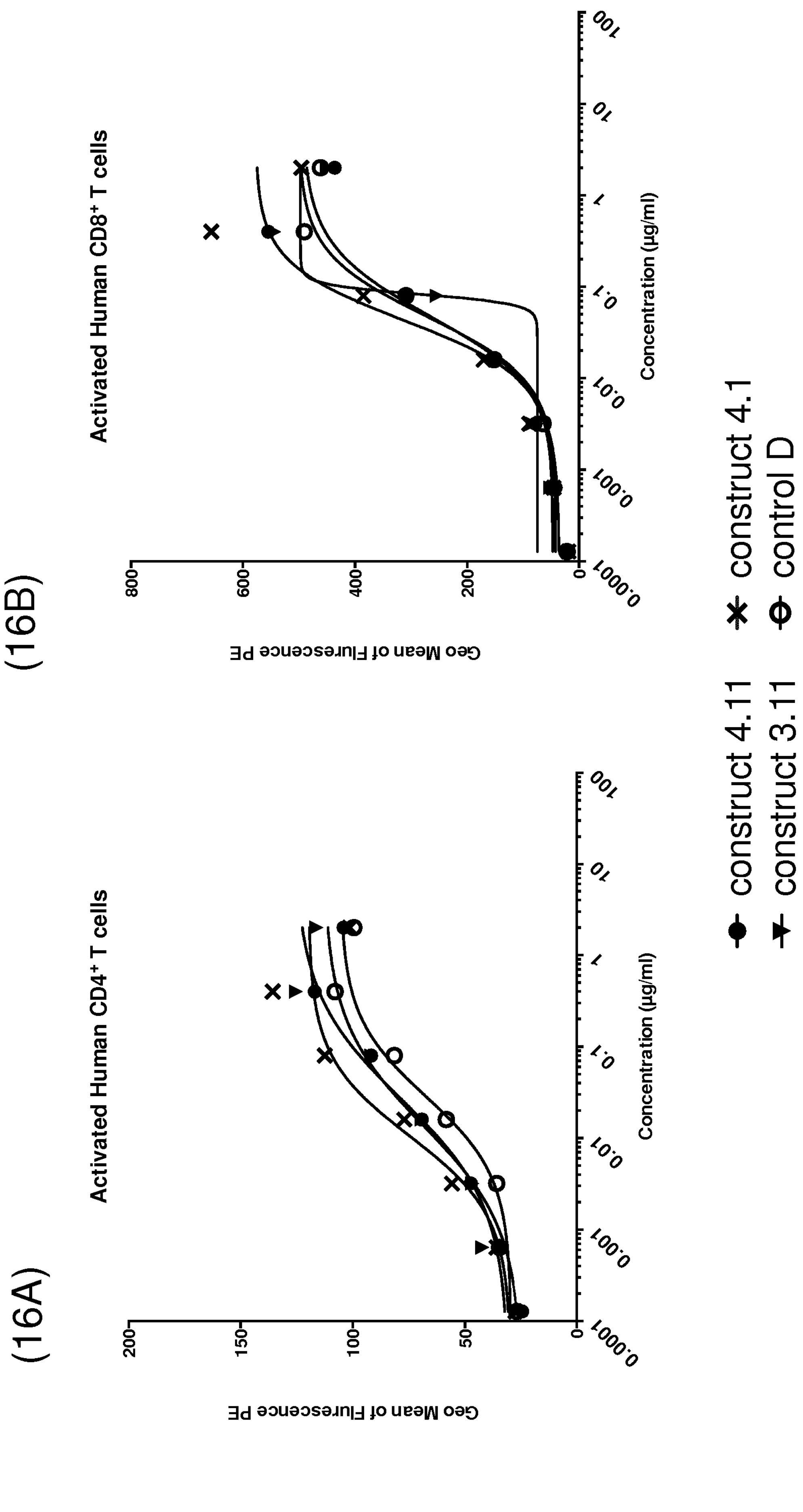
(16A)
Activated Human CD4⁺ T cells
Geo Mean of Fluresecence PE
200
150
100
50
0
0.0001
0.001
0.01
0.1
1
10
100
Concentration (µg/ml)
(16B)
Activated Human CD8⁺ T cells
Geo Mean of Fluresecence PE
800
600
400
200
0
0.0001
0.001
0.01
0.1
1
10
100
Concentration (µg/ml)
construct 4.11
construct 3.11
construct 4.1
control D

Fig. 17

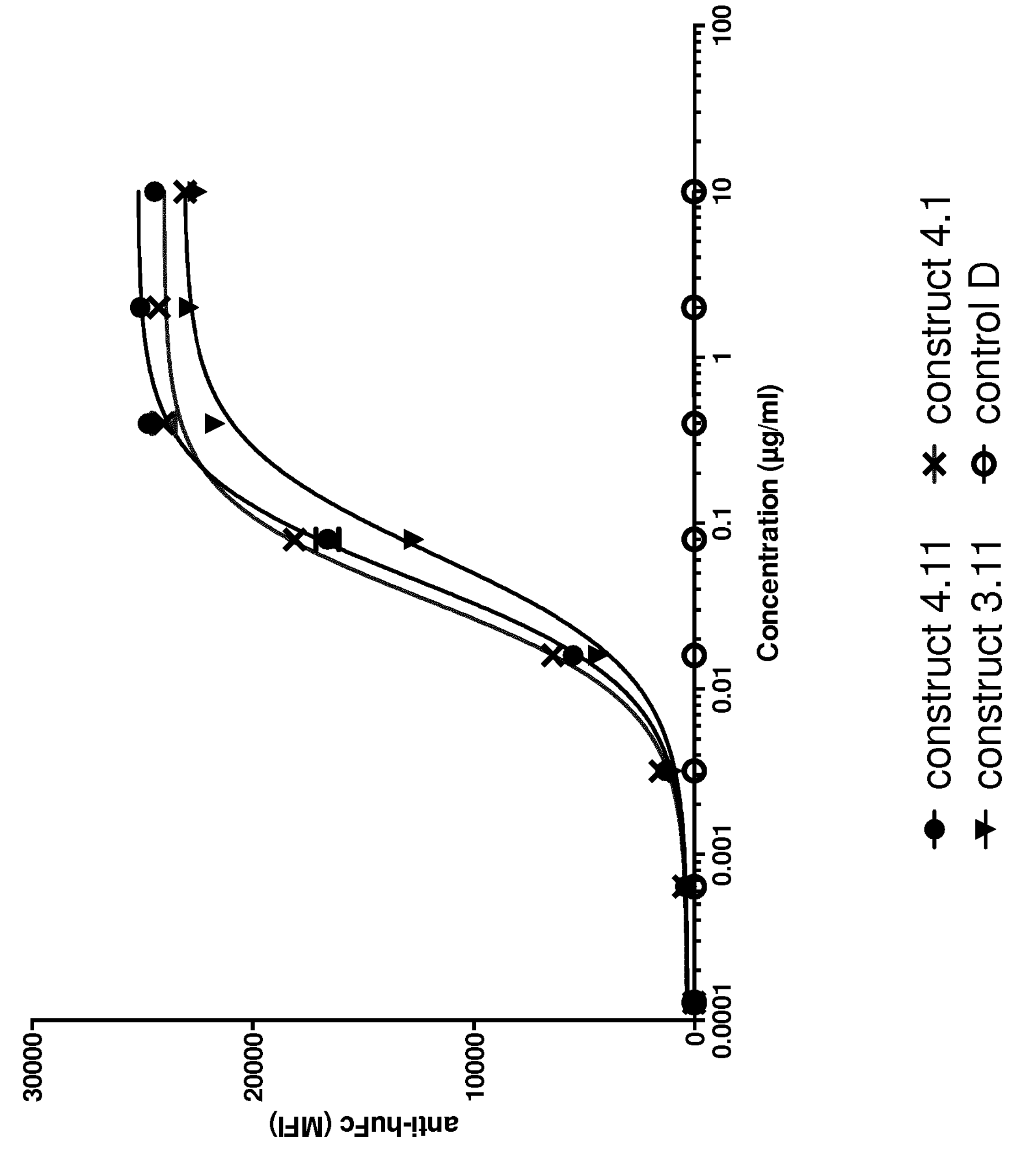
WSU-DLCL2
anti-huFc (MFI)
30000
20000
10000
0
0.0001
0.001
0.01
0.1
1
10
100
Concentration (µg/ml)
construct 4.11
construct 3.11
construct 4.1
control D

Fig. 18

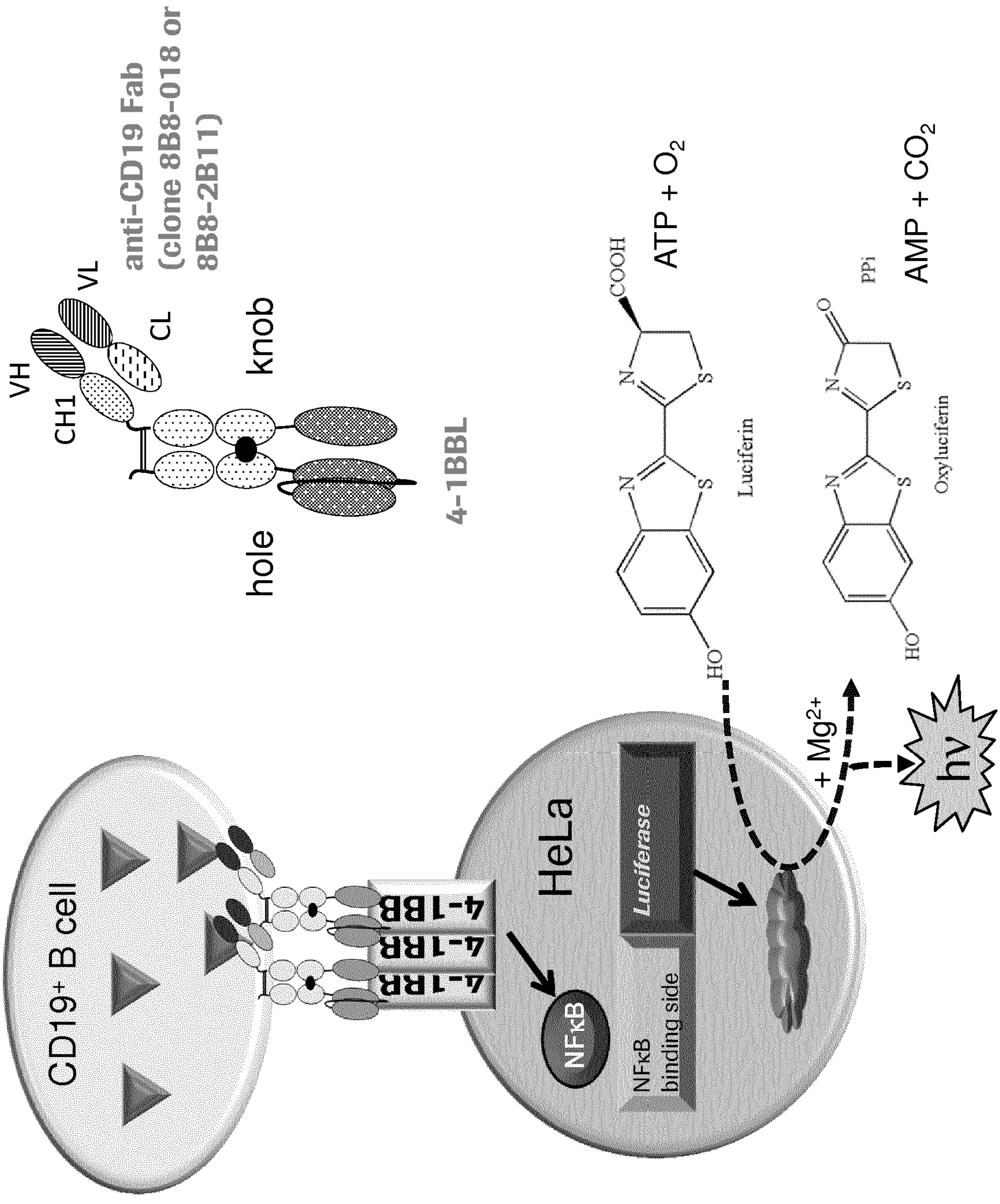
CD19+ B cell
4-1BB
4-1BB
4-1BB
HeLa
NFκB
NFκB binding side
Luciferase
+ $Mg^{2+}$
hν
VH
VL
CH1
CL
anti-CD19 Fab (clone 8B8-018 or 8B8-2B11)
hole
knob
4-1BBL
Luciferin
ATP + $O_2$
Oxyluciferin
PPi
AMP + $CO_2$

Fig. 19

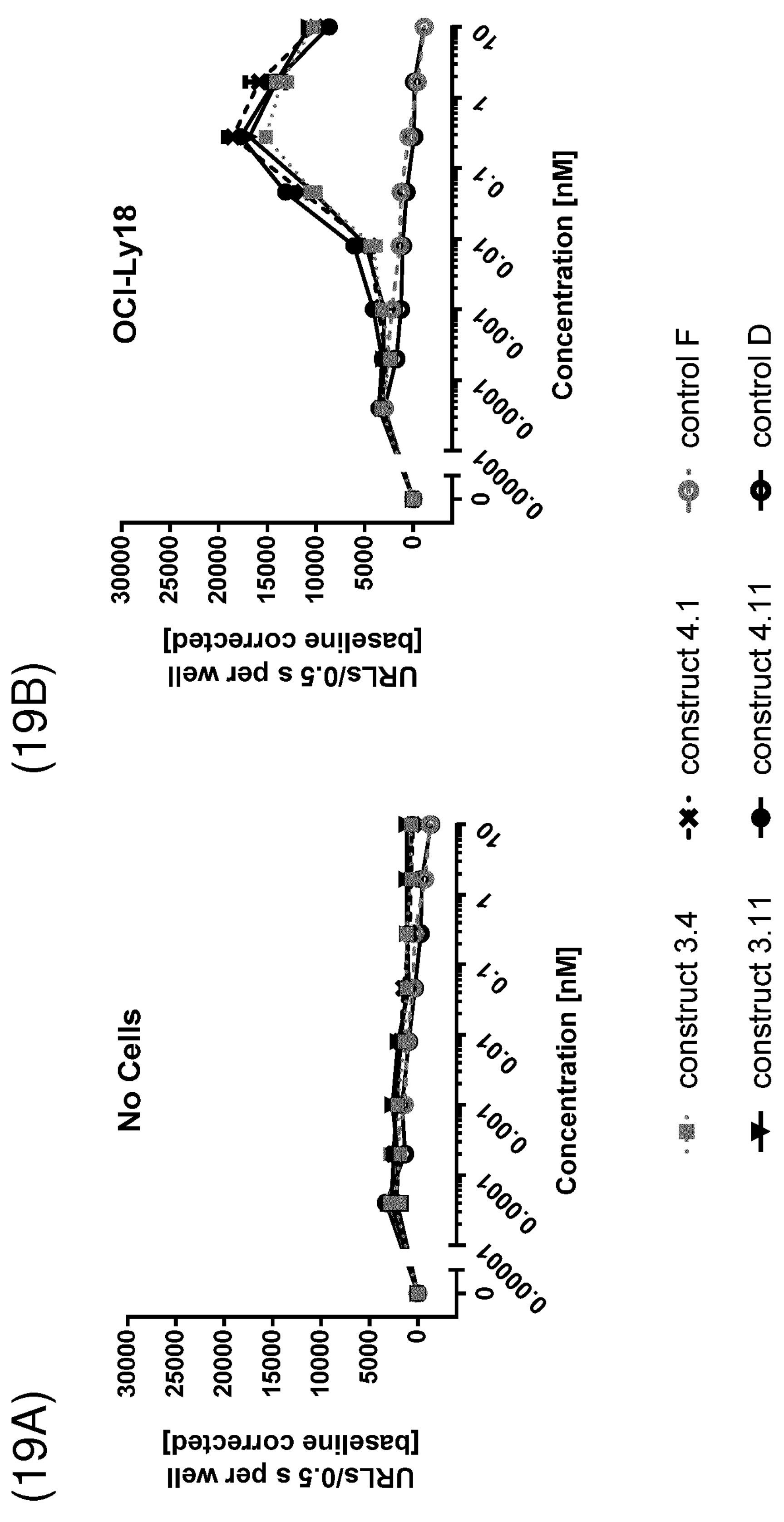
(19A)
No Cells
URLs/0.5 s per well [baseline corrected]
30000
25000
20000
15000
10000
5000
0
0
0.00001
0.0001
0.001
0.01
0.1
1
10
Concentration [nM]
(19B)
OCI-Ly18
URLs/0.5 s per well [baseline corrected]
30000
25000
20000
15000
10000
5000
0
0
0.00001
0.0001
0.001
0.01
0.1
1
10
Concentration [nM]
construct 3.4
construct 3.11
construct 4.1
construct 4.11
control F
control D

Fig. 19

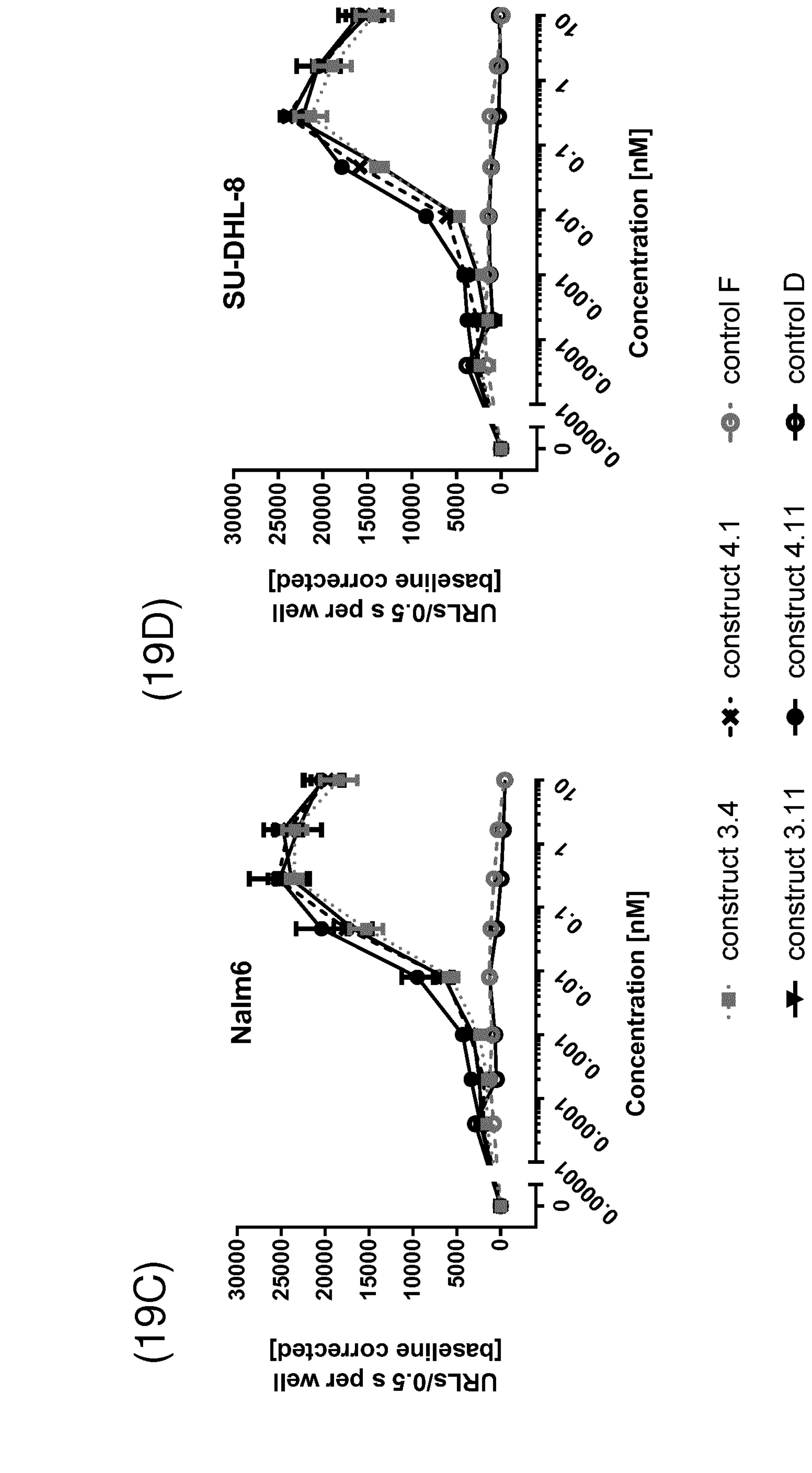

(19C)
Nalm6
URLs/0.5 s per well [baseline corrected]
30000
25000
20000
15000
10000
5000
0
0
0.00001
0.0001
0.001
0.01
0.1
1
10
Concentration [nM]
(19D)
SU-DHL-8
URLs/0.5 s per well [baseline corrected]
30000
25000
20000
15000
10000
5000
0
0
0.00001
0.0001
0.001
0.01
0.1
1
10
Concentration [nM]
construct 3.4
construct 3.11
construct 4.1
construct 4.11
control F
control D

Fig. 20

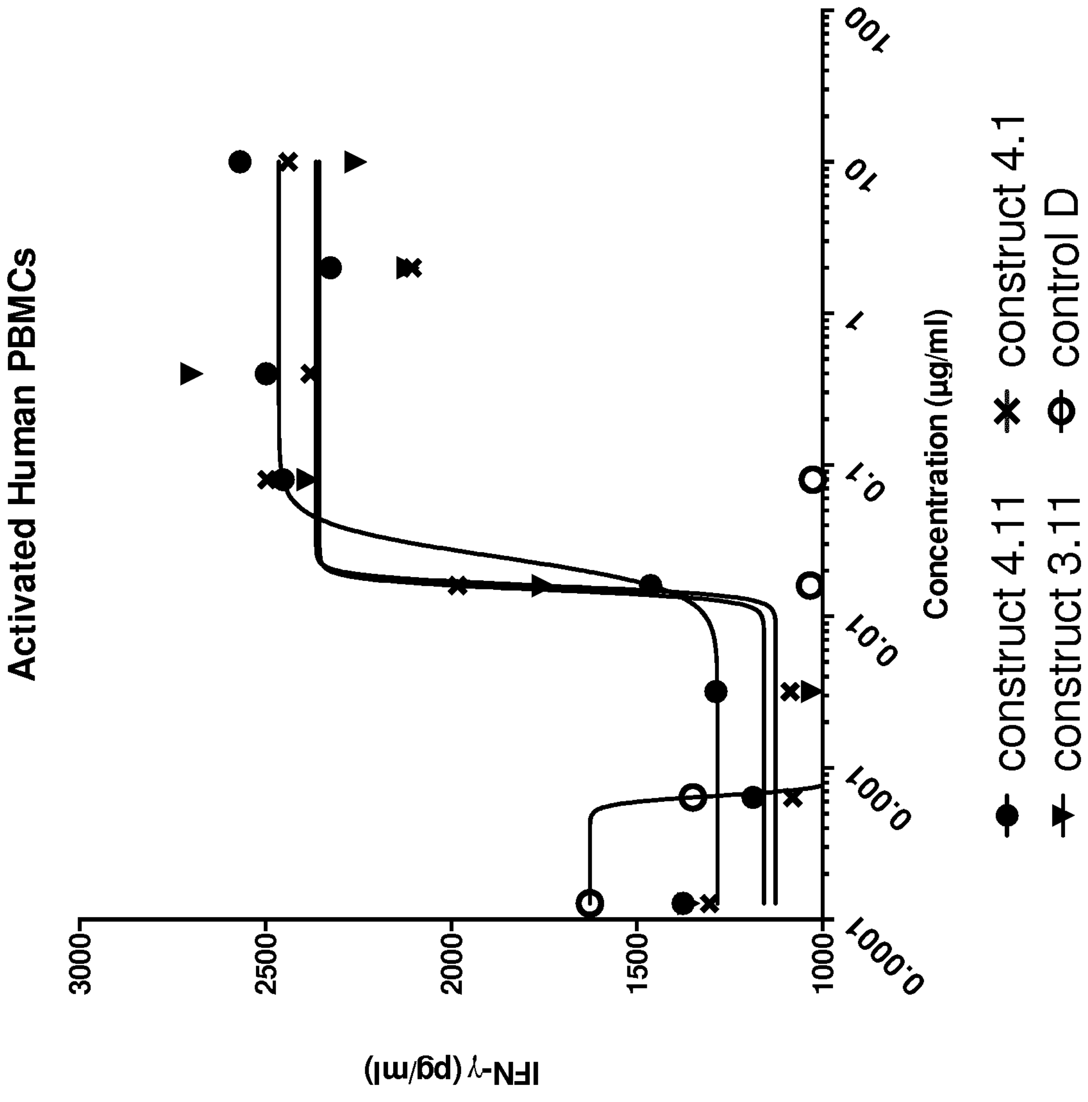
Activated Human PBMCs
IFN-γ (pg/ml)
3000
2500
2000
1500
1000
0.0001
0.001
0.01
0.1
1
10
100
Concentration (µg/ml)
construct 4.11
construct 4.1
construct 3.11
control D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010010051 A
- WO 2002020565 A
- WO 9316185 A
- US 5571894 A
- US 5587458 A
- US 5869046 A
- EP 404097 A
- WO 199301161 A
- US 6248516 B1
- WO 2012020006 A2
- US 5821333 A
- US 5731168 A
- US 7695936 B
- US 20030157108 A, Presta, L.
- US 20040093621 A
- WO 2003011878 A, Jean-Mairet
- US 6602684 B, Umana
- US 20050123546 A, Umana
- WO 199730087 A, Patel
- WO 199858964 A, Raju, S
- WO 199922764 A, Raju, S
- US 7521541 B
- WO 2012130831 A1
- US 6737056 B
- US 7332581 B
- WO 2012130831 A
- US 5500362 A
- US 5821337 A
- WO 2006029879 A
- WO 2005100402 A
- WO 2009089004 A
- US 5959177 A
- US 6040498 A
- US 6420548 B
- US 7125978 B
- US 6417429 B
- US 4186567 A
- US 5969108 A
- US 5565332 A
- US 7527791 B
- US 6982321 B
- US 7087409 B
- WO 2012020006 A
- WO 20040132066 A
- US 20050260186 A
- US 20060104968 A
- US 6267958 B
- US 6171586 B
- WO 2006044908 A
- WO 2011147834 A
- WO 2010145792 A1
- US 6054297 A
- EP 16169237


### Non-patent literature cited in the description


- **MUELLER et al.** *J. Immunotherapy,* 2008, vol. 31, 714-722
- **HORNIG et al.** *J. Immunotherapy,* 2012, vol. 35, 418-429
- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **HOUSTON, J.S.** *Methods in Enzymol.,* 1991, vol. 203, 46-96
- **GEBAUER ; SKERRA.** Engineered protein scaffolds as next-generation antibody therapeutics. *Curr Opin Chem Biol,* 2009, vol. 13, 245-255
- **STUMPP et al.** Darpins: A new generation of protein therapeutics. *Drug Discovery Today,* 2008, vol. 13, 695-701
- **LILJEBLAD et al.** *Glyco J,* 2000, vol. 17, 323-329
- **HEELEY.** *Endocr Res,* 2002, vol. 28, 217-229
- **GOLD ; FREEDMAN.** *J Exp Med.,* 1965, vol. 121, 439-462
- **BERINSTEIN N. L.** *J Clin Oncol.,* 2002, vol. 20, 2197-2207
- **NAP et al.** *Tumour Biol.,* 1988, vol. 9 (2-3), 145-53
- **NAP et al.** *Cancer Res.,* 1992, vol. 52 (8), 2329-2339
- **HAMMARSTRÖM S.** *Semin Cancer Biol.,* 1999, vol. 9 (2), 67-81
- **MARSHALL J.** *Semin Oncol.,* 2003, vol. 30 (8), 30-6
- **GOLDENBERG D M.** *The International Journal of Biological Markers,* 1992, vol. 7, 183-188

- **CHAU I. et al.** *J Clin Oncol.,* 2004, vol. 22, 1420-1429
- **FLAMINI et al.** *Clin Cancer Res,* 2006, vol. 12 (23), 6985-6988
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ALMAGRO ; FRANSSON.** *Front. Biosci.,* 2008, vol. 13, 1619-1633
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **BOWIE, J. U. et al.** *Science,* 1990, vol. 247, 1306-10
- **CUNNINGHAM ; WELLS.** *Science,* 1989, vol. 244, 1081-1085
- **WRIGHT et al.** *TIBTECH,* 1997, vol. 15, 26-32
- **KAM, N.W. et al.** *Proc. Natl. Acad. Sci. USA,* 2005, vol. 102, 11600-11605
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202, 163
- **CRAGG et al.** *Blood,* 2003, vol. 101, 1045-1052
- **CRAGG ; GLENNIE.** *Blood,* 2004, vol. 103, 2738-2743
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Remington's Pharmaceutical Sciences. Mack Printing Company, 1990
- **FINGL et al.** The Pharmacological Basis of Therapeutics. 1975, 1

- **KABAT, E.A. et al.** Sequences of Proteins of Immunological Interest. Public Health Service, National Institutes of Health, 1991
- **SAMBROOK et al.** Molecular cloning: A laboratory manual. Cold Spring Harbor Laboratory Press, 1989
- **KABAT, E.A. et al.** Sequences of Proteins of Immunological Interest. 1991
- Current Protocols in Cell Biology. John Wiley & Sons, Inc, 2000
- **MOREA, V. et al.** *Methods,* 2000, vol. 20 (3), 267-279
- **JENDEBERG L. et al.** *J. Immunological methods,* 1997
- **WAGENER et al.** *J Immunol,* 1983, vol. 130, 2308
- **NEUMAIER et al.** *J Immunol,* 1985, vol. 135, 3604
- **VAN MUIJEN GN ; JANSEN KF ; CORNELISSEN IM ; SMEETS DF ; BECK JL ; RUITER DJ.** Establishment and characterization of a human melanoma cell line (MV3) which is highly metastatic in nude mice. *Int J Cancer,* 22 April 1991, vol. 48 (1), 85-91
- **VAN MUIJEN GN ; JANSEN KF ; CORNELISSEN IM ; SMEETS DF ; BECK JL ; RUITER DJ.** Establishment and characterization of a human melanoma cell line (MV3) which is highly metastatic in nude mice. *Int J Cancer.,* 22 April 1991, vol. 48 (1), 85-91
- **RODRIGUES et al.** *Int J Cancer Suppl,* 1992, vol. 7, 45-50
- **ASCIERTO, P. A. ; E. SIMEONE ; M. SZNOL ; Y. X. FU ; I. MELERO.** Clinical experiences with anti-CD137 and anti-PDl therapeutic antibodies. *Semin Oncol,* 2010, vol. 37, 508-516
- **AGGARWAL B.B.** Signalling pathways of the TNF superfamily: a double-edged sword. *Nat. Rev. Immunol.,* 2003, vol. 3 (9), 745-56
- **BANNER D. et al.** Crystal structure of the soluble human 55 kd TNF receptor-human TNF beta complex: implications for TNF receptor activation. *Cell,* 1993, vol. 73, 431-445
- **BODMER J. ; SCHNEIDER P. ; TSCHOPP, J.** The molecular architecture of the TNF superfamily. *Trends in Biochemical Sciences,* 2002, vol. 27 (1), 19-26
- **BROLL, K. ; RICHTER, G. ; PAULY, S. ; HOFSTAEDTER, F. ; SCHWARZ, H.** CD137 expression in tumor vessel walls. High correlation with malignant tumors. *Am J Clin Pathol,* 2001, vol. 115, 543-549
- **BUECHELE, C. ; BAESSLER, T. ; SCHMIEDEL, B.J. ; SCHUMACHER, C.E. ; GROSSE-HOVEST, L. ; RITTIG, K. ; SALIH, H.R.** 4-1BB ligand modulates direct and Rituximab-induced NK-cell reactivity in chronic lymphocytic leukemia. *Eur J Immunol,* 2012, vol. 42, 737-748
- **CHOI, B.K. ; KIM, Y.H. ; KWON, P.M. ; LEE, S.C. ; KANG, S.W. ; KIM, M.S. ; LEE, M.J. ; KWON, B.S.** 4-1BB functions as a survival factor in dendritic cells. *J Immunol,* 2009, vol. 182, 4107-4115
- **CUADROS, C. ; DOMINGUEZ, A.L. ; LOLLINI, P.L. ; CROFT, M. ; MITTLER, R.S. ; BORGSTROM, P ; LUSTGARTEN, J.** Vaccination with dendritic cells pulsed with apoptotic tumors in combination with anti-OX40 and anti-4-1BB monoclonal antibodies induces T cell-mediated protective immunity in Her-2/neu transgenic mice. *Int J Cancer,* 2005, vol. 116, 934-943
- **CURRAN, M.A. ; KIM, M. ; MONTALVO, W. ; AL-SHAMKHANI, A. ; ALLISON, J.P.** Combination CTLA-4 blockade and 4-1BB activation enhances tumor rejection by increasing T-cell infiltration, proliferation, and cytokine production. *PLoS One,* 2011, vol. 6, e19499
- **DIEHL, L. ; VAN MIERLO, G.J. ; DEN BOER, A.T. ; VAN DER VOORT, E. ; FRANSEN, M. ; VAN BOSTELEN, L. ; KRIMPENFORT, P. ; MELIEF, C.J. ; MITTLER, R. ; TOES, R.E.** In vivo triggering through 4-1BB enables Th-independent priming of CTL in the presence of an intact CD28 costimulatory pathway. *J Immunol,* 2002, vol. 168, 3755-3762
- **DUBROT, J. ; MILHEIRO, F. ; ALFARO, C. ; PALAZON, A. ; MARTINEZ-FORERO, I. ; PEREZ-GRACIA, J.L. ; MORALES-KASTRESANA, A. ; ROMERO-TREVEJO, J.L. ; OCHOA, M.C. ; HERVAS-STUBBS, S. et al.** Treatment with anti-CD137 mAbs causes intense accumulations of liver T cells without selective antitumor immunotherapeutic effects in this organ. *Cancer Immunol Immunother,* 2010, vol. 59, 1223-1233
- **FUTAGAWA, T. ; AKIBA, H. ; KODAMA, T. ; TAKEDA, K. ; HOSODA, Y. ; YAGITA, H. ; OKUMURA, K.** Expression and function of 4-1BB and 4-1BB ligand on murine dendritic cells. *Int Immunol,* 2002, vol. 14, 275-286
- **GUO, Z. ; CHENG, D. ; XIA, Z. ; LUAN, M. ; WU, L. ; WANG, G. ; ZHANG, S.** Combined TIM-3 blockade and CD137 activation affords the long-term protection in a murine model of ovarian cancer. *J Transl Med,* 2013, vol. 11, 215
- **HEINISCH, I.V. ; DAIGLE, I. ; KNOPFLI, B. ; SIMON, H.U.** CD137 activation abrogates granulocyte-macrophage colony-stimulating factor-mediated anti-apoptosis in neutrophils. *Eur J Immunol,* 2000, vol. 30, 3441-3446
- **HORNIG, N. ; KERMER, V. ; FREY, K. ; DIEBOLDER, P. ; KONTERMANN, R.E. ; MUELLER, D.** Combination of a bispecific antibody and costimulatory antibody-ligand fusion proteins for targeted cancer immunotherapy. *J. Immunother.,* 2012, vol. 35, 418-429

- **JU, S.A. ; CHEON, S.H. ; PARK, S.M. ; TAM, N.Q. ; KIM, Y.M. ; AN, W.G. ; KIM, B.S.** Eradication of established renal cell carcinoma by a combination of 5-fluorouracil and anti-4-1BB monoclonal antibody in mice. *Int J Cancer,* 2008, vol. 122, 2784-2790
- **KIENZLE, G. ; VON KEMPIS, J.** CD137 (ILA/4-1BB), expressed by primary human monocytes, induces monocyte activation and apoptosis of B lymphocytes. *Int Immunol,* 2000, vol. 12, 73-82
- **KIM, D.H. ; CHANG, W.S. ; LEE, Y.S. ; LEE, K.A. ; KIM, Y.K. ; KWON, B.S. ; KANG, C.Y.** 4-1BB engagement costimulates NKT cell activation and exacerbates NKT cell ligand-induced airway hyperresponsiveness and inflammation. *J Immunol,* 2008, vol. 180, 2062-2068
- **KIM, Y.H. ; CHOI, B.K. ; OH, H.S. ; KANG, W.J. ; MITTLER, R.S. ; KWON, B.S.** Mechanisms involved in synergistic anticancer effects of anti-4-1BB and cyclophosphamide therapy. *Mol Cancer Ther,* 2009, vol. 8, 469-478
- **KWON, B.S. ; WEISSMAN, S.M.** cDNA sequences of two inducible T-cell genes. *Proc Natl Acad Sci U S A,* 1989, vol. 86, 1963-1967
- **LEE, H. ; PARK, H.J. ; SOHN, H.J. ; KIM, J.M. ; KIM, S.J.** Combinatorial therapy for liver metastatic colon cancer: dendritic cell vaccine and low-dose agonistic anti-4-1BB antibody costimulatory signal. *J Surg Res,* 2011, vol. 169, e43-50
- **LEVITSKY, V. ; DE CAMPOS-LIMA, P.O. ; FRISAN, T. ; MASUCCI, M.G.** The clonal composition of a peptide-specific oligoclonal CTL repertoire selected in response to persistent EBV infection is stable over time. *J Immunol,* 1998, vol. 161, 594-601
- **LI, F. ; RAVETCH, J.V.** Inhibitory Fcgamma receptor engagement drives adjuvant and anti-tumor activities of agonistic CD40 antibodies. *Science,* 2011, vol. 333, 1030-1034
- **LIN, W. ; VOSKENS, C.J. ; ZHANG, X. ; SCHINDLER, D.G. ; WOOD, A. ; BURCH, E. ; WEI, Y. ; CHEN, L. ; TIAN, G. ; TAMADA, K. et al.** Fc-dependent expression of CD137 on human NK cells: insights into "agonistic" effects of anti-CD137 monoclonal antibodies. *Blood,* 2008, vol. 112, 699-707
- **MELERO, I. ; JOHNSTON, J.V. ; SHUFFORD, W.W. ; MITTLER, R.S. ; CHEN, L.** NK1.1 cells express 4-1BB (CDw137) costimulatory molecule and are required for tumor immunity elicited by anti-4-1BB monoclonal antibodies. *Cell Immunol,* 1998, vol. 190, 167-172
- **MELERO, I. ; SHUFORD, W.W. ; NEWBY, S.A. ; ARUFFO, A. ; LEDBETTER, J.A. ; HELLSTROM, K.E. ; MITTLER, R.S. ; CHEN, L.** Monoclonal antibodies against the 4-1BB T-cell activation molecule eradicate established tumors. *Nat Med,* 1997, vol. 3, 682-685
- **MERCHANT, A.M. ; ZHU, Z. ; YUAN, J.Q. ; GODDARD, A. ; ADAMS, C.W. ; PRESTA, L.G. ; CARTER, P.** An efficient route to human bispecific IgG. *Nat Biotechnol,* 1998, vol. 16, 677-681
- **MORALES-KASTRESANA, A. ; SANMAMED, M.F. ; RODRIGUEZ, I. ; PALAZON, A. ; MARTINEZ-FORERO, I ; LABIANO, S. ; HERVAS-STUBBS, S. ; SANGRO, B. ; OCHOA, C. ; ROUZAUT, A. et al.** Combined immunostimulatory monoclonal antibodies extend survival in an aggressive transgenic hepatocellular carcinoma mouse model. *Clin Cancer Res,* 2013, vol. 19, 6151-6162
- **MUELLER, D. ; FREY, K. ; KONTERMANN, R.E.** A novel antibody-4-1BB1 fusion protein for targeted costimulation in cancer immunotherapy. *J. Immunother.,* 2008, vol. 31, 714-722
- **MURILLO, O. ; DUBROT, J. ; PALAZON, A. ; ARINA, A. ; AZPILIKUETA, A. ; ALFARO, C. ; SOLANO, S. ; OCHOA, M.C. ; BERASAIN, C. ; GABARI, I. et al.** In vivo depletion of DC impairs the anti-tumor effect of agonistic anti-CD137 mAb. *Eur J Immunol,* 2009, vol. 39, 2424-2436
- **NARAZAKI, H. ; ZHU, Y. ; LUO, L. ; ZHU, G. ; CHEN, L.** CD137 agonist antibody prevents cancer recurrence: contribution of CD137 on both hematopoietic and nonhematopoietic cells. *Blood,* 2010, vol. 115, 1941-1948
- **NISHIMOTO, H. ; LEE, S.W. ; HONG, H. ; POTTER, K.G. ; MAEDA-YAMAMOTO, M. ; KINOSHITA, T. ; KAWAKAMI, Y. ; MITTLER, R.S. ; KWON, B.S. ; WARE, C.F. et al.** Costimulation of mast cells by 4-1BB, a member of the tumor necrosis factor receptor superfamily, with the high-affinity IgE receptor. *Blood,* 2005, vol. 106, 4241-4248
- **OLOFSSON, P.S. ; SODERSTROM, L.A. ; WAGSATER, D. ; SHEIKINE, Y. ; OCAYA, P. ; LANG, F. ; RABU, C. ; CHEN, L. ; RUDLING, M. ; AUKRUST, P. et al.** CD137 is expressed in human atherosclerosis and promotes development of plaque inflammation in hypercholesterolemic mice. *Circulation,* 2008, vol. 117, 1292-1301
- **PALAZON, A. ; TEIJEIRA, A ; MARTINEZ-FORERO, I. ; HERVAS-STUBBS, S. ; RONCAL, C. ; PENUELAS, I. ; DUBROT, J. ; MORALES-KASTRESANA, A. ; PEREZ-GRACIA, J.L. ; OCHOA, M.C. et al.** Agonist anti-CD137 mAb act on tumor endothelial cells to enhance recruitment of activated T lymphocytes. *Cancer Res,* 2011, vol. 71, 801-811
- **SCHWARZ, H. ; VALBRACHT, J. ; TUCKWELL, J. ; VON KEMPIS, J. ; LOTZ, M.** ILA, the human 4-1BB homologue, is inducible in lymphoid and other cell lineages. *Blood,* 1995, vol. 85, 1043-1052
- **SHAO, Z. ; SCHWARZ, H.** CD137 ligand, a member of the tumor necrosis factor family, regulates immune responses via reverse signal transduction. *J Leukoc Biol,* 2011, vol. 89, 21-29

- **SHI, W. ; SIEMANN, D.W.** Augmented antitumor effects of radiation therapy by 4-1BB antibody (BMS-469492) treatment. *Anticancer Res,* 2006, vol. 26, 3445-3453
- **SIMEONE, E. ; ASCIERTO, P.A.** Immunomodulating antibodies in the treatment of metastatic melanoma: the experience with anti-CTLA-4, anti-CD137, and anti-PD1. *J Immunotoxicol,* 2012, vol. 9, 241-247
- **SNELL, L.M. ; LIN, G.H. ; MCPHERSON, A.J. ; MORAES, T.J. ; WATTS, T.H.** T-cell intrinsic effects of GITR and 4-1BB during viral infection and cancer immunotherapy. *Immunol Rev,* 2011, vol. 244, 197-217
- **STAGG, J. ; LOI, S. ; DIVISEKERA, U. ; NGIOW, S.F. ; DURET, H. ; YAGITA, H. ; TENG, M.W. ; SMYTH, M.J.** Anti-ErbB-2 mAb therapy requires type I and IIinterferons and synergizes with anti-PD-1 or anti-CD137 mAb therapy. *Proc Natl Acad Sci U S A,* 2011, vol. 108, 7142-7147
- **TENG, M.W. ; SHARKEY, J. ; MCLAUGHLIN, N.M. ; EXLEY, M.A. ; SMYTH, M.J.** CD1d-based combination therapy eradicates established tumors in mice. *J Immunol,* 2009, vol. 183, 1911-1920
- **VON KEMPIS, J. ; SCHWARZ, H. ; LOTZ, M.** Differentiation-dependent and stimulus-specific expression of ILA, the human 4-1BB-homologue, in cells of mesenchymal origin. *Osteoarthritis Cartilage,* 1997, vol. 5, 394-406
- **WEI, H. ; ZHAO, L. ; LI, W. ; FAN, K. ; QIAN, W. ; HOU, S. ; WANG, H ; DAI, M. ; HELLSTROM, I. ; HELLSTROM, K.E.** Combinatorial PD-1 blockade and CD137 activation has therapeutic efficacy in murine cancer models and synergizes with cisplatin. *PLoS One,* 2013, vol. 8, e84927
- **WILCOX, R.A. ; CHAPOVAL, A.I. ; GORSKI, K.S. ; OTSUJI, M. ; SHIN, T. ; FLIES, D.B. ; TAMADA, K. ; MITTLER, R.S. ; TSUCHIYA, H. ; PARDOLL, D.M.** Cutting edge: Expression of functional CD137 receptor by dendritic cells. *J Immunol,* 2002, vol. 168, 4262-4267
- **WILCOX, R.A. ; TAMADA, K. ; FLIES, D.B ; ZHU, G. ; CHAPOVAL, A.I. ; BLAZAR, B.R. ; KAST, W.M. ; CHEN, L.** Ligation of CD137 receptor prevents and reverses established anergy of CD8+ cytolytic T lymphocytes in vivo. *Blood,* 2004, vol. 103, 177-184
- **ZHANG, N ; SADUN, R.E. ; ARIAS, R.S. ; FLANAGAN, M.L. ; SACHSMAN, S.M. ; NIEN, Y ; KHAWLI, L.A. ; HU, P. ; EPSTEIN, A.L.** Targeted and untargeted CD137L fusion proteins for the immunotherapy of experimental solid tumors. *Clin. Cancer Res.,* 2007, vol. 13, 2758-2767
- **ZHANG, X. ; VOSKENS, C.J. ; SALLIN, M. ; MANIAR, A. ; MONTES, C.L. ; ZHANG, Y. ; LIN, W. ; LI, G. ; BURCH, E. ; TAN, M. et al.** CD137 promotes proliferation and survival of human B cells. *J Immunol,* 2010, vol. 184, 787-795

SEQUENCE LISTING

<110> F. Hoffmann-La Roche AG

<120> C-terminally fused TNF family ligand trimer-containing antigen binding molecules

<130> P33591-WO

<150> EP16169237.1
<151> 2016-05-11

<160> 245

<170> PatentIn version 3.5

<210> 1
<211> 184
<212> PRT
<213> Homo sapiens

<400> 1

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Pro Ser Pro Arg Ser Glu
            180
```

<210> 2
<211> 170
<212> PRT
<213> Homo sapiens

<400> 2

```
Leu Asp Leu Arg Gln Gly Met Phe Ala Gln Leu Val Ala Gln Asn Val
1               5                   10                  15

Leu Leu Ile Asp Gly Pro Leu Ser Trp Tyr Ser Asp Pro Gly Leu Ala
            20                  25                  30

Gly Val Ser Leu Thr Gly Gly Leu Ser Tyr Lys Glu Asp Thr Lys Glu
        35                  40                  45

Leu Val Val Ala Lys Ala Gly Val Tyr Tyr Val Phe Phe Gln Leu Glu
    50                  55                  60

Leu Arg Arg Val Val Ala Gly Glu Gly Ser Gly Ser Val Ser Leu Ala
65                  70                  75                  80

Leu His Leu Gln Pro Leu Arg Ser Ala Ala Gly Ala Ala Ala Leu Ala
                85                  90                  95

Leu Thr Val Asp Leu Pro Pro Ala Ser Ser Glu Ala Arg Asn Ser Ala
            100                 105                 110

Phe Gly Phe Gln Gly Arg Leu Leu His Leu Ser Ala Gly Gln Arg Leu
        115                 120                 125

Gly Val His Leu His Thr Glu Ala Arg Ala Arg His Ala Trp Gln Leu
    130                 135                 140

Thr Gln Gly Ala Thr Val Leu Gly Leu Phe Arg Val Thr Pro Glu Ile
145                 150                 155                 160

Pro Ala Gly Leu Pro Ser Pro Arg Ser Glu
                165                 170
```

<210> 3
<211> 175
<212> PRT
<213> Homo sapiens

<400> 3

```
Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg
    130                 135                 140

His Ala Trp Gln Leu Thr Gln Gly Ala Thr Val Leu Gly Leu Phe Arg
145                 150                 155                 160

Val Thr Pro Glu Ile Pro Ala Gly Leu Pro Ser Pro Arg Ser Glu
                165                 170                 175
```

<210> 4
<211> 203
<212> PRT
<213> Homo sapiens

<400> 4

```
Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
```

```
            35                  40                  45

        Val Leu Leu Ile Asp Gly Pro Leu Ser Trp Tyr Ser Asp Pro Gly Leu
            50                  55                  60

        Ala Gly Val Ser Leu Thr Gly Gly Leu Ser Tyr Lys Glu Asp Thr Lys
        65                  70                  75                  80

        Glu Leu Val Val Ala Lys Ala Gly Val Tyr Tyr Val Phe Phe Gln Leu
                        85                  90                  95

        Glu Leu Arg Arg Val Val Ala Gly Glu Gly Ser Gly Ser Val Ser Leu
                    100                 105                 110

        Ala Leu His Leu Gln Pro Leu Arg Ser Ala Ala Gly Ala Ala Ala Leu
                115                 120                 125

        Ala Leu Thr Val Asp Leu Pro Pro Ala Ser Ser Glu Ala Arg Asn Ser
            130                 135                 140

        Ala Phe Gly Phe Gln Gly Arg Leu Leu His Leu Ser Ala Gly Gln Arg
        145                 150                 155                 160

        Leu Gly Val His Leu His Thr Glu Ala Arg Ala Arg His Ala Trp Gln
                        165                 170                 175

        Leu Thr Gln Gly Ala Thr Val Leu Gly Leu Phe Arg Val Thr Pro Glu
                    180                 185                 190

        Ile Pro Ala Gly Leu Pro Ser Pro Arg Ser Glu
                195                 200
```

<210> 5
<211> 178
<212> PRT
<213> Homo sapiens

<400> 5

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val

    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu
```

<210> 6
<211> 164
<212> PRT
<213> Homo sapiens

<400> 6

```
Leu Asp Leu Arg Gln Gly Met Phe Ala Gln Leu Val Ala Gln Asn Val
1               5                   10                  15

Leu Leu Ile Asp Gly Pro Leu Ser Trp Tyr Ser Asp Pro Gly Leu Ala
            20                  25                  30

Gly Val Ser Leu Thr Gly Gly Leu Ser Tyr Lys Glu Asp Thr Lys Glu
        35                  40                  45

Leu Val Val Ala Lys Ala Gly Val Tyr Tyr Val Phe Phe Gln Leu Glu
    50                  55                  60

Leu Arg Arg Val Val Ala Gly Glu Gly Ser Gly Ser Val Ser Leu Ala
65                  70                  75                  80

Leu His Leu Gln Pro Leu Arg Ser Ala Ala Gly Ala Ala Ala Leu Ala

                85                  90                  95

Leu Thr Val Asp Leu Pro Pro Ala Ser Ser Glu Ala Arg Asn Ser Ala
            100                 105                 110

Phe Gly Phe Gln Gly Arg Leu Leu His Leu Ser Ala Gly Gln Arg Leu
        115                 120                 125

Gly Val His Leu His Thr Glu Ala Arg Ala Arg His Ala Trp Gln Leu
    130                 135                 140

Thr Gln Gly Ala Thr Val Leu Gly Leu Phe Arg Val Thr Pro Glu Ile
145                 150                 155                 160

Pro Ala Gly Leu
```

<210> 7
<211> 169
<212> PRT
<213> Homo sapiens

<400> 7

```
Asp Pro Ala Gly Leu Leu Asp Leu Arg Gln Gly Met Phe Ala Gln Leu
1               5                   10                  15

Val Ala Gln Asn Val Leu Leu Ile Asp Gly Pro Leu Ser Trp Tyr Ser
            20                  25                  30

Asp Pro Gly Leu Ala Gly Val Ser Leu Thr Gly Gly Leu Ser Tyr Lys
        35                  40                  45

Glu Asp Thr Lys Glu Leu Val Val Ala Lys Ala Gly Val Tyr Tyr Val
    50                  55                  60

Phe Phe Gln Leu Glu Leu Arg Arg Val Val Ala Gly Glu Gly Ser Gly
65                  70                  75                  80

Ser Val Ser Leu Ala Leu His Leu Gln Pro Leu Arg Ser Ala Ala Gly
                85                  90                  95

Ala Ala Ala Leu Ala Leu Thr Val Asp Leu Pro Pro Ala Ser Ser Glu
            100                 105                 110

Ala Arg Asn Ser Ala Phe Gly Phe Gln Gly Arg Leu Leu His Leu Ser
        115                 120                 125

Ala Gly Gln Arg Leu Gly Val His Leu His Thr Glu Ala Arg Ala Arg


    130                 135                 140

His Ala Trp Gln Leu Thr Gln Gly Ala Thr Val Leu Gly Leu Phe Arg
145                 150                 155                 160

Val Thr Pro Glu Ile Pro Ala Gly Leu
                165
```

<210> 8
<211> 197
<212> PRT
<213> Homo sapiens

<400> 8

```
Pro Trp Ala Val Ser Gly Ala Arg Ala Ser Pro Gly Ser Ala Ala Ser
1               5                   10                  15

Pro Arg Leu Arg Glu Gly Pro Glu Leu Ser Pro Asp Asp Pro Ala Gly
            20                  25                  30

Leu Leu Asp Leu Arg Gln Gly Met Phe Ala Gln Leu Val Ala Gln Asn
        35                  40                  45

Val Leu Leu Ile Asp Gly Pro Leu Ser Trp Tyr Ser Asp Pro Gly Leu
    50                  55                  60

Ala Gly Val Ser Leu Thr Gly Gly Leu Ser Tyr Lys Glu Asp Thr Lys
65                  70                  75                  80

Glu Leu Val Val Ala Lys Ala Gly Val Tyr Tyr Val Phe Phe Gln Leu
                85                  90                  95

Glu Leu Arg Arg Val Val Ala Gly Glu Gly Ser Gly Ser Val Ser Leu
            100                 105                 110

Ala Leu His Leu Gln Pro Leu Arg Ser Ala Ala Gly Ala Ala Ala Leu
        115                 120                 125

Ala Leu Thr Val Asp Leu Pro Pro Ala Ser Ser Glu Ala Arg Asn Ser
    130                 135                 140

Ala Phe Gly Phe Gln Gly Arg Leu Leu His Leu Ser Ala Gly Gln Arg
145                 150                 155                 160

Leu Gly Val His Leu His Thr Glu Ala Arg Ala Arg His Ala Trp Gln
                165                 170                 175

Leu Thr Gln Gly Ala Thr Val Leu Gly Leu Phe Arg Val Thr Pro Glu


                180                 185                 190

    Ile Pro Ala Gly Leu
            195
```

<210> 9
<211> 378
<212> PRT
<213> Artificial Sequence

<220>
<223> dimeric hu 4-1BBL (71-254) connected by (G4S)2 linker

<400> 9

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Pro Ser Pro Arg Ser Glu Gly Gly Gly Gly Ser Gly Gly Gly
            180                 185                 190
```

Gly Ser Arg Glu Gly Pro Glu Leu Ser Pro Asp Asp Pro Ala Gly Leu
195 200 205

Leu Asp Leu Arg Gln Gly Met Phe Ala Gln Leu Val Ala Gln Asn Val
210 215 220

Leu Leu Ile Asp Gly Pro Leu Ser Trp Tyr Ser Asp Pro Gly Leu Ala
225 230 235 240

Gly Val Ser Leu Thr Gly Gly Leu Ser Tyr Lys Glu Asp Thr Lys Glu
245 250 255

Leu Val Val Ala Lys Ala Gly Val Tyr Tyr Val Phe Phe Gln Leu Glu
260 265 270

Leu Arg Arg Val Val Ala Gly Glu Gly Ser Gly Ser Val Ser Leu Ala
275 280 285

Leu His Leu Gln Pro Leu Arg Ser Ala Ala Gly Ala Ala Ala Leu Ala
290 295 300

Leu Thr Val Asp Leu Pro Pro Ala Ser Ser Glu Ala Arg Asn Ser Ala
305 310 315 320

Phe Gly Phe Gln Gly Arg Leu Leu His Leu Ser Ala Gly Gln Arg Leu
325 330 335

Gly Val His Leu His Thr Glu Ala Arg Ala Arg His Ala Trp Gln Leu
340 345 350

Thr Gln Gly Ala Thr Val Leu Gly Leu Phe Arg Val Thr Pro Glu Ile
355 360 365

Pro Ala Gly Leu Pro Ser Pro Arg Ser Glu
370 375

<210> 10
<211> 366
<212> PRT
<213> Artificial Sequence

<220>
<223> dimeric hu 4-1BBL (71-248) connected by (G4S)2 linker

<400> 10

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Gly Gly Gly Gly Ser Gly Gly Gly Gly Ser Arg Glu Gly Pro
180 185 190

Glu Leu Ser Pro Asp Asp Pro Ala Gly Leu Leu Asp Leu Arg Gln Gly
195 200 205

Met Phe Ala Gln Leu Val Ala Gln Asn Val Leu Leu Ile Asp Gly Pro
210 215 220

Leu Ser Trp Tyr Ser Asp Pro Gly Leu Ala Gly Val Ser Leu Thr Gly
225 230 235 240

Gly Leu Ser Tyr Lys Glu Asp Thr Lys Glu Leu Val Val Ala Lys Ala
245 250 255

Gly Val Tyr Tyr Val Phe Phe Gln Leu Glu Leu Arg Arg Val Val Ala
260 265 270

Gly Glu Gly Ser Gly Ser Val Ser Leu Ala Leu His Leu Gln Pro Leu
275 280 285

Arg Ser Ala Ala Gly Ala Ala Ala Leu Ala Leu Thr Val Asp Leu Pro
290 295 300

Pro Ala Ser Ser Glu Ala Arg Asn Ser Ala Phe Gly Phe Gln Gly Arg
305 310 315 320

Leu Leu His Leu Ser Ala Gly Gln Arg Leu Gly Val His Leu His Thr
325 330 335

Glu Ala Arg Ala Arg His Ala Trp Gln Leu Thr Gln Gly Ala Thr Val
340 345 350

Leu Gly Leu Phe Arg Val Thr Pro Glu Ile Pro Ala Gly Leu
355 360 365

<210> 11
<211> 133
<212> PRT
<213> Homo sapiens

<400> 11

Gln Val Ser His Arg Tyr Pro Arg Ile Gln Ser Ile Lys Val Gln Phe
1 5 10 15

Thr Glu Tyr Lys Lys Glu Lys Gly Phe Ile Leu Thr Ser Gln Lys Glu
20 25 30

Asp Glu Ile Met Lys Val Gln Asn Asn Ser Val Ile Ile Asn Cys Asp
35 40 45

Gly Phe Tyr Leu Ile Ser Leu Lys Gly Tyr Phe Ser Gln Glu Val Asn
50 55 60

Ile Ser Leu His Tyr Gln Lys Asp Glu Glu Pro Leu Phe Gln Leu Lys
65 70 75 80

Lys Val Arg Ser Val Asn Ser Leu Met Val Ala Ser Leu Thr Tyr Lys
85 90 95

Asp Lys Val Tyr Leu Asn Val Thr Thr Asp Asn Thr Ser Leu Asp Asp
100 105 110

Phe His Val Asn Gly Gly Glu Leu Ile Leu Ile His Gln Asn Pro Gly
115 120 125

Glu Phe Cys Val Leu
130

<210> 12
<211> 132
<212> PRT
<213> Homo sapiens

<400> 12

Val Ser His Arg Tyr Pro Arg Ile Gln Ser Ile Lys Val Gln Phe Thr
1 5 10 15

Glu Tyr Lys Lys Glu Lys Gly Phe Ile Leu Thr Ser Gln Lys Glu Asp
20 25 30

Glu Ile Met Lys Val Gln Asn Asn Ser Val Ile Ile Asn Cys Asp Gly
35 40 45

Phe Tyr Leu Ile Ser Leu Lys Gly Tyr Phe Ser Gln Glu Val Asn Ile
50 55 60

Ser Leu His Tyr Gln Lys Asp Glu Glu Pro Leu Phe Gln Leu Lys Lys
65 70 75 80

Val Arg Ser Val Asn Ser Leu Met Val Ala Ser Leu Thr Tyr Lys Asp
85 90 95

Lys Val Tyr Leu Asn Val Thr Thr Asp Asn Thr Ser Leu Asp Asp Phe
100 105 110

His Val Asn Gly Gly Glu Leu Ile Leu Ile His Gln Asn Pro Gly Glu
115 120 125

Phe Cys Val Leu
130

<210> 13
<211> 5
<212> PRT
<213> Artificial Sequence

<220>
<223> FAP(28H1) CDR-H1

<400> 13

Ser His Ala Met Ser
1 5

<210> 14
<211> 16

<212> PRT
<213> Artificial Sequence

<220>
<223> FAP(28H1) CDR-H2

<400> 14

```
Ala Ile Trp Ala Ser Gly Glu Gln Tyr Tyr Ala Asp Ser Val Lys Gly
1               5                   10                  15
```

<210> 15
<211> 8
<212> PRT
<213> Artificial Sequence

<220>
<223> FAP(28H1) CDR-H3

<400> 15

```
Gly Trp Leu Gly Asn Phe Asp Tyr
1               5
```

<210> 16
<211> 12
<212> PRT
<213> Artificial Sequence

<220>
<223> FAP(28H1) CDR-L1

<400> 16

```
Arg Ala Ser Gln Ser Val Ser Arg Ser Tyr Leu Ala
1               5                   10
```

<210> 17
<211> 7
<212> PRT
<213> Artificial Sequence

<220>
<223> FAP(28H1) CDR-L2

<400> 17

```
Gly Ala Ser Thr Arg Ala Thr
1               5
```

<210> 18
<211> 9
<212> PRT
<213> Artificial Sequence

<220>
<223> FAP(28H1) CDR-L3

<400> 18

Gln Gln Gly Gln Val Ile Pro Pro Thr
1 5

<210> 19
<211> 5
<212> PRT
<213> Artificial Sequence

<220>
<223> FAP(4B9) CDR-H1

<400> 19

Ser Tyr Ala Met Ser
1 5

<210> 20
<211> 17
<212> PRT
<213> Artificial Sequence

<220>
<223> FAP(4B9) CDR-H2

<400> 20

Ala Ile Ile Gly Ser Gly Ala Ser Thr Tyr Tyr Ala Asp Ser Val Lys
1 5 10 15

Gly

<210> 21
<211> 8
<212> PRT
<213> Artificial Sequence

<220>
<223> FAP(4B9) CDR-H3

<400> 21

Gly Trp Phe Gly Gly Phe Asn Tyr
1 5

<210> 22
<211> 12
<212> PRT
<213> Artificial Sequence

<220>
<223> FAP(4B9) CDR-L1

<400> 22

Arg Ala Ser Gln Ser Val Thr Ser Ser Tyr Leu Ala
1               5                   10

<210> 23
<211> 7
<212> PRT
<213> Artificial Sequence

<220>
<223> FAP(4B9) CDR-L2

<400> 23

Val Gly Ser Arg Arg Ala Thr
1               5

<210> 24
<211> 9
<212> PRT
<213> Artificial Sequence

<220>
<223> FAP(4B9) CDR-L3

<400> 24

Gln Gln Gly Ile Met Leu Pro Pro Thr
1               5

<210> 25
<211> 116
<212> PRT
<213> Artificial Sequence

<220>
<223> FAP(28H1) VH

<400> 25

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala

                85                  90                  95

Lys Gly Trp Leu Gly Asn Phe Asp Tyr Trp Gly Gln Gly Thr Leu Val
            100                 105                 110

Thr Val Ser Ser
        115
```

<210> 26
<211> 108
<212> PRT
<213> Artificial Sequence

<220>
<223> FAP(28H1) VL

<400> 26

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys
            100                 105
```

<210> 27
<211> 117
<212> PRT
<213> Artificial Sequence

<220>
<223> FAP(4B9) VH

<400> 27

```
Glu Val Gln Leu Leu Glu Ser Gly Gly Gly Leu Val Gln Pro Gly Gly
1               5                   10                  15
```

```
Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser
        115
```

<210> 28
<211> 108
<212> PRT
<213> Artificial Sequence

<220>
<223> FAP(4B9) VL

<400> 28

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

Tyr Leu Ala Trp Tyr Gln Gln Lys Pro Gly Gln Ala Pro Arg Leu Leu
        35                  40                  45

Ile Asn Val Gly Ser Arg Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Ile Met Leu Pro
                85                  90                  95

Pro Thr Phe Gly Gln Gly Thr Lys Val Glu Ile Lys

                    100                 105
```

<210> 29
<211> 5
<212> PRT
<213> Artificial Sequence

<220>
<223> CD19 (8B8-018) CDR-H1

<400> 29

```
Asp Tyr Ile Met His
1               5
```

<210> 30
<211> 17
<212> PRT
<213> Artificial Sequence

<220>
<223> CD19 (8B8-018) CDR-H2

<400> 30

```
Tyr Ile Asn Pro Tyr Asn Asp Gly Ser Lys Tyr Thr Glu Lys Phe Gln
1               5                   10                  15

Gly
```

<210> 31

<211> 12
<212> PRT
<213> Artificial Sequence

<220>
<223> CD19 (8B8-018) CDR-H3

<400> 31

```
Gly Thr Tyr Tyr Tyr Gly Ser Ala Leu Phe Asp Tyr
1               5                   10
```

<210> 32
<211> 16
<212> PRT
<213> Artificial Sequence

<220>
<223> CD19 (8B8-018) CDR-L1

<400> 32

```
Lys Ser Ser Gln Ser Leu Glu Asn Pro Asn Gly Asn Thr Tyr Leu Asn
1               5                   10                  15
```

<210> 33
<211> 7
<212> PRT
<213> Artificial Sequence

<220>
<223> CD19 (8B8-018) CDR-L2

<400> 33

```
Arg Val Ser Lys Arg Phe Ser
1               5
```

<210> 34
<211> 9
<212> PRT
<213> Artificial Sequence

<220>
<223> CD19 (8B8-018) CDR-L3

<400> 34

```
Leu Gln Leu Thr His Val Pro Tyr Thr
1               5
```

<210> 35
<211> 5
<212> PRT
<213> Artificial Sequence

<220>
<223> CD19 (8B8-2B11) CDR-H1

<400> 35

Asp Tyr Ile Met His
1               5

<210> 36
<211> 17
<212> PRT
<213> Artificial Sequence

<220>
<223> CD19 (8B8-2B11) CDR-H2

<400> 36

Tyr Ile Asn Pro Tyr Asn Asp Gly Ser Lys Tyr Thr Glu Lys Phe Gln
1               5                   10                  15

Gly

<210> 37
<211> 12
<212> PRT
<213> Artificial Sequence

<220>
<223> CD19 (8B8-2B11) CDR-H3

<400> 37

Gly Thr Tyr Tyr Tyr Gly Pro Gln Leu Phe Asp Tyr
1               5                   10

<210> 38
<211> 16
<212> PRT
<213> Artificial Sequence

<220>
<223> CD19 (8B8-2B11) CDR-L1

<400> 38

Lys Ser Ser Gln Ser Leu Glu Thr Ser Thr Gly Thr Thr Tyr Leu Asn
1               5                   10                  15

<210> 39
<211> 7
<212> PRT
<213> Artificial Sequence

<220>
<223> CD19 (8B8-2B11) CDR-L2

<400> 39

Arg Val Ser Lys Arg Phe Ser
1 5

<210> 40
<211> 9
<212> PRT
<213> Artificial Sequence

<220>
<223> CD19 (8B8-2B11) CDR-L3

<400> 40

Leu Gln Leu Leu Glu Asp Pro Tyr Thr
1 5

<210> 41
<211> 121
<212> PRT
<213> Artificial Sequence

<220>
<223> CD19 (8B8-018) VH

<400> 41

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Ile Met His Trp Val Arg Gln Ala Pro Gly Gln Gly Leu Glu Trp Met
35 40 45

Gly Tyr Ile Asn Pro Tyr Asn Asp Gly Ser Lys Tyr Thr Glu Lys Phe
50 55 60

Gln Gly Arg Val Thr Met Thr Ser Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Gly Thr Tyr Tyr Tyr Gly Ser Ala Leu Phe Asp Tyr Trp Gly
100 105 110

Gln Gly Thr Thr Val Thr Val Ser Ser
115 120

<210> 42
<211> 112

<212> PRT
<213> Artificial Sequence

<220>
<223> CD19 (8B8-018) VL

<400> 42

```
Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Asn Pro
            20                  25                  30

Asn Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu

                85                  90                  95

Thr His Val Pro Tyr Thr Phe Gly Gln Gly Thr Lys Leu Glu Ile Lys
            100                 105                 110
```

<210> 43
<211> 121
<212> PRT
<213> Artificial Sequence

<220>
<223> CD19 (8B8-2B11) VH

<400> 43

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Ile Met His Trp Val Arg Gln Ala Pro Gly Gln Gly Leu Glu Trp Met
35 40 45

Gly Tyr Ile Asn Pro Tyr Asn Asp Gly Ser Lys Tyr Thr Glu Lys Phe
50 55 60

Gln Gly Arg Val Thr Met Thr Ser Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Gly Thr Tyr Tyr Tyr Gly Pro Gln Leu Phe Asp Tyr Trp Gly
100 105 110

Gln Gly Thr Thr Val Thr Val Ser Ser
115 120

<210> 44
<211> 112
<212> PRT
<213> Artificial Sequence

<220>
<223> CD19 (8B8-2B11) VL

<400> 44

Asp Ile Val Met Thr Gln Thr Pro Leu Ser Leu Ser Val Thr Pro Gly
1 5 10 15

Gln Pro Ala Ser Ile Ser Cys Lys Ser Ser Gln Ser Leu Glu Thr Ser
20 25 30

Thr Gly Thr Thr Tyr Leu Asn Trp Tyr Leu Gln Lys Pro Gly Gln Ser
35 40 45

Pro Gln Leu Leu Ile Tyr Arg Val Ser Lys Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Leu Gln Leu
85 90 95

Leu Glu Asp Pro Tyr Thr Phe Gly Gln Gly Thr Lys Leu Glu Ile Lys
100 105 110

<210> 45
<211> 5
<212> PRT
<213> Artificial Sequence

<220>
<223> CEA CDR-H1

<400> 45

Asp Thr Tyr Met His
1 5

<210> 46
<211> 17
<212> PRT
<213> Artificial Sequence

<220>
<223> CEA CDR-H2

<400> 46

Arg Ile Asp Pro Ala Asn Gly Asn Ser Lys Tyr Val Pro Lys Phe Gln
1 5 10 15

Gly

<210> 47
<211> 12
<212> PRT
<213> Artificial Sequence

<220>
<223> CEA CDR-H3

<400> 47

Phe Gly Tyr Tyr Val Ser Asp Tyr Ala Met Ala Tyr
1 5 10

<210> 48
<211> 15
<212> PRT
<213> Artificial Sequence

<220>
<223> CEA CDR-L1

<400> 48

Arg Ala Gly Glu Ser Val Asp Ile Phe Gly Val Gly Phe Leu His
1 5 10 15

<210> 49
<211> 7
<212> PRT
<213> Artificial Sequence

<220>
<223> CEA CDR-L2

<400> 49

Arg Ala Ser Asn Arg Ala Thr
1 5

<210> 50
<211> 9
<212> PRT
<213> Artificial Sequence

<220>
<223> CEA CDR-L3

<400> 50

Gln Gln Thr Asn Glu Asp Pro Tyr Thr
1 5

<210> 51
<211> 121
<212> PRT
<213> Artificial Sequence

<220>
<223> Humanized CEA binder VH

<400> 51

Gln Val Gln Leu Val Gln Ser Gly Ala Glu Val Lys Lys Pro Gly Ser
1 5 10 15

Ser Val Lys Val Ser Cys Lys Ala Ser Gly Phe Asn Ile Lys Asp Thr
20 25 30

Tyr Met His Trp Val Arg Gln Ala Pro Gly Gln Gly Leu Glu Trp Met
35 40 45

Gly Arg Ile Asp Pro Ala Asn Gly Asn Ser Lys Tyr Val Pro Lys Phe
50 55 60

Gln Gly Arg Val Thr Ile Thr Ala Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Pro Phe Gly Tyr Tyr Val Ser Asp Tyr Ala Met Ala Tyr Trp Gly
100 105 110

Gln Gly Thr Leu Val Thr Val Ser Ser
115 120

<210> 52
<211> 111
<212> PRT
<213> Artificial Sequence

<220>
<223> Humanized CEA binder VL

<400> 52

```
Glu Ile Val Leu Thr Gln Ser Pro Ala Thr Leu Ser Leu Ser Pro Gly
1               5                   10                  15

Glu Arg Ala Thr Leu Ser Cys Arg Ala Gly Glu Ser Val Asp Ile Phe
            20                  25                  30

Gly Val Gly Phe Leu His Trp Tyr Gln Gln Lys Pro Gly Gln Ala Pro
        35                  40                  45

Arg Leu Leu Ile Tyr Arg Ala Ser Asn Arg Ala Thr Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Thr Asn
                85                  90                  95

Glu Asp Pro Tyr Thr Phe Gly Gln Gly Thr Lys Leu Glu Ile Lys

            100                 105                 110
```

<210> 53
<211> 760
<212> PRT
<213> Homo sapiens

<400> 53

```
Met Lys Thr Trp Val Lys Ile Val Phe Gly Val Ala Thr Ser Ala Val
1               5                   10                  15

Leu Ala Leu Leu Val Met Cys Ile Val Leu Arg Pro Ser Arg Val His
            20                  25                  30

Asn Ser Glu Glu Asn Thr Met Arg Ala Leu Thr Leu Lys Asp Ile Leu
        35                  40                  45

Asn Gly Thr Phe Ser Tyr Lys Thr Phe Phe Pro Asn Trp Ile Ser Gly
    50                  55                  60

Gln Glu Tyr Leu His Gln Ser Ala Asp Asn Asn Ile Val Leu Tyr Asn
65                  70                  75                  80

Ile Glu Thr Gly Gln Ser Tyr Thr Ile Leu Ser Asn Arg Thr Met Lys
                85                  90                  95

Ser Val Asn Ala Ser Asn Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Ser Asn Gly Glu Phe Val Arg Gly Asn
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Phe Asn Gly Arg Glu Asn Lys Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
```

210 215 220

Glu Phe Asn Asp Thr Asp Ile Pro Val Ile Ala Tyr Ser Tyr Tyr Gly
225 230 235 240

Asp Glu Gln Tyr Pro Arg Thr Ile Asn Ile Pro Tyr Pro Lys Ala Gly
245 250 255

Ala Lys Asn Pro Val Val Arg Ile Phe Ile Ile Asp Thr Thr Tyr Pro
260 265 270

Ala Tyr Val Gly Pro Gln Glu Val Pro Val Pro Ala Met Ile Ala Ser
275 280 285

Ser Asp Tyr Tyr Phe Ser Trp Leu Thr Trp Val Thr Asp Glu Arg Val
290 295 300

Cys Leu Gln Trp Leu Lys Arg Val Gln Asn Val Ser Val Leu Ser Ile
305 310 315 320

Cys Asp Phe Arg Glu Asp Trp Gln Thr Trp Asp Cys Pro Lys Thr Gln
325 330 335

Glu His Ile Glu Glu Ser Arg Thr Gly Trp Ala Gly Gly Phe Phe Val
340 345 350

Ser Thr Pro Val Phe Ser Tyr Asp Ala Ile Ser Tyr Tyr Lys Ile Phe
355 360 365

Ser Asp Lys Asp Gly Tyr Lys His Ile His Tyr Ile Lys Asp Thr Val
370 375 380

Glu Asn Ala Ile Gln Ile Thr Ser Gly Lys Trp Glu Ala Ile Asn Ile
385 390 395 400

Phe Arg Val Thr Gln Asp Ser Leu Phe Tyr Ser Ser Asn Glu Phe Glu
405 410 415

Glu Tyr Pro Gly Arg Arg Asn Ile Tyr Arg Ile Ser Ile Gly Ser Tyr
420 425 430

Pro Pro Ser Lys Lys Cys Val Thr Cys His Leu Arg Lys Glu Arg Cys
435 440 445

Gln Tyr Tyr Thr Ala Ser Phe Ser Asp Tyr Ala Lys Tyr Tyr Ala Leu
450 455 460

```
Val Cys Tyr Gly Pro Gly Ile Pro Ile Ser Thr Leu His Asp Gly Arg
465                 470                 475                 480

Thr Asp Gln Glu Ile Lys Ile Leu Glu Glu Asn Lys Glu Leu Glu Asn
                485                 490                 495

Ala Leu Lys Asn Ile Gln Leu Pro Lys Glu Glu Ile Lys Lys Leu Glu
            500                 505                 510

Val Asp Glu Ile Thr Leu Trp Tyr Lys Met Ile Leu Pro Pro Gln Phe
        515                 520                 525

Asp Arg Ser Lys Lys Tyr Pro Leu Leu Ile Gln Val Tyr Gly Gly Pro
    530                 535                 540

Cys Ser Gln Ser Val Arg Ser Val Phe Ala Val Asn Trp Ile Ser Tyr
545                 550                 555                 560

Leu Ala Ser Lys Glu Gly Met Val Ile Ala Leu Val Asp Gly Arg Gly
                565                 570                 575

Thr Ala Phe Gln Gly Asp Lys Leu Leu Tyr Ala Val Tyr Arg Lys Leu
            580                 585                 590

Gly Val Tyr Glu Val Glu Asp Gln Ile Thr Ala Val Arg Lys Phe Ile
        595                 600                 605

Glu Met Gly Phe Ile Asp Glu Lys Arg Ile Ala Ile Trp Gly Trp Ser
    610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
                645                 650                 655

Ala Ser Val Tyr Thr Glu Arg Phe Met Gly Leu Pro Thr Lys Asp Asp
            660                 665                 670

Asn Leu Glu His Tyr Lys Asn Ser Thr Val Met Ala Arg Ala Glu Tyr
        675                 680                 685

Phe Arg Asn Val Asp Tyr Leu Leu Ile His Gly Thr Ala Asp Asp Asn
    690                 695                 700

Val His Phe Gln Asn Ser Ala Gln Ile Ala Lys Ala Leu Val Asn Ala
705                 710                 715                 720
```

Gln Val Asp Phe Gln Ala Met Trp Tyr Ser Asp Gln Asn His Gly Leu
725 730 735

Ser Gly Leu Ser Thr Asn His Leu Tyr Thr His Met Thr His Phe Leu
740 745 750

Lys Gln Cys Phe Ser Leu Ser Asp
755 760

<210> 54
<211> 748
<212> PRT
<213> Artificial Sequence

<220>
<223> hu FAP ectodomain+poly-lys-tag+his6-tag

<400> 54

Arg Pro Ser Arg Val His Asn Ser Glu Glu Asn Thr Met Arg Ala Leu
1 5 10 15

Thr Leu Lys Asp Ile Leu Asn Gly Thr Phe Ser Tyr Lys Thr Phe Phe
20 25 30

Pro Asn Trp Ile Ser Gly Gln Glu Tyr Leu His Gln Ser Ala Asp Asn
35 40 45

Asn Ile Val Leu Tyr Asn Ile Glu Thr Gly Gln Ser Tyr Thr Ile Leu
50 55 60

Ser Asn Arg Thr Met Lys Ser Val Asn Ala Ser Asn Tyr Gly Leu Ser
65 70 75 80

Pro Asp Arg Gln Phe Val Tyr Leu Glu Ser Asp Tyr Ser Lys Leu Trp
85 90 95

Arg Tyr Ser Tyr Thr Ala Thr Tyr Tyr Ile Tyr Asp Leu Ser Asn Gly
100 105 110

Glu Phe Val Arg Gly Asn Glu Leu Pro Arg Pro Ile Gln Tyr Leu Cys
115 120 125

Trp Ser Pro Val Gly Ser Lys Leu Ala Tyr Val Tyr Gln Asn Asn Ile
130 135 140

Tyr Leu Lys Gln Arg Pro Gly Asp Pro Pro Phe Gln Ile Thr Phe Asn
145 150 155 160

Gly Arg Glu Asn Lys Ile Phe Asn Gly Ile Pro Asp Trp Val Tyr Glu
165 170 175

Glu Glu Met Leu Ala Thr Lys Tyr Ala Leu Trp Trp Ser Pro Asn Gly
180 185 190

Lys Phe Leu Ala Tyr Ala Glu Phe Asn Asp Thr Asp Ile Pro Val Ile
195 200 205

Ala Tyr Ser Tyr Tyr Gly Asp Glu Gln Tyr Pro Arg Thr Ile Asn Ile
210 215 220

Pro Tyr Pro Lys Ala Gly Ala Lys Asn Pro Val Val Arg Ile Phe Ile
225 230 235 240

Ile Asp Thr Thr Tyr Pro Ala Tyr Val Gly Pro Gln Glu Val Pro Val
245 250 255

Pro Ala Met Ile Ala Ser Ser Asp Tyr Tyr Phe Ser Trp Leu Thr Trp
260 265 270

Val Thr Asp Glu Arg Val Cys Leu Gln Trp Leu Lys Arg Val Gln Asn
275 280 285

Val Ser Val Leu Ser Ile Cys Asp Phe Arg Glu Asp Trp Gln Thr Trp
290 295 300

Asp Cys Pro Lys Thr Gln Glu His Ile Glu Glu Ser Arg Thr Gly Trp
305 310 315 320

Ala Gly Gly Phe Phe Val Ser Thr Pro Val Phe Ser Tyr Asp Ala Ile
325 330 335

Ser Tyr Tyr Lys Ile Phe Ser Asp Lys Asp Gly Tyr Lys His Ile His
340 345 350

Tyr Ile Lys Asp Thr Val Glu Asn Ala Ile Gln Ile Thr Ser Gly Lys
355 360 365

Trp Glu Ala Ile Asn Ile Phe Arg Val Thr Gln Asp Ser Leu Phe Tyr
370 375 380

Ser Ser Asn Glu Phe Glu Glu Tyr Pro Gly Arg Arg Asn Ile Tyr Arg
385 390 395 400

Ile Ser Ile Gly Ser Tyr Pro Pro Ser Lys Lys Cys Val Thr Cys His
405 410 415

```
Leu Arg Lys Glu Arg Cys Gln Tyr Tyr Thr Ala Ser Phe Ser Asp Tyr
            420                 425                 430

Ala Lys Tyr Tyr Ala Leu Val Cys Tyr Gly Pro Gly Ile Pro Ile Ser
        435                 440                 445

Thr Leu His Asp Gly Arg Thr Asp Gln Glu Ile Lys Ile Leu Glu Glu
    450                 455                 460

Asn Lys Glu Leu Glu Asn Ala Leu Lys Asn Ile Gln Leu Pro Lys Glu
465                 470                 475                 480

Glu Ile Lys Lys Leu Glu Val Asp Glu Ile Thr Leu Trp Tyr Lys Met
                485                 490                 495

Ile Leu Pro Pro Gln Phe Asp Arg Ser Lys Lys Tyr Pro Leu Leu Ile
            500                 505                 510

Gln Val Tyr Gly Gly Pro Cys Ser Gln Ser Val Arg Ser Val Phe Ala
        515                 520                 525

Val Asn Trp Ile Ser Tyr Leu Ala Ser Lys Glu Gly Met Val Ile Ala
    530                 535                 540

Leu Val Asp Gly Arg Gly Thr Ala Phe Gln Gly Asp Lys Leu Leu Tyr
545                 550                 555                 560

Ala Val Tyr Arg Lys Leu Gly Val Tyr Glu Val Glu Asp Gln Ile Thr
                565                 570                 575

Ala Val Arg Lys Phe Ile Glu Met Gly Phe Ile Asp Glu Lys Arg Ile
            580                 585                 590

Ala Ile Trp Gly Trp Ser Tyr Gly Gly Tyr Val Ser Ser Leu Ala Leu
        595                 600                 605

Ala Ser Gly Thr Gly Leu Phe Lys Cys Gly Ile Ala Val Ala Pro Val
    610                 615                 620

Ser Ser Trp Glu Tyr Tyr Ala Ser Val Tyr Thr Glu Arg Phe Met Gly
625                 630                 635                 640

Leu Pro Thr Lys Asp Asp Asn Leu Glu His Tyr Lys Asn Ser Thr Val
                645                 650                 655

Met Ala Arg Ala Glu Tyr Phe Arg Asn Val Asp Tyr Leu Leu Ile His
            660                 665                 670
```

Gly Thr Ala Asp Asp Asn Val His Phe Gln Asn Ser Ala Gln Ile Ala
675 680 685

Lys Ala Leu Val Asn Ala Gln Val Asp Phe Gln Ala Met Trp Tyr Ser
690 695 700

Asp Gln Asn His Gly Leu Ser Gly Leu Ser Thr Asn His Leu Tyr Thr
705 710 715 720

His Met Thr His Phe Leu Lys Gln Cys Phe Ser Leu Ser Asp Gly Lys
725 730 735

Lys Lys Lys Lys Lys Gly His His His His His His
740 745

<210> 55
<211> 2244
<212> DNA
<213> Artificial Sequence

<220>
<223> nucleotide sequence of hu FAP ectodomain+poly-lys-tag+his6-tag

<400> 55

cgcccttcaa gagttcataa ctctgaagaa aatacaatga gagcactcac actgaaggat 60
attttaaatg gaacattttc ttataaaaca tttttccaa actggatttc aggacaagaa 120
tatcttcatc aatctgcaga taacaatata gtactttata atattgaaac aggacaatca 180
tataccattt tgagtaatag aaccatgaaa agtgtgaatg cttcaaatta cggcttatca 240
cctgatcggc aatttgtata tctagaaagt gattattcaa agctttggag atactcttac 300
acagcaacat attacatcta tgaccttagc aatggagaat ttgtaagagg aaatgagctt 360
cctcgtccaa ttcagtattt atgctggtcg cctgttggga gtaaattagc atatgtctat 420
caaaacaata tctatttgaa acaaagacca ggagatccac cttttcaaat aacatttaat 480
ggaagagaaa ataaaatatt taatggaatc ccagactggg tttatgaaga ggaaatgctt 540
gctacaaaat atgctctctg gtggtctcct aatggaaaat ttttggcata tgcggaattt 600
aatgatacgg atataccagt tattgcctat tcctattatg gcgatgaaca atatcctaga 660
acaataaata ttccataccc aaaggctgga gctaagaatc ccgttgttcg gatatttatt 720
atcgatacca cttaccctgc gtatgtaggt ccccaggaag tgcctgttcc agcaatgata 780
gcctcaagtg attattattt cagttggctc acgtgggtta ctgatgaacg agtatgtttg 840
cagtggctaa aaagagtcca gaatgtttcg gtcctgtcta tatgtgactt cagggaagac 900
tggcagacat gggattgtcc aaagacccag gagcatatag aagaaagcag aactggatgg 960

gctggtggat tctttgtttc aacaccagtt ttcagctatg atgccatttc gtactacaaa 1020

atatttagtg acaaggatgg ctacaaacat attcactata tcaaagacac tgtggaaaat 1080

gctattcaaa ttacaagtgg caagtgggag gccataaata tattcagagt aacacaggat 1140

tcactgtttt attctagcaa tgaatttgaa gaataccctg gaagaagaaa catctacaga 1200

attagcattg gaagctatcc tccaagcaag aagtgtgtta cttgccatct aaggaaagaa 1260

aggtgccaat attacacagc aagtttcagc gactacgcca agtactatgc acttgtctgc 1320

tacggcccag gcatccccat ttccaccctt catgatggac gcactgatca agaaattaaa 1380

atcctggaag aaaacaagga attggaaaat gctttgaaaa atatccagct gcctaaagag 1440

gaaattaaga aacttgaagt agatgaaatt actttatggt acaagatgat tcttcctcct 1500

caatttgaca gatcaaagaa gtatcccttg ctaattcaag tgtatggtgg tccctgcagt 1560

cagagtgtaa ggtctgtatt tgctgttaat tggatatctt atcttgcaag taaggaaggg 1620

atggtcattg ccttggtgga tggtcgagga acagctttcc aaggtgacaa actcctctat 1680

gcagtgtatc gaaagctggg tgtttatgaa gttgaagacc agattacagc tgtcagaaaa 1740

ttcatagaaa tgggtttcat tgatgaaaaa agaatagcca tatggggctg gtcctatgga 1800

ggatacgttt catcactggc ccttgcatct ggaactggtc ttttcaaatg tggtatagca 1860

gtggctccag tctccagctg ggaatattac gcgtctgtct acacagagag attcatgggt 1920

ctcccaacaa aggatgataa tcttgagcac tataagaatt caactgtgat ggcaagagca 1980

gaatatttca gaaatgtaga ctatcttctc atccacggaa cagcagatga taatgtgcac 2040

tttcaaaact cagcacagat tgctaaagct ctggttaatg cacaagtgga tttccaggca 2100

atgtggtact ctgaccagaa ccacggctta tccggcctgt ccacgaacca cttatacacc 2160

cacatgaccc acttcctaaa gcagtgtttc tctttgtcag acggcaaaaa gaaaaagaaa 2220

aagggccacc accatcacca tcac 2244

<210> 56
<211> 761
<212> PRT
<213> mus musculus

<400> 56

```
Met Lys Thr Trp Leu Lys Thr Val Phe Gly Val Thr Thr Leu Ala Ala
1               5                   10                  15

Leu Ala Leu Val Val Ile Cys Ile Val Leu Arg Pro Ser Arg Val Tyr
            20                  25                  30

Lys Pro Glu Gly Asn Thr Lys Arg Ala Leu Thr Leu Lys Asp Ile Leu
        35                  40                  45
```

```
Asn Gly Thr Phe Ser Tyr Lys Thr Tyr Phe Pro Asn Trp Ile Ser Glu
    50                  55                  60

Gln Glu Tyr Leu His Gln Ser Glu Asp Asp Asn Ile Val Phe Tyr Asn
65                  70                  75                  80

Ile Glu Thr Arg Glu Ser Tyr Ile Ile Leu Ser Asn Ser Thr Met Lys
                85                  90                  95

Ser Val Asn Ala Thr Asp Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Gln Asn Gly Glu Phe Val Arg Gly Tyr
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Tyr Thr Gly Arg Glu Asn Arg Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asp Gly Lys Phe Leu Ala Tyr Val
    210                 215                 220

Glu Phe Asn Asp Ser Asp Ile Pro Ile Ile Ala Tyr Ser Tyr Tyr Gly
225                 230                 235                 240

Asp Gly Gln Tyr Pro Arg Thr Ile Asn Ile Pro Tyr Pro Lys Ala Gly
                245                 250                 255

Ala Lys Asn Pro Val Val Arg Val Phe Ile Val Asp Thr Thr Tyr Pro
            260                 265                 270

His His Val Gly Pro Met Glu Val Pro Val Pro Glu Met Ile Ala Ser
        275                 280                 285

Ser Asp Tyr Tyr Phe Ser Trp Leu Thr Trp Val Ser Ser Glu Arg Val
    290                 295                 300
```

Cys Leu Gln Trp Leu Lys Arg Val Gln Asn Val Ser Val Leu Ser Ile
305 310 315 320

Cys Asp Phe Arg Glu Asp Trp His Ala Trp Glu Cys Pro Lys Asn Gln
325 330 335

Glu His Val Glu Glu Ser Arg Thr Gly Trp Ala Gly Gly Phe Phe Val
340 345 350

Ser Thr Pro Ala Phe Ser Gln Asp Ala Thr Ser Tyr Tyr Lys Ile Phe
355 360 365

Ser Asp Lys Asp Gly Tyr Lys His Ile His Tyr Ile Lys Asp Thr Val
370 375 380

Glu Asn Ala Ile Gln Ile Thr Ser Gly Lys Trp Glu Ala Ile Tyr Ile
385 390 395 400

Phe Arg Val Thr Gln Asp Ser Leu Phe Tyr Ser Ser Asn Glu Phe Glu
405 410 415

Gly Tyr Pro Gly Arg Arg Asn Ile Tyr Arg Ile Ser Ile Gly Asn Ser
420 425 430

Pro Pro Ser Lys Lys Cys Val Thr Cys His Leu Arg Lys Glu Arg Cys
435 440 445

Gln Tyr Tyr Thr Ala Ser Phe Ser Tyr Lys Ala Lys Tyr Tyr Ala Leu
450 455 460

Val Cys Tyr Gly Pro Gly Leu Pro Ile Ser Thr Leu His Asp Gly Arg
465 470 475 480

Thr Asp Gln Glu Ile Gln Val Leu Glu Glu Asn Lys Glu Leu Glu Asn
485 490 495

Ser Leu Arg Asn Ile Gln Leu Pro Lys Val Glu Ile Lys Lys Leu Lys
500 505 510

Asp Gly Gly Leu Thr Phe Trp Tyr Lys Met Ile Leu Pro Pro Gln Phe
515 520 525

Asp Arg Ser Lys Lys Tyr Pro Leu Leu Ile Gln Val Tyr Gly Gly Pro
530 535 540

Cys Ser Gln Ser Val Lys Ser Val Phe Ala Val Asn Trp Ile Thr Tyr

545 550 555 560

Leu Ala Ser Lys Glu Gly Ile Val Ile Ala Leu Val Asp Gly Arg Gly
565 570 575

Thr Ala Phe Gln Gly Asp Lys Phe Leu His Ala Val Tyr Arg Lys Leu
580 585 590

Gly Val Tyr Glu Val Glu Asp Gln Leu Thr Ala Val Arg Lys Phe Ile
595 600 605

Glu Met Gly Phe Ile Asp Glu Glu Arg Ile Ala Ile Trp Gly Trp Ser
610 615 620

Tyr Gly Gly Tyr Val Ser Ser Leu Ala Leu Ala Ser Gly Thr Gly Leu
625 630 635 640

Phe Lys Cys Gly Ile Ala Val Ala Pro Val Ser Ser Trp Glu Tyr Tyr
645 650 655

Ala Ser Ile Tyr Ser Glu Arg Phe Met Gly Leu Pro Thr Lys Asp Asp
660 665 670

Asn Leu Glu His Tyr Lys Asn Ser Thr Val Met Ala Arg Ala Glu Tyr
675 680 685

Phe Arg Asn Val Asp Tyr Leu Leu Ile His Gly Thr Ala Asp Asp Asn
690 695 700

Val His Phe Gln Asn Ser Ala Gln Ile Ala Lys Ala Leu Val Asn Ala
705 710 715 720

Gln Val Asp Phe Gln Ala Met Trp Tyr Ser Asp Gln Asn His Gly Ile
725 730 735

Ser Ser Gly Arg Ser Gln Asn His Leu Tyr Thr His Met Thr His Phe
740 745 750

Leu Lys Gln Cys Phe Ser Leu Ser Asp
755 760

<210> 57
<211> 749
<212> PRT
<213> Artificial Sequence

<220>
<223> Murine FAP ectodomain+poly-lys-tag+his6-tag

<400> 57

```
Arg Pro Ser Arg Val Tyr Lys Pro Glu Gly Asn Thr Lys Arg Ala Leu
1               5                   10                  15

Thr Leu Lys Asp Ile Leu Asn Gly Thr Phe Ser Tyr Lys Thr Tyr Phe
            20                  25                  30

Pro Asn Trp Ile Ser Glu Gln Glu Tyr Leu His Gln Ser Glu Asp Asp
        35                  40                  45

Asn Ile Val Phe Tyr Asn Ile Glu Thr Arg Glu Ser Tyr Ile Ile Leu
    50                  55                  60

Ser Asn Ser Thr Met Lys Ser Val Asn Ala Thr Asp Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Gln Asn Gly
            100                 105                 110

Glu Phe Val Arg Gly Tyr Glu Leu Pro Arg Pro Ile Gln Tyr Leu Cys
        115                 120                 125

Trp Ser Pro Val Gly Ser Lys Leu Ala Tyr Val Tyr Gln Asn Asn Ile
    130                 135                 140

Tyr Leu Lys Gln Arg Pro Gly Asp Pro Pro Phe Gln Ile Thr Tyr Thr
145                 150                 155                 160

Gly Arg Glu Asn Arg Ile Phe Asn Gly Ile Pro Asp Trp Val Tyr Glu
                165                 170                 175

Glu Glu Met Leu Ala Thr Lys Tyr Ala Leu Trp Trp Ser Pro Asp Gly
            180                 185                 190

Lys Phe Leu Ala Tyr Val Glu Phe Asn Asp Ser Asp Ile Pro Ile Ile
        195                 200                 205

Ala Tyr Ser Tyr Tyr Gly Asp Gly Gln Tyr Pro Arg Thr Ile Asn Ile
    210                 215                 220

Pro Tyr Pro Lys Ala Gly Ala Lys Asn Pro Val Val Arg Val Phe Ile
225                 230                 235                 240

Val Asp Thr Thr Tyr Pro His His Val Gly Pro Met Glu Val Pro Val
```

245 250 255

Pro Glu Met Ile Ala Ser Ser Asp Tyr Tyr Phe Ser Trp Leu Thr Trp
260 265 270

Val Ser Ser Glu Arg Val Cys Leu Gln Trp Leu Lys Arg Val Gln Asn
275 280 285

Val Ser Val Leu Ser Ile Cys Asp Phe Arg Glu Asp Trp His Ala Trp
290 295 300

Glu Cys Pro Lys Asn Gln Glu His Val Glu Glu Ser Arg Thr Gly Trp
305 310 315 320

Ala Gly Gly Phe Phe Val Ser Thr Pro Ala Phe Ser Gln Asp Ala Thr
325 330 335

Ser Tyr Tyr Lys Ile Phe Ser Asp Lys Asp Gly Tyr Lys His Ile His
340 345 350

Tyr Ile Lys Asp Thr Val Glu Asn Ala Ile Gln Ile Thr Ser Gly Lys
355 360 365

Trp Glu Ala Ile Tyr Ile Phe Arg Val Thr Gln Asp Ser Leu Phe Tyr
370 375 380

Ser Ser Asn Glu Phe Glu Gly Tyr Pro Gly Arg Arg Asn Ile Tyr Arg
385 390 395 400

Ile Ser Ile Gly Asn Ser Pro Pro Ser Lys Lys Cys Val Thr Cys His
405 410 415

Leu Arg Lys Glu Arg Cys Gln Tyr Tyr Thr Ala Ser Phe Ser Tyr Lys
420 425 430

Ala Lys Tyr Tyr Ala Leu Val Cys Tyr Gly Pro Gly Leu Pro Ile Ser
435 440 445

Thr Leu His Asp Gly Arg Thr Asp Gln Glu Ile Gln Val Leu Glu Glu
450 455 460

Asn Lys Glu Leu Glu Asn Ser Leu Arg Asn Ile Gln Leu Pro Lys Val
465 470 475 480

Glu Ile Lys Lys Leu Lys Asp Gly Gly Leu Thr Phe Trp Tyr Lys Met
485 490 495

```
Ile Leu Pro Pro Gln Phe Asp Arg Ser Lys Lys Tyr Pro Leu Leu Ile
            500                 505                 510

Gln Val Tyr Gly Gly Pro Cys Ser Gln Ser Val Lys Ser Val Phe Ala
        515                 520                 525

Val Asn Trp Ile Thr Tyr Leu Ala Ser Lys Glu Gly Ile Val Ile Ala
    530                 535                 540

Leu Val Asp Gly Arg Gly Thr Ala Phe Gln Gly Asp Lys Phe Leu His
545                 550                 555                 560

Ala Val Tyr Arg Lys Leu Gly Val Tyr Glu Val Glu Asp Gln Leu Thr
                565                 570                 575

Ala Val Arg Lys Phe Ile Glu Met Gly Phe Ile Asp Glu Glu Arg Ile
            580                 585                 590

Ala Ile Trp Gly Trp Ser Tyr Gly Gly Tyr Val Ser Ser Leu Ala Leu
        595                 600                 605

Ala Ser Gly Thr Gly Leu Phe Lys Cys Gly Ile Ala Val Ala Pro Val
    610                 615                 620

Ser Ser Trp Glu Tyr Tyr Ala Ser Ile Tyr Ser Glu Arg Phe Met Gly
625                 630                 635                 640

Leu Pro Thr Lys Asp Asp Asn Leu Glu His Tyr Lys Asn Ser Thr Val
                645                 650                 655

Met Ala Arg Ala Glu Tyr Phe Arg Asn Val Asp Tyr Leu Leu Ile His
            660                 665                 670

Gly Thr Ala Asp Asp Asn Val His Phe Gln Asn Ser Ala Gln Ile Ala
        675                 680                 685

Lys Ala Leu Val Asn Ala Gln Val Asp Phe Gln Ala Met Trp Tyr Ser
    690                 695                 700

Asp Gln Asn His Gly Ile Leu Ser Gly Arg Ser Gln Asn His Leu Tyr
705                 710                 715                 720

Thr His Met Thr His Phe Leu Lys Gln Cys Phe Ser Leu Ser Asp Gly
                725                 730                 735

Lys Lys Lys Lys Lys Lys Gly His His His His His His
            740                 745
```

<210> 58
<211> 2247
<212> DNA
<213> Artificial Sequence

<220>
<223> nucleotide sequence of murine FAP ectodomain+poly-lys-tag+his6-tag

<400> 58

```
cgtccctcaa gagtttacaa acctgaagga aacacaaaga gagctcttac cttgaaggat      60
attttaaatg gaacattctc atataaaaca tattttccca actggatttc agaacaagaa     120
tatcttcatc aatctgagga tgataacata gtattttata atattgaaac aagagaatca     180
tatatcattt tgagtaatag caccatgaaa agtgtgaatg ctacagatta tggtttgtca     240
cctgatcggc aatttgtgta tctagaaagt gattattcaa agctctggcg atattcatac     300
acagcgacat actacatcta cgaccttcag aatggggaat ttgtaagagg atacgagctc     360
cctcgtccaa ttcagtatct atgctggtcg cctgttggga gtaaattagc atatgtatat     420
caaaacaata tttatttgaa acaaagacca ggagatccac cttttcaaat aacttatact     480
ggaagagaaa atagaatatt taatggaata ccagactggg tttatgaaga ggaaatgctt     540
gccacaaaat atgctctttg gtggtctcca gatggaaaat tttggcata tgtagaattt      600
aatgattcag atataccaat tattgcctat tcttattatg gtgatggaca gtatcctaga     660
actataaata ttccatatcc aaaggctggg gctaagaatc cggttgttcg tgtttttatt     720
gttgacacca cctaccctca ccacgtgggc ccaatggaag tgccagttcc agaaatgata     780
gcctcaagtg actattattt cagctggctc acatgggtgt ccagtgaacg agtatgcttg     840
cagtggctaa aaagagtgca gaatgtctca gtcctgtcta tatgtgattt cagggaagac     900
tggcatgcat gggaatgtcc aaagaaccag gagcatgtag aagaaagcag aacaggatgg     960
gctggtggat tctttgtttc gacaccagct tttagccagg atgccacttc ttactacaaa    1020
atatttagcg acaaggatgg ttacaaacat attcactaca tcaaagacac tgtggaaaat    1080
gctattcaaa ttacaagtgg caagtgggag gccatatata tattccgcgt aacacaggat    1140
tcactgtttt attctagcaa tgaatttgaa ggttaccctg gaagaagaaa catctacaga    1200
attagcattg gaaactctcc tccgagcaag aagtgtgtta cttgccatct aaggaaagaa    1260
aggtgccaat attacacagc aagtttcagc tacaaagcca agtactatgc actcgtctgc    1320
tatggccctg gcctccccat ttccaccctc catgatggcc gcacagacca agaaatacaa    1380
gtattagaag aaaacaaaga actggaaaat tctctgagaa atatccagct gcctaaagtg    1440
gagattaaga agctcaaaga cgggggactg actttctggt acaagatgat tctgcctcct    1500
cagtttgaca gatcaaagaa gtaccctttg ctaattcaag tgtatggtgg tccttgtagc    1560
```

```
cagagtgtta agtctgtgtt tgctgttaat tggataactt atctcgcaag taaggagggg     1620

atagtcattg ccctggtaga tggtcggggc actgctttcc aaggtgacaa attcctgcat     1680

gccgtgtatc gaaaactggg tgtatatgaa gttgaggacc agctcacagc tgtcagaaaa     1740

ttcatagaaa tgggtttcat tgatgaagaa agaatagcca tatggggctg gtcctacgga     1800

ggttatgttt catccctggc ccttgcatct ggaactggtc ttttcaaatg tggcatagca     1860

gtggctccag tctccagctg ggaatattac gcatctatct actcagagag attcatgggc     1920

ctcccaacaa aggacgacaa tctcgaacac tataaaaatt caactgtgat ggcaagagca     1980

gaatatttca gaaatgtaga ctatcttctc atccacggaa cagcagatga taatgtgcac     2040

tttcagaact cagcacagat tgctaaagct ttggttaatg cacaagtgga tttccaggcg     2100

atgtggtact ctgaccagaa ccatggtata ttatctgggc gctcccagaa tcatttatat     2160

acccacatga cgcacttcct caagcaatgc ttttctttat cagacggcaa aaagaaaaag     2220

aaaaagggcc accaccatca ccatcac                                         2247
```

<210> 59
<211> 748
<212> PRT
<213> Artificial Sequence

<220>
<223> Cynomolgus FAP ectodomain+poly-lys-tag+his6-tag

<400> 59

```
Arg Pro Pro Arg Val His Asn Ser Glu Glu Asn Thr Met Arg Ala Leu
1               5                   10                  15

Thr Leu Lys Asp Ile Leu Asn Gly Thr Phe Ser Tyr Lys Thr Phe Phe
            20                  25                  30

Pro Asn Trp Ile Ser Gly Gln Glu Tyr Leu His Gln Ser Ala Asp Asn
        35                  40                  45

Asn Ile Val Leu Tyr Asn Ile Glu Thr Gly Gln Ser Tyr Thr Ile Leu
    50                  55                  60

Ser Asn Arg Thr Met Lys Ser Val Asn Ala Ser Asn Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Ser Asn Gly
            100                 105                 110
```

```
Glu Phe Val Arg Gly Asn Glu Leu Pro Arg Pro Ile Gln Tyr Leu Cys
        115                 120                 125

Trp Ser Pro Val Gly Ser Lys Leu Ala Tyr Val Tyr Gln Asn Asn Ile
    130                 135                 140

Tyr Leu Lys Gln Arg Pro Gly Asp Pro Pro Phe Gln Ile Thr Phe Asn
145                 150                 155                 160

Gly Arg Glu Asn Lys Ile Phe Asn Gly Ile Pro Asp Trp Val Tyr Glu
                165                 170                 175

Glu Glu Met Leu Ala Thr Lys Tyr Ala Leu Trp Trp Ser Pro Asn Gly
            180                 185                 190

Lys Phe Leu Ala Tyr Ala Glu Phe Asn Asp Thr Asp Ile Pro Val Ile
        195                 200                 205

Ala Tyr Ser Tyr Tyr Gly Asp Glu Gln Tyr Pro Arg Thr Ile Asn Ile
    210                 215                 220

Pro Tyr Pro Lys Ala Gly Ala Lys Asn Pro Phe Val Arg Ile Phe Ile
225                 230                 235                 240

Ile Asp Thr Thr Tyr Pro Ala Tyr Val Gly Pro Gln Glu Val Pro Val
                245                 250                 255

Pro Ala Met Ile Ala Ser Ser Asp Tyr Tyr Phe Ser Trp Leu Thr Trp
            260                 265                 270

Val Thr Asp Glu Arg Val Cys Leu Gln Trp Leu Lys Arg Val Gln Asn
        275                 280                 285

Val Ser Val Leu Ser Ile Cys Asp Phe Arg Glu Asp Trp Gln Thr Trp
    290                 295                 300

Asp Cys Pro Lys Thr Gln Glu His Ile Glu Glu Ser Arg Thr Gly Trp
305                 310                 315                 320

Ala Gly Gly Phe Phe Val Ser Thr Pro Val Phe Ser Tyr Asp Ala Ile
                325                 330                 335

Ser Tyr Tyr Lys Ile Phe Ser Asp Lys Asp Gly Tyr Lys His Ile His
            340                 345                 350

Tyr Ile Lys Asp Thr Val Glu Asn Ala Ile Gln Ile Thr Ser Gly Lys
        355                 360                 365
```

```
Trp Glu Ala Ile Asn Ile Phe Arg Val Thr Gln Asp Ser Leu Phe Tyr
    370                 375                 380

Ser Ser Asn Glu Phe Glu Asp Tyr Pro Gly Arg Arg Asn Ile Tyr Arg
385                 390                 395                 400

Ile Ser Ile Gly Ser Tyr Pro Pro Ser Lys Lys Cys Val Thr Cys His
                405                 410                 415

Leu Arg Lys Glu Arg Cys Gln Tyr Tyr Thr Ala Ser Phe Ser Asp Tyr
            420                 425                 430

Ala Lys Tyr Tyr Ala Leu Val Cys Tyr Gly Pro Gly Ile Pro Ile Ser
        435                 440                 445

Thr Leu His Asp Gly Arg Thr Asp Gln Glu Ile Lys Ile Leu Glu Glu
    450                 455                 460

Asn Lys Glu Leu Glu Asn Ala Leu Lys Asn Ile Gln Leu Pro Lys Glu
465                 470                 475                 480

Glu Ile Lys Lys Leu Glu Val Asp Glu Ile Thr Leu Trp Tyr Lys Met
                485                 490                 495

Ile Leu Pro Pro Gln Phe Asp Arg Ser Lys Lys Tyr Pro Leu Leu Ile
            500                 505                 510

Gln Val Tyr Gly Gly Pro Cys Ser Gln Ser Val Arg Ser Val Phe Ala
        515                 520                 525

Val Asn Trp Ile Ser Tyr Leu Ala Ser Lys Glu Gly Met Val Ile Ala
    530                 535                 540

Leu Val Asp Gly Arg Gly Thr Ala Phe Gln Gly Asp Lys Leu Leu Tyr
545                 550                 555                 560

Ala Val Tyr Arg Lys Leu Gly Val Tyr Glu Val Glu Asp Gln Ile Thr
                565                 570                 575

Ala Val Arg Lys Phe Ile Glu Met Gly Phe Ile Asp Glu Lys Arg Ile
            580                 585                 590

Ala Ile Trp Gly Trp Ser Tyr Gly Gly Tyr Val Ser Ser Leu Ala Leu
        595                 600                 605

Ala Ser Gly Thr Gly Leu Phe Lys Cys Gly Ile Ala Val Ala Pro Val
    610                 615                 620
```

Ser Ser Trp Glu Tyr Tyr Ala Ser Val Tyr Thr Glu Arg Phe Met Gly
625 630 635 640

Leu Pro Thr Lys Asp Asp Asn Leu Glu His Tyr Lys Asn Ser Thr Val
645 650 655

Met Ala Arg Ala Glu Tyr Phe Arg Asn Val Asp Tyr Leu Leu Ile His
660 665 670

Gly Thr Ala Asp Asp Asn Val His Phe Gln Asn Ser Ala Gln Ile Ala
675 680 685

Lys Ala Leu Val Asn Ala Gln Val Asp Phe Gln Ala Met Trp Tyr Ser
690 695 700

Asp Gln Asn His Gly Leu Ser Gly Leu Ser Thr Asn His Leu Tyr Thr
705 710 715 720

His Met Thr His Phe Leu Lys Gln Cys Phe Ser Leu Ser Asp Gly Lys
725 730 735

Lys Lys Lys Lys Lys Gly His His His His His His
740 745

<210> 60
<211> 2244
<212> DNA
<213> Artificial Sequence

<220>
<223> nucleotide sequence of cynomolgus FAP ectodomain+poly-lys-tag+his6-tag

<400> 60

cgccctccaa gagttcataa ctctgaagaa aatacaatga gagcactcac actgaaggat 60

attttaaatg ggacattttc ttataaaaca tttttccaa actggatttc aggacaagaa 120

tatcttcatc aatctgcaga taacaatata gtactttata atattgaaac aggacaatca 180

tataccattt tgagtaacag aaccatgaaa agtgtgaatg cttcaaatta tggcttatca 240

cctgatcggc aatttgtata tctagaaagt gattattcaa agctttggag atactcttac 300

acagcaacat attacatcta tgaccttagc aatggagaat ttgtaagagg aaatgagctt 360

cctcgtccaa ttcagtattt atgctggtcg cctgttggga gtaaattagc atatgtctat 420

caaaacaata tctatttgaa acaaagacca ggagatccac cttttcaaat aacatttaat 480

ggaagagaaa ataaaatatt taatggaatc ccagactggg tttatgaaga ggaaatgctt 540

gctacaaaat atgctctctg gtggtctcct aatggaaaat ttttggcata tgcggaattt 600

aatgatacag atataccagt tattgcctat tcctattatg gcgatgaaca atatcccaga 660

acaataaata ttccataccc aaaggccgga gctaagaatc cttttgttcg gatatttatt 720

atcgatacca cttaccctgc gtatgtaggt ccccaggaag tgcctgttcc agcaatgata 780

gcctcaagtg attattattt cagttggctc acgtgggtta ctgatgaacg agtatgtttg 840

cagtggctaa aaagagtcca gaatgtttcg gtcttgtcta tatgtgattt cagggaagac 900

tggcagacat gggattgtcc aaagacccag gagcatatag aagaaagcag aactggatgg 960

gctggtggat tctttgtttc aacaccagtt ttcagctatg atgccatttc atactacaaa 1020

atatttagtg acaaggatgg ctacaaacat attcactata tcaaagacac tgtggaaaat 1080

gctattcaaa ttacaagtgg caagtgggag gccataaata tattcagagt aacacaggat 1140

tcactgtttt attctagcaa tgaatttgaa gattaccctg gaagaagaaa catctacaga 1200

attagcattg gaagctatcc tccaagcaag aagtgtgtta cttgccatct aaggaaagaa 1260

aggtgccaat attacacagc aagtttcagc gactacgcca agtactatgc acttgtctgc 1320

tatggcccag gcatccccat ttccaccctt catgacggac gcactgatca agaaattaaa 1380

atcctggaag aaaacaagga attggaaaat gctttgaaaa atatccagct gcctaaagag 1440

gaaattaaga aacttgaagt agatgaaatt actttatggt acaagatgat tcttcctcct 1500

caatttgaca gatcaaagaa gtatcccttg ctaattcaag tgtatggtgg tccctgcagt 1560

cagagtgtaa ggtctgtatt tgctgttaat tggatatctt atcttgcaag taaggaaggg 1620

atggtcattg ccttggtgga tggtcgggga acagctttcc aaggtgacaa actcctgtat 1680

gcagtgtatc gaaagctggg tgtttatgaa gttgaagacc agattacagc tgtcagaaaa 1740

ttcatagaaa tgggtttcat tgatgaaaaa agaatagcca tatggggctg gtcctatgga 1800

ggatatgttt catcactggc ccttgcatct ggaactggtc ttttcaaatg tgggatagca 1860

gtggctccag tctccagctg ggaatattac gcgtctgtct acacagagag attcatgggt 1920

ctcccaacaa aggatgataa tcttgagcac tataagaatt caactgtgat ggcaagagca 1980

gaatatttca gaaatgtaga ctatcttctc atccacggaa cagcagatga taatgtgcac 2040

tttcaaaact cagcacagat tgctaaagct ctggttaatg cacaagtgga tttccaggca 2100

atgtggtact ctgaccagaa ccacggctta tccggcctgt ccacgaacca cttatacacc 2160

cacatgaccc acttcctaaa gcagtgtttc tctttgtcag acggcaaaaa gaaaaagaaa 2220

aagggccacc accatcacca tcac 2244

<210> 61
<211> 702
<212> PRT
<213> Homo sapiens

<400> 61

```
Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
```

245 250 255

Leu Ser Cys His Ala Ala Ser Asn Pro Pro Ala Gln Tyr Ser Trp Phe
260 265 270

Val Asn Gly Thr Phe Gln Gln Ser Thr Gln Glu Leu Phe Ile Pro Asn
275 280 285

Ile Thr Val Asn Asn Ser Gly Ser Tyr Thr Cys Gln Ala His Asn Ser
290 295 300

Asp Thr Gly Leu Asn Arg Thr Thr Val Thr Thr Ile Thr Val Tyr Ala
305 310 315 320

Glu Pro Pro Lys Pro Phe Ile Thr Ser Asn Asn Ser Asn Pro Val Glu
325 330 335

Asp Glu Asp Ala Val Ala Leu Thr Cys Glu Pro Glu Ile Gln Asn Thr
340 345 350

Thr Tyr Leu Trp Trp Val Asn Asn Gln Ser Leu Pro Val Ser Pro Arg
355 360 365

Leu Gln Leu Ser Asn Asp Asn Arg Thr Leu Thr Leu Leu Ser Val Thr
370 375 380

Arg Asn Asp Val Gly Pro Tyr Glu Cys Gly Ile Gln Asn Lys Leu Ser
385 390 395 400

Val Asp His Ser Asp Pro Val Ile Leu Asn Val Leu Tyr Gly Pro Asp
405 410 415

Asp Pro Thr Ile Ser Pro Ser Tyr Thr Tyr Tyr Arg Pro Gly Val Asn
420 425 430

Leu Ser Leu Ser Cys His Ala Ala Ser Asn Pro Pro Ala Gln Tyr Ser
435 440 445

Trp Leu Ile Asp Gly Asn Ile Gln Gln His Thr Gln Glu Leu Phe Ile
450 455 460

Ser Asn Ile Thr Glu Lys Asn Ser Gly Leu Tyr Thr Cys Gln Ala Asn
465 470 475 480

Asn Ser Ala Ser Gly His Ser Arg Thr Thr Val Lys Thr Ile Thr Val
485 490 495

Ser Ala Glu Leu Pro Lys Pro Ser Ile Ser Ser Asn Asn Ser Lys Pro
500 505 510

Val Glu Asp Lys Asp Ala Val Ala Phe Thr Cys Glu Pro Glu Ala Gln
515 520 525

Asn Thr Thr Tyr Leu Trp Trp Val Asn Gly Gln Ser Leu Pro Val Ser
530 535 540

Pro Arg Leu Gln Leu Ser Asn Gly Asn Arg Thr Leu Thr Leu Phe Asn
545 550 555 560

Val Thr Arg Asn Asp Ala Arg Ala Tyr Val Cys Gly Ile Gln Asn Ser
565 570 575

Val Ser Ala Asn Arg Ser Asp Pro Val Thr Leu Asp Val Leu Tyr Gly
580 585 590

Pro Asp Thr Pro Ile Ile Ser Pro Pro Asp Ser Ser Tyr Leu Ser Gly
595 600 605

Ala Asn Leu Asn Leu Ser Cys His Ser Ala Ser Asn Pro Ser Pro Gln
610 615 620

Tyr Ser Trp Arg Ile Asn Gly Ile Pro Gln Gln His Thr Gln Val Leu
625 630 635 640

Phe Ile Ala Lys Ile Thr Pro Asn Asn Asn Gly Thr Tyr Ala Cys Phe
645 650 655

Val Ser Asn Leu Ala Thr Gly Arg Asn Asn Ser Ile Val Lys Ser Ile
660 665 670

Thr Val Ser Ala Ser Gly Thr Ser Pro Gly Leu Ser Ala Gly Ala Thr
675 680 685

Val Gly Ile Met Ile Gly Val Leu Val Gly Val Ala Leu Ile
690 695 700

<210> 62
<211> 2322
<212> PRT
<213> Homo sapiens

<400> 62

Met Gln Ser Gly Pro Arg Pro Pro Leu Pro Ala Pro Gly Leu Ala Leu
1 5 10 15

```
Ala Leu Thr Leu Thr Met Leu Ala Arg Leu Ala Ser Ala Ala Ser Phe
            20                  25                  30

Phe Gly Glu Asn His Leu Glu Val Pro Val Ala Thr Ala Leu Thr Asp
        35                  40                  45

Ile Asp Leu Gln Leu Gln Phe Ser Thr Ser Gln Pro Glu Ala Leu Leu
    50                  55                  60

Leu Leu Ala Ala Gly Pro Ala Asp His Leu Leu Leu Gln Leu Tyr Ser
65                  70                  75                  80

Gly Arg Leu Gln Val Arg Leu Val Leu Gly Gln Glu Glu Leu Arg Leu
                85                  90                  95

Gln Thr Pro Ala Glu Thr Leu Leu Ser Asp Ser Ile Pro His Thr Val
            100                 105                 110

Val Leu Thr Val Val Glu Gly Trp Ala Thr Leu Ser Val Asp Gly Phe
        115                 120                 125

Leu Asn Ala Ser Ser Ala Val Pro Gly Ala Pro Leu Glu Val Pro Tyr
    130                 135                 140

Gly Leu Phe Val Gly Gly Thr Gly Thr Leu Gly Leu Pro Tyr Leu Arg
145                 150                 155                 160

Gly Thr Ser Arg Pro Leu Arg Gly Cys Leu His Ala Ala Thr Leu Asn
                165                 170                 175

Gly Arg Ser Leu Leu Arg Pro Leu Thr Pro Asp Val His Glu Gly Cys
            180                 185                 190

Ala Glu Glu Phe Ser Ala Ser Asp Asp Val Ala Leu Gly Phe Ser Gly
        195                 200                 205

Pro His Ser Leu Ala Ala Phe Pro Ala Trp Gly Thr Gln Asp Glu Gly
    210                 215                 220

Thr Leu Glu Phe Thr Leu Thr Thr Gln Ser Arg Gln Ala Pro Leu Ala
225                 230                 235                 240

Phe Gln Ala Gly Gly Arg Arg Gly Asp Phe Ile Tyr Val Asp Ile Phe
                245                 250                 255

Glu Gly His Leu Arg Ala Val Val Glu Lys Gly Gln Gly Thr Val Leu
            260                 265                 270
```

Leu His Asn Ser Val Pro Val Ala Asp Gly Gln Pro His Glu Val Ser
275 280 285

Val His Ile Asn Ala His Arg Leu Glu Ile Ser Val Asp Gln Tyr Pro
290 295 300

Thr His Thr Ser Asn Arg Gly Val Leu Ser Tyr Leu Glu Pro Arg Gly
305 310 315 320

Ser Leu Leu Leu Gly Gly Leu Asp Ala Glu Ala Ser Arg His Leu Gln
325 330 335

Glu His Arg Leu Gly Leu Thr Pro Glu Ala Thr Asn Ala Ser Leu Leu
340 345 350

Gly Cys Met Glu Asp Leu Ser Val Asn Gly Gln Arg Arg Gly Leu Arg
355 360 365

Glu Ala Leu Leu Thr Arg Asn Met Ala Ala Gly Cys Arg Leu Glu Glu
370 375 380

Glu Glu Tyr Glu Asp Asp Ala Tyr Gly His Tyr Glu Ala Phe Ser Thr
385 390 395 400

Leu Ala Pro Glu Ala Trp Pro Ala Met Glu Leu Pro Glu Pro Cys Val
405 410 415

Pro Glu Pro Gly Leu Pro Pro Val Phe Ala Asn Phe Thr Gln Leu Leu
420 425 430

Thr Ile Ser Pro Leu Val Val Ala Glu Gly Gly Thr Ala Trp Leu Glu
435 440 445

Trp Arg His Val Gln Pro Thr Leu Asp Leu Met Glu Ala Glu Leu Arg
450 455 460

Lys Ser Gln Val Leu Phe Ser Val Thr Arg Gly Ala Arg His Gly Glu
465 470 475 480

Leu Glu Leu Asp Ile Pro Gly Ala Gln Ala Arg Lys Met Phe Thr Leu
485 490 495

Leu Asp Val Val Asn Arg Lys Ala Arg Phe Ile His Asp Gly Ser Glu
500 505 510

Asp Thr Ser Asp Gln Leu Val Leu Glu Val Ser Val Thr Ala Arg Val
515 520 525

Pro Met Pro Ser Cys Leu Arg Arg Gly Gln Thr Tyr Leu Leu Pro Ile
    530                 535                 540

Gln Val Asn Pro Val Asn Asp Pro Pro His Ile Ile Phe Pro His Gly
545                 550                 555                 560

Ser Leu Met Val Ile Leu Glu His Thr Gln Lys Pro Leu Gly Pro Glu
                565                 570                 575

Val Phe Gln Ala Tyr Asp Pro Asp Ser Ala Cys Glu Gly Leu Thr Phe
            580                 585                 590

Gln Val Leu Gly Thr Ser Ser Gly Leu Pro Val Glu Arg Arg Asp Gln
        595                 600                 605

Pro Gly Glu Pro Ala Thr Glu Phe Ser Cys Arg Glu Leu Glu Ala Gly
    610                 615                 620

Ser Leu Val Tyr Val His Arg Gly Gly Pro Ala Gln Asp Leu Thr Phe
625                 630                 635                 640

Arg Val Ser Asp Gly Leu Gln Ala Ser Pro Pro Ala Thr Leu Lys Val
                645                 650                 655

Val Ala Ile Arg Pro Ala Ile Gln Ile His Arg Ser Thr Gly Leu Arg
            660                 665                 670

Leu Ala Gln Gly Ser Ala Met Pro Ile Leu Pro Ala Asn Leu Ser Val
        675                 680                 685

Glu Thr Asn Ala Val Gly Gln Asp Val Ser Val Leu Phe Arg Val Thr
    690                 695                 700

Gly Ala Leu Gln Phe Gly Glu Leu Gln Lys Gln Gly Ala Gly Gly Val
705                 710                 715                 720

Glu Gly Ala Glu Trp Trp Ala Thr Gln Ala Phe His Gln Arg Asp Val
                725                 730                 735

Glu Gln Gly Arg Val Arg Tyr Leu Ser Thr Asp Pro Gln His His Ala
            740                 745                 750

Tyr Asp Thr Val Glu Asn Leu Ala Leu Glu Val Gln Val Gly Gln Glu
        755                 760                 765

Ile Leu Ser Asn Leu Ser Phe Pro Val Thr Ile Gln Arg Ala Thr Val

```
    770                 775                 780

Trp Met Leu Arg Leu Glu Pro Leu His Thr Gln Asn Thr Gln Gln Glu
785                 790                 795                 800

Thr Leu Thr Thr Ala His Leu Glu Ala Thr Leu Glu Glu Ala Gly Pro
                805                 810                 815

Ser Pro Pro Thr Phe His Tyr Glu Val Val Gln Ala Pro Arg Lys Gly
            820                 825                 830

Asn Leu Gln Leu Gln Gly Thr Arg Leu Ser Asp Gly Gln Gly Phe Thr
        835                 840                 845

Gln Asp Asp Ile Gln Ala Gly Arg Val Thr Tyr Gly Ala Thr Ala Arg
    850                 855                 860

Ala Ser Glu Ala Val Glu Asp Thr Phe Arg Phe Arg Val Thr Ala Pro
865                 870                 875                 880

Pro Tyr Phe Ser Pro Leu Tyr Thr Phe Pro Ile His Ile Gly Gly Asp
                885                 890                 895

Pro Asp Ala Pro Val Leu Thr Asn Val Leu Leu Val Val Pro Glu Gly
            900                 905                 910

Gly Glu Gly Val Leu Ser Ala Asp His Leu Phe Val Lys Ser Leu Asn
        915                 920                 925

Ser Ala Ser Tyr Leu Tyr Glu Val Met Glu Arg Pro Arg His Gly Arg
    930                 935                 940

Leu Ala Trp Arg Gly Thr Gln Asp Lys Thr Thr Met Val Thr Ser Phe
945                 950                 955                 960

Thr Asn Glu Asp Leu Leu Arg Gly Arg Leu Val Tyr Gln His Asp Asp
                965                 970                 975

Ser Glu Thr Thr Glu Asp Asp Ile Pro Phe Val Ala Thr Arg Gln Gly
            980                 985                 990

Glu Ser Ser Gly Asp Met Ala Trp  Glu Glu Val Arg Gly  Val Phe Arg
        995                 1000                 1005

Val Ala  Ile Gln Pro Val Asn  Asp His Ala Pro Val  Gln Thr Ile
    1010                 1015                 1020
```

```
Ser Arg  Ile Phe His Val Ala  Arg Gly Gly Arg Arg  Leu Leu Thr
    1025                 1030                 1035

Thr Asp  Asp Val Ala Phe Ser  Asp Ala Asp Ser Gly  Phe Ala Asp
    1040                 1045                 1050

Ala Gln  Leu Val Leu Thr Arg  Lys Asp Leu Leu Phe  Gly Ser Ile
    1055                 1060                 1065

Val Ala  Val Asp Glu Pro Thr  Arg Pro Ile Tyr Arg  Phe Thr Gln
    1070                 1075                 1080

Glu Asp  Leu Arg Lys Arg Arg  Val Leu Phe Val His  Ser Gly Ala
    1085                 1090                 1095

Asp Arg  Gly Trp Ile Gln Leu  Gln Val Ser Asp Gly  Gln His Gln
    1100                 1105                 1110

Ala Thr  Ala Leu Leu Glu Val  Gln Ala Ser Glu Pro  Tyr Leu Arg
    1115                 1120                 1125

Val Ala  Asn Gly Ser Ser Leu  Val Val Pro Gln Gly  Gly Gln Gly
    1130                 1135                 1140

Thr Ile  Asp Thr Ala Val Leu  His Leu Asp Thr Asn  Leu Asp Ile
    1145                 1150                 1155

Arg Ser  Gly Asp Glu Val His  Tyr His Val Thr Ala  Gly Pro Arg
    1160                 1165                 1170

Trp Gly  Gln Leu Val Arg Ala  Gly Gln Pro Ala Thr  Ala Phe Ser
    1175                 1180                 1185

Gln Gln  Asp Leu Leu Asp Gly  Ala Val Leu Tyr Ser  His Asn Gly
    1190                 1195                 1200

Ser Leu  Ser Pro Arg Asp Thr  Met Ala Phe Ser Val  Glu Ala Gly
    1205                 1210                 1215

Pro Val  His Thr Asp Ala Thr  Leu Gln Val Thr Ile  Ala Leu Glu
    1220                 1225                 1230

Gly Pro  Leu Ala Pro Leu Lys  Leu Val Arg His Lys  Lys Ile Tyr
    1235                 1240                 1245

Val Phe  Gln Gly Glu Ala Ala  Glu Ile Arg Arg Asp  Gln Leu Glu
    1250                 1255                 1260
```

Ala Ala Gln Glu Ala Val Pro Pro Ala Asp Ile Val Phe Ser Val
1265 1270 1275

Lys Ser Pro Pro Ser Ala Gly Tyr Leu Val Met Val Ser Arg Gly
1280 1285 1290

Ala Leu Ala Asp Glu Pro Pro Ser Leu Asp Pro Val Gln Ser Phe
1295 1300 1305

Ser Gln Glu Ala Val Asp Thr Gly Arg Val Leu Tyr Leu His Ser
1310 1315 1320

Arg Pro Glu Ala Trp Ser Asp Ala Phe Ser Leu Asp Val Ala Ser
1325 1330 1335

Gly Leu Gly Ala Pro Leu Glu Gly Val Leu Val Glu Leu Glu Val
1340 1345 1350

Leu Pro Ala Ala Ile Pro Leu Glu Ala Gln Asn Phe Ser Val Pro
1355 1360 1365

Glu Gly Gly Ser Leu Thr Leu Ala Pro Pro Leu Leu Arg Val Ser
1370 1375 1380

Gly Pro Tyr Phe Pro Thr Leu Leu Gly Leu Ser Leu Gln Val Leu
1385 1390 1395

Glu Pro Pro Gln His Gly Ala Leu Gln Lys Glu Asp Gly Pro Gln
1400 1405 1410

Ala Arg Thr Leu Ser Ala Phe Ser Trp Arg Met Val Glu Glu Gln
1415 1420 1425

Leu Ile Arg Tyr Val His Asp Gly Ser Glu Thr Leu Thr Asp Ser
1430 1435 1440

Phe Val Leu Met Ala Asn Ala Ser Glu Met Asp Arg Gln Ser His
1445 1450 1455

Pro Val Ala Phe Thr Val Thr Val Leu Pro Val Asn Asp Gln Pro
1460 1465 1470

Pro Ile Leu Thr Thr Asn Thr Gly Leu Gln Met Trp Glu Gly Ala
1475 1480 1485

Thr Ala Pro Ile Pro Ala Glu Ala Leu Arg Ser Thr Asp Gly Asp
1490 1495 1500

Ser Gly Ser Glu Asp Leu Val Tyr Thr Ile Glu Gln Pro Ser Asn
1505 1510 1515

Gly Arg Val Val Leu Arg Gly Ala Pro Gly Thr Glu Val Arg Ser
1520 1525 1530

Phe Thr Gln Ala Gln Leu Asp Gly Gly Leu Val Leu Phe Ser His
1535 1540 1545

Arg Gly Thr Leu Asp Gly Gly Phe Arg Phe Arg Leu Ser Asp Gly
1550 1555 1560

Glu His Thr Ser Pro Gly His Phe Phe Arg Val Thr Ala Gln Lys
1565 1570 1575

Gln Val Leu Leu Ser Leu Lys Gly Ser Gln Thr Leu Thr Val Cys
1580 1585 1590

Pro Gly Ser Val Gln Pro Leu Ser Ser Gln Thr Leu Arg Ala Ser
1595 1600 1605

Ser Ser Ala Gly Thr Asp Pro Gln Leu Leu Leu Tyr Arg Val Val
1610 1615 1620

Arg Gly Pro Gln Leu Gly Arg Leu Phe His Ala Gln Gln Asp Ser
1625 1630 1635

Thr Gly Glu Ala Leu Val Asn Phe Thr Gln Ala Glu Val Tyr Ala
1640 1645 1650

Gly Asn Ile Leu Tyr Glu His Glu Met Pro Pro Glu Pro Phe Trp
1655 1660 1665

Glu Ala His Asp Thr Leu Glu Leu Gln Leu Ser Ser Pro Pro Ala
1670 1675 1680

Arg Asp Val Ala Ala Thr Leu Ala Val Ala Val Ser Phe Glu Ala
1685 1690 1695

Ala Cys Pro Gln Arg Pro Ser His Leu Trp Lys Asn Lys Gly Leu
1700 1705 1710

Trp Val Pro Glu Gly Gln Arg Ala Arg Ile Thr Val Ala Ala Leu
1715 1720 1725

Asp Ala Ser Asn Leu Leu Ala Ser Val Pro Ser Pro Gln Arg Ser

1730 1735 1740

Glu His Asp Val Leu Phe Gln Val Thr Gln Phe Pro Ser Arg Gly
1745 1750 1755

Gln Leu Leu Val Ser Glu Glu Pro Leu His Ala Gly Gln Pro His
1760 1765 1770

Phe Leu Gln Ser Gln Leu Ala Ala Gly Gln Leu Val Tyr Ala His
1775 1780 1785

Gly Gly Gly Gly Thr Gln Gln Asp Gly Phe His Phe Arg Ala His
1790 1795 1800

Leu Gln Gly Pro Ala Gly Ala Ser Val Ala Gly Pro Gln Thr Ser
1805 1810 1815

Glu Ala Phe Ala Ile Thr Val Arg Asp Val Asn Glu Arg Pro Pro
1820 1825 1830

Gln Pro Gln Ala Ser Val Pro Leu Arg Leu Thr Arg Gly Ser Arg
1835 1840 1845

Ala Pro Ile Ser Arg Ala Gln Leu Ser Val Val Asp Pro Asp Ser
1850 1855 1860

Ala Pro Gly Glu Ile Glu Tyr Glu Val Gln Arg Ala Pro His Asn
1865 1870 1875

Gly Phe Leu Ser Leu Val Gly Gly Gly Leu Gly Pro Val Thr Arg
1880 1885 1890

Phe Thr Gln Ala Asp Val Asp Ser Gly Arg Leu Ala Phe Val Ala
1895 1900 1905

Asn Gly Ser Ser Val Ala Gly Ile Phe Gln Leu Ser Met Ser Asp
1910 1915 1920

Gly Ala Ser Pro Pro Leu Pro Met Ser Leu Ala Val Asp Ile Leu
1925 1930 1935

Pro Ser Ala Ile Glu Val Gln Leu Arg Ala Pro Leu Glu Val Pro
1940 1945 1950

Gln Ala Leu Gly Arg Ser Ser Leu Ser Gln Gln Gln Leu Arg Val
1955 1960 1965

Val Ser Asp Arg Glu Glu Pro Glu Ala Ala Tyr Arg Leu Ile Gln
1970 1975 1980

Gly Pro Gln Tyr Gly His Leu Leu Val Gly Gly Arg Pro Thr Ser
1985 1990 1995

Ala Phe Ser Gln Phe Gln Ile Asp Gln Gly Glu Val Val Phe Ala
2000 2005 2010

Phe Thr Asn Phe Ser Ser Ser His Asp His Phe Arg Val Leu Ala
2015 2020 2025

Leu Ala Arg Gly Val Asn Ala Ser Ala Val Val Asn Val Thr Val
2030 2035 2040

Arg Ala Leu Leu His Val Trp Ala Gly Gly Pro Trp Pro Gln Gly
2045 2050 2055

Ala Thr Leu Arg Leu Asp Pro Thr Val Leu Asp Ala Gly Glu Leu
2060 2065 2070

Ala Asn Arg Thr Gly Ser Val Pro Arg Phe Arg Leu Leu Glu Gly
2075 2080 2085

Pro Arg His Gly Arg Val Val Arg Val Pro Arg Ala Arg Thr Glu
2090 2095 2100

Pro Gly Gly Ser Gln Leu Val Glu Gln Phe Thr Gln Gln Asp Leu
2105 2110 2115

Glu Asp Gly Arg Leu Gly Leu Glu Val Gly Arg Pro Glu Gly Arg
2120 2125 2130

Ala Pro Gly Pro Ala Gly Asp Ser Leu Thr Leu Glu Leu Trp Ala
2135 2140 2145

Gln Gly Val Pro Pro Ala Val Ala Ser Leu Asp Phe Ala Thr Glu
2150 2155 2160

Pro Tyr Asn Ala Ala Arg Pro Tyr Ser Val Ala Leu Leu Ser Val
2165 2170 2175

Pro Glu Ala Ala Arg Thr Glu Ala Gly Lys Pro Glu Ser Ser Thr
2180 2185 2190

Pro Thr Gly Glu Pro Gly Pro Met Ala Ser Ser Pro Glu Pro Ala
2195 2200 2205

Val Ala Lys Gly Gly Phe Leu Ser Phe Leu Glu Ala Asn Met Phe
2210 2215 2220

Ser Val Ile Ile Pro Met Cys Leu Val Leu Leu Leu Leu Ala Leu
2225 2230 2235

Ile Leu Pro Leu Leu Phe Tyr Leu Arg Lys Arg Asn Lys Thr Gly
2240 2245 2250

Lys His Asp Val Gln Val Leu Thr Ala Lys Pro Arg Asn Gly Leu
2255 2260 2265

Ala Gly Asp Thr Glu Thr Phe Arg Lys Val Glu Pro Gly Gln Ala
2270 2275 2280

Ile Pro Leu Thr Ala Val Pro Gly Gln Gly Pro Pro Pro Gly Gly
2285 2290 2295

Gln Pro Asp Pro Glu Leu Leu Gln Phe Cys Arg Thr Pro Asn Pro
2300 2305 2310

Ala Leu Lys Asn Gly Gln Tyr Trp Val
2315 2320

<210> 63
<211> 1209
<212> PRT
<213> Homo sapiens

<400> 63

Arg Pro Ser Gly Thr Ala Gly Ala Ala Leu Leu Ala Leu Leu Ala Ala
1 5 10 15

Leu Cys Pro Ala Ser Arg Ala Leu Glu Glu Lys Lys Val Cys Gln Gly
20 25 30

Thr Ser Asn Lys Leu Thr Gln Leu Gly Thr Phe Glu Asp His Phe Leu
35 40 45

Ser Leu Gln Arg Met Phe Asn Asn Cys Glu Val Val Leu Gly Asn Leu
50 55 60

Glu Ile Thr Tyr Val Gln Arg Asn Tyr Asp Leu Ser Phe Leu Lys Thr
65 70 75 80

Ile Gln Glu Val Ala Gly Tyr Val Leu Ile Ala Leu Asn Thr Val Glu
85 90 95

```
Arg Ile Pro Leu Glu Asn Leu Gln Ile Ile Arg Gly Asn Met Tyr Tyr
            100                 105                 110

Glu Asn Ser Tyr Ala Leu Ala Val Leu Ser Asn Tyr Asp Ala Asn Lys
        115                 120                 125

Thr Gly Leu Lys Glu Leu Pro Met Arg Asn Leu Gln Glu Ile Leu His
    130                 135                 140

Gly Ala Val Arg Phe Ser Asn Asn Pro Ala Leu Cys Asn Val Glu Ser
145                 150                 155                 160

Ile Gln Trp Arg Asp Ile Val Ser Ser Asp Phe Leu Ser Asn Met Ser
                165                 170                 175

Met Asp Phe Gln Asn His Leu Gly Ser Cys Gln Lys Cys Asp Pro Ser
            180                 185                 190

Cys Pro Asn Gly Ser Cys Trp Gly Ala Gly Glu Glu Asn Cys Gln Lys
        195                 200                 205

Leu Thr Lys Ile Ile Cys Ala Gln Gln Cys Ser Gly Arg Cys Arg Gly
    210                 215                 220

Lys Ser Pro Ser Asp Cys Cys His Asn Gln Cys Ala Ala Gly Cys Thr
225                 230                 235                 240

Gly Pro Arg Glu Ser Asp Cys Leu Val Cys Arg Lys Phe Arg Asp Glu
                245                 250                 255

Ala Thr Cys Lys Asp Thr Cys Pro Pro Leu Met Leu Tyr Asn Pro Thr
            260                 265                 270

Thr Tyr Gln Met Asp Val Asn Pro Glu Gly Lys Tyr Ser Phe Gly Ala
        275                 280                 285

Thr Cys Val Lys Lys Cys Pro Arg Asn Tyr Val Val Thr Asp His Gly
    290                 295                 300

Ser Cys Val Arg Ala Cys Gly Ala Asp Ser Tyr Glu Met Glu Glu Asp
305                 310                 315                 320

Gly Val Arg Lys Cys Lys Lys Cys Glu Gly Pro Cys Arg Lys Val Cys
                325                 330                 335

Asn Gly Ile Gly Ile Gly Glu Phe Lys Asp Ser Leu Ser Ile Asn Ala
            340                 345                 350
```

Thr Asn Ile Lys His Phe Lys Asn Cys Thr Ser Ile Ser Gly Asp Leu
355 360 365

His Ile Leu Pro Val Ala Phe Arg Gly Asp Ser Phe Thr His Thr Pro
370 375 380

Pro Leu Asp Pro Gln Glu Leu Asp Ile Leu Lys Thr Val Lys Glu Ile
385 390 395 400

Thr Gly Phe Leu Leu Ile Gln Ala Trp Pro Glu Asn Arg Thr Asp Leu
405 410 415

His Ala Phe Glu Asn Leu Glu Ile Ile Arg Gly Arg Thr Lys Gln His
420 425 430

Gly Gln Phe Ser Leu Ala Val Val Ser Leu Asn Ile Thr Ser Leu Gly
435 440 445

Leu Arg Ser Leu Lys Glu Ile Ser Asp Gly Asp Val Ile Ile Ser Gly
450 455 460

Asn Lys Asn Leu Cys Tyr Ala Asn Thr Ile Asn Trp Lys Lys Leu Phe
465 470 475 480

Gly Thr Ser Gly Gln Lys Thr Lys Ile Ile Ser Asn Arg Gly Glu Asn
485 490 495

Ser Cys Lys Ala Thr Gly Gln Val Cys His Ala Leu Cys Ser Pro Glu
500 505 510

Gly Cys Trp Gly Pro Glu Pro Arg Asp Cys Val Ser Cys Arg Asn Val
515 520 525

Ser Arg Gly Arg Glu Cys Val Asp Lys Cys Asn Leu Leu Glu Gly Glu
530 535 540

Pro Arg Glu Phe Val Glu Asn Ser Glu Cys Ile Gln Cys His Pro Glu
545 550 555 560

Cys Leu Pro Gln Ala Met Asn Ile Thr Cys Thr Gly Arg Gly Pro Asp
565 570 575

Asn Cys Ile Gln Cys Ala His Tyr Ile Asp Gly Pro His Cys Val Lys
580 585 590

Thr Cys Pro Ala Gly Val Met Gly Glu Asn Asn Thr Leu Val Trp Lys

595 600 605

Tyr Ala Asp Ala Gly His Val Cys His Leu Cys His Pro Asn Cys Thr
610 615 620

Tyr Gly Cys Thr Gly Pro Gly Leu Glu Gly Cys Pro Thr Asn Gly Pro
625 630 635 640

Lys Ile Pro Ser Ile Ala Thr Gly Met Val Gly Ala Leu Leu Leu Leu
645 650 655

Leu Val Val Ala Leu Gly Ile Gly Leu Phe Met Arg Arg Arg His Ile
660 665 670

Val Arg Lys Arg Thr Leu Arg Arg Leu Leu Gln Glu Arg Glu Leu Val
675 680 685

Glu Pro Leu Thr Pro Ser Gly Glu Ala Pro Asn Gln Ala Leu Leu Arg
690 695 700

Ile Leu Lys Glu Thr Glu Phe Lys Lys Ile Lys Val Leu Gly Ser Gly
705 710 715 720

Ala Phe Gly Thr Val Tyr Lys Gly Leu Trp Ile Pro Glu Gly Glu Lys
725 730 735

Val Lys Ile Pro Val Ala Ile Lys Glu Leu Arg Glu Ala Thr Ser Pro
740 745 750

Lys Ala Asn Lys Glu Ile Leu Asp Glu Ala Tyr Val Met Ala Ser Val
755 760 765

Asp Asn Pro His Val Cys Arg Leu Leu Gly Ile Cys Leu Thr Ser Thr
770 775 780

Val Gln Leu Ile Thr Gln Leu Met Pro Phe Gly Cys Leu Leu Asp Tyr
785 790 795 800

Val Arg Glu His Lys Asp Asn Ile Gly Ser Gln Tyr Leu Leu Asn Trp
805 810 815

Cys Val Gln Ile Ala Lys Gly Met Asn Tyr Leu Glu Asp Arg Arg Leu
820 825 830

Val His Arg Asp Leu Ala Ala Arg Asn Val Leu Val Lys Thr Pro Gln
835 840 845

His Val Lys Ile Thr Asp Phe Gly Leu Ala Lys Leu Leu Gly Ala Glu
850 855 860

Glu Lys Glu Tyr His Ala Glu Gly Gly Lys Val Pro Ile Lys Trp Met
865 870 875 880

Ala Leu Glu Ser Ile Leu His Arg Ile Tyr Thr His Gln Ser Asp Val
885 890 895

Trp Ser Tyr Gly Val Thr Val Trp Glu Leu Met Thr Phe Gly Ser Lys
900 905 910

Pro Tyr Asp Gly Ile Pro Ala Ser Glu Ile Ser Ser Ile Leu Glu Lys
915 920 925

Gly Glu Arg Leu Pro Gln Pro Pro Ile Cys Thr Ile Asp Val Tyr Met
930 935 940

Ile Met Val Lys Cys Trp Met Ile Asp Ala Asp Ser Arg Pro Lys Phe
945 950 955 960

Arg Glu Leu Ile Ile Glu Phe Ser Lys Met Ala Arg Asp Pro Gln Arg
965 970 975

Tyr Leu Val Ile Gln Gly Asp Glu Arg Met His Leu Pro Ser Pro Thr
980 985 990

Asp Ser Asn Phe Tyr Arg Ala Leu Met Asp Glu Glu Asp Met Asp Asp
995 1000 1005

Val Val Asp Ala Asp Glu Tyr Leu Ile Pro Gln Gln Gly Phe Phe
1010 1015 1020

Ser Ser Pro Ser Thr Ser Arg Thr Pro Leu Leu Ser Ser Leu Ser
1025 1030 1035

Ala Thr Ser Asn Asn Ser Thr Val Ala Cys Ile Asp Arg Asn Gly
1040 1045 1050

Leu Gln Ser Cys Pro Ile Lys Glu Asp Ser Phe Leu Gln Arg Tyr
1055 1060 1065

Ser Ser Asp Pro Thr Gly Ala Leu Thr Glu Asp Ser Ile Asp Asp
1070 1075 1080

Thr Phe Leu Pro Val Pro Glu Tyr Ile Asn Gln Ser Val Pro Lys
1085 1090 1095

```
Arg Pro Ala Gly Ser Val Gln Asn Pro Val Tyr His Asn Gln Pro
    1100                1105                1110

Leu Asn Pro Ala Pro Ser Arg Asp Pro His Tyr Gln Asp Pro His
    1115                1120                1125

Ser Thr Ala Val Gly Asn Pro Glu Tyr Leu Asn Thr Val Gln Pro
    1130                1135                1140

Thr Cys Val Asn Ser Thr Phe Asp Ser Pro Ala His Trp Ala Gln
    1145                1150                1155

Lys Gly Ser His Gln Ile Ser Leu Asp Asn Pro Asp Tyr Gln Gln
    1160                1165                1170

Asp Phe Phe Pro Lys Glu Ala Lys Pro Asn Gly Ile Phe Lys Gly
    1175                1180                1185

Ser Thr Ala Glu Asn Ala Glu Tyr Leu Arg Val Ala Pro Gln Ser
    1190                1195                1200

Ser Glu Phe Ile Gly Ala
    1205
```

<210> 64
<211> 556
<212> PRT
<213> Homo sapiens

<400> 64

```
Met Pro Pro Pro Arg Leu Leu Phe Phe Leu Leu Phe Leu Thr Pro Met
1               5                   10                  15

Glu Val Arg Pro Glu Glu Pro Leu Val Val Lys Val Glu Glu Gly Asp
            20                  25                  30

Asn Ala Val Leu Gln Cys Leu Lys Gly Thr Ser Asp Gly Pro Thr Gln
        35                  40                  45

Gln Leu Thr Trp Ser Arg Glu Ser Pro Leu Lys Pro Phe Leu Lys Leu
    50                  55                  60

Ser Leu Gly Leu Pro Gly Leu Gly Ile His Met Arg Pro Leu Ala Ile
65                  70                  75                  80

Trp Leu Phe Ile Phe Asn Val Ser Gln Gln Met Gly Gly Phe Tyr Leu
                85                  90                  95
```

```
Cys Gln Pro Gly Pro Pro Ser Glu Lys Ala Trp Gln Pro Gly Trp Thr
            100                 105                 110

Val Asn Val Glu Gly Ser Gly Glu Leu Phe Arg Trp Asn Val Ser Asp
        115                 120                 125

Leu Gly Gly Leu Gly Cys Gly Leu Lys Asn Arg Ser Ser Glu Gly Pro
    130                 135                 140

Ser Ser Pro Ser Gly Lys Leu Met Ser Pro Lys Leu Tyr Val Trp Ala
145                 150                 155                 160

Lys Asp Arg Pro Glu Ile Trp Glu Gly Glu Pro Pro Cys Leu Pro Pro
                165                 170                 175

Arg Asp Ser Leu Asn Gln Ser Leu Ser Gln Asp Leu Thr Met Ala Pro
            180                 185                 190

Gly Ser Thr Leu Trp Leu Ser Cys Gly Val Pro Pro Asp Ser Val Ser
        195                 200                 205

Arg Gly Pro Leu Ser Trp Thr His Val His Pro Lys Gly Pro Lys Ser
    210                 215                 220

Leu Leu Ser Leu Glu Leu Lys Asp Asp Arg Pro Ala Arg Asp Met Trp
225                 230                 235                 240

Val Met Glu Thr Gly Leu Leu Leu Pro Arg Ala Thr Ala Gln Asp Ala
                245                 250                 255

Gly Lys Tyr Tyr Cys His Arg Gly Asn Leu Thr Met Ser Phe His Leu
            260                 265                 270

Glu Ile Thr Ala Arg Pro Val Leu Trp His Trp Leu Leu Arg Thr Gly
        275                 280                 285

Gly Trp Lys Val Ser Ala Val Thr Leu Ala Tyr Leu Ile Phe Cys Leu
    290                 295                 300

Cys Ser Leu Val Gly Ile Leu His Leu Gln Arg Ala Leu Val Leu Arg
305                 310                 315                 320

Arg Lys Arg Lys Arg Met Thr Asp Pro Thr Arg Arg Phe Phe Lys Val
                325                 330                 335

Thr Pro Pro Pro Gly Ser Gly Pro Gln Asn Gln Tyr Gly Asn Val Leu
            340                 345                 350
```

```
Ser Leu Pro Thr Pro Thr Ser Gly Leu Gly Arg Ala Gln Arg Trp Ala
        355                 360                 365

Ala Gly Leu Gly Gly Thr Ala Pro Ser Tyr Gly Asn Pro Ser Ser Asp
    370                 375                 380

Val Gln Ala Asp Gly Ala Leu Gly Ser Arg Ser Pro Pro Gly Val Gly
385                 390                 395                 400

Pro Glu Glu Glu Glu Gly Glu Gly Tyr Glu Glu Pro Asp Ser Glu Glu
                405                 410                 415

Asp Ser Glu Phe Tyr Glu Asn Asp Ser Asn Leu Gly Gln Asp Gln Leu
            420                 425                 430

Ser Gln Asp Gly Ser Gly Tyr Glu Asn Pro Glu Asp Glu Pro Leu Gly
        435                 440                 445

Pro Glu Asp Glu Asp Ser Phe Ser Asn Ala Glu Ser Tyr Glu Asn Glu
    450                 455                 460

Asp Glu Glu Leu Thr Gln Pro Val Ala Arg Thr Met Asp Phe Leu Ser
465                 470                 475                 480

Pro His Gly Ser Ala Trp Asp Pro Ser Arg Glu Ala Thr Ser Leu Gly
                485                 490                 495

Ser Gln Ser Tyr Glu Asp Met Arg Gly Ile Leu Tyr Ala Ala Pro Gln
            500                 505                 510

Leu Arg Ser Ile Arg Gly Gln Pro Gly Pro Asn His Glu Glu Asp Ala
        515                 520                 525

Asp Ser Tyr Glu Asn Met Asp Asn Pro Asp Gly Pro Asp Pro Ala Trp
    530                 535                 540

Gly Gly Gly Gly Arg Met Gly Thr Trp Ser Thr Arg
545                 550                 555
```

<210> 65
<211> 297
<212> PRT
<213> Homo sapiens

<400> 65

```
Met Thr Thr Pro Arg Asn Ser Val Asn Gly Thr Phe Pro Ala Glu Pro
1               5                   10                  15
```

```
Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
```

260 265 270

Glu Glu Thr Glu Thr Asn Phe Pro Glu Pro Pro Gln Asp Gln Glu Ser
275 280 285

Ser Pro Ile Glu Asn Asp Ser Ser Pro
290 295

<210> 66
<211> 364
<212> PRT
<213> Homo sapiens

<400> 66

```
Met Pro Leu Leu Leu Leu Leu Pro Leu Leu Trp Ala Gly Ala Leu Ala
1               5                   10                  15

Met Asp Pro Asn Phe Trp Leu Gln Val Gln Glu Ser Val Thr Val Gln
            20                  25                  30

Glu Gly Leu Cys Val Leu Val Pro Cys Thr Phe Phe His Pro Ile Pro
        35                  40                  45

Tyr Tyr Asp Lys Asn Ser Pro Val His Gly Tyr Trp Phe Arg Glu Gly
    50                  55                  60

Ala Ile Ile Ser Arg Asp Ser Pro Val Ala Thr Asn Lys Leu Asp Gln
65                  70                  75                  80

Glu Val Gln Glu Glu Thr Gln Gly Arg Phe Arg Leu Leu Gly Asp Pro
                85                  90                  95

Ser Arg Asn Asn Cys Ser Leu Ser Ile Val Asp Ala Arg Arg Arg Asp
            100                 105                 110

Asn Gly Ser Tyr Phe Phe Arg Met Glu Arg Gly Ser Thr Lys Tyr Ser
        115                 120                 125

Tyr Lys Ser Pro Gln Leu Ser Val His Val Thr Asp Leu Thr His Arg
    130                 135                 140

Pro Lys Ile Leu Ile Pro Gly Thr Leu Glu Pro Gly His Ser Lys Asn
145                 150                 155                 160

Leu Thr Cys Ser Val Ser Trp Ala Cys Glu Gln Gly Thr Pro Pro Ile
                165                 170                 175

Phe Ser Trp Leu Ser Ala Ala Pro Thr Ser Leu Gly Pro Arg Thr Thr
```

```
                180                 185                 190

His Ser Ser Val Leu Ile Ile Thr Pro Arg Pro Gln Asp His Gly Thr
            195                 200                 205

Asn Leu Thr Cys Gln Val Lys Phe Ala Gly Ala Gly Val Thr Thr Glu
    210                 215                 220

Arg Thr Ile Gln Leu Asn Val Thr Tyr Val Pro Gln Asn Pro Thr Thr
225                 230                 235                 240

Gly Ile Phe Pro Gly Asp Gly Ser Gly Lys Gln Glu Thr Arg Ala Gly
                245                 250                 255

Val Val His Gly Ala Ile Gly Gly Ala Gly Val Thr Ala Leu Leu Ala
            260                 265                 270

Leu Cys Leu Cys Leu Ile Phe Phe Ile Val Lys Thr His Arg Arg Lys
        275                 280                 285

Ala Ala Arg Thr Ala Val Gly Arg Asn Asp Thr His Pro Thr Thr Gly
    290                 295                 300

Ser Ala Ser Pro Lys His Gln Lys Lys Ser Lys Leu His Gly Pro Thr
305                 310                 315                 320

Glu Thr Ser Ser Cys Ser Gly Ala Ala Pro Thr Val Glu Met Asp Glu
                325                 330                 335

Glu Leu His Tyr Ala Ser Leu Asn Phe His Gly Met Asn Pro Ser Lys
            340                 345                 350

Asp Thr Ser Thr Glu Tyr Ser Glu Val Arg Thr Gln
        355                 360
```

<210> 67
<211> 205
<212> PRT
<213> Homo sapiens

<400> 67

```
Met Thr Pro Pro Glu Arg Leu Phe Leu Pro Arg Val Cys Gly Thr Thr
1               5                   10                  15

Leu His Leu Leu Leu Leu Gly Leu Leu Leu Val Leu Leu Pro Gly Ala
            20                  25                  30

Gln Gly Leu Pro Gly Val Gly Leu Thr Pro Ser Ala Ala Gln Thr Ala

        35                  40                  45

Arg Gln His Pro Lys Met His Leu Ala His Ser Thr Leu Lys Pro Ala
    50                  55                  60

Ala His Leu Ile Gly Asp Pro Ser Lys Gln Asn Ser Leu Leu Trp Arg
65                  70                  75                  80

Ala Asn Thr Asp Arg Ala Phe Leu Gln Asp Gly Phe Ser Leu Ser Asn
                85                  90                  95

Asn Ser Leu Leu Val Pro Thr Ser Gly Ile Tyr Phe Val Tyr Ser Gln
            100                 105                 110

Val Val Phe Ser Gly Lys Ala Tyr Ser Pro Lys Ala Thr Ser Ser Pro
        115                 120                 125

Leu Tyr Leu Ala His Glu Val Gln Leu Phe Ser Ser Gln Tyr Pro Phe
    130                 135                 140

His Val Pro Leu Leu Ser Ser Gln Lys Met Val Tyr Pro Gly Leu Gln
145                 150                 155                 160

Glu Pro Trp Leu His Ser Met Tyr His Gly Ala Ala Phe Gln Leu Thr
                165                 170                 175

Gln Gly Asp Gln Leu Ser Thr His Thr Asp Gly Ile Pro His Leu Val
            180                 185                 190

Leu Ser Pro Ser Thr Val Phe Phe Gly Ala Phe Ala Leu
        195                 200                 205
```

<210> 68
<211> 233
<212> PRT
<213> Homo sapiens

<400> 68

```
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro

    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230
```

<210> 69
<211> 244
<212> PRT
<213> Homo sapiens

<400> 69

```
Met Gly Ala Leu Gly Leu Glu Gly Arg Gly Gly Arg Leu Gln Gly Arg
1               5                   10                  15

Gly Ser Leu Leu Leu Ala Val Ala Gly Ala Thr Ser Leu Val Thr Leu
            20                  25                  30

Leu Leu Ala Val Pro Ile Thr Val Leu Ala Val Leu Ala Leu Val Pro
```

35 40 45

Gln Asp Gln Gly Gly Leu Val Thr Glu Thr Ala Asp Pro Gly Ala Gln
50 55 60

Ala Gln Gln Gly Leu Gly Phe Gln Lys Leu Pro Glu Glu Glu Pro Glu
65 70 75 80

Thr Asp Leu Ser Pro Gly Leu Pro Ala Ala His Leu Ile Gly Ala Pro
85 90 95

Leu Lys Gly Gln Gly Leu Gly Trp Glu Thr Thr Lys Glu Gln Ala Phe
100 105 110

Leu Thr Ser Gly Thr Gln Phe Ser Asp Ala Glu Gly Leu Ala Leu Pro
115 120 125

Gln Asp Gly Leu Tyr Tyr Leu Tyr Cys Leu Val Gly Tyr Arg Gly Arg
130 135 140

Ala Pro Pro Gly Gly Gly Asp Pro Gln Gly Arg Ser Val Thr Leu Arg
145 150 155 160

Ser Ser Leu Tyr Arg Ala Gly Gly Ala Tyr Gly Pro Gly Thr Pro Glu
165 170 175

Leu Leu Leu Glu Gly Ala Glu Thr Val Thr Pro Val Leu Asp Pro Ala
180 185 190

Arg Arg Gln Gly Tyr Gly Pro Leu Trp Tyr Thr Ser Val Gly Phe Gly
195 200 205

Gly Leu Val Gln Leu Arg Arg Gly Glu Arg Val Tyr Val Asn Ile Ser
210 215 220

His Pro Asp Met Val Asp Phe Ala Arg Gly Lys Thr Phe Phe Gly Ala
225 230 235 240

Val Met Val Gly

<210> 70
<211> 183
<212> PRT
<213> Homo sapiens

<400> 70

```
Met Glu Arg Val Gln Pro Leu Glu Glu Asn Val Gly Asn Ala Ala Arg
1               5                   10                  15

Pro Arg Phe Glu Arg Asn Lys Leu Leu Leu Val Ala Ser Val Ile Gln
            20                  25                  30

Gly Leu Gly Leu Leu Leu Cys Phe Thr Tyr Ile Cys Leu His Phe Ser
        35                  40                  45

Ala Leu Gln Val Ser His Arg Tyr Pro Arg Ile Gln Ser Ile Lys Val
    50                  55                  60

Gln Phe Thr Glu Tyr Lys Lys Glu Lys Gly Phe Ile Leu Thr Ser Gln
65                  70                  75                  80

Lys Glu Asp Glu Ile Met Lys Val Gln Asn Asn Ser Val Ile Ile Asn
                85                  90                  95

Cys Asp Gly Phe Tyr Leu Ile Ser Leu Lys Gly Tyr Phe Ser Gln Glu
            100                 105                 110

Val Asn Ile Ser Leu His Tyr Gln Lys Asp Glu Glu Pro Leu Phe Gln
        115                 120                 125

Leu Lys Lys Val Arg Ser Val Asn Ser Leu Met Val Ala Ser Leu Thr
    130                 135                 140

Tyr Lys Asp Lys Val Tyr Leu Asn Val Thr Thr Asp Asn Thr Ser Leu
145                 150                 155                 160

Asp Asp Phe His Val Asn Gly Gly Glu Leu Ile Leu Ile His Gln Asn
                165                 170                 175

Pro Gly Glu Phe Cys Val Leu
            180
```

<210> 71
<211> 261
<212> PRT
<213> Homo sapiens

<400> 71

Met Ile Glu Thr Tyr Asn Gln Thr Ser Pro Arg Ser Ala Ala Thr Gly
1 5 10 15

Leu Pro Ile Ser Met Lys Ile Phe Met Tyr Leu Leu Thr Val Phe Leu
20 25 30

Ile Thr Gln Met Ile Gly Ser Ala Leu Phe Ala Val Tyr Leu His Arg

35 40 45

Arg Leu Asp Lys Ile Glu Asp Glu Arg Asn Leu His Glu Asp Phe Val
50 55 60

Phe Met Lys Thr Ile Gln Arg Cys Asn Thr Gly Glu Arg Ser Leu Ser
65 70 75 80

Leu Leu Asn Cys Glu Glu Ile Lys Ser Gln Phe Glu Gly Phe Val Lys
85 90 95

Asp Ile Met Leu Asn Lys Glu Glu Thr Lys Lys Glu Asn Ser Phe Glu
100 105 110

Met Gln Lys Gly Asp Gln Asn Pro Gln Ile Ala Ala His Val Ile Ser
115 120 125

Glu Ala Ser Ser Lys Thr Thr Ser Val Leu Gln Trp Ala Glu Lys Gly
130 135 140

Tyr Tyr Thr Met Ser Asn Asn Leu Val Thr Leu Glu Asn Gly Lys Gln
145 150 155 160

Leu Thr Val Lys Arg Gln Gly Leu Tyr Tyr Ile Tyr Ala Gln Val Thr
165 170 175

Phe Cys Ser Asn Arg Glu Ala Ser Ser Gln Ala Pro Phe Ile Ala Ser
180 185 190

Leu Cys Leu Lys Ser Pro Gly Arg Phe Glu Arg Ile Leu Leu Arg Ala
195 200 205

Ala Asn Thr His Ser Ser Ala Lys Pro Cys Gly Gln Gln Ser Ile His
210 215 220

Leu Gly Gly Val Phe Glu Leu Gln Pro Gly Ala Ser Val Phe Val Asn
225 230 235 240

Val Thr Asp Pro Ser Gln Val Ser His Gly Thr Gly Phe Thr Ser Phe
245 250 255

Gly Leu Leu Lys Leu
260

<210> 72
<211> 281
<212> PRT

<213> Homo sapiens

<400> 72

```
Met Gln Gln Pro Phe Asn Tyr Pro Tyr Pro Gln Ile Tyr Trp Val Asp
1               5                   10                  15

Ser Ser Ala Ser Ser Pro Trp Ala Pro Pro Gly Thr Val Leu Pro Cys
            20                  25                  30

Pro Thr Ser Val Pro Arg Arg Pro Gly Gln Arg Arg Pro Pro Pro Pro
        35                  40                  45

Pro Pro Pro Pro Pro Leu Pro Pro Pro Pro Pro Pro Pro Pro Leu Pro
    50                  55                  60

Pro Leu Pro Leu Pro Pro Leu Lys Lys Arg Gly Asn His Ser Thr Gly
65                  70                  75                  80

Leu Cys Leu Leu Val Met Phe Phe Met Val Leu Val Ala Leu Val Gly
                85                  90                  95

Leu Gly Leu Gly Met Phe Gln Leu Phe His Leu Gln Lys Glu Leu Ala
            100                 105                 110

Glu Leu Arg Glu Ser Thr Ser Gln Met His Thr Ala Ser Ser Leu Glu
        115                 120                 125

Lys Gln Ile Gly His Pro Ser Pro Pro Pro Glu Lys Lys Glu Leu Arg
    130                 135                 140

Lys Val Ala His Leu Thr Gly Lys Ser Asn Ser Arg Ser Met Pro Leu
145                 150                 155                 160

Glu Trp Glu Asp Thr Tyr Gly Ile Val Leu Leu Ser Gly Val Lys Tyr
                165                 170                 175

Lys Lys Gly Gly Leu Val Ile Asn Glu Thr Gly Leu Tyr Phe Val Tyr
            180                 185                 190

Ser Lys Val Tyr Phe Arg Gly Gln Ser Cys Asn Asn Leu Pro Leu Ser
        195                 200                 205

His Lys Val Tyr Met Arg Asn Ser Lys Tyr Pro Gln Asp Leu Val Met
    210                 215                 220

Met Glu Gly Lys Met Met Ser Tyr Cys Thr Thr Gly Gln Met Trp Ala
225                 230                 235                 240
```

Arg Ser Ser Tyr Leu Gly Ala Val Phe Asn Leu Thr Ser Ala Asp His
245 250 255

Leu Tyr Val Asn Val Ser Glu Leu Ser Leu Val Asn Phe Glu Glu Ser
260 265 270

Gln Thr Phe Phe Gly Leu Tyr Lys Leu
275 280

<210> 73
<211> 193
<212> PRT
<213> Homo sapiens

<400> 73

Met Pro Glu Glu Gly Ser Gly Cys Ser Val Arg Arg Arg Pro Tyr Gly
1 5 10 15

Cys Val Leu Arg Ala Ala Leu Val Pro Leu Val Ala Gly Leu Val Ile
20 25 30

Cys Leu Val Val Cys Ile Gln Arg Phe Ala Gln Ala Gln Gln Gln Leu
35 40 45

Pro Leu Glu Ser Leu Gly Trp Asp Val Ala Glu Leu Gln Leu Asn His
50 55 60

Thr Gly Pro Gln Gln Asp Pro Arg Leu Tyr Trp Gln Gly Gly Pro Ala
65 70 75 80

Leu Gly Arg Ser Phe Leu His Gly Pro Glu Leu Asp Lys Gly Gln Leu
85 90 95

Arg Ile His Arg Asp Gly Ile Tyr Met Val His Ile Gln Val Thr Leu
100 105 110

Ala Ile Cys Ser Ser Thr Thr Ala Ser Arg His His Pro Thr Thr Leu
115 120 125

Ala Val Gly Ile Cys Ser Pro Ala Ser Arg Ser Ile Ser Leu Leu Arg
130 135 140

Leu Ser Phe His Gln Gly Cys Thr Ile Ala Ser Gln Arg Leu Thr Pro
145 150 155 160

Leu Ala Arg Gly Asp Thr Leu Cys Thr Asn Leu Thr Gly Thr Leu Leu
165 170 175

Pro Ser Arg Asn Thr Asp Glu Thr Phe Phe Gly Val Gln Trp Val Arg
180 185 190

Pro

<210> 74
<211> 234
<212> PRT
<213> Homo sapiens

<400> 74

Met Asp Pro Gly Leu Gln Gln Ala Leu Asn Gly Met Ala Pro Pro Gly
1 5 10 15

Asp Thr Ala Met His Val Pro Ala Gly Ser Val Ala Ser His Leu Gly
20 25 30

Thr Thr Ser Arg Ser Tyr Phe Tyr Leu Thr Thr Ala Thr Leu Ala Leu
35 40 45

Cys Leu Val Phe Thr Val Ala Thr Ile Met Val Leu Val Val Gln Arg
50 55 60

Thr Asp Ser Ile Pro Asn Ser Pro Asp Asn Val Pro Leu Lys Gly Gly
65 70 75 80

Asn Cys Ser Glu Asp Leu Leu Cys Ile Leu Lys Arg Ala Pro Phe Lys
85 90 95

Lys Ser Trp Ala Tyr Leu Gln Val Ala Lys His Leu Asn Lys Thr Lys
100 105 110

Leu Ser Trp Asn Lys Asp Gly Ile Leu His Gly Val Arg Tyr Gln Asp
115 120 125

Gly Asn Leu Val Ile Gln Phe Pro Gly Leu Tyr Phe Ile Ile Cys Gln
130 135 140

Leu Gln Phe Leu Val Gln Cys Pro Asn Asn Ser Val Asp Leu Lys Leu
145 150 155 160

Glu Leu Leu Ile Asn Lys His Ile Lys Lys Gln Ala Leu Val Thr Val
165 170 175

Cys Glu Ser Gly Met Gln Thr Lys His Val Tyr Gln Asn Leu Ser Gln
180 185 190

Phe Leu Leu Asp Tyr Leu Gln Val Asn Thr Thr Ile Ser Val Asn Val
        195                 200                 205

Asp Thr Phe Gln Tyr Ile Asp Thr Ser Thr Phe Pro Leu Glu Asn Val
    210                 215                 220

Leu Ser Ile Phe Leu Tyr Ser Asn Ser Asp
225                 230

<210> 75
<211> 254
<212> PRT
<213> Homo sapiens

<400> 75

Met Glu Tyr Ala Ser Asp Ala Ser Leu Asp Pro Glu Ala Pro Trp Pro
1               5                   10                  15

Pro Ala Pro Arg Ala Arg Ala Cys Arg Val Leu Pro Trp Ala Leu Val
            20                  25                  30

Ala Gly Leu Leu Leu Leu Leu Leu Leu Ala Ala Ala Cys Ala Val Phe
        35                  40                  45

Leu Ala Cys Pro Trp Ala Val Ser Gly Ala Arg Ala Ser Pro Gly Ser
    50                  55                  60

Ala Ala Ser Pro Arg Leu Arg Glu Gly Pro Glu Leu Ser Pro Asp Asp
65                  70                  75                  80

Pro Ala Gly Leu Leu Asp Leu Arg Gln Gly Met Phe Ala Gln Leu Val
                85                  90                  95

Ala Gln Asn Val Leu Leu Ile Asp Gly Pro Leu Ser Trp Tyr Ser Asp
            100                 105                 110

Pro Gly Leu Ala Gly Val Ser Leu Thr Gly Gly Leu Ser Tyr Lys Glu
        115                 120                 125

Asp Thr Lys Glu Leu Val Val Ala Lys Ala Gly Val Tyr Tyr Val Phe
    130                 135                 140

Phe Gln Leu Glu Leu Arg Arg Val Val Ala Gly Glu Gly Ser Gly Ser
145                 150                 155                 160

Val Ser Leu Ala Leu His Leu Gln Pro Leu Arg Ser Ala Ala Gly Ala
                165                 170                 175

Ala Ala Leu Ala Leu Thr Val Asp Leu Pro Pro Ala Ser Ser Glu Ala
180 185 190

Arg Asn Ser Ala Phe Gly Phe Gln Gly Arg Leu Leu His Leu Ser Ala
195 200 205

Gly Gln Arg Leu Gly Val His Leu His Thr Glu Ala Arg Ala Arg His
210 215 220

Ala Trp Gln Leu Thr Gln Gly Ala Thr Val Leu Gly Leu Phe Arg Val
225 230 235 240

Thr Pro Glu Ile Pro Ala Gly Leu Pro Ser Pro Arg Ser Glu
245 250

<210> 76
<211> 281
<212> PRT
<213> Homo sapiens

<400> 76

Met Ala Met Met Glu Val Gln Gly Gly Pro Ser Leu Gly Gln Thr Cys
1 5 10 15

Val Leu Ile Val Ile Phe Thr Val Leu Leu Gln Ser Leu Cys Val Ala
20 25 30

Val Thr Tyr Val Tyr Phe Thr Asn Glu Leu Lys Gln Met Gln Asp Lys
35 40 45

Tyr Ser Lys Ser Gly Ile Ala Cys Phe Leu Lys Glu Asp Asp Ser Tyr
50 55 60

Trp Asp Pro Asn Asp Glu Glu Ser Met Asn Ser Pro Cys Trp Gln Val
65 70 75 80

Lys Trp Gln Leu Arg Gln Leu Val Arg Lys Met Ile Leu Arg Thr Ser
85 90 95

Glu Glu Thr Ile Ser Thr Val Gln Glu Lys Gln Gln Asn Ile Ser Pro
100 105 110

Leu Val Arg Glu Arg Gly Pro Gln Arg Val Ala Ala His Ile Thr Gly
115 120 125

Thr Arg Gly Arg Ser Asn Thr Leu Ser Ser Pro Asn Ser Lys Asn Glu
130 135 140

Lys Ala Leu Gly Arg Lys Ile Asn Ser Trp Glu Ser Ser Arg Ser Gly
145 150 155 160

His Ser Phe Leu Ser Asn Leu His Leu Arg Asn Gly Glu Leu Val Ile
165 170 175

His Glu Lys Gly Phe Tyr Tyr Ile Tyr Ser Gln Thr Tyr Phe Arg Phe
180 185 190

Gln Glu Glu Ile Lys Glu Asn Thr Lys Asn Asp Lys Gln Met Val Gln
195 200 205

Tyr Ile Tyr Lys Tyr Thr Ser Tyr Pro Asp Pro Ile Leu Leu Met Lys
210 215 220

Ser Ala Arg Asn Ser Cys Trp Ser Lys Asp Ala Glu Tyr Gly Leu Tyr
225 230 235 240

Ser Ile Tyr Gln Gly Gly Ile Phe Glu Leu Lys Glu Asn Asp Arg Ile
245 250 255

Phe Val Ser Val Thr Asn Glu His Leu Ile Asp Met Asp His Glu Ala
260 265 270

Ser Phe Phe Gly Ala Phe Leu Val Gly
275 280

<210> 77
<211> 317
<212> PRT
<213> Homo sapiens

<400> 77

Met Arg Arg Ala Ser Arg Asp Tyr Thr Lys Tyr Leu Arg Gly Ser Glu
1 5 10 15

Glu Met Gly Gly Gly Pro Gly Ala Pro His Glu Gly Pro Leu His Ala
20 25 30

Pro Pro Pro Pro Ala Pro His Gln Pro Pro Ala Ala Ser Arg Ser Met
35 40 45

Phe Val Ala Leu Leu Gly Leu Gly Leu Gly Gln Val Val Cys Ser Val
50 55 60

Ala Leu Phe Phe Tyr Phe Arg Ala Gln Met Asp Pro Asn Arg Ile Ser
65 70 75 80

Glu Asp Gly Thr His Cys Ile Tyr Arg Ile Leu Arg Leu His Glu Asn
85 90 95

Ala Asp Phe Gln Asp Thr Thr Leu Glu Ser Gln Asp Thr Lys Leu Ile
100 105 110

Pro Asp Ser Cys Arg Arg Ile Lys Gln Ala Phe Gln Gly Ala Val Gln
115 120 125

Lys Glu Leu Gln His Ile Val Gly Ser Gln His Ile Arg Ala Glu Lys
130 135 140

Ala Met Val Asp Gly Ser Trp Leu Asp Leu Ala Lys Arg Ser Lys Leu
145 150 155 160

Glu Ala Gln Pro Phe Ala His Leu Thr Ile Asn Ala Thr Asp Ile Pro
165 170 175

Ser Gly Ser His Lys Val Ser Leu Ser Ser Trp Tyr His Asp Arg Gly
180 185 190

Trp Ala Lys Ile Ser Asn Met Thr Phe Ser Asn Gly Lys Leu Ile Val
195 200 205

Asn Gln Asp Gly Phe Tyr Tyr Leu Tyr Ala Asn Ile Cys Phe Arg His
210 215 220

His Glu Thr Ser Gly Asp Leu Ala Thr Glu Tyr Leu Gln Leu Met Val
225 230 235 240

Tyr Val Thr Lys Thr Ser Ile Lys Ile Pro Ser Ser His Thr Leu Met
245 250 255

Lys Gly Gly Ser Thr Lys Tyr Trp Ser Gly Asn Ser Glu Phe His Phe
260 265 270

Tyr Ser Ile Asn Val Gly Gly Phe Phe Lys Leu Arg Ser Gly Glu Glu
275 280 285

Ile Ser Ile Glu Val Ser Asn Pro Ser Leu Leu Asp Pro Asp Gln Asp
290 295 300

Ala Thr Tyr Phe Gly Ala Phe Lys Val Arg Asp Ile Asp
305 310 315

<210> 78
<211> 249

<212> PRT
<213> Homo sapiens

<400> 78

```
Met Ala Ala Arg Arg Ser Gln Arg Arg Arg Gly Arg Arg Gly Glu Pro
1               5                   10                  15

Gly Thr Ala Leu Leu Val Pro Leu Ala Leu Gly Leu Gly Leu Ala Leu
            20                  25                  30

Ala Cys Leu Gly Leu Leu Leu Ala Val Val Ser Leu Gly Ser Arg Ala
        35                  40                  45

Ser Leu Ser Ala Gln Glu Pro Ala Gln Glu Glu Leu Val Ala Glu Glu
    50                  55                  60

Asp Gln Asp Pro Ser Glu Leu Asn Pro Gln Thr Glu Glu Ser Gln Asp
65                  70                  75                  80

Pro Ala Pro Phe Leu Asn Arg Leu Val Arg Pro Arg Arg Ser Ala Pro
                85                  90                  95

Lys Gly Arg Lys Thr Arg Ala Arg Arg Ala Ile Ala Ala His Tyr Glu
            100                 105                 110

Val His Pro Arg Pro Gly Gln Asp Gly Ala Gln Ala Gly Val Asp Gly
        115                 120                 125

Thr Val Ser Gly Trp Glu Glu Ala Arg Ile Asn Ser Ser Ser Pro Leu
    130                 135                 140

Arg Tyr Asn Arg Gln Ile Gly Glu Phe Ile Val Thr Arg Ala Gly Leu
145                 150                 155                 160

Tyr Tyr Leu Tyr Cys Gln Val His Phe Asp Glu Gly Lys Ala Val Tyr
                165                 170                 175

Leu Lys Leu Asp Leu Leu Val Asp Gly Val Leu Ala Leu Arg Cys Leu
            180                 185                 190

Glu Glu Phe Ser Ala Thr Ala Ala Ser Ser Leu Gly Pro Gln Leu Arg
        195                 200                 205

Leu Cys Gln Val Ser Gly Leu Leu Ala Leu Arg Pro Gly Ser Ser Leu
    210                 215                 220

Arg Ile Arg Thr Leu Pro Trp Ala His Leu Lys Ala Ala Pro Phe Leu
225                 230                 235                 240

Thr Tyr Phe Gly Leu Phe Gln Val His
                245
```

<210> 79
<211> 250
<212> PRT
<213> Homo sapiens

<400> 79

```
Met Pro Ala Ser Ser Pro Phe Leu Leu Ala Pro Lys Gly Pro Pro Gly
1               5                   10                  15

Asn Met Gly Gly Pro Val Arg Glu Pro Ala Leu Ser Val Ala Leu Trp
            20                  25                  30

Leu Ser Trp Gly Ala Ala Leu Gly Ala Val Ala Cys Ala Met Ala Leu
        35                  40                  45

Leu Thr Gln Gln Thr Glu Leu Gln Ser Leu Arg Arg Glu Val Ser Arg
    50                  55                  60

Leu Gln Gly Thr Gly Gly Pro Ser Gln Asn Gly Glu Gly Tyr Pro Trp
65                  70                  75                  80

Gln Ser Leu Pro Glu Gln Ser Ser Asp Ala Leu Glu Ala Trp Glu Asn
                85                  90                  95

Gly Glu Arg Ser Arg Lys Arg Arg Ala Val Leu Thr Gln Lys Gln Lys
            100                 105                 110

Lys Gln His Ser Val Leu His Leu Val Pro Ile Asn Ala Thr Ser Lys
        115                 120                 125

Asp Asp Ser Asp Val Thr Glu Val Met Trp Gln Pro Ala Leu Arg Arg
    130                 135                 140

Gly Arg Gly Leu Gln Ala Gln Gly Tyr Gly Val Arg Ile Gln Asp Ala
145                 150                 155                 160

Gly Val Tyr Leu Leu Tyr Ser Gln Val Leu Phe Gln Asp Val Thr Phe
                165                 170                 175

Thr Met Gly Gln Val Val Ser Arg Glu Gly Gln Gly Arg Gln Glu Thr
            180                 185                 190

Leu Phe Arg Cys Ile Arg Ser Met Pro Ser His Pro Asp Arg Ala Tyr
        195                 200                 205
```

```
Asn Ser Cys Tyr Ser Ala Gly Val Phe His Leu His Gln Gly Asp Ile
    210                 215                 220

Leu Ser Val Ile Ile Pro Arg Ala Arg Ala Lys Leu Asn Leu Ser Pro
225                 230                 235                 240

His Gly Thr Phe Leu Gly Phe Val Lys Leu
                245                 250
```

<210> 80
<211> 285
<212> PRT
<213> Homo sapiens

<400> 80

```
Met Asp Asp Ser Thr Glu Arg Glu Gln Ser Arg Leu Thr Ser Cys Leu
1               5                   10                  15

Lys Lys Arg Glu Glu Met Lys Leu Lys Glu Cys Val Ser Ile Leu Pro
            20                  25                  30

Arg Lys Glu Ser Pro Ser Val Arg Ser Ser Lys Asp Gly Lys Leu Leu
        35                  40                  45

Ala Ala Thr Leu Leu Leu Ala Leu Leu Ser Cys Cys Leu Thr Val Val
    50                  55                  60

Ser Phe Tyr Gln Val Ala Ala Leu Gln Gly Asp Leu Ala Ser Leu Arg
65                  70                  75                  80

Ala Glu Leu Gln Gly His His Ala Glu Lys Leu Pro Ala Gly Ala Gly
                85                  90                  95

Ala Pro Lys Ala Gly Leu Glu Glu Ala Pro Ala Val Thr Ala Gly Leu
            100                 105                 110

Lys Ile Phe Glu Pro Pro Ala Pro Gly Glu Gly Asn Ser Ser Gln Asn
        115                 120                 125

Ser Arg Asn Lys Arg Ala Val Gln Gly Pro Glu Glu Thr Val Thr Gln
    130                 135                 140

Asp Cys Leu Gln Leu Ile Ala Asp Ser Glu Thr Pro Thr Ile Gln Lys
145                 150                 155                 160

Gly Ser Tyr Thr Phe Val Pro Trp Leu Leu Ser Phe Lys Arg Gly Ser
                165                 170                 175
```

```
Ala Leu Glu Glu Lys Glu Asn Lys Ile Leu Val Lys Glu Thr Gly Tyr
            180                 185                 190

Phe Phe Ile Tyr Gly Gln Val Leu Tyr Thr Asp Lys Thr Tyr Ala Met
        195                 200                 205

Gly His Leu Ile Gln Arg Lys Lys Val His Val Phe Gly Asp Glu Leu
    210                 215                 220

Ser Leu Val Thr Leu Phe Arg Cys Ile Gln Asn Met Pro Glu Thr Leu
225                 230                 235                 240

Pro Asn Asn Ser Cys Tyr Ser Ala Gly Ile Ala Lys Leu Glu Glu Gly
                245                 250                 255

Asp Glu Leu Gln Leu Ala Ile Pro Arg Glu Asn Ala Gln Ile Ser Leu
            260                 265                 270

Asp Gly Asp Val Thr Phe Phe Gly Ala Leu Lys Leu Leu
        275                 280                 285
```

<210> 81
<211> 240
<212> PRT
<213> Homo sapiens

<400> 81

```
Met Glu Glu Ser Val Val Arg Pro Ser Val Phe Val Val Asp Gly Gln
1               5                   10                  15

Thr Asp Ile Pro Phe Thr Arg Leu Gly Arg Ser His Arg Arg Gln Ser
            20                  25                  30

Cys Ser Val Ala Arg Val Gly Leu Gly Leu Leu Leu Leu Leu Met Gly
        35                  40                  45

Ala Gly Leu Ala Val Gln Gly Trp Phe Leu Leu Gln Leu His Trp Arg
    50                  55                  60

Leu Gly Glu Met Val Thr Arg Leu Pro Asp Gly Pro Ala Gly Ser Trp
65                  70                  75                  80

Glu Gln Leu Ile Gln Glu Arg Arg Ser His Glu Val Asn Pro Ala Ala
                85                  90                  95

His Leu Thr Gly Ala Asn Ser Ser Leu Thr Gly Ser Gly Gly Pro Leu
            100                 105                 110
```

Leu Trp Glu Thr Gln Leu Gly Leu Ala Phe Leu Arg Gly Leu Ser Tyr
115 120 125

His Asp Gly Ala Leu Val Val Thr Lys Ala Gly Tyr Tyr Tyr Ile Tyr
130 135 140

Ser Lys Val Gln Leu Gly Gly Val Gly Cys Pro Leu Gly Leu Ala Ser
145 150 155 160

Thr Ile Thr His Gly Leu Tyr Lys Arg Thr Pro Arg Tyr Pro Glu Glu
165 170 175

Leu Glu Leu Leu Val Ser Gln Gln Ser Pro Cys Gly Arg Ala Thr Ser
180 185 190

Ser Ser Arg Val Trp Trp Asp Ser Ser Phe Leu Gly Gly Val Val His
195 200 205

Leu Glu Ala Gly Glu Lys Val Val Val Arg Val Leu Asp Glu Arg Leu
210 215 220

Val Arg Leu Arg Asp Gly Thr Arg Ser Tyr Phe Gly Ala Phe Met Val
225 230 235 240

<210> 82
<211> 251
<212> PRT
<213> Homo sapiens

<400> 82

Met Ala Glu Asp Leu Gly Leu Ser Phe Gly Glu Thr Ala Ser Val Glu
1 5 10 15

Met Leu Pro Glu His Gly Ser Cys Arg Pro Lys Ala Arg Ser Ser Ser
20 25 30

Ala Arg Trp Ala Leu Thr Cys Cys Leu Val Leu Leu Pro Phe Leu Ala
35 40 45

Gly Leu Thr Thr Tyr Leu Leu Val Ser Gln Leu Arg Ala Gln Gly Glu
50 55 60

Ala Cys Val Gln Phe Gln Ala Leu Lys Gly Gln Glu Phe Ala Pro Ser
65 70 75 80

His Gln Gln Val Tyr Ala Pro Leu Arg Ala Asp Gly Asp Lys Pro Arg
85 90 95

```
Ala His Leu Thr Val Val Arg Gln Thr Pro Thr Gln His Phe Lys Asn
            100                 105                 110

Gln Phe Pro Ala Leu His Trp Glu His Glu Leu Gly Leu Ala Phe Thr
        115                 120                 125

Lys Asn Arg Met Asn Tyr Thr Asn Lys Phe Leu Leu Ile Pro Glu Ser
    130                 135                 140

Gly Asp Tyr Phe Ile Tyr Ser Gln Val Thr Phe Arg Gly Met Thr Ser
145                 150                 155                 160

Glu Cys Ser Glu Ile Arg Gln Ala Gly Arg Pro Asn Lys Pro Asp Ser
                165                 170                 175

Ile Thr Val Val Ile Thr Lys Val Thr Asp Ser Tyr Pro Glu Pro Thr
            180                 185                 190

Gln Leu Leu Met Gly Thr Lys Ser Val Cys Glu Val Gly Ser Asn Trp
        195                 200                 205

Phe Gln Pro Ile Tyr Leu Gly Ala Met Phe Ser Leu Gln Glu Gly Asp
    210                 215                 220

Lys Leu Met Val Asn Val Ser Asp Ile Ser Leu Val Asp Tyr Thr Lys
225                 230                 235                 240

Glu Asp Lys Thr Phe Phe Gly Ala Phe Leu Leu
                245                 250
```

<210> 83
<211> 199
<212> PRT
<213> Homo sapiens

<400> 83

```
Met Thr Leu His Pro Ser Pro Ile Thr Cys Glu Phe Leu Phe Ser Thr
1               5                   10                  15

Ala Leu Ile Ser Pro Lys Met Cys Leu Ser His Leu Glu Asn Met Pro
            20                  25                  30

Leu Ser His Ser Arg Thr Gln Gly Ala Gln Arg Ser Ser Trp Lys Leu
        35                  40                  45

Trp Leu Phe Cys Ser Ile Val Met Leu Leu Phe Leu Cys Ser Phe Ser
    50                  55                  60
```

Trp Leu Ile Phe Ile Phe Leu Gln Leu Glu Thr Ala Lys Glu Pro Cys
65 70 75 80

Met Ala Lys Phe Gly Pro Leu Pro Ser Lys Trp Gln Met Ala Ser Ser
85 90 95

Glu Pro Pro Cys Val Asn Lys Val Ser Asp Trp Lys Leu Glu Ile Leu
100 105 110

Gln Asn Gly Leu Tyr Leu Ile Tyr Gly Gln Val Ala Pro Asn Ala Asn
115 120 125

Tyr Asn Asp Val Ala Pro Phe Glu Val Arg Leu Tyr Lys Asn Lys Asp
130 135 140

Met Ile Gln Thr Leu Thr Asn Lys Ser Lys Ile Gln Asn Val Gly Gly
145 150 155 160

Thr Tyr Glu Leu His Val Gly Asp Thr Ile Asp Leu Ile Phe Asn Ser
165 170 175

Glu His Gln Val Leu Lys Asn Asn Thr Tyr Trp Gly Ile Ile Leu Leu
180 185 190

Ala Asn Pro Gln Phe Ile Ser
195

<210> 84
<211> 391
<212> PRT
<213> Homo sapiens

<400> 84

Met Gly Tyr Pro Glu Val Glu Arg Arg Glu Leu Leu Pro Ala Ala Ala
1 5 10 15

Pro Arg Glu Arg Gly Ser Gln Gly Cys Gly Cys Gly Gly Ala Pro Ala
20 25 30

Arg Ala Gly Glu Gly Asn Ser Cys Leu Leu Phe Leu Gly Phe Phe Gly
35 40 45

Leu Ser Leu Ala Leu His Leu Leu Thr Leu Cys Cys Tyr Leu Glu Leu
50 55 60

Arg Ser Glu Leu Arg Arg Glu Arg Gly Ala Glu Ser Arg Leu Gly Gly
65 70 75 80

Ser Gly Thr Pro Gly Thr Ser Gly Thr Leu Ser Ser Leu Gly Gly Leu
85 90 95

Asp Pro Asp Ser Pro Ile Thr Ser His Leu Gly Gln Pro Ser Pro Lys
100 105 110

Gln Gln Pro Leu Glu Pro Gly Glu Ala Ala Leu His Ser Asp Ser Gln
115 120 125

Asp Gly His Gln Met Ala Leu Leu Asn Phe Phe Phe Pro Asp Glu Lys
130 135 140

Pro Tyr Ser Glu Glu Glu Ser Arg Arg Val Arg Arg Asn Lys Arg Ser
145 150 155 160

Lys Ser Asn Glu Gly Ala Asp Gly Pro Val Lys Asn Lys Lys Lys Gly
165 170 175

Lys Lys Ala Gly Pro Pro Gly Pro Asn Gly Pro Pro Gly Pro Pro Gly
180 185 190

Pro Pro Gly Pro Gln Gly Pro Pro Gly Ile Pro Gly Ile Pro Gly Ile
195 200 205

Pro Gly Thr Thr Val Met Gly Pro Pro Gly Pro Pro Gly Pro Pro Gly
210 215 220

Pro Gln Gly Pro Pro Gly Leu Gln Gly Pro Ser Gly Ala Ala Asp Lys
225 230 235 240

Ala Gly Thr Arg Glu Asn Gln Pro Ala Val Val His Leu Gln Gly Gln
245 250 255

Gly Ser Ala Ile Gln Val Lys Asn Asp Leu Ser Gly Gly Val Leu Asn
260 265 270

Asp Trp Ser Arg Ile Thr Met Asn Pro Lys Val Phe Lys Leu His Pro
275 280 285

Arg Ser Gly Glu Leu Glu Val Leu Val Asp Gly Thr Tyr Phe Ile Tyr
290 295 300

Ser Gln Val Glu Val Tyr Tyr Ile Asn Phe Thr Asp Phe Ala Ser Tyr
305 310 315 320

Glu Val Val Val Asp Glu Lys Pro Phe Leu Gln Cys Thr Arg Ser Ile
325 330 335

Glu Thr Gly Lys Thr Asn Tyr Asn Thr Cys Tyr Thr Ala Gly Val Cys
340 345 350

Leu Leu Lys Ala Arg Gln Lys Ile Ala Val Lys Met Val His Ala Asp
355 360 365

Ile Ser Ile Asn Met Ser Lys His Thr Thr Phe Phe Gly Ala Ile Arg
370 375 380

Leu Gly Glu Ala Pro Ala Ser
385 390

<210> 85
<211> 205
<212> PRT
<213> Homo sapiens

<400> 85

Ala Cys Pro Trp Ala Val Ser Gly Ala Arg Ala Ser Pro Gly Ser Ala
1 5 10 15

Ala Ser Pro Arg Leu Arg Glu Gly Pro Glu Leu Ser Pro Asp Asp Pro
20 25 30

Ala Gly Leu Leu Asp Leu Arg Gln Gly Met Phe Ala Gln Leu Val Ala
35 40 45

Gln Asn Val Leu Leu Ile Asp Gly Pro Leu Ser Trp Tyr Ser Asp Pro
50 55 60

Gly Leu Ala Gly Val Ser Leu Thr Gly Gly Leu Ser Tyr Lys Glu Asp
65 70 75 80

Thr Lys Glu Leu Val Val Ala Lys Ala Gly Val Tyr Tyr Val Phe Phe
85 90 95

Gln Leu Glu Leu Arg Arg Val Val Ala Gly Glu Gly Ser Gly Ser Val
100 105 110

Ser Leu Ala Leu His Leu Gln Pro Leu Arg Ser Ala Ala Gly Ala Ala
115 120 125

Ala Leu Ala Leu Thr Val Asp Leu Pro Pro Ala Ser Ser Glu Ala Arg
130 135 140

Asn Ser Ala Phe Gly Phe Gln Gly Arg Leu Leu His Leu Ser Ala Gly
145 150 155 160

Gln Arg Leu Gly Val His Leu His Thr Glu Ala Arg Ala Arg His Ala
165 170 175

Trp Gln Leu Thr Gln Gly Ala Thr Val Leu Gly Leu Phe Arg Val Thr
180 185 190

Pro Glu Ile Pro Ala Gly Leu Pro Ser Pro Arg Ser Glu
195 200 205

<210> 86
<211> 5
<212> PRT
<213> Artificial Sequence

<220>
<223> Peptide linker G4S

<400> 86

Gly Gly Gly Gly Ser
1 5

<210> 87
<211> 10
<212> PRT
<213> Artificial Sequence

<220>
<223> Peptide linker (G4S)2

<400> 87

Gly Gly Gly Gly Ser Gly Gly Gly Gly Ser
1 5 10

<210> 88
<211> 10
<212> PRT
<213> Artificial Sequence

<220>
<223> Peptide linker (SG4)2

<400> 88

Ser Gly Gly Gly Gly Ser Gly Gly Gly Gly
1 5 10

<210> 89
<211> 15
<212> PRT
<213> Artificial Sequence

<220>
<223> Peptide linker (G4S)3

<400> 89

```
Gly Gly Gly Gly Ser Gly Gly Gly Gly Ser Gly Gly Gly Gly Ser
1               5                   10                  15
```

<210> 90
<211> 14
<212> PRT
<213> Artificial Sequence

<220>
<223> Peptide linker G4(SG4)2

<400> 90

```
Gly Gly Gly Gly Ser Gly Gly Gly Gly Ser Gly Gly Gly Gly
1               5                   10
```

<210> 91
<211> 20
<212> PRT
<213> Artificial Sequence

<220>
<223> Peptide linker (G4S)4

<400> 91

```
Gly Gly Gly Gly Ser Gly Gly Gly Gly Ser Gly Gly Gly Gly Ser Gly
1               5                   10                  15

Gly Gly Gly Ser
            20
```

<210> 92
<211> 10
<212> PRT
<213> Artificial Sequence

<220>
<223> Peptide linker GSPGSSSSGS

<400> 92

```
Gly Ser Pro Gly Ser Ser Ser Ser Gly Ser
1               5                   10
```

<210> 93
<211> 8
<212> PRT
<213> Artificial Sequence

<220>
<223> Peptide linker GSGSGSGS

<400> 93

Gly Ser Gly Ser Gly Ser Gly Ser
1 5

<210> 94
<211> 8
<212> PRT
<213> Artificial Sequence

<220>
<223> Peptide linker GSGSGNGS

<400> 94

Gly Ser Gly Ser Gly Asn Gly Ser
1 5

<210> 95
<211> 8
<212> PRT
<213> Artificial Sequence

<220>
<223> Peptide linker GGSGSGSG

<400> 95

Gly Gly Ser Gly Ser Gly Ser Gly
1 5

<210> 96
<211> 6
<212> PRT
<213> Artificial Sequence

<220>
<223> Peptide linker GGSGSG

<400> 96

Gly Gly Ser Gly Ser Gly
1 5

<210> 97
<211> 4
<212> PRT
<213> Artificial Sequence

<220>
<223> Peptide linker GGSG

<400> 97

Gly Gly Ser Gly
1

<210> 98
<211> 8
<212> PRT

<213> Artificial Sequence

<220>
<223> GGSGNGSG

<400> 98

Gly Gly Ser Gly Asn Gly Ser Gly
1 5

<210> 99
<211> 8
<212> PRT
<213> Artificial Sequence

<220>
<223> Peptide inker GGNGSGSG

<400> 99

Gly Gly Asn Gly Ser Gly Ser Gly
1 5

<210> 100
<211> 6
<212> PRT
<213> Artificial Sequence

<220>
<223> Peptide linker GGNGSG

<400> 100

Gly Gly Asn Gly Ser Gly
1 5

<210> 101
<211> 1806
<212> DNA
<213> Artificial Sequence

<220>
<223> nucleotide sequence of Fc hole dimeric 4-1BB ligand (71-248) chain

<400> 101

```
gacaaaactc acacatgccc accgtgccca gcacctgaag ctgcaggggg accgtcagtc       60
ttcctcttcc ccccaaaacc caaggacacc ctcatgatct cccggacccc tgaggtcaca      120
tgcgtggtgg tggacgtgag ccacgaagac cctgaggtca agttcaactg gtacgtggac      180
ggcgtggagg tgcataatgc caagacaaag ccgcgggagg agcagtacaa cagcacgtac      240
cgtgtggtca gcgtcctcac cgtcctgcac caggactggc tgaatggcaa ggagtacaag      300
tgcaaggtct ccaacaaagc cctcggcgcc cccatcgaga aaaccatctc caaagccaaa      360
gggcagcccc gagaaccaca ggtgtgcacc ctgcccccat cccgggatga gctgaccaag      420
```

aaccaggtca gcctctcgtg cgcagtcaaa ggcttctatc ccagcgacat cgccgtggag 480
tgggagagca atgggcagcc ggagaacaac tacaagacca cgcctcccgt gctggactcc 540
gacggctcct tcttcctcgt gagcaagctc accgtggaca agagcaggtg gcagcagggg 600
aacgtcttct catgctccgt gatgcatgag gctctgcaca accactacac gcagaagagc 660
ctctccctgt ctccgggtgg aggcggcgga agcggaggag gaggatccag agagggccct 720
gagctgagcc ctgatgatcc tgccggactg ctggacctgc ggcagggaat gtttgcccag 780
ctggtggccc agaacgtgct gctgatcgat ggccccctgt cctggtacag cgatcctgga 840
ctggctggcg tgtcactgac aggcggcctg agctacaaag aggacaccaa agaactggtg 900
gtggccaagg ccggcgtgta ctacgtgttc tttcagctgg aactgcggag agtggtggcc 960
ggcgaaggat ctggctctgt gtctctggcc ctgcatctgc agcctctgag atctgctgct 1020
ggcgccgctg ctctggcact gacagtggat ctgcctcctg ccagcagcga ggcccggaat 1080
agcgcatttg ggtttcaagg caggctgctg cacctgtctg ccggccagag gctgggagtg 1140
catctgcaca cagaggccag ggctagacac gcctggcagc tgacacaggg cgctacagtg 1200
ctgggcctgt tcagagtgac ccccgagatt ccagcaggcc tgggaggcgg cggatctggc 1260
ggcggaggat ctagagaagg acccgagctg tcccccgacg atcccgctgg gctgctggat 1320
ctgagacagg gcatgttcgc tcagctggtg gctcagaatg tgctgctgat tgacggacct 1380
ctgagctggt actccgaccc agggctggca ggggtgtccc tgactggggg actgtcctac 1440
aaagaagata caaaagaact ggtggtggct aaagctgggg tgtactatgt gttttttcag 1500
ctggaactga ggcgggtggt ggctggggag ggctcaggat ctgtgtccct ggctctgcat 1560
ctgcagccac tgcgctctgc agcaggggct gcagcactgg ccctgactgt ggacctgccc 1620
ccagcttctt ccgaggccag aaacagcgcc ttcgggttcc aaggacgcct gctgcatctg 1680
agcgccggac agcgcctggg agtgcatctg catactgaag ccagagcccg gcatgcttgg 1740
cagctgactc agggggcaac tgtgctggga ctgtttcgcg tgacacctga gatcccagcc 1800
gggctc 1806

<210> 102
<211> 1902
<212> DNA
<213> Artificial Sequence

<220>
<223> nucleotide sequence of anti-FAP(4B9) Fc knob monomeric 4-1BB (71-248) ligand

<400> 102

gaggtgcagc tgctcgaaag cggcggagga ctggtgcagc ctggcggcag cctgagactg 60

tcttgcgccg ccagcggctt caccttcagc agctacgcca tgagctgggt ccgccaggcc 120

```
cctggcaagg gactggaatg ggtgtccgcc atcatcggct ctggcgccag cacctactac      180
gccgacagcg tgaagggccg gttcaccatc agccgggaca acagcaagaa caccctgtac      240
ctgcagatga acagcctgcg ggccgaggac accgccgtgt actactgcgc caagggatgg      300
ttcggcggct tcaactactg gggacagggc accctggtca cagtgtccag cgctagcacc      360
aagggcccat cggtcttccc cctggcaccc tcctccaaga gcacctctgg gggcacagcg      420
gccctgggct gcctggtcaa ggactacttc cccgaaccgg tgacggtgtc gtggaactca      480
ggcgccctga ccagcggcgt gcacaccttc ccggctgtcc tacagtcctc aggactctac      540
tccctcagca gcgtggtgac cgtgccctcc agcagcttgg gcacccagac ctacatctgc      600
aacgtgaatc acaagcccag caacaccaag gtggacaaga aagttgagcc caaatcttgt      660
gacaaaactc acacatgccc accgtgccca gcacctgaag ctgcaggggg accgtcagtc      720
ttcctcttcc ccccaaaacc caaggacacc ctcatgatct cccggacccc tgaggtcaca      780
tgcgtggtgg tggacgtgag ccacgaagac cctgaggtca agttcaactg gtacgtggac      840
ggcgtggagg tgcataatgc caagacaaag ccgcgggagg agcagtacaa cagcacgtac      900
cgtgtggtca gcgtcctcac cgtcctgcac caggactggc tgaatggcaa ggagtacaag      960
tgcaaggtct ccaacaaagc cctcggcgcc cccatcgaga aaaccatctc caaagccaaa     1020
gggcagcccc gagaaccaca ggtgtacacc ctgcccccct gcagagatga gctgaccaag     1080
aaccaggtgt ccctgtggtg tctggtcaag ggcttctacc ccagcgatat cgccgtggag     1140
tgggagagca acggccagcc tgagaacaac tacaagacca ccccccctgt gctggacagc     1200
gacggcagct tcttcctgta ctccaaactg accgtggaca agagccggtg gcagcagggc     1260
aacgtgttca gctgcagcgt gatgcacgag gccctgcaca accactacac ccagaagtcc     1320
ctgagcctga gccccggcgg aggcggcgga agcggaggag gaggatccag agagggccct     1380
gagctgagcc ctgatgatcc tgccggactg ctggacctgc ggcagggaat gtttgcccag     1440
ctggtggccc agaacgtgct gctgatcgat ggccccctgt cctggtacag cgatcctgga     1500
ctggctggcg tgtcactgac aggcggcctg agctacaaag aggacaccaa agaactggtg     1560
gtggccaagg ccggcgtgta ctacgtgttc tttcagctgg aactgcggag agtggtggcc     1620
ggcgaaggat ctggctctgt gtctctggcc ctgcatctgc agcctctgag atctgctgct     1680
ggcgccgctg ctctggcact gacagtggat ctgcctcctg ccagcagcga ggcccggaat     1740
agcgcatttg ggtttcaagg caggctgctg cacctgtctg ccggccagag gctgggagtg     1800
catctgcaca cagaggccag ggctagacac gcctggcagc tgacacaggg cgctacagtg     1860
ctgggcctgt tcagagtgac ccccgagatt cctgccgggc tc                        1902
```

<210> 103

<211> 645
<212> DNA
<213> Artificial Sequence

<220>
<223> nucleotide sequence of anti-FAP(4B9) light chain

<400> 103

```
gagatcgtgc tgacccagtc ccccggcacc ctgtctctga gccctggcga gagagccacc     60
ctgtcctgca gagcctccca gtccgtgacc tcctcctacc tcgcctggta tcagcagaag    120
cccggccagg cccctcggct gctgatcaac gtgggcagtc ggagagccac cggcatccct    180
gaccggttct ccggctctgg ctccggcacc gacttcaccc tgaccatctc ccggctggaa    240
cccgaggact tcgccgtgta ctactgccag cagggcatca tgctgccccc cacctttggc    300
cagggcacca aggtggaaat caagcgtacg gtggctgcac catctgtctt catcttcccg    360
ccatctgatg agcagttgaa atctggaact gcctctgttg tgtgcctgct gaataacttc    420
tatcccagag aggccaaagt acagtggaag gtggataacg ccctccaatc gggtaactcc    480
caggagagtg tcacagagca ggacagcaag gacagcacct acagcctcag cagcaccctg    540
acgctgagca aagcagacta cgagaaacac aaagtctacg cctgcgaagt cacccatcag    600
ggcctgagct cgcccgtcac aaagagcttc aacaggggag agtgt                   645
```

<210> 104
<211> 602
<212> PRT
<213> Artificial Sequence

<220>
<223> Fc hole dimeric 4-1BB (71-248) ligand chain

<400> 104

```
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95
```

```
Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Gly Gly Gly Gly Ser Gly Gly Gly Gly Ser Arg Glu Gly Pro
225                 230                 235                 240

Glu Leu Ser Pro Asp Asp Pro Ala Gly Leu Leu Asp Leu Arg Gln Gly
                245                 250                 255

Met Phe Ala Gln Leu Val Ala Gln Asn Val Leu Leu Ile Asp Gly Pro
            260                 265                 270

Leu Ser Trp Tyr Ser Asp Pro Gly Leu Ala Gly Val Ser Leu Thr Gly
        275                 280                 285

Gly Leu Ser Tyr Lys Glu Asp Thr Lys Glu Leu Val Val Ala Lys Ala
    290                 295                 300

Gly Val Tyr Tyr Val Phe Phe Gln Leu Glu Leu Arg Arg Val Val Ala
305                 310                 315                 320

Gly Glu Gly Ser Gly Ser Val Ser Leu Ala Leu His Leu Gln Pro Leu
                325                 330                 335

Arg Ser Ala Ala Gly Ala Ala Ala Leu Ala Leu Thr Val Asp Leu Pro
            340                 345                 350
```

```
Pro Ala Ser Ser Glu Ala Arg Asn Ser Ala Phe Gly Phe Gln Gly Arg
        355                 360                 365

Leu Leu His Leu Ser Ala Gly Gln Arg Leu Gly Val His Leu His Thr
    370                 375                 380

Glu Ala Arg Ala Arg His Ala Trp Gln Leu Thr Gln Gly Ala Thr Val
385                 390                 395                 400

Leu Gly Leu Phe Arg Val Thr Pro Glu Ile Pro Ala Gly Leu Gly Gly
                405                 410                 415

Gly Gly Ser Gly Gly Gly Gly Ser Arg Glu Gly Pro Glu Leu Ser Pro
            420                 425                 430

Asp Asp Pro Ala Gly Leu Leu Asp Leu Arg Gln Gly Met Phe Ala Gln
        435                 440                 445

Leu Val Ala Gln Asn Val Leu Leu Ile Asp Gly Pro Leu Ser Trp Tyr
    450                 455                 460

Ser Asp Pro Gly Leu Ala Gly Val Ser Leu Thr Gly Gly Leu Ser Tyr
465                 470                 475                 480

Lys Glu Asp Thr Lys Glu Leu Val Val Ala Lys Ala Gly Val Tyr Tyr
                485                 490                 495

Val Phe Phe Gln Leu Glu Leu Arg Arg Val Val Ala Gly Glu Gly Ser
            500                 505                 510

Gly Ser Val Ser Leu Ala Leu His Leu Gln Pro Leu Arg Ser Ala Ala
        515                 520                 525

Gly Ala Ala Ala Leu Ala Leu Thr Val Asp Leu Pro Pro Ala Ser Ser
    530                 535                 540

Glu Ala Arg Asn Ser Ala Phe Gly Phe Gln Gly Arg Leu Leu His Leu
545                 550                 555                 560

Ser Ala Gly Gln Arg Leu Gly Val His Leu His Thr Glu Ala Arg Ala
                565                 570                 575

Arg His Ala Trp Gln Leu Thr Gln Gly Ala Thr Val Leu Gly Leu Phe
            580                 585                 590

Arg Val Thr Pro Glu Ile Pro Ala Gly Leu
```

595 600

<210> 105
<211> 634
<212> PRT
<213> Artificial Sequence

<220>
<223> anti-FAP(4B9) Fc knob monomeric 41-BB (71-248) ligand

<400> 105

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205
```

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Tyr Thr Leu Pro
340 345 350

Pro Cys Arg Asp Glu Leu Thr Lys Asn Gln Val Ser Leu Trp Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Arg Glu Gly Pro Glu Leu Ser Pro

450 455 460

Asp Asp Pro Ala Gly Leu Leu Asp Leu Arg Gln Gly Met Phe Ala Gln
465 470 475 480

Leu Val Ala Gln Asn Val Leu Leu Ile Asp Gly Pro Leu Ser Trp Tyr
485 490 495

Ser Asp Pro Gly Leu Ala Gly Val Ser Leu Thr Gly Gly Leu Ser Tyr
500 505 510

Lys Glu Asp Thr Lys Glu Leu Val Val Ala Lys Ala Gly Val Tyr Tyr
515 520 525

Val Phe Phe Gln Leu Glu Leu Arg Arg Val Val Ala Gly Glu Gly Ser
530 535 540

Gly Ser Val Ser Leu Ala Leu His Leu Gln Pro Leu Arg Ser Ala Ala
545 550 555 560

Gly Ala Ala Ala Leu Ala Leu Thr Val Asp Leu Pro Pro Ala Ser Ser
565 570 575

Glu Ala Arg Asn Ser Ala Phe Gly Phe Gln Gly Arg Leu Leu His Leu
580 585 590

Ser Ala Gly Gln Arg Leu Gly Val His Leu His Thr Glu Ala Arg Ala
595 600 605

Arg His Ala Trp Gln Leu Thr Gln Gly Ala Thr Val Leu Gly Leu Phe
610 615 620

Arg Val Thr Pro Glu Ile Pro Ala Gly Leu
625 630

<210> 106
<211> 215
<212> PRT
<213> Artificial Sequence

<220>
<223> anti-FAP(4B9) light chain

<400> 106

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

Tyr Leu Ala Trp Tyr Gln Gln Lys Pro Gly Gln Ala Pro Arg Leu Leu
        35                  40                  45

Ile Asn Val Gly Ser Arg Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Ile Met Leu Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 107
<211> 1212
<212> DNA
<213> Artificial Sequence

<220>
<223> nucleotide sequence of Fc hole monomeric 4-1BBL (71-248) chain

<400> 107

```
gacaaaactc acacatgccc accgtgccca gcacctgaag ctgcaggggg accgtcagtc      60
ttcctcttcc ccccaaaacc caaggacacc ctcatgatct cccggacccc tgaggtcaca     120
tgcgtggtgg tggacgtgag ccacgaagac cctgaggtca agttcaactg gtacgtggac     180
ggcgtggagg tgcataatgc caagacaaag ccgcgggagg agcagtacaa cagcacgtac     240
cgtgtggtca gcgtcctcac cgtcctgcac caggactggc tgaatggcaa ggagtacaag     300
tgcaaggtct ccaacaaagc cctcggcgcc cccatcgaga aaaccatctc caaagccaaa     360
gggcagcccc gagaaccaca ggtgtgcacc ctgcccccat cccgggatga gctgaccaag     420
aaccaggtca gcctctcgtg cgcagtcaaa ggcttctatc ccagcgacat cgccgtggag     480
tgggagagca atgggcagcc ggagaacaac tacaagacca cgcctcccgt gctggactcc     540
gacggctcct tcttcctcgt gagcaagctc accgtggaca agagcaggtg gcagcagggg     600
aacgtcttct catgctccgt gatgcatgag gctctgcaca accactacac gcagaagagc     660
ctctccctgt ctccgggtag agagggccct gagctgagcc ctgatgatcc tgccggactg     720
ctggacctgc ggcagggaat gtttgcccag ctggtggccc agaacgtgct gctgatcgat     780
ggcccccctgt cctggtacag cgatcctgga ctggctggcg tgtcactgac aggcggcctg     840
agctacaaag aggacaccaa agaactggtg gtggccaagg ccggcgtgta ctacgtgttc     900
tttcagctgg aactgcggag agtggtggcc ggcgaaggat ctggctctgt gtctctggcc     960
ctgcatctgc agcctctgag atctgctgct ggcgccgctg ctctggcact gacagtggat    1020
ctgcctcctg ccagcagcga ggcccggaat agcgcatttg ggtttcaagg caggctgctg    1080
cacctgtctg ccggccagag gctgggagtg catctgcaca cagaggccag ggctagacac    1140
gcctggcagc tgacacaggg cgctacagtg ctgggcctgt tcagagtgac ccccgagatt    1200
cctgccgggc tc                                                         1212
```

<210> 108
<211> 2436
<212> DNA
<213> Artificial Sequence

<220>
<223> nucleotide sequence of anti-FAP(4B9) Fc knob dimeric 4-1BB ligand (71-248)

<400> 108

gaggtgcagc tgctcgaaag cggcggagga ctggtgcagc ctggcggcag cctgagactg 60
tcttgcgccg ccagcggctt caccttcagc agctacgcca tgagctgggt ccgccaggcc 120
cctggcaagg gactggaatg ggtgtccgcc atcatcggct ctggcgccag cacctactac 180
gccgacagcg tgaagggccg gttcaccatc agccgggaca acagcaagaa caccctgtac 240
ctgcagatga acagcctgcg ggccgaggac accgccgtgt actactgcgc caagggatgg 300
ttcggcggct tcaactactg gggacagggc accctggtca cagtgtccag cgctagcacc 360
aagggcccat cggtcttccc cctggcaccc tcctccaaga gcacctctgg gggcacagcg 420

gccctgggct gcctggtcaa ggactacttc cccgaaccgg tgacggtgtc gtggaactca 480
ggcgccctga ccagcggcgt gcacaccttc ccggctgtcc tacagtcctc aggactctac 540
tccctcagca gcgtggtgac cgtgccctcc agcagcttgg gcacccagac ctacatctgc 600
aacgtgaatc acaagcccag caacaccaag gtggacaaga aagttgagcc caaatcttgt 660
gacaaaactc acacatgccc accgtgccca gcacctgaag ctgcaggggg accgtcagtc 720
ttcctcttcc ccccaaaacc caaggacacc ctcatgatct cccggacccc tgaggtcaca 780
tgcgtggtgg tggacgtgag ccacgaagac cctgaggtca agttcaactg gtacgtggac 840
ggcgtggagg tgcataatgc caagacaaag ccgcgggagg agcagtacaa cagcacgtac 900
cgtgtggtca gcgtcctcac cgtcctgcac caggactggc tgaatggcaa ggagtacaag 960
tgcaaggtct ccaacaaagc cctcggcgcc cccatcgaga aaaccatctc caaagccaaa 1020
gggcagcccc gagaaccaca ggtgtacacc ctgcccccct gcagagatga gctgaccaag 1080
aaccaggtgt ccctgtggtg tctggtcaag ggcttctacc ccagcgatat cgccgtggag 1140
tgggagagca acggccagcc tgagaacaac tacaagacca cccccccctgt gctggacagc 1200
gacggcagct tcttcctgta ctccaaactg accgtggaca agagccggtg gcagcagggc 1260
aacgtgttca gctgcagcgt gatgcacgag gccctgcaca accactacac ccagaagtcc 1320
ctgagcctga gccccggcag agagggccct gagctgagcc ctgatgatcc tgccggactg 1380
ctggacctgc ggcagggaat gtttgcccag ctggtggccc agaacgtgct gctgatcgat 1440
ggcccccctgt cctggtacag cgatcctgga ctggctggcg tgtcactgac aggcggcctg 1500
agctacaaag aggacaccaa agaactggtg gtggccaagg ccggcgtgta ctacgtgttc 1560
tttcagctgg aactgcggag agtggtggcc ggcgaaggat ctggctctgt gtctctggcc 1620
ctgcatctgc agcctctgag atctgctgct ggcgccgctg ctctggcact gacagtggat 1680
ctgcctcctg ccagcagcga ggcccggaat agcgcatttg ggtttcaagg caggctgctg 1740
cacctgtctg ccggccagag gctgggagtg catctgcaca cagaggccag ggctagacac 1800
gcctggcagc tgacacaggg cgctacagtg ctgggcctgt tcagagtgac ccccgagatt 1860
ccagcaggcc tgggaggcgg cggatctggc ggcggaggat ctagagaagg acccgagctg 1920
tcccccgacg atcccgctgg gctgctggat ctgagacagg gcatgttcgc tcagctggtg 1980
gctcagaatg tgctgctgat tgacggacct ctgagctggt actccgaccc agggctggca 2040
ggggtgtccc tgactggggg actgtcctac aaagaagata caaaagaact ggtggtggct 2100
aaagctgggg tgtactatgt gtttttttcag ctggaactga ggcgggtggt ggctggggag 2160
ggctcaggat ctgtgtccct ggctctgcat ctgcagccac tgcgctctgc agcaggggct 2220
gcagcactgg ccctgactgt ggacctgccc ccagcttctt ccgaggccag aaacagcgcc 2280
ttcgggttcc aaggacgcct gctgcatctg agcgccggac agcgcctggg agtgcatctg 2340

catactgaag ccagagcccg gcatgcttgg cagctgactc agggggcaac tgtgctggga 2400

ctgtttcgcg tgacacctga gatcccagcc gggctc 2436

<210> 109
<211> 404
<212> PRT
<213> Artificial Sequence

<220>
<223> Fc hole monomeric 4-1BB ligand (71-248) chain

<400> 109

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Gly Ala Pro Ile
100 105 110

Glu Lys Thr Ile Ser Lys Ala Lys Gly Gln Pro Arg Glu Pro Gln Val
115 120 125

Cys Thr Leu Pro Pro Ser Arg Asp Glu Leu Thr Lys Asn Gln Val Ser
130 135 140

Leu Ser Cys Ala Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Val Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Arg Glu Gly Pro Glu Leu Ser Pro Asp Asp Pro Ala Gly Leu
225 230 235 240

Leu Asp Leu Arg Gln Gly Met Phe Ala Gln Leu Val Ala Gln Asn Val
245 250 255

Leu Leu Ile Asp Gly Pro Leu Ser Trp Tyr Ser Asp Pro Gly Leu Ala
260 265 270

Gly Val Ser Leu Thr Gly Gly Leu Ser Tyr Lys Glu Asp Thr Lys Glu
275 280 285

Leu Val Val Ala Lys Ala Gly Val Tyr Tyr Val Phe Phe Gln Leu Glu
290 295 300

Leu Arg Arg Val Val Ala Gly Glu Gly Ser Gly Ser Val Ser Leu Ala
305 310 315 320

Leu His Leu Gln Pro Leu Arg Ser Ala Ala Gly Ala Ala Ala Leu Ala
325 330 335

Leu Thr Val Asp Leu Pro Pro Ala Ser Ser Glu Ala Arg Asn Ser Ala
340 345 350

Phe Gly Phe Gln Gly Arg Leu Leu His Leu Ser Ala Gly Gln Arg Leu
355 360 365

Gly Val His Leu His Thr Glu Ala Arg Ala Arg His Ala Trp Gln Leu
370 375 380

Thr Gln Gly Ala Thr Val Leu Gly Leu Phe Arg Val Thr Pro Glu Ile
385 390 395 400

Pro Ala Gly Leu

<210> 110
<211> 812
<212> PRT
<213> Artificial Sequence

<220>
<223> anti-FAP(4B9) Fc knob dimeric 4-1BB ligand (71-248)

<400> 110

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240
```

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Tyr Thr Leu Pro
340 345 350

Pro Cys Arg Asp Glu Leu Thr Lys Asn Gln Val Ser Leu Trp Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Arg Glu
435 440 445

Gly Pro Glu Leu Ser Pro Asp Asp Pro Ala Gly Leu Leu Asp Leu Arg
450 455 460

Gln Gly Met Phe Ala Gln Leu Val Ala Gln Asn Val Leu Leu Ile Asp
465 470 475 480

Gly Pro Leu Ser Trp Tyr Ser Asp Pro Gly Leu Ala Gly Val Ser Leu
485 490 495

Thr Gly Gly Leu Ser Tyr Lys Glu Asp Thr Lys Glu Leu Val Val Ala
500 505 510

Lys Ala Gly Val Tyr Tyr Val Phe Phe Gln Leu Glu Leu Arg Arg Val
515 520 525

Val Ala Gly Glu Gly Ser Gly Ser Val Ser Leu Ala Leu His Leu Gln
530 535 540

Pro Leu Arg Ser Ala Ala Gly Ala Ala Ala Leu Ala Leu Thr Val Asp
545 550 555 560

Leu Pro Pro Ala Ser Ser Glu Ala Arg Asn Ser Ala Phe Gly Phe Gln
565 570 575

Gly Arg Leu Leu His Leu Ser Ala Gly Gln Arg Leu Gly Val His Leu
580 585 590

His Thr Glu Ala Arg Ala Arg His Ala Trp Gln Leu Thr Gln Gly Ala
595 600 605

Thr Val Leu Gly Leu Phe Arg Val Thr Pro Glu Ile Pro Ala Gly Leu
610 615 620

Gly Gly Gly Gly Ser Gly Gly Gly Gly Ser Arg Glu Gly Pro Glu Leu
625 630 635 640

Ser Pro Asp Asp Pro Ala Gly Leu Leu Asp Leu Arg Gln Gly Met Phe
645 650 655

Ala Gln Leu Val Ala Gln Asn Val Leu Leu Ile Asp Gly Pro Leu Ser
660 665 670

Trp Tyr Ser Asp Pro Gly Leu Ala Gly Val Ser Leu Thr Gly Gly Leu
675 680 685

Ser Tyr Lys Glu Asp Thr Lys Glu Leu Val Val Ala Lys Ala Gly Val
690 695 700

Tyr Tyr Val Phe Phe Gln Leu Glu Leu Arg Arg Val Val Ala Gly Glu
705 710 715 720

Gly Ser Gly Ser Val Ser Leu Ala Leu His Leu Gln Pro Leu Arg Ser
725 730 735

Ala Ala Gly Ala Ala Ala Leu Ala Leu Thr Val Asp Leu Pro Pro Ala

740 745 750

Ser Ser Glu Ala Arg Asn Ser Ala Phe Gly Phe Gln Gly Arg Leu Leu
755 760 765

His Leu Ser Ala Gly Gln Arg Leu Gly Val His Leu His Thr Glu Ala
770 775 780

Arg Ala Arg His Ala Trp Gln Leu Thr Gln Gly Ala Thr Val Leu Gly
785 790 795 800

Leu Phe Arg Val Thr Pro Glu Ile Pro Ala Gly Leu
805 810

<210> 111
<211> 1899
<212> DNA
<213> Artificial Sequence

<220>
<223> nucleotide sequence of anti-FAP(28H1) Fc knob monomeric 4-1BB ligand (71-248)

<400> 111

```
gaagtgcagc tgctggaatc cggcggaggc ctggtgcagc ctggcggatc tctgagactg      60
tcctgcgccg cctccggctt caccttctcc tcccacgcca tgtcctgggt ccgacaggct     120
cctggcaaag gcctggaatg ggtgtccgcc atctgggcct ccggcgagca gtactacgcc     180
gactctgtga agggccggtt caccatctcc cgggacaact ccaagaacac cctgtacctg     240
cagatgaact ccctgcgggc cgaggacacc gccgtgtact actgtgccaa gggctggctg     300
ggcaacttcg actactgggg acagggcacc ctggtcaccg tgtccagcgc tagcaccaag     360
ggcccatcgg tcttcccct ggcaccctcc tccaagagca cctctggggg cacagcggcc      420
ctgggctgcc tggtcaagga ctacttcccc gaaccggtga cggtgtcgtg gaactcaggc     480
gccctgacca gcggcgtgca caccttcccg gctgtcctac agtcctcagg actctactcc     540
ctcagcagcg tggtgaccgt gccctccagc agcttgggca cccagaccta catctgcaac     600
gtgaatcaca agcccagcaa caccaaggtg gacaagaaag ttgagcccaa atcttgtgac     660
aaaactcaca catgcccacc gtgcccagca cctgaagctg caggggggacc gtcagtcttc    720
ctcttccccc caaaacccaa ggacaccctc atgatctccc ggacccctga ggtcacatgc     780
gtggtggtgg acgtgagcca cgaagaccct gaggtcaagt tcaactggta cgtggacggc     840
gtggaggtgc ataatgccaa gacaaagccg cgggaggagc agtacaacag cacgtaccgt     900
gtggtcagcg tcctcaccgt cctgcaccag gactggctga atggcaagga gtacaagtgc     960
aaggtctcca acaaagccct cggcgccccc atcgagaaaa ccatctccaa agccaaaggg    1020
```

cagccccgag aaccacaggt gtacaccctg ccccctgca gagatgagct gaccaagaac 1080

caggtgtccc tgtggtgtct ggtcaagggc ttctacccca gcgatatcgc cgtggagtgg 1140

gagagcaacg gccagcctga gaacaactac aagaccaccc cccctgtgct ggacagcgac 1200

ggcagcttct tcctgtactc caaactgacc gtggacaaga gccggtggca gcagggcaac 1260

gtgttcagct gcagcgtgat gcacgaggcc ctgcacaacc actacaccca gaagtccctg 1320

agcctgagcc ccggcggagg cggcggaagc ggaggaggag gatccagaga gggccctgag 1380

ctgagccctg atgatcctgc cggactgctg gacctgcggc agggaatgtt tgcccagctg 1440

gtggcccaga acgtgctgct gatcgatggc cccctgtcct ggtacagcga tcctggactg 1500

gctggcgtgt cactgacagg cggcctgagc tacaaagagg acaccaaaga actggtggtg 1560

gccaaggccg gcgtgtacta cgtgttcttt cagctggaac tgcggagagt ggtggccggc 1620

gaaggatctg gctctgtgtc tctggccctg catctgcagc ctctgagatc tgctgctggc 1680

gccgctgctc tggcactgac agtggatctg cctcctgcca gcagcgaggc ccggaatagc 1740

gcatttgggt ttcaaggcag gctgctgcac ctgtctgccg gccagaggct gggagtgcat 1800

ctgcacacag aggccagggc tagacacgcc tggcagctga cacagggcgc tacagtgctg 1860

ggcctgttca gagtgacccc cgagattcct gccgggctc 1899

<210> 112
<211> 645
<212> DNA
<213> Artificial Sequence

<220>
<223> nucleotide sequence of anti-FAP(28H1) light chain

<400> 112

```
gagatcgtgc tgacccagtc ccccggcacc ctgtctctga gccctggcga gagagccacc      60
ctgtcctgca gagcctccca gtccgtgtcc cggtcctacc tcgcctggta tcagcagaag     120
cccggccagg cccctcggct gctgatcatc ggcgcctcta ccagagccac cggcatccct     180
gaccggttct ccggctctgg ctccggcacc gacttcaccc tgaccatctc ccggctggaa     240
cccgaggact tcgccgtgta ctactgccag cagggccagg tcatccctcc cacctttggc     300
cagggcacca aggtggaaat caagcgtacg gtggctgcac catctgtctt catcttcccg     360
ccatctgatg agcagttgaa atctggaact gcctctgttg tgtgcctgct gaataacttc     420
tatcccagag aggccaaagt acagtggaag gtggataacg ccctccaatc gggtaactcc     480
caggagagtg tcacagagca ggacagcaag gacagcacct acagcctcag cagcaccctg     540
acgctgagca aagcagacta cgagaaacac aaagtctacg cctgcgaagt cacccatcag     600
ggcctgagct cgcccgtcac aaagagcttc aacaggggag agtgt                     645
```

<210> 113
<211> 633
<212> PRT
<213> Artificial Sequence

<220>
<223> anti-FAP(28H1) Fc knob monomeric 4-1BB ligand (71-248)

<400> 113

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
```

210 215 220

Cys Pro Pro Cys Pro Ala Pro Glu Ala Ala Gly Gly Pro Ser Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Asn Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Gly Ala Pro Ile Glu Lys Thr Ile Ser
325 330 335

Lys Ala Lys Gly Gln Pro Arg Glu Pro Gln Val Tyr Thr Leu Pro Pro
340 345 350

Cys Arg Asp Glu Leu Thr Lys Asn Gln Val Ser Leu Trp Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Gly Gly Gly
435 440 445

Gly Ser Gly Gly Gly Gly Ser Arg Glu Gly Pro Glu Leu Ser Pro Asp
450 455 460

Asp Pro Ala Gly Leu Leu Asp Leu Arg Gln Gly Met Phe Ala Gln Leu
465 470 475 480

Val Ala Gln Asn Val Leu Leu Ile Asp Gly Pro Leu Ser Trp Tyr Ser
485 490 495

Asp Pro Gly Leu Ala Gly Val Ser Leu Thr Gly Gly Leu Ser Tyr Lys
500 505 510

Glu Asp Thr Lys Glu Leu Val Val Ala Lys Ala Gly Val Tyr Tyr Val
515 520 525

Phe Phe Gln Leu Glu Leu Arg Arg Val Val Ala Gly Glu Gly Ser Gly
530 535 540

Ser Val Ser Leu Ala Leu His Leu Gln Pro Leu Arg Ser Ala Ala Gly
545 550 555 560

Ala Ala Ala Leu Ala Leu Thr Val Asp Leu Pro Pro Ala Ser Ser Glu
565 570 575

Ala Arg Asn Ser Ala Phe Gly Phe Gln Gly Arg Leu Leu His Leu Ser
580 585 590

Ala Gly Gln Arg Leu Gly Val His Leu His Thr Glu Ala Arg Ala Arg
595 600 605

His Ala Trp Gln Leu Thr Gln Gly Ala Thr Val Leu Gly Leu Phe Arg
610 615 620

Val Thr Pro Glu Ile Pro Ala Gly Leu
625 630

<210> 114
<211> 215
<212> PRT
<213> Artificial Sequence

<220>
<223> anti-FAP(28H1) light chain

<400> 114

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 115
<211> 2433
<212> DNA
<213> Artificial Sequence

<220>
<223> nucleotide sequence of anti-FAP(28H1) Fc knob dimeric 4-1BB ligand (71-248)

<400> 115

```
gaagtgcagc tgctggaatc cggcggaggc ctggtgcagc ctggcggatc tctgagactg      60
tcctgcgccg cctccggctt caccttctcc tcccacgcca tgtcctgggt ccgacaggct     120
cctggcaaag gcctggaatg ggtgtccgcc atctgggcct ccggcgagca gtactacgcc     180
```

gactctgtga agggccggtt caccatctcc cgggacaact ccaagaacac cctgtacctg 240
cagatgaact ccctgcgggc cgaggacacc gccgtgtact actgtgccaa gggctggctg 300
ggcaacttcg actactgggg acagggcacc ctggtcaccg tgtccagcgc tagcaccaag 360
ggcccatcgg tcttccccct ggcaccctcc tccaagagca cctctggggg cacagcggcc 420
ctgggctgcc tggtcaagga ctacttcccc gaaccggtga cggtgtcgtg gaactcaggc 480
gccctgacca gcggcgtgca caccttcccg gctgtcctac agtcctcagg actctactcc 540
ctcagcagcg tggtgaccgt gccctccagc agcttgggca cccagaccta catctgcaac 600
gtgaatcaca agcccagcaa caccaaggtg gacaagaaag ttgagcccaa atcttgtgac 660
aaaactcaca catgcccacc gtgcccagca cctgaagctg caggggggacc gtcagtcttc 720
ctcttccccc caaaacccaa ggacaccctc atgatctccc ggacccctga ggtcacatgc 780
gtggtggtgg acgtgagcca cgaagaccct gaggtcaagt tcaactggta cgtggacggc 840
gtggaggtgc ataatgccaa gacaaagccg cgggaggagc agtacaacag cacgtaccgt 900
gtggtcagcg tcctcaccgt cctgcaccag gactggctga atggcaagga gtacaagtgc 960
aaggtctcca acaaagccct cggcgccccc atcgagaaaa ccatctccaa agccaaaggg 1020
cagccccgag aaccacaggt gtacaccctg ccccctgca gagatgagct gaccaagaac 1080
caggtgtccc tgtggtgtct ggtcaagggc ttctacccca gcgatatcgc cgtggagtgg 1140
gagagcaacg gccagcctga gaacaactac aagaccaccc cccctgtgct ggacagcgac 1200
ggcagcttct tcctgtactc caaactgacc gtggacaaga gccggtggca gcagggcaac 1260
gtgttcagct gcagcgtgat gcacgaggcc ctgcacaacc actacaccca gaagtccctg 1320
agcctgagcc ccggcagaga gggccctgag ctgagccctg atgatcctgc cggactgctg 1380
gacctgcggc agggaatgtt tgcccagctg gtggcccaga acgtgctgct gatcgatggc 1440
cccctgtcct ggtacagcga tcctggactg gctggcgtgt cactgacagg cggcctgagc 1500
tacaaagagg acaccaaaga actggtggtg gccaaggccg gcgtgtacta cgtgttcttt 1560
cagctggaac tgcggagagt ggtggccggc gaaggatctg gctctgtgtc tctggccctg 1620
catctgcagc ctctgagatc tgctgctggc gccgctgctc tggcactgac agtggatctg 1680
cctcctgcca gcagcgaggc ccggaatagc gcatttgggt ttcaaggcag gctgctgcac 1740
ctgtctgccg gccagaggct gggagtgcat ctgcacacag aggccagggc tagacacgcc 1800
tggcagctga cacagggcgc tacagtgctg ggcctgttca gagtgacccc cgagattcca 1860
gcaggcctgg gaggcggcgg atctggcggc ggaggatcta gagaaggacc cgagctgtcc 1920
cccgacgatc ccgctgggct gctggatctg agacagggca tgttcgctca gctggtggct 1980
cagaatgtgc tgctgattga cggacctctg agctggtact ccgacccagg gctggcaggg 2040
gtgtccctga ctgggggact gtcctacaaa gaagatacaa aagaactggt ggtggctaaa 2100

```
gctggggtgt actatgtgtt ttttcagctg gaactgaggc gggtggtggc tggggagggc     2160
tcaggatctg tgtccctggc tctgcatctg cagccactgc gctctgcagc aggggctgca     2220
gcactggccc tgactgtgga cctgcccccca gcttcttccg aggccagaaa cagcgccttc     2280
gggttccaag gacgcctgct gcatctgagc gccggacagc gcctgggagt gcatctgcat     2340
actgaagcca gagcccggca tgcttggcag ctgactcagg gggcaactgt gctgggactg     2400
tttcgcgtga cacctgagat cccagccggg ctc                                  2433
```

<210> 116
<211> 811
<212> PRT
<213> Artificial Sequence

<220>
<223> anti-FAP(28H1) Fc knob dimeric 4-1BB ligand (71-248)

<400> 116

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160
```

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Ala Ala Gly Gly Pro Ser Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Asn Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Gly Ala Pro Ile Glu Lys Thr Ile Ser
325 330 335

Lys Ala Lys Gly Gln Pro Arg Glu Pro Gln Val Tyr Thr Leu Pro Pro
340 345 350

Cys Arg Asp Glu Leu Thr Lys Asn Gln Val Ser Leu Trp Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Arg Glu Gly
435 440 445

Pro Glu Leu Ser Pro Asp Asp Pro Ala Gly Leu Leu Asp Leu Arg Gln
450 455 460

Gly Met Phe Ala Gln Leu Val Ala Gln Asn Val Leu Leu Ile Asp Gly
465 470 475 480

Pro Leu Ser Trp Tyr Ser Asp Pro Gly Leu Ala Gly Val Ser Leu Thr
485 490 495

Gly Gly Leu Ser Tyr Lys Glu Asp Thr Lys Glu Leu Val Val Ala Lys
500 505 510

Ala Gly Val Tyr Tyr Val Phe Phe Gln Leu Glu Leu Arg Arg Val Val
515 520 525

Ala Gly Glu Gly Ser Gly Ser Val Ser Leu Ala Leu His Leu Gln Pro
530 535 540

Leu Arg Ser Ala Ala Gly Ala Ala Ala Leu Ala Leu Thr Val Asp Leu
545 550 555 560

Pro Pro Ala Ser Ser Glu Ala Arg Asn Ser Ala Phe Gly Phe Gln Gly
565 570 575

Arg Leu Leu His Leu Ser Ala Gly Gln Arg Leu Gly Val His Leu His
580 585 590

Thr Glu Ala Arg Ala Arg His Ala Trp Gln Leu Thr Gln Gly Ala Thr
595 600 605

Val Leu Gly Leu Phe Arg Val Thr Pro Glu Ile Pro Ala Gly Leu Gly
610 615 620

Gly Gly Gly Ser Gly Gly Gly Gly Ser Arg Glu Gly Pro Glu Leu Ser
625 630 635 640

Pro Asp Asp Pro Ala Gly Leu Leu Asp Leu Arg Gln Gly Met Phe Ala
645 650 655

Gln Leu Val Ala Gln Asn Val Leu Leu Ile Asp Gly Pro Leu Ser Trp

```
                660                 665                 670

Tyr Ser Asp Pro Gly Leu Ala Gly Val Ser Leu Thr Gly Gly Leu Ser
        675                 680                 685

Tyr Lys Glu Asp Thr Lys Glu Leu Val Val Ala Lys Ala Gly Val Tyr
    690                 695                 700

Tyr Val Phe Phe Gln Leu Glu Leu Arg Arg Val Val Ala Gly Glu Gly
705                 710                 715                 720

Ser Gly Ser Val Ser Leu Ala Leu His Leu Gln Pro Leu Arg Ser Ala
                725                 730                 735

Ala Gly Ala Ala Ala Leu Ala Leu Thr Val Asp Leu Pro Pro Ala Ser
            740                 745                 750

Ser Glu Ala Arg Asn Ser Ala Phe Gly Phe Gln Gly Arg Leu Leu His
        755                 760                 765

Leu Ser Ala Gly Gln Arg Leu Gly Val His Leu His Thr Glu Ala Arg
    770                 775                 780

Ala Arg His Ala Trp Gln Leu Thr Gln Gly Ala Thr Val Leu Gly Leu
785                 790                 795                 800

Phe Arg Val Thr Pro Glu Ile Pro Ala Gly Leu
                805                 810
```

<210> 117
<211> 1914
<212> DNA
<213> Artificial Sequence

<220>
<223> nucleotide sequence of anti-CD19(8B8-018) Fc knob monomeric 4-1BB (71-248) ligand

<400> 117

```
caggtccagc tggtgcagtc cggcgccgag gtcaagaaac ccggggcttc tgtgaaggtt      60
tcatgcaagg caagcggata caccttcacc gactatatca tgcattgggt caggcaggcc     120
cctggccaag gtctcgaatg gatgggctac attaacccat ataatgatgg ctccaaatac     180
accgagaagt ttcagggaag agtcactatg acatctgaca ccagtatcag cactgcttac     240
atggagctgt cccgccttcg gtctgatgac accgcagtgt attactgtgc caggggcaca     300
tattactacg gctcagctct gttcgactat tgggggcagg gaaccacagt aaccgtgagc     360
tccgctagca ccaagggccc atcggtcttc cccctggcac cctcctccaa gagcacctct     420
```

gggggcacag cggccctggg ctgcctggtc aaggactact tccccgaacc ggtgacggtg 480

tcgtggaact caggcgccct gaccagcggc gtgcacacct tcccggctgt cctacagtcc 540

tcaggactct actccctcag cagcgtggtg accgtgccct ccagcagctt gggcacccag 600

acctacatct gcaacgtgaa tcacaagccc agcaacacca aggtggacaa gaaagttgag 660

cccaaatctt gtgacaaaac tcacacatgc ccaccgtgcc cagcacctga agctgcaggg 720

ggaccgtcag tcttcctctt ccccccaaaa cccaaggaca ccctcatgat ctcccggacc 780

cctgaggtca catgcgtggt ggtggacgtg agccacgaag accctgaggt caagttcaac 840

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtaca ccctgccccc ctgcagagat 1080

gagctgacca agaaccaggt gtccctgtgg tgtctggtca agggcttcta ccccagcgat 1140

atcgccgtgg agtgggagag caacggccag cctgagaaca actacaagac cacccccccct 1200

gtgctggaca gcgacggcag cttcttcctg tactccaaac tgaccgtgga caagagccgg 1260

tggcagcagg gcaacgtgtt cagctgcagc gtgatgcacg aggccctgca caaccactac 1320

acccagaagt ccctgagcct gagccccggc ggaggcggcg gaagcggagg aggaggatcc 1380

agagagggcc ctgagctgag ccctgatgat cctgccggac tgctggacct gcggcaggga 1440

atgtttgccc agctggtggc ccagaacgtg ctgctgatcg atggccccct gtcctggtac 1500

agcgatcctg gactggctgg cgtgtcactg acaggcggcc tgagctacaa agaggacacc 1560

aaagaactgg tggtggccaa ggccggcgtg tactacgtgt tctttcagct ggaactgcgg 1620

agagtggtgg ccggcgaagg atctggctct gtgtctctgg ccctgcatct gcagcctctg 1680

agatctgctg ctggcgccgc tgctctggca ctgacagtgg atctgcctcc tgccagcagc 1740

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttcctgccgg gctc 1914

<210> 118
<211> 657
<212> DNA
<213> Artificial Sequence

<220>
<223> nucleotide sequence of anti-CD19(8B8-018) light chain

<400> 118

```
gacatcgtca tgacccagac acccctgtcc ctctctgtga cccctggcca gccagcctca        60

attagctgca agtcctctca aagtctggag aaccccaatg ggaacactta ccttaattgg       120

tatctgcaga aacccggaca atcccctcaa ctcctgatct acagggtctc taagagattc       180

tcaggcgtgc cagatcgctt tagcggttcc gggtctggca cagacttcac cttgaagatt       240

agtcgggttg aagctgagga tgtgggagtc tattactgtc tgcagctcac tcatgtgccc       300

tacacctttg gtcagggcac aaaactggag atcaagcgga ccgtggccgc tccctccgtg       360

ttcatcttcc caccctccga cgagcagctg aagtccggca ccgccagcgt ggtgtgcctg       420

ctgaacaact tctacccccg cgaggccaag gtgcagtgga aggtggacaa cgccctgcag       480

tccggcaact cccaggaatc cgtgaccgag caggactcca aggacagcac ctactccctg       540

tcctccaccc tgaccctgtc caaggccgac tacgagaagc acaaggtgta cgcctgcgaa       600

gtgacccacc agggcctgtc cagccccgtg accaagtcct tcaaccgggg cgagtgc          657
```

<210> 119
<211> 638
<212> PRT
<213> Artificial Sequence

<220>
<223> anti-CD19(8B8-018) Fc knob monomeric 4-1-BB (71-248) ligand

<400> 119

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala

130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Ala Ala Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Tyr Thr Leu Pro Pro Cys Arg Asp Glu Leu Thr Lys Asn Gln Val Ser
355 360 365

Leu Trp Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly
465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu
625 630 635

<210> 120
<211> 219
<212> PRT
<213> Artificial Sequence

<220>
<223> anti-CD19(8B8-018) light chain

<400> 120

```
Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Asn Pro
            20                  25                  30

Asn Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Thr His Val Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205
```

```
Pro Val Thr Lys Ser Phe Asn Arg Gly Glu Cys
    210                 215
```

<210> 121
<211> 2448
<212> DNA
<213> Artificial Sequence

<220>
<223> nucleotide sequence of anti-CD19(8B8-018) Fc knob dimeric 4-1BB (71-248) ligand

<400> 121

caggtccagc tggtgcagtc cggcgccgag gtcaagaaac ccggggcttc tgtgaaggtt 60
tcatgcaagg caagcggata caccttcacc gactatatca tgcattgggt caggcaggcc 120
cctggccaag gtctcgaatg gatgggctac attaacccat ataatgatgg ctccaaatac 180
accgagaagt ttcagggaag agtcactatg acatctgaca ccagtatcag cactgcttac 240
atggagctgt cccgccttcg gtctgatgac accgcagtgt attactgtgc caggggcaca 300
tattactacg gctcagctct gttcgactat tggggggcagg gaaccacagt aaccgtgagc 360
tccgctagca ccaagggccc atcggtcttc cccctggcac cctcctccaa gagcacctct 420
gggggcacag cggccctggg ctgcctggtc aaggactact tccccgaacc ggtgacggtg 480
tcgtggaact caggcgccct gaccagcggc gtgcacacct tcccggctgt cctacagtcc 540
tcaggactct actccctcag cagcgtggtg accgtgccct ccagcagctt gggcacccag 600
acctacatct gcaacgtgaa tcacaagccc agcaacacca aggtggacaa gaaagttgag 660
cccaaatctt gtgacaaaac tcacacatgc ccaccgtgcc cagcacctga agctgcaggg 720
ggaccgtcag tcttcctctt ccccccaaaa cccaaggaca ccctcatgat ctcccggacc 780
cctgaggtca catgcgtggt ggtggacgtg agccacgaag accctgaggt caagttcaac 840
tggtacgtgg acggcgtgga ggtgcataat gccaagacaa agccgcggga ggagcagtac 900
aacagcacgt accgtgtggt cagcgtcctc accgtcctgc accaggactg gctgaatggc 960
aaggagtaca agtgcaaggt ctccaacaaa gccctcggcg cccccatcga gaaaaccatc 1020
tccaaagcca aagggcagcc ccgagaacca caggtgtaca ccctgccccc ctgcagagat 1080
gagctgacca agaaccaggt gtccctgtgg tgtctggtca agggcttcta ccccagcgat 1140
atcgccgtgg agtgggagag caacggccag cctgagaaca actacaagac caccccccct 1200
gtgctggaca gcgacggcag cttcttcctg tactccaaac tgaccgtgga caagagccgg 1260
tggcagcagg gcaacgtgtt cagctgcagc gtgatgcacg aggccctgca caaccactac 1320
acccagaagt ccctgagcct gagccccggc agagagggcc ctgagctgag ccctgatgat 1380
cctgccggac tgctggacct gcggcaggga atgtttgccc agctggtggc ccagaacgtg 1440
ctgctgatcg atggccccct gtcctggtac agcgatcctg gactggctgg cgtgtcactg 1500

```
acaggcggcc tgagctacaa agaggacacc aaagaactgg tggtggccaa ggccggcgtg     1560
tactacgtgt tctttcagct ggaactgcgg agagtggtgg ccggcgaagg atctggctct     1620
gtgtctctgg ccctgcatct gcagcctctg agatctgctg ctggcgccgc tgctctggca     1680
ctgacagtgg atctgcctcc tgccagcagc gaggcccgga atagcgcatt tgggtttcaa     1740
ggcaggctgc tgcacctgtc tgccggccag aggctgggag tgcatctgca cacagaggcc     1800
agggctagac acgcctggca gctgacacag ggcgctacag tgctgggcct gttcagagtg     1860
acccccgaga ttccagcagg cctgggaggc ggcggatctg gcggcggagg atctagagaa     1920
ggacccgagc tgtcccccga cgatcccgct gggctgctgg atctgagaca gggcatgttc     1980
gctcagctgg tggctcagaa tgtgctgctg attgacggac ctctgagctg gtactccgac     2040
ccagggctgg caggggtgtc cctgactggg ggactgtcct acaaagaaga tacaaaagaa     2100
ctggtggtgg ctaaagctgg ggtgtactat gtgtttttc agctggaact gaggcgggtg     2160
gtggctgggg agggctcagg atctgtgtcc ctggctctgc atctgcagcc actgcgctct     2220
gcagcagggg ctgcagcact ggccctgact gtggacctgc cccagcttc ttccgaggcc     2280
agaaacagcg ccttcgggtt ccaaggacgc ctgctgcatc tgagcgccgg acagcgcctg     2340
ggagtgcatc tgcatactga agccagagcc cggcatgctt ggcagctgac tcagggggca     2400
actgtgctgg gactgtttcg cgtgacacct gagatcccag ccgggctc                  2448
```

<210> 122
<211> 816
<212> PRT
<213> Artificial Sequence

<220>
<223> anti-CD19(8B8-018) Fc knob dimeric 4-1BB (71-248) ligand

<400> 122

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80
```

```
Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335
```

Glu Lys Thr Ile Ser Lys Ala Lys Gly Gln Pro Arg Glu Pro Gln Val
340 345 350

Tyr Thr Leu Pro Pro Cys Arg Asp Glu Leu Thr Lys Asn Gln Val Ser
355 360 365

Leu Trp Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Arg Glu Gly Pro Glu Leu Ser Pro Asp Asp Pro Ala Gly Leu
450 455 460

Leu Asp Leu Arg Gln Gly Met Phe Ala Gln Leu Val Ala Gln Asn Val
465 470 475 480

Leu Leu Ile Asp Gly Pro Leu Ser Trp Tyr Ser Asp Pro Gly Leu Ala
485 490 495

Gly Val Ser Leu Thr Gly Gly Leu Ser Tyr Lys Glu Asp Thr Lys Glu
500 505 510

Leu Val Val Ala Lys Ala Gly Val Tyr Tyr Val Phe Phe Gln Leu Glu
515 520 525

Leu Arg Arg Val Val Ala Gly Glu Gly Ser Gly Ser Val Ser Leu Ala
530 535 540

Leu His Leu Gln Pro Leu Arg Ser Ala Ala Gly Ala Ala Ala Leu Ala
545 550 555 560

Leu Thr Val Asp Leu Pro Pro Ala Ser Ser Glu Ala Arg Asn Ser Ala
565 570 575

Phe Gly Phe Gln Gly Arg Leu Leu His Leu Ser Ala Gly Gln Arg Leu

580 585 590

Gly Val His Leu His Thr Glu Ala Arg Ala Arg His Ala Trp Gln Leu
595 600 605

Thr Gln Gly Ala Thr Val Leu Gly Leu Phe Arg Val Thr Pro Glu Ile
610 615 620

Pro Ala Gly Leu Gly Gly Gly Gly Ser Gly Gly Gly Gly Ser Arg Glu
625 630 635 640

Gly Pro Glu Leu Ser Pro Asp Asp Pro Ala Gly Leu Leu Asp Leu Arg
645 650 655

Gln Gly Met Phe Ala Gln Leu Val Ala Gln Asn Val Leu Leu Ile Asp
660 665 670

Gly Pro Leu Ser Trp Tyr Ser Asp Pro Gly Leu Ala Gly Val Ser Leu
675 680 685

Thr Gly Gly Leu Ser Tyr Lys Glu Asp Thr Lys Glu Leu Val Val Ala
690 695 700

Lys Ala Gly Val Tyr Tyr Val Phe Phe Gln Leu Glu Leu Arg Arg Val
705 710 715 720

Val Ala Gly Glu Gly Ser Gly Ser Val Ser Leu Ala Leu His Leu Gln
725 730 735

Pro Leu Arg Ser Ala Ala Gly Ala Ala Ala Leu Ala Leu Thr Val Asp
740 745 750

Leu Pro Pro Ala Ser Ser Glu Ala Arg Asn Ser Ala Phe Gly Phe Gln
755 760 765

Gly Arg Leu Leu His Leu Ser Ala Gly Gln Arg Leu Gly Val His Leu
770 775 780

His Thr Glu Ala Arg Ala Arg His Ala Trp Gln Leu Thr Gln Gly Ala
785 790 795 800

Thr Val Leu Gly Leu Phe Arg Val Thr Pro Glu Ile Pro Ala Gly Leu
805 810 815

<210> 123
<211> 1914
<212> DNA

<213> Artificial Sequence

<220>
<223> nucleotide sequence of anti-CD19(8B8-2B11) Fc knob monomeric 4-1BB (71-248) ligand

<400> 123

```
caggtgcaat tggttcaatc tggtgctgaa gtaaaaaaac cgggcgcttc cgttaaagtg      60
agctgcaaag catctggtta caccttcact gactatatca tgcactgggt tcgtcaggcc     120
ccgggccagg gtctggagtg gatgggctac attaacccat acaacgacgg ttccaaatat     180
accgagaaat tccagggccg cgtcacgatg accagcgaca cttctatctc caccgcgtac     240
atggaactgt ctagactgcg ttctgacgac accgctgttt actattgtgc acgcggtacc     300
tactactacg gtccacagct gtttgattac tggggccaag gtaccacggt gaccgtaagc     360
tctgctagca ccaagggccc atcggtcttc cccctggcac cctcctccaa gagcacctct     420
gggggcacag cggccctggg ctgcctggtc aaggactact tccccgaacc ggtgacggtg     480
tcgtggaact caggcgccct gaccagcggc gtgcacacct tcccggctgt cctacagtcc     540
tcaggactct actccctcag cagcgtggtg accgtgccct ccagcagctt gggcacccag     600
acctacatct gcaacgtgaa tcacaagccc agcaacacca aggtggacaa gaaagttgag     660
cccaaatctt gtgacaaaac tcacacatgc ccaccgtgcc cagcacctga agctgcaggg     720
ggaccgtcag tcttcctctt ccccccaaaa cccaaggaca ccctcatgat ctcccggacc     780
cctgaggtca catgcgtggt ggtggacgtg agccacgaag accctgaggt caagttcaac     840
tggtacgtgg acggcgtgga ggtgcataat gccaagacaa agccgcggga ggagcagtac     900
aacagcacgt accgtgtggt cagcgtcctc accgtcctgc accaggactg gctgaatggc     960
aaggagtaca agtgcaaggt ctccaacaaa gccctcggcg cccccatcga gaaaaccatc    1020
tccaaagcca aagggcagcc ccgagaacca caggtgtaca ccctgccccc ctgcagagat    1080
gagctgacca agaaccaggt gtccctgtgg tgtctggtca agggcttcta ccccagcgat    1140
atcgccgtgg agtgggagag caacggccag cctgagaaca actacaagac caccccccct    1200
gtgctggaca gcgacggcag cttcttcctg tactccaaac tgaccgtgga caagagccgg    1260
tggcagcagg gcaacgtgtt cagctgcagc gtgatgcacg aggccctgca caaccactac    1320
acccagaagt ccctgagcct gagccccggc ggaggcggcg gaagcggagg aggaggatcc    1380
agagagggcc ctgagctgag ccctgatgat cctgccggac tgctggacct gcggcaggga    1440
atgtttgccc agctggtggc ccagaacgtg ctgctgatcg atggcccccct gtcctggtac    1500
agcgatcctg gactggctgg cgtgtcactg acaggcggcc tgagctacaa agaggacacc    1560
aaagaactgg tggtggccaa ggccggcgtg tactacgtgt tctttcagct ggaactgcgg    1620
agagtggtgg ccggcgaagg atctggctct gtgtctctgg ccctgcatct gcagcctctg    1680
agatctgctg ctggcgccgc tgctctggca ctgacagtgg atctgcctcc tgccagcagc    1740
```

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttcctgccgg gctc 1914

<210> 124
<211> 1314
<212> DNA
<213> Artificial Sequence

<220>
<223> nucleotide sequence of anti-CD19(8B8-2B11) light chain

<400> 124

gatattgtca tgactcaaac tccactgtct ctgtccgtga ccccgggtca gccagcgagc 60

atttcttgca aatccagcca atctctggaa acctccaccg gcaccacgta cctgaactgg 120

tatctccaga aaccgggtca gagcccgcag ctgctgatct accgtgtatc taagcgcttc 180

tccggcgttc ctgatcgttt cagcggttct ggatccggca ccgactttac tctgaaaatc 240

agccgtgtgg aagctgaaga cgttggcgtc tactattgtc tgcagctgct ggaagatcca 300

tacaccttcg gtcaaggaac taaactggaa attaaacgta cggtggctgc accatctgtc 360

ttcatcttcc cgccatctga tgagcagttg aaatctggaa ctgcctctgt tgtgtgcctg 420

ctgaataact tctatcccag agaggccaaa gtacagtgga aggtggataa cgccctccaa 480

tcgggtaact cccaggagag tgtcacagag caggacagca aggacagcac ctacagcctc 540

agcagcaccc tgacgctgag caaagcagac tacgagaaac acaaagtcta cgcctgcgaa 600

gtcacccatc agggcctgag ctcgcccgtc acaaagagct tcaacagggg agagtgtgat 660

attgtcatga ctcaaactcc actgtctctg tccgtgaccc cgggtcagcc agcgagcatt 720

tcttgcaaat ccagccaatc tctggaaacc tccaccggca ccacgtacct gaactggtat 780

ctccagaaac cgggtcagag cccgcagctg ctgatctacc gtgtatctaa gcgcttctcc 840

ggcgttcctg atcgtttcag cggttctgga tccggcaccg actttactct gaaaatcagc 900

cgtgtggaag ctgaagacgt tggcgtctac tattgtctgc agctgctgga agatccatac 960

accttcggtc aaggaacgaa actggaaatt aaacgtacgg tggctgcacc atctgtcttc 1020

atcttcccgc catctgatga gcagttgaaa tctggaactg cctctgttgt gtgcctgctg 1080

aataacttct atcccagaga ggccaaagta cagtggaagg tggataacgc cctccaatcg 1140

ggtaactccc aggagagtgt cacagagcag gacagcaagg acagcaccta cagcctcagc 1200

agcaccctga cgctgagcaa agcagactac gagaaacaca aagtctacgc ctgcgaagtc 1260

acccatcagg gcctgagctc gcccgtcaca aagagcttca acaggggaga gtgt 1314

<210> 125
<211> 638
<212> PRT
<213> Artificial Sequence

<220>
<223> anti- CD19(8B8-2B11) Fc knob monomeric 4-1BB ligand (71-248)

<400> 125

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Ile Met His Trp Val Arg Gln Ala Pro Gly Gln Gly Leu Glu Trp Met
35 40 45

Gly Tyr Ile Asn Pro Tyr Asn Asp Gly Ser Lys Tyr Thr Glu Lys Phe
50 55 60

Gln Gly Arg Val Thr Met Thr Ser Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Gly Thr Tyr Tyr Tyr Gly Pro Gln Leu Phe Asp Tyr Trp Gly
100 105 110

Gln Gly Thr Thr Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Ala Ala Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Tyr Thr Leu Pro Pro Cys Arg Asp Glu Leu Thr Lys Asn Gln Val Ser
355 360 365

Leu Trp Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly

465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu
625 630 635

<210> 126
<211> 219
<212> PRT
<213> Artificial Sequence

<220>
<223> anti- CD19(8B8-2B11) light chain

<400> 126

```
Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Thr Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Leu Glu Asp Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 127
<211> 2448
<212> DNA
<213> Artificial Sequence

<220>

<223> nucleotide sequence of anti- CD19(8B8-2B11) Fc knob dimeric 4-1BB (71-248) ligand

<400> 127

```
caggtgcaat tggttcaatc tggtgctgaa gtaaaaaaac cgggcgcttc cgttaaagtg        60
agctgcaaag catctggtta caccttcact gactatatca tgcactgggt tcgtcaggcc       120
ccgggccagg gtctggagtg gatgggctac attaacccat acaacgacgg ttccaaatat       180
accgagaaat tccagggccg cgtcacgatg accagcgaca cttctatctc caccgcgtac       240
```

atggaactgt ctagactgcg ttctgacgac accgctgttt actattgtgc acgcggtacc 300

tactactacg gtccacagct gtttgattac tggggccaag gtaccacggt gaccgtaagc 360

tctgctagca ccaagggccc atcggtcttc cccctggcac cctcctccaa gagcacctct 420

gggggcacag cggccctggg ctgcctggtc aaggactact tccccgaacc ggtgacggtg 480

tcgtggaact caggcgccct gaccagcggc gtgcacacct tcccggctgt cctacagtcc 540

tcaggactct actccctcag cagcgtggtg accgtgccct ccagcagctt gggcacccag 600

acctacatct gcaacgtgaa tcacaagccc agcaacacca aggtggacaa gaaagttgag 660

cccaaatctt gtgacaaaac tcacacatgc ccaccgtgcc cagcacctga agctgcaggg 720

ggaccgtcag tcttcctctt ccccccaaaa cccaaggaca ccctcatgat ctcccggacc 780

cctgaggtca catgcgtggt ggtggacgtg agccacgaag accctgaggt caagttcaac 840

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtaca ccctgccccc ctgcagagat 1080

gagctgacca agaaccaggt gtccctgtgg tgtctggtca agggcttcta ccccagcgat 1140

atcgccgtgg agtgggagag caacggccag cctgagaaca actacaagac cacccccccct 1200

gtgctggaca gcgacggcag cttcttcctg tactccaaac tgaccgtgga caagagccgg 1260

tggcagcagg gcaacgtgtt cagctgcagc gtgatgcacg aggccctgca caaccactac 1320

acccagaagt ccctgagcct gagccccggc agagagggcc ctgagctgag ccctgatgat 1380

cctgccggac tgctggacct gcggcaggga atgtttgccc agctggtggc ccagaacgtg 1440

ctgctgatcg atggcccccct gtcctggtac agcgatcctg gactggctgg cgtgtcactg 1500

acaggcggcc tgagctacaa agaggacacc aaagaactgg tggtggccaa ggccggcgtg 1560

tactacgtgt tctttcagct ggaactgcgg agagtggtgg ccggcgaagg atctggctct 1620

gtgtctctgg ccctgcatct gcagcctctg agatctgctg ctggcgccgc tgctctggca 1680

ctgacagtgg atctgcctcc tgccagcagc gaggcccgga atagcgcatt tgggtttcaa 1740

ggcaggctgc tgcacctgtc tgccggccag aggctgggag tgcatctgca cacagaggcc 1800

agggctagac acgcctggca gctgacacag ggcgctacag tgctgggcct gttcagagtg 1860

acccccgaga ttccagcagg cctgggaggc ggcggatctg gcggcggagg atctagagaa 1920

ggacccgagc tgtcccccga cgatcccgct gggctgctgg atctgagaca gggcatgttc 1980

gctcagctgg tggctcagaa tgtgctgctg attgacggac ctctgagctg gtactccgac 2040

ccagggctgg caggggtgtc cctgactggg ggactgtcct acaaagaaga tacaaaagaa 2100

```
ctggtggtgg ctaaagctgg ggtgtactat gtgtttttc agctggaact gaggcgggtg     2160

gtggctgggg agggctcagg atctgtgtcc ctggctctgc atctgcagcc actgcgctct     2220

gcagcagggg ctgcagcact ggccctgact gtggacctgc ccccagcttc ttccgaggcc     2280

agaaacagcg ccttcgggtt ccaaggacgc ctgctgcatc tgagcgccgg acagcgcctg     2340

ggagtgcatc tgcatactga agccagagcc cggcatgctt ggcagctgac tcagggggca     2400

actgtgctgg gactgtttcg cgtgacacct gagatcccag ccgggctc                  2448
```

<210> 128
<211> 816
<212> PRT
<213> Artificial Sequence

<220>
<223> anti- CD19(8B8-2B11) Fc knob dimeric 4-1BB ligand (71-248)

<400> 128

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Gln Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160
```

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Cys Arg Asp Glu Leu Thr Lys Asn Gln Val Ser
        355                 360                 365

Leu Trp Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415
```

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Arg Glu Gly Pro Glu Leu Ser Pro Asp Asp Pro Ala Gly Leu
450 455 460

Leu Asp Leu Arg Gln Gly Met Phe Ala Gln Leu Val Ala Gln Asn Val
465 470 475 480

Leu Leu Ile Asp Gly Pro Leu Ser Trp Tyr Ser Asp Pro Gly Leu Ala
485 490 495

Gly Val Ser Leu Thr Gly Gly Leu Ser Tyr Lys Glu Asp Thr Lys Glu
500 505 510

Leu Val Val Ala Lys Ala Gly Val Tyr Tyr Val Phe Phe Gln Leu Glu
515 520 525

Leu Arg Arg Val Val Ala Gly Glu Gly Ser Gly Ser Val Ser Leu Ala
530 535 540

Leu His Leu Gln Pro Leu Arg Ser Ala Ala Gly Ala Ala Ala Leu Ala
545 550 555 560

Leu Thr Val Asp Leu Pro Pro Ala Ser Ser Glu Ala Arg Asn Ser Ala
565 570 575

Phe Gly Phe Gln Gly Arg Leu Leu His Leu Ser Ala Gly Gln Arg Leu
580 585 590

Gly Val His Leu His Thr Glu Ala Arg Ala Arg His Ala Trp Gln Leu
595 600 605

Thr Gln Gly Ala Thr Val Leu Gly Leu Phe Arg Val Thr Pro Glu Ile
610 615 620

Pro Ala Gly Leu Gly Gly Gly Gly Ser Gly Gly Gly Gly Ser Arg Glu
625 630 635 640

Gly Pro Glu Leu Ser Pro Asp Asp Pro Ala Gly Leu Leu Asp Leu Arg
645 650 655

Gln Gly Met Phe Ala Gln Leu Val Ala Gln Asn Val Leu Leu Ile Asp
660 665 670

Gly Pro Leu Ser Trp Tyr Ser Asp Pro Gly Leu Ala Gly Val Ser Leu
675 680 685

Thr Gly Gly Leu Ser Tyr Lys Glu Asp Thr Lys Glu Leu Val Val Ala
690 695 700

Lys Ala Gly Val Tyr Tyr Val Phe Phe Gln Leu Glu Leu Arg Arg Val
705 710 715 720

Val Ala Gly Glu Gly Ser Gly Ser Val Ser Leu Ala Leu His Leu Gln
725 730 735

Pro Leu Arg Ser Ala Ala Gly Ala Ala Ala Leu Ala Leu Thr Val Asp
740 745 750

Leu Pro Pro Ala Ser Ser Glu Ala Arg Asn Ser Ala Phe Gly Phe Gln
755 760 765

Gly Arg Leu Leu His Leu Ser Ala Gly Gln Arg Leu Gly Val His Leu
770 775 780

His Thr Glu Ala Arg Ala Arg His Ala Trp Gln Leu Thr Gln Gly Ala
785 790 795 800

Thr Val Leu Gly Leu Phe Arg Val Thr Pro Glu Ile Pro Ala Gly Leu
805 810 815

<210> 129
<211> 6
<212> PRT
<213> Artificial Sequence

<220>
<223> HVR-L1 of anti-CD19 8B8

<400> 129

Asn Ser Asn Gly Asn Thr
1 5

<210> 130
<211> 4
<212> PRT
<213> Artificial Sequence

<220>
<223> HVR-L2 of anti-CD19 8B8

<400> 130

Lys Phe Asn Gly

1

<210> 131
<211> 681
<212> DNA
<213> Artificial Sequence

<220>
<223> Nucleotide sequence of Fc hole chain with HYRF mutation

<400> 131

```
gacaaaactc acacatgccc accgtgccca gcacctgaag ctgcaggggg accgtcagtc        60
ttcctcttcc ccccaaaacc caaggacacc ctcatgatct cccggacccc tgaggtcaca       120
tgcgtggtgg tggacgtgag ccacgaagac cctgaggtca agttcaactg gtacgtggac       180
ggcgtggagg tgcataatgc caagacaaag ccgcgggagg agcagtacaa cagcacgtac       240
cgtgtggtca gcgtcctcac cgtcctgcac caggactggc tgaatggcaa ggagtacaag       300
tgcaaggtct ccaacaaagc cctcggcgcc cccatcgaga aaaccatctc caaagccaaa       360
gggcagcccc gagaaccaca ggtgtgcacc ctgcccccat cccgggatga gctgaccaag       420
aaccaggtca gcctctcgtg cgcagtcaaa ggcttctatc ccagcgacat cgccgtggag       480
tgggagagca atgggcagcc ggagaacaac tacaagacca cgcctcccgt gctggactcc       540
gacggctcct tcttcctcgt gagcaagctc accgtggaca agagcaggtg gcagcagggg       600
aacgtcttct catgctccgt gatgcatgag gctctgcaca accgcttcac gcagaagagc       660
ctctccctgt ctccgggtaa a                                                 681
```

<210> 132
<211> 1602
<212> DNA
<213> Artificial Sequence

<220>
<223> Nucleotide sequence of human CD19 antigen Fc knob chain avi tag

<400> 132

```
cccgaggaac ccctggtcgt gaaggtggaa gagggcgaca atgccgtgct gcagtgcctg      60
aagggcacct ccgatggccc tacccagcag ctgacctggt ccagagagag ccccctgaag     120
cccttcctga agctgtctct gggcctgcct ggcctgggca tccatatgag gcctctggcc     180
atctggctgt tcatcttcaa cgtgtcccag cagatgggcg gcttctacct gtgtcagcct     240
ggcccccat ctgagaaggc ttggcagcct ggctggaccg tgaacgtgga aggatccggc      300
gagctgttcc ggtggaacgt gtccgatctg ggcggcctgg gatgcggcct gaagaacaga     360
tctagcgagg gccccagcag ccccagcggc aaactgatga gccccaagct gtacgtgtgg     420
gccaaggaca gacccgagat ctgggagggc gagcctcctt gcctgccccc tagagacagc     480
ctgaaccaga gcctgagcca ggacctgaca atggcccctg gcagcacact gtggctgagc     540
tgtggcgtgc cacccgactc tgtgtctaga ggccctctga gctggaccca cgtgcaccct     600
aagggcccta agagcctgct gagcctggaa ctgaaggacg acaggcccgc cagagatatg     660
tgggtcatgg aaaccggcct gctgctgcct agagccacag cccaggatgc cggcaagtac     720
tactgccaca gaggcaacct gaccatgagc ttccacctgg aaatcaccgc cagacccgtg     780
ctgtggcact ggctgctgag aacaggcggc tggaaggtcg acgctagcgg tggtagtccg     840
acacctccga cacccggggg tggttctgca gacaaaactc acacatgccc accgtgccca     900
gcacctgaag ccgcaggggg accgtcagtc ttcctcttcc ccccaaaacc caaggacacc     960
ctcatgatct cccggacccc tgaggtcaca tgcgtggtgg tggacgtgag ccacgaagac    1020
cctgaggtca agttcaactg gtacgtggac ggcgtggagg tgcataatgc caagacaaag    1080
ccgcgggagg agcagtacaa cagcacgtac cgtgtggtca gcgtcctcac cgtcctgcac    1140
caggactggc tgaatggcaa ggagtacaag tgcaaggtct ccaacaaagc cctcggagcc    1200
cccatcgaga aaaccatctc caaagccaaa gggcagcccc gagaaccaca ggtgtacacc    1260
ctgcccccat gccgggatga gctgaccaag aaccaggtca gcctgtggtg cctggtcaaa    1320
ggcttctatc ccagcgacat cgccgtggag tgggagagca atgggcagcc ggagaacaac    1380
tacaagacca cgcctcccgt gctggactcc gacggctcct tcttcctcta cagcaagctc    1440
accgtggaca agagcaggtg gcagcagggg aacgtcttct catgctccgt gatgcatgag    1500
gctctgcaca accactacac gcagaagagc ctctccctgt ctccgggtaa atccggaggc    1560
ctgaacgaca tcttcgaggc ccagaagatt gaatggcacg ag                       1602
```

<210> 133
<211> 227
<212> PRT
<213> Artificial Sequence

<220>
<223> Fc hole chain with HYRF mutation

<400> 133

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn Arg Phe Thr Gln Lys Ser Leu Ser Leu Ser
    210                 215                 220

Pro Gly Lys
225

<210> 134
<211> 534
<212> PRT
<213> Artificial Sequence

<220>
<223> human CD19 antigen Fc knob chain avi tag

<400> 134

```
Pro Glu Glu Pro Leu Val Val Lys Val Glu Glu Gly Asp Asn Ala Val
1               5                   10                  15

Leu Gln Cys Leu Lys Gly Thr Ser Asp Gly Pro Thr Gln Gln Leu Thr
            20                  25                  30
```

```
Trp Ser Arg Glu Ser Pro Leu Lys Pro Phe Leu Lys Leu Ser Leu Gly
        35                  40                  45

Leu Pro Gly Leu Gly Ile His Met Arg Pro Leu Ala Ile Trp Leu Phe
    50                  55                  60

Ile Phe Asn Val Ser Gln Gln Met Gly Gly Phe Tyr Leu Cys Gln Pro
65                  70                  75                  80

Gly Pro Pro Ser Glu Lys Ala Trp Gln Pro Gly Trp Thr Val Asn Val
                85                  90                  95

Glu Gly Ser Gly Glu Leu Phe Arg Trp Asn Val Ser Asp Leu Gly Gly
            100                 105                 110

Leu Gly Cys Gly Leu Lys Asn Arg Ser Ser Glu Gly Pro Ser Ser Pro
        115                 120                 125

Ser Gly Lys Leu Met Ser Pro Lys Leu Tyr Val Trp Ala Lys Asp Arg
    130                 135                 140

Pro Glu Ile Trp Glu Gly Glu Pro Pro Cys Leu Pro Pro Arg Asp Ser
145                 150                 155                 160

Leu Asn Gln Ser Leu Ser Gln Asp Leu Thr Met Ala Pro Gly Ser Thr
                165                 170                 175

Leu Trp Leu Ser Cys Gly Val Pro Pro Asp Ser Val Ser Arg Gly Pro
            180                 185                 190

Leu Ser Trp Thr His Val His Pro Lys Gly Pro Lys Ser Leu Leu Ser
        195                 200                 205

Leu Glu Leu Lys Asp Asp Arg Pro Ala Arg Asp Met Trp Val Met Glu
    210                 215                 220

Thr Gly Leu Leu Leu Pro Arg Ala Thr Ala Gln Asp Ala Gly Lys Tyr
225                 230                 235                 240

Tyr Cys His Arg Gly Asn Leu Thr Met Ser Phe His Leu Glu Ile Thr
                245                 250                 255

Ala Arg Pro Val Leu Trp His Trp Leu Leu Arg Thr Gly Gly Trp Lys
            260                 265                 270

Val Asp Ala Ser Gly Gly Ser Pro Thr Pro Pro Thr Pro Gly Gly Gly
        275                 280                 285
```

```
Ser Ala Asp Lys Thr His Thr Cys Pro Pro Cys Pro Ala Pro Glu Ala
    290                 295                 300

Ala Gly Gly Pro Ser Val Phe Leu Phe Pro Pro Lys Pro Lys Asp Thr
305                 310                 315                 320

Leu Met Ile Ser Arg Thr Pro Glu Val Thr Cys Val Val Val Asp Val
                325                 330                 335

Ser His Glu Asp Pro Glu Val Lys Phe Asn Trp Tyr Val Asp Gly Val
            340                 345                 350

Glu Val His Asn Ala Lys Thr Lys Pro Arg Glu Glu Gln Tyr Asn Ser
        355                 360                 365

Thr Tyr Arg Val Val Ser Val Leu Thr Val Leu His Gln Asp Trp Leu
    370                 375                 380

Asn Gly Lys Glu Tyr Lys Cys Lys Val Ser Asn Lys Ala Leu Gly Ala
385                 390                 395                 400

Pro Ile Glu Lys Thr Ile Ser Lys Ala Lys Gly Gln Pro Arg Glu Pro
                405                 410                 415

Gln Val Tyr Thr Leu Pro Pro Cys Arg Asp Glu Leu Thr Lys Asn Gln
            420                 425                 430

Val Ser Leu Trp Cys Leu Val Lys Gly Phe Tyr Pro Ser Asp Ile Ala
        435                 440                 445

Val Glu Trp Glu Ser Asn Gly Gln Pro Glu Asn Asn Tyr Lys Thr Thr
    450                 455                 460

Pro Pro Val Leu Asp Ser Asp Gly Ser Phe Phe Leu Tyr Ser Lys Leu
465                 470                 475                 480

Thr Val Asp Lys Ser Arg Trp Gln Gln Gly Asn Val Phe Ser Cys Ser
                485                 490                 495

Val Met His Glu Ala Leu His Asn His Tyr Thr Gln Lys Ser Leu Ser
            500                 505                 510

Leu Ser Pro Gly Lys Ser Gly Gly Leu Asn Asp Ile Phe Glu Ala Gln
        515                 520                 525

Lys Ile Glu Trp His Glu
    530
```

<210> 135
<211> 1605
<212> DNA
<213> Artificial Sequence

<220>
<223> Nucleotide sequence of cynomolgus CD19 antigen Fc knob chain avi tag

<400> 135

```
ccccaggaac ccctggtcgt gaaggtggaa gagggcgaca atgccgtgct ccagtgcctg      60
gaaggcacct ccgatggccc tacacagcag ctcgtgtggt gcagagacag cccccttcgag     120
cccttcctga acctgtctct gggcctgcct ggcatgggca tcagaatggg ccctctgggc     180
atctggctgc tgatcttcaa cgtgtccaac cagaccggcg gcttctacct gtgtcagcct     240
ggcctgccaa gcgagaaggc ttggcagcct ggatggaccg tgtccgtgga aggatctggc     300
gagctgttcc ggtggaacgt gtccgatctg ggcggcctgg gatgcggcct gaagaacaga     360
agcagcgagg gccctagcag ccccagcggc aagctgaata gcagccagct gtacgtgtgg     420
gccaaggaca gacccgagat gtgggagggc gagcctgtgt gtggcccccc tagagatagc     480
ctgaaccaga gcctgagcca ggacctgaca atggcccctg gcagcacact gtggctgagc     540
tgtggcgtgc cacccgactc tgtgtccaga ggccctctga gctggacaca cgtgcggcca     600
aagggcccta agagcagcct gctgagcctg gaactgaagg acgaccggcc cgaccgggat     660
atgtgggtgg tggatacagg cctgctgctg accagagcca cagcccagga tgccggcaag     720
tactactgcc acagaggcaa ctggaccaag agcttttacc tggaaatcac cgccagaccc     780
gccctgtggc actggctgct gagaatcgga ggctggaagg tcgacgctag cggtggtagt     840
ccgacacctc cgacacccgg gggtggttct gcagacaaaa ctcacacatg cccaccgtgc     900
ccagcacctg aagccgcagg gggaccgtca gtcttcctct tccccccaaa acccaaggac     960
accctcatga tctcccggac ccctgaggtc acatgcgtgg tggtggacgt gagccacgaa    1020
gaccctgagg tcaagttcaa ctggtacgtg gacggcgtgg aggtgcataa tgccaagaca    1080
aagccgcggg aggagcagta caacagcacg taccgtgtgg tcagcgtcct caccgtcctg    1140
caccaggact ggctgaatgg caaggagtac aagtgcaagg tctccaacaa agccctcgga    1200
gcccccatcg agaaaaccat ctccaaagcc aaagggcagc cccgagaacc acaggtgtac    1260
accctgcccc catgccggga tgagctgacc aagaaccagg tcagcctgtg gtgcctggtc    1320
aaaggcttct atcccagcga catcgccgtg gagtgggaga gcaatgggca gccggagaac    1380
aactacaaga ccacgcctcc cgtgctggac tccgacggct ccttcttcct ctacagcaag    1440
ctcaccgtgg acaagagcag gtggcagcag gggaacgtct tctcatgctc cgtgatgcat    1500
gaggctctgc acaaccacta cacgcagaag agcctctccc tgtctccggg taaatccgga    1560
```

ggcctgaacg acatcttcga ggcccagaag attgaatggc acgag 1605

<210> 136
<211> 535
<212> PRT
<213> Artificial Sequence

<220>
<223> cynomolgus CD19 antigen Fc knob chain avi tag

<400> 136

```
Pro Gln Glu Pro Leu Val Val Lys Val Glu Glu Gly Asp Asn Ala Val
1               5                   10                  15

Leu Gln Cys Leu Glu Gly Thr Ser Asp Gly Pro Thr Gln Gln Leu Val
            20                  25                  30

Trp Cys Arg Asp Ser Pro Phe Glu Pro Phe Leu Asn Leu Ser Leu Gly
        35                  40                  45

Leu Pro Gly Met Gly Ile Arg Met Gly Pro Leu Gly Ile Trp Leu Leu
    50                  55                  60

Ile Phe Asn Val Ser Asn Gln Thr Gly Gly Phe Tyr Leu Cys Gln Pro
65                  70                  75                  80

Gly Leu Pro Ser Glu Lys Ala Trp Gln Pro Gly Trp Thr Val Ser Val
                85                  90                  95

Glu Gly Ser Gly Glu Leu Phe Arg Trp Asn Val Ser Asp Leu Gly Gly
            100                 105                 110

Leu Gly Cys Gly Leu Lys Asn Arg Ser Ser Glu Gly Pro Ser Ser Pro
        115                 120                 125

Ser Gly Lys Leu Asn Ser Ser Gln Leu Tyr Val Trp Ala Lys Asp Arg
    130                 135                 140

Pro Glu Met Trp Glu Gly Glu Pro Val Cys Gly Pro Pro Arg Asp Ser
145                 150                 155                 160

Leu Asn Gln Ser Leu Ser Gln Asp Leu Thr Met Ala Pro Gly Ser Thr
                165                 170                 175

Leu Trp Leu Ser Cys Gly Val Pro Pro Asp Ser Val Ser Arg Gly Pro
            180                 185                 190

Leu Ser Trp Thr His Val Arg Pro Lys Gly Pro Lys Ser Ser Leu Leu
```

195 200 205

Ser Leu Glu Leu Lys Asp Asp Arg Pro Asp Arg Asp Met Trp Val Val
210 215 220

Asp Thr Gly Leu Leu Leu Thr Arg Ala Thr Ala Gln Asp Ala Gly Lys
225 230 235 240

Tyr Tyr Cys His Arg Gly Asn Trp Thr Lys Ser Phe Tyr Leu Glu Ile
245 250 255

Thr Ala Arg Pro Ala Leu Trp His Trp Leu Leu Arg Ile Gly Gly Trp
260 265 270

Lys Val Asp Ala Ser Gly Gly Ser Pro Thr Pro Pro Thr Pro Gly Gly
275 280 285

Gly Ser Ala Asp Lys Thr His Thr Cys Pro Pro Cys Pro Ala Pro Glu
290 295 300

Ala Ala Gly Gly Pro Ser Val Phe Leu Phe Pro Pro Lys Pro Lys Asp
305 310 315 320

Thr Leu Met Ile Ser Arg Thr Pro Glu Val Thr Cys Val Val Val Asp
325 330 335

Val Ser His Glu Asp Pro Glu Val Lys Phe Asn Trp Tyr Val Asp Gly
340 345 350

Val Glu Val His Asn Ala Lys Thr Lys Pro Arg Glu Glu Gln Tyr Asn
355 360 365

Ser Thr Tyr Arg Val Val Ser Val Leu Thr Val Leu His Gln Asp Trp
370 375 380

Leu Asn Gly Lys Glu Tyr Lys Cys Lys Val Ser Asn Lys Ala Leu Gly
385 390 395 400

Ala Pro Ile Glu Lys Thr Ile Ser Lys Ala Lys Gly Gln Pro Arg Glu
405 410 415

Pro Gln Val Tyr Thr Leu Pro Pro Cys Arg Asp Glu Leu Thr Lys Asn
420 425 430

Gln Val Ser Leu Trp Cys Leu Val Lys Gly Phe Tyr Pro Ser Asp Ile
435 440 445

Ala Val Glu Trp Glu Ser Asn Gly Gln Pro Glu Asn Asn Tyr Lys Thr
450 455 460

Thr Pro Pro Val Leu Asp Ser Asp Gly Ser Phe Phe Leu Tyr Ser Lys
465 470 475 480

Leu Thr Val Asp Lys Ser Arg Trp Gln Gln Gly Asn Val Phe Ser Cys
485 490 495

Ser Val Met His Glu Ala Leu His Asn His Tyr Thr Gln Lys Ser Leu
500 505 510

Ser Leu Ser Pro Gly Lys Ser Gly Gly Leu Asn Asp Ile Phe Glu Ala
515 520 525

Gln Lys Ile Glu Trp His Glu
530 535

<210> 137
<211> 363
<212> DNA
<213> Artificial Sequence

<220>
<223> Nucleotide sequence CD19 (8B8) VH Parental clone

<400> 137

caagttcaat tggttcaatc tggtgctgaa gtaaaaaaac cgggcgcttc cgttaaagtg 60

agctgcaaag catctggtta caccttcact gactatatca tgcactgggt tcgtcaggcc 120

ccgggccagg gtctggagtg gatgggctac attaacccat acaacgacgg ttccaaatat 180

accgagaaat tccagggccg cgtcacgatg accagcgaca cttctatctc caccgcgtac 240

atggaactgt ctagactgcg ttctgacgac accgctgttt actattgtgc acgcggtact 300

tactactacg gttccgccct ctttgattac tggggccaag gtaccacggt gaccgtaagc 360

tct 363

<210> 138
<211> 336
<212> DNA
<213> Artificial Sequence

<220>
<223> Nucleotide sequence CD19 (8B8) VL Parental clone

<400> 138

gatattgtta tgactcaaac tccactgtct ctgtccgtga ccccgggtca gccagcgagc 60

atttcttgca aatccagcca atctctggaa aactccaacg gcaacacgta cctgaactgg 120

tatctccaga aaccgggtca gagcccgcag ctgctgatct accgtgtatc taagcgcttc 180

tccggcgttc ctgatcgttt cagcggttct ggatccggca ccgactttac tctgaaaatc 240

agccgtgtgg aagctgaaga cgttggcgtc tactattgtc tgcagttgac ccacgttccg 300

tacaccttcg gtcaaggaac taaactggaa attaaa 336

<210> 139
<211> 94
<212> DNA
<213> Artificial Sequence

<220>
<223> 43-45: 40% Y, 6% A/S/T/G/P/D/N/E/Q/V, 49-51: 40% N, 6% A/S/T/Y/G/P/D/E/Q/V, 55-57: 25% S/T/Q/E, 61-63: 25% S/T/Q/E

<220>
<221> misc_feature
<222> (43)..(45)
<223> n is a, c, g or t

<220>
<221> misc_feature
<222> (49)..(51)
<223> n is a, c, g or t

<220>
<221> misc_feature
<222> (55) .. (57)
<223> n is a, c, g or t

<220>
<221> misc_feature
<222> (61) .. (63)
<223> n is a, c, g or t

<400> 139

cagctgcggg ctctgacccg gtttctggag ataccagttc agnnncgtnn ngccnnngga 60

nnnttccaga gattggctgg atttgcaaga aatg 94

<210> 140
<211> 99
<212> DNA
<213> Artificial Sequence

<220>
<223> 40-42: 30% R, 20% E, 5% A/S/T/Y/G/P/D/N/Q/V. 49-51: 30% K, 20% S, 5% A/N/T/Y/G/P/D/E/Q/V, 55-57: 40% F, 5% A/S/T/Y/G/P/D/E/Q/V/I/L, 58-60: 40% S, 6.6% A/T/Y/G/P/D/E/Q/V, 67-69: 50% P, 50% L

<220>

<221> misc_feature
<222> (40) .. (42)
<223> n is a, c, g or t

<220>
<221> misc_feature
<222> (49) .. (51)
<223> n is a, c, g or t

<220>
<221> misc_feature
<222> (55) .. (57)
<223> n is a, c, g or t

<220>
<221> misc_feature
<222> (58) .. (60)
<223> n is a, c, g or t

<220>
<221> misc_feature
<222> (67) .. (69)
<223> n is a, c, g or t

<400> 140

```
ctccagaaac cgggtcagag cccgcagctg ctgatctacn nngtatctnn ncgcnnnnnn      60

ggcgttnnng atcgtttcag cggttctgga tccggcacc                             99
```

<210> 141
<211> 99
<212> DNA
<213> Artificial Sequence

<220>
<223> 40-42: 52% H, 4% G/A/S/P/T/N/Y/D/E/Q/V/I, 46-48: 30% I, 15% Y, 5% G/A/S/T/P/N/H/D/E/Q/V, 49-51: 52% Y, 4% G/A/S/P/T/N/H/D/E/Q/V/I, 52-54: 30% D, 15% G, 5% A/S/P/Y/N/H/D/E/Q/V/I, 55-57: 52% T, 4% G/A/S/P/Y/N/H/D/E/Q/V/I, 61-63: 52% T, 4% G/A/S/P/Y/N/H/D/E/Q/V/I

<220>
<221> misc_feature
<222> (40) .. (42)
<223> n is a, c, g or t

<220>
<221> misc_feature
<222> (46) .. (48)
<223> n is a, c, g or t

<220>
<221> misc_feature
<222> (49) .. (51)
<223> n is a, c, g or t

<220>
<221> misc_feature

<222> (52) .. (54)
<223> n is a, c, g or t

<220>
<221> misc_feature
<222> (55) .. (57)
<223> n is a, c, g or t

<220>
<221> misc_feature
<222> (61) .. (63)
<223> n is a, c, g or t

<400> 141

catccactcc agaccctggc ccggggcctg acgaacccan nncatnnnnn nnnnnnngaa 60

nnngtaacca gatgctttgc agctcacttt aacggaagc 99

<210> 142
<211> 99
<212> DNA
<213> Artificial Sequence

<220>
<223> 34-36: 45% Y, 5% others, 40-42: 52% N, 4% others, 46-48: 40% Y, 5% others, 49-51: 30% N, 15% S, 5% others, 52-54: 30% D, 15% G, 5% others, 55-57: 52% G, 4% others, 61-63: 30% K, 15% N, 4% others, 70-72: 30% E, 15% Q, 5% others

<220>
<221> misc_feature
<222> (34) .. (36)
<223> n is a, c, g or t

<220>
<221> misc_feature
<222> (40) .. (42)
<223> n is a, c, g or t

<220>
<221> misc_feature
<222> (46) .. (48)
<223> n is a, c, g or t

<220>
<221> misc_feature
<222> (49) .. (51)
<223> n is a, c, g or t

<220>
<221> misc_feature
<222> (52) .. (54)
<223> n is a, c, g or t

<220>
<221> misc_feature
<222> (55) .. (57)

<223> n is a, c, g or t

<220>
<221> misc_feature
<222> (61) .. (63)
<223> n is a, c, g or t

<220>
<221> misc_feature
<222> (70) .. (72)
<223> n is a, c, g or t

<400> 142

caggccccgg gccagggtct ggagtggatg ggcnnnattn nnccannnnn nnnnnnntcc 60

nnntataccn nnaaattcca gggccgcgtc acgatgacc 99

<210> 143
<211> 33
<212> DNA
<213> Artificial Sequence

<220>
<223> CD19 H3 reverse constant

<400> 143
cgtcaccggt tcggggaagt agtccttgac cag 33

<210> 144
<211> 26
<212> DNA
<213> Artificial Sequence

<220>
<223> LMB3

<400> 144
caggaaacag ctatgaccat gattac 26

<210> 145
<211> 33
<212> DNA
<213> Artificial Sequence

<220>
<223> CD19 L1 forward constant

<400> 145
tggtatctcc agaaaccggg tcagagcccg cag 33

<210> 146
<211> 84
<212> DNA
<213> Artificial Sequence

<220>

<223> 34-36: 52% Y, 4% others, 37-39: 52% P, 4% others, 40-42: 42% V, 10% L, 4% others, 43-45: 52% H, 4% others, 46-48: 42% T, 10% I, 4% others, 49-51: 45% L, 11% G, 4% others

<220>
<221> misc_feature
<222> (34) .. (36)
<223> n is a, c, g or t

<220>
<221> misc_feature
<222> (37) .. (39)
<223> n is a, c, g or t

<220>
<221> misc_feature
<222> (40) .. (42)
<223> n is a, c, g or t

<220>
<221> misc_feature
<222> (43)..(45)
<223> n is a, c, g or t

<220>
<221> misc_feature
<222> (46) .. (48)
<223> n is a, c, g or t

<220>
<221> misc_feature
<222> (49)..(51)
<223> n is a, c, g or t

<400> 146
tttaatttcc agtttagttc cttgaccgaa ggtnnnnnnn nnnnnnnnnn nctgcagaca 60

atagtagacg ccaacgtctt cagc 84

<210> 147
<211> 35
<212> DNA
<213> Artificial Sequence

<220>
<223> CD19 L3 forward constant

<400> 147
accttcggtc aaggaactaa actggaaatt aaacg 35

<210> 148
<211> 107
<212> DNA
<213> Artificial Sequence

<220>
<223> pos. 59-61: 50% L, 3.8% others, 62-64: 50% A, 4.2% others, 65-67: 50% S, 4.2% others, 68-70: 50% G, 4.2% others, 71-73: 50% Y, 4.2% others, 74-76: 50% Y, 4.2% others, 77-79: 50% Y, 4.2% others, 80-82: 50% T, 4.2% others, 83-85: 50% G, 4.2% others.

<220>
<221> misc_feature
<222> (54) .. (58)
<223> n is a, c, g, or t

<220>
<221> misc_feature
<222> (59)..(61)
<223> n is a, c, g or t

<220>
<221> misc_feature
<222> (62)..(64)
<223> n is a, c, g or t

<220>
<221> misc_feature
<222> (65) .. (67)
<223> n is a, c, g or t

<220>
<221> misc_feature
<222> (68) .. (70)
<223> n is a, c, g or t

<220>
<221> misc_feature
<222> (71) .. (73)
<223> n is a, c, g or t

<220>
<221> misc_feature
<222> (74) .. (76)
<223> n is a, c, g or t

<220>
<221> misc_feature
<222> (77) .. (79)
<223> n is a, c, g or t

<220>
<221> misc_feature
<222> (80) .. (82)
<223> n is a, c, g or t

<220>
<221> misc_feature
<222> (83) .. (85)
<223> n is a, c, g or t

<400> 148

**ttggtgctag cagagcttac ggtcaccgtg gtaccttggc cccagtaatc aaannnnnnn 60**

**nnnnnnnnnn nnnnnnnnnn gcgtgcacaa tagtaaacag cggtgtc 107**

<210> 149

<211> 1615
<212> DNA
<213> Artificial Sequence

<220>
<223> SNAP tag human CD19 ECD- PDGFR DNA

<400> 149

```
ggccgccgct agcggcatcg actacaagga cgacgatgac aaggccggca tcgatgccat      60
catggacaaa gactgcgaaa tgaagcgcac caccctggat agccctctgg gcaagctgga     120
actgtctggg tgcgaacagg gcctgcacga gatcaagctg ctgggcaaag gaacatctgc     180
cgccgacgcc gtggaagtgc ctgccccagc cgccgtgctg ggcggaccag agccactgat     240
gcaggccacc gcctggctca acgcctactt tcaccagcct gaggccatcg aggagttccc     300
tgtgccagcc ctgcaccacc cagtgttcca gcaggagagc tttacccgcc aggtgctgtg     360
gaaactgctg aaagtggtga agttcggaga ggtcatcagc taccagcagc tggccgccct     420
ggccggcaat cccgccgcca ccgccgccgt gaaaaccgcc ctgagcggaa atcccgtgcc     480
```

```
cattctgatc ccctgccacc gggtggtgtc tagctctggc gccgtggggg gctacgaggg    540
cgggctcgcc gtgaaagagt ggctgctggc ccacgagggc cacagactgg gcaagcctgg    600
gctgggtgat atccccgagg aacccctggt cgtgaaggtg gaagagggcg acaatgccgt    660
gctgcagtgc ctgaagggca cctccgatgg ccctacccag cagctgacct ggtccagaga    720
gagccccctg aagcccttcc tgaagctgtc tctgggcctg cctggcctgg gcatccatat    780
gaggcctctg gccatctggc tgttcatctt caacgtgtcc cagcagatgg gcggcttcta    840
cctgtgtcag cctggccccc catctgagaa ggcttggcag cctggctgga ccgtgaacgt    900
ggaaggatcc ggcgagctgt tccggtggaa cgtgtccgat ctgggcggcc tgggatgcgg    960
cctgaagaac agatctagcg agggccccag cagccccagc ggcaaactga tgagccccaa   1020
gctgtacgtg tgggccaagg acagacccga gatctgggag ggcgagcctc cttgcctgcc   1080
ccctagagac agcctgaacc agagcctgag ccaggacctg acaatggccc ctggcagcac   1140
actgtggctg agctgtggcg tgccacccga ctctgtgtct agaggccctc tgagctggac   1200
ccacgtgcac cctaagggcc ctaagagcct gctgagcctg gaactgaagg acgacaggcc   1260
cgccagagat atgtgggtca tggaaaccgg cctgctgctg cctagagcca cagcccagga   1320
tgccggcaag tactactgcc acagaggcaa cctgaccatg agcttccacc tggaaatcac   1380
cgccagaccc gtgctgtggc actggctgct gagaacaggc ggctggaagg tcgacgaaca   1440
aaaactcatc tcagaagagg atctgaatgc tgtgggccag gacacgcagg aggtcatcgt   1500
ggtgccacac tccttgccct ttaaggtggt ggtgatctca gccatcctgg ccctggtggt   1560
gctcaccatc atctccctta tcatcctcat catgctttgg cagaagaagc cacgt         1615
```

<210> 150
<211> 1620
<212> DNA
<213> Artificial Sequence

<220>
<223> SNAP tag cynomolgus CD19 ECD- PDGFR DNA

<400> 150

```
ccggccgccg ctagcggcat cgactacaag gacgacgatg acaaggccgg catcgatgcc        60
atcatggaca aagactgcga aatgaagcgc accaccctgg atagccctct gggcaagctg       120
gaactgtctg ggtgcgaaca gggcctgcac gagatcaagc tgctgggcaa aggaacatct       180
gccgccgacg ccgtggaagt gcctgcccca gccgccgtgc tgggcggacc agagccactg       240
atgcaggcca ccgcctggct caacgcctac tttcaccagc ctgaggccat cgaggagttc       300
cctgtgccag ccctgcacca cccagtgttc cagcaggaga gctttacccg ccaggtgctg       360
tggaaactgc tgaaagtggt gaagttcgga gaggtcatca gctaccagca gctggccgcc       420
ctggccggca atcccgccgc caccgccgcc gtgaaaaccg ccctgagcgg aaatcccgtg       480
cccattctga tcccctgcca ccgggtggtg tctagctctg gcgccgtggg gggctacgag       540
ggcgggctcg ccgtgaaaga gtggctgctg gcccacgagg gccacagact gggcaagcct       600
gggctgggtg atatccccca ggaacccctg gtcgtgaagg tggaagaggg cgacaatgcc       660
gtgctccagt gtctcgaggg cacctccgat ggccctacac agcagctcgt gtggtgcaga       720
gacagcccct tcgagccctt cctgaacctg tctctgggcc tgcctggcat gggcatcaga       780
atgggccctc tgggcatctg gctgctgatc ttcaacgtgt ccaaccagac cggcggcttc       840
tacctgtgtc agcctggcct gccaagcgag aaggcttggc agcctggatg gaccgtgtcc       900
gtggaaggat ctggcgagct gttccggtgg aacgtgtccg atctgggcgg cctgggatgc       960
ggcctgaaga acagaagcag cgagggccct agcagcccca gcggcaagct gaatagcagc      1020
cagctgtacg tgtgggccaa ggacagaccc gagatgtggg agggcgagcc tgtgtgtggc      1080
ccccctagag atagcctgaa ccagagcctg agccaggacc tgacaatggc ccctggcagc      1140
acactgtggc tgagctgtgg cgtgccaccc gactctgtgt ccagaggccc tctgagctgg      1200
acacacgtgc ggcctaaggg ccctaagagc agcctgctga gcctggaact gaaggacgac      1260
cggcccgacc gggatatgtg ggtggtggat acaggcctgc tgctgaccag agccacagcc      1320
caggatgccg gcaagtacta ctgccacaga ggcaactgga ccaagagctt ttacctggaa      1380
atcaccgcca gacccgccct gtggcactgg ctgctgagaa tcggaggctg gaaggtcgac      1440
gagcagaagc tgatctccga agaggacctg aacgccgtgg gccaggatac ccaggaagtg      1500
atcgtggtgc cccacagcct gcccttcaag gtggtcgtga tcagcgccat tctggccctg      1560
gtggtgctga ccatcatcag cctgatcatc ctgattatgc tgtggcagaa aaagccccgc      1620
```

<210> 151
<211> 539
<212> PRT
<213> Artificial Sequence

<220>

<223> SNAP tag human CD19 ECD- PDGFR

<400> 151

```
Pro Ala Ala Ala Ser Gly Ile Asp Tyr Lys Asp Asp Asp Asp Lys Ala
1               5                   10                  15

Gly Ile Asp Ala Ile Met Asp Lys Asp Cys Glu Met Lys Arg Thr Thr
            20                  25                  30

Leu Asp Ser Pro Leu Gly Lys Leu Glu Leu Ser Gly Cys Glu Gln Gly
        35                  40                  45
```

Leu His Glu Ile Lys Leu Leu Gly Lys Gly Thr Ser Ala Ala Asp Ala
    50                  55                  60

Val Glu Val Pro Ala Pro Ala Ala Val Leu Gly Gly Pro Glu Pro Leu
65                  70                  75                  80

Met Gln Ala Thr Ala Trp Leu Asn Ala Tyr Phe His Gln Pro Glu Ala
                85                  90                  95

Ile Glu Glu Phe Pro Val Pro Ala Leu His His Pro Val Phe Gln Gln
            100                 105                 110

Glu Ser Phe Thr Arg Gln Val Leu Trp Lys Leu Leu Lys Val Val Lys
        115                 120                 125

Phe Gly Glu Val Ile Ser Tyr Gln Gln Leu Ala Ala Leu Ala Gly Asn
    130                 135                 140

Pro Ala Ala Thr Ala Ala Val Lys Thr Ala Leu Ser Gly Asn Pro Val
145                 150                 155                 160

Pro Ile Leu Ile Pro Cys His Arg Val Val Ser Ser Ser Gly Ala Val
                165                 170                 175

Gly Gly Tyr Glu Gly Gly Leu Ala Val Lys Glu Trp Leu Leu Ala His
            180                 185                 190

Glu Gly His Arg Leu Gly Lys Pro Gly Leu Gly Asp Ile Pro Glu Glu
        195                 200                 205

Pro Leu Val Val Lys Val Glu Glu Gly Asp Asn Ala Val Leu Gln Cys
    210                 215                 220

Leu Lys Gly Thr Ser Asp Gly Pro Thr Gln Gln Leu Thr Trp Ser Arg
225                 230                 235                 240

Glu Ser Pro Leu Lys Pro Phe Leu Lys Leu Ser Leu Gly Leu Pro Gly
                245                 250                 255

Leu Gly Ile His Met Arg Pro Leu Ala Ile Trp Leu Phe Ile Phe Asn
            260                 265                 270

Val Ser Gln Gln Met Gly Gly Phe Tyr Leu Cys Gln Pro Gly Pro Pro
        275                 280                 285

Ser Glu Lys Ala Trp Gln Pro Gly Trp Thr Val Asn Val Glu Gly Ser
    290                 295                 300

```
Gly Glu Leu Phe Arg Trp Asn Val Ser Asp Leu Gly Gly Leu Gly Cys
305                 310                 315                 320

Gly Leu Lys Asn Arg Ser Ser Glu Gly Pro Ser Ser Pro Ser Gly Lys
                325                 330                 335

Leu Met Ser Pro Lys Leu Tyr Val Trp Ala Lys Asp Arg Pro Glu Ile
            340                 345                 350

Trp Glu Gly Glu Pro Pro Cys Leu Pro Pro Arg Asp Ser Leu Asn Gln
        355                 360                 365

Ser Leu Ser Gln Asp Leu Thr Met Ala Pro Gly Ser Thr Leu Trp Leu
    370                 375                 380

Ser Cys Gly Val Pro Pro Asp Ser Val Ser Arg Gly Pro Leu Ser Trp
385                 390                 395                 400

Thr His Val His Pro Lys Gly Pro Lys Ser Leu Leu Ser Leu Glu Leu
                405                 410                 415

Lys Asp Asp Arg Pro Ala Arg Asp Met Trp Val Met Glu Thr Gly Leu
            420                 425                 430

Leu Leu Pro Arg Ala Thr Ala Gln Asp Ala Gly Lys Tyr Tyr Cys His
        435                 440                 445

Arg Gly Asn Leu Thr Met Ser Phe His Leu Glu Ile Thr Ala Arg Pro
    450                 455                 460

Val Leu Trp His Trp Leu Leu Arg Thr Gly Gly Trp Lys Val Asp Glu
465                 470                 475                 480

Gln Lys Leu Ile Ser Glu Glu Asp Leu Asn Ala Val Gly Gln Asp Thr
                485                 490                 495

Gln Glu Val Ile Val Val Pro His Ser Leu Pro Phe Lys Val Val Val
            500                 505                 510

Ile Ser Ala Ile Leu Ala Leu Val Val Leu Thr Ile Ile Ser Leu Ile
        515                 520                 525

Ile Leu Ile Met Leu Trp Gln Lys Lys Pro Arg
    530                 535
```

<210> 152
<211> 540

<212> PRT
<213> Artificial Sequence

<220>
<223> SNAP tag cynomolgus CD19 ECD- PDGFR

<400> 152

```
Pro Ala Ala Ala Ser Gly Ile Asp Tyr Lys Asp Asp Asp Asp Lys Ala
1               5                   10                  15

Gly Ile Asp Ala Ile Met Asp Lys Asp Cys Glu Met Lys Arg Thr Thr
            20                  25                  30

Leu Asp Ser Pro Leu Gly Lys Leu Glu Leu Ser Gly Cys Glu Gln Gly
        35                  40                  45

Leu His Glu Ile Lys Leu Leu Gly Lys Gly Thr Ser Ala Ala Asp Ala
    50                  55                  60

Val Glu Val Pro Ala Pro Ala Ala Val Leu Gly Gly Pro Glu Pro Leu
65                  70                  75                  80

Met Gln Ala Thr Ala Trp Leu Asn Ala Tyr Phe His Gln Pro Glu Ala
                85                  90                  95

Ile Glu Glu Phe Pro Val Pro Ala Leu His His Pro Val Phe Gln Gln
            100                 105                 110

Glu Ser Phe Thr Arg Gln Val Leu Trp Lys Leu Leu Lys Val Val Lys
        115                 120                 125

Phe Gly Glu Val Ile Ser Tyr Gln Gln Leu Ala Ala Leu Ala Gly Asn
    130                 135                 140

Pro Ala Ala Thr Ala Ala Val Lys Thr Ala Leu Ser Gly Asn Pro Val
145                 150                 155                 160

Pro Ile Leu Ile Pro Cys His Arg Val Val Ser Ser Ser Gly Ala Val
                165                 170                 175

Gly Gly Tyr Glu Gly Gly Leu Ala Val Lys Glu Trp Leu Leu Ala His
            180                 185                 190

Glu Gly His Arg Leu Gly Lys Pro Gly Leu Gly Asp Ile Pro Gln Glu
        195                 200                 205

Pro Leu Val Val Lys Val Glu Glu Gly Asp Asn Ala Val Leu Gln Cys
    210                 215                 220
```

Leu Glu Gly Thr Ser Asp Gly Pro Thr Gln Gln Leu Val Trp Cys Arg
225 230 235 240

Asp Ser Pro Phe Glu Pro Phe Leu Asn Leu Ser Leu Gly Leu Pro Gly
245 250 255

Met Gly Ile Arg Met Gly Pro Leu Gly Ile Trp Leu Leu Ile Phe Asn
260 265 270

Val Ser Asn Gln Thr Gly Gly Phe Tyr Leu Cys Gln Pro Gly Leu Pro
275 280 285

Ser Glu Lys Ala Trp Gln Pro Gly Trp Thr Val Ser Val Glu Gly Ser
290 295 300

Gly Glu Leu Phe Arg Trp Asn Val Ser Asp Leu Gly Gly Leu Gly Cys
305 310 315 320

Gly Leu Lys Asn Arg Ser Ser Glu Gly Pro Ser Ser Pro Ser Gly Lys
325 330 335

Leu Asn Ser Ser Gln Leu Tyr Val Trp Ala Lys Asp Arg Pro Glu Met
340 345 350

Trp Glu Gly Glu Pro Val Cys Gly Pro Pro Arg Asp Ser Leu Asn Gln
355 360 365

Ser Leu Ser Gln Asp Leu Thr Met Ala Pro Gly Ser Thr Leu Trp Leu
370 375 380

Ser Cys Gly Val Pro Pro Asp Ser Val Ser Arg Gly Pro Leu Ser Trp
385 390 395 400

Thr His Val Arg Pro Lys Gly Pro Lys Ser Ser Leu Leu Ser Leu Glu
405 410 415

Leu Lys Asp Asp Arg Pro Asp Arg Asp Met Trp Val Val Asp Thr Gly
420 425 430

Leu Leu Leu Thr Arg Ala Thr Ala Gln Asp Ala Gly Lys Tyr Tyr Cys
435 440 445

His Arg Gly Asn Trp Thr Lys Ser Phe Tyr Leu Glu Ile Thr Ala Arg
450 455 460

Pro Ala Leu Trp His Trp Leu Leu Arg Ile Gly Gly Trp Lys Val Asp
465 470 475 480

```
Glu Gln Lys Leu Ile Ser Glu Glu Asp Leu Asn Ala Val Gly Gln Asp
                485                 490                 495

Thr Gln Glu Val Ile Val Val Pro His Ser Leu Pro Phe Lys Val Val
            500                 505                 510

Val Ile Ser Ala Ile Leu Ala Leu Val Val Leu Thr Ile Ile Ser Leu
        515                 520                 525

Ile Ile Leu Ile Met Leu Trp Gln Lys Lys Pro Arg
    530                 535                 540
```

<210> 153
<211> 1914
<212> DNA
<213> Artificial Sequence

<220>
<223> nucleotide sequence of anti- CEA(T84.66-LCHA) Fc knob monomeric 4-1BB (71-248) ligand

<400> 153

```
caggtgcagc tggtgcagtc tggcgccgaa gtgaagaaac ccggcagcag cgtgaaggtg      60
tcctgcaagg ccagcggctt caacatcaag gacacctaca tgcactgggt gcgccaggcc     120
cctggacagg gactggaatg gatgggcaga atcgaccccg ccaacggcaa cagcaaatac     180
gtgcccaagt tccagggcag agtgaccatc accgccgaca ccagcacctc caccgcctac     240
atggaactga gcagcctgcg gagcgaggac accgccgtgt actactgtgc cccctcggc      300
tactacgtgt ccgactacgc catggcctat tggggccagg gcacactcgt gaccgtgtcc     360
tctgctagca ccaagggccc atcggtcttc cccctggcac cctcctccaa gagcacctct     420
gggggcacag cggccctggg ctgcctggtc aaggactact tccccgaacc ggtgacggtg     480
tcgtggaact caggcgccct gaccagcggc gtgcacacct tcccggctgt cctacagtcc     540
tcaggactct actccctcag cagcgtggtg accgtgccct ccagcagctt gggcacccag     600
acctacatct gcaacgtgaa tcacaagccc agcaacacca aggtggacaa gaaagttgag     660
cccaaatctt gtgacaaaac tcacacatgc ccaccgtgcc cagcacctga agctgcaggg     720
ggaccgtcag tcttcctctt ccccccaaaa cccaaggaca ccctcatgat ctcccggacc     780
cctgaggtca catgcgtggt ggtggacgtg agccacgaag accctgaggt caagttcaac     840
tggtacgtgg acggcgtgga ggtgcataat gccaagacaa agccgcggga ggagcagtac     900
aacagcacgt accgtgtggt cagcgtcctc accgtcctgc accaggactg gctgaatggc     960
aaggagtaca agtgcaaggt ctccaacaaa gccctcggcg cccccatcga gaaaaccatc    1020
tccaaagcca aagggcagcc ccgagaacca caggtgtaca ccctgccccc ctgcagagat    1080
```

gagctgacca agaaccaggt gtccctgtgg tgtctggtca agggcttcta ccccagcgat 1140

atcgccgtgg agtgggagag caacggccag cctgagaaca actacaagac cacccccct 1200

gtgctggaca gcgacggcag cttcttcctg tactccaaac tgaccgtgga caagagccgg 1260

tggcagcagg gcaacgtgtt cagctgcagc gtgatgcacg aggccctgca caaccactac 1320

acccagaagt ccctgagcct gagccccggc ggaggcggcg gaagcggagg aggaggatcc 1380

agagagggcc ctgagctgag ccctgatgat cctgccggac tgctggacct gcggcaggga 1440

atgtttgccc agctggtggc ccagaacgtg ctgctgatcg atggccccct gtcctggtac 1500

agcgatcctg gactggctgg cgtgtcactg acaggcggcc tgagctacaa agaggacacc 1560

aaagaactgg tggtggccaa ggccggcgtg tactacgtgt tctttcagct ggaactgcgg 1620

agagtggtgg ccggcgaagg atctggctct gtgtctctgg ccctgcatct gcagcctctg 1680

agatctgctg ctggcgccgc tgctctggca ctgacagtgg atctgcctcc tgccagcagc 1740

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttcctgccgg gctc 1914

<210> 154
<211> 654
<212> DNA
<213> Artificial Sequence

<220>
<223> nucleotide sequence of anti- CEA(T84.66-LCHA) light chain

<400> 154

gagatcgtgc tgacccagag ccctgccacc ctgtcactgt ctccaggcga gagagccacc 60

ctgagctgta gagccggcga gagcgtggac atcttcggcg tgggatttct gcactggtat 120

cagcagaagc ccggccaggc ccccagactg ctgatctaca gagccagcaa ccgggccaca 180

ggcatccccg ccagattttc tggctctggc agcggcaccg acttcaccct gacaatcagc 240

agcctggaac ccgaggactt cgccgtgtac tactgccagc agaccaacga ggacccctac 300

acctttggcc agggcaccaa gctggaaatc aagcgtacgg tggctgcacc atctgtcttc 360

atcttcccgc catctgatga gcagttgaaa tctggaactg cctctgttgt gtgcctgctg 420

aataacttct atcccagaga ggccaaagta cagtggaagg tggataacgc cctccaatcg 480

ggtaactccc aggagagtgt cacagagcag gacagcaagg acagcaccta cagcctcagc 540

agcaccctga cgctgagcaa agcagactac gagaaacaca aagtctacgc ctgcgaagtc 600

acccatcagg gcctgagctc gcccgtcaca aagagcttca acaggggaga gtgt 654

<210> 155
<211> 638
<212> PRT
<213> Artificial Sequence

<220>
<223> anti- CEA (T84.66-LCHA) Fc knob monomeric 4-1-BB (71-248) ligand

<400> 155

```
Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220
```

Asp Lys Thr His Thr Cys Pro Pro Cys Pro Ala Pro Glu Ala Ala Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Tyr Thr Leu Pro Pro Cys Arg Asp Glu Leu Thr Lys Asn Gln Val Ser
355 360 365

Leu Trp Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly

465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu
625 630 635

<210> 156
<211> 218
<212> PRT
<213> Artificial Sequence

<220>
<223> anti- CEA(T84.66-LCHA) light chain

<400> 156

```
Glu Ile Val Leu Thr Gln Ser Pro Ala Thr Leu Ser Leu Ser Pro Gly
1               5                   10                  15

Glu Arg Ala Thr Leu Ser Cys Arg Ala Gly Glu Ser Val Asp Ile Phe
            20                  25                  30

Gly Val Gly Phe Leu His Trp Tyr Gln Gln Lys Pro Gly Gln Ala Pro
        35                  40                  45

Arg Leu Leu Ile Tyr Arg Ala Ser Asn Arg Ala Thr Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Thr Asn
                85                  90                  95

Glu Asp Pro Tyr Thr Phe Gly Gln Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 157
<211> 2448
<212> DNA
<213> Artificial Sequence

<220>

<223> nucleotide sequence of anti- CEA(T84.66-LCHA) Fc knob dimeric 4-1BB (71-248) ligand

<400> 157

```
caggtgcagc tggtgcagtc tggcgccgaa gtgaagaaac ccggcagcag cgtgaaggtg      60
tcctgcaagg ccagcggctt caacatcaag gacacctaca tgcactgggt gcgccaggcc     120
cctggacagg gactggaatg gatgggcaga atcgaccccg ccaacggcaa cagcaaatac     180
gtgcccaagt tccagggcag agtgaccatc accgccgaca ccagcacctc caccgcctac     240
```

atggaactga gcagcctgcg gagcgaggac accgccgtgt actactgtgc cccttcggc 300
tactacgtgt ccgactacgc catggcctat tggggccagg gcacactcgt gaccgtgtcc 360
tctgctagca ccaagggccc atcggtcttc cccctggcac cctcctccaa gagcacctct 420
gggggcacag cggccctggg ctgcctggtc aaggactact tccccgaacc ggtgacggtg 480
tcgtggaact caggcgccct gaccagcggc gtgcacacct tcccggctgt cctacagtcc 540
tcaggactct actccctcag cagcgtggtg accgtgccct ccagcagctt gggcacccag 600
acctacatct gcaacgtgaa tcacaagccc agcaacacca aggtggacaa gaaagttgag 660
cccaaatctt gtgacaaaac tcacacatgc ccaccgtgcc cagcacctga agctgcaggg 720
ggaccgtcag tcttcctctt ccccccaaaa cccaaggaca ccctcatgat ctcccggacc 780
cctgaggtca catgcgtggt ggtggacgtg agccacgaag accctgaggt caagttcaac 840
tggtacgtgg acggcgtgga ggtgcataat gccaagacaa agccgcggga ggagcagtac 900
aacagcacgt accgtgtggt cagcgtcctc accgtcctgc accaggactg gctgaatggc 960
aaggagtaca agtgcaaggt ctccaacaaa gccctcggcg cccccatcga gaaaaccatc 1020
tccaaagcca aagggcagcc ccgagaacca caggtgtaca ccctgccccc ctgcagagat 1080
gagctgacca agaaccaggt gtccctgtgg tgtctggtca agggcttcta ccccagcgat 1140
atcgccgtgg agtgggagag caacggccag cctgagaaca actacaagac caccccccct 1200
gtgctggaca gcgacggcag cttcttcctg tactccaaac tgaccgtgga caagagccgg 1260
tggcagcagg gcaacgtgtt cagctgcagc gtgatgcacg aggccctgca caaccactac 1320
acccagaagt ccctgagcct gagccccggc agagagggcc ctgagctgag ccctgatgat 1380
cctgccggac tgctggacct gcggcaggga atgtttgccc agctggtggc ccagaacgtg 1440
ctgctgatcg atggccccct gtcctggtac agcgatcctg gactggctgg cgtgtcactg 1500
acaggcggcc tgagctacaa agaggacacc aaagaactgg tggtggccaa ggccggcgtg 1560
tactacgtgt tctttcagct ggaactgcgg agagtggtgg ccggcgaagg atctggctct 1620
gtgtctctgg ccctgcatct gcagcctctg agatctgctg ctggcgccgc tgctctggca 1680
ctgacagtgg atctgcctcc tgccagcagc gaggcccgga atagcgcatt tgggtttcaa 1740
ggcaggctgc tgcacctgtc tgccggccag aggctgggag tgcatctgca cacagaggcc 1800
agggctagac acgcctggca gctgacacag ggcgctacag tgctgggcct gttcagagtg 1860
acccccgaga ttccagcagg cctgggaggc ggcggatctg gcggcggagg atctagagaa 1920
ggacccgagc tgtcccccga cgatcccgct gggctgctgg atctgagaca gggcatgttc 1980
gctcagctgg tggctcagaa tgtgctgctg attgacggac ctctgagctg gtactccgac 2040
ccagggctgg caggggtgtc cctgactggg ggactgtcct acaaagaaga tacaaaagaa 2100

ctggtggtgg ctaaagctgg ggtgtactat gtgtttttc agctggaact gaggcgggtg 2160

gtggctgggg agggctcagg atctgtgtcc ctggctctgc atctgcagcc actgcgctct 2220

gcagcagggg ctgcagcact ggccctgact gtggacctgc cccagcttc ttccgaggcc 2280

agaaacagcg ccttcgggtt ccaaggacgc ctgctgcatc tgagcgccgg acagcgcctg 2340

ggagtgcatc tgcatactga agccagagcc cggcatgctt ggcagctgac tcagggggca 2400

actgtgctgg gactgtttcg cgtgacacct gagatcccag ccgggctc 2448

<210> 158
<211> 816
<212> PRT
<213> Artificial Sequence

<220>
<223> anti- CEA(T84.66-LCHA) Fc knob dimeric 4-1BB (71-248) ligand

<400> 158

Gln Val Gln Leu Val Gln Ser Gly Ala Glu Val Lys Lys Pro Gly Ser
1 5 10 15

Ser Val Lys Val Ser Cys Lys Ala Ser Gly Phe Asn Ile Lys Asp Thr
20 25 30

Tyr Met His Trp Val Arg Gln Ala Pro Gly Gln Gly Leu Glu Trp Met
35 40 45

Gly Arg Ile Asp Pro Ala Asn Gly Asn Ser Lys Tyr Val Pro Lys Phe
50 55 60

Gln Gly Arg Val Thr Ile Thr Ala Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Pro Phe Gly Tyr Tyr Val Ser Asp Tyr Ala Met Ala Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Cys Arg Asp Glu Leu Thr Lys Asn Gln Val Ser
        355                 360                 365

Leu Trp Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415
```

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Arg Glu Gly Pro Glu Leu Ser Pro Asp Asp Pro Ala Gly Leu
450 455 460

Leu Asp Leu Arg Gln Gly Met Phe Ala Gln Leu Val Ala Gln Asn Val
465 470 475 480

Leu Leu Ile Asp Gly Pro Leu Ser Trp Tyr Ser Asp Pro Gly Leu Ala
485 490 495

Gly Val Ser Leu Thr Gly Gly Leu Ser Tyr Lys Glu Asp Thr Lys Glu
500 505 510

Leu Val Val Ala Lys Ala Gly Val Tyr Tyr Val Phe Phe Gln Leu Glu
515 520 525

Leu Arg Arg Val Val Ala Gly Glu Gly Ser Gly Ser Val Ser Leu Ala
530 535 540

Leu His Leu Gln Pro Leu Arg Ser Ala Ala Gly Ala Ala Ala Leu Ala
545 550 555 560

Leu Thr Val Asp Leu Pro Pro Ala Ser Ser Glu Ala Arg Asn Ser Ala
565 570 575

Phe Gly Phe Gln Gly Arg Leu Leu His Leu Ser Ala Gly Gln Arg Leu
580 585 590

Gly Val His Leu His Thr Glu Ala Arg Ala Arg His Ala Trp Gln Leu
595 600 605

Thr Gln Gly Ala Thr Val Leu Gly Leu Phe Arg Val Thr Pro Glu Ile
610 615 620

Pro Ala Gly Leu Gly Gly Gly Gly Ser Gly Gly Gly Gly Ser Arg Glu
625 630 635 640

Gly Pro Glu Leu Ser Pro Asp Asp Pro Ala Gly Leu Leu Asp Leu Arg
645 650 655

Gln Gly Met Phe Ala Gln Leu Val Ala Gln Asn Val Leu Leu Ile Asp
660 665 670

```
Gly Pro Leu Ser Trp Tyr Ser Asp Pro Gly Leu Ala Gly Val Ser Leu
        675                 680                 685

Thr Gly Gly Leu Ser Tyr Lys Glu Asp Thr Lys Glu Leu Val Val Ala
    690                 695                 700

Lys Ala Gly Val Tyr Tyr Val Phe Phe Gln Leu Glu Leu Arg Arg Val
705                 710                 715                 720

Val Ala Gly Glu Gly Ser Gly Ser Val Ser Leu Ala Leu His Leu Gln
                725                 730                 735

Pro Leu Arg Ser Ala Ala Gly Ala Ala Ala Leu Ala Leu Thr Val Asp
            740                 745                 750

Leu Pro Pro Ala Ser Ser Glu Ala Arg Asn Ser Ala Phe Gly Phe Gln
        755                 760                 765

Gly Arg Leu Leu His Leu Ser Ala Gly Gln Arg Leu Gly Val His Leu
    770                 775                 780

His Thr Glu Ala Arg Ala Arg His Ala Trp Gln Leu Thr Gln Gly Ala
785                 790                 795                 800

Thr Val Leu Gly Leu Phe Arg Val Thr Pro Glu Ile Pro Ala Gly Leu
                805                 810                 815
```

<210> 159
<211> 140
<212> PRT
<213> Artificial Sequence

<220>
<223> anti-mu CEA T84.66 VH

<400> 159

```
Met Lys Cys Ser Trp Val Ile Phe Phe Leu Met Ala Val Val Thr Gly
1               5                   10                  15

Val Asn Ser Glu Val Gln Leu Gln Gln Ser Gly Ala Glu Leu Val Glu
            20                  25                  30

Pro Gly Ala Ser Val Lys Leu Ser Cys Thr Ala Ser Gly Phe Asn Ile
        35                  40                  45

Lys Asp Thr Tyr Met His Trp Val Lys Gln Arg Pro Glu Gln Gly Leu
    50                  55                  60
```

Glu Trp Ile Gly Arg Ile Asp Pro Ala Asn Gly Asn Ser Lys Tyr Val
65 70 75 80

Pro Lys Phe Gln Gly Lys Ala Thr Ile Thr Ala Asp Thr Ser Ser Asn
85 90 95

Thr Ala Tyr Leu Gln Leu Thr Ser Leu Thr Ser Glu Asp Thr Ala Val
100 105 110

Tyr Tyr Cys Ala Pro Phe Gly Tyr Tyr Val Ser Asp Tyr Ala Met Ala
115 120 125

Tyr Trp Gly Gln Gly Thr Ser Val Thr Val Ser Ser
130 135 140

<210> 160
<211> 131
<212> PRT
<213> Artificial Sequence

<220>
<223> anti-mu CEA T84.66 VL

<400> 160

Met Glu Thr Asp Thr Leu Leu Leu Trp Val Leu Leu Leu Trp Val Pro
1 5 10 15

Gly Ser Thr Gly Asp Ile Val Leu Thr Gln Ser Pro Ala Ser Leu Ala
20 25 30

Val Ser Leu Gly Gln Arg Ala Thr Met Ser Cys Arg Ala Gly Glu Ser
35 40 45

Val Asp Ile Phe Gly Val Gly Phe Leu His Trp Tyr Gln Gln Lys Pro
50 55 60

Gly Gln Pro Pro Lys Leu Leu Ile Tyr Arg Ala Ser Asn Leu Glu Ser
65 70 75 80

Gly Ile Pro Val Arg Phe Ser Gly Thr Gly Ser Arg Thr Asp Phe Thr
85 90 95

Leu Ile Ile Asp Pro Val Glu Ala Asp Asp Val Ala Thr Tyr Tyr Cys
100 105 110

Gln Gln Thr Asn Glu Asp Pro Tyr Thr Phe Gly Gly Gly Thr Lys Leu
115 120 125

Glu Ile Lys
130

<210> 161
<211> 392
<212> DNA
<213> Artificial Sequence

<220>
<223> heavy chain framework acceptor sequence

<400> 161

taaggggctt cctagtccta aggctgagga agggatcctg gtttagttaa agaggatttt 60

attcacccct gtgtcctctc cacaggtgtc cagtcccagg tccagctggt gcaatctggg 120

gctgaggtga agaagcctgg gtcctcggtg aaggtctcct gcaaggcttc tggaggcacc 180

ttcagcagct atactatcag ctgggtgcga caggcccctg gacaagggct tgagtggatg 240

ggaaggatca tccctatcct tggtacagca aactacgcac agaagttcca gggcagagtc 300

acgattaccg cggacaaatc cacgagcaca gcctacatgg agctgagcag cctgagatct 360

gaggacacgg ccgtgtatta ctgtgcgaga ga 392

<210> 162
<211> 797
<212> DNA
<213> Artificial Sequence

<220>
<223> light chain framework acceptor sequence

<400> 162

```
ctgcagctgg aagctcagct cccacccagc tgctttgcat gtccctccca gctgccctac        60
cttccagagc ccatatcaat gcctgtgtca gagccctggg gaggaactgc tcagttagga       120
cccagaggga accatggaag ccccagctca gcttctcttc ctcctgctac tctggctccc       180
aggtgagggg aacatgaggt ggttttgcac attagtgaaa actcttgcca cctctgctca       240
gcaagaaata taattaaaat tcaaagtata tcaacaattt tggctctact caaagacagt       300
tggtttgatc ttgattacat gagtgcattt ctgttttatt tccaatttca gataccaccg       360
gagaaattgt gttgacacag tctccagcca ccctgtcttt gtctccaggg gaaagagcca       420
ccctctcctg cagggccagt cagagtgtta gcagctactt agcctggtac caacagaaac       480
ctggccaggc tcccaggctc ctcatctatg atgcatccaa cagggccact ggcatcccag       540
ccaggttcag tggcagtggg tctgggacag acttcactct caccatcagc agcctagagc       600
ctgaagattt tgcagtttat tactgtcagc agcgtagcaa ctggcctccc acagtgattc       660
cacatgaaac aaaaaccccca acaagaccat cagtgtttac tagattatta taccagctgc      720
ttcctttaca gacagctagt ggggtggcca ctcagtgtta gcatctcagc tctatttggc       780

cattttggag ttcaagt                                                      797
```

<210> 163
<211> 121
<212> PRT
<213> Artificial Sequence

<220>
<223> Parental CEA binder VH

<400> 163

```
Glu Val Gln Leu Gln Gln Ser Gly Ala Glu Leu Val Glu Pro Gly Ala
1               5                   10                  15

Ser Val Lys Leu Ser Cys Thr Ala Ser Gly Phe Asn Ile Lys Asp Thr
            20                  25                  30

Tyr Met His Trp Val Lys Gln Arg Pro Glu Gln Gly Leu Glu Trp Ile
        35                  40                  45

Gly Arg Ile Asp Pro Ala Asn Gly Asn Ser Lys Tyr Val Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Thr Ser Leu Thr Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Ser Val Thr Val Ser Ser
        115                 120
```

<210> 164
<211> 111
<212> PRT
<213> Artificial Sequence

<220>
<223> Parental CEA binder VL

<400> 164

```
Asp Ile Val Leu Thr Gln Ser Pro Ala Ser Leu Ala Val Ser Leu Gly
1               5                   10                  15

Gln Arg Ala Thr Met Ser Cys Arg Ala Gly Glu Ser Val Asp Ile Phe
            20                  25                  30
```

```
Gly Val Gly Phe Leu His Trp Tyr Gln Gln Lys Pro Gly Gln Pro Pro
        35                  40                  45

Lys Leu Leu Ile Tyr Arg Ala Ser Asn Leu Glu Ser Gly Ile Pro Val
    50                  55                  60

Arg Phe Ser Gly Thr Gly Ser Arg Thr Asp Phe Thr Leu Ile Ile Asp
65                  70                  75                  80

Pro Val Glu Ala Asp Asp Val Ala Thr Tyr Tyr Cys Gln Gln Thr Asn
                85                  90                  95

Glu Asp Pro Tyr Thr Phe Gly Gly Gly Thr Lys Leu Glu Ile Lys
            100                 105                 110
```

<210> 165
<211> 1896
<212> DNA
<213> Artificial Sequence

<220>
<223> nucleotide sequence of DP47 Fc knob monomeric 4-1BB (71-248) ligand

<400> 165

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagtgctag caccaagggc     360
ccatcggtct tccccctggc accctcctcc aagagcacct ctgggggcac agcggccctg     420
ggctgcctgg tcaaggacta cttccccgaa ccggtgacgg tgtcgtggaa ctcaggcgcc     480
ctgaccagcg gcgtgcacac cttcccggct gtcctacagt cctcaggact ctactccctc     540
agcagcgtgg tgaccgtgcc ctccagcagc ttgggcaccc agacctacat ctgcaacgtg     600
aatcacaagc ccagcaacac caaggtggac aagaaagttg agcccaaatc ttgtgacaaa     660
actcacacat gcccaccgtg cccagcacct gaagctgcag gggggaccgtc agtcttcctc    720
ttccccccaa aacccaagga caccctcatg atctcccgga cccctgaggt cacatgcgtg     780
gtggtggacg tgagccacga agaccctgag gtcaagttca actggtacgt ggacggcgtg     840
gaggtgcata atgccaagac aaagccgcgg gaggagcagt acaacagcac gtaccgtgtg     900
gtcagcgtcc tcaccgtcct gcaccaggac tggctgaatg gcaaggagta caagtgcaag     960
gtctccaaca aagccctcgg cgcccccatc gagaaaacca tctccaaagc caaagggcag    1020
```

ccccgagaac cacaggtgta caccctgccc ccctgcagag atgagctgac caagaaccag 1080
gtgtccctgt ggtgtctggt caagggcttc taccccagcg atatcgccgt ggagtgggag 1140
agcaacggcc agcctgagaa caactacaag accacccccc ctgtgctgga cagcgacggc 1200
agcttcttcc tgtactccaa actgaccgtg gacaagagcc ggtggcagca gggcaacgtg 1260
ttcagctgca gcgtgatgca cgaggccctg cacaaccact acacccagaa gtccctgagc 1320
ctgagccccg gcggaggcgg cggaagcgga ggaggaggat ccagagaggg ccctgagctg 1380
agccctgatg atcctgccgg actgctggac ctgcggcagg gaatgtttgc ccagctggtg 1440
gcccagaacg tgctgctgat cgatggcccc ctgtcctggt acagcgatcc tggactggct 1500
ggcgtgtcac tgacaggcgg cctgagctac aaagaggaca ccaaagaact ggtggtggcc 1560
aaggccggcg tgtactacgt gttctttcag ctggaactgc ggagagtggt ggccggcgaa 1620
ggatctggct ctgtgtctct ggccctgcat ctgcagcctc tgagatctgc tgctggcgcc 1680
gctgctctgg cactgacagt ggatctgcct cctgccagca gcgaggcccg gaatagcgca 1740
tttgggtttc aaggcaggct gctgcacctg tctgccggcc agaggctggg agtgcatctg 1800
cacacagagg ccagggctag acacgcctgg cagctgacac agggcgctac agtgctgggc 1860
ctgttcagag tgacccccga gattcctgcc gggctc 1896

<210> 166
<211> 645
<212> DNA
<213> Artificial Sequence

<220>
<223> nucleotide sequence of DP47 light chain

<400> 166

```
gaaatcgtgt taacgcagtc tccaggcacc ctgtctttgt ctccagggga aagagccacc      60
ctctcttgca gggccagtca gagtgttagc agcagctact tagcctggta ccagcagaaa     120
cctggccagg ctcccaggct cctcatctat ggagcatcca gcagggccac tggcatccca     180
gacaggttca gtggcagtgg atccgggaca gacttcactc tcaccatcag cagactggag     240
cctgaagatt ttgcagtgta ttactgtcag cagtatggta gctcaccgct gacgttcggc     300
caggggacca aagtggaaat caaacgtacg gtggctgcac catctgtctt catcttcccg     360
ccatctgatg agcagttgaa atctggaact gcctctgttg tgtgcctgct gaataacttc     420
tatcccagag aggccaaagt acagtggaag gtggataacg ccctccaatc gggtaactcc     480
caggagagtg tcacagagca ggacagcaag gacagcacct acagcctcag cagcaccctg     540
acgctgagca aagcagacta cgagaaacac aaagtctacg cctgcgaagt cacccatcag     600
ggcctgagct cgcccgtcac aaagagcttc aacaggggag agtgt                     645
```

<210> 167
<211> 632
<212> PRT
<213> Artificial Sequence

<220>
<223> DP47 Fc knob monomeric 4-1-BB (71-248) ligand

<400> 167

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Gly Ala Pro Ile Glu Lys Thr Ile Ser Lys
                325                 330                 335

Ala Lys Gly Gln Pro Arg Glu Pro Gln Val Tyr Thr Leu Pro Pro Cys
            340                 345                 350

Arg Asp Glu Leu Thr Lys Asn Gln Val Ser Leu Trp Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Gly Gly Gly Gly
        435                 440                 445

Ser Gly Gly Gly Gly Ser Arg Glu Gly Pro Glu Leu Ser Pro Asp Asp
    450                 455                 460

Pro Ala Gly Leu Leu Asp Leu Arg Gln Gly Met Phe Ala Gln Leu Val
465                 470                 475                 480

Ala Gln Asn Val Leu Leu Ile Asp Gly Pro Leu Ser Trp Tyr Ser Asp
                485                 490                 495

Pro Gly Leu Ala Gly Val Ser Leu Thr Gly Gly Leu Ser Tyr Lys Glu
            500                 505                 510

Asp Thr Lys Glu Leu Val Val Ala Lys Ala Gly Val Tyr Tyr Val Phe
        515                 520                 525

Phe Gln Leu Glu Leu Arg Arg Val Val Ala Gly Glu Gly Ser Gly Ser
    530                 535                 540

Val Ser Leu Ala Leu His Leu Gln Pro Leu Arg Ser Ala Ala Gly Ala
545                 550                 555                 560

Ala Ala Leu Ala Leu Thr Val Asp Leu Pro Pro Ala Ser Ser Glu Ala
                565                 570                 575

Arg Asn Ser Ala Phe Gly Phe Gln Gly Arg Leu Leu His Leu Ser Ala
            580                 585                 590

Gly Gln Arg Leu Gly Val His Leu His Thr Glu Ala Arg Ala Arg His
        595                 600                 605

Ala Trp Gln Leu Thr Gln Gly Ala Thr Val Leu Gly Leu Phe Arg Val
    610                 615                 620

Thr Pro Glu Ile Pro Ala Gly Leu
625                 630

<210> 168
<211> 215
<212> PRT
<213> Artificial Sequence

<220>
<223> DP47 light chain

<400> 168

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

```
Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Leu Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 169
<211> 2430
<212> DNA
<213> Artificial Sequence

<220>
<223> nucleotide sequence of DP47 Fc knob dimeric 4-1BBL (71-248)

<400> 169

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
```

gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagtgctag caccaagggc 360
ccatcggtct tccccctggc accctcctcc aagagcacct ctgggggcac agcggccctg 420
ggctgcctgg tcaaggacta cttccccgaa ccggtgacgg tgtcgtggaa ctcaggcgcc 480
ctgaccagcg gcgtgcacac cttcccggct gtcctacagt cctcaggact ctactccctc 540
agcagcgtgg tgaccgtgcc ctccagcagc ttgggcaccc agacctacat ctgcaacgtg 600
aatcacaagc ccagcaacac caaggtggac aagaaagttg agcccaaatc ttgtgacaaa 660
actcacacat gcccaccgtg cccagcacct gaagctgcag gggggaccgtc agtcttcctc 720
ttccccccaa aacccaagga caccctcatg atctcccgga cccctgaggt cacatgcgtg 780
gtggtggacg tgagccacga agaccctgag gtcaagttca actggtacgt ggacggcgtg 840
gaggtgcata atgccaagac aaagccgcgg gaggagcagt acaacagcac gtaccgtgtg 900
gtcagcgtcc tcaccgtcct gcaccaggac tggctgaatg gcaaggagta caagtgcaag 960
gtctccaaca aagccctcgg cgcccccatc gagaaaacca tctccaaagc caaagggcag 1020
ccccgagaac cacaggtgta caccctgccc ccctgcagag atgagctgac caagaaccag 1080
gtgtccctgt ggtgtctggt caagggcttc taccccagcg atatcgccgt ggagtgggag 1140
agcaacggcc agcctgagaa caactacaag accacccccc ctgtgctgga cagcgacggc 1200
agcttcttcc tgtactccaa actgaccgtg gacaagagcc ggtggcagca gggcaacgtg 1260
ttcagctgca gcgtgatgca cgaggccctg cacaaccact acacccagaa gtccctgagc 1320
ctgagccccg gcagagaggg ccctgagctg agccctgatg atcctgccgg actgctggac 1380
ctgcggcagg gaatgtttgc ccagctggtg gcccagaacg tgctgctgat cgatggcccc 1440
ctgtcctggt acagcgatcc tggactggct ggcgtgtcac tgacaggcgg cctgagctac 1500
aaagaggaca ccaaagaact ggtggtggcc aaggccggcg tgtactacgt gttctttcag 1560
ctggaactgc ggagagtggt ggccggcgaa ggatctggct ctgtgtctct ggccctgcat 1620
ctgcagcctc tgagatctgc tgctggcgcc gctgctctgg cactgacagt ggatctgcct 1680
cctgccagca gcgaggcccg gaatagcgca tttgggtttc aaggcaggct gctgcacctg 1740
tctgccggcc agaggctggg agtgcatctg cacacagagg ccagggctag acacgcctgg 1800
cagctgacac agggcgctac agtgctgggc ctgttcagag tgacccccga gattccagca 1860
ggcctgggag gcggcggatc tggcggcgga ggatctagag aaggacccga gctgtccccc 1920
gacgatcccg ctgggctgct ggatctgaga cagggcatgt tcgctcagct ggtggctcag 1980
aatgtgctgc tgattgacgg acctctgagc tggtactccg acccagggct ggcaggggtg 2040
tccctgactg ggggactgtc ctacaaagaa gatacaaaag aactggtggt ggctaaagct 2100

```
ggggtgtact atgtgttttt tcagctggaa ctgaggcggg tggtggctgg ggagggctca     2160

ggatctgtgt ccctggctct gcatctgcag ccactgcgct ctgcagcagg ggctgcagca     2220

ctggccctga ctgtggacct gcccccagct tcttccgagg ccagaaacag cgccttcggg     2280

ttccaaggac gcctgctgca tctgagcgcc ggacagcgcc tgggagtgca tctgcatact     2340

gaagccagag cccggcatgc ttggcagctg actcaggggg caactgtgct gggactgttt     2400

cgcgtgacac ctgagatccc agccgggctc                                      2430
```

<210> 170
<211> 810
<212> PRT
<213> Artificial Sequence

<220>
<223> DP47 Fc knob dimeric 4-1BBL (71-248)

<400> 170

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160
```

```
Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Gly Ala Pro Ile Glu Lys Thr Ile Ser Lys
                325                 330                 335

Ala Lys Gly Gln Pro Arg Glu Pro Gln Val Tyr Thr Leu Pro Pro Cys
            340                 345                 350

Arg Asp Glu Leu Thr Lys Asn Gln Val Ser Leu Trp Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415
```

Gln Gly Asn Val Phe Ser Cys Ser Val Met His Glu Ala Leu His Asn
420 425 430

His Tyr Thr Gln Lys Ser Leu Ser Leu Ser Pro Gly Arg Glu Gly Pro
435 440 445

Glu Leu Ser Pro Asp Asp Pro Ala Gly Leu Leu Asp Leu Arg Gln Gly
450 455 460

Met Phe Ala Gln Leu Val Ala Gln Asn Val Leu Leu Ile Asp Gly Pro
465 470 475 480

Leu Ser Trp Tyr Ser Asp Pro Gly Leu Ala Gly Val Ser Leu Thr Gly
485 490 495

Gly Leu Ser Tyr Lys Glu Asp Thr Lys Glu Leu Val Val Ala Lys Ala
500 505 510

Gly Val Tyr Tyr Val Phe Phe Gln Leu Glu Leu Arg Arg Val Val Ala
515 520 525

Gly Glu Gly Ser Gly Ser Val Ser Leu Ala Leu His Leu Gln Pro Leu
530 535 540

Arg Ser Ala Ala Gly Ala Ala Ala Leu Ala Leu Thr Val Asp Leu Pro
545 550 555 560

Pro Ala Ser Ser Glu Ala Arg Asn Ser Ala Phe Gly Phe Gln Gly Arg
565 570 575

Leu Leu His Leu Ser Ala Gly Gln Arg Leu Gly Val His Leu His Thr
580 585 590

Glu Ala Arg Ala Arg His Ala Trp Gln Leu Thr Gln Gly Ala Thr Val
595 600 605

Leu Gly Leu Phe Arg Val Thr Pro Glu Ile Pro Ala Gly Leu Gly Gly
610 615 620

Gly Gly Ser Gly Gly Gly Gly Ser Arg Glu Gly Pro Glu Leu Ser Pro
625 630 635 640

Asp Asp Pro Ala Gly Leu Leu Asp Leu Arg Gln Gly Met Phe Ala Gln
645 650 655

Leu Val Ala Gln Asn Val Leu Leu Ile Asp Gly Pro Leu Ser Trp Tyr

660 665 670

Ser Asp Pro Gly Leu Ala Gly Val Ser Leu Thr Gly Gly Leu Ser Tyr
675 680 685

Lys Glu Asp Thr Lys Glu Leu Val Val Ala Lys Ala Gly Val Tyr Tyr
690 695 700

Val Phe Phe Gln Leu Glu Leu Arg Arg Val Val Ala Gly Glu Gly Ser
705 710 715 720

Gly Ser Val Ser Leu Ala Leu His Leu Gln Pro Leu Arg Ser Ala Ala
725 730 735

Gly Ala Ala Ala Leu Ala Leu Thr Val Asp Leu Pro Pro Ala Ser Ser
740 745 750

Glu Ala Arg Asn Ser Ala Phe Gly Phe Gln Gly Arg Leu Leu His Leu
755 760 765

Ser Ala Gly Gln Arg Leu Gly Val His Leu His Thr Glu Ala Arg Ala
770 775 780

Arg His Ala Trp Gln Leu Thr Gln Gly Ala Thr Val Leu Gly Leu Phe
785 790 795 800

Arg Val Thr Pro Glu Ile Pro Ala Gly Leu
805 810

<210> 171
<211> 2466
<212> DNA
<213> Artificial Sequence

<220>
<223> nucleotide sequence of anti-FAP(4B9) Fc hole dimeric 4-1BB ligand (71-248) chain

<400> 171

```
gaggtgcagc tgctcgaaag cggcggagga ctggtgcagc ctggcggcag cctgagactg      60
tcttgcgccg ccagcggctt caccttcagc agctacgcca tgagctgggt ccgccaggcc     120
cctggcaagg gactggaatg ggtgtccgcc atcatcggct ctggcgccag cacctactac     180
gccgacagcg tgaagggccg gttcaccatc agccgggaca acagcaagaa caccctgtac     240
ctgcagatga acagcctgcg ggccgaggac accgccgtgt actactgcgc caagggatgg     300
ttcggcggct tcaactactg gggacagggc accctggtca cagtgtccag cgctagcacc     360
aagggcccct ccgtgttccc cctggccccc agcagcaaga gcaccagcgg cggcacagcc     420
```

gctctgggct gcctggtcaa ggactacttc cccgagcccg tgaccgtgtc ctggaacagc 480
ggagccctga cctccggcgt gcacaccttc cccgccgtgc tgcagagttc tggcctgtat 540
agcctgagca gcgtggtcac cgtgccttct agcagcctgg gcacccagac ctacatctgc 600
aacgtgaacc acaagcccag caacaccaag gtggacaaga aggtggagcc caagagctgc 660
gacaaaactc acacatgccc accgtgccca gcacctgaag ctgcaggggg accgtcagtc 720
ttcctcttcc ccccaaaacc caaggacacc ctcatgatct cccggacccc tgaggtcaca 780
tgcgtggtgg tggacgtgag ccacgaagac cctgaggtca agttcaactg gtacgtggac 840
ggcgtggagg tgcataatgc caagacaaag ccgcgggagg agcagtacaa cagcacgtac 900
cgtgtggtca gcgtcctcac cgtcctgcac caggactggc tgaatggcaa ggagtacaag 960
tgcaaggtct ccaacaaagc cctcggcgcc cccatcgaga aaaccatctc caaagccaaa 1020
gggcagcccc gagaaccaca ggtgtgcacc ctgcccccat cccgggatga gctgaccaag 1080
aaccaggtca gcctctcgtg cgcagtcaaa ggcttctatc ccagcgacat cgccgtggag 1140
tgggagagca atgggcagcc ggagaacaac tacaagacca cgcctcccgt gctggactcc 1200
gacggctcct tcttcctcgt gagcaagctc accgtggaca agagcaggtg gcagcagggg 1260
aacgtcttct catgctccgt gatgcatgag gctctgcaca accactacac gcagaagagc 1320
ctctccctgt ctccgggtgg aggcggcgga agcggaggag gaggatccag agagggccct 1380
gagctgagcc ctgatgatcc tgccggactg ctggacctgc ggcagggaat gtttgcccag 1440
ctggtggccc agaacgtgct gctgatcgat ggccccctgt cctggtacag cgatcctgga 1500
ctggctggcg tgtcactgac aggcggcctg agctacaaag aggacaccaa agaactggtg 1560
gtggccaagg ccggcgtgta ctacgtgttc tttcagctgg aactgcggag agtggtggcc 1620
ggcgaaggat ctggctctgt gtctctggcc ctgcatctgc agcctctgag atctgctgct 1680
ggcgccgctg ctctggcact gacagtggat ctgcctcctg ccagcagcga ggcccggaat 1740
agcgcatttg ggtttcaagg caggctgctg cacctgtctg ccggccagag gctgggagtg 1800
catctgcaca cagaggccag ggctagacac gcctggcagc tgacacaggg cgctacagtg 1860
ctgggcctgt tcagagtgac ccccgagatt ccagcaggcc tgggaggcgg cggatctggc 1920
ggcggaggat ctagagaagg acccgagctg tcccccgacg atcccgctgg gctgctggat 1980
ctgagacagg gcatgttcgc tcagctggtg gctcagaatg tgctgctgat tgacggacct 2040
ctgagctggt actccgaccc agggctggca ggggtgtccc tgactggggg actgtcctac 2100
aaagaagata caaaagaact ggtggtggct aaagctgggg tgtactatgt gtttttttcag 2160
ctggaactga ggcgggtggt ggctggggag ggctcaggat ctgtgtccct ggctctgcat 2220
ctgcagccac tgcgctctgc agcaggggct gcagcactgg ccctgactgt ggacctgccc 2280
ccagcttctt ccgaggccag aaacagcgcc ttcgggttcc aaggacgcct gctgcatctg 2340

```
agcgccggac agcgcctggg agtgcatctg catactgaag ccagagcccg gcatgcttgg     2400
cagctgactc agggggcaac tgtgctggga ctgtttcgcg tgacacctga gatcccagcc     2460
gggctc                                                                2466
```

<210> 172
<211> 1242
<212> DNA
<213> Artificial Sequence

<220>
<223> nucleotide sequence of Fc knob monomeric 4-1BB (71-248) ligand

<400> 172

```
gacaaaactc acacatgccc accgtgccca gcacctgaag ctgcaggggg accgtcagtc       60
ttcctcttcc ccccaaaacc caaggacacc ctcatgatct cccggacccc tgaggtcaca      120
tgcgtggtgg tggacgtgag ccacgaagac cctgaggtca agttcaactg gtacgtggac      180
ggcgtggagg tgcataatgc caagacaaag ccgcgggagg agcagtacaa cagcacgtac      240
cgtgtggtca gcgtcctcac cgtcctgcac caggactggc tgaatggcaa ggagtacaag      300
tgcaaggtct ccaacaaagc cctcggcgcc cccatcgaga aaaccatctc caaagccaaa      360
gggcagcccc gagaaccaca ggtgtacacc ctgcccccct gcagagatga gctgaccaag      420
aaccaggtgt ccctgtggtg tctggtcaag ggcttctacc ccagcgatat cgccgtggag      480
tgggagagca acggccagcc tgagaacaac tacaagacca cccccctgt gctggacagc       540
gacggcagct tcttcctgta ctccaaactg accgtggaca agagccggtg gcagcagggc      600
aacgtgttca gctgcagcgt gatgcacgag gccctgcaca accactacac ccagaagtcc      660
ctgagcctga gccccggcgg aggcggcgga agcggaggag gaggatccag agagggccct      720
gagctgagcc ctgatgatcc tgccggactg ctggacctgc ggcagggaat gtttgcccag      780
ctggtggccc agaacgtgct gctgatcgat ggccccctgt cctggtacag cgatcctgga      840
ctggctggcg tgtcactgac aggcggcctg agctacaaag aggacaccaa agaactggtg      900
gtggccaagg ccggcgtgta ctacgtgttc tttcagctgg aactgcggag agtggtggcc      960
ggcgaaggat ctggctctgt gtctctggcc ctgcatctgc agcctctgag atctgctgct     1020
ggcgccgctg ctctggcact gacagtggat ctgcctcctg ccagcagcga ggcccggaat     1080
agcgcatttg ggtttcaagg caggctgctg cacctgtctg ccggccagag gctgggagtg     1140
catctgcaca cagaggccag ggctagacac gcctggcagc tgacacaggg cgctacagtg     1200
ctgggcctgt tcagagtgac ccccgagatt cctgccgggc tc                        1242
```

<210> 173
<211> 822

<212> PRT
<213> Artificial Sequence

<220>
<223> anti-FAP(4B9) Fc hole dimeric 4-1BB (71-248) ligand chain

<400> 173

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Cys Thr Leu Pro
340 345 350

Pro Ser Arg Asp Glu Leu Thr Lys Asn Gln Val Ser Leu Ser Cys Ala
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Val Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Arg Glu Gly Pro Glu Leu Ser Pro
450 455 460

Asp Asp Pro Ala Gly Leu Leu Asp Leu Arg Gln Gly Met Phe Ala Gln
465 470 475 480

Leu Val Ala Gln Asn Val Leu Leu Ile Asp Gly Pro Leu Ser Trp Tyr
485 490 495

Ser Asp Pro Gly Leu Ala Gly Val Ser Leu Thr Gly Gly Leu Ser Tyr
500 505 510

Lys Glu Asp Thr Lys Glu Leu Val Val Ala Lys Ala Gly Val Tyr Tyr
515 520 525

Val Phe Phe Gln Leu Glu Leu Arg Arg Val Val Ala Gly Glu Gly Ser
530 535 540

Gly Ser Val Ser Leu Ala Leu His Leu Gln Pro Leu Arg Ser Ala Ala
545 550 555 560

Gly Ala Ala Ala Leu Ala Leu Thr Val Asp Leu Pro Pro Ala Ser Ser
565 570 575

Glu Ala Arg Asn Ser Ala Phe Gly Phe Gln Gly Arg Leu Leu His Leu
580 585 590

Ser Ala Gly Gln Arg Leu Gly Val His Leu His Thr Glu Ala Arg Ala
595 600 605

Arg His Ala Trp Gln Leu Thr Gln Gly Ala Thr Val Leu Gly Leu Phe
610 615 620

Arg Val Thr Pro Glu Ile Pro Ala Gly Leu Gly Gly Gly Gly Ser Gly
625 630 635 640

Gly Gly Gly Ser Arg Glu Gly Pro Glu Leu Ser Pro Asp Asp Pro Ala
645 650 655

Gly Leu Leu Asp Leu Arg Gln Gly Met Phe Ala Gln Leu Val Ala Gln
660 665 670

Asn Val Leu Leu Ile Asp Gly Pro Leu Ser Trp Tyr Ser Asp Pro Gly
675 680 685

Leu Ala Gly Val Ser Leu Thr Gly Gly Leu Ser Tyr Lys Glu Asp Thr
690 695 700

Lys Glu Leu Val Val Ala Lys Ala Gly Val Tyr Tyr Val Phe Phe Gln
705 710 715 720

Leu Glu Leu Arg Arg Val Val Ala Gly Glu Gly Ser Gly Ser Val Ser
725 730 735

Leu Ala Leu His Leu Gln Pro Leu Arg Ser Ala Ala Gly Ala Ala Ala
740 745 750

Leu Ala Leu Thr Val Asp Leu Pro Pro Ala Ser Ser Glu Ala Arg Asn
755 760 765

Ser Ala Phe Gly Phe Gln Gly Arg Leu Leu His Leu Ser Ala Gly Gln
770 775 780

Arg Leu Gly Val His Leu His Thr Glu Ala Arg Ala Arg His Ala Trp
785 790 795 800

Gln Leu Thr Gln Gly Ala Thr Val Leu Gly Leu Phe Arg Val Thr Pro
805 810 815

Glu Ile Pro Ala Gly Leu
820

<210> 174
<211> 414
<212> PRT
<213> Artificial Sequence

<220>
<223> Fc knob monomeric 41-BBL (71-248)

<400> 174

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Gly Ala Pro Ile
100 105 110

Glu Lys Thr Ile Ser Lys Ala Lys Gly Gln Pro Arg Glu Pro Gln Val
115 120 125

Tyr Thr Leu Pro Pro Cys Arg Asp Glu Leu Thr Lys Asn Gln Val Ser
130 135 140

Leu Trp Cys Leu Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Gly Gly Gly Gly Ser Gly Gly Gly Gly Ser Arg Glu Gly Pro
225 230 235 240

Glu Leu Ser Pro Asp Asp Pro Ala Gly Leu Leu Asp Leu Arg Gln Gly
245 250 255

Met Phe Ala Gln Leu Val Ala Gln Asn Val Leu Leu Ile Asp Gly Pro
260 265 270

Leu Ser Trp Tyr Ser Asp Pro Gly Leu Ala Gly Val Ser Leu Thr Gly
275 280 285

Gly Leu Ser Tyr Lys Glu Asp Thr Lys Glu Leu Val Val Ala Lys Ala
290 295 300

Gly Val Tyr Tyr Val Phe Phe Gln Leu Glu Leu Arg Arg Val Val Ala
305 310 315 320

Gly Glu Gly Ser Gly Ser Val Ser Leu Ala Leu His Leu Gln Pro Leu
325 330 335

Arg Ser Ala Ala Gly Ala Ala Ala Leu Ala Leu Thr Val Asp Leu Pro
340 345 350

Pro Ala Ser Ser Glu Ala Arg Asn Ser Ala Phe Gly Phe Gln Gly Arg
355 360 365

```
Leu Leu His Leu Ser Ala Gly Gln Arg Leu Gly Val His Leu His Thr
    370                 375                 380

Glu Ala Arg Ala Arg His Ala Trp Gln Leu Thr Gln Gly Ala Thr Val
385                 390                 395                 400

Leu Gly Leu Phe Arg Val Thr Pro Glu Ile Pro Ala Gly Leu
                405                 410
```

<210> 175
<211> 1902
<212> DNA
<213> Artificial Sequence

<220>
<223> nucleotide sequence of anti-FAP(4B9) Fc hole monomeric 4-1BBL (71-248) chain

<400> 175

```
gaggtgcagc tgctcgaaag cggcggagga ctggtgcagc ctggcggcag cctgagactg      60
tcttgcgccg ccagcggctt caccttcagc agctacgcca tgagctgggt ccgccaggcc     120
cctggcaagg gactggaatg ggtgtccgcc atcatcggct ctggcgccag cacctactac     180
gccgacagcg tgaagggccg gttcaccatc agccgggaca acagcaagaa caccctgtac     240
ctgcagatga acagcctgcg ggccgaggac accgccgtgt actactgcgc caagggatgg     300
ttcggcggct tcaactactg gggacagggc accctggtca cagtgtccag cgctagcacc     360
aagggcccct ccgtgttccc cctggccccc agcagcaaga gcaccagcgg cggcacagcc     420
gctctgggct gcctggtcaa ggactacttc cccgagcccg tgaccgtgtc ctggaacagc     480
ggagccctga cctccggcgt gcacaccttc cccgccgtgc tgcagagttc tggcctgtat     540
agcctgagca gcgtggtcac cgtgccttct agcagcctgg gcacccagac ctacatctgc     600
aacgtgaacc acaagcccag caacaccaag gtggacaaga aggtggagcc caagagctgc     660
gacaaaactc acacatgccc accgtgccca gcacctgaag ctgcaggggg accgtcagtc     720
ttcctcttcc ccccaaaacc caaggacacc ctcatgatct cccggacccc tgaggtcaca     780
tgcgtggtgg tggacgtgag ccacgaagac cctgaggtca agttcaactg gtacgtggac     840
ggcgtggagg tgcataatgc caagacaaag ccgcgggagg agcagtacaa cagcacgtac     900
cgtgtggtca gcgtcctcac cgtcctgcac caggactggc tgaatggcaa ggagtacaag     960
tgcaaggtct ccaacaaagc cctcggcgcc cccatcgaga aaaccatctc caaagccaaa    1020
gggcagcccc gagaaccaca ggtgtgcacc ctgcccccat cccgggatga gctgaccaag    1080
aaccaggtca gcctctcgtg cgcagtcaaa ggcttctatc ccagcgacat cgccgtggag    1140
tgggagagca atgggcagcc ggagaacaac tacaagacca cgcctcccgt gctggactcc    1200
```

gacggctcct tcttcctcgt gagcaagctc accgtggaca agagcaggtg gcagcagggg 1260

aacgtcttct catgctccgt gatgcatgag gctctgcaca accactacac gcagaagagc 1320

ctctccctgt ctccgggtgg aggcggcgga agcggaggag gaggatccag agagggccct 1380

gagctgagcc ctgatgatcc tgccggactg ctggacctgc ggcagggaat gtttgcccag 1440

ctggtggccc agaacgtgct gctgatcgat ggccccctgt cctggtacag cgatcctgga 1500

ctggctggcg tgtcactgac aggcggcctg agctacaaag aggacaccaa agaactggtg 1560

gtggccaagg ccggcgtgta ctacgtgttc tttcagctgg aactgcggag agtggtggcc 1620

ggcgaaggat ctggctctgt gtctctggcc ctgcatctgc agcctctgag atctgctgct 1680

ggcgccgctg ctctggcact gacagtggat ctgcctcctg ccagcagcga ggcccggaat 1740

agcgcatttg ggtttcaagg caggctgctg cacctgtctg ccggccagag gctgggagtg 1800

catctgcaca cagaggccag ggctagacac gcctggcagc tgacacaggg cgctacagtg 1860

ctgggcctgt tcagagtgac ccccgagatt cctgccgggc tc 1902

<210> 176
<211> 1806
<212> DNA
<213> Artificial Sequence

<220>
<223> nucleotide sequence of Fc knob dimeric 4-1BB ligand (71-248)

<400> 176

```
gacaaaactc acacatgccc accgtgccca gcacctgaag ctgcaggggg accgtcagtc        60
ttcctcttcc ccccaaaacc caaggacacc ctcatgatct cccggacccc tgaggtcaca       120
tgcgtggtgg tggacgtgag ccacgaagac cctgaggtca agttcaactg gtacgtggac       180
ggcgtggagg tgcataatgc caagacaaag ccgcgggagg agcagtacaa cagcacgtac       240
cgtgtggtca gcgtcctcac cgtcctgcac caggactggc tgaatggcaa ggagtacaag       300
tgcaaggtct ccaacaaagc cctcggcgcc cccatcgaga aaaccatctc caaagccaaa       360
gggcagcccc gagaaccaca ggtgtacacc ctgcccccct gcagagatga gctgaccaag       420
aaccaggtgt ccctgtggtg tctggtcaag ggcttctacc ccagcgatat cgccgtggag       480
tgggagagca acggccagcc tgagaacaac tacaagacca ccccccctgt gctggacagc       540
gacggcagct tcttcctgta ctccaaactg accgtggaca agagccggtg gcagcagggc       600
aacgtgttca gctgcagcgt gatgcacgag gccctgcaca accactacac ccagaagtcc       660
ctgagcctga gccccggcgg aggcggcgga agcggaggag gaggatccag agagggccct       720
gagctgagcc ctgatgatcc tgccggactg ctggacctgc ggcagggaat gtttgcccag       780
ctggtggccc agaacgtgct gctgatcgat ggccccctgt cctggtacag cgatcctgga       840
```

```
ctggctggcg tgtcactgac aggcggcctg agctacaaag aggacaccaa agaactggtg      900
gtggccaagg ccggcgtgta ctacgtgttc tttcagctgg aactgcggag agtggtggcc      960
ggcgaaggat ctggctctgt gtctctggcc ctgcatctgc agcctctgag atctgctgct     1020
ggcgccgctg ctctggcact gacagtggat ctgcctcctg ccagcagcga ggcccggaat     1080
agcgcatttg ggtttcaagg caggctgctg cacctgtctg ccggccagag gctgggagtg     1140
catctgcaca cagaggccag ggctagacac gcctggcagc tgacacaggg cgctacagtg     1200
ctgggcctgt tcagagtgac ccccgagatt ccagcaggcc tgggaggcgg cggatctggc     1260
ggcggaggat ctagagaagg acccgagctg tcccccgacg atcccgctgg gctgctggat     1320
ctgagacagg gcatgttcgc tcagctggtg gctcagaatg tgctgctgat tgacggacct     1380
ctgagctggt actccgaccc agggctggca ggggtgtccc tgactggggg actgtcctac     1440
aaagaagata caaaagaact ggtggtggct aaagctgggg tgtactatgt gtttttttcag     1500
ctggaactga ggcgggtggt ggctggggag ggctcaggat ctgtgtccct ggctctgcat     1560
ctgcagccac tgcgctctgc agcaggggct gcagcactgg ccctgactgt ggacctgccc     1620
ccagcttctt ccgaggccag aaacagcgcc ttcgggttcc aaggacgcct gctgcatctg     1680
agcgccggac agcgcctggg agtgcatctg catactgaag ccagagcccg gcatgcttgg     1740
cagctgactc agggggcaac tgtgctggga ctgtttcgcg tgacacctga gatcccagcc     1800
gggctc                                                                1806
```

<210> 177
<211> 634
<212> PRT
<213> Artificial Sequence

<220>
<223> anti-FAP(4B9) Fc hole monomeric 4-1BB ligand (71-248) chain

<400> 177

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80
```

Leu Gln Met Asn Ser Leu Arg Ala Glu Asp Thr Ala Val Tyr Tyr Cys
85 90 95

Ala Lys Gly Trp Phe Gly Gly Phe Asn Tyr Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile

325 330 335

Ser Lys Ala Lys Gly Gln Pro Arg Glu Pro Gln Val Cys Thr Leu Pro
340 345 350

Pro Ser Arg Asp Glu Leu Thr Lys Asn Gln Val Ser Leu Ser Cys Ala
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Val Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Arg Glu Gly Pro Glu Leu Ser Pro
450 455 460

Asp Asp Pro Ala Gly Leu Leu Asp Leu Arg Gln Gly Met Phe Ala Gln
465 470 475 480

Leu Val Ala Gln Asn Val Leu Leu Ile Asp Gly Pro Leu Ser Trp Tyr
485 490 495

Ser Asp Pro Gly Leu Ala Gly Val Ser Leu Thr Gly Gly Leu Ser Tyr
500 505 510

Lys Glu Asp Thr Lys Glu Leu Val Val Ala Lys Ala Gly Val Tyr Tyr
515 520 525

Val Phe Phe Gln Leu Glu Leu Arg Arg Val Val Ala Gly Glu Gly Ser
530 535 540

Gly Ser Val Ser Leu Ala Leu His Leu Gln Pro Leu Arg Ser Ala Ala
545 550 555 560

Gly Ala Ala Ala Leu Ala Leu Thr Val Asp Leu Pro Pro Ala Ser Ser
565 570 575

```
Glu Ala Arg Asn Ser Ala Phe Gly Phe Gln Gly Arg Leu Leu His Leu
            580                 585                 590

Ser Ala Gly Gln Arg Leu Gly Val His Leu His Thr Glu Ala Arg Ala
        595                 600                 605

Arg His Ala Trp Gln Leu Thr Gln Gly Ala Thr Val Leu Gly Leu Phe
    610                 615                 620

Arg Val Thr Pro Glu Ile Pro Ala Gly Leu
625                 630
```

<210> 178
<211> 602
<212> PRT
<213> Artificial Sequence

<220>
<223> Fc knob dimeric 4-1BB ligand (71-248)

<400> 178

```
Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Cys Arg Asp Glu Leu Thr Lys Asn Gln Val Ser
    130                 135                 140

Leu Trp Cys Leu Val Lys Gly Phe Tyr Pro Ser Asp Ile Ala Val Glu
```

145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Gly Gly Gly Gly Ser Gly Gly Gly Gly Ser Arg Glu Gly Pro
225 230 235 240

Glu Leu Ser Pro Asp Asp Pro Ala Gly Leu Leu Asp Leu Arg Gln Gly
245 250 255

Met Phe Ala Gln Leu Val Ala Gln Asn Val Leu Leu Ile Asp Gly Pro
260 265 270

Leu Ser Trp Tyr Ser Asp Pro Gly Leu Ala Gly Val Ser Leu Thr Gly
275 280 285

Gly Leu Ser Tyr Lys Glu Asp Thr Lys Glu Leu Val Val Ala Lys Ala
290 295 300

Gly Val Tyr Tyr Val Phe Phe Gln Leu Glu Leu Arg Arg Val Val Ala
305 310 315 320

Gly Glu Gly Ser Gly Ser Val Ser Leu Ala Leu His Leu Gln Pro Leu
325 330 335

Arg Ser Ala Ala Gly Ala Ala Ala Leu Ala Leu Thr Val Asp Leu Pro
340 345 350

Pro Ala Ser Ser Glu Ala Arg Asn Ser Ala Phe Gly Phe Gln Gly Arg
355 360 365

Leu Leu His Leu Ser Ala Gly Gln Arg Leu Gly Val His Leu His Thr
370 375 380

Glu Ala Arg Ala Arg His Ala Trp Gln Leu Thr Gln Gly Ala Thr Val
385 390 395 400

Leu Gly Leu Phe Arg Val Thr Pro Glu Ile Pro Ala Gly Leu Gly Gly
405 410 415

Gly Gly Ser Gly Gly Gly Gly Ser Arg Glu Gly Pro Glu Leu Ser Pro
420 425 430

Asp Asp Pro Ala Gly Leu Leu Asp Leu Arg Gln Gly Met Phe Ala Gln
435 440 445

Leu Val Ala Gln Asn Val Leu Leu Ile Asp Gly Pro Leu Ser Trp Tyr
450 455 460

Ser Asp Pro Gly Leu Ala Gly Val Ser Leu Thr Gly Gly Leu Ser Tyr
465 470 475 480

Lys Glu Asp Thr Lys Glu Leu Val Val Ala Lys Ala Gly Val Tyr Tyr
485 490 495

Val Phe Phe Gln Leu Glu Leu Arg Arg Val Val Ala Gly Glu Gly Ser
500 505 510

Gly Ser Val Ser Leu Ala Leu His Leu Gln Pro Leu Arg Ser Ala Ala
515 520 525

Gly Ala Ala Ala Leu Ala Leu Thr Val Asp Leu Pro Pro Ala Ser Ser
530 535 540

Glu Ala Arg Asn Ser Ala Phe Gly Phe Gln Gly Arg Leu Leu His Leu
545 550 555 560

Ser Ala Gly Gln Arg Leu Gly Val His Leu His Thr Glu Ala Arg Ala
565 570 575

Arg His Ala Trp Gln Leu Thr Gln Gly Ala Thr Val Leu Gly Leu Phe
580 585 590

Arg Val Thr Pro Glu Ile Pro Ala Gly Leu
595 600

<210> 179
<211> 2463
<212> DNA
<213> Artificial Sequence

<220>
<223> nucleotide sequence of anti-FAP(28H1) Fc hole dimeric 4-1BB ligand (71-248) chain

<400> 179

gaagtgcagc tgctggaatc cggcggaggc ctggtgcagc ctggcggatc tctgagactg 60

```
tcctgcgccg cctccggctt caccttctcc tcccacgcca tgtcctgggt ccgacaggct       120
cctggcaaag gcctggaatg ggtgtccgcc atctgggcct ccggcgagca gtactacgcc       180
gactctgtga agggccggtt caccatctcc cgggacaact ccaagaacac cctgtacctg       240
cagatgaact ccctgcgggc cgaggacacc gccgtgtact actgtgccaa gggctggctg       300
ggcaacttcg actactgggg acagggcacc ctggtcaccg tgtccagcgc tagcaccaag       360
ggcccctccg tgttccccct ggcccccagc agcaagagca ccagcggcgg cacagccgct       420
ctgggctgcc tggtcaagga ctacttcccc gagcccgtga ccgtgtcctg gaacagcgga       480
gccctgacct ccggcgtgca caccttcccc gccgtgctgc agagttctgg cctgtatagc       540
ctgagcagcg tggtcaccgt gccttctagc agcctgggca cccagaccta catctgcaac       600
gtgaaccaca agcccagcaa caccaaggtg gacaagaagg tggagcccaa gagctgcgac       660
aaaactcaca catgcccacc gtgcccagca cctgaagctg caggggggacc gtcagtcttc     720
ctcttccccc caaaacccaa ggacaccctc atgatctccc ggacccctga ggtcacatgc       780
gtggtggtgg acgtgagcca cgaagaccct gaggtcaagt tcaactggta cgtggacggc       840
gtggaggtgc ataatgccaa gacaaagccg cgggaggagc agtacaacag cacgtaccgt       900
gtggtcagcg tcctcaccgt cctgcaccag gactggctga atggcaagga gtacaagtgc       960
aaggtctcca acaaagccct cggcgccccc atcgagaaaa ccatctccaa agccaaaggg      1020
cagccccgag aaccacaggt gtgcaccctg cccccatccc gggatgagct gaccaagaac      1080
caggtcagcc tctcgtgcgc agtcaaaggc ttctatccca gcgacatcgc cgtggagtgg      1140
gagagcaatg ggcagccgga gaacaactac aagaccacgc ctcccgtgct ggactccgac      1200
ggctccttct tcctcgtgag caagctcacc gtggacaaga gcaggtggca gcaggggaac      1260
gtcttctcat gctccgtgat gcatgaggct ctgcacaacc actacacgca gaagagcctc      1320
tccctgtctc cgggtggagg cggcggaagc ggaggaggag gatccagaga gggccctgag      1380
ctgagccctg atgatcctgc cggactgctg gacctgcggc agggaatgtt tgcccagctg      1440
gtggcccaga acgtgctgct gatcgatggc cccctgtcct ggtacagcga tcctggactg      1500
gctggcgtgt cactgacagg cggcctgagc tacaaagagg acaccaaaga actggtggtg      1560
gccaaggccg gcgtgtacta cgtgttcttt cagctggaac tgcggagagt ggtggccggc      1620
gaaggatctg gctctgtgtc tctggccctg catctgcagc ctctgagatc tgctgctggc      1680
gccgctgctc tggcactgac agtggatctg cctcctgcca gcagcgaggc ccggaatagc      1740
gcatttgggt ttcaaggcag gctgctgcac ctgtctgccg gccagaggct gggagtgcat      1800
ctgcacacag aggccagggc tagacacgcc tggcagctga cacagggcgc tacagtgctg      1860
ggcctgttca gagtgacccc cgagattcca gcaggcctgg gaggcggcgg atctggcggc      1920
```

ggaggatcta gagaaggacc cgagctgtcc cccgacgatc ccgctgggct gctggatctg 1980
agacagggca tgttcgctca gctggtggct cagaatgtgc tgctgattga cggacctctg 2040
agctggtact ccgacccagg gctggcaggg gtgtccctga ctggggggact gtcctacaaa 2100
gaagatacaa aagaactggt ggtggctaaa gctggggtgt actatgtgtt ttttcagctg 2160
gaactgaggc gggtggtggc tggggagggc tcaggatctg tgtccctggc tctgcatctg 2220
cagccactgc gctctgcagc aggggctgca gcactggccc tgactgtgga cctgccccca 2280
gcttcttccg aggccagaaa cagcgccttc gggttccaag gacgcctgct gcatctgagc 2340
gccggacagc gcctgggagt gcatctgcat actgaagcca gagcccggca tgcttggcag 2400
ctgactcagg gggcaactgt gctgggactg tttcgcgtga cacctgagat cccagccggg 2460
ctc 2463

<210> 180
<211> 821
<212> PRT
<213> Artificial Sequence

<220>
<223> anti-FAP(28H1) Fc hole dimeric 4-1BB ligand (71-248) chain

<400> 180

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

```
Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Ala Ala Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Gly Ala Pro Ile Glu Lys Thr Ile Ser
                325                 330                 335

Lys Ala Lys Gly Gln Pro Arg Glu Pro Gln Val Cys Thr Leu Pro Pro
            340                 345                 350

Ser Arg Asp Glu Leu Thr Lys Asn Gln Val Ser Leu Ser Cys Ala Val
        355                 360                 365

Lys Gly Phe Tyr Pro Ser Asp Ile Ala Val Glu Trp Glu Ser Asn Gly
    370                 375                 380
```

```
Gln Pro Glu Asn Asn Tyr Lys Thr Thr Pro Pro Val Leu Asp Ser Asp
385                 390                 395                 400

Gly Ser Phe Phe Leu Val Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Gly Gly Gly
        435                 440                 445

Gly Ser Gly Gly Gly Gly Ser Arg Glu Gly Pro Glu Leu Ser Pro Asp
    450                 455                 460

Asp Pro Ala Gly Leu Leu Asp Leu Arg Gln Gly Met Phe Ala Gln Leu
465                 470                 475                 480

Val Ala Gln Asn Val Leu Leu Ile Asp Gly Pro Leu Ser Trp Tyr Ser
                485                 490                 495

Asp Pro Gly Leu Ala Gly Val Ser Leu Thr Gly Gly Leu Ser Tyr Lys
            500                 505                 510

Glu Asp Thr Lys Glu Leu Val Val Ala Lys Ala Gly Val Tyr Tyr Val
        515                 520                 525

Phe Phe Gln Leu Glu Leu Arg Arg Val Val Ala Gly Glu Gly Ser Gly
    530                 535                 540

Ser Val Ser Leu Ala Leu His Leu Gln Pro Leu Arg Ser Ala Ala Gly
545                 550                 555                 560

Ala Ala Ala Leu Ala Leu Thr Val Asp Leu Pro Pro Ala Ser Ser Glu
                565                 570                 575

Ala Arg Asn Ser Ala Phe Gly Phe Gln Gly Arg Leu Leu His Leu Ser
            580                 585                 590

Ala Gly Gln Arg Leu Gly Val His Leu His Thr Glu Ala Arg Ala Arg
        595                 600                 605

His Ala Trp Gln Leu Thr Gln Gly Ala Thr Val Leu Gly Leu Phe Arg
    610                 615                 620

Val Thr Pro Glu Ile Pro Ala Gly Leu Gly Gly Gly Gly Ser Gly Gly
625                 630                 635                 640
```

```
Gly Gly Ser Arg Glu Gly Pro Glu Leu Ser Pro Asp Asp Pro Ala Gly
                645                 650                 655

Leu Leu Asp Leu Arg Gln Gly Met Phe Ala Gln Leu Val Ala Gln Asn
            660                 665                 670

Val Leu Leu Ile Asp Gly Pro Leu Ser Trp Tyr Ser Asp Pro Gly Leu
        675                 680                 685

Ala Gly Val Ser Leu Thr Gly Gly Leu Ser Tyr Lys Glu Asp Thr Lys
    690                 695                 700

Glu Leu Val Val Ala Lys Ala Gly Val Tyr Tyr Val Phe Phe Gln Leu
705                 710                 715                 720

Glu Leu Arg Arg Val Val Ala Gly Glu Gly Ser Gly Ser Val Ser Leu
                725                 730                 735

Ala Leu His Leu Gln Pro Leu Arg Ser Ala Ala Gly Ala Ala Ala Leu
            740                 745                 750

Ala Leu Thr Val Asp Leu Pro Pro Ala Ser Ser Glu Ala Arg Asn Ser
        755                 760                 765

Ala Phe Gly Phe Gln Gly Arg Leu Leu His Leu Ser Ala Gly Gln Arg
    770                 775                 780

Leu Gly Val His Leu His Thr Glu Ala Arg Ala Arg His Ala Trp Gln
785                 790                 795                 800

Leu Thr Gln Gly Ala Thr Val Leu Gly Leu Phe Arg Val Thr Pro Glu
                805                 810                 815

Ile Pro Ala Gly Leu
            820
```

<210> 181
<211> 1899
<212> DNA
<213> Artificial Sequence

<220>
<223> nucleotide sequence of anti-FAP(28H1) Fc hole monomeric 4-1BB ligand (71-248) chain

<400> 181

```
gaagtgcagc tgctggaatc cggcggaggc ctggtgcagc ctggcggatc tctgagactg       60

tcctgcgccg cctccggctt caccttctcc tcccacgcca tgtcctgggt ccgacaggct      120
```

cctggcaaag gcctggaatg ggtgtccgcc atctgggcct ccggcgagca gtactacgcc 180

gactctgtga agggccggtt caccatctcc cgggacaact ccaagaacac cctgtacctg 240

cagatgaact ccctgcgggc cgaggacacc gccgtgtact actgtgccaa gggctggctg 300

ggcaacttcg actactgggg acagggcacc ctggtcaccg tgtccagcgc tagcaccaag 360

ggcccctccg tgttcccct ggcccccagc agcaagagca ccagcggcgg cacagccgct 420

ctgggctgcc tggtcaagga ctacttcccc gagcccgtga ccgtgtcctg gaacagcgga 480

gccctgacct ccggcgtgca caccttcccc gccgtgctgc agagttctgg cctgtatagc 540

ctgagcagcg tggtcaccgt gccttctagc agcctgggca cccagaccta catctgcaac 600

gtgaaccaca agcccagcaa caccaaggtg gacaagaagg tggagcccaa gagctgcgac 660

aaaactcaca catgcccacc gtgcccagca cctgaagctg caggggacc gtcagtcttc 720

ctcttccccc caaaacccaa ggacaccctc atgatctccc ggacccctga ggtcacatgc 780

gtggtggtgg acgtgagcca cgaagaccct gaggtcaagt tcaactggta cgtggacggc 840

gtggaggtgc ataatgccaa gacaaagccg cgggaggagc agtacaacag cacgtaccgt 900

gtggtcagcg tcctcaccgt cctgcaccag gactggctga atggcaagga gtacaagtgc 960

aaggtctcca acaaagccct cggcgccccc atcgagaaaa ccatctccaa agccaaaggg 1020

cagccccgag aaccacaggt gtgcaccctg cccccatccc gggatgagct gaccaagaac 1080

caggtcagcc tctcgtgcgc agtcaaaggc ttctatccca gcgacatcgc cgtggagtgg 1140

gagagcaatg ggcagccgga gaacaactac aagaccacgc ctcccgtgct ggactccgac 1200

ggctccttct tcctcgtgag caagctcacc gtggacaaga gcaggtggca gcaggggaac 1260

gtcttctcat gctccgtgat gcatgaggct ctgcacaacc actacacgca gaagagcctc 1320

tccctgtctc cgggtggagg cggcggaagc ggaggaggag gatccagaga gggccctgag 1380

ctgagccctg atgatcctgc cggactgctg gacctgcggc agggaatgtt tgcccagctg 1440

gtggcccaga acgtgctgct gatcgatggc cccctgtcct ggtacagcga tcctggactg 1500

gctggcgtgt cactgacagg cggcctgagc tacaaagagg acaccaaaga actggtggtg 1560

gccaaggccg gcgtgtacta cgtgttcttt cagctggaac tgcggagagt ggtggccggc 1620

gaaggatctg gctctgtgtc tctggccctg catctgcagc ctctgagatc tgctgctggc 1680

gccgctgctc tggcactgac agtggatctg cctcctgcca gcagcgaggc ccggaatagc 1740

gcatttgggt ttcaaggcag gctgctgcac ctgtctgccg gccagaggct gggagtgcat 1800

ctgcacacag aggccagggc tagacacgcc tggcagctga cacagggcgc tacagtgctg 1860

ggcctgttca gagtgacccc cgagattcct gccgggctc 1899

<210> 182

<211> 633
<212> PRT
<213> Artificial Sequence

<220>
<223> anti-FAP(28H1) Fc hole monomeric 4-1BBL (71-248) chain

<400> 182

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220
```

Cys Pro Pro Cys Pro Ala Pro Glu Ala Ala Gly Gly Pro Ser Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Asn Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Gly Ala Pro Ile Glu Lys Thr Ile Ser
325 330 335

Lys Ala Lys Gly Gln Pro Arg Glu Pro Gln Val Cys Thr Leu Pro Pro
340 345 350

Ser Arg Asp Glu Leu Thr Lys Asn Gln Val Ser Leu Ser Cys Ala Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Val Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Gly Gly Gly
435 440 445

Gly Ser Gly Gly Gly Gly Ser Arg Glu Gly Pro Glu Leu Ser Pro Asp
450 455 460

Asp Pro Ala Gly Leu Leu Asp Leu Arg Gln Gly Met Phe Ala Gln Leu

465 470 475 480

Val Ala Gln Asn Val Leu Leu Ile Asp Gly Pro Leu Ser Trp Tyr Ser
485 490 495

Asp Pro Gly Leu Ala Gly Val Ser Leu Thr Gly Gly Leu Ser Tyr Lys
500 505 510

Glu Asp Thr Lys Glu Leu Val Val Ala Lys Ala Gly Val Tyr Tyr Val
515 520 525

Phe Phe Gln Leu Glu Leu Arg Arg Val Val Ala Gly Glu Gly Ser Gly
530 535 540

Ser Val Ser Leu Ala Leu His Leu Gln Pro Leu Arg Ser Ala Ala Gly
545 550 555 560

Ala Ala Ala Leu Ala Leu Thr Val Asp Leu Pro Pro Ala Ser Ser Glu
565 570 575

Ala Arg Asn Ser Ala Phe Gly Phe Gln Gly Arg Leu Leu His Leu Ser
580 585 590

Ala Gly Gln Arg Leu Gly Val His Leu His Thr Glu Ala Arg Ala Arg
595 600 605

His Ala Trp Gln Leu Thr Gln Gly Ala Thr Val Leu Gly Leu Phe Arg
610 615 620

Val Thr Pro Glu Ile Pro Ala Gly Leu
625 630

<210> 183
<211> 2478
<212> DNA
<213> Artificial Sequence

<220>
<223> nucleotide sequence of anti- CD19(8B8-018) Fc hole dimeric 4-1BB ligand (71-248) chain

<400> 183

```
caggtccagc tggtgcagtc cggcgccgag gtcaagaaac ccggggcttc tgtgaaggtt      60
tcatgcaagg caagcggata caccttcacc gactatatca tgcattgggt caggcaggcc     120
cctggccaag gtctcgaatg gatgggctac attaacccat ataatgatgg ctccaaatac     180
accgagaagt ttcagggaag agtcactatg acatctgaca ccagtatcag cactgcttac     240
atggagctgt cccgccttcg gtctgatgac accgcagtgt attactgtgc caggggcaca     300
```

tattactacg gctcagctct gttcgactat tgggggcagg gaaccacagt aaccgtgagc 360
tccgctagca ccaagggccc ctccgtgttc cccctggccc ccagcagcaa gagcaccagc 420
ggcggcacag ccgctctggg ctgcctggtc aaggactact tccccgagcc cgtgaccgtg 480
tcctggaaca gcggagccct gacctccggc gtgcacacct tccccgccgt gctgcagagt 540
tctggcctgt atagcctgag cagcgtggtc accgtgcctt ctagcagcct gggcacccag 600
acctacatct gcaacgtgaa ccacaagccc agcaacacca aggtggacaa gaaggtggag 660
cccaagagct gcgacaaaac tcacacatgc ccaccgtgcc cagcacctga agctgcaggg 720
ggaccgtcag tcttcctctt ccccccaaaa cccaaggaca ccctcatgat ctcccggacc 780
cctgaggtca catgcgtggt ggtggacgtg agccacgaag accctgaggt caagttcaac 840
tggtacgtgg acggcgtgga ggtgcataat gccaagacaa agccgcggga ggagcagtac 900
aacagcacgt accgtgtggt cagcgtcctc accgtcctgc accaggactg gctgaatggc 960
aaggagtaca agtgcaaggt ctccaacaaa gccctcggcg cccccatcga gaaaaccatc 1020
tccaaagcca aagggcagcc ccgagaacca caggtgtgca ccctgccccc atcccgggat 1080
gagctgacca agaaccaggt cagcctctcg tgcgcagtca aaggcttcta tcccagcgac 1140
atcgccgtgg agtgggagag caatgggcag ccggagaaca actacaagac cacgcctccc 1200
gtgctggact ccgacggctc cttcttcctc gtgagcaagc tcaccgtgga caagagcagg 1260
tggcagcagg ggaacgtctt ctcatgctcc gtgatgcatg aggctctgca caaccactac 1320
acgcagaaga gcctctccct gtctccgggt ggaggcggcg gaagcggagg aggaggatcc 1380
agagagggcc ctgagctgag ccctgatgat cctgccggac tgctggacct gcggcaggga 1440
atgtttgccc agctggtggc ccagaacgtg ctgctgatcg atggccccct gtcctggtac 1500
agcgatcctg gactggctgg cgtgtcactg acaggcggcc tgagctacaa agaggacacc 1560
aaagaactgg tggtggccaa ggccggcgtg tactacgtgt tctttcagct ggaactgcgg 1620
agagtggtgg ccggcgaagg atctggctct gtgtctctgg ccctgcatct gcagcctctg 1680
agatctgctg ctggcgccgc tgctctggca ctgacagtgg atctgcctcc tgccagcagc 1740
gaggcccgga atagcgcatt tgggtttcaa ggcaggctgc tgcacctgtc tgccggccag 1800
aggctgggag tgcatctgca cacagaggcc agggctagac acgcctggca gctgacacag 1860
ggcgctacag tgctgggcct gttcagagtg acccccgaga ttccagcagg cctgggaggc 1920
ggcggatctg gcggcggagg atctagagaa ggacccgagc tgtcccccga cgatcccgct 1980
gggctgctgg atctgagaca gggcatgttc gctcagctgg tggctcagaa tgtgctgctg 2040
attgacggac ctctgagctg gtactccgac ccagggctgg caggggtgtc cctgactggg 2100
ggactgtcct acaaagaaga tacaaaagaa ctggtggtgg ctaaagctgg ggtgtactat 2160
gtgtttttc agctggaact gaggcgggtg gtggctgggg agggctcagg atctgtgtcc 2220

ctggctctgc atctgcagcc actgcgctct gcagcagggg ctgcagcact ggccctgact 2280

gtggacctgc ccccagcttc ttccgaggcc agaaacagcg ccttcgggtt ccaaggacgc 2340

ctgctgcatc tgagcgccgg acagcgcctg ggagtgcatc tgcatactga agccagagcc 2400

cggcatgctt ggcagctgac tcagggggca actgtgctgg gactgtttcg cgtgacacct 2460

gagatcccag ccgggctc 2478

<210> 184
<211> 826
<212> PRT
<213> Artificial Sequence

<220>
<223> anti- CD19(8B8-018) Fc hole dimeric 4-1BB (71-248) ligand chain

<400> 184

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
```

165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Ala Ala Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Cys Thr Leu Pro Pro Ser Arg Asp Glu Leu Thr Lys Asn Gln Val Ser
355 360 365

Leu Ser Cys Ala Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly
465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu Gly Gly
625 630 635 640

Gly Gly Ser Gly Gly Gly Gly Ser Arg Glu Gly Pro Glu Leu Ser Pro
645 650 655

Asp Asp Pro Ala Gly Leu Leu Asp Leu Arg Gln Gly Met Phe Ala Gln
660 665 670

Leu Val Ala Gln Asn Val Leu Leu Ile Asp Gly Pro Leu Ser Trp Tyr
675 680 685

Ser Asp Pro Gly Leu Ala Gly Val Ser Leu Thr Gly Gly Leu Ser Tyr
690 695 700

Lys Glu Asp Thr Lys Glu Leu Val Val Ala Lys Ala Gly Val Tyr Tyr
705 710 715 720

Val Phe Phe Gln Leu Glu Leu Arg Arg Val Val Ala Gly Glu Gly Ser
725 730 735

Gly Ser Val Ser Leu Ala Leu His Leu Gln Pro Leu Arg Ser Ala Ala
740 745 750

Gly Ala Ala Ala Leu Ala Leu Thr Val Asp Leu Pro Pro Ala Ser Ser
755 760 765

Glu Ala Arg Asn Ser Ala Phe Gly Phe Gln Gly Arg Leu Leu His Leu
770 775 780

Ser Ala Gly Gln Arg Leu Gly Val His Leu His Thr Glu Ala Arg Ala
785 790 795 800

Arg His Ala Trp Gln Leu Thr Gln Gly Ala Thr Val Leu Gly Leu Phe
805 810 815

Arg Val Thr Pro Glu Ile Pro Ala Gly Leu
820 825

<210> 185
<211> 1914
<212> DNA
<213> Artificial Sequence

<220>
<223> nucleotide sequence of anti- CD19(8B8-018) Fc hole monomeric 4-1BBL (71-248) chain

<400> 185

caggtccagc tggtgcagtc cggcgccgag gtcaagaaac ccggggcttc tgtgaaggtt 60

tcatgcaagg caagcggata caccttcacc gactatatca tgcattgggt caggcaggcc 120

cctggccaag gtctcgaatg gatgggctac attaacccat ataatgatgg ctccaaatac 180

accgagaagt ttcagggaag agtcactatg acatctgaca ccagtatcag cactgcttac 240

atggagctgt cccgccttcg gtctgatgac accgcagtgt attactgtgc caggggcaca 300

tattactacg gctcagctct gttcgactat tgggggcagg gaaccacagt aaccgtgagc 360

```
tccgctagca ccaagggccc ctccgtgttc cccctggccc ccagcagcaa gagcaccagc      420
ggcggcacag ccgctctggg ctgcctggtc aaggactact tccccgagcc cgtgaccgtg      480
tcctggaaca gcggagccct gacctccggc gtgcacacct tccccgccgt gctgcagagt      540
tctggcctgt atagcctgag cagcgtggtc accgtgcctt ctagcagcct gggcacccag      600
acctacatct gcaacgtgaa ccacaagccc agcaacacca aggtggacaa gaaggtggag      660
cccaagagct gcgacaaaac tcacacatgc ccaccgtgcc cagcacctga agctgcaggg      720
ggaccgtcag tcttcctctt ccccccaaaa cccaaggaca ccctcatgat ctcccggacc      780
cctgaggtca catgcgtggt ggtggacgtg agccacgaag accctgaggt caagttcaac      840
tggtacgtgg acggcgtgga ggtgcataat gccaagacaa agccgcggga ggagcagtac      900
aacagcacgt accgtgtggt cagcgtcctc accgtcctgc accaggactg gctgaatggc      960
aaggagtaca agtgcaaggt ctccaacaaa gccctcggcg cccccatcga gaaaaccatc     1020
tccaaagcca aagggcagcc ccgagaacca caggtgtgca ccctgccccc atcccgggat     1080
gagctgacca agaaccaggt cagcctctcg tgcgcagtca aaggcttcta tcccagcgac     1140
atcgccgtgg agtgggagag caatgggcag ccggagaaca actacaagac cacgcctccc     1200
gtgctggact ccgacggctc cttcttcctc gtgagcaagc tcaccgtgga caagagcagg     1260
tggcagcagg ggaacgtctt ctcatgctcc gtgatgcatg aggctctgca caaccactac     1320
acgcagaaga gcctctccct gtctccgggt ggaggcggcg gaagcggagg aggaggatcc     1380
agagagggcc ctgagctgag ccctgatgat cctgccggac tgctggacct gcggcaggga     1440
atgtttgccc agctggtggc ccagaacgtg ctgctgatcg atggccccct gtcctggtac     1500
agcgatcctg gactggctgg cgtgtcactg acaggcggcc tgagctacaa agaggacacc     1560
aaagaactgg tggtggccaa ggccggcgtg tactacgtgt tctttcagct ggaactgcgg     1620
agagtggtgg ccggcgaagg atctggctct gtgtctctgg ccctgcatct gcagcctctg     1680
agatctgctg ctggcgccgc tgctctggca ctgacagtgg atctgcctcc tgccagcagc     1740
gaggcccgga atagcgcatt tgggtttcaa ggcaggctgc tgcacctgtc tgccggccag     1800
aggctgggag tgcatctgca cacagaggcc agggctagac acgcctggca gctgacacag     1860
ggcgctacag tgctgggcct gttcagagtg acccccgaga ttcctgccgg gctc           1914
```

<210> 186
<211> 638
<212> PRT
<213> Artificial Sequence

<220>
<223> anti- CD19(8B8-018) Fc hole monomeric 4-1BB ligand (71-248) chain

<400> 186

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255
```

```
Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Gly Gly Gly Gly Ser Gly Gly Gly Gly Ser Arg Glu Gly Pro
    450                 455                 460

Glu Leu Ser Pro Asp Asp Pro Ala Gly Leu Leu Asp Leu Arg Gln Gly
465                 470                 475                 480

Met Phe Ala Gln Leu Val Ala Gln Asn Val Leu Leu Ile Asp Gly Pro
                485                 490                 495

Leu Ser Trp Tyr Ser Asp Pro Gly Leu Ala Gly Val Ser Leu Thr Gly
            500                 505                 510
```

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu
625 630 635

<210> 187
<211> 2478
<212> DNA
<213> Artificial Sequence

<220>
<223> nucleotide sequence of anti- CD19(8B8-2B11) Fc hole dimeric 4-1BB ligand (71-248) chain

<400> 187

caggtgcaat tggttcaatc tggtgctgaa gtaaaaaaac cgggcgcttc cgttaaagtg 60
agctgcaaag catctggtta caccttcact gactatatca tgcactgggt tcgtcaggcc 120
ccgggccagg gtctggagtg gatgggctac attaacccat acaacgacgg ttccaaatat 180
accgagaaat tccagggccg cgtcacgatg accagcgaca cttctatctc caccgcgtac 240
atggaactgt ctagactgcg ttctgacgac accgctgttt actattgtgc acgcggtacc 300
tactactacg gtccacagct gtttgattac tggggccaag gtaccacggt gaccgtaagc 360
tctgctagca ccaagggccc ctccgtgttc cccctggccc ccagcagcaa gagcaccagc 420
ggcggcacag ccgctctggg ctgcctggtc aaggactact tccccgagcc cgtgaccgtg 480
tcctggaaca gcggagccct gacctccggc gtgcacacct tccccgccgt gctgcagagt 540
tctggcctgt atagcctgag cagcgtggtc accgtgcctt ctagcagcct gggcacccag 600

acctacatct gcaacgtgaa ccacaagccc agcaacacca aggtggacaa gaaggtggag 660

cccaagagct gcgacaaaac tcacacatgc ccaccgtgcc cagcacctga agctgcaggg 720

ggaccgtcag tcttcctctt ccccccaaaa cccaaggaca ccctcatgat ctcccggacc 780

cctgaggtca catgcgtggt ggtggacgtg agccacgaag accctgaggt caagttcaac 840

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtgca ccctgccccc atcccgggat 1080

gagctgacca agaaccaggt cagcctctcg tgcgcagtca aaggcttcta tcccagcgac 1140

atcgccgtgg agtgggagag caatgggcag ccggagaaca actacaagac cacgcctccc 1200

gtgctggact ccgacggctc cttcttcctc gtgagcaagc tcaccgtgga caagagcagg 1260

tggcagcagg ggaacgtctt ctcatgctcc gtgatgcatg aggctctgca caaccactac 1320

acgcagaaga gcctctccct gtctccgggt ggaggcggcg gaagcggagg aggaggatcc 1380

agagagggcc ctgagctgag ccctgatgat cctgccggac tgctggacct gcggcaggga 1440

atgtttgccc agctggtggc ccagaacgtg ctgctgatcg atggccccct gtcctggtac 1500

agcgatcctg gactggctgg cgtgtcactg acaggcggcc tgagctacaa agaggacacc 1560

aaagaactgg tggtggccaa ggccggcgtg tactacgtgt tctttcagct ggaactgcgg 1620

agagtggtgg ccggcgaagg atctggctct gtgtctctgg ccctgcatct gcagcctctg 1680

agatctgctg ctggcgccgc tgctctggca ctgacagtgg atctgcctcc tgccagcagc 1740

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttccagcagg cctgggaggc 1920

ggcggatctg gcggcggagg atctagagaa ggacccgagc tgtcccccga cgatcccgct 1980

gggctgctgg atctgagaca gggcatgttc gctcagctgg tggctcagaa tgtgctgctg 2040

attgacggac ctctgagctg gtactccgac ccagggctgg caggggtgtc cctgactggg 2100

ggactgtcct acaaagaaga tacaaaagaa ctggtggtgg ctaaagctgg ggtgtactat 2160

gtgtttttc agctggaact gaggcgggtg gtggctgggg agggctcagg atctgtgtcc 2220

ctggctctgc atctgcagcc actgcgctct gcagcagggg ctgcagcact ggccctgact 2280

gtggacctgc ccccagcttc ttccgaggcc agaaacagcg ccttcgggtt ccaaggacgc 2340

ctgctgcatc tgagcgccgg acagcgcctg ggagtgcatc tgcatactga agccagagcc 2400

cggcatgctt ggcagctgac tcagggggca actgtgctgg gactgtttcg cgtgacacct 2460

gagatcccag ccgggctc 2478

<210> 188
<211> 826
<212> PRT
<213> Artificial Sequence

<220>
<223> anti- CD19(8B8-2B11) Fc hole dimeric 4-1BB ligand (71-248) chain

<400> 188

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Gln Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
```

```
Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Gly Gly Gly Gly Ser Gly Gly Gly Gly Ser Arg Glu Gly Pro
```

450 455 460

Glu Leu Ser Pro Asp Asp Pro Ala Gly Leu Leu Asp Leu Arg Gln Gly
465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu Gly Gly
625 630 635 640

Gly Gly Ser Gly Gly Gly Gly Ser Arg Glu Gly Pro Glu Leu Ser Pro
645 650 655

Asp Asp Pro Ala Gly Leu Leu Asp Leu Arg Gln Gly Met Phe Ala Gln
660 665 670

Leu Val Ala Gln Asn Val Leu Leu Ile Asp Gly Pro Leu Ser Trp Tyr
675 680 685

Ser Asp Pro Gly Leu Ala Gly Val Ser Leu Thr Gly Gly Leu Ser Tyr
690 695 700

```
Lys Glu Asp Thr Lys Glu Leu Val Val Ala Lys Ala Gly Val Tyr Tyr
705                 710                 715                 720

Val Phe Phe Gln Leu Glu Leu Arg Arg Val Val Ala Gly Glu Gly Ser
                725                 730                 735

Gly Ser Val Ser Leu Ala Leu His Leu Gln Pro Leu Arg Ser Ala Ala
            740                 745                 750

Gly Ala Ala Ala Leu Ala Leu Thr Val Asp Leu Pro Pro Ala Ser Ser
        755                 760                 765

Glu Ala Arg Asn Ser Ala Phe Gly Phe Gln Gly Arg Leu Leu His Leu
    770                 775                 780

Ser Ala Gly Gln Arg Leu Gly Val His Leu His Thr Glu Ala Arg Ala
785                 790                 795                 800

Arg His Ala Trp Gln Leu Thr Gln Gly Ala Thr Val Leu Gly Leu Phe
                805                 810                 815

Arg Val Thr Pro Glu Ile Pro Ala Gly Leu
            820                 825
```

<210> 189
<211> 1914
<212> DNA
<213> Artificial Sequence

<220>
<223> nucleotide sequence of anti- CD19(8B8-2B11) Fc hole monomeric 4-1BB ligand (71-248) chain

<400> 189

```
caggtgcaat tggttcaatc tggtgctgaa gtaaaaaaac cgggcgcttc cgttaaagtg       60
agctgcaaag catctggtta caccttcact gactatatca tgcactgggt tcgtcaggcc      120
ccgggccagg gtctggagtg gatgggctac attaacccat acaacgacgg ttccaaatat      180
accgagaaat tccagggccg cgtcacgatg accagcgaca cttctatctc caccgcgtac      240
atggaactgt ctagactgcg ttctgacgac accgctgttt actattgtgc acgcggtacc      300
tactactacg gtccacagct gtttgattac tggggccaag gtaccacggt gaccgtaagc      360
tctgctagca ccaagggccc ctccgtgttc cccctggccc ccagcagcaa gagcaccagc      420
ggcggcacag ccgctctggg ctgcctggtc aaggactact tccccgagcc cgtgaccgtg      480
tcctggaaca gcggagccct gacctccggc gtgcacacct tccccgccgt gctgcagagt      540
tctggcctgt atagcctgag cagcgtggtc accgtgcctt ctagcagcct gggcacccag      600
acctacatct gcaacgtgaa ccacaagccc agcaacacca aggtggacaa gaaggtggag      660
```

cccaagagct gcgacaaaac tcacacatgc ccaccgtgcc cagcacctga agctgcaggg 720

ggaccgtcag tcttcctctt ccccccaaaa cccaaggaca ccctcatgat ctcccggacc 780

cctgaggtca catgcgtggt ggtggacgtg agccacgaag accctgaggt caagttcaac 840

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtgca ccctgccccc atcccgggat 1080

gagctgacca agaaccaggt cagcctctcg tgcgcagtca aaggcttcta tcccagcgac 1140

atcgccgtgg agtgggagag caatgggcag ccggagaaca actacaagac cacgcctccc 1200

gtgctggact ccgacggctc cttcttcctc gtgagcaagc tcaccgtgga caagagcagg 1260

tggcagcagg ggaacgtctt ctcatgctcc gtgatgcatg aggctctgca caaccactac 1320

acgcagaaga gcctctccct gtctccgggt ggaggcggcg gaagcggagg aggaggatcc 1380

agagagggcc ctgagctgag ccctgatgat cctgccggac tgctggacct gcggcaggga 1440

atgtttgccc agctggtggc ccagaacgtg ctgctgatcg atggccccct gtcctggtac 1500

agcgatcctg gactggctgg cgtgtcactg acaggcggcc tgagctacaa agaggacacc 1560

aaagaactgg tggtggccaa ggccggcgtg tactacgtgt tctttcagct ggaactgcgg 1620

agagtggtgg ccggcgaagg atctggctct gtgtctctgg ccctgcatct gcagcctctg 1680

agatctgctg ctggcgccgc tgctctggca ctgacagtgg atctgcctcc tgccagcagc 1740

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttcctgccgg gctc 1914

<210> 190
<211> 638
<212> PRT
<213> Artificial Sequence

<220>
<223> anti- CD19(8B8-2B11) Fc hole monomeric 4-1BBL (71-248) chain

<400> 190

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
```

35 40 45

Gly Tyr Ile Asn Pro Tyr Asn Asp Gly Ser Lys Tyr Thr Glu Lys Phe
50 55 60

Gln Gly Arg Val Thr Met Thr Ser Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Gly Thr Tyr Tyr Tyr Gly Pro Gln Leu Phe Asp Tyr Trp Gly
100 105 110

Gln Gly Thr Thr Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Ala Ala Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

```
His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Gly Gly Gly Gly Ser Gly Gly Gly Gly Ser Arg Glu Gly Pro
    450                 455                 460

Glu Leu Ser Pro Asp Asp Pro Ala Gly Leu Leu Asp Leu Arg Gln Gly
465                 470                 475                 480

Met Phe Ala Gln Leu Val Ala Gln Asn Val Leu Leu Ile Asp Gly Pro
                485                 490                 495

Leu Ser Trp Tyr Ser Asp Pro Gly Leu Ala Gly Val Ser Leu Thr Gly
            500                 505                 510

Gly Leu Ser Tyr Lys Glu Asp Thr Lys Glu Leu Val Val Ala Lys Ala
        515                 520                 525

Gly Val Tyr Tyr Val Phe Phe Gln Leu Glu Leu Arg Arg Val Val Ala
    530                 535                 540
```

```
Gly Glu Gly Ser Gly Ser Val Ser Leu Ala Leu His Leu Gln Pro Leu
545                 550                 555                 560

Arg Ser Ala Ala Gly Ala Ala Ala Leu Ala Leu Thr Val Asp Leu Pro
                565                 570                 575

Pro Ala Ser Ser Glu Ala Arg Asn Ser Ala Phe Gly Phe Gln Gly Arg
            580                 585                 590

Leu Leu His Leu Ser Ala Gly Gln Arg Leu Gly Val His Leu His Thr
        595                 600                 605

Glu Ala Arg Ala Arg His Ala Trp Gln Leu Thr Gln Gly Ala Thr Val
    610                 615                 620

Leu Gly Leu Phe Arg Val Thr Pro Glu Ile Pro Ala Gly Leu
625                 630                 635
```

<210> 191
<211> 2478
<212> DNA
<213> Artificial Sequence

<220>
<223> nucleotide sequence of anti- CEA(T84.66-LCHA) Fc hole dimeric 4-1BB ligand (71-248) chain

<400> 191

```
caggtgcagc tggtgcagtc tggcgccgaa gtgaagaaac ccggcagcag cgtgaaggtg      60
tcctgcaagg ccagcggctt caacatcaag gacacctaca tgcactgggt gcgccaggcc     120
cctggacagg gactggaatg gatgggcaga atcgaccccg ccaacggcaa cagcaaatac     180
gtgcccaagt tccagggcag agtgaccatc accgccgaca ccagcacctc caccgcctac     240
atggaactga gcagcctgcg gagcgaggac accgccgtgt actactgtgc cccccttcggc     300
tactacgtgt ccgactacgc catggcctat tggggccagg gcacactcgt gaccgtgtcc     360
tctgctagca ccaagggccc ctccgtgttc cccctggccc ccagcagcaa gagcaccagc     420
ggcggcacag ccgctctggg ctgcctggtc aaggactact tccccgagcc cgtgaccgtg     480
tcctggaaca gcggagccct gacctccggc gtgcacacct tccccgccgt gctgcagagt     540
tctggcctgt atagcctgag cagcgtggtc accgtgcctt ctagcagcct gggcacccag     600
acctacatct gcaacgtgaa ccacaagccc agcaacacca aggtggacaa gaaggtggag     660
cccaagagct gcgacaaaac tcacacatgc ccaccgtgcc cagcacctga agctgcaggg     720
ggaccgtcag tcttcctctt ccccccaaaa cccaaggaca ccctcatgat ctcccggacc     780
cctgaggtca catgcgtggt ggtggacgtg agccacgaag accctgaggt caagttcaac     840
```

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtgca ccctgccccc atcccgggat 1080

gagctgacca agaaccaggt cagcctctcg tgcgcagtca aaggcttcta tcccagcgac 1140

atcgccgtgg agtgggagag caatgggcag ccggagaaca actacaagac cacgcctccc 1200

gtgctggact ccgacggctc cttcttcctc gtgagcaagc tcaccgtgga caagagcagg 1260

tggcagcagg ggaacgtctt ctcatgctcc gtgatgcatg aggctctgca caaccactac 1320

acgcagaaga gcctctccct gtctccgggt ggaggcggcg gaagcggagg aggaggatcc 1380

agagagggcc ctgagctgag ccctgatgat cctgccggac tgctggacct gcggcaggga 1440

atgtttgccc agctggtggc ccagaacgtg ctgctgatcg atggcccct gtcctggtac 1500

agcgatcctg gactggctgg cgtgtcactg acaggcggcc tgagctacaa agaggacacc 1560

aaagaactgg tggtggccaa ggccggcgtg tactacgtgt tctttcagct ggaactgcgg 1620

agagtggtgg ccggcgaagg atctggctct gtgtctctgg ccctgcatct gcagcctctg 1680

agatctgctg ctggcgccgc tgctctggca ctgacagtgg atctgcctcc tgccagcagc 1740

gaggcccgga atagcgcatt tgggtttcaa ggcaggctgc tgcacctgtc tgccggccag 1800

aggctgggag tgcatctgca cacagaggcc agggctagac acgcctggca gctgacacag 1860

ggcgctacag tgctgggcct gttcagagtg acccccgaga ttccagcagg cctgggaggc 1920

ggcggatctg gcggcggagg atctagagaa ggacccgagc tgtcccccga cgatcccgct 1980

gggctgctgg atctgagaca gggcatgttc gctcagctgg tggctcagaa tgtgctgctg 2040

attgacggac ctctgagctg gtactccgac ccagggctgg caggggtgtc cctgactggg 2100

ggactgtcct acaaagaaga tacaaaagaa ctggtggtgg ctaaagctgg ggtgtactat 2160

gtgtttttc agctggaact gaggcgggtg gtggctgggg agggctcagg atctgtgtcc 2220

ctggctctgc atctgcagcc actgcgctct gcagcagggg ctgcagcact ggccctgact 2280

gtggacctgc ccccagcttc ttccgaggcc agaaacagcg ccttcgggtt ccaaggacgc 2340

ctgctgcatc tgagcgccgg acagcgcctg ggagtgcatc tgcatactga agccagagcc 2400

cggcatgctt ggcagctgac tcagggggca actgtgctgg gactgtttcg cgtgacacct 2460

gagatcccag ccgggctc 2478

<210> 192
<211> 826
<212> PRT
<213> Artificial Sequence

<220>

<223> anti- CEA(T84.66-LCHA) Fc hole dimeric 4-1BB (71-248) ligand chain

<400> 192

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Cys Thr Leu Pro Pro Ser Arg Asp Glu Leu Thr Lys Asn Gln Val Ser
355 360 365

Leu Ser Cys Ala Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly
465 470 475 480

Met Phe Ala Gln Leu Val Ala Gln Asn Val Leu Leu Ile Asp Gly Pro

485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu Gly Gly
625 630 635 640

Gly Gly Ser Gly Gly Gly Gly Ser Arg Glu Gly Pro Glu Leu Ser Pro
645 650 655

Asp Asp Pro Ala Gly Leu Leu Asp Leu Arg Gln Gly Met Phe Ala Gln
660 665 670

Leu Val Ala Gln Asn Val Leu Leu Ile Asp Gly Pro Leu Ser Trp Tyr
675 680 685

Ser Asp Pro Gly Leu Ala Gly Val Ser Leu Thr Gly Gly Leu Ser Tyr
690 695 700

Lys Glu Asp Thr Lys Glu Leu Val Val Ala Lys Ala Gly Val Tyr Tyr
705 710 715 720

Val Phe Phe Gln Leu Glu Leu Arg Arg Val Val Ala Gly Glu Gly Ser
725 730 735

```
Gly Ser Val Ser Leu Ala Leu His Leu Gln Pro Leu Arg Ser Ala Ala
            740                 745                 750

Gly Ala Ala Ala Leu Ala Leu Thr Val Asp Leu Pro Pro Ala Ser Ser
        755                 760                 765

Glu Ala Arg Asn Ser Ala Phe Gly Phe Gln Gly Arg Leu Leu His Leu
    770                 775                 780

Ser Ala Gly Gln Arg Leu Gly Val His Leu His Thr Glu Ala Arg Ala
785                 790                 795                 800

Arg His Ala Trp Gln Leu Thr Gln Gly Ala Thr Val Leu Gly Leu Phe
                805                 810                 815

Arg Val Thr Pro Glu Ile Pro Ala Gly Leu
            820                 825
```

<210> 193
<211> 1914
<212> DNA
<213> Artificial Sequence

<220>
<223> nucleotide sequence of anti- CEA(T84.66-LCHA) Fc hole monomeric 4-1BBL (71-248) chain

<400> 193

```
caggtgcagc tggtgcagtc tggcgccgaa gtgaagaaac ccggcagcag cgtgaaggtg      60
tcctgcaagg ccagcggctt caacatcaag gacacctaca tgcactgggt gcgccaggcc     120
cctggacagg gactggaatg gatgggcaga atcgaccccg ccaacggcaa cagcaaatac     180
gtgcccaagt tccagggcag agtgaccatc accgccgaca ccagcacctc caccgcctac     240
atggaactga gcagcctgcg gagcgaggac accgccgtgt actactgtgc cccccttcggc     300
tactacgtgt ccgactacgc catggcctat tggggccagg gcacactcgt gaccgtgtcc     360
tctgctagca ccaagggccc ctccgtgttc cccctggccc ccagcagcaa gagcaccagc     420
ggcggcacag ccgctctggg ctgcctggtc aaggactact tccccgagcc cgtgaccgtg     480
tcctggaaca gcggagccct gacctccggc gtgcacacct tccccgccgt gctgcagagt     540
tctggcctgt atagcctgag cagcgtggtc accgtgcctt ctagcagcct gggcacccag     600
acctacatct gcaacgtgaa ccacaagccc agcaacacca aggtggacaa gaaggtggag     660
cccaagagct gcgacaaaac tcacacatgc ccaccgtgcc cagcacctga agctgcaggg     720
ggaccgtcag tcttcctctt ccccccaaaa cccaaggaca ccctcatgat ctcccggacc     780
cctgaggtca catgcgtggt ggtggacgtg agccacgaag accctgaggt caagttcaac     840
tggtacgtgg acggcgtgga ggtgcataat gccaagacaa agccgcggga ggagcagtac     900
```

```
aacagcacgt accgtgtggt cagcgtcctc accgtcctgc accaggactg gctgaatggc      960
aaggagtaca agtgcaaggt ctccaacaaa gccctcggcg cccccatcga gaaaaccatc     1020
tccaaagcca aagggcagcc ccgagaacca caggtgtgca ccctgccccc atcccgggat     1080
gagctgacca agaaccaggt cagcctctcg tgcgcagtca aaggcttcta tcccagcgac     1140
atcgccgtgg agtgggagag caatgggcag ccggagaaca actacaagac cacgcctccc     1200
gtgctggact ccgacggctc cttcttcctc gtgagcaagc tcaccgtgga caagagcagg     1260
tggcagcagg ggaacgtctt ctcatgctcc gtgatgcatg aggctctgca caaccactac     1320
acgcagaaga gcctctccct gtctccgggt ggaggcggcg gaagcggagg aggaggatcc     1380
agagagggcc ctgagctgag ccctgatgat cctgccggac tgctggacct gcggcaggga     1440
atgtttgccc agctggtggc ccagaacgtg ctgctgatcg atggccccct gtcctggtac     1500
agcgatcctg gactggctgg cgtgtcactg acaggcggcc tgagctacaa agaggacacc     1560
aaagaactgg tggtggccaa ggccggcgtg tactacgtgt tctttcagct ggaactgcgg     1620
agagtggtgg ccggcgaagg atctggctct gtgtctctgg ccctgcatct gcagcctctg     1680
agatctgctg ctggcgccgc tgctctggca ctgacagtgg atctgcctcc tgccagcagc     1740
gaggcccgga atagcgcatt tgggtttcaa ggcaggctgc tgcacctgtc tgccggccag     1800
aggctgggag tgcatctgca cacagaggcc agggctagac acgcctggca gctgacacag     1860
ggcgctacag tgctgggcct gttcagagtg acccccgaga ttcctgccgg gctc           1914
```

<210> 194
<211> 638
<212> PRT
<213> Artificial Sequence

<220>
<223> anti- CEA(T84.66-LCHA) Fc hole monomeric 4-1BB ligand (71-248) chain

<400> 194

```
Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60
```

```
Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320
```

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Cys Thr Leu Pro Pro Ser Arg Asp Glu Leu Thr Lys Asn Gln Val Ser
355 360 365

Leu Ser Cys Ala Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Gly Gly Gly Gly Ser Gly Gly Gly Gly Ser Arg Glu Gly Pro
450 455 460

Glu Leu Ser Pro Asp Asp Pro Ala Gly Leu Leu Asp Leu Arg Gln Gly
465 470 475 480

Met Phe Ala Gln Leu Val Ala Gln Asn Val Leu Leu Ile Asp Gly Pro
485 490 495

Leu Ser Trp Tyr Ser Asp Pro Gly Leu Ala Gly Val Ser Leu Thr Gly
500 505 510

Gly Leu Ser Tyr Lys Glu Asp Thr Lys Glu Leu Val Val Ala Lys Ala
515 520 525

Gly Val Tyr Tyr Val Phe Phe Gln Leu Glu Leu Arg Arg Val Val Ala
530 535 540

Gly Glu Gly Ser Gly Ser Val Ser Leu Ala Leu His Leu Gln Pro Leu
545 550 555 560

Arg Ser Ala Ala Gly Ala Ala Ala Leu Ala Leu Thr Val Asp Leu Pro
565 570 575

Pro Ala Ser Ser Glu Ala Arg Asn Ser Ala Phe Gly Phe Gln Gly Arg
580 585 590

Leu Leu His Leu Ser Ala Gly Gln Arg Leu Gly Val His Leu His Thr
595 600 605

Glu Ala Arg Ala Arg His Ala Trp Gln Leu Thr Gln Gly Ala Thr Val
610 615 620

Leu Gly Leu Phe Arg Val Thr Pro Glu Ile Pro Ala Gly Leu
625 630 635

<210> 195
<211> 2460
<212> DNA
<213> Artificial Sequence

<220>
<223> nucleotide sequence of DP47 Fc hole dimeric 4-1BB ligand (71-248) chain

<400> 195

gaggtgcaat tgttggagtc tgggggaggc ttggtacagc ctggggggtc cctgagactc 60

tcctgtgcag cctccggatt cacctttagc agttatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcagatga acagcctgag agccgaggac acggccgtat attactgtgc gaaaggcagc 300

ggatttgact actggggcca aggaaccctg gtcaccgtct cgagtgctag caccaagggc 360

ccctccgtgt tccccctggc ccccagcagc aagagcacca gcggcggcac agccgctctg 420

ggctgcctgg tcaaggacta cttccccgag cccgtgaccg tgtcctggaa cagcggagcc 480

ctgacctccg gcgtgcacac cttccccgcc gtgctgcaga gttctggcct gtatagcctg 540

agcagcgtgg tcaccgtgcc ttctagcagc ctgggcaccc agacctacat ctgcaacgtg 600

aaccacaagc ccagcaacac caaggtggac aagaaggtgg agcccaagag ctgcgacaaa 660

actcacacat gcccaccgtg cccagcacct gaagctgcag gggggaccgtc agtcttcctc 720

ttccccccaa aacccaagga caccctcatg atctcccgga cccctgaggt cacatgcgtg 780

gtggtggacg tgagccacga agaccctgag gtcaagttca actggtacgt ggacggcgtg 840

gaggtgcata atgccaagac aaagccgcgg gaggagcagt acaacagcac gtaccgtgtg 900

gtcagcgtcc tcaccgtcct gcaccaggac tggctgaatg gcaaggagta caagtgcaag 960

gtctccaaca aagccctcgg cgcccccatc gagaaaacca tctccaaagc caaagggcag 1020

ccccgagaac cacaggtgtg caccctgccc ccatcccggg atgagctgac caagaaccag 1080

gtcagcctct cgtgcgcagt caaaggcttc tatcccagcg acatcgccgt ggagtgggag 1140

agcaatgggc agccggagaa caactacaag accacgcctc ccgtgctgga ctccgacggc 1200

tccttcttcc tcgtgagcaa gctcaccgtg gacaagagca ggtggcagca ggggaacgtc 1260

ttctcatgct ccgtgatgca tgaggctctg cacaaccact acacgcagaa gagcctctcc 1320

ctgtctccgg gtggaggcgg cggaagcgga ggaggaggat ccagagaggg ccctgagctg 1380

agccctgatg atcctgccgg actgctggac ctgcggcagg gaatgtttgc ccagctggtg 1440

gcccagaacg tgctgctgat cgatggcccc ctgtcctggt acagcgatcc tggactggct 1500

ggcgtgtcac tgacaggcgg cctgagctac aaagaggaca ccaaagaact ggtggtggcc 1560

aaggccggcg tgtactacgt gttctttcag ctggaactgc ggagagtggt ggccggcgaa 1620

ggatctggct ctgtgtctct ggccctgcat ctgcagcctc tgagatctgc tgctggcgcc 1680

gctgctctgg cactgacagt ggatctgcct cctgccagca gcgaggcccg gaatagcgca 1740

tttgggtttc aaggcaggct gctgcacctg tctgccggcc agaggctggg agtgcatctg 1800

cacacagagg ccagggctag acacgcctgg cagctgacac agggcgctac agtgctgggc 1860

ctgttcagag tgacccccga gattccagca ggcctgggag gcggcggatc tggcggcgga 1920

ggatctagag aaggacccga gctgtccccc gacgatcccg ctgggctgct ggatctgaga 1980

cagggcatgt tcgctcagct ggtggctcag aatgtgctgc tgattgacgg acctctgagc 2040

tggtactccg acccagggct ggcaggggtg tccctgactg gggggactgtc ctacaaagaa 2100

gatacaaaag aactggtggt ggctaaagct ggggtgtact atgtgttttt tcagctggaa 2160

ctgaggcggg tggtggctgg ggagggctca ggatctgtgt ccctggctct gcatctgcag 2220

ccactgcgct ctgcagcagg ggctgcagca ctggccctga ctgtggacct gcccccagct 2280

tcttccgagg ccagaaacag cgccttcggg ttccaaggac gcctgctgca tctgagcgcc 2340

ggacagcgcc tgggagtgca tctgcatact gaagccagag cccggcatgc ttggcagctg 2400

actcaggggg caactgtgct gggactgttt cgcgtgacac ctgagatccc agccgggctc 2460

<210> 196
<211> 820
<212> PRT
<213> Artificial Sequence

<220>
<223> DP47 Fc hole dimeric 4-1BB ligand (71-248) chain

<400> 196

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Ala Ala Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Cys Thr Leu Pro Pro Ser
340 345 350

Arg Asp Glu Leu Thr Lys Asn Gln Val Ser Leu Ser Cys Ala Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Val Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Gly Gly Gly Gly
435 440 445

Ser Gly Gly Gly Gly Ser Arg Glu Gly Pro Glu Leu Ser Pro Asp Asp
450 455 460

Pro Ala Gly Leu Leu Asp Leu Arg Gln Gly Met Phe Ala Gln Leu Val
465 470 475 480

Ala Gln Asn Val Leu Leu Ile Asp Gly Pro Leu Ser Trp Tyr Ser Asp
485 490 495

Pro Gly Leu Ala Gly Val Ser Leu Thr Gly Gly Leu Ser Tyr Lys Glu
500 505 510

Asp Thr Lys Glu Leu Val Val Ala Lys Ala Gly Val Tyr Tyr Val Phe
515 520 525

```
Phe Gln Leu Glu Leu Arg Arg Val Val Ala Gly Glu Gly Ser Gly Ser
    530                 535                 540

Val Ser Leu Ala Leu His Leu Gln Pro Leu Arg Ser Ala Ala Gly Ala
545                 550                 555                 560

Ala Ala Leu Ala Leu Thr Val Asp Leu Pro Pro Ala Ser Ser Glu Ala
                565                 570                 575

Arg Asn Ser Ala Phe Gly Phe Gln Gly Arg Leu Leu His Leu Ser Ala
            580                 585                 590

Gly Gln Arg Leu Gly Val His Leu His Thr Glu Ala Arg Ala Arg His
        595                 600                 605

Ala Trp Gln Leu Thr Gln Gly Ala Thr Val Leu Gly Leu Phe Arg Val
    610                 615                 620

Thr Pro Glu Ile Pro Ala Gly Leu Gly Gly Gly Gly Ser Gly Gly Gly
625                 630                 635                 640

Gly Ser Arg Glu Gly Pro Glu Leu Ser Pro Asp Asp Pro Ala Gly Leu
                645                 650                 655

Leu Asp Leu Arg Gln Gly Met Phe Ala Gln Leu Val Ala Gln Asn Val
            660                 665                 670

Leu Leu Ile Asp Gly Pro Leu Ser Trp Tyr Ser Asp Pro Gly Leu Ala
        675                 680                 685

Gly Val Ser Leu Thr Gly Gly Leu Ser Tyr Lys Glu Asp Thr Lys Glu
    690                 695                 700

Leu Val Val Ala Lys Ala Gly Val Tyr Tyr Val Phe Phe Gln Leu Glu
705                 710                 715                 720

Leu Arg Arg Val Val Ala Gly Glu Gly Ser Gly Ser Val Ser Leu Ala
                725                 730                 735

Leu His Leu Gln Pro Leu Arg Ser Ala Ala Gly Ala Ala Ala Leu Ala
            740                 745                 750

Leu Thr Val Asp Leu Pro Pro Ala Ser Ser Glu Ala Arg Asn Ser Ala
        755                 760                 765

Phe Gly Phe Gln Gly Arg Leu Leu His Leu Ser Ala Gly Gln Arg Leu
    770                 775                 780
```

Gly Val His Leu His Thr Glu Ala Arg Ala Arg His Ala Trp Gln Leu
785 790 795 800

Thr Gln Gly Ala Thr Val Leu Gly Leu Phe Arg Val Thr Pro Glu Ile
805 810 815

Pro Ala Gly Leu
820

<210> 197
<211> 1896
<212> DNA
<213> Artificial Sequence

<220>
<223> nucleotide sequence of DP47 Fc hole monomeric 4-1BBL (71-248) chain

<400> 197

gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagtgctag caccaagggc 360
ccctccgtgt tccccctggc ccccagcagc aagagcacca gcggcggcac agccgctctg 420
ggctgcctgg tcaaggacta cttccccgag cccgtgaccg tgtcctggaa cagcggagcc 480
ctgacctccg gcgtgcacac cttccccgcc gtgctgcaga gttctggcct gtatagcctg 540
agcagcgtgg tcaccgtgcc ttctagcagc ctgggcaccc agacctacat ctgcaacgtg 600
aaccacaagc ccagcaacac caaggtggac aagaaggtgg agcccaagag ctgcgacaaa 660
actcacacat gcccaccgtg cccagcacct gaagctgcag gggggaccgtc agtcttcctc 720
ttccccccaa aacccaagga caccctcatg atctcccgga cccctgaggt cacatgcgtg 780
gtggtggacg tgagccacga agaccctgag gtcaagttca actggtacgt ggacggcgtg 840
gaggtgcata atgccaagac aaagccgcgg gaggagcagt acaacagcac gtaccgtgtg 900
gtcagcgtcc tcaccgtcct gcaccaggac tggctgaatg gcaaggagta caagtgcaag 960
gtctccaaca aagccctcgg cgcccccatc gagaaaacca tctccaaagc caaagggcag 1020
ccccgagaac cacaggtgtg caccctgccc ccatcccggg atgagctgac caagaaccag 1080
gtcagcctct cgtgcgcagt caaaggcttc tatcccagcg acatcgccgt ggagtgggag 1140
agcaatgggc agccggagaa caactacaag accacgcctc ccgtgctgga ctccgacggc 1200

```
tccttcttcc tcgtgagcaa gctcaccgtg gacaagagca ggtggcagca ggggaacgtc     1260
ttctcatgct ccgtgatgca tgaggctctg cacaaccact acacgcagaa gagcctctcc     1320
ctgtctccgg gtggaggcgg cggaagcgga ggaggaggat ccagagaggg ccctgagctg     1380
agccctgatg atcctgccgg actgctggac ctgcggcagg gaatgtttgc ccagctggtg     1440
gcccagaacg tgctgctgat cgatggcccc ctgtcctggt acagcgatcc tggactggct     1500
ggcgtgtcac tgacaggcgg cctgagctac aaagaggaca ccaaagaact ggtggtggcc     1560
aaggccggcg tgtactacgt gttctttcag ctggaactgc ggagagtggt ggccggcgaa     1620
ggatctggct ctgtgtctct ggccctgcat ctgcagcctc tgagatctgc tgctggcgcc     1680
gctgctctgg cactgacagt ggatctgcct cctgccagca gcgaggcccg gaatagcgca     1740
tttgggtttc aaggcaggct gctgcacctg tctgccggcc agaggctggg agtgcatctg     1800
cacacagagg ccagggctag acacgcctgg cagctgacac agggcgctac agtgctgggc     1860
ctgttcagag tgacccccga gattcctgcc gggctc                               1896
```

<210> 198
<211> 632
<212> PRT
<213> Artificial Sequence

<220>
<223> DP47 Fc hole monomeric 4-1BBL (71-248) chain

<400> 198

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110
```

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Ala Ala Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Cys Thr Leu Pro Pro Ser
340 345 350

Arg Asp Glu Leu Thr Lys Asn Gln Val Ser Leu Ser Cys Ala Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Val Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Gly Gly Gly Gly
435 440 445

Ser Gly Gly Gly Gly Ser Arg Glu Gly Pro Glu Leu Ser Pro Asp Asp
450 455 460

Pro Ala Gly Leu Leu Asp Leu Arg Gln Gly Met Phe Ala Gln Leu Val
465 470 475 480

Ala Gln Asn Val Leu Leu Ile Asp Gly Pro Leu Ser Trp Tyr Ser Asp
485 490 495

Pro Gly Leu Ala Gly Val Ser Leu Thr Gly Gly Leu Ser Tyr Lys Glu
500 505 510

Asp Thr Lys Glu Leu Val Val Ala Lys Ala Gly Val Tyr Tyr Val Phe
515 520 525

Phe Gln Leu Glu Leu Arg Arg Val Val Ala Gly Glu Gly Ser Gly Ser
530 535 540

Val Ser Leu Ala Leu His Leu Gln Pro Leu Arg Ser Ala Ala Gly Ala
545 550 555 560

Ala Ala Leu Ala Leu Thr Val Asp Leu Pro Pro Ala Ser Ser Glu Ala
565 570 575

Arg Asn Ser Ala Phe Gly Phe Gln Gly Arg Leu Leu His Leu Ser Ala
580 585 590

Gly Gln Arg Leu Gly Val His Leu His Thr Glu Ala Arg Ala Arg His
595 600 605

Ala Trp Gln Leu Thr Gln Gly Ala Thr Val Leu Gly Leu Phe Arg Val

610 615 620

Thr Pro Glu Ile Pro Ala Gly Leu
625 630

<210> 199
<211> 2130
<212> DNA
<213> Artificial Sequence

<220>
<223> nucleotide sequence of dimeric 4-1BB ligand (71-248) - CL* Fc knob chain

<400> 199

agagagggcc ctgagctgag ccccgatgat cctgctggac tgctggacct gcggcagggc 60
atgtttgctc agctggtggc ccagaacgtg ctgctgatcg atggccccct gtcctggtac 120
agcgatcctg gactggctgg cgtgtcactg acaggcggcc tgagctacaa agaggacacc 180
aaagaactgg tggtggccaa ggccggcgtg tactacgtgt tctttcagct ggaactgcgg 240
agagtggtgg ccggcgaagg atctggctct gtgtctctgg ccctgcatct gcagcctctg 300
agatctgctg ctggcgccgc tgctctggca ctgacagtgg atctgcctcc tgccagcagc 360
gaggcccgga atagcgcatt tgggtttcaa ggcaggctgc tgcacctgtc tgccggccag 420
aggctgggag tgcatctgca cacagaggcc agggctagac acgcctggca gctgacacag 480
ggcgctacag tgctgggcct gttcagagtg acccccgaga ttccagccgg actgggaggc 540
ggcggatctg gcggcggagg atctagagaa ggacccgagc tgtcccctga cgatccagcc 600
gggctgctgg atctgagaca gggaatgttc gcccagctgg tggctcagaa tgtgctgctg 660
attgacggac ctctgagctg gtactccgac ccagggctgg caggggtgtc cctgactggg 720
ggactgtcct acaaagaaga tacaaaagaa ctggtggtgg ctaaagctgg ggtgtactat 780
gtgttttttc agctggaact gaggcgggtg gtggctgggg agggctcagg atctgtgtcc 840
ctggctctgc atctgcagcc actgcgctct gcagcagggg ctgcagcact ggccctgact 900
gtggacctgc cccccagcttc ttccgaggcc agaaacagcg ccttcgggtt ccaaggacgc 960
ctgctgcatc tgagcgccgg acagcgcctg ggagtgcatc tgcatactga agccagagcc 1020
cggcatgctt ggcagctgac tcagggggca actgtgctgg gactgtttcg cgtgacacct 1080
gagatccccg ctggactggg cggaggcggt tccggagggg gaggatctcg tacggtggct 1140
gcaccatctg tctttatctt cccacccagc gaccggaagc tgaagtctgg cacagccagc 1200
gtcgtgtgcc tgctgaataa cttctacccc cgcgaggcca aggtgcagtg gaaggtggac 1260
aatgccctgc agagcggcaa cagccaggaa agcgtgaccg agcaggacag caaggactcc 1320
acctacagcc tgagcagcac cctgaccctg agcaaggccg actacgagaa gcacaaggtg 1380

```
tacgcctgcg aagtgaccca ccagggcctg tctagccccg tgaccaagag cttcaaccgg     1440
ggcgagtgcg acaagaccca cacctgtcct ccatgccctg cccctgaagc tgctggcggc     1500
cctagcgtgt tcctgttccc cccaaagccc aaggacaccc tgatgatcag ccggacccct     1560
gaagtgacct gcgtggtggt ggatgtgtcc cacgaggacc ctgaagtgaa gttcaattgg     1620
tacgtggacg gcgtggaagt gcacaatgcc aagaccaagc cgcgggagga gcagtacaac     1680
agcacgtacc gtgtggtcag cgtcctcacc gtcctgcacc aggactggct gaatggcaag     1740
gagtacaagt gcaaggtctc caacaaagcc ctcggcgccc ccatcgagaa aaccatctcc     1800
aaagccaaag ggcagccccg agaaccacag gtgtacaccc tgcccccatg ccgggatgag     1860
ctgaccaaga accaggtcag cctgtggtgc ctggtcaaag gcttctatcc cagcgacatc     1920
gccgtggagt gggagagcaa tgggcagccg gagaacaact acaagaccac gcctcccgtg     1980
ctggactccg acggctcctt cttcctctac agcaagctca ccgtggacaa gagcaggtgg     2040
cagcagggga acgtcttctc atgctccgtg atgcatgagg ctctgcacaa ccactacacg     2100
cagaagagcc tctccctgtc tccgggtaaa                                      2130
```

<210> 200
<211> 873
<212> DNA
<213> Artificial Sequence

<220>
<223> nucleotide sequence of monomeric 4-1BBL (71-248) -CH1*

<400> 200

agagagggcc ctgagctgag ccccgatgat cctgctggac tgctggacct gcggcagggc 60
atgtttgctc agctggtggc ccagaacgtg ctgctgatcg atggcccct gtcctggtac 120
agcgatcctg gactggctgg cgtgtcactg acaggcggcc tgagctacaa agaggacacc 180
aaagaactgg tggtggccaa ggccggcgtg tactacgtgt tctttcagct ggaactgcgg 240
agagtggtgg ccggcgaagg atctggctct gtgtctctgg ccctgcatct gcagcctctg 300
agatctgctg ctggcgccgc tgctctggca ctgacagtgg atctgcctcc tgccagcagc 360
gaggcccgga atagcgcatt tgggtttcaa ggcaggctgc tgcacctgtc tgccggccag 420
aggctgggag tgcatctgca cacagaggcc agggctagac acgcctggca gctgacacag 480
ggcgctacag tgctgggcct gttcagagtg acccccgaga ttccagccgg actgggaggc 540
ggaggttccg gaggcggagg atctgctagc acaaagggcc ccagcgtgtt ccctctggcc 600
cctagcagca agagcacatc tggcggaaca gccgccctgg gctgcctggt ggaagattac 660
ttccccgagc ccgtgaccgt gtcctggaat tctggcgccc tgacaagcgg cgtgcacacc 720
tttccagccg tgctgcagag cagcggcctg tactctctga gcagcgtcgt gacagtgccc 780
agcagctctc tgggcaccca gacctacatc tgcaacgtga accacaagcc cagcaacacc 840

aaggtggacg agaaggtgga acccaagtcc tgc 873

<210> 201
<211> 1335
<212> DNA
<213> Artificial Sequence

<220>
<223> nucleotide sequence of DP47 Fc hole chain

<400> 201

gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagtgctag caccaagggc 360
ccatcggtct tccccctggc accctcctcc aagagcacct ctgggggcac agcggccctg 420
ggctgcctgg tcaaggacta cttccccgaa ccggtgacgg tgtcgtggaa ctcaggcgcc 480
ctgaccagcg gcgtgcacac cttcccggct gtcctacagt cctcaggact ctactccctc 540
agcagcgtgg tgaccgtgcc ctccagcagc ttgggcaccc agacctacat ctgcaacgtg 600
aatcacaagc ccagcaacac caaggtggac aagaaagttg agcccaaatc ttgtgacaaa 660
actcacacat gcccaccgtg cccagcacct gaagctgcag gggggaccgtc agtcttcctc 720
ttccccccaa aacccaagga caccctcatg atctcccgga cccctgaggt cacatgcgtg 780
gtggtggacg tgagccacga agaccctgag gtcaagttca actggtacgt ggacggcgtg 840
gaggtgcata atgccaagac aaagccgcgg gaggagcagt acaacagcac gtaccgtgtg 900
gtcagcgtcc tcaccgtcct gcaccaggac tggctgaatg gcaaggagta caagtgcaag 960
gtctccaaca aagccctcgg cgcccccatc gagaaaacca tctccaaagc caaagggcag 1020
ccccgagaac cacaggtgtg caccctgccc ccatcccggg atgagctgac caagaaccag 1080
gtcagcctct cgtgcgcagt caaaggcttc tatcccagcg acatcgccgt ggagtgggag 1140
agcaatgggc agccggagaa caactacaag accacgcctc ccgtgctgga ctccgacggc 1200
tccttcttcc tcgtgagcaa gctcaccgtg gacaagagca ggtggcagca ggggaacgtc 1260
ttctcatgct ccgtgatgca tgaggctctg cacaaccact acacgcagaa gagcctctcc 1320
ctgtctccgg gtaaa 1335

<210> 202
<211> 710
<212> PRT
<213> Artificial Sequence

<220>
<223> dimeric 4-1BB ligand (71-248) - CL* Fc knob chain

<400> 202

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Gly Gly Gly Gly Ser Gly Gly Gly Gly Ser Arg Glu Gly Pro
            180                 185                 190

Glu Leu Ser Pro Asp Asp Pro Ala Gly Leu Leu Asp Leu Arg Gln Gly
        195                 200                 205

Met Phe Ala Gln Leu Val Ala Gln Asn Val Leu Leu Ile Asp Gly Pro
    210                 215                 220
```

Leu Ser Trp Tyr Ser Asp Pro Gly Leu Ala Gly Val Ser Leu Thr Gly
225 230 235 240

Gly Leu Ser Tyr Lys Glu Asp Thr Lys Glu Leu Val Val Ala Lys Ala
245 250 255

Gly Val Tyr Tyr Val Phe Phe Gln Leu Glu Leu Arg Arg Val Val Ala
260 265 270

Gly Glu Gly Ser Gly Ser Val Ser Leu Ala Leu His Leu Gln Pro Leu
275 280 285

Arg Ser Ala Ala Gly Ala Ala Ala Leu Ala Leu Thr Val Asp Leu Pro
290 295 300

Pro Ala Ser Ser Glu Ala Arg Asn Ser Ala Phe Gly Phe Gln Gly Arg
305 310 315 320

Leu Leu His Leu Ser Ala Gly Gln Arg Leu Gly Val His Leu His Thr
325 330 335

Glu Ala Arg Ala Arg His Ala Trp Gln Leu Thr Gln Gly Ala Thr Val
340 345 350

Leu Gly Leu Phe Arg Val Thr Pro Glu Ile Pro Ala Gly Leu Gly Gly
355 360 365

Gly Gly Ser Gly Gly Gly Gly Ser Arg Thr Val Ala Ala Pro Ser Val
370 375 380

Phe Ile Phe Pro Pro Ser Asp Arg Lys Leu Lys Ser Gly Thr Ala Ser
385 390 395 400

Val Val Cys Leu Leu Asn Asn Phe Tyr Pro Arg Glu Ala Lys Val Gln
405 410 415

Trp Lys Val Asp Asn Ala Leu Gln Ser Gly Asn Ser Gln Glu Ser Val
420 425 430

Thr Glu Gln Asp Ser Lys Asp Ser Thr Tyr Ser Leu Ser Ser Thr Leu
435 440 445

Thr Leu Ser Lys Ala Asp Tyr Glu Lys His Lys Val Tyr Ala Cys Glu
450 455 460

Val Thr His Gln Gly Leu Ser Ser Pro Val Thr Lys Ser Phe Asn Arg
465 470 475 480

Gly Glu Cys Asp Lys Thr His Thr Cys Pro Pro Cys Pro Ala Pro Glu
485 490 495

Ala Ala Gly Gly Pro Ser Val Phe Leu Phe Pro Pro Lys Pro Lys Asp
500 505 510

Thr Leu Met Ile Ser Arg Thr Pro Glu Val Thr Cys Val Val Val Asp
515 520 525

Val Ser His Glu Asp Pro Glu Val Lys Phe Asn Trp Tyr Val Asp Gly
530 535 540

Val Glu Val His Asn Ala Lys Thr Lys Pro Arg Glu Glu Gln Tyr Asn
545 550 555 560

Ser Thr Tyr Arg Val Val Ser Val Leu Thr Val Leu His Gln Asp Trp
565 570 575

Leu Asn Gly Lys Glu Tyr Lys Cys Lys Val Ser Asn Lys Ala Leu Gly
580 585 590

Ala Pro Ile Glu Lys Thr Ile Ser Lys Ala Lys Gly Gln Pro Arg Glu
595 600 605

Pro Gln Val Tyr Thr Leu Pro Pro Cys Arg Asp Glu Leu Thr Lys Asn
610 615 620

Gln Val Ser Leu Trp Cys Leu Val Lys Gly Phe Tyr Pro Ser Asp Ile
625 630 635 640

Ala Val Glu Trp Glu Ser Asn Gly Gln Pro Glu Asn Asn Tyr Lys Thr
645 650 655

Thr Pro Pro Val Leu Asp Ser Asp Gly Ser Phe Phe Leu Tyr Ser Lys
660 665 670

Leu Thr Val Asp Lys Ser Arg Trp Gln Gln Gly Asn Val Phe Ser Cys
675 680 685

Ser Val Met His Glu Ala Leu His Asn His Tyr Thr Gln Lys Ser Leu
690 695 700

Ser Leu Ser Pro Gly Lys
705 710

<210> 203
<211> 291

<212> PRT
<213> Artificial Sequence

<220>
<223> monomeric 4-1BBL (71-248) -CH1*

<400> 203

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160

Gly Ala Thr Val Leu Gly Leu Phe Arg Val Thr Pro Glu Ile Pro Ala
                165                 170                 175

Gly Leu Gly Gly Gly Gly Ser Gly Gly Gly Gly Ser Ala Ser Thr Lys
            180                 185                 190

Gly Pro Ser Val Phe Pro Leu Ala Pro Ser Ser Lys Ser Thr Ser Gly
        195                 200                 205

Gly Thr Ala Ala Leu Gly Cys Leu Val Glu Asp Tyr Phe Pro Glu Pro
    210                 215                 220
```

Val Thr Val Ser Trp Asn Ser Gly Ala Leu Thr Ser Gly Val His Thr
225 230 235 240

Phe Pro Ala Val Leu Gln Ser Ser Gly Leu Tyr Ser Leu Ser Ser Val
245 250 255

Val Thr Val Pro Ser Ser Ser Leu Gly Thr Gln Thr Tyr Ile Cys Asn
260 265 270

Val Asn His Lys Pro Ser Asn Thr Lys Val Asp Glu Lys Val Glu Pro
275 280 285

Lys Ser Cys
290

<210> 204
<211> 445
<212> PRT
<213> Artificial Sequence

<220>
<223> DP47 Fc hole chain

<400> 204

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Ala Ala Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Gly Ala Pro Ile Glu Lys Thr Ile Ser Lys
325 330 335

Ala Lys Gly Gln Pro Arg Glu Pro Gln Val Cys Thr Leu Pro Pro Ser
340 345 350

Arg Asp Glu Leu Thr Lys Asn Gln Val Ser Leu Ser Cys Ala Val Lys
355 360 365

Gly Phe Tyr Pro Ser Asp Ile Ala Val Glu Trp Glu Ser Asn Gly Gln
370 375 380

```
Pro Glu Asn Asn Tyr Lys Thr Thr Pro Pro Val Leu Asp Ser Asp Gly
385                 390                 395                 400

Ser Phe Phe Leu Val Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445
```

<210> 205
<211> 1335
<212> DNA
<213> Artificial Sequence

<220>
<223> nucleotide sequence of DP47 heavy chain (huIgG1 PGLALA)

<400> 205

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagtgctag caccaagggc     360
ccatcggtct tccccctggc accctcctcc aagagcacct ctgggggcac agcggccctg     420
ggctgcctgg tcaaggacta cttccccgaa ccggtgacgg tgtcgtggaa ctcaggcgcc     480
ctgaccagcg gcgtgcacac cttcccggct gtcctacagt cctcaggact ctactccctc     540
agcagcgtgg tgaccgtgcc ctccagcagc ttgggcaccc agacctacat ctgcaacgtg     600
aatcacaagc ccagcaacac caaggtggac aagaaagttg agcccaaatc ttgtgacaaa     660
actcacacat gcccaccgtg cccagcacct gaagctgcag ggggaccgtc agtcttcctc     720
ttccccccaa aacccaagga caccctcatg atctcccgga cccctgaggt cacatgcgtg     780
gtggtggacg tgagccacga agaccctgag gtcaagttca actggtacgt ggacggcgtg     840
gaggtgcata atgccaagac aaagccgcgg gaggagcagt acaacagcac gtaccgtgtg     900
gtcagcgtcc tcaccgtcct gcaccaggac tggctgaatg gcaaggagta caagtgcaag     960
gtctccaaca aagccctcgg cgcccccatc gagaaaacca tctccaaagc caaagggcag    1020
ccccgagaac cacaggtgta caccctgccc ccatcccggg atgagctgac caagaaccag    1080
gtcagcctga cctgcctggt caaaggcttc tatcccagcg acatcgccgt ggagtgggag    1140
agcaatgggc agccggagaa caactacaag accacgcctc ccgtgctgga ctccgacggc    1200
tccttcttcc tctacagcaa gctcaccgtg gacaagagca ggtggcagca ggggaacgtc    1260
ttctcatgct ccgtgatgca tgaggctctg cacaaccact acacgcagaa gagcctctcc    1320
ctgtctccgg gtaaa                                                     1335
```

<210> 206
<211> 445
<212> PRT
<213> Artificial Sequence

<220>
<223> DP47 heavy chain (huIgG1 PGLALA)

<400> 206

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

```
Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Ala Ala Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Gly Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430
```

His Tyr Thr Gln Lys Ser Leu Ser Leu Ser Pro Gly Lys
435 440 445

<210> 207
<211> 594
<212> DNA
<213> Artificial Sequence

<220>
<223> nucleotide sequence of human 4-1BB His

<400> 207

ctgcaggacc cctgcagcaa ctgccctgcc ggcaccttct gcgacaacaa ccggaaccag 60

atctgcagcc cctgcccccc caacagcttc agctctgccg gcggacagcg gacctgcgac 120

atctgcagac agtgcaaggg cgtgttcaga acccggaaag agtgcagcag caccagcaac 180

gccgagtgcg actgcacccc cggcttccat tgtctgggag ccggctgcag catgtgcgag 240

caggactgca agcagggcca ggaactgacc aagaagggct gcaaggactg ctgcttcggc 300

accttcaacg accagaagcg gggcatctgc cggccctgga ccaactgtag cctggacggc 360

aagagcgtgc tggtcaacgg caccaaagaa cgggacgtcg tgtgcggccc cagccctgct 420

gatctgtctc ctggggccag cagcgtgacc cctcctgccc ctgccagaga gcctggccac 480

tctcctcagg tcgacgaaca gttatatttt cagggcggct caggcctgaa cgacatcttc 540

gaggcccaga agatcgagtg gcacgaggct cgagctcacc accatcacca tcac 594

<210> 208
<211> 198
<212> PRT
<213> Artificial Sequence

<220>
<223> human 4-1BB His

<400> 208

```
Leu Gln Asp Pro Cys Ser Asn Cys Pro Ala Gly Thr Phe Cys Asp Asn
1               5                   10                  15

Asn Arg Asn Gln Ile Cys Ser Pro Cys Pro Pro Asn Ser Phe Ser Ser
            20                  25                  30

Ala Gly Gly Gln Arg Thr Cys Asp Ile Cys Arg Gln Cys Lys Gly Val
        35                  40                  45

Phe Arg Thr Arg Lys Glu Cys Ser Ser Thr Ser Asn Ala Glu Cys Asp
    50                  55                  60

Cys Thr Pro Gly Phe His Cys Leu Gly Ala Gly Cys Ser Met Cys Glu

65                  70                  75                  80

Gln Asp Cys Lys Gln Gly Gln Glu Leu Thr Lys Lys Gly Cys Lys Asp
                85                  90                  95

Cys Cys Phe Gly Thr Phe Asn Asp Gln Lys Arg Gly Ile Cys Arg Pro
            100                 105                 110

Trp Thr Asn Cys Ser Leu Asp Gly Lys Ser Val Leu Val Asn Gly Thr
        115                 120                 125

Lys Glu Arg Asp Val Val Cys Gly Pro Ser Pro Ala Asp Leu Ser Pro
    130                 135                 140

Gly Ala Ser Ser Val Thr Pro Pro Ala Pro Ala Arg Glu Pro Gly His
145                 150                 155                 160

Ser Pro Gln Val Asp Glu Gln Leu Tyr Phe Gln Gly Gly Ser Gly Leu
                165                 170                 175

Asn Asp Ile Phe Glu Ala Gln Lys Ile Glu Trp His Glu Ala Arg Ala
            180                 185                 190

His His His His His His
        195
```

<210> 209
<211> 2502
<212> DNA
<213> Artificial Sequence

<220>
<223> nucleotide sequence of anti-FAP(4B9) Fc hole dimeric 4-1BB ligand (71-254) chain

<400> 209

```
gaggtgcagc tgctcgaaag cggcggagga ctggtgcagc ctggcggcag cctgagactg      60
tcttgcgccg ccagcggctt caccttcagc agctacgcca tgagctgggt ccgccaggcc     120
cctggcaagg gactggaatg ggtgtccgcc atcatcggct ctggcgccag cacctactac     180
gccgacagcg tgaagggccg gttcaccatc agccgggaca acagcaagaa caccctgtac     240
ctgcagatga acagcctgcg ggccgaggac accgccgtgt actactgcgc caagggatgg     300
ttcggcggct tcaactactg gggacagggc accctggtca cagtgtccag cgctagcacc     360
aagggcccct ccgtgttccc cctggccccc agcagcaaga gcaccagcgg cggcacagcc     420
gctctgggct gcctggtcaa ggactacttc cccgagcccg tgaccgtgtc ctggaacagc     480
ggagccctga cctccggcgt gcacaccttc cccgccgtgc tgcagagttc tggcctgtat     540
```

agcctgagca gcgtggtcac cgtgccttct agcagcctgg gcacccagac ctacatctgc 600

aacgtgaacc acaagcccag caacaccaag gtggacaaga aggtggagcc caagagctgc 660

gacaaaactc acacatgccc accgtgccca gcacctgaag ctgcaggggg accgtcagtc 720

ttcctcttcc ccccaaaacc caaggacacc ctcatgatct cccggacccc tgaggtcaca 780

tgcgtggtgg tggacgtgag ccacgaagac cctgaggtca agttcaactg gtacgtggac 840

ggcgtggagg tgcataatgc caagacaaag ccgcgggagg agcagtacaa cagcacgtac 900

cgtgtggtca gcgtcctcac cgtcctgcac caggactggc tgaatggcaa ggagtacaag 960

tgcaaggtct ccaacaaagc cctcggcgcc cccatcgaga aaaccatctc caaagccaaa 1020

gggcagcccc gagaaccaca ggtgtgcacc ctgcccccat cccgggatga gctgaccaag 1080

aaccaggtca gcctctcgtg cgcagtcaaa ggcttctatc ccagcgacat cgccgtggag 1140

tgggagagca atgggcagcc ggagaacaac tacaagacca cgcctcccgt gctggactcc 1200

gacggctcct tcttcctcgt gagcaagctc accgtggaca agagcaggtg gcagcagggg 1260

aacgtcttct catgctccgt gatgcatgag gctctgcaca accactacac gcagaagagc 1320

ctctccctgt ctccgggtgg aggcggcgga agcggaggag gaggatccag agagggccct 1380

gagctgagcc ccgatgatcc tgctggactg ctggacctgc ggcagggcat gtttgctcag 1440

ctggtggccc agaacgtgct gctgatcgat ggccccctgt cctggtacag cgatcctgga 1500

ctggctggcg tgtcactgac aggcggcctg agctacaaag aggacaccaa agaactggtg 1560

gtggccaagg ccggcgtgta ctacgtgttc tttcagctgg aactgcggag agtggtggcc 1620

ggcgaaggat ctggctctgt gtctctggcc ctgcatctgc agcctctgag aagcgctgct 1680

ggcgctgcag ctctggcact gacagtggat ctgcctcctg ccagctccga ggcccggaat 1740

agcgcatttg ggtttcaagg caggctgctg cacctgtctg ccggccagag gctgggagtg 1800

catctgcaca cagaggccag ggctagacac gcctggcagc tgacacaggg cgctacagtg 1860

ctgggcctgt tcagagtgac ccccgagatt ccagccggcc tgccttctcc aagaagcgaa 1920

ggcggaggcg gatctggcgg cggaggatct agagagggac ccgaactgtc ccctgacgat 1980

ccagccgggc tgctggatct gagacaggga atgttcgccc agctggtggc tcagaatgtg 2040

ctgctgattg acggacctct gagctggtac tccgacccag ggctggcagg ggtgtccctg 2100

actgggggac tgtcctacaa agaagataca aaagaactgg tggtggctaa agctggggtg 2160

tactatgtgt tttttcagct ggaactgagg cgggtggtgg ctggggaggg ctcaggatct 2220

gtgtccctgg ctctgcatct gcagccactg cgctctgctg ctggcgcagc tgcactggct 2280

ctgactgtgg acctgccacc agcctctagc gaggccagaa acagcgcctt cgggttccaa 2340

ggacgcctgc tgcatctgag cgccggacag cgcctgggag tgcatctgca tactgaagcc 2400

agagcccggc atgcttggca gctgactcag gggcaactg tgctgggact gtttcgcgtg 2460

acacctgaga tccctgccgg actgccaagc cctagatcag aa 2502

<210> 210
<211> 1926
<212> DNA
<213> Artificial Sequence

<220>
<223> nucleotide sequence of DP47 (VHCL) Fc knob monomeric 4-1BB (71-254) ligand

<400> 210

gaggtgcaat tgttggagtc tgggggaggc ttggtacagc ctggggggtc cctgagactc 60

tcctgtgcag cctccggatt cacctttagc agttatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcagatga acagcctgag agccgaggac acggccgtat attactgtgc gaaaggcagc 300

ggatttgact actggggcca aggaaccctg gtcaccgtct cgagcgctag tgtggccgct 360

ccctccgtgt ttatctttcc cccatccgat gaacagctga aaagcggcac cgcctccgtc 420

gtgtgtctgc tgaacaattt ttaccctagg gaagctaaag tgcagtggaa agtggataac 480

gcactgcagt ccggcaactc ccaggaatct gtgacagaac aggactccaa ggacagcacc 540

tactccctgt cctccaccct gacactgtct aaggctgatt atgagaaaca caaagtctac 600

gcctgcgaag tcacccatca gggcctgagc tcgcccgtca caaagagctt caacagggga 660

gagtgtgaca agacccacac ctgtccccct tgtcctgccc ctgaagctgc tggcggccct 720

tctgtgttcc tgttcccccc aaagcccaag gacaccctga tgatcagccg gacccccgaa 780

gtgacctgcg tggtggtgga tgtgtcccac gaggaccctg aagtgaagtt caattggtac 840

gtggacggcg tggaagtgca caacgccaag acaaagccgc gggaggagca gtacaacagc 900

acgtaccgtg tggtcagcgt cctcaccgtc ctgcaccagg actggctgaa tggcaaggag 960

tacaagtgca aggtctccaa caaagccctc ggcgccccca tcgagaaaac catctccaaa 1020

gccaaagggc agccccgaga accacaggtg tacaccctgc ccccctgcag agatgagctg 1080

accaagaacc aggtgtccct gtggtgtctg gtcaagggct tctaccccag cgatatcgcc 1140

gtggagtggg agagcaacgg ccagcctgag aacaactaca agaccacccc ccctgtgctg 1200

gacagcgacg gcagcttctt cctgtactcc aaactgaccg tggacaagag ccggtggcag 1260

cagggcaacg tgttcagctg cagcgtgatg cacgaggccc tgcacaacca ctacacccag 1320

aagtccctga gcctgagccc cggcggaggc ggcggaagcg gaggaggagg atccagagag 1380

ggccctgagc tgagccccga tgatcctgct ggactgctgg acctgcggca gggcatgttt 1440

gctcagctgg tggcccagaa cgtgctgctg atcgatggcc ccctgtcctg gtacagcgat 1500

cctggactgg ctggcgtgtc actgacaggc ggcctgagct acaaagagga caccaaagaa 1560

ctggtggtgg ccaaggccgg cgtgtactac gtgttctttc agctggaact gcggagagtg 1620

gtggccggcg aaggatctgg ctctgtgtct ctggccctgc atctgcagcc tctgagaagc 1680

gctgctggcg ctgcagctct ggcactgaca gtggatctgc ctcctgccag ctccgaggcc 1740

cggaatagcg catttgggtt tcaaggcagg ctgctgcacc tgtctgccgg ccagaggctg 1800

ggagtgcatc tgcacacaga ggccagggct agacacgcct ggcagctgac acagggcgct 1860

acagtgctgg gcctgttcag agtgaccccc gagattccag ccggcctgcc ttctccaaga 1920

agcgaa 1926

<210> 211
<211> 639
<212> DNA
<213> Artificial Sequence

<220>
<223> nucleotide sequence of DP47 (VL-CH1) light chain

<400> 211

gaaatcgtgt taacgcagtc tccaggcacc ctgtctttgt ctccagggga aagagccacc 60

ctctcttgca gggccagtca gagtgttagc agcagctact tagcctggta ccagcagaaa 120

cctggccagg ctcccaggct cctcatctat ggagcatcca gcagggccac tggcatccca 180

gacaggttca gtggcagtgg atccgggaca gacttcactc tcaccatcag cagactggag 240

cctgaagatt ttgcagtgta ttactgtcag cagtatggta gctcaccgct gacgttcggc 300

caggggacca aagtggaaat caagagctcc gctagcacca aaggcccttc cgtgtttcct 360

ctggctccta gctccaagtc cacctctgga ggcaccgctg ctctcggatg cctcgtgaag 420

gattattttc ctgagcctgt gacagtgtcc tggaatagcg gagcactgac ctctggagtg 480

catactttcc ccgctgtgct gcagtcctct ggactgtaca gcctgagcag cgtggtgaca 540

gtgcccagca gcagcctggg cacccagacc tacatctgca acgtgaacca caagcccagc 600

aacaccaagg tggacaagaa ggtggaaccc aagtcttgt 639

<210> 212
<211> 834
<212> PRT
<213> Artificial Sequence

<220>
<223> anti-FAP(4B9) Fc hole dimeric 4-1BB ligand (71-254) chain

<400> 212

```
Glu Val Gln Leu Leu Glu Ser Gly Gly Gly Leu Val Gln Pro Gly Gly
1               5                   10                  15
```

```
Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270
```

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Cys Thr Leu Pro
340 345 350

Pro Ser Arg Asp Glu Leu Thr Lys Asn Gln Val Ser Leu Ser Cys Ala
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Val Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Arg Glu Gly Pro Glu Leu Ser Pro
450 455 460

Asp Asp Pro Ala Gly Leu Leu Asp Leu Arg Gln Gly Met Phe Ala Gln
465 470 475 480

Leu Val Ala Gln Asn Val Leu Leu Ile Asp Gly Pro Leu Ser Trp Tyr
485 490 495

Ser Asp Pro Gly Leu Ala Gly Val Ser Leu Thr Gly Gly Leu Ser Tyr
500 505 510

Lys Glu Asp Thr Lys Glu Leu Val Val Ala Lys Ala Gly Val Tyr Tyr

```
            515                 520                 525

Val Phe Phe Gln Leu Glu Leu Arg Arg Val Val Ala Gly Glu Gly Ser
    530                 535                 540

Gly Ser Val Ser Leu Ala Leu His Leu Gln Pro Leu Arg Ser Ala Ala
545                 550                 555                 560

Gly Ala Ala Ala Leu Ala Leu Thr Val Asp Leu Pro Pro Ala Ser Ser
                565                 570                 575

Glu Ala Arg Asn Ser Ala Phe Gly Phe Gln Gly Arg Leu Leu His Leu
            580                 585                 590

Ser Ala Gly Gln Arg Leu Gly Val His Leu His Thr Glu Ala Arg Ala
        595                 600                 605

Arg His Ala Trp Gln Leu Thr Gln Gly Ala Thr Val Leu Gly Leu Phe
    610                 615                 620

Arg Val Thr Pro Glu Ile Pro Ala Gly Leu Pro Ser Pro Arg Ser Glu
625                 630                 635                 640

Gly Gly Gly Gly Ser Gly Gly Gly Gly Ser Arg Glu Gly Pro Glu Leu
                645                 650                 655

Ser Pro Asp Asp Pro Ala Gly Leu Leu Asp Leu Arg Gln Gly Met Phe
            660                 665                 670

Ala Gln Leu Val Ala Gln Asn Val Leu Leu Ile Asp Gly Pro Leu Ser
        675                 680                 685

Trp Tyr Ser Asp Pro Gly Leu Ala Gly Val Ser Leu Thr Gly Gly Leu
    690                 695                 700

Ser Tyr Lys Glu Asp Thr Lys Glu Leu Val Val Ala Lys Ala Gly Val
705                 710                 715                 720

Tyr Tyr Val Phe Phe Gln Leu Glu Leu Arg Arg Val Val Ala Gly Glu
                725                 730                 735

Gly Ser Gly Ser Val Ser Leu Ala Leu His Leu Gln Pro Leu Arg Ser
            740                 745                 750

Ala Ala Gly Ala Ala Ala Leu Ala Leu Thr Val Asp Leu Pro Pro Ala
        755                 760                 765
```

Ser Ser Glu Ala Arg Asn Ser Ala Phe Gly Phe Gln Gly Arg Leu Leu
    770                 775                 780

His Leu Ser Ala Gly Gln Arg Leu Gly Val His Leu His Thr Glu Ala
785                 790                 795                 800

Arg Ala Arg His Ala Trp Gln Leu Thr Gln Gly Ala Thr Val Leu Gly
                805                 810                 815

Leu Phe Arg Val Thr Pro Glu Ile Pro Ala Gly Leu Pro Ser Pro Arg
            820                 825                 830

Ser Glu

<210> 213
<211> 642
<212> PRT
<213> Artificial Sequence

<220>
<223> DP47 (VH-CL) Fc knob monomeric 4-1BB (71-254) ligand

<400> 213

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Val Ala Ala Pro Ser Val Phe Ile Phe Pro Pro
        115                 120                 125

Ser Asp Glu Gln Leu Lys Ser Gly Thr Ala Ser Val Val Cys Leu Leu
```

```
        130                 135                 140

Asn Asn Phe Tyr Pro Arg Glu Ala Lys Val Gln Trp Lys Val Asp Asn
145                 150                 155                 160

Ala Leu Gln Ser Gly Asn Ser Gln Glu Ser Val Thr Glu Gln Asp Ser
                165                 170                 175

Lys Asp Ser Thr Tyr Ser Leu Ser Ser Thr Leu Thr Leu Ser Lys Ala
            180                 185                 190

Asp Tyr Glu Lys His Lys Val Tyr Ala Cys Glu Val Thr His Gln Gly
        195                 200                 205

Leu Ser Ser Pro Val Thr Lys Ser Phe Asn Arg Gly Glu Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Cys Arg Asp Glu Leu Thr Lys Asn Gln Val Ser Leu Trp
        355                 360                 365

Cys Leu Val Lys Gly Phe Tyr Pro Ser Asp Ile Ala Val Glu Trp Glu
    370                 375                 380
```

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Gly Gly Gly Gly Ser Gly Gly Gly Gly Ser Arg Glu Gly Pro Glu Leu
450 455 460

Ser Pro Asp Asp Pro Ala Gly Leu Leu Asp Leu Arg Gln Gly Met Phe
465 470 475 480

Ala Gln Leu Val Ala Gln Asn Val Leu Leu Ile Asp Gly Pro Leu Ser
485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu Pro Ser Pro Arg
625 630 635 640

Ser Glu

<210> 214
<211> 213
<212> PRT
<213> Artificial Sequence

<220>
<223> DP47 (VL-CH1) light chain

<400> 214

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Leu Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190
```

```
Cys Asn Val Asn His Lys Pro Ser Asn Thr Lys Val Asp Lys Lys Val
        195                 200                 205

Glu Pro Lys Ser Cys
    210
```

<210> 215
<211> 1920
<212> DNA
<213> Artificial Sequence

<220>
<223> nucleotide sequence of anti-FAP(4B9) Fc hole monomeric 4-1BBL (71-254) chain

<400> 215

gaggtgcagc tgctcgaaag cggcggagga ctggtgcagc ctggcggcag cctgagactg 60
tcttgcgccg ccagcggctt caccttcagc agctacgcca tgagctgggt ccgccaggcc 120
cctggcaagg gactggaatg ggtgtccgcc atcatcggct ctggcgccag cacctactac 180
gccgacagcg tgaagggccg gttcaccatc agccgggaca acagcaagaa caccctgtac 240
ctgcagatga acagcctgcg ggccgaggac accgccgtgt actactgcgc caagggatgg 300
ttcggcggct tcaactactg gggacagggc accctggtca cagtgtccag cgctagcacc 360
aagggcccct ccgtgttccc cctggccccc agcagcaaga gcaccagcgg cggcacagcc 420
gctctgggct gcctggtcaa ggactacttc cccgagcccg tgaccgtgtc ctggaacagc 480
ggagccctga cctccggcgt gcacaccttc cccgccgtgc tgcagagttc tggcctgtat 540
agcctgagca gcgtggtcac cgtgccttct agcagcctgg gcacccagac ctacatctgc 600
aacgtgaacc acaagcccag caacaccaag gtggacaaga aggtggagcc caagagctgc 660
gacaaaactc acacatgccc accgtgccca gcacctgaag ctgcaggggg accgtcagtc 720
ttcctcttcc ccccaaaacc caaggacacc ctcatgatct cccggacccc tgaggtcaca 780
tgcgtggtgg tggacgtgag ccacgaagac cctgaggtca agttcaactg gtacgtggac 840
ggcgtggagg tgcataatgc caagacaaag ccgcgggagg agcagtacaa cagcacgtac 900
cgtgtggtca gcgtcctcac cgtcctgcac caggactggc tgaatggcaa ggagtacaag 960
tgcaaggtct ccaacaaagc cctcggcgcc cccatcgaga aaaccatctc caaagccaaa 1020
gggcagcccc gagaaccaca ggtgtgcacc ctgcccccat cccgggatga gctgaccaag 1080
aaccaggtca gcctctcgtg cgcagtcaaa ggcttctatc ccagcgacat cgccgtggag 1140
tgggagagca atgggcagcc ggagaacaac tacaagacca cgcctcccgt gctggactcc 1200
gacggctcct tcttcctcgt gagcaagctc accgtggaca agagcaggtg gcagcagggg 1260
aacgtcttct catgctccgt gatgcatgag gctctgcaca accactacac gcagaagagc 1320
ctctccctgt ctccgggtgg aggcggcgga agcggaggag gaggatccag agagggccct 1380

gagctgagcc ccgatgatcc tgctggactg ctggacctgc ggcagggcat gtttgctcag 1440

ctggtggccc agaacgtgct gctgatcgat ggcccctgt cctggtacag cgatcctgga 1500

ctggctggcg tgtcactgac aggcggcctg agctacaaag aggacaccaa agaactggtg 1560

gtggccaagg ccggcgtgta ctacgtgttc tttcagctgg aactgcggag agtggtggcc 1620

ggcgaaggat ctggctctgt gtctctggcc ctgcatctgc agcctctgag aagcgctgct 1680

ggcgctgcag ctctggcact gacagtggat ctgcctcctg ccagctccga ggcccggaat 1740

agcgcatttg ggtttcaagg caggctgctg cacctgtctg ccggccagag gctgggagtg 1800

catctgcaca cagaggccag ggctagacac gcctggcagc tgacacaggg cgctacagtg 1860

ctgggcctgt tcagagtgac ccccgagatt ccagccggcc tgccttctcc aagaagcgaa 1920

<210> 216
<211> 2508
<212> DNA
<213> Artificial Sequence

<220>
<223> nucleotide sequence of DP47 (VH-CL) Fc knob dimeric 4-1BB ligand (71-254) chain

<400> 216

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagcgctag tgtggccgct     360
ccctccgtgt ttatctttcc cccatccgat gaacagctga aaagcggcac cgcctccgtc     420
gtgtgtctgc tgaacaattt ttaccctagg gaagctaaag tgcagtggaa agtggataac     480
gcactgcagt ccggcaactc ccaggaatct gtgacagaac aggactccaa ggacagcacc     540
tactccctgt cctccaccct gacactgtct aaggctgatt atgagaaaca caaagtctac     600
gcctgcgaag tcacccatca gggcctgagc tcgcccgtca caaagagctt caacagggga     660
gagtgtgaca agacccacac ctgtccccct tgtcctgccc ctgaagctgc tggcggccct     720
tctgtgttcc tgttcccccc aaagcccaag gacaccctga tgatcagccg gacccccgaa     780
gtgacctgcg tggtggtgga tgtgtcccac gaggaccctg aagtgaagtt caattggtac     840
gtggacggcg tggaagtgca caacgccaag acaaagccgc gggaggagca gtacaacagc     900
acgtaccgtg tggtcagcgt cctcaccgtc ctgcaccagg actggctgaa tggcaaggag     960
tacaagtgca aggtctccaa caaagccctc ggcgccccca tcgagaaaac catctccaaa    1020
```

gccaaagggc agccccgaga accacaggtg tacaccctgc ccccctgcag agatgagctg 1080

accaagaacc aggtgtccct gtggtgtctg gtcaagggct tctaccccag cgatatcgcc 1140

gtggagtggg agagcaacgg ccagcctgag aacaactaca agaccacccc ccctgtgctg 1200

gacagcgacg gcagcttctt cctgtactcc aaactgaccg tggacaagag ccggtggcag 1260

cagggcaacg tgttcagctg cagcgtgatg cacgaggccc tgcacaacca ctacacccag 1320

aagtccctga gcctgagccc cggcggaggc ggcggaagcg gaggaggagg atccagagag 1380

ggccctgagc tgagccccga tgatcctgct ggactgctgg acctgcggca gggcatgttt 1440

gctcagctgg tggcccagaa cgtgctgctg atcgatggcc ccctgtcctg gtacagcgat 1500

cctggactgg ctggcgtgtc actgacaggc ggcctgagct acaaagagga caccaaagaa 1560

ctggtggtgg ccaaggccgg cgtgtactac gtgttctttc agctggaact gcggagagtg 1620

gtggccggcg aaggatctgg ctctgtgtct ctggccctgc atctgcagcc tctgagaagc 1680

gctgctggcg ctgcagctct ggcactgaca gtggatctgc ctcctgccag ctccgaggcc 1740

cggaatagcg catttgggtt tcaaggcagg ctgctgcacc tgtctgccgg ccagaggctg 1800

ggagtgcatc tgcacacaga ggccagggct agacacgcct ggcagctgac acagggcgct 1860

acagtgctgg gcctgttcag agtgaccccc gagattccag ccggcctgcc ttctccaaga 1920

agcgaaggcg gaggcggatc tggcggcgga ggatctagag agggacccga actgtcccct 1980

gacgatccag ccgggctgct ggatctgaga cagggaatgt tcgcccagct ggtggctcag 2040

aatgtgctgc tgattgacgg acctctgagc tggtactccg acccagggct ggcaggggtg 2100

tccctgactg ggggactgtc ctacaaagaa gatacaaaag aactggtggt ggctaaagct 2160

ggggtgtact atgtgttttt tcagctggaa ctgaggcggg tggtggctgg ggagggctca 2220

ggatctgtgt ccctggctct gcatctgcag ccactgcgct ctgctgctgg cgcagctgca 2280

ctggctctga ctgtggacct gccaccagcc tctagcgagg ccagaaacag cgccttcggg 2340

ttccaaggac gcctgctgca tctgagcgcc ggacagcgcc tgggagtgca tctgcatact 2400

gaagccagag cccggcatgc ttggcagctg actcaggggg caactgtgct gggactgttt 2460

cgcgtgacac ctgagatccc tgccggactg ccaagcccta gatcagaa 2508

<210> 217
<211> 640
<212> PRT
<213> Artificial Sequence

<220>
<223> anti-FAP(4B9) Fc hole monomeric 4-1BBL (71-254) chain

<400> 217

Glu Val Gln Leu Leu Glu Ser Gly Gly Gly Leu Val Gln Pro Gly Gly

1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ile Gly Ser Gly Ala Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Trp Phe Gly Gly Phe Asn Tyr Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Cys Thr Leu Pro
340 345 350

Pro Ser Arg Asp Glu Leu Thr Lys Asn Gln Val Ser Leu Ser Cys Ala
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Val Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Arg Glu Gly Pro Glu Leu Ser Pro
450 455 460

Asp Asp Pro Ala Gly Leu Leu Asp Leu Arg Gln Gly Met Phe Ala Gln
465 470 475 480

Leu Val Ala Gln Asn Val Leu Leu Ile Asp Gly Pro Leu Ser Trp Tyr
485 490 495

Ser Asp Pro Gly Leu Ala Gly Val Ser Leu Thr Gly Gly Leu Ser Tyr
500 505 510

Lys Glu Asp Thr Lys Glu Leu Val Val Ala Lys Ala Gly Val Tyr Tyr
515 520 525

Val Phe Phe Gln Leu Glu Leu Arg Arg Val Val Ala Gly Glu Gly Ser
530 535 540

Gly Ser Val Ser Leu Ala Leu His Leu Gln Pro Leu Arg Ser Ala Ala
545 550 555 560

Gly Ala Ala Ala Leu Ala Leu Thr Val Asp Leu Pro Pro Ala Ser Ser
565 570 575

Glu Ala Arg Asn Ser Ala Phe Gly Phe Gln Gly Arg Leu Leu His Leu
580 585 590

Ser Ala Gly Gln Arg Leu Gly Val His Leu His Thr Glu Ala Arg Ala
595 600 605

Arg His Ala Trp Gln Leu Thr Gln Gly Ala Thr Val Leu Gly Leu Phe
610 615 620

Arg Val Thr Pro Glu Ile Pro Ala Gly Leu Pro Ser Pro Arg Ser Glu
625 630 635 640

<210> 218
<211> 836
<212> PRT
<213> Artificial Sequence

<220>
<223> DP47 (VH-CL) Fc knob dimeric 4-1BB ligand (71-254) chain

<400> 218

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Val Ala Ala Pro Ser Val Phe Ile Phe Pro Pro
115 120 125

Ser Asp Glu Gln Leu Lys Ser Gly Thr Ala Ser Val Val Cys Leu Leu
130 135 140

Asn Asn Phe Tyr Pro Arg Glu Ala Lys Val Gln Trp Lys Val Asp Asn
145 150 155 160

Ala Leu Gln Ser Gly Asn Ser Gln Glu Ser Val Thr Glu Gln Asp Ser
165 170 175

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Gly Ala Pro Ile Glu Lys
325 330 335

Thr Ile Ser Lys Ala Lys Gly Gln Pro Arg Glu Pro Gln Val Tyr Thr
340 345 350

Leu Pro Pro Cys Arg Asp Glu Leu Thr Lys Asn Gln Val Ser Leu Trp
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Gly Gly Gly Gly Ser Gly Gly Gly Gly Ser Arg Glu Gly Pro Glu Leu
450 455 460

Ser Pro Asp Asp Pro Ala Gly Leu Leu Asp Leu Arg Gln Gly Met Phe
465 470 475 480

Ala Gln Leu Val Ala Gln Asn Val Leu Leu Ile Asp Gly Pro Leu Ser
485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu Pro Ser Pro Arg
625 630 635 640

Ser Glu Gly Gly Gly Gly Ser Gly Gly Gly Gly Ser Arg Glu Gly Pro
645 650 655

Glu Leu Ser Pro Asp Asp Pro Ala Gly Leu Leu Asp Leu Arg Gln Gly
660 665 670

Met Phe Ala Gln Leu Val Ala Gln Asn Val Leu Leu Ile Asp Gly Pro
675 680 685

Leu Ser Trp Tyr Ser Asp Pro Gly Leu Ala Gly Val Ser Leu Thr Gly
690 695 700

Gly Leu Ser Tyr Lys Glu Asp Thr Lys Glu Leu Val Val Ala Lys Ala
705 710 715 720

Gly Val Tyr Tyr Val Phe Phe Gln Leu Glu Leu Arg Arg Val Val Ala
725 730 735

Gly Glu Gly Ser Gly Ser Val Ser Leu Ala Leu His Leu Gln Pro Leu
740 745 750

Arg Ser Ala Ala Gly Ala Ala Ala Leu Ala Leu Thr Val Asp Leu Pro
755 760 765

Pro Ala Ser Ser Glu Ala Arg Asn Ser Ala Phe Gly Phe Gln Gly Arg
770 775 780

Leu Leu His Leu Ser Ala Gly Gln Arg Leu Gly Val His Leu His Thr
785 790 795 800

Glu Ala Arg Ala Arg His Ala Trp Gln Leu Thr Gln Gly Ala Thr Val
805 810 815

Leu Gly Leu Phe Arg Val Thr Pro Glu Ile Pro Ala Gly Leu Pro Ser
820 825 830

Pro Arg Ser Glu

835

<210> 219
<211> 2508
<212> DNA
<213> Artificial Sequence

<220>
<223> nucleotide sequence of DP47(VH-CL) Fc hole dimeric 4-1BB ligand (71-254) chain

<400> 219

gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagcgctag tgtggccgct 360
ccctccgtgt ttatctttcc cccatccgat gaacagctga aaagcggcac cgcctccgtc 420
gtgtgtctgc tgaacaattt ttaccctagg gaagctaaag tgcagtggaa agtggataac 480
gcactgcagt ccggcaactc ccaggaatct gtgacagaac aggactccaa ggacagcacc 540
tactccctgt cctccaccct gacactgtct aaggctgatt atgagaaaca caaagtctac 600
gcctgcgaag tcacccatca gggcctgagc tcgcccgtca caaagagctt caacagggga 660
gagtgtgaca aaactcacac atgcccaccg tgcccagcac ctgaagctgc aggggggaccg 720
tcagtcttcc tcttcccccc aaaacccaag gacaccctca tgatctcccg gacccctgag 780
gtcacatgcg tggtggtgga cgtgagccac gaagaccctg aggtcaagtt caactggtac 840
gtggacggcg tggaggtgca taatgccaag acaaagccgc gggaggagca gtacaacagc 900
acgtaccgtg tggtcagcgt cctcaccgtc ctgcaccagg actggctgaa tggcaaggag 960
tacaagtgca aggtctccaa caaagccctc ggcgccccca tcgagaaaac catctccaaa 1020
gccaaagggc agccccgaga accacaggtg tgcaccctgc ccccatcccg ggatgagctg 1080
accaagaacc aggtcagcct ctcgtgcgca gtcaaaggct tctatcccag cgacatcgcc 1140
gtggagtggg agagcaatgg gcagccggag aacaactaca agaccacgcc tcccgtgctg 1200
gactccgacg gctccttctt cctcgtgagc aagctcaccg tggacaagag caggtggcag 1260
caggggaacg tcttctcatg ctccgtgatg catgaggctc tgcacaacca ctacacgcag 1320
aagagcctct ccctgtctcc gggtggaggc ggcggaagcg gaggaggagg atccagagag 1380
ggccctgagc tgagccccga tgatcctgct ggactgctgg acctgcggca gggcatgttt 1440
gctcagctgg tggcccagaa cgtgctgctg atcgatggcc ccctgtcctg gtacagcgat 1500

cctggactgg ctggcgtgtc actgacaggc ggcctgagct acaaagagga caccaaagaa 1560
ctggtggtgg ccaaggccgg cgtgtactac gtgttctttc agctggaact gcggagagtg 1620
gtggccggcg aaggatctgg ctctgtgtct ctggccctgc atctgcagcc tctgagaagc 1680
gctgctggcg ctgcagctct ggcactgaca gtggatctgc ctcctgccag ctccgaggcc 1740
cggaatagcg catttgggtt tcaaggcagg ctgctgcacc tgtctgccgg ccagaggctg 1800
ggagtgcatc tgcacacaga ggccagggct agacacgcct ggcagctgac acagggcgct 1860
acagtgctgg gcctgttcag agtgaccccc gagattccag ccggcctgcc ttctccaaga 1920
agcgaaggcg gaggcggatc tggcggcgga ggatctagag agggacccga actgtcccct 1980
gacgatccag ccgggctgct ggatctgaga cagggaatgt tcgcccagct ggtggctcag 2040
aatgtgctgc tgattgacgg acctctgagc tggtactccg acccagggct ggcaggggtg 2100
tccctgactg gggactgtc ctacaaagaa gatacaaaag aactggtggt ggctaaagct 2160
ggggtgtact atgtgttttt tcagctggaa ctgaggcggg tggtggctgg ggagggctca 2220
ggatctgtgt ccctggctct gcatctgcag ccactgcgct ctgctgctgg cgcagctgca 2280
ctggctctga ctgtggacct gccaccagcc tctagcgagg ccagaaacag cgccttcggg 2340
ttccaaggac gcctgctgca tctgagcgcc ggacagcgcc tgggagtgca tctgcatact 2400
gaagccagag cccggcatgc ttggcagctg actcaggggg caactgtgct gggactgttt 2460
cgcgtgacac ctgagatccc tgccggactg ccaagcccta gatcagaa 2508

<210> 220
<211> 1920
<212> DNA
<213> Artificial Sequence

<220>
<223> nucleotide sequence of FAP (4B9) Fc knob monomeric 4-1BB (71-254) ligand

<400> 220

gaggtgcagc tgctcgaaag cggcggagga ctggtgcagc ctggcggcag cctgagactg 60

tcttgcgccg ccagcggctt caccttcagc agctacgcca tgagctgggt ccgccaggcc 120

cctggcaagg gactggaatg ggtgtccgcc atcatcggct ctggcgccag cacctactac 180

gccgacagcg tgaagggccg gttcaccatc agccgggaca acagcaagaa caccctgtac 240

ctgcagatga acagcctgcg ggccgaggac accgccgtgt actactgcgc caagggatgg 300

ttcggcggct tcaactactg gggacagggc accctggtca cagtgtccag cgctagcacc 360

aagggcccct ccgtgttccc cctggccccc agcagcaaga gcaccagcgg cggcacagcc 420

gctctgggct gcctggtcaa ggactacttc cccgagcccg tgaccgtgtc ctggaacagc 480

ggagccctga cctccggcgt gcacaccttc cccgccgtgc tgcagagttc tggcctgtat 540

agcctgagca gcgtggtcac cgtgccttct agcagcctgg gcacccagac ctacatctgc 600

aacgtgaacc acaagcccag caacaccaag gtggacaaga aggtggagcc caagagctgc 660

gacaagaccc acacctgtcc cccttgtcct gcccctgaag ctgctggcgg cccttctgtg 720

ttcctgttcc ccccaaagcc caaggacacc ctgatgatca gccggacccc cgaagtgacc 780

tgcgtggtgg tggatgtgtc ccacgaggac cctgaagtga agttcaattg gtacgtggac 840

ggcgtggaag tgcacaacgc caagacaaag ccgcgggagg agcagtacaa cagcacgtac 900

cgtgtggtca gcgtcctcac cgtcctgcac caggactggc tgaatggcaa ggagtacaag 960

tgcaaggtct ccaacaaagc cctcggcgcc cccatcgaga aaaccatctc caaagccaaa 1020

gggcagcccc gagaaccaca ggtgtacacc ctgcccccct gcagagatga gctgaccaag 1080

aaccaggtgt ccctgtggtg tctggtcaag ggcttctacc ccagcgatat cgccgtggag 1140

tgggagagca acggccagcc tgagaacaac tacaagacca ccccccctgt gctggacagc 1200

gacggcagct tcttcctgta ctccaaactg accgtggaca agagccggtg gcagcagggc 1260

aacgtgttca gctgcagcgt gatgcacgag gccctgcaca accactacac ccagaagtcc 1320

ctgagcctga gccccggcgg aggcggcgga agcggaggag gaggatccag agagggccct 1380

gagctgagcc ccgatgatcc tgctggactg ctggacctgc ggcagggcat gtttgctcag 1440

ctggtggccc agaacgtgct gctgatcgat ggccccctgt cctggtacag cgatcctgga 1500

ctggctggcg tgtcactgac aggcggcctg agctacaaag aggacaccaa agaactggtg 1560

gtggccaagg ccggcgtgta ctacgtgttc tttcagctgg aactgcggag agtggtggcc 1620

ggcgaaggat ctggctctgt gtctctggcc ctgcatctgc agcctctgag aagcgctgct 1680

ggcgctgcag ctctggcact gacagtggat ctgcctcctg ccagctccga ggcccggaat 1740

agcgcatttg ggtttcaagg caggctgctg cacctgtctg ccggccagag gctgggagtg 1800

catctgcaca cagaggccag ggctagacac gcctggcagc tgacacaggg cgctacagtg 1860

ctgggcctgt tcagagtgac ccccgagatt ccagccggcc tgccttctcc aagaagcgaa 1920

<210> 221
<211> 836
<212> PRT
<213> Artificial Sequence

<220>
<223> DP47(VH-CL) Fc hole dimeric 4-1BB ligand (71-254) chain

<400> 221

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30
```

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Val Ala Ala Pro Ser Val Phe Ile Phe Pro Pro
115 120 125

Ser Asp Glu Gln Leu Lys Ser Gly Thr Ala Ser Val Val Cys Leu Leu
130 135 140

Asn Asn Phe Tyr Pro Arg Glu Ala Lys Val Gln Trp Lys Val Asp Asn
145 150 155 160

Ala Leu Gln Ser Gly Asn Ser Gln Glu Ser Val Thr Glu Gln Asp Ser
165 170 175

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn

275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Gly Ala Pro Ile Glu Lys
325 330 335

Thr Ile Ser Lys Ala Lys Gly Gln Pro Arg Glu Pro Gln Val Cys Thr
340 345 350

Leu Pro Pro Ser Arg Asp Glu Leu Thr Lys Asn Gln Val Ser Leu Ser
355 360 365

Cys Ala Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Val Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Gly Gly Gly Gly Ser Gly Gly Gly Gly Ser Arg Glu Gly Pro Glu Leu
450 455 460

Ser Pro Asp Asp Pro Ala Gly Leu Leu Asp Leu Arg Gln Gly Met Phe
465 470 475 480

Ala Gln Leu Val Ala Gln Asn Val Leu Leu Ile Asp Gly Pro Leu Ser
485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu Pro Ser Pro Arg
625 630 635 640

Ser Glu Gly Gly Gly Gly Ser Gly Gly Gly Gly Ser Arg Glu Gly Pro
645 650 655

Glu Leu Ser Pro Asp Asp Pro Ala Gly Leu Leu Asp Leu Arg Gln Gly
660 665 670

Met Phe Ala Gln Leu Val Ala Gln Asn Val Leu Leu Ile Asp Gly Pro
675 680 685

Leu Ser Trp Tyr Ser Asp Pro Gly Leu Ala Gly Val Ser Leu Thr Gly
690 695 700

Gly Leu Ser Tyr Lys Glu Asp Thr Lys Glu Leu Val Val Ala Lys Ala
705 710 715 720

Gly Val Tyr Tyr Val Phe Phe Gln Leu Glu Leu Arg Arg Val Val Ala
725 730 735

Gly Glu Gly Ser Gly Ser Val Ser Leu Ala Leu His Leu Gln Pro Leu
740 745 750

Arg Ser Ala Ala Gly Ala Ala Ala Leu Ala Leu Thr Val Asp Leu Pro
755 760 765

Pro Ala Ser Ser Glu Ala Arg Asn Ser Ala Phe Gly Phe Gln Gly Arg
770 775 780

Leu Leu His Leu Ser Ala Gly Gln Arg Leu Gly Val His Leu His Thr
785 790 795 800

Glu Ala Arg Ala Arg His Ala Trp Gln Leu Thr Gln Gly Ala Thr Val
805 810 815

Leu Gly Leu Phe Arg Val Thr Pro Glu Ile Pro Ala Gly Leu Pro Ser
820 825 830

Pro Arg Ser Glu
835

<210> 222
<211> 640
<212> PRT
<213> Artificial Sequence

<220>
<223> FAP (4B9) Fc knob monomeric 4-1BB (71-254) ligand

<400> 222

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ile Gly Ser Gly Ala Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Trp Phe Gly Gly Phe Asn Tyr Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Tyr Thr Leu Pro
340 345 350

Pro Cys Arg Asp Glu Leu Thr Lys Asn Gln Val Ser Leu Trp Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

```
Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Gly Gly
        435                 440                 445

Gly Gly Ser Gly Gly Gly Gly Ser Arg Glu Gly Pro Glu Leu Ser Pro
    450                 455                 460

Asp Asp Pro Ala Gly Leu Leu Asp Leu Arg Gln Gly Met Phe Ala Gln
465                 470                 475                 480

Leu Val Ala Gln Asn Val Leu Leu Ile Asp Gly Pro Leu Ser Trp Tyr
                485                 490                 495

Ser Asp Pro Gly Leu Ala Gly Val Ser Leu Thr Gly Gly Leu Ser Tyr
            500                 505                 510

Lys Glu Asp Thr Lys Glu Leu Val Val Ala Lys Ala Gly Val Tyr Tyr
        515                 520                 525

Val Phe Phe Gln Leu Glu Leu Arg Arg Val Val Ala Gly Glu Gly Ser
    530                 535                 540

Gly Ser Val Ser Leu Ala Leu His Leu Gln Pro Leu Arg Ser Ala Ala
545                 550                 555                 560

Gly Ala Ala Ala Leu Ala Leu Thr Val Asp Leu Pro Pro Ala Ser Ser
                565                 570                 575

Glu Ala Arg Asn Ser Ala Phe Gly Phe Gln Gly Arg Leu Leu His Leu
            580                 585                 590

Ser Ala Gly Gln Arg Leu Gly Val His Leu His Thr Glu Ala Arg Ala
        595                 600                 605

Arg His Ala Trp Gln Leu Thr Gln Gly Ala Thr Val Leu Gly Leu Phe
    610                 615                 620

Arg Val Thr Pro Glu Ile Pro Ala Gly Leu Pro Ser Pro Arg Ser Glu
625                 630                 635                 640
```

<210> 223
<211> 1926

<212> DNA
<213> Artificial Sequence

<220>
<223> nucleotide sequence of DP47 (VH-CL) Fc hole monomeric 4-1BBL (71-254) chain

<400> 223

gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagcgctag tgtggccgct 360
ccctccgtgt ttatctttcc cccatccgat gaacagctga aaagcggcac cgcctccgtc 420
gtgtgtctgc tgaacaattt ttaccctagg gaagctaaag tgcagtggaa agtggataac 480
gcactgcagt ccggcaactc ccaggaatct gtgacagaac aggactccaa ggacagcacc 540
tactccctgt cctccaccct gacactgtct aaggctgatt atgagaaaca caaagtctac 600
gcctgcgaag tcacccatca gggcctgagc tcgcccgtca caaagagctt caacagggga 660
gagtgtgaca aaactcacac atgcccaccg tgcccagcac ctgaagctgc aggggggaccg 720
tcagtcttcc tcttcccccc aaaacccaag gacaccctca tgatctcccg gacccctgag 780
gtcacatgcg tggtggtgga cgtgagccac gaagaccctg aggtcaagtt caactggtac 840
gtggacggcg tggaggtgca taatgccaag acaaagccgc gggaggagca gtacaacagc 900
acgtaccgtg tggtcagcgt cctcaccgtc ctgcaccagg actggctgaa tggcaaggag 960
tacaagtgca aggtctccaa caaagccctc ggcgccccca tcgagaaaac catctccaaa 1020
gccaaagggc agccccgaga accacaggtg tgcaccctgc ccccatcccg ggatgagctg 1080
accaagaacc aggtcagcct ctcgtgcgca gtcaaaggct tctatcccag cgacatcgcc 1140
gtggagtggg agagcaatgg gcagccggag aacaactaca agaccacgcc tcccgtgctg 1200
gactccgacg gctccttctt cctcgtgagc aagctcaccg tggacaagag caggtggcag 1260
caggggaacg tcttctcatg ctccgtgatg catgaggctc tgcacaacca ctacacgcag 1320
aagagcctct ccctgtctcc gggtggaggc ggcggaagcg gaggaggagg atccagagag 1380
ggccctgagc tgagccccga tgatcctgct ggactgctgg acctgcggca gggcatgttt 1440
gctcagctgg tggcccagaa cgtgctgctg atcgatggcc ccctgtcctg gtacagcgat 1500
cctggactgg ctggcgtgtc actgacaggc ggcctgagct acaaagagga caccaaagaa 1560
ctggtggtgg ccaaggccgg cgtgtactac gtgttctttc agctggaact gcggagagtg 1620
gtggccggcg aaggatctgg ctctgtgtct ctggccctgc atctgcagcc tctgagaagc 1680

gctgctggcg ctgcagctct ggcactgaca gtggatctgc ctcctgccag ctccgaggcc 1740

cggaatagcg catttgggtt tcaaggcagg ctgctgcacc tgtctgccgg ccagaggctg 1800

ggagtgcatc tgcacacaga ggccagggct agacacgcct ggcagctgac acagggcgct 1860

acagtgctgg gcctgttcag agtgaccccc gagattccag ccggcctgcc ttctccaaga 1920

agcgaa 1926

<210> 224
<211> 2502
<212> DNA
<213> Artificial Sequence

<220>
<223> nucleotide sequence of FAP (4B9) Fc knob dimeric 4-1BB ligand (71-254) chain

<400> 224

gaggtgcagc tgctcgaaag cggcggagga ctggtgcagc ctggcggcag cctgagactg 60
tcttgcgccg ccagcggctt caccttcagc agctacgcca tgagctgggt ccgccaggcc 120
cctggcaagg gactggaatg ggtgtccgcc atcatcggct ctggcgccag cacctactac 180
gccgacagcg tgaagggccg gttcaccatc agccgggaca acagcaagaa caccctgtac 240
ctgcagatga acagcctgcg ggccgaggac accgccgtgt actactgcgc caagggatgg 300
ttcggcggct tcaactactg gggacagggc accctggtca cagtgtccag cgctagcacc 360
aagggcccct ccgtgttccc cctggccccc agcagcaaga gcaccagcgg cggcacagcc 420
gctctgggct gcctggtcaa ggactacttc cccgagcccg tgaccgtgtc ctggaacagc 480
ggagccctga cctccggcgt gcacaccttc cccgccgtgc tgcagagttc tggcctgtat 540
agcctgagca gcgtggtcac cgtgccttct agcagcctgg gcacccagac ctacatctgc 600
aacgtgaacc acaagcccag caacaccaag gtggacaaga aggtggagcc caagagctgc 660
gacaagaccc acacctgtcc cccttgtcct gcccctgaag ctgctggcgg cccttctgtg 720
ttcctgttcc ccccaaagcc caaggacacc ctgatgatca gccggacccc cgaagtgacc 780
tgcgtggtgg tggatgtgtc ccacgaggac cctgaagtga agttcaattg gtacgtggac 840
ggcgtggaag tgcacaacgc caagacaaag ccgcgggagg agcagtacaa cagcacgtac 900
cgtgtggtca gcgtcctcac cgtcctgcac caggactggc tgaatggcaa ggagtacaag 960
tgcaaggtct ccaacaaagc cctcggcgcc cccatcgaga aaaccatctc caaagccaaa 1020
gggcagcccc gagaaccaca ggtgtacacc ctgccccct gcagagatga gctgaccaag 1080
aaccaggtgt ccctgtggtg tctggtcaag ggcttctacc ccagcgatat cgccgtggag 1140
tgggagagca acggccagcc tgagaacaac tacaagacca cccccctgt gctggacagc 1200
gacggcagct tcttcctgta ctccaaactg accgtggaca agagccggtg gcagcagggc 1260

aacgtgttca gctgcagcgt gatgcacgag gccctgcaca accactacac ccagaagtcc 1320
ctgagcctga gccccggcgg aggcggcgga agcggaggag gaggatccag agagggccct 1380
gagctgagcc ccgatgatcc tgctggactg ctggacctgc ggcagggcat gtttgctcag 1440
ctggtggccc agaacgtgct gctgatcgat ggccccctgt cctggtacag cgatcctgga 1500
ctggctggcg tgtcactgac aggcggcctg agctacaaag aggacaccaa agaactggtg 1560
gtggccaagg ccggcgtgta ctacgtgttc tttcagctgg aactgcggag agtggtggcc 1620
ggcgaaggat ctggctctgt gtctctggcc ctgcatctgc agcctctgag aagcgctgct 1680
ggcgctgcag ctctggcact gacagtggat ctgcctcctg ccagctccga ggcccggaat 1740
agcgcatttg ggtttcaagg caggctgctg cacctgtctg ccggccagag gctgggagtg 1800
catctgcaca cagaggccag ggctagacac gcctggcagc tgacacaggg cgctacagtg 1860
ctgggcctgt tcagagtgac ccccgagatt ccagccggcc tgccttctcc aagaagcgaa 1920
ggcggaggcg gatctggcgg cggaggatct agagagggac ccgaactgtc ccctgacgat 1980
ccagccgggc tgctggatct gagacaggga atgttcgccc agctggtggc tcagaatgtg 2040
ctgctgattg acggacctct gagctggtac tccgacccag ggctggcagg ggtgtccctg 2100
actgggggac tgtcctacaa agaagataca aaagaactgg tggtggctaa agctggggtg 2160
tactatgtgt tttttcagct ggaactgagg cgggtggtgg ctggggaggg ctcaggatct 2220
gtgtccctgg ctctgcatct gcagccactg cgctctgctg ctggcgcagc tgcactggct 2280
ctgactgtgg acctgccacc agcctctagc gaggccagaa acagcgcctt cgggttccaa 2340
ggacgcctgc tgcatctgag cgccggacag cgcctgggag tgcatctgca tactgaagcc 2400
agagcccggc atgcttggca gctgactcag gggcaactg tgctgggact gtttcgcgtg 2460
acacctgaga tccctgccgg actgccaagc cctagatcag aa 2502

<210> 225
<211> 642
<212> PRT
<213> Artificial Sequence

<220>
<223> DP47 (VH-CL) Fc hole monomeric 4-1BBL (71-254) chain

<400> 225

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val

35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Val Ala Ala Pro Ser Val Phe Ile Phe Pro Pro
115 120 125

Ser Asp Glu Gln Leu Lys Ser Gly Thr Ala Ser Val Val Cys Leu Leu
130 135 140

Asn Asn Phe Tyr Pro Arg Glu Ala Lys Val Gln Trp Lys Val Asp Asn
145 150 155 160

Ala Leu Gln Ser Gly Asn Ser Gln Glu Ser Val Thr Glu Gln Asp Ser
165 170 175

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

```
Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Gly Gly Gly Ser Gly Gly Gly Gly Ser Arg Glu Gly Pro Glu Leu
    450                 455                 460

Ser Pro Asp Asp Pro Ala Gly Leu Leu Asp Leu Arg Gln Gly Met Phe
465                 470                 475                 480

Ala Gln Leu Val Ala Gln Asn Val Leu Leu Ile Asp Gly Pro Leu Ser
                485                 490                 495

Trp Tyr Ser Asp Pro Gly Leu Ala Gly Val Ser Leu Thr Gly Gly Leu
            500                 505                 510

Ser Tyr Lys Glu Asp Thr Lys Glu Leu Val Val Ala Lys Ala Gly Val
        515                 520                 525

Tyr Tyr Val Phe Phe Gln Leu Glu Leu Arg Arg Val Val Ala Gly Glu
    530                 535                 540
```

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu Pro Ser Pro Arg
625 630 635 640

Ser Glu

<210> 226
<211> 834
<212> PRT
<213> Artificial Sequence

<220>
<223> anti-FAP(4B9) Fc knob dimeric 4-1BB ligand (71-254) chain

<400> 226

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ile Gly Ser Gly Ala Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Trp Phe Gly Gly Phe Asn Tyr Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Tyr Thr Leu Pro
340 345 350

```
Pro Cys Arg Asp Glu Leu Thr Lys Asn Gln Val Ser Leu Trp Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Gly Gly
        435                 440                 445

Gly Gly Ser Gly Gly Gly Gly Ser Arg Glu Gly Pro Glu Leu Ser Pro
    450                 455                 460

Asp Asp Pro Ala Gly Leu Leu Asp Leu Arg Gln Gly Met Phe Ala Gln
465                 470                 475                 480

Leu Val Ala Gln Asn Val Leu Leu Ile Asp Gly Pro Leu Ser Trp Tyr
                485                 490                 495

Ser Asp Pro Gly Leu Ala Gly Val Ser Leu Thr Gly Gly Leu Ser Tyr
            500                 505                 510

Lys Glu Asp Thr Lys Glu Leu Val Val Ala Lys Ala Gly Val Tyr Tyr
        515                 520                 525

Val Phe Phe Gln Leu Glu Leu Arg Arg Val Val Ala Gly Glu Gly Ser
    530                 535                 540

Gly Ser Val Ser Leu Ala Leu His Leu Gln Pro Leu Arg Ser Ala Ala
545                 550                 555                 560

Gly Ala Ala Ala Leu Ala Leu Thr Val Asp Leu Pro Pro Ala Ser Ser
                565                 570                 575

Glu Ala Arg Asn Ser Ala Phe Gly Phe Gln Gly Arg Leu Leu His Leu
            580                 585                 590

Ser Ala Gly Gln Arg Leu Gly Val His Leu His Thr Glu Ala Arg Ala
        595                 600                 605
```

```
Arg His Ala Trp Gln Leu Thr Gln Gly Ala Thr Val Leu Gly Leu Phe
    610                 615                 620

Arg Val Thr Pro Glu Ile Pro Ala Gly Leu Pro Ser Pro Arg Ser Glu
625                 630                 635                 640

Gly Gly Gly Gly Ser Gly Gly Gly Gly Ser Arg Glu Gly Pro Glu Leu
                645                 650                 655

Ser Pro Asp Asp Pro Ala Gly Leu Leu Asp Leu Arg Gln Gly Met Phe
            660                 665                 670

Ala Gln Leu Val Ala Gln Asn Val Leu Leu Ile Asp Gly Pro Leu Ser
        675                 680                 685

Trp Tyr Ser Asp Pro Gly Leu Ala Gly Val Ser Leu Thr Gly Gly Leu
    690                 695                 700

Ser Tyr Lys Glu Asp Thr Lys Glu Leu Val Val Ala Lys Ala Gly Val
705                 710                 715                 720

Tyr Tyr Val Phe Phe Gln Leu Glu Leu Arg Arg Val Val Ala Gly Glu
                725                 730                 735

Gly Ser Gly Ser Val Ser Leu Ala Leu His Leu Gln Pro Leu Arg Ser
            740                 745                 750

Ala Ala Gly Ala Ala Ala Leu Ala Leu Thr Val Asp Leu Pro Pro Ala
        755                 760                 765

Ser Ser Glu Ala Arg Asn Ser Ala Phe Gly Phe Gln Gly Arg Leu Leu
    770                 775                 780

His Leu Ser Ala Gly Gln Arg Leu Gly Val His Leu His Thr Glu Ala
785                 790                 795                 800

Arg Ala Arg His Ala Trp Gln Leu Thr Gln Gly Ala Thr Val Leu Gly
                805                 810                 815

Leu Phe Arg Val Thr Pro Glu Ile Pro Ala Gly Leu Pro Ser Pro Arg
            820                 825                 830

Ser Glu
```

<210> 227
<211> 1908
<212> DNA

<213> Artificial Sequence

<220>
<223> nucleotide sequence of DP47 (VHCL) Fc knob monomeric 4-1BB (71-248) ligand

<400> 227

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagcgctag tgtggccgct     360
ccctccgtgt ttatctttcc cccatccgat gaacagctga aaagcggcac cgcctccgtc     420
gtgtgtctgc tgaacaattt ttaccctagg gaagctaaag tgcagtggaa agtggataac     480
gcactgcagt ccggcaactc ccaggaatct gtgacagaac aggactccaa ggacagcacc     540
tactccctgt cctccaccct gacactgtct aaggctgatt atgagaaaca caaagtctac     600
gcctgcgaag tcacccatca gggcctgagc tcgcccgtca caaagagctt caacagggga     660
gagtgtgaca agacccacac ctgtcccccct tgtcctgccc ctgaagctgc tggcggccct     720
tctgtgttcc tgttcccccc aaagcccaag gacaccctga tgatcagccg gacccccgaa     780
gtgacctgcg tggtggtgga tgtgtcccac gaggaccctg aagtgaagtt caattggtac     840
gtggacggcg tggaagtgca caacgccaag acaaagccgc gggaggagca gtacaacagc     900
acgtaccgtg tggtcagcgt cctcaccgtc ctgcaccagg actggctgaa tggcaaggag     960
tacaagtgca aggtctccaa caaagccctc ggcgccccca tcgagaaaac catctccaaa    1020
gccaaagggc agccccgaga accacaggtg tacaccctgc cccctgcag agatgagctg    1080
accaagaacc aggtgtccct gtggtgtctg gtcaagggct tctaccccag cgatatcgcc    1140
gtggagtggg agagcaacgg ccagcctgag aacaactaca agaccacccc ccctgtgctg    1200
gacagcgacg gcagcttctt cctgtactcc aaactgaccg tggacaagag ccggtggcag    1260
cagggcaacg tgttcagctg cagcgtgatg cacgaggccc tgcacaacca ctacacccag    1320
aagtccctga gcctgagccc cggcggaggc ggcggaagcg gaggaggagg atccagagag    1380
ggccctgagc tgagccctga tgatcctgcc ggactgctgg acctgcggca gggaatgttt    1440
gcccagctgg tggcccagaa cgtgctgctg atcgatggcc ccctgtcctg gtacagcgat    1500
cctggactgg ctggcgtgtc actgacaggc ggcctgagct acaaagagga caccaaagaa    1560
ctggtggtgg ccaaggccgg cgtgtactac gtgttctttc agctggaact gcggagagtg    1620
```

```
gtggccggcg aaggatctgg ctctgtgtct ctggccctgc atctgcagcc tctgagatct      1680
gctgctggcg ccgctgctct ggcactgaca gtggatctgc ctcctgccag cagcgaggcc      1740
cggaatagcg catttgggtt tcaaggcagg ctgctgcacc tgtctgccgg ccagaggctg      1800
ggagtgcatc tgcacacaga ggccagggct agacacgcct ggcagctgac acagggcgct      1860
acagtgctgg gcctgttcag agtgaccccc gagattcctg ccgggctc                   1908
```

<210> 228
<211> 636
<212> PRT
<213> Artificial Sequence

<220>
<223> DP47 (VH-CL) Fc knob monomeric 4-1BB (71-248) ligand

<400> 228

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Val Ala Ala Pro Ser Val Phe Ile Phe Pro Pro
        115                 120                 125

Ser Asp Glu Gln Leu Lys Ser Gly Thr Ala Ser Val Val Cys Leu Leu
    130                 135                 140

Asn Asn Phe Tyr Pro Arg Glu Ala Lys Val Gln Trp Lys Val Asp Asn
145                 150                 155                 160

Ala Leu Gln Ser Gly Asn Ser Gln Glu Ser Val Thr Glu Gln Asp Ser
                165                 170                 175
```

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Gly Ala Pro Ile Glu Lys
325 330 335

Thr Ile Ser Lys Ala Lys Gly Gln Pro Arg Glu Pro Gln Val Tyr Thr
340 345 350

Leu Pro Pro Cys Arg Asp Glu Leu Thr Lys Asn Gln Val Ser Leu Trp
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu

```
                420                 425                 430

Ala Leu His Asn His Tyr Thr Gln Lys Ser Leu Ser Leu Ser Pro Gly
            435                 440                 445

Gly Gly Gly Gly Ser Gly Gly Gly Gly Ser Arg Glu Gly Pro Glu Leu
        450                 455                 460

Ser Pro Asp Asp Pro Ala Gly Leu Leu Asp Leu Arg Gln Gly Met Phe
465                 470                 475                 480

Ala Gln Leu Val Ala Gln Asn Val Leu Leu Ile Asp Gly Pro Leu Ser
                485                 490                 495

Trp Tyr Ser Asp Pro Gly Leu Ala Gly Val Ser Leu Thr Gly Gly Leu
            500                 505                 510

Ser Tyr Lys Glu Asp Thr Lys Glu Leu Val Val Ala Lys Ala Gly Val
        515                 520                 525

Tyr Tyr Val Phe Phe Gln Leu Glu Leu Arg Arg Val Val Ala Gly Glu
    530                 535                 540

Gly Ser Gly Ser Val Ser Leu Ala Leu His Leu Gln Pro Leu Arg Ser
545                 550                 555                 560

Ala Ala Gly Ala Ala Ala Leu Ala Leu Thr Val Asp Leu Pro Pro Ala
                565                 570                 575

Ser Ser Glu Ala Arg Asn Ser Ala Phe Gly Phe Gln Gly Arg Leu Leu
            580                 585                 590

His Leu Ser Ala Gly Gln Arg Leu Gly Val His Leu His Thr Glu Ala
        595                 600                 605

Arg Ala Arg His Ala Trp Gln Leu Thr Gln Gly Ala Thr Val Leu Gly
    610                 615                 620

Leu Phe Arg Val Thr Pro Glu Ile Pro Ala Gly Leu
625                 630                 635
```

<210> 229
<211> 2472
<212> DNA
<213> Artificial Sequence

<220>
<223> nucleotide sequence of DP47 (VH-CL) Fc knob dimeric 4-1BB ligand (71-248) chain

<400> 229

gaggtgcaat tgttggagtc tgggggaggc ttggtacagc ctggggggtc cctgagactc 60
tcctgtgcag cctccggatt cacctttagc agttatgcca tgagctgggt ccgccaggct 120
ccagggaagg ggctggagtg ggtctcagct attagtggta gtggtggtag cacatactac 180
gcagactccg tgaagggccg gttcaccatc tccagagaca attccaagaa cacgctgtat 240
ctgcagatga acagcctgag agccgaggac acggccgtat attactgtgc gaaaggcagc 300
ggatttgact actggggcca aggaaccctg gtcaccgtct cgagcgctag tgtggccgct 360
ccctccgtgt ttatctttcc cccatccgat gaacagctga aaagcggcac cgcctccgtc 420
gtgtgtctgc tgaacaattt ttaccctagg gaagctaaag tgcagtggaa agtggataac 480
gcactgcagt ccggcaactc ccaggaatct gtgacagaac aggactccaa ggacagcacc 540
tactccctgt cctccaccct gacactgtct aaggctgatt atgagaaaca caaagtctac 600
gcctgcgaag tcacccatca gggcctgagc tcgcccgtca caaagagctt caacagggga 660
gagtgtgaca agacccacac ctgtccccct tgtcctgccc ctgaagctgc tggcggccct 720
tctgtgttcc tgttcccccc aaagcccaag gacaccctga tgatcagccg gacccccgaa 780
gtgacctgcg tggtggtgga tgtgtcccac gaggaccctg aagtgaagtt caattggtac 840
gtggacggcg tggaagtgca caacgccaag acaaagccgc gggaggagca gtacaacagc 900
acgtaccgtg tggtcagcgt cctcaccgtc ctgcaccagg actggctgaa tggcaaggag 960
tacaagtgca aggtctccaa caaagccctc ggcgccccca tcgagaaaac catctccaaa 1020
gccaaagggc agccccgaga accacaggtg tacaccctgc ccccctgcag agatgagctg 1080
accaagaacc aggtgtccct gtggtgtctg gtcaagggct tctaccccag cgatatcgcc 1140
gtggagtggg agagcaacgg ccagcctgag aacaactaca agaccacccc ccctgtgctg 1200
gacagcgacg gcagcttctt cctgtactcc aaactgaccg tggacaagag ccggtggcag 1260
cagggcaacg tgttcagctg cagcgtgatg cacgaggccc tgcacaacca ctacacccag 1320
aagtccctga gcctgagccc cggcggaggc ggcggaagcg gaggaggagg atccagagag 1380
ggccctgagc tgagccctga tgatcctgcc ggactgctgg acctgcggca gggaatgttt 1440
gcccagctgg tggcccagaa cgtgctgctg atcgatggcc ccctgtcctg gtacagcgat 1500
cctggactgg ctggcgtgtc actgacaggc ggcctgagct acaaagagga caccaaagaa 1560
ctggtggtgg ccaaggccgg cgtgtactac gtgttctttc agctggaact gcggagagtg 1620
gtggccggcg aaggatctgg ctctgtgtct ctggccctgc atctgcagcc tctgagatct 1680
gctgctggcg ccgctgctct ggcactgaca gtggatctgc ctcctgccag cagcgaggcc 1740
cggaatagcg catttgggtt tcaaggcagg ctgctgcacc tgtctgccgg ccagaggctg 1800
ggagtgcatc tgcacacaga ggccagggct agacacgcct ggcagctgac acagggcgct 1860

acagtgctgg gcctgttcag agtgaccccc gagattccag caggcctggg aggcggcgga 1920
tctggcggcg gaggatctag agaaggaccc gagctgtccc ccgacgatcc cgctgggctg 1980
ctggatctga gacagggcat gttcgctcag ctggtggctc agaatgtgct gctgattgac 2040
ggacctctga gctggtactc cgacccaggg ctggcagggg tgtccctgac tgggggactg 2100
tcctacaaag aagatacaaa agaactggtg gtggctaaag ctggggtgta ctatgtgttt 2160
tttcagctgg aactgaggcg ggtggtggct ggggagggct caggatctgt gtccctggct 2220
ctgcatctgc agccactgcg ctctgcagca ggggctgcag cactggccct gactgtggac 2280
ctgcccccag cttcttccga ggccagaaac agcgccttcg ggttccaagg acgcctgctg 2340
catctgagcg ccggacagcg cctgggagtg catctgcata ctgaagccag agcccggcat 2400
gcttggcagc tgactcaggg ggcaactgtg ctgggactgt ttcgcgtgac acctgagatc 2460
ccagccgggc tc 2472

<210> 230
<211> 824
<212> PRT
<213> Artificial Sequence

<220>
<223> DP47 (VH-CL) Fc knob dimeric 4-1BB ligand (71-248) chain

<400> 230

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Val Ala Ala Pro Ser Val Phe Ile Phe Pro Pro
```

115 120 125

Ser Asp Glu Gln Leu Lys Ser Gly Thr Ala Ser Val Val Cys Leu Leu
130 135 140

Asn Asn Phe Tyr Pro Arg Glu Ala Lys Val Gln Trp Lys Val Asp Asn
145 150 155 160

Ala Leu Gln Ser Gly Asn Ser Gln Glu Ser Val Thr Glu Gln Asp Ser
165 170 175

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Gly Ala Pro Ile Glu Lys
325 330 335

Thr Ile Ser Lys Ala Lys Gly Gln Pro Arg Glu Pro Gln Val Tyr Thr
340 345 350

Leu Pro Pro Cys Arg Asp Glu Leu Thr Lys Asn Gln Val Ser Leu Trp
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Gly Gly Gly Gly Ser Gly Gly Gly Gly Ser Arg Glu Gly Pro Glu Leu
450 455 460

Ser Pro Asp Asp Pro Ala Gly Leu Leu Asp Leu Arg Gln Gly Met Phe
465 470 475 480

Ala Gln Leu Val Ala Gln Asn Val Leu Leu Ile Asp Gly Pro Leu Ser
485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

```
Leu Phe Arg Val Thr Pro Glu Ile Pro Ala Gly Leu Gly Gly Gly Gly
625                 630                 635                 640

Ser Gly Gly Gly Gly Ser Arg Glu Gly Pro Glu Leu Ser Pro Asp Asp
                645                 650                 655

Pro Ala Gly Leu Leu Asp Leu Arg Gln Gly Met Phe Ala Gln Leu Val
            660                 665                 670

Ala Gln Asn Val Leu Leu Ile Asp Gly Pro Leu Ser Trp Tyr Ser Asp
        675                 680                 685

Pro Gly Leu Ala Gly Val Ser Leu Thr Gly Gly Leu Ser Tyr Lys Glu
    690                 695                 700

Asp Thr Lys Glu Leu Val Val Ala Lys Ala Gly Val Tyr Tyr Val Phe
705                 710                 715                 720

Phe Gln Leu Glu Leu Arg Arg Val Val Ala Gly Glu Gly Ser Gly Ser
                725                 730                 735

Val Ser Leu Ala Leu His Leu Gln Pro Leu Arg Ser Ala Ala Gly Ala
            740                 745                 750

Ala Ala Leu Ala Leu Thr Val Asp Leu Pro Pro Ala Ser Ser Glu Ala
        755                 760                 765

Arg Asn Ser Ala Phe Gly Phe Gln Gly Arg Leu Leu His Leu Ser Ala
    770                 775                 780

Gly Gln Arg Leu Gly Val His Leu His Thr Glu Ala Arg Ala Arg His
785                 790                 795                 800

Ala Trp Gln Leu Thr Gln Gly Ala Thr Val Leu Gly Leu Phe Arg Val
                805                 810                 815

Thr Pro Glu Ile Pro Ala Gly Leu
            820
```

<210> 231
<211> 1908
<212> DNA
<213> Artificial Sequence

<220>
<223> nucleotide sequence of DP47(VH-CL) Fc hole dimeric 4-1BB ligand (71-248) chain

<400> 231

```
gaggtgcaat tgttggagtc tgggggaggc ttggtacagc ctggggggtc cctgagactc      60
tcctgtgcag cctccggatt cacctttagc agttatgcca tgagctgggt ccgccaggct     120
ccagggaagg ggctggagtg ggtctcagct attagtggta gtggtggtag cacatactac     180
gcagactccg tgaagggccg gttcaccatc tccagagaca attccaagaa cacgctgtat     240
ctgcagatga acagcctgag agccgaggac acggccgtat attactgtgc gaaaggcagc     300
ggatttgact actggggcca aggaaccctg gtcaccgtct cgagcgctag tgtggccgct     360
ccctccgtgt ttatctttcc cccatccgat gaacagctga aaagcggcac cgcctccgtc     420
gtgtgtctgc tgaacaattt ttaccctagg gaagctaaag tgcagtggaa agtggataac     480
gcactgcagt ccggcaactc ccaggaatct gtgacagaac aggactccaa ggacagcacc     540
tactccctgt cctccaccct gacactgtct aaggctgatt atgagaaaca caaagtctac     600
gcctgcgaag tcacccatca gggcctgagc tcgcccgtca caaagagctt caacagggga     660
gagtgtgaca aaactcacac atgcccaccg tgcccagcac ctgaagctgc aggggggaccg     720
tcagtcttcc tcttcccccc aaaacccaag gacaccctca tgatctcccg gacccctgag     780
gtcacatgcg tggtggtgga cgtgagccac gaagaccctg aggtcaagtt caactggtac     840
gtggacggcg tggaggtgca taatgccaag acaaagccgc gggaggagca gtacaacagc     900
acgtaccgtg tggtcagcgt cctcaccgtc ctgcaccagg actggctgaa tggcaaggag     960
tacaagtgca aggtctccaa caaagccctc ggcgccccca tcgagaaaac catctccaaa    1020
gccaaagggc agccccgaga accacaggtg tgcaccctgc ccccatcccg ggatgagctg    1080
accaagaacc aggtcagcct ctcgtgcgca gtcaaaggct tctatcccag cgacatcgcc    1140
gtggagtggg agagcaatgg gcagccggag aacaactaca agaccacgcc tcccgtgctg    1200
gactccgacg gctccttctt cctcgtgagc aagctcaccg tggacaagag caggtggcag    1260
caggggaacg tcttctcatg ctccgtgatg catgaggctc tgcacaacca ctacacgcag    1320
aagagcctct ccctgtctcc gggtggaggc ggcggaagcg gaggaggagg atccagagag    1380
ggccctgagc tgagccctga tgatcctgcc ggactgctgg acctgcggca gggaatgttt    1440
gcccagctgg tggcccagaa cgtgctgctg atcgatggcc ccctgtcctg gtacagcgat    1500
cctggactgg ctggcgtgtc actgacaggc ggcctgagct acaaagagga caccaaagaa    1560
ctggtggtgg ccaaggccgg cgtgtactac gtgttctttc agctggaact gcggagagtg    1620
gtggccggcg aaggatctgg ctctgtgtct ctggccctgc atctgcagcc tctgagatct    1680
gctgctggcg ccgctgctct ggcactgaca gtggatctgc ctcctgccag cagcgaggcc    1740
cggaatagcg catttgggtt tcaaggcagg ctgctgcacc tgtctgccgg ccagaggctg    1800
ggagtgcatc tgcacacaga ggccagggct agacacgcct ggcagctgac acagggcgct    1860
acagtgctgg gcctgttcag agtgaccccc gagattcctg ccgggctc                 1908
```

<210> 232
<211> 636
<212> PRT
<213> Artificial Sequence

<220>
<223> DP47(VH-CL) Fc hole dimeric 4-1BB ligand (71-248) chain

<400> 232

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Gly Phe Asp Tyr Trp Gly Gln Gly Thr Leu Val Thr
            100                 105                 110

Val Ser Ser Ala Ser Val Ala Ala Pro Ser Val Phe Ile Phe Pro Pro
        115                 120                 125

Ser Asp Glu Gln Leu Lys Ser Gly Thr Ala Ser Val Val Cys Leu Leu
    130                 135                 140

Asn Asn Phe Tyr Pro Arg Glu Ala Lys Val Gln Trp Lys Val Asp Asn
145                 150                 155                 160

Ala Leu Gln Ser Gly Asn Ser Gln Glu Ser Val Thr Glu Gln Asp Ser
                165                 170                 175

Lys Asp Ser Thr Tyr Ser Leu Ser Ser Thr Leu Thr Leu Ser Lys Ala
            180                 185                 190

Asp Tyr Glu Lys His Lys Val Tyr Ala Cys Glu Val Thr His Gln Gly
        195                 200                 205
```

Leu Ser Ser Pro Val Thr Lys Ser Phe Asn Arg Gly Glu Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Gly Gly Gly Ser Gly Gly Gly Gly Ser Arg Glu Gly Pro Glu Leu
    450                 455                 460

Ser Pro Asp Asp Pro Ala Gly Leu Leu Asp Leu Arg Gln Gly Met Phe
465 470 475 480

Ala Gln Leu Val Ala Gln Asn Val Leu Leu Ile Asp Gly Pro Leu Ser
485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu
625 630 635

<210> 233
<211> 1908
<212> DNA
<213> Artificial Sequence

<220>
<223> nucleotide sequence of DP47 (VH-CL) Fc hole monomeric 4-1BBL (71-248) chain

<400> 233

gaggtgcaat tgttggagtc tgggggaggc ttggtacagc ctggggggtc cctgagactc 60

tcctgtgcag cctccggatt cacctttagc agttatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcagatga acagcctgag agccgaggac acggccgtat attactgtgc gaaaggcagc 300

ggatttgact actggggcca aggaaccctg gtcaccgtct cgagcgctag tgtggccgct 360

ccctccgtgt ttatctttcc cccatccgat gaacagctga aaagcggcac cgcctccgtc 420

gtgtgtctgc tgaacaattt ttaccctagg gaagctaaag tgcagtggaa agtggataac 480

gcactgcagt ccggcaactc ccaggaatct gtgacagaac aggactccaa ggacagcacc 540

tactccctgt cctccaccct gacactgtct aaggctgatt atgagaaaca caaagtctac 600

gcctgcgaag tcacccatca gggcctgagc tcgcccgtca caaagagctt caacagggga 660

gagtgtgaca aaactcacac atgcccaccg tgcccagcac ctgaagctgc aggggggaccg 720

tcagtcttcc tcttcccccc aaaacccaag gacaccctca tgatctcccg gacccctgag 780

gtcacatgcg tggtggtgga cgtgagccac gaagaccctg aggtcaagtt caactggtac 840

gtggacggcg tggaggtgca taatgccaag acaaagccgc gggaggagca gtacaacagc 900

acgtaccgtg tggtcagcgt cctcaccgtc ctgcaccagg actggctgaa tggcaaggag 960

tacaagtgca aggtctccaa caaagccctc ggcgccccca tcgagaaaac catctccaaa 1020

gccaaagggc agccccgaga accacaggtg tgcaccctgc ccccatcccg ggatgagctg 1080

accaagaacc aggtcagcct ctcgtgcgca gtcaaaggct tctatcccag cgacatcgcc 1140

gtggagtggg agagcaatgg gcagccggag aacaactaca agaccacgcc tcccgtgctg 1200

gactccgacg gctccttctt cctcgtgagc aagctcaccg tggacaagag caggtggcag 1260

caggggaacg tcttctcatg ctccgtgatg catgaggctc tgcacaacca ctacacgcag 1320

aagagcctct ccctgtctcc gggtggaggc ggcggaagcg gaggaggagg atccagagag 1380

ggccctgagc tgagccctga tgatcctgcc ggactgctgg acctgcggca gggaatgttt 1440

gcccagctgg tggcccagaa cgtgctgctg atcgatggcc ccctgtcctg gtacagcgat 1500

cctggactgg ctggcgtgtc actgacaggc ggcctgagct acaaagagga caccaaagaa 1560

ctggtggtgg ccaaggccgg cgtgtactac gtgttctttc agctggaact gcggagagtg 1620

gtggccggcg aaggatctgg ctctgtgtct ctggccctgc atctgcagcc tctgagatct 1680

gctgctggcg ccgctgctct ggcactgaca gtggatctgc ctcctgccag cagcgaggcc 1740

cggaatagcg catttgggtt tcaaggcagg ctgctgcacc tgtctgccgg ccagaggctg 1800

ggagtgcatc tgcacacaga ggccagggct agacacgcct ggcagctgac acagggcgct 1860

acagtgctgg gcctgttcag agtgaccccc gagattcctg ccgggctc 1908

<210> 234
<211> 636
<212> PRT
<213> Artificial Sequence

<220>
<223> DP47 (VH-CL) Fc hole monomeric 4-1BBL (71-248) chain

<400> 234

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Ser Gly Phe Asp Tyr Trp Gly Gln Gly Thr Leu Val Thr
100 105 110

Val Ser Ser Ala Ser Val Ala Ala Pro Ser Val Phe Ile Phe Pro Pro
115 120 125

Ser Asp Glu Gln Leu Lys Ser Gly Thr Ala Ser Val Val Cys Leu Leu
130 135 140

Asn Asn Phe Tyr Pro Arg Glu Ala Lys Val Gln Trp Lys Val Asp Asn
145 150 155 160

Ala Leu Gln Ser Gly Asn Ser Gln Glu Ser Val Thr Glu Gln Asp Ser
165 170 175

Lys Asp Ser Thr Tyr Ser Leu Ser Ser Thr Leu Thr Leu Ser Lys Ala
180 185 190

Asp Tyr Glu Lys His Lys Val Tyr Ala Cys Glu Val Thr His Gln Gly
195 200 205

Leu Ser Ser Pro Val Thr Lys Ser Phe Asn Arg Gly Glu Cys Asp Lys
210 215 220

Thr His Thr Cys Pro Pro Cys Pro Ala Pro Glu Ala Ala Gly Gly Pro
225 230 235 240

```
Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Gly Gly Gly Ser Gly Gly Gly Gly Ser Arg Glu Gly Pro Glu Leu
    450                 455                 460

Ser Pro Asp Asp Pro Ala Gly Leu Leu Asp Leu Arg Gln Gly Met Phe
465                 470                 475                 480

Ala Gln Leu Val Ala Gln Asn Val Leu Leu Ile Asp Gly Pro Leu Ser
```

485 490 495

Trp Tyr Ser Asp Pro Gly Leu Ala Gly Val Ser Leu Thr Gly Gly Leu
500 505 510

Ser Tyr Lys Glu Asp Thr Lys Glu Leu Val Val Ala Lys Ala Gly Val
515 520 525

Tyr Tyr Val Phe Phe Gln Leu Glu Leu Arg Arg Val Val Ala Gly Glu
530 535 540

Gly Ser Gly Ser Val Ser Leu Ala Leu His Leu Gln Pro Leu Arg Ser
545 550 555 560

Ala Ala Gly Ala Ala Ala Leu Ala Leu Thr Val Asp Leu Pro Pro Ala
565 570 575

Ser Ser Glu Ala Arg Asn Ser Ala Phe Gly Phe Gln Gly Arg Leu Leu
580 585 590

His Leu Ser Ala Gly Gln Arg Leu Gly Val His Leu His Thr Glu Ala
595 600 605

Arg Ala Arg His Ala Trp Gln Leu Thr Gln Gly Ala Thr Val Leu Gly
610 615 620

Leu Phe Arg Val Thr Pro Glu Ile Pro Ala Gly Leu
625 630 635

<210> 235
<211> 1341
<212> DNA
<213> Artificial Sequence

<220>
<223> nucleotide sequence of anti-FAP (4B9) Fc hole chain

<400> 235

gaggtgcagc tgctcgaaag cggcggagga ctggtgcagc ctggcggcag cctgagactg 60
tcttgcgccg ccagcggctt caccttcagc agctacgcca tgagctgggt ccgccaggcc 120
cctggcaagg gactggaatg ggtgtccgcc atcatcggct ctggcgccag cacctactac 180
gccgacagcg tgaagggccg gttcaccatc agccgggaca acagcaagaa caccctgtac 240
ctgcagatga acagcctgcg ggccgaggac accgccgtgt actactgcgc caagggatgg 300
ttcggcggct tcaactactg gggacagggc accctggtca cagtgtccag cgctagcacc 360
aagggcccct ccgtgttccc cctggccccc agcagcaaga gcaccagcgg cggcacagcc 420
gctctgggct gcctggtcaa ggactacttc cccgagcccg tgaccgtgtc ctggaacagc 480

ggagccctga cctccggcgt gcacaccttc cccgccgtgc tgcagagttc tggcctgtat 540
agcctgagca gcgtggtcac cgtgccttct agcagcctgg gcacccagac ctacatctgc 600
aacgtgaacc acaagcccag caacaccaag gtggacaaga aggtggagcc caagagctgc 660
gacaaaactc acacatgccc accgtgccca gcacctgaag ctgcaggggg accgtcagtc 720
ttcctcttcc ccccaaaacc caaggacacc ctcatgatct cccggacccc tgaggtcaca 780
tgcgtggtgg tggacgtgag ccacgaagac cctgaggtca agttcaactg gtacgtggac 840
ggcgtggagg tgcataatgc caagacaaag ccgcgggagg agcagtacaa cagcacgtac 900
cgtgtggtca gcgtcctcac cgtcctgcac caggactggc tgaatggcaa ggagtacaag 960
tgcaaggtct ccaacaaagc cctcggcgcc cccatcgaga aaaccatctc caaagccaaa 1020
gggcagcccc gagaaccaca ggtgtgcacc ctgcccccat cccgggatga gctgaccaag 1080
aaccaggtca gcctctcgtg cgcagtcaaa ggcttctatc ccagcgacat cgccgtggag 1140
tgggagagca atgggcagcc ggagaacaac tacaagacca cgcctcccgt gctggactcc 1200
gacggctcct tcttcctcgt gagcaagctc accgtggaca agagcaggtg gcagcagggg 1260
aacgtcttct catgctccgt gatgcatgag gctctgcaca accactacac gcagaagagc 1320
ctctccctgt ctccgggtaa a 1341

<210> 236
<211> 447
<212> PRT
<213> Artificial Sequence

<220>
<223> anti-FAP (4B9) Fc hole chain

<400> 236

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85 90 95

Ala Lys Gly Trp Phe Gly Gly Phe Asn Tyr Trp Gly Gln Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Ala Ala Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Gly Ala Pro Ile Glu Lys Thr Ile
325 330 335

Ser Lys Ala Lys Gly Gln Pro Arg Glu Pro Gln Val Cys Thr Leu Pro
340 345 350

Pro Ser Arg Asp Glu Leu Thr Lys Asn Gln Val Ser Leu Ser Cys Ala
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Val Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 237
<211> 1353
<212> DNA
<213> Artificial Sequence

<220>
<223> Nucleotide sequence anti-CD19(8B8-018) Fc hole chain

<400> 237

caggtccagc tggtgcagtc cggcgccgag gtcaagaaac ccggggcttc tgtgaaggtt 60

tcatgcaagg caagcggata caccttcacc gactatatca tgcattgggt caggcaggcc 120

cctggccaag gtctcgaatg gatgggctac attaacccat ataatgatgg ctccaaatac 180

accgagaagt ttcagggaag agtcactatg acatctgaca ccagtatcag cactgcttac 240

atggagctgt cccgccttcg gtctgatgac accgcagtgt attactgtgc caggggcaca 300

tattactacg gctcagctct gttcgactat tgggggcagg gaaccacagt aaccgtgagc 360

tccgctagca ccaagggccc ctccgtgttc cccctggccc ccagcagcaa gagcaccagc 420

ggcggcacag ccgctctggg ctgcctggtc aaggactact tccccgagcc cgtgaccgtg 480

tcctggaaca gcggagccct gacctccggc gtgcacacct tccccgccgt gctgcagagt 540

tctggcctgt atagcctgag cagcgtggtc accgtgcctt ctagcagcct gggcacccag 600

acctacatct gcaacgtgaa ccacaagccc agcaacacca aggtggacaa gaaggtggag 660

cccaagagct gcgacaaaac tcacacatgc ccaccgtgcc cagcacctga agctgcaggg 720

ggaccgtcag tcttcctctt ccccccaaaa cccaaggaca ccctcatgat ctcccggacc 780

cctgaggtca catgcgtggt ggtggacgtg agccacgaag accctgaggt caagttcaac 840

tggtacgtgg acggcgtgga ggtgcataat gccaagacaa agccgcggga ggagcagtac 900

aacagcacgt accgtgtggt cagcgtcctc accgtcctgc accaggactg gctgaatggc 960

aaggagtaca agtgcaaggt ctccaacaaa gccctcggcg cccccatcga gaaaaccatc 1020

tccaaagcca aagggcagcc ccgagaacca caggtgtgca ccctgccccc atcccgggat 1080

gagctgacca agaaccaggt cagcctctcg tgcgcagtca aaggcttcta tcccagcgac 1140

atcgccgtgg agtgggagag caatgggcag ccggagaaca actacaagac cacgcctccc 1200

gtgctggact ccgacggctc cttcttcctc gtgagcaagc tcaccgtgga caagagcagg 1260

tggcagcagg ggaacgtctt ctcatgctcc gtgatgcatg aggctctgca caaccactac 1320

acgcagaaga gcctctccct gtctccgggt aaa 1353

<210> 238
<211> 451
<212> PRT
<213> Artificial Sequence

<220>
<223> anti-CD19(8B8-018) Fc hole chain

<400> 238

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125
```

```
Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380
```

Trp Glu Ser Asn Gly Gln Pro Glu Asn Asn Tyr Lys Thr Thr Pro Pro
385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 239
<211> 2166
<212> DNA
<213> Artificial Sequence

<220>
<223> nucleotide sequence of dimeric hu 4-1BBL (71-254) - CL* Fc knob chain

<400> 239

agagagggcc ctgagctgag ccccgatgat cctgctggac tgctggacct gcggcagggc 60
atgtttgctc agctggtggc ccagaacgtg ctgctgatcg atggccccct gtcctggtac 120
agcgatcctg gactggctgg cgtgtcactg acaggcggcc tgagctacaa agaggacacc 180
aaagaactgg tggtggccaa ggccggcgtg tactacgtgt tctttcagct ggaactgcgg 240
agagtggtgg ccggcgaagg atctggctct gtgtctctgg ccctgcatct gcagcctctg 300
agaagcgctg ctggcgctgc agctctggca ctgacagtgg atctgcctcc tgccagctcc 360
gaggcccgga atagcgcatt tgggtttcaa ggcaggctgc tgcacctgtc tgccggccag 420
aggctgggag tgcatctgca cacagaggcc agggctagac acgcctggca gctgacacag 480
ggcgctacag tgctgggcct gttcagagtg acccccgaga ttccagccgg cctgccttct 540
ccaagaagcg aaggcggagg cggatctggc ggcggaggat ctagagaggg acccgaactg 600
tcccctgacg atccagccgg gctgctggat ctgagacagg gaatgttcgc ccagctggtg 660
gctcagaatg tgctgctgat tgacggacct ctgagctggt actccgaccc agggctggca 720
ggggtgtccc tgactggggg actgtcctac aaagaagata caaaagaact ggtggtggct 780
aaagctgggg tgtactatgt gtttttttcag ctggaactga ggcgggtggt ggctggggag 840
ggctcaggat ctgtgtccct ggctctgcat ctgcagccac tgcgctctgc tgctggcgca 900
gctgcactgg ctctgactgt ggacctgcca ccagcctcta gcgaggccag aaacagcgcc 960

ttcgggttcc aaggacgcct gctgcatctg agcgccggac agcgcctggg agtgcatctg 1020
catactgaag ccagagcccg gcatgcttgg cagctgactc aggggcaac tgtgctggga 1080
ctgtttcgcg tgacacctga gatccctgcc ggactgccaa gccctagatc agaagggggc 1140
ggaggttccg gaggggagg atctcgtacg gtggctgcac catctgtctt tatcttccca 1200
cccagcgacc ggaagctgaa gtctggcaca gccagcgtcg tgtgcctgct gaataacttc 1260
tacccccgcg aggccaaggt gcagtggaag gtggacaatg ccctgcagag cggcaacagc 1320
caggaaagcg tgaccgagca ggacagcaag gactccacct acagcctgag cagcaccctg 1380
accctgagca aggccgacta cgagaagcac aaggtgtacg cctgcgaagt gacccaccag 1440
ggcctgtcta gccccgtgac caagagcttc aaccggggcg agtgcgacaa gacccacacc 1500
tgtcctccat gccctgcccc tgaagctgct ggcggcccta gcgtgttcct gttcccccca 1560
aagcccaagg acaccctgat gatcagccgg acccctgaag tgacctgcgt ggtggtggat 1620
gtgtcccacg aggaccctga agtgaagttc aattggtacg tggacggcgt ggaagtgcac 1680
aatgccaaga ccaagccgcg ggaggagcag tacaacagca cgtaccgtgt ggtcagcgtc 1740
ctcaccgtcc tgcaccagga ctggctgaat ggcaaggagt acaagtgcaa ggtctccaac 1800
aaagccctcg gcgcccccat cgagaaaacc atctccaaag ccaaagggca gccccgagaa 1860
ccacaggtgt acaccctgcc cccatgccgg gatgagctga ccaagaacca ggtcagcctg 1920
tggtgcctgg tcaaaggctt ctatcccagc gacatcgccg tggagtggga gagcaatggg 1980
cagccggaga acaactacaa gaccacgcct cccgtgctgg actccgacgg ctccttcttc 2040
ctctacagca agctcaccgt ggacaagagc aggtggcagc aggggaacgt cttctcatgc 2100
tccgtgatgc atgaggctct gcacaaccac tacacgcaga agagcctctc cctgtctccg 2160
ggtaaa 2166

<210> 240
<211> 891
<212> DNA
<213> Artificial Sequence

<220>
<223> nucleotide sequence of monomeric hu 4-1BBL (71-254) - CH1*

<400> 240

```
agagagggcc ctgagctgag ccccgatgat cctgctggac tgctggacct gcggcagggc      60
atgtttgctc agctggtggc ccagaacgtg ctgctgatcg atggcccct gtcctggtac      120
agcgatcctg gactggctgg cgtgtcactg acaggcggcc tgagctacaa agaggacacc     180
aaagaactgg tggtggccaa ggccggcgtg tactacgtgt tctttcagct ggaactgcgg     240
agagtggtgg ccggcgaagg atctggctct gtgtctctgg ccctgcatct gcagcctctg     300
agaagcgctg ctggcgctgc agctctggct ctgacagtgg atctgcctcc tgccagctcc     360

gaggcccgga atagcgcatt tgggtttcaa ggccggctgc tgcacctgtc tgccggccag     420
agactgggag tgcatctgca cacagaggcc agagccaggc acgcctggca gctgacacag     480
ggcgctacag tgctgggcct gttcagagtg acccccgaga ttcctgccgg cctgcctagc     540
cctagatctg aaggcggcgg aggttccgga ggcggaggat ctgctagcac aaagggcccc     600
agcgtgttcc ctctggcccc tagcagcaag agcacatctg gcggaacagc cgccctgggc     660
tgcctggtgg aagattactt ccccgagccc gtgaccgtgt cctggaattc tggcgccctg     720
acaagcggcg tgcacacctt tccagccgtg ctgcagagca gcggcctgta ctctctgagc     780
agcgtcgtga cagtgcccag cagctctctg ggcacccaga cctacatctg caacgtgaac     840
cacaagccca gcaacaccaa ggtggacgag aaggtggaac ccaagtcctg c              891
```

<210> 241
<211> 1353
<212> DNA
<213> Artificial Sequence

<220>
<223> nucleotide sequence of anti- CD19(8B8-2B11) Fc hole chain

<400> 241

```
caggtgcaat tggttcaatc tggtgctgaa gtaaaaaaac cgggcgcttc cgttaaagtg      60
agctgcaaag catctggtta caccttcact gactatatca tgcactgggt tcgtcaggcc     120
ccgggccagg gtctggagtg gatgggctac attaacccat acaacgacgg ttccaaatat     180
accgagaaat tccagggccg cgtcacgatg accagcgaca cttctatctc caccgcgtac     240
atggaactgt ctagactgcg ttctgacgac accgctgttt actattgtgc acgcggtacc     300
tactactacg gtccacagct gtttgattac tggggccaag gtaccacggt gaccgtaagc     360
tctgctagca ccaagggccc ctccgtgttc cccctggccc ccagcagcaa gagcaccagc     420
ggcggcacag ccgctctggg ctgcctggtc aaggactact tccccgagcc cgtgaccgtg     480
tcctggaaca gcggagccct gacctccggc gtgcacacct tccccgccgt gctgcagagt     540
tctggcctgt atagcctgag cagcgtggtc accgtgcctt ctagcagcct gggcacccag     600
acctacatct gcaacgtgaa ccacaagccc agcaacacca aggtggacaa gaaggtggag     660
cccaagagct gcgacaaaac tcacacatgc ccaccgtgcc cagcacctga agctgcaggg     720
ggaccgtcag tcttcctctt ccccccaaaa cccaaggaca ccctcatgat ctcccggacc     780
cctgaggtca catgcgtggt ggtggacgtg agccacgaag accctgaggt caagttcaac     840
tggtacgtgg acggcgtgga ggtgcataat gccaagacaa agccgcggga ggagcagtac     900
aacagcacgt accgtgtggt cagcgtcctc accgtcctgc accaggactg gctgaatggc     960
aaggagtaca agtgcaaggt ctccaacaaa gccctcggcg cccccatcga gaaaaccatc    1020
tccaaagcca aagggcagcc ccgagaacca caggtgtgca ccctgccccc atcccgggat    1080
gagctgacca agaaccaggt cagcctctcg tgcgcagtca aaggcttcta tcccagcgac    1140
atcgccgtgg agtgggagag caatgggcag ccggagaaca actacaagac cacgcctccc    1200
gtgctggact ccgacggctc cttcttcctc gtgagcaagc tcaccgtgga caagagcagg    1260
tggcagcagg ggaacgtctt ctcatgctcc gtgatgcatg aggctctgca caaccactac    1320
acgcagaaga gcctctccct gtctccgggt aaa                                 1353
```

<210> 242
<211> 722
<212> PRT
<213> Artificial Sequence

<220>
<223> Dimeric hu 4-1BBL (71-254) - CL* Fc knob chain

<400> 242

```
Arg Glu Gly Pro Glu Leu Ser Pro Asp Asp Pro Ala Gly Leu Leu Asp
1               5                   10                  15

Leu Arg Gln Gly Met Phe Ala Gln Leu Val Ala Gln Asn Val Leu Leu
            20                  25                  30

Ile Asp Gly Pro Leu Ser Trp Tyr Ser Asp Pro Gly Leu Ala Gly Val
        35                  40                  45

Ser Leu Thr Gly Gly Leu Ser Tyr Lys Glu Asp Thr Lys Glu Leu Val
    50                  55                  60

Val Ala Lys Ala Gly Val Tyr Tyr Val Phe Phe Gln Leu Glu Leu Arg
65                  70                  75                  80

Arg Val Val Ala Gly Glu Gly Ser Gly Ser Val Ser Leu Ala Leu His
                85                  90                  95

Leu Gln Pro Leu Arg Ser Ala Ala Gly Ala Ala Ala Leu Ala Leu Thr
            100                 105                 110

Val Asp Leu Pro Pro Ala Ser Ser Glu Ala Arg Asn Ser Ala Phe Gly
        115                 120                 125

Phe Gln Gly Arg Leu Leu His Leu Ser Ala Gly Gln Arg Leu Gly Val
    130                 135                 140

His Leu His Thr Glu Ala Arg Ala Arg His Ala Trp Gln Leu Thr Gln
145                 150                 155                 160
```

Gly Ala Thr Val Leu Gly Leu Phe Arg Val Thr Pro Glu Ile Pro Ala
165 170 175

Gly Leu Pro Ser Pro Arg Ser Glu Gly Gly Gly Gly Ser Gly Gly Gly
180 185 190

Gly Ser Arg Glu Gly Pro Glu Leu Ser Pro Asp Asp Pro Ala Gly Leu
195 200 205

Leu Asp Leu Arg Gln Gly Met Phe Ala Gln Leu Val Ala Gln Asn Val
210 215 220

Leu Leu Ile Asp Gly Pro Leu Ser Trp Tyr Ser Asp Pro Gly Leu Ala
225 230 235 240

Gly Val Ser Leu Thr Gly Gly Leu Ser Tyr Lys Glu Asp Thr Lys Glu
245 250 255

Leu Val Val Ala Lys Ala Gly Val Tyr Tyr Val Phe Phe Gln Leu Glu
260 265 270

Leu Arg Arg Val Val Ala Gly Glu Gly Ser Gly Ser Val Ser Leu Ala
275 280 285

Leu His Leu Gln Pro Leu Arg Ser Ala Ala Gly Ala Ala Ala Leu Ala
290 295 300

Leu Thr Val Asp Leu Pro Pro Ala Ser Ser Glu Ala Arg Asn Ser Ala
305 310 315 320

Phe Gly Phe Gln Gly Arg Leu Leu His Leu Ser Ala Gly Gln Arg Leu
325 330 335

Gly Val His Leu His Thr Glu Ala Arg Ala Arg His Ala Trp Gln Leu
340 345 350

Thr Gln Gly Ala Thr Val Leu Gly Leu Phe Arg Val Thr Pro Glu Ile
355 360 365

Pro Ala Gly Leu Pro Ser Pro Arg Ser Glu Gly Gly Gly Gly Ser Gly
370 375 380

Gly Gly Gly Ser Arg Thr Val Ala Ala Pro Ser Val Phe Ile Phe Pro
385 390 395 400

Pro Ser Asp Arg Lys Leu Lys Ser Gly Thr Ala Ser Val Val Cys Leu
405 410 415

```
Leu Asn Asn Phe Tyr Pro Arg Glu Ala Lys Val Gln Trp Lys Val Asp
            420                 425                 430

Asn Ala Leu Gln Ser Gly Asn Ser Gln Glu Ser Val Thr Glu Gln Asp
        435                 440                 445

Ser Lys Asp Ser Thr Tyr Ser Leu Ser Ser Thr Leu Thr Leu Ser Lys
    450                 455                 460

Ala Asp Tyr Glu Lys His Lys Val Tyr Ala Cys Glu Val Thr His Gln
465                 470                 475                 480

Gly Leu Ser Ser Pro Val Thr Lys Ser Phe Asn Arg Gly Glu Cys Asp
                485                 490                 495

Lys Thr His Thr Cys Pro Pro Cys Pro Ala Pro Glu Ala Ala Gly Gly
            500                 505                 510

Pro Ser Val Phe Leu Phe Pro Pro Lys Pro Lys Asp Thr Leu Met Ile
        515                 520                 525

Ser Arg Thr Pro Glu Val Thr Cys Val Val Val Asp Val Ser His Glu
    530                 535                 540

Asp Pro Glu Val Lys Phe Asn Trp Tyr Val Asp Gly Val Glu Val His
545                 550                 555                 560

Asn Ala Lys Thr Lys Pro Arg Glu Glu Gln Tyr Asn Ser Thr Tyr Arg
                565                 570                 575

Val Val Ser Val Leu Thr Val Leu His Gln Asp Trp Leu Asn Gly Lys
            580                 585                 590

Glu Tyr Lys Cys Lys Val Ser Asn Lys Ala Leu Gly Ala Pro Ile Glu
        595                 600                 605

Lys Thr Ile Ser Lys Ala Lys Gly Gln Pro Arg Glu Pro Gln Val Tyr
    610                 615                 620

Thr Leu Pro Pro Cys Arg Asp Glu Leu Thr Lys Asn Gln Val Ser Leu
625                 630                 635                 640

Trp Cys Leu Val Lys Gly Phe Tyr Pro Ser Asp Ile Ala Val Glu Trp
                645                 650                 655

Glu Ser Asn Gly Gln Pro Glu Asn Asn Tyr Lys Thr Thr Pro Pro Val
            660                 665                 670
```

Leu Asp Ser Asp Gly Ser Phe Phe Leu Tyr Ser Lys Leu Thr Val Asp
675 680 685

Lys Ser Arg Trp Gln Gln Gly Asn Val Phe Ser Cys Ser Val Met His
690 695 700

Glu Ala Leu His Asn His Tyr Thr Gln Lys Ser Leu Ser Leu Ser Pro
705 710 715 720

Gly Lys

<210> 243
<211> 297
<212> PRT
<213> Artificial Sequence

<220>
<223> Monomeric hu 4-1BBL (71-254) - CH1*

<400> 243

Arg Glu Gly Pro Glu Leu Ser Pro Asp Asp Pro Ala Gly Leu Leu Asp
1 5 10 15

Leu Arg Gln Gly Met Phe Ala Gln Leu Val Ala Gln Asn Val Leu Leu
20 25 30

Ile Asp Gly Pro Leu Ser Trp Tyr Ser Asp Pro Gly Leu Ala Gly Val
35 40 45

Ser Leu Thr Gly Gly Leu Ser Tyr Lys Glu Asp Thr Lys Glu Leu Val
50 55 60

Val Ala Lys Ala Gly Val Tyr Tyr Val Phe Phe Gln Leu Glu Leu Arg
65 70 75 80

Arg Val Val Ala Gly Glu Gly Ser Gly Ser Val Ser Leu Ala Leu His
85 90 95

Leu Gln Pro Leu Arg Ser Ala Ala Gly Ala Ala Ala Leu Ala Leu Thr
100 105 110

Val Asp Leu Pro Pro Ala Ser Ser Glu Ala Arg Asn Ser Ala Phe Gly
115 120 125

Phe Gln Gly Arg Leu Leu His Leu Ser Ala Gly Gln Arg Leu Gly Val
130 135 140

His Leu His Thr Glu Ala Arg Ala Arg His Ala Trp Gln Leu Thr Gln
145 150 155 160

Gly Ala Thr Val Leu Gly Leu Phe Arg Val Thr Pro Glu Ile Pro Ala
165 170 175

Gly Leu Pro Ser Pro Arg Ser Glu Gly Gly Gly Gly Ser Gly Gly Gly
180 185 190

Gly Ser Ala Ser Thr Lys Gly Pro Ser Val Phe Pro Leu Ala Pro Ser
195 200 205

Ser Lys Ser Thr Ser Gly Gly Thr Ala Ala Leu Gly Cys Leu Val Glu
210 215 220

Asp Tyr Phe Pro Glu Pro Val Thr Val Ser Trp Asn Ser Gly Ala Leu
225 230 235 240

Thr Ser Gly Val His Thr Phe Pro Ala Val Leu Gln Ser Ser Gly Leu
245 250 255

Tyr Ser Leu Ser Ser Val Val Thr Val Pro Ser Ser Ser Leu Gly Thr
260 265 270

Gln Thr Tyr Ile Cys Asn Val Asn His Lys Pro Ser Asn Thr Lys Val
275 280 285

Asp Glu Lys Val Glu Pro Lys Ser Cys
290 295

<210> 244
<211> 451
<212> PRT
<213> Artificial Sequence

<220>
<223> CD19(8B8-2B11) Fc hole chain

<400> 244

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Ile Met His Trp Val Arg Gln Ala Pro Gly Gln Gly Leu Glu Trp Met
35 40 45

```
Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Gln Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300
```

Arg Val Val Ser Val Leu Thr Val Leu His Gln Asp Trp Leu Asn Gly
305 310 315 320

Lys Glu Tyr Lys Cys Lys Val Ser Asn Lys Ala Leu Gly Ala Pro Ile
325 330 335

Glu Lys Thr Ile Ser Lys Ala Lys Gly Gln Pro Arg Glu Pro Gln Val
340 345 350

Cys Thr Leu Pro Pro Ser Arg Asp Glu Leu Thr Lys Asn Gln Val Ser
355 360 365

Leu Ser Cys Ala Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 245
<211> 15
<212> PRT
<213> Artificial Sequence

<220>
<223> avi tag

<400> 245

Gly Leu Asn Asp Ile Phe Glu Ala Gln Lys Ile Glu Trp His Glu
1 5 10 15